# EUROPEAN PATENT APPLICATION

(11) **EP 4 775 567 A1**
(43) Date of publication of application: **15.07.2026**
(21) Application number: 24861497.6
(22) Date of filing: 12.04.2024
(51) Int. Cl.: C07C 237/08

(54) **IONIZABLE CATIONIC LIPID COMPOUNDS AS WELL AS PREPARATION METHOD THEREFOR AND USE THEREOF**

(30) Priority: 07.09.2023 CN 202311149923
(71) Applicant: Scindy Pharmaceutical (Suzhou) Co., Ltd, Suzhou, Jiangsu 215124 (CN)
(72) Inventor: CHEN, Xiaobao, Suzhou, Jiangsu 215124 (CN)
(74) Representative: CH Kilger Anwaltspartnerschaft mbB
(86) International application number: PCT/CN2024/087610
(87) International publication number: WO 2025/050647

(57) **Abstract**

Provided in the present invention are ionizable cationic lipid compounds as well as a preparation method therefor and the use thereof, belonging to the technical field of biological medicines. The ionizable cationic lipid compounds provided by the present invention are compounds of Formula (1), or salts, stereoisomers and tautomers thereof, can be used as surfactants for various fields, and particularly can be used for lipid compositions capable of delivering therapeutic agents. Lipid nanoparticle prepared from said compounds can show good effectiveness and safety in cells and in animal bodies.

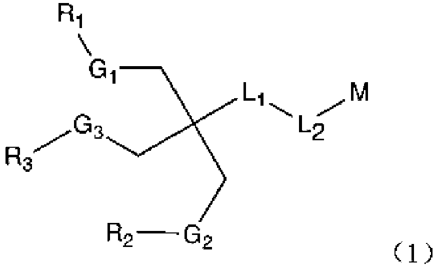

## Description

### TECHNICAL FIELD

The present invention belongs to the technical field of biological medicine, and specifically relates to an ionizable cationic lipid compound, as well as a preparation method therefor and use thereof.

### BACKGROUND ART

Gene therapy uses nucleic acids as functional molecules to activate biological therapy. Owing to global efforts in human genome annotation and recent discoveries such as RNA interference (RNAi) and CRISPR-based genome editing, nucleic acids have gained extensive attention. Gene therapy uses genetic materials to alter the expression of target genes or modify the biological characteristics of living cells to meet therapeutic requirements. In recent years, a number of gene therapy products have been approved by regulatory authorities for various applications, for example, the use of mRNA vaccines against the COVID-19 pandemic. Gene therapy may be divided into three main approaches: 1) editing mutant genes using CRISPR-Cas technology; 2) up-regulating gene expression by insertion of functional gene copies, the molecules used including DNA plasmids (pDNA), minicircle DNA (mcDNA), synthetic mRNA, circular RNA, and self-amplifying RNA (saRNA); 3) down-regulating gene expression using molecules such as small interfering RNA (siRNA), antisense oligonucleotides (ASO), short hairpin RNA (shRNA), and microRNA (miRNA).

Due to their negative charge, high molecular weight and hydrophilicity, nucleic acids exhibit poor permeability across cell membranes. In addition, nucleic acids have low stability *in vivo* and are rapidly cleared by the host extracellularly. Therefore, delivering nucleic acids to active sites within cells is the most challenging part of gene therapy. This is particularly true in the delivery of RNA molecules, which usually have short lifespan and low retention rates in cells, requiring more frequent administration. Thus, the payloads and the toxicity of vectors are two very important evaluation indicators. The vector itself needs to overcome extracellular and intracellular obstacles, to provide protection against nuclease activity in the blood, to enhance and assist cellular uptake, and to promote endosomal escape after entering cells.

Commonly used nucleic acid vectors are classified into two categories: viral vectors and non-viral vectors. Viral gene delivery systems have high cell transfection efficiency. However, since the human body produces neutralizing antibodies against the vectors or pre-existing antibodies against viral vectors, the vectors are rapidly cleared. Meanwhile, the capacity of viral vectors is generally relatively small (usually less than 7 kb). Therefore, lipid-based non-viral gene delivery systems have become the mainstream of the vector delivery systems.

Ionizable cationic lipids are an important component of lipid non-viral vectors. In terms of chemical structure, ionizable cationic lipids are amphiphilic molecules with a chemical structure composed of three parts: a head group, a linker, and a tail chain. Generally, the head group contains one or more ionizable sites, so the molecules may be neutral or positively charged under different pH conditions. Below their pKa, ionizable lipids may form lipophilic drug complexes with oppositely charged nucleic acids, anionic small molecules, peptides, proteins, and heparin. Lipids and nucleic acids may form lipid nanoparticles (LNPs) through electrostatic interactions.

In pharmaceutical products, ionizable cationic lipids possess pH-dependent cationic properties and have certain safety problems. First-generation cationic lipids (e.g., DOTMA, DOTAP, etc.) may bind to cell membranes and nuclear membranes, triggering lysosomal release of degradative enzymes, leading to failure of mitochondrial permeability and functioning, generating reactive oxygen species, altering the functions of cytoplasmic enzymes, and damaging DNA. To overcome this defect of cationic lipids, around 2001, Acuitas designed second-generation lipid structures with dimethyl tertiary amine as the head group. Compared with the first-generation lipid structures, the charge property of lipid structures with dimethyl tertiary amine as the head group may be regulated by pH. Therefore, starting from the second-generation lipid structures, cationic lipids for nucleic acid vectors are also referred to as ionizable cationic lipids (see CN105873902B, CN107207428A, CN110003066B, CN110325511B, and CN110337429B). Common second-generation ionizable cationic lipids include D-Lin-MC3-DMA, DLin-K-DMA, DlinDMA, and the like. (CN107922364B and CN108368028B). The second-generation ionizable lipids have been successfully used as vectors for siRNA drugs. For example, Acuitas Therapeutics successfully developed the lipid D-Lin-MC3-DMA, and the siRNA product Onpattro developed in cooperation with Alnylam became the world's first nucleic acid drug, which was approved for marketing in 2018. Thereafter, Moderna developed third-generation lipids with hydroxy tertiary amine as the head group (CN110520409A and CN114746398A). The third-generation ionizable lipids are positively charged under acidic pH conditions, which is desirable for complexation with nucleic acids and enabling endosomal escape after entering cells. Currently, ionizable cationic lipids with desirable performance include ALC-0315 (4-hydroxybutyl)azanediyl-2-(hexane-6,1-diyl)bis(2-hexyldecanoate) and Lipid H (SM-102)
(9-heptadecyl-8-{(2-hydroxyethyl)[6-oxo-6-(undecyloxy)hexyl]amino } octanoate), which are used in the coronavirus (COVID-19) mRNA vaccines BNT 162b and mRNA-1273, respectively.

At present, most ionizable cationic lipids protected in patents use tertiary amine groups as a central functional group for introduction of head groups and tail chains. Among the three functional groups on the tertiary amine, one of them is used to introduce the head group, and the other two are used to introduce tail chains. The ionizable cationic lipid compounds provided by the present invention (including stereoisomers, tautomers thereof and salts corresponding to these lipids) use a tetrafunctional compound as a core unit, and corresponding head groups and tail chains are introduced through terminal functional groups of the tetrafunctional compound. In the present invention, one of the four functional groups is used to introduce the head group, and the other three functional groups are used to introduce tail chains, as shown in Formula (1):

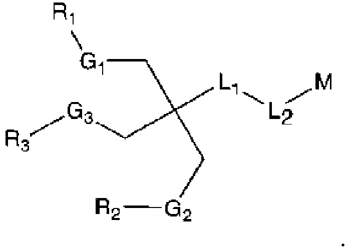

The ionizable cationic lipid compounds provided by the present invention are amphiphilic lipid compound constructed by using an ionizable cationic compound and analogs thereof as the ionizable lipid head group, lipids such as fatty acids as tail chains, and a tetrafunctional compound as a linker, where the ionizable lipid head group, tail chains and linker are connected by biodegradable chemical bonds (such as ester bonds or amide bonds, etc.). During the construction of the structure of the ionizable cationic lipid compound in the present invention, biodegradable functional groups are introduced. Since the biodegradable chemical bonds may be cleaved by various enzymes *in vivo,* such as lipases, esterases, and proteases and rapidly hydrolyzed into endogenous metabolites, cytotoxicity may be reduced and the safety of use may be improved.

### SUMMARY OF THE INVENTION

In view of the problems existing in the prior art, the present invention provides an ionizable cationic lipid compound, as well as a preparation method and use thereof. The ionizable cationic lipid compounds provided by the present invention are amphiphilic lipid compound constructed by using an ionizable cationic compound and analogs thereof as the ionizable lipid head group, lipids such as fatty acids as tail chains, and a tetrafunctional compound as a linker, where the ionizable lipid head group, tail chains and linker are connected by biodegradable chemical bonds (such as ester bonds or amide bonds, etc.). The biodegradable chemical bonds may be cleaved by various enzymes *in vivo,* such as lipases, esterases and proteases, and rapidly hydrolyzed into endogenous metabolites, resulting in reduced cytotoxicity.

To achieve the above objectives, in a first aspect, the present invention provides an ionizable cationic lipid compound, which is a compound of Formula (1), or a salt, a stereoisomer or a tautomer thereof: 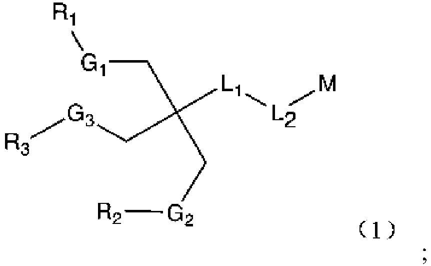
wherein R₁, R₂ and R₃ are each independently H, a C₅₋₄₀ linear or branched alkyl group, a C₅₋₄₀ linear or branched alkenyl group, a C₅₋₄₀ linear or branched alkynyl group, a 3-6 membered saturated or partially unsaturated cyclic hydrocarbon group containing 1-3 side chains, or a 6-10 membered aromatic group containing 1-3 side chains; the side chains are each independently selected from a C₁₀₋₃₀ linear or branched alkyl group, a C₁₀₋₃₀ linear or branched alkenyl group, and a C₁₀₋₃₀ linear or branched alkynyl group; with the proviso that at most one of R₁, R₂ and R₃ is H;
M is selected from -NR₄R₅, a saturated or partially unsaturated 3-6 membered heterocyclic group containing at least one nitrogen atom, and a 6-10 membered heteroaryl group containing at least one nitrogen atom, the heterocyclic group or heteroaryl group are unsubstituted or substituted with one or more of -OH, carboxyl group, amino group, oxo group, or halogen;
R₄ and R₅ are each independently H, a C₁₋₆ linear or branched alkyl group, a C₂₋₆ linear or branched alkenyl group, or a C₂₋₆ linear or branched alkynyl group, the C₁₋₆ linear or branched alkyl group, C₂₋₆ linear or branched alkenyl group or C₂₋₆ linear or branched alkynyl group are unsubstituted or substituted with one or more of -OH, carboxyl group, amino group, amido group, amidino group, guanidino group and halogen;
G₁, G₂ and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)-, or -OP(=O)(OR₆)O-; wherein each of R₆ and R₁₃ is independently selected from H, hydroxyl group, a C₁₋₃₀ linear or branched alkyl or cycloalkyl group, and a C₂₋₃₀ linear or branched alkenyl group;
L₁ is selected from -X₁- or -(CR₇R₈)ₘ-X₁-, wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)-, or -OP(=O)(OR₁₄)O-; m is an integer of 2-6; R₇ and R₈ are each independently H, hydroxyl group, halogen, a C₁₋₆ linear or branched alkyl or cycloalkyl group, or a C₂₋₆ linear or branched alkenyl group; each R₁₄ and R₁₅ are independently selected from H, a C₁₋₃₀ linear or branched alkyl or cycloalkyl group, and a C₂₋₃₀ linear or branched alkenyl group;
L₂ is -(CR₉R₁₀)ₙ- or -(CR₉R₁₀)ₙ-X₂-(CR₁₁R₁₂)ₖ-, wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, NR₁₆-S(=O)₂-, -P(=O)(OR₁₆)O-, -OP(=O)(OR₁₆)-, or -OP(=O)(OR₁₆)O-; n is an integer of 1-6; k is an integer of 1-6; R₉, R₁₀, R₁₁ and R₁₂ are each independently H, hydroxyl group, halogen, a C₁₋₆ linear or branched alkyl or cycloalkyl group, or a C₂₋₆ linear or branched alkenyl group; each of R₁₆ and R₁₇ is independently selected from H, a C₁₋₃₀ linear or branched alkyl or cycloalkyl group, and a C₂₋₃₀ linear or branched alkenyl group;
wherein the alkyl, cycloalkyl and alkenyl groups in R₄ to R₁₇ are unsubstituted or substituted with one or more groups selected from hydroxyl group, mercapto group, amino group, substituted amino group, and halogen;
the salt does not include quaternary ammonium salts.

In a preferred embodiment, R₁, R₂ and R₃ are each independently the following group: 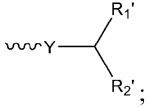 wherein Y is absent, or is a C₁₋₃₀ linear or branched alkyl or cycloalkyl group, a C₂₋₂₀ linear or branched alkenyl group, or a C₂₋₂₀ linear or branched alkynyl group; R₁' and R₂' are each independently H, a C₁₋₃₀ linear or branched alkyl group, a C₂₋₃₀ linear or branched alkenyl group, or a C₂₋₃₀ linear or branched alkynyl group, and the total length of the carbon chain of Y, R₁' and R₂' is 8-40.

In a preferred embodiment, R₁, R₂ and R₃ are each independently selected from the following groups: 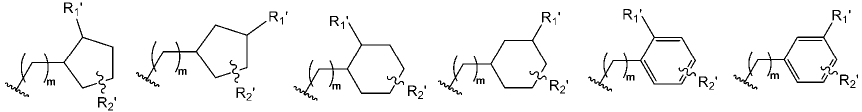 wherein R₁' and R₂' are each independently H, a C₁₋₃₀ linear or branched alkyl group, a C₂₋₃₀ linear or branched alkenyl group, or a C₂₋₃₀ linear or branched alkynyl group, and the total length of the carbon chain of R₁' and R₂' is 8-30.

In a preferred embodiment, R₁, R₂ and R₃ are each independently any one of the following groups:

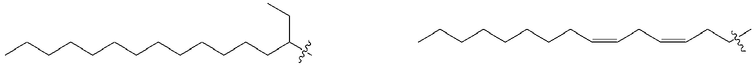

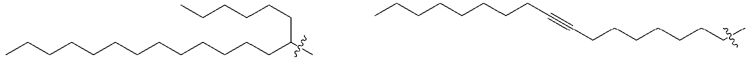

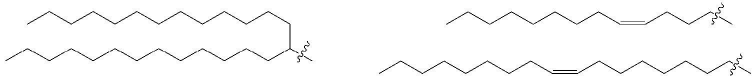

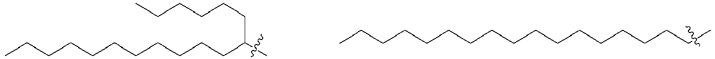

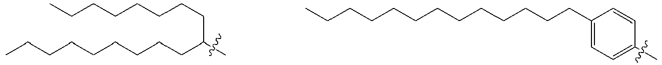

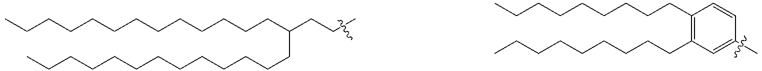

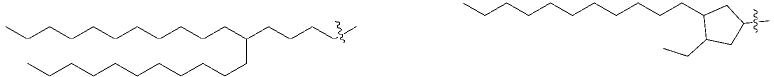

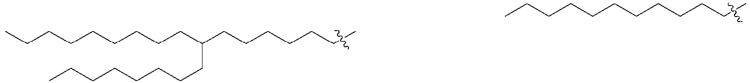

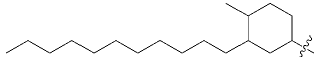

In a preferred embodiment, G₁, G₂ and G₃ are each independently -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)-, or -OP(=O)(OR₆)O-.

In a preferred embodiment, L₁ is selected from -(CR₇R₈)ₘ-X₁-, wherein X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)-, or -OP(=O)(OR₁₄)O-.

In a preferred embodiment, L₂ is -(CR₉R₁₀)ₙ-X₂-(CR₁₁R₁₂)ₖ-, wherein X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)-, or -OP(=O)(OR₁₆)O-.

In a preferred embodiment, M is selected from the following structures: 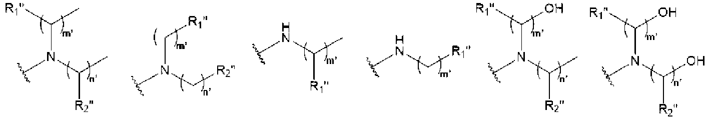 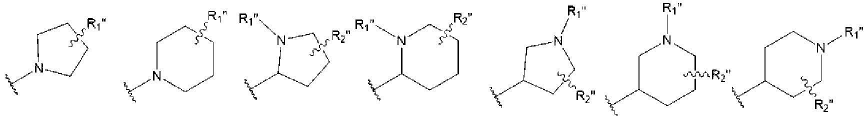 wherein m' and n' are each independently an integer of 0-6; R₁" and R₂" are each independently H, a C₁₋₆ alkyl group, a C₂₋₆ alkenyl group, guanidino group, amidino group, amido group, aliphatic amino group, or a 3-10 membered nitrogen-containing heterocycle; the nitrogen-containing heterocycle is selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine and pyrimidine, and tautomers thereof, which are unsubstituted or optionally substituted with one or more organic groups selected from hydroxyl group, mercapto group, amino group, substituted amino group, halogen, C₁₋₆ alkyl group, C₂₋₆ alkenyl group, C₂₋₆ alkynyl group and C₆₋₁₄ aryl group.

In a preferred embodiment, the compound of Formula (1) is selected from compounds represented by Formula (1A):

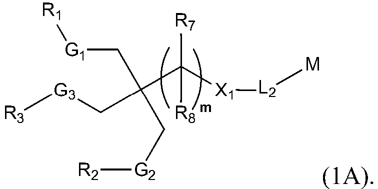

In a preferred embodiment, the compound of Formula (1) is selected from compounds represented by Formula (1B):

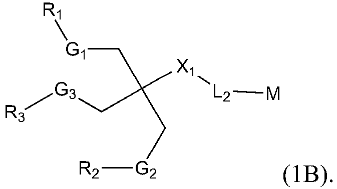

In a preferred embodiment, the compound of Formula (1) is selected from compounds of Formula (1 C):

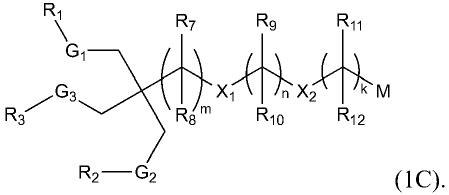

In a preferred embodiment, the compound of Formula (1) is selected from compounds represented by Formula (1D):

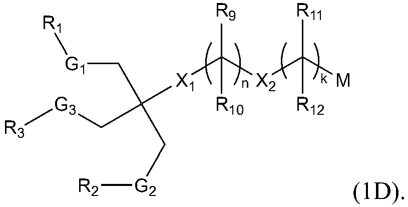

In a preferred embodiment, the compound of Formula (1) is selected from compounds represented by Formula (1E):

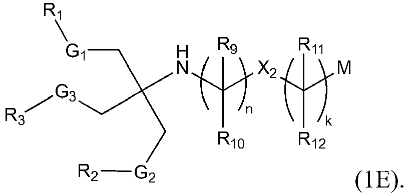

In a preferred embodiment, the compound of Formula (1) is selected from compounds represented by Formula (1F):

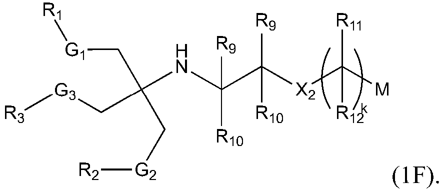

In a preferred embodiment, the compound of Formula (1) is selected from compounds represented by Formula (1G):

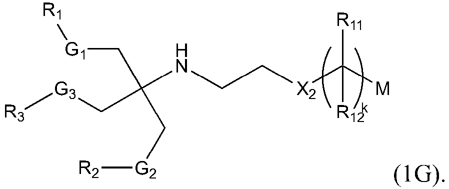

In a preferred embodiment, the compound of Formula (1) is selected from compounds represented by Formula (1H):

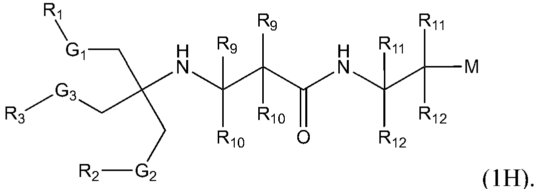

In a preferred embodiment, M is any one of the following groups:

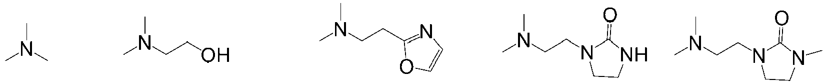

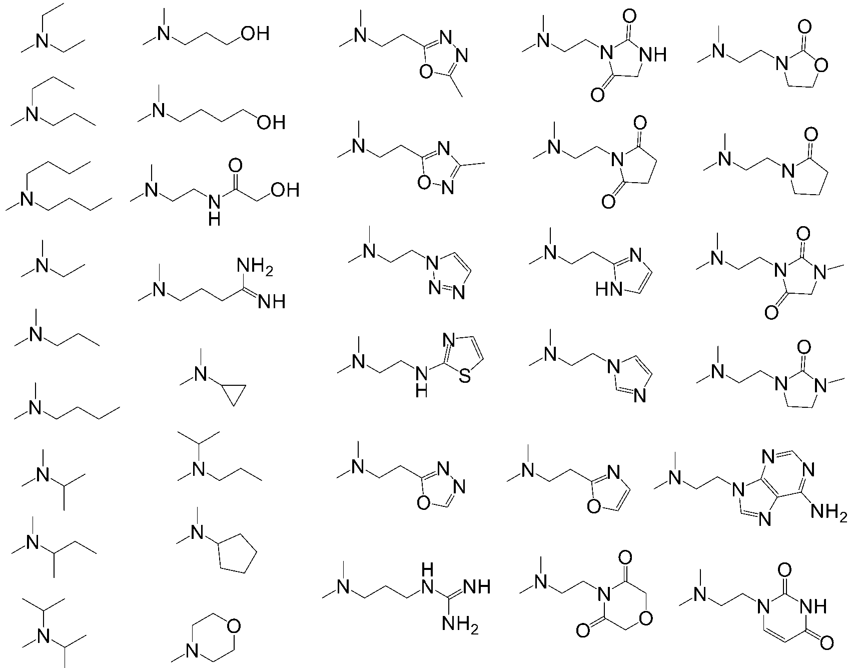

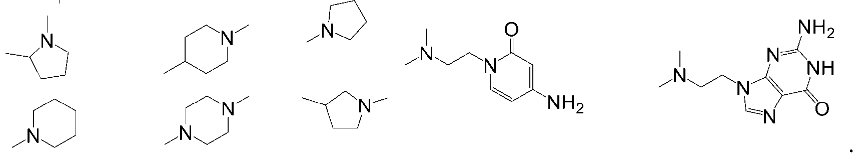

In a preferred embodiment, the compound of Formula (1) is selected from compounds represented by Formula (1I):

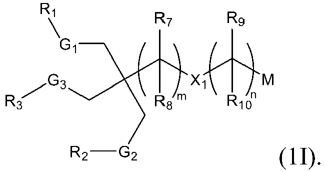

In a preferred embodiment, the compound of Formula (1) is selected from compounds represented by Formula (1J):

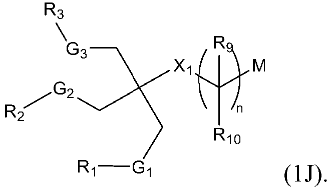

In a preferred embodiment, the compound of Formula (1) is selected from compounds represented by Formula (1K):

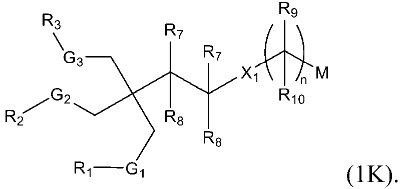

In a preferred embodiment, Y is absent, and the compound of Formula (1) is selected from compounds represented by Formula (1L):

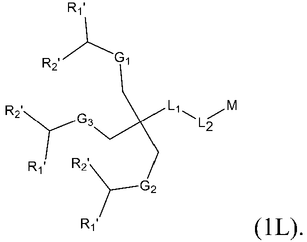

In a preferred embodiment, the compound of Formula (1) is selected from compounds represented by Formula (1M):

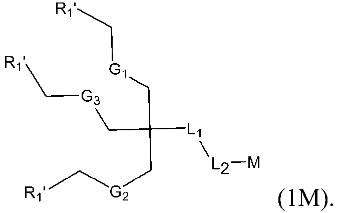

In a preferred embodiment, the compound of Formula (1) is selected from:

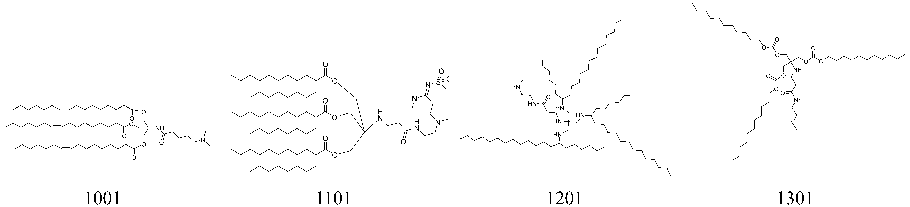

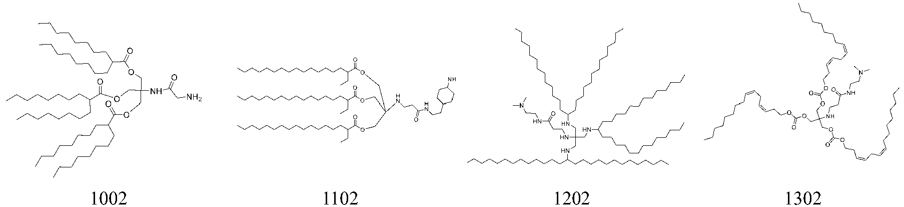

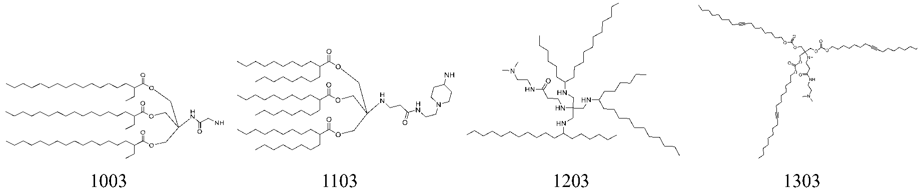

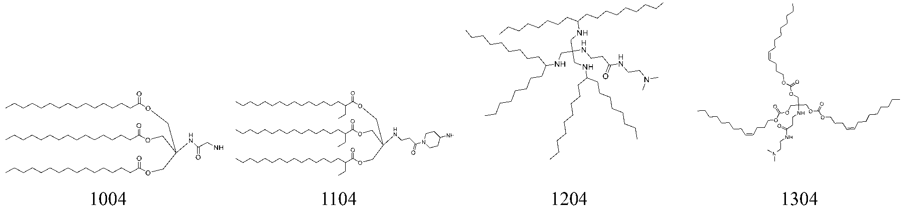

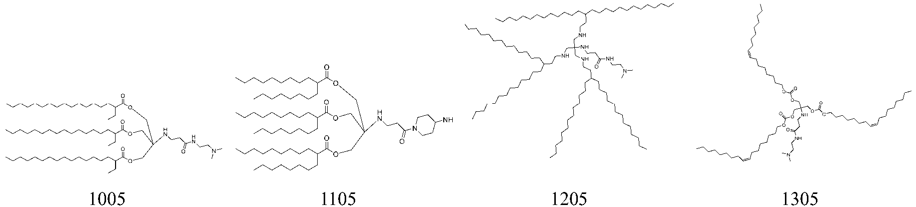

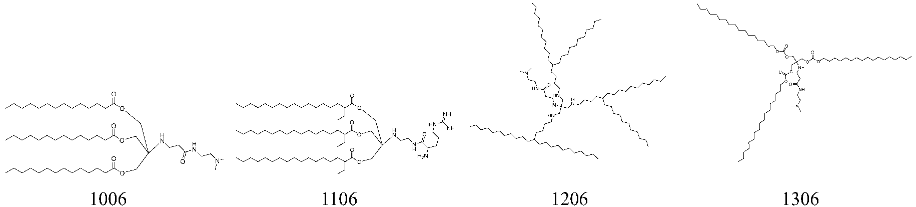

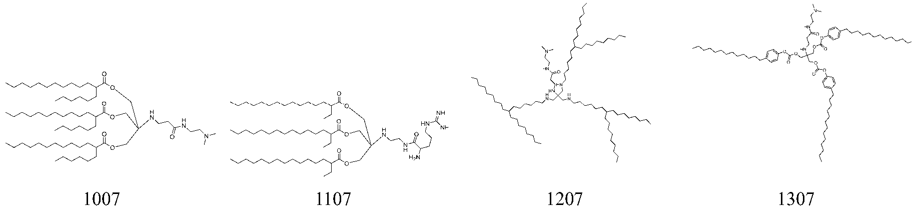

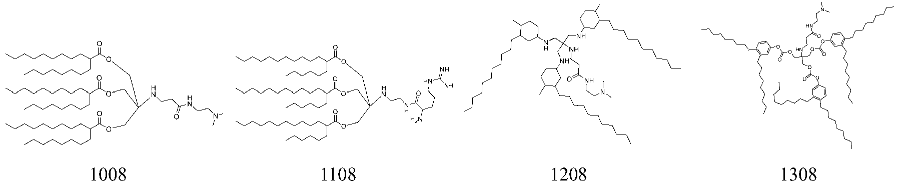

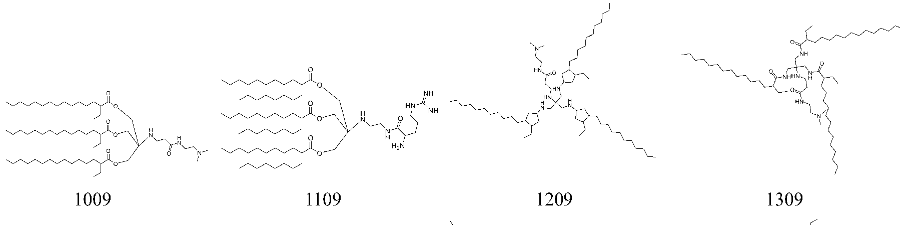

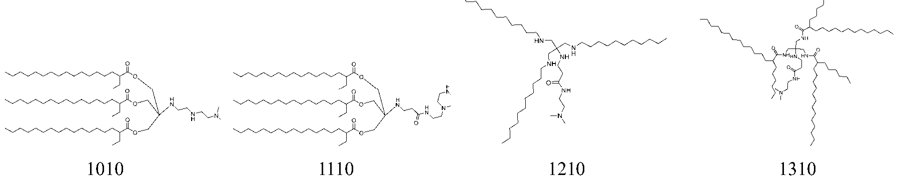

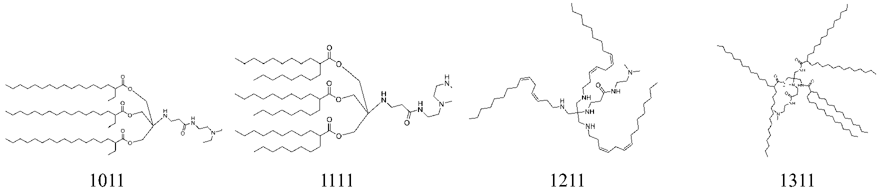

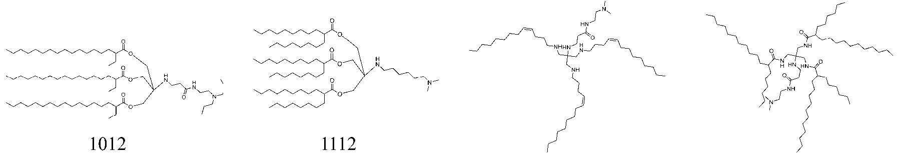

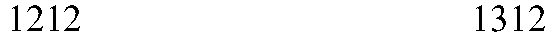

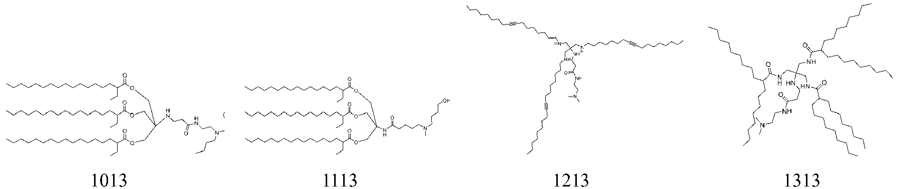

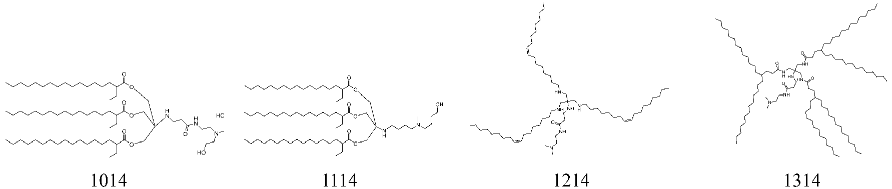

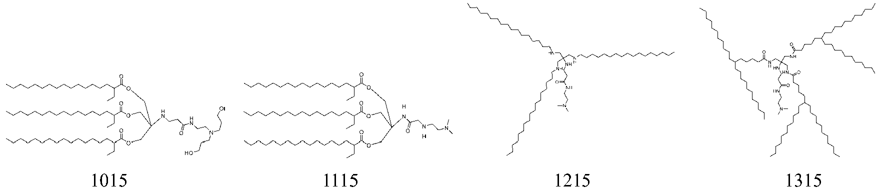

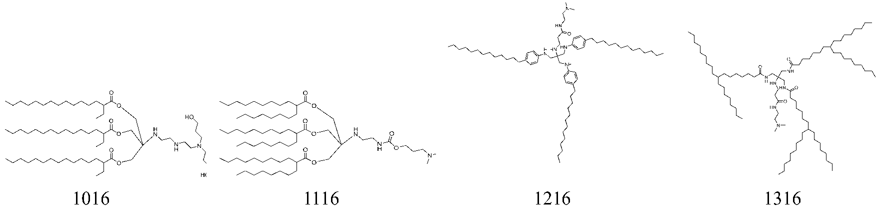

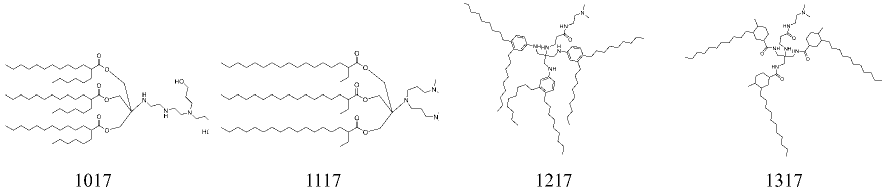

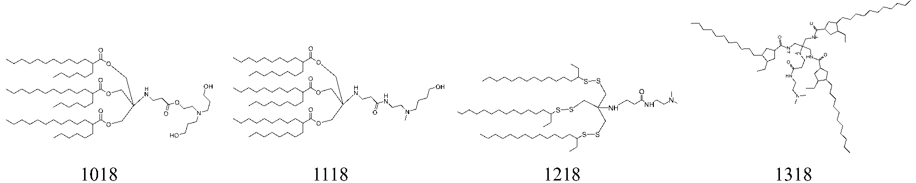

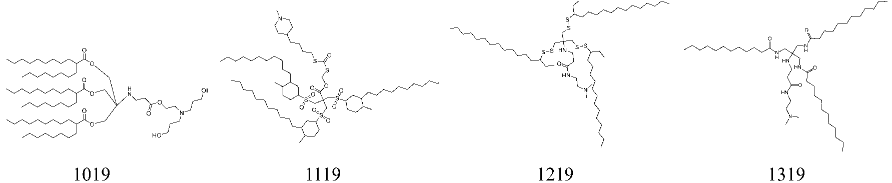

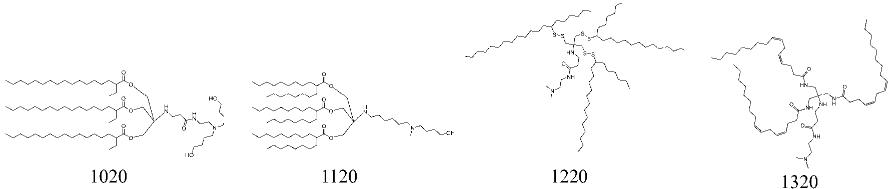

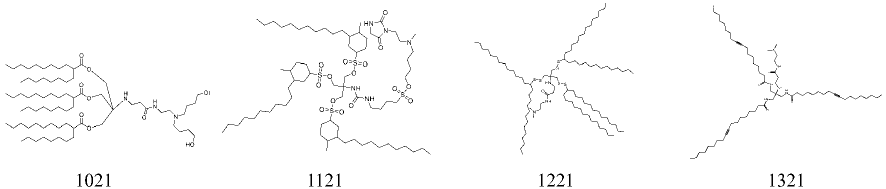

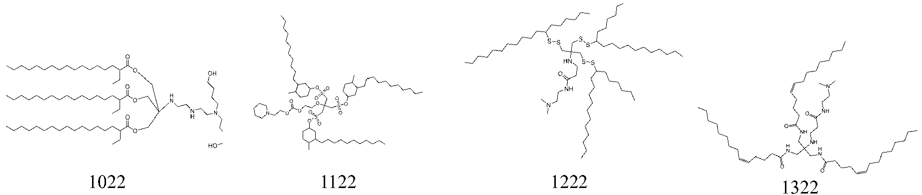

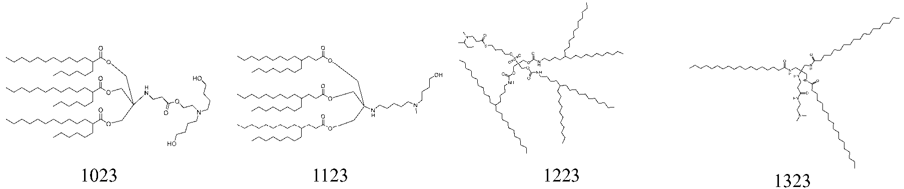

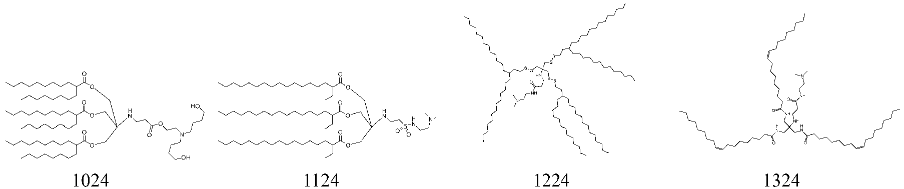

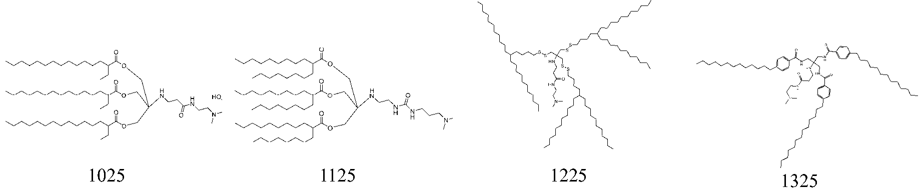

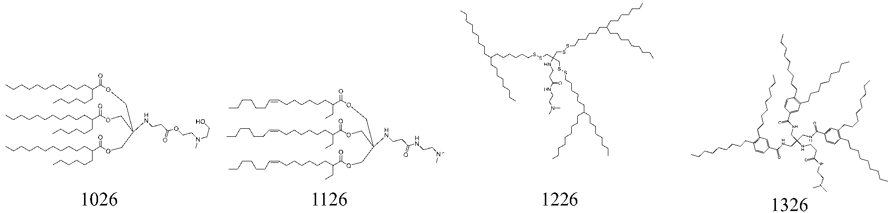

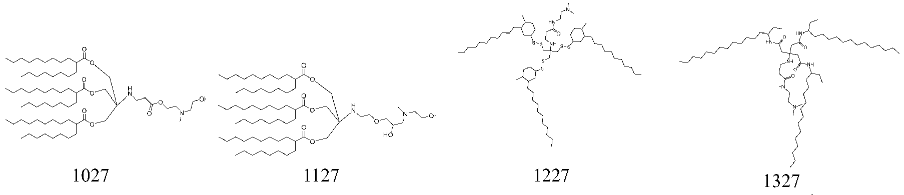

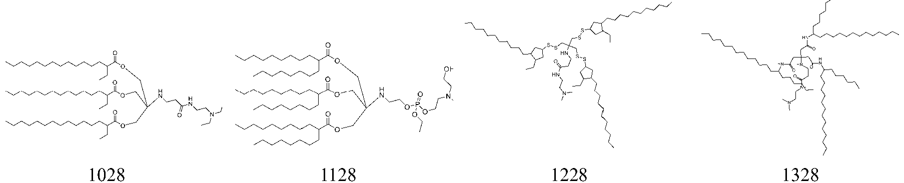

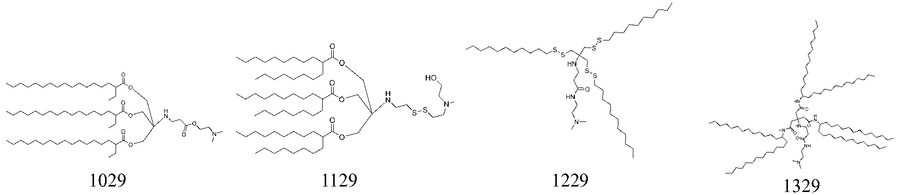

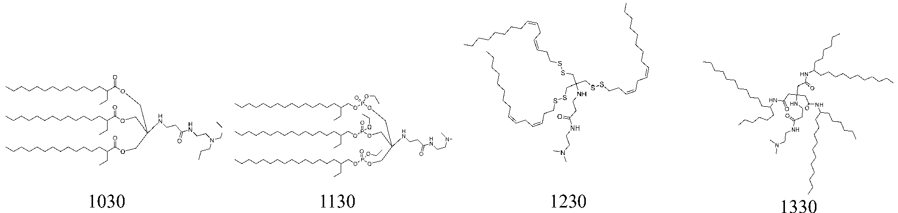

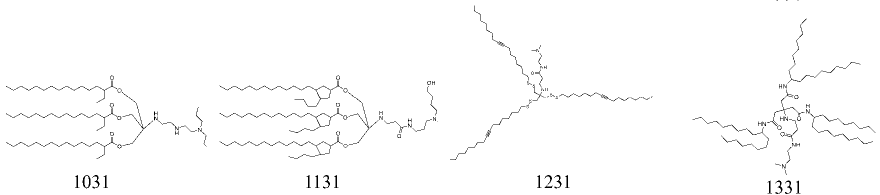

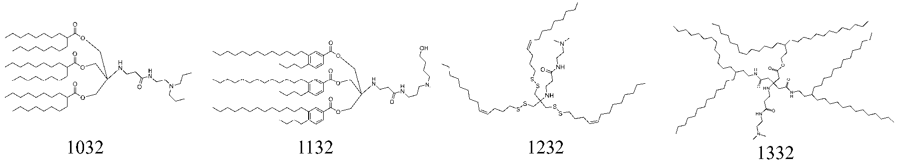

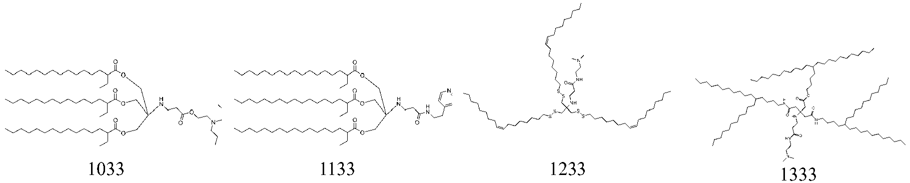

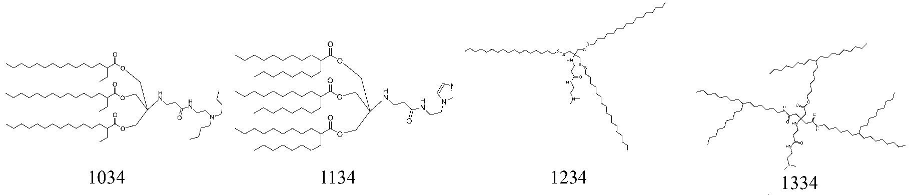

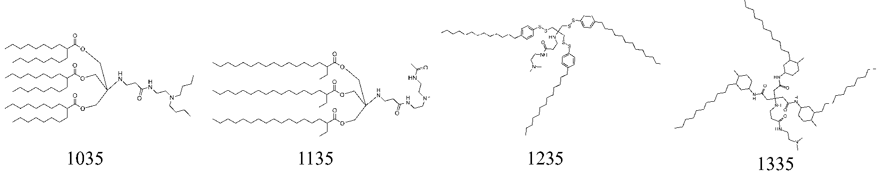

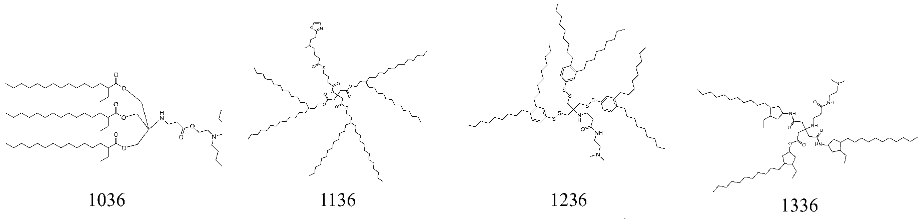

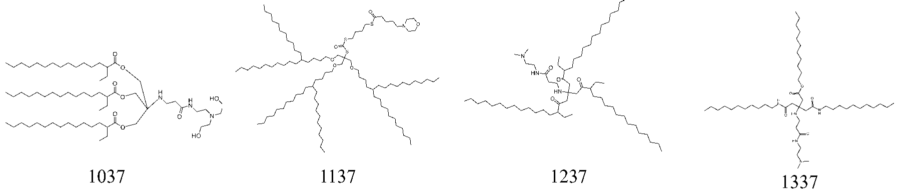

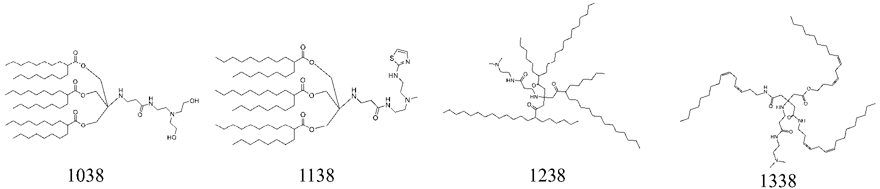

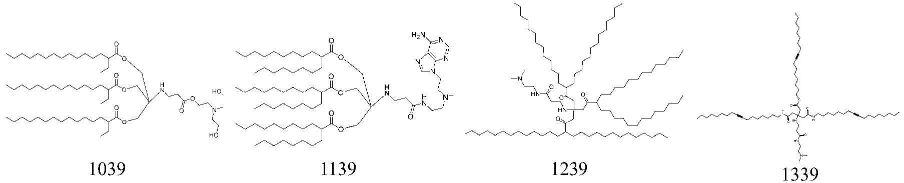

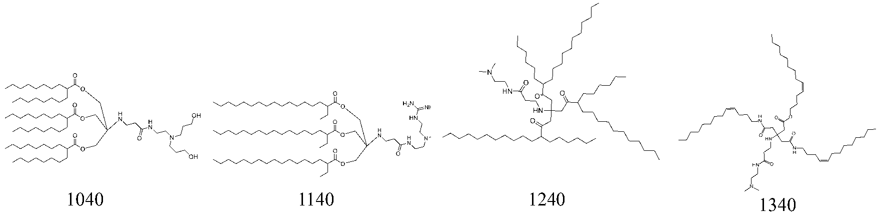

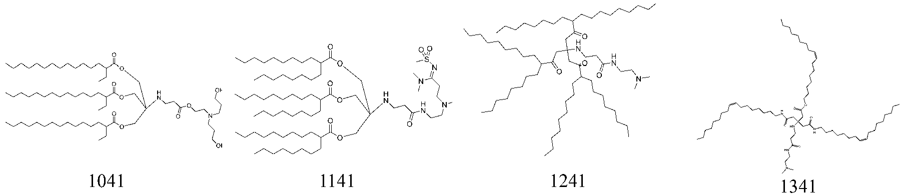

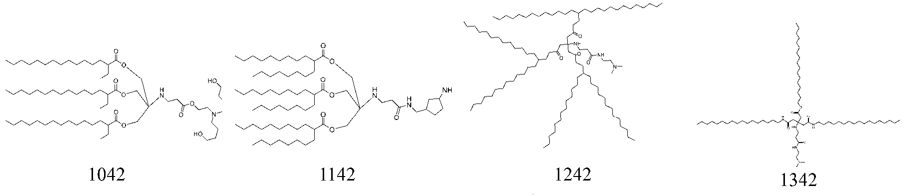

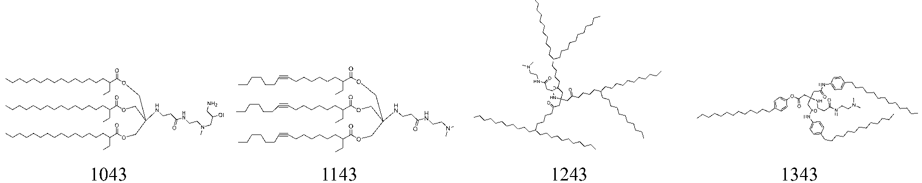

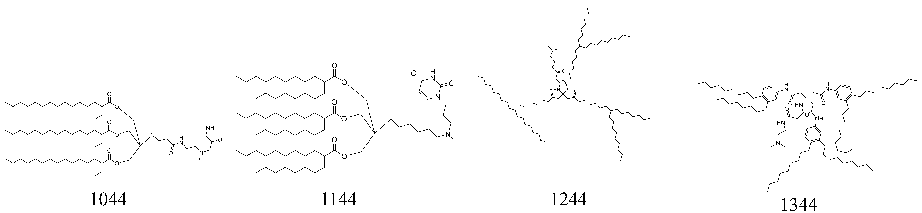

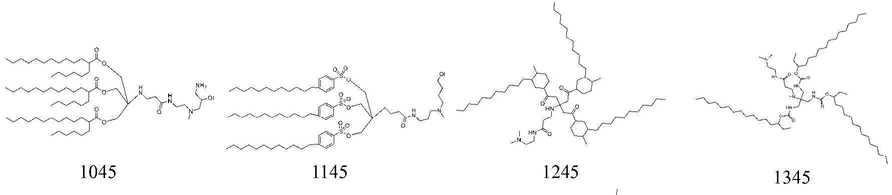

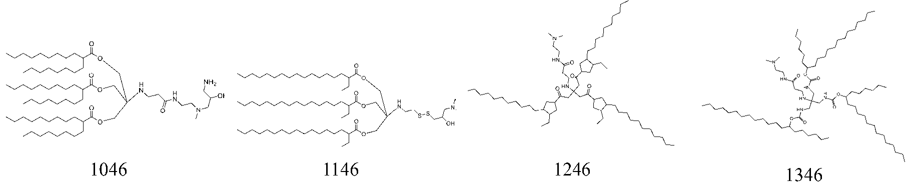

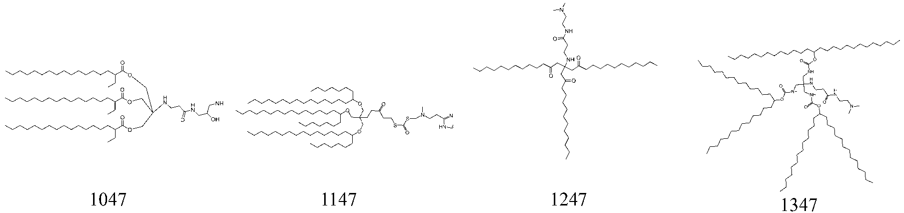

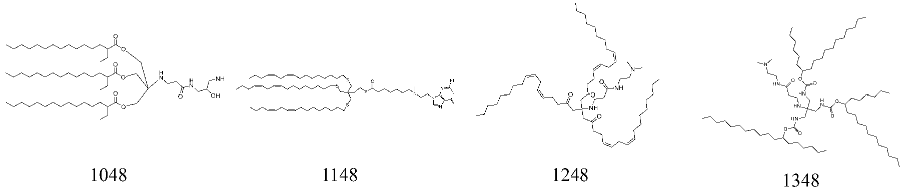

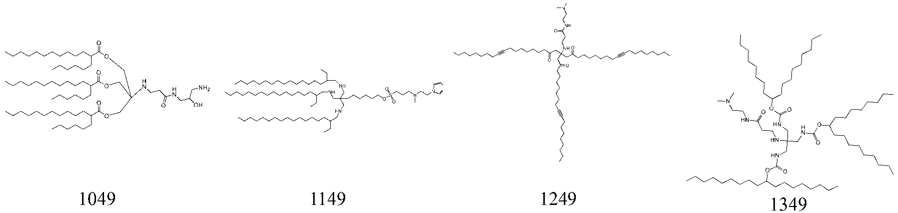

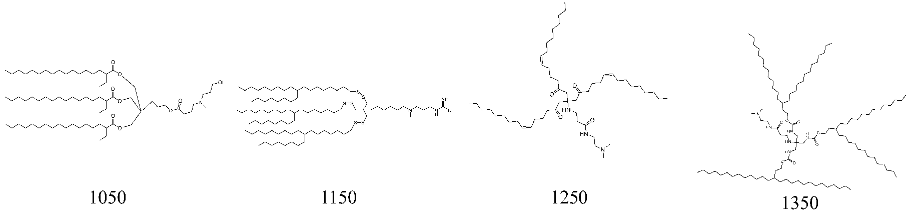

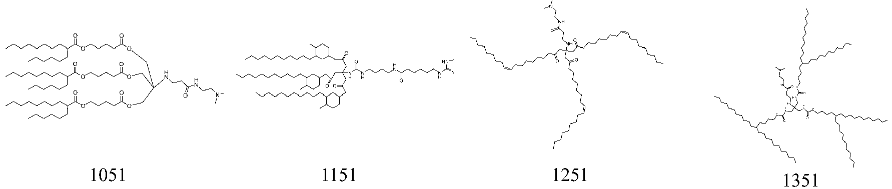

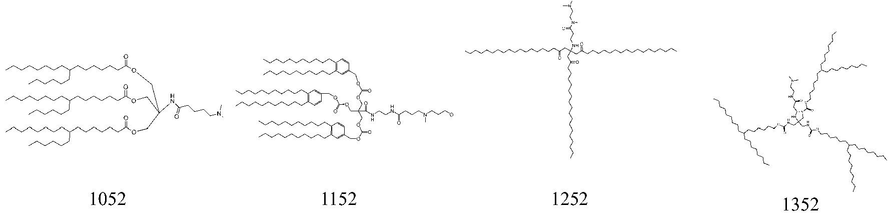

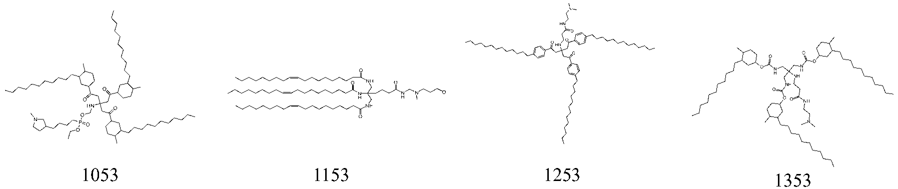

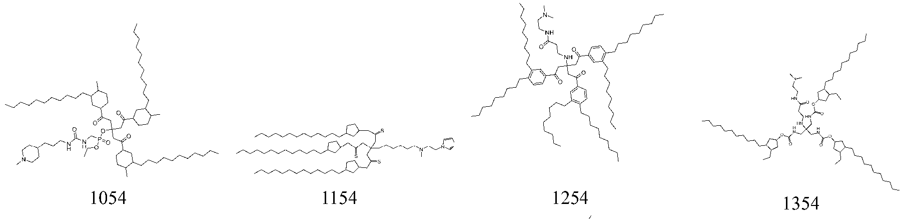

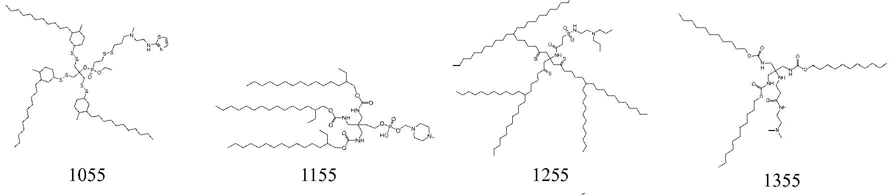

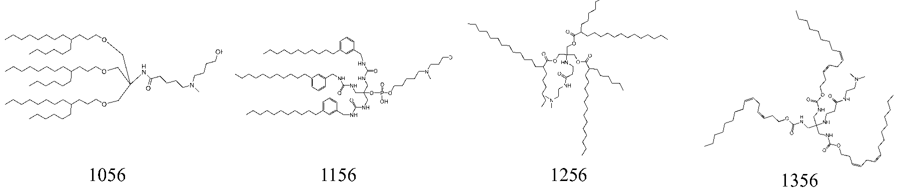

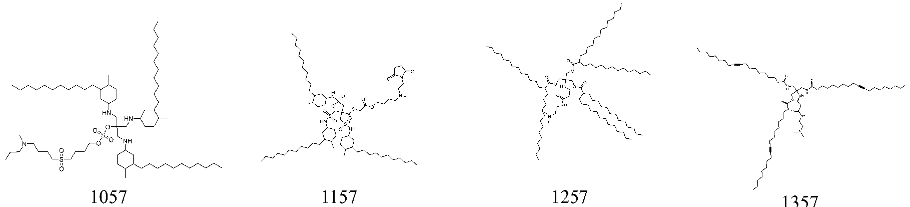

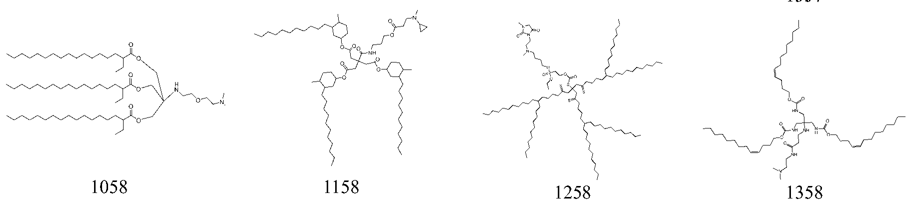

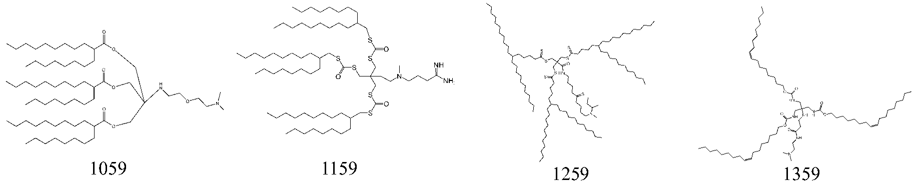

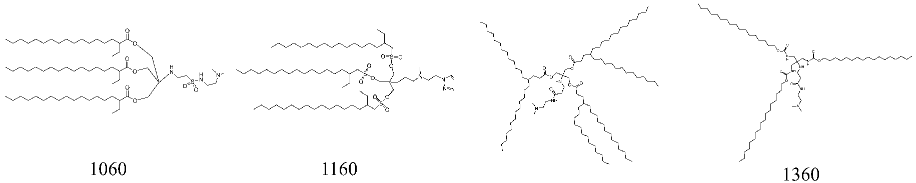

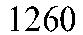

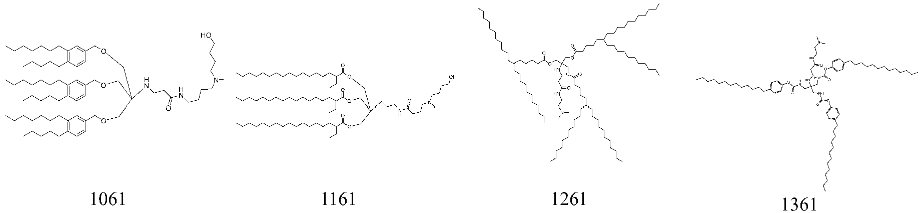

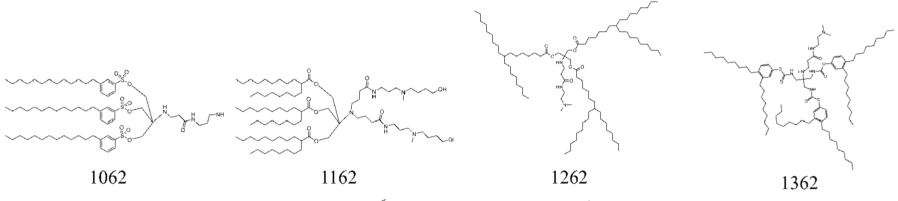

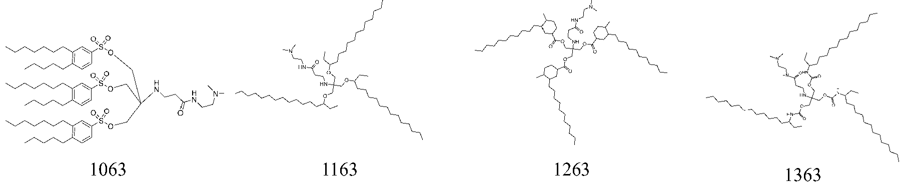

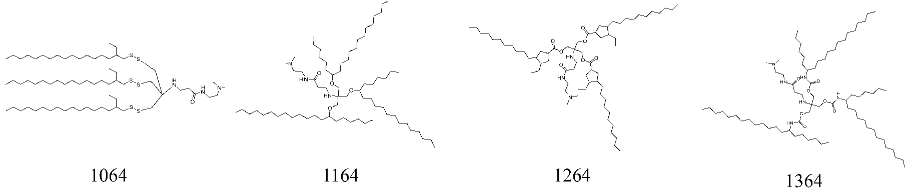

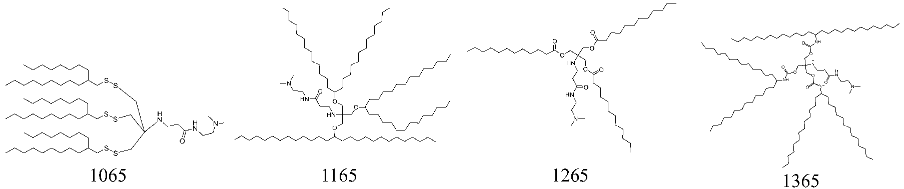

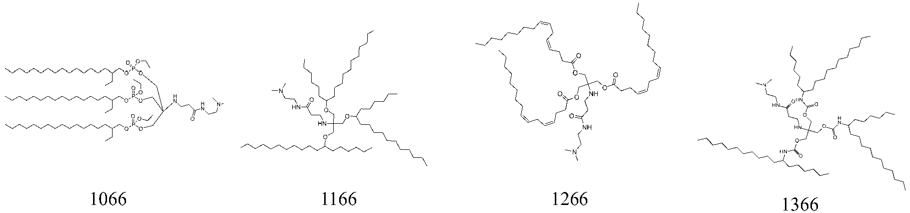

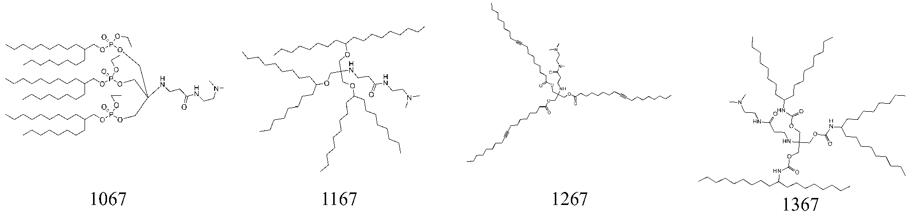

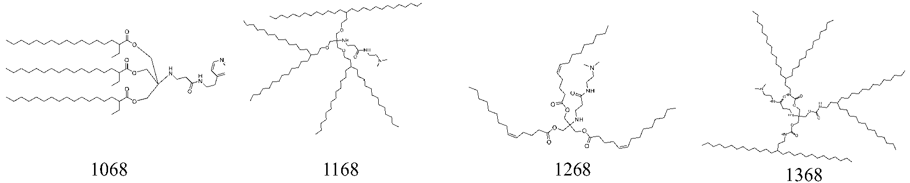

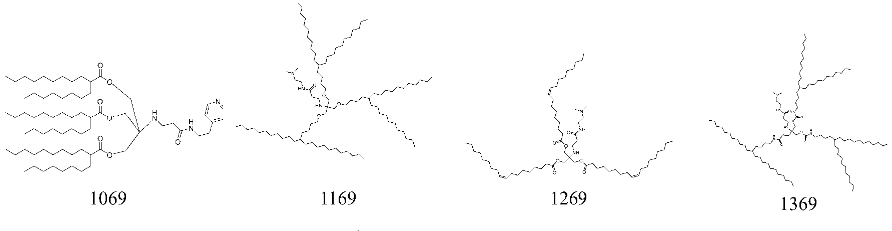

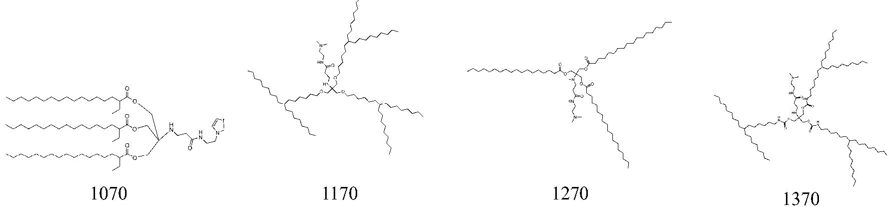

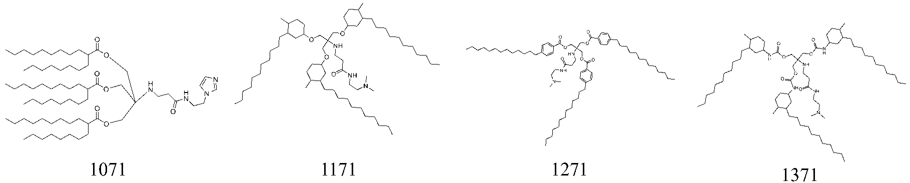

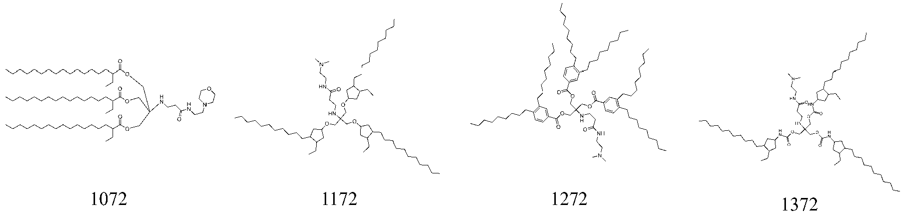

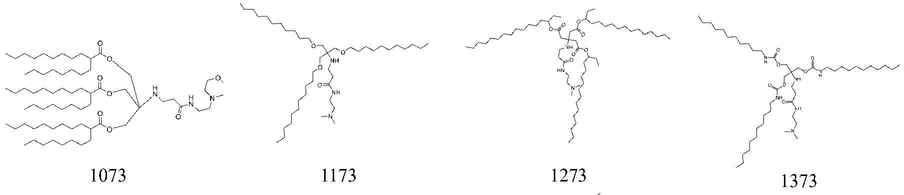

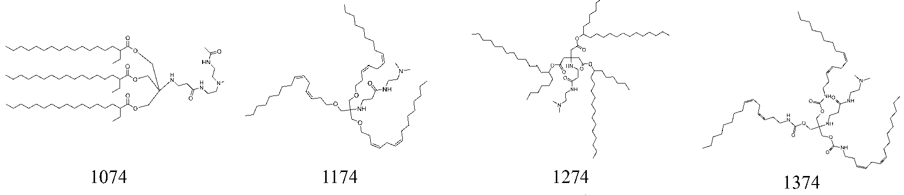

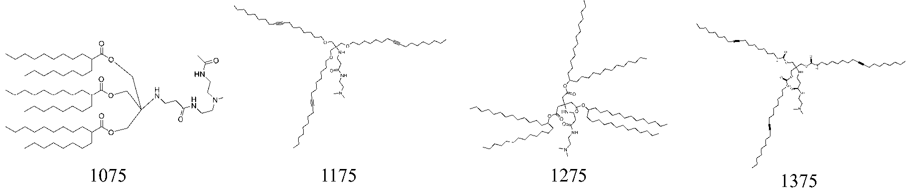

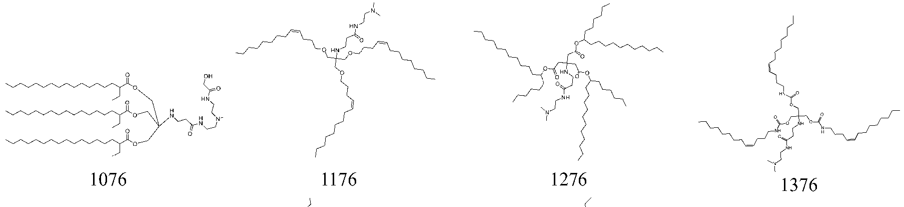

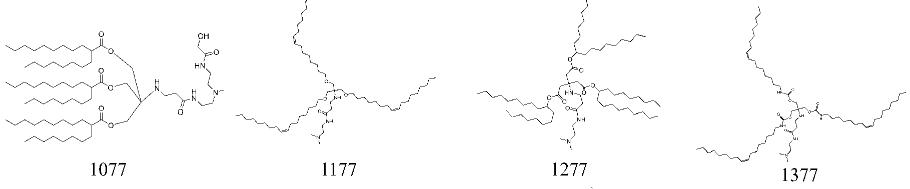

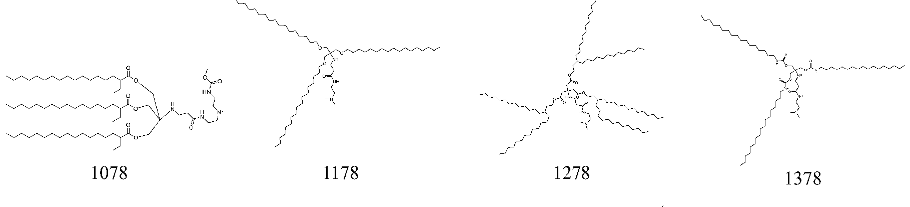

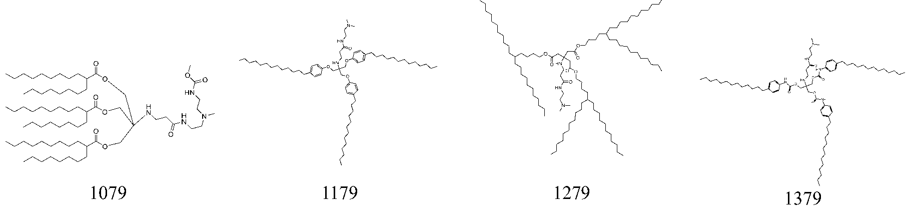

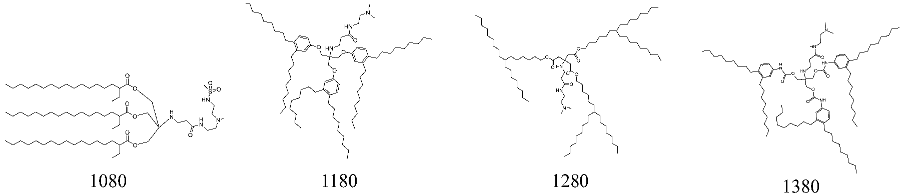

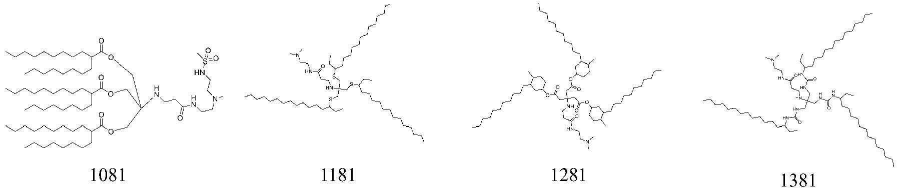

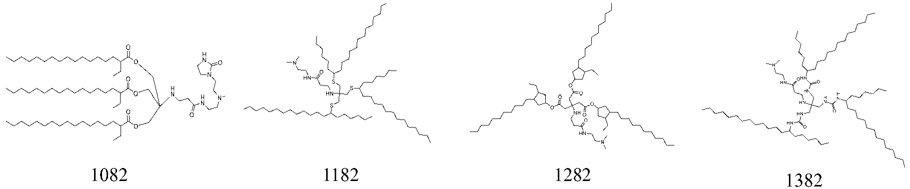

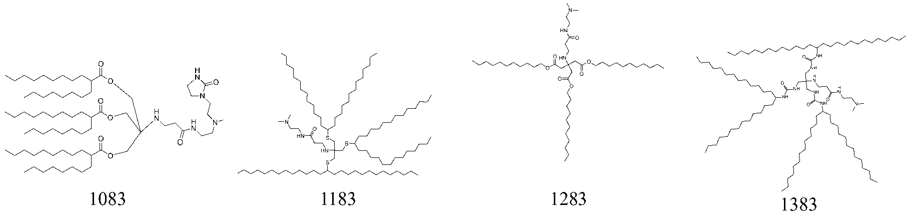

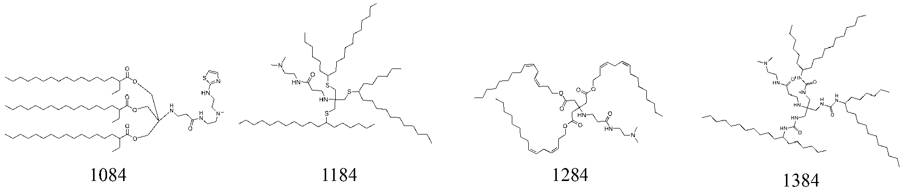

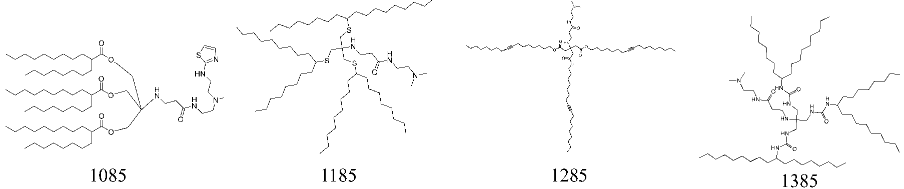

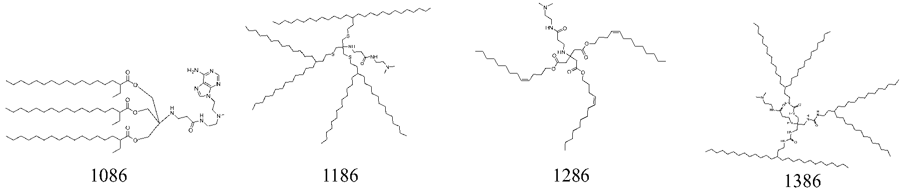

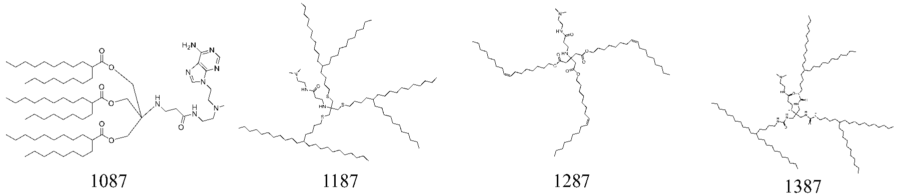

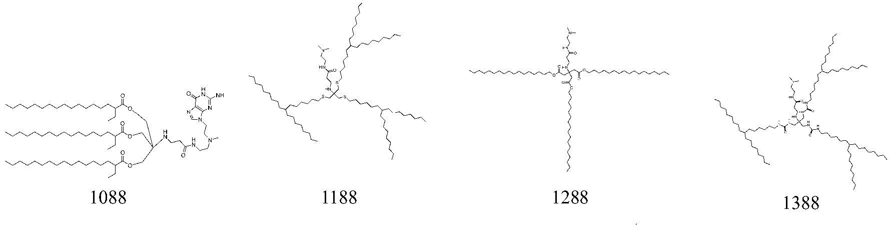

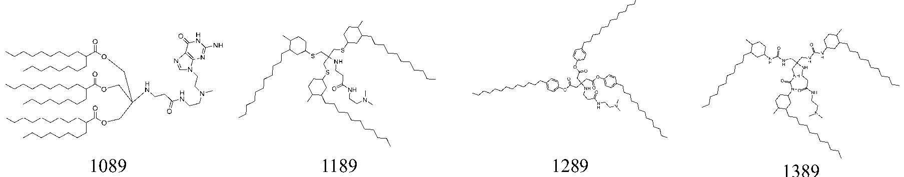

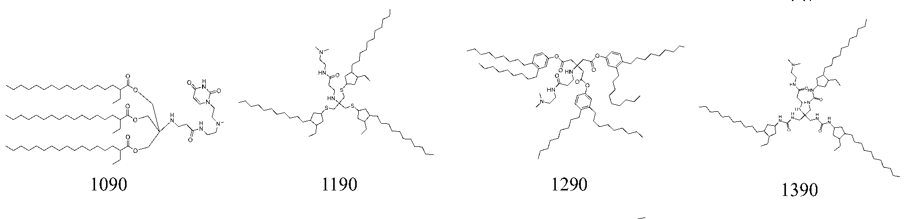

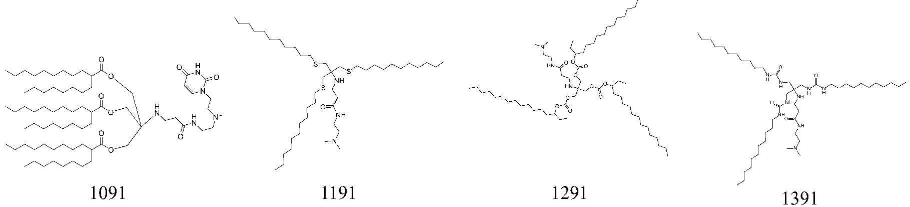

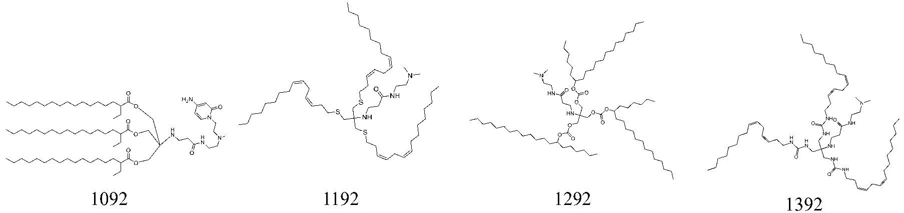

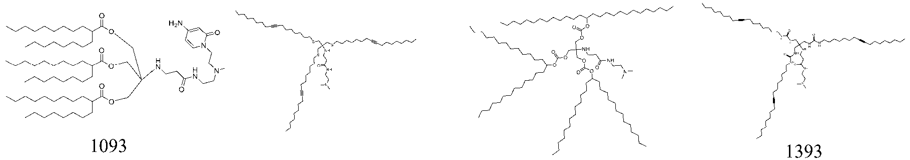

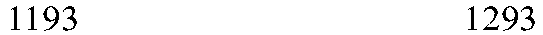

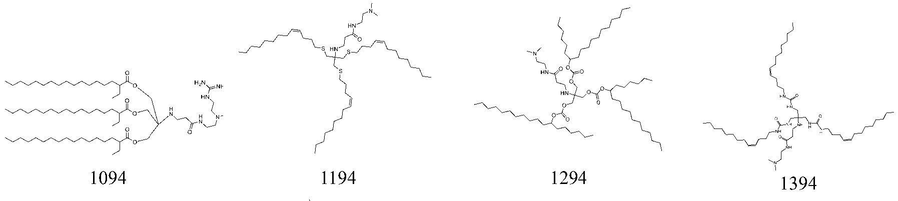

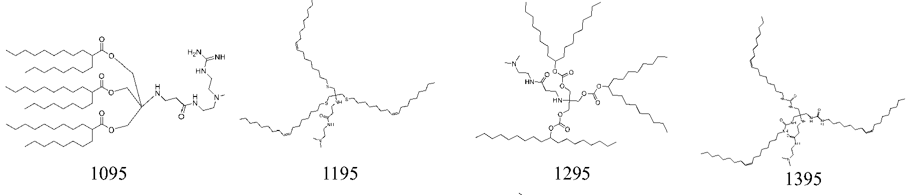

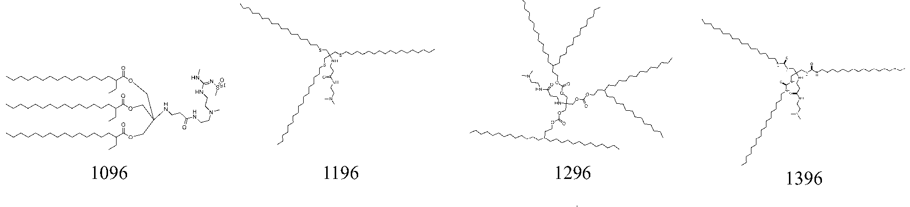

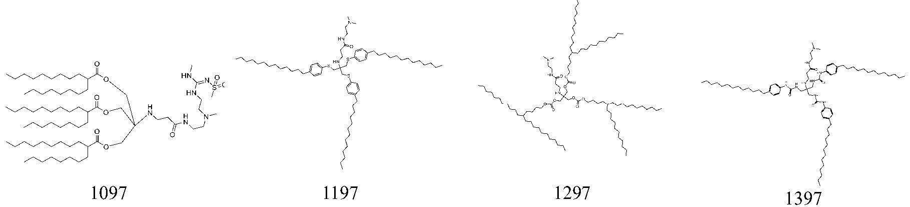

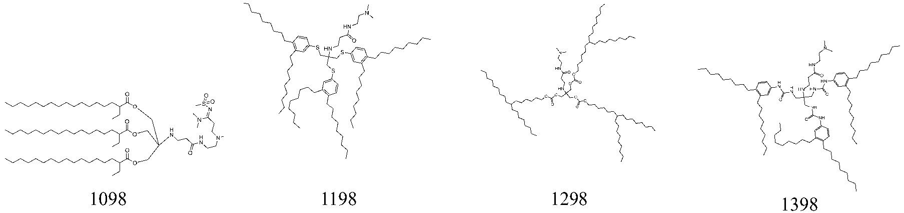

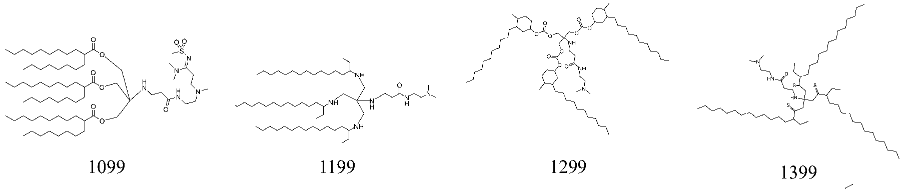

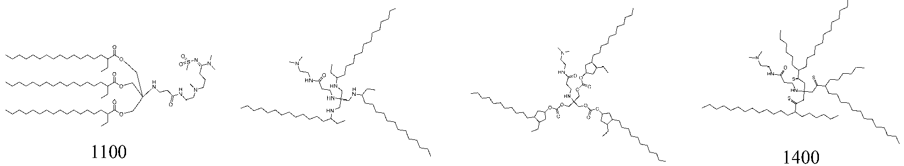

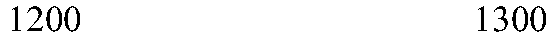

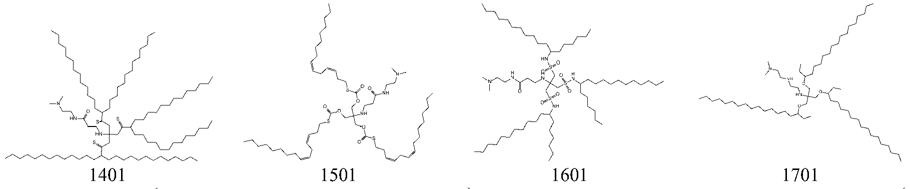

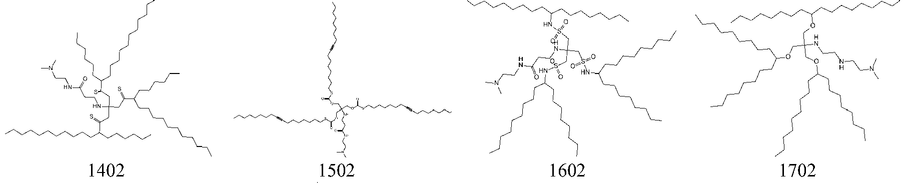

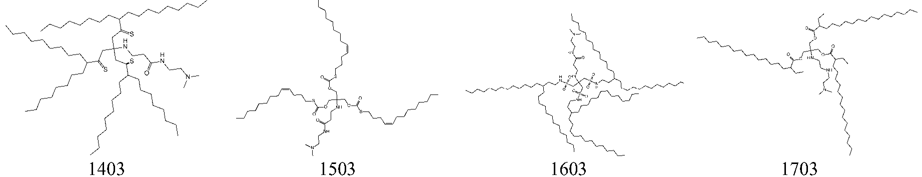

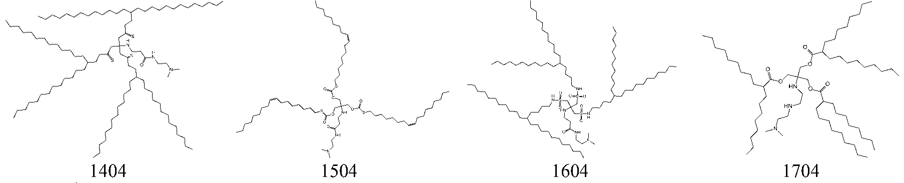

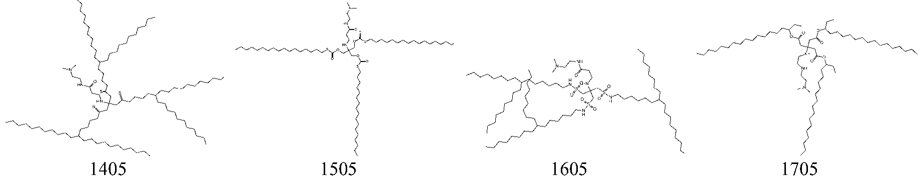

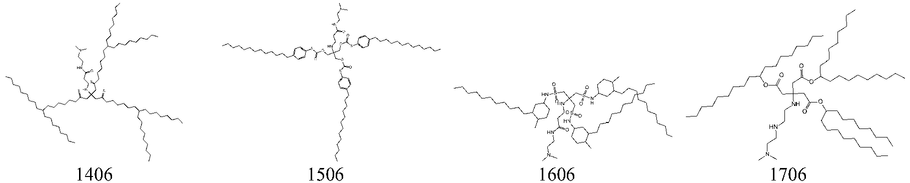

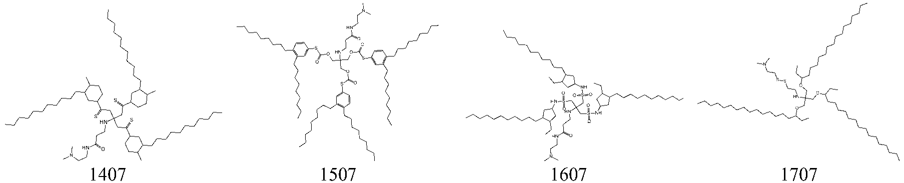

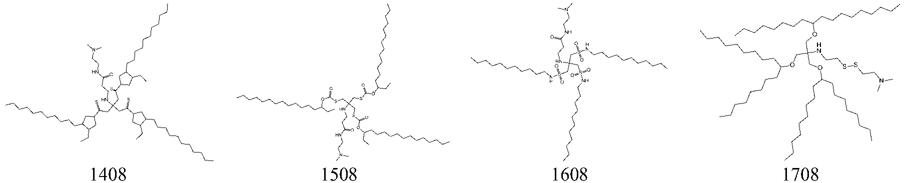

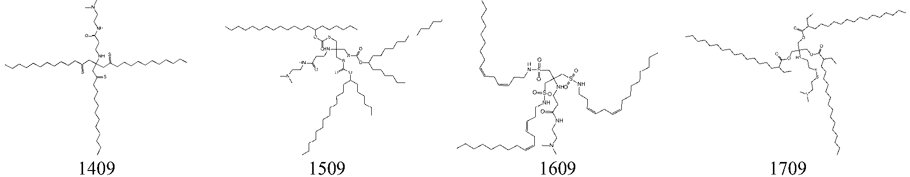

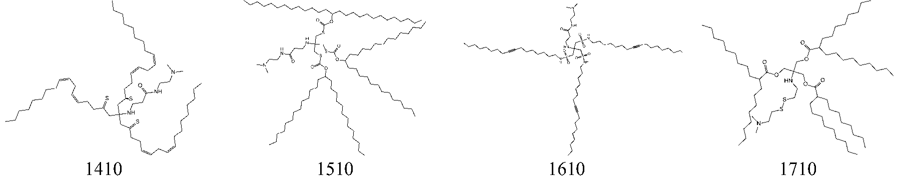

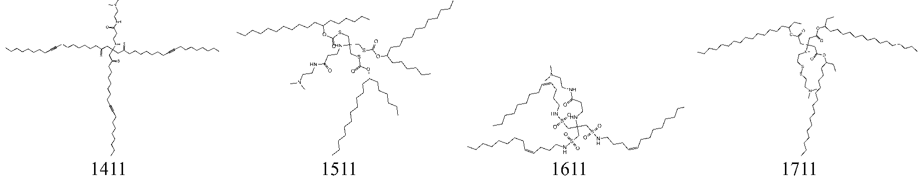

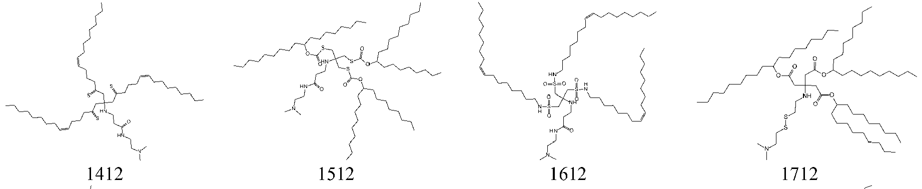

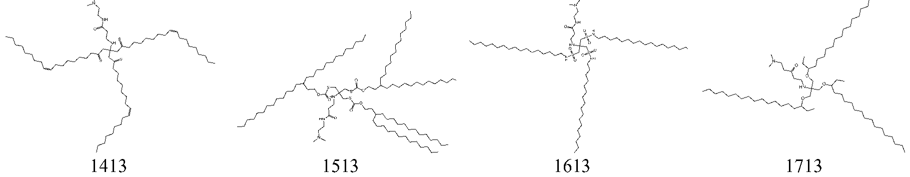

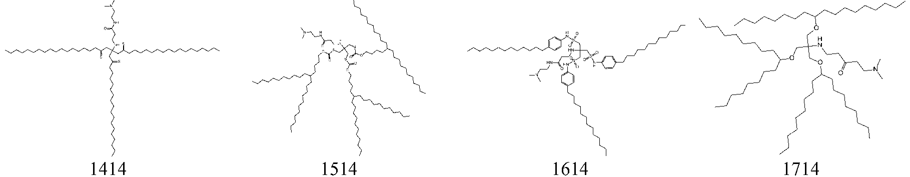

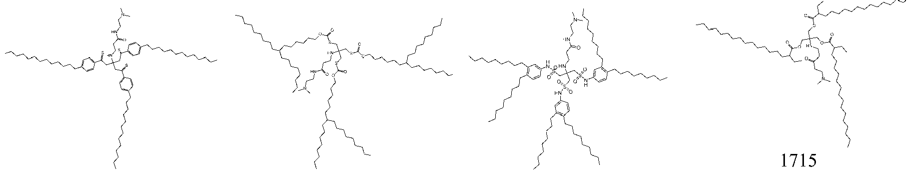

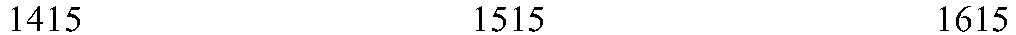

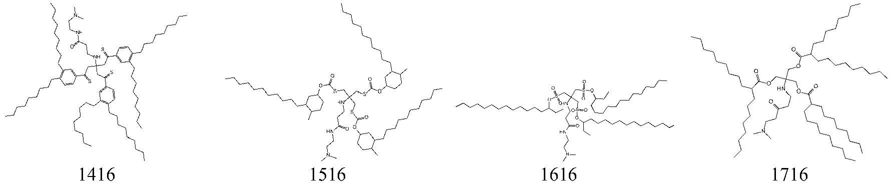

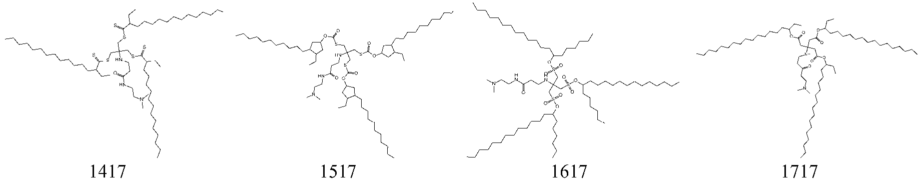

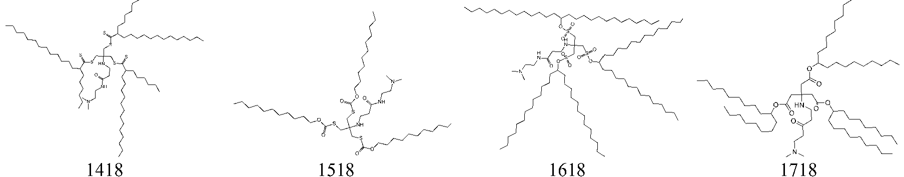

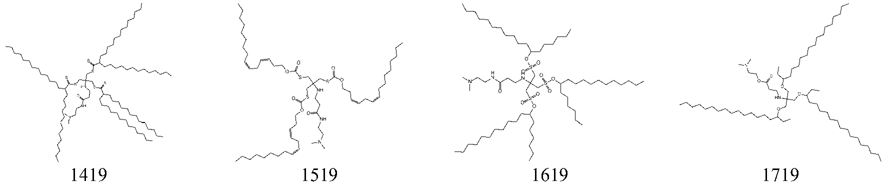

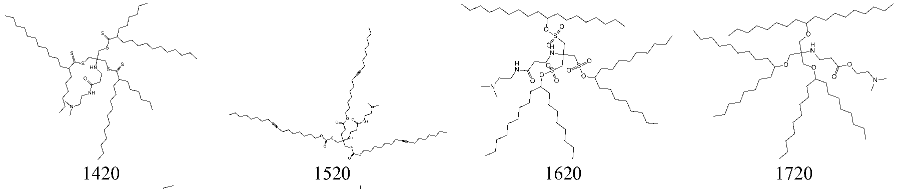

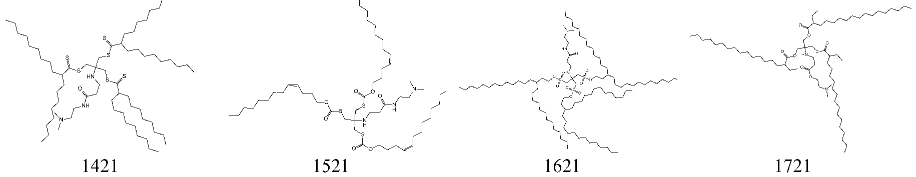

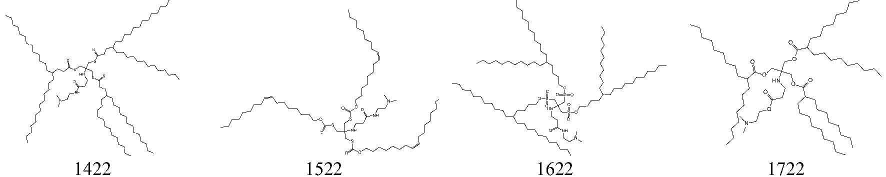

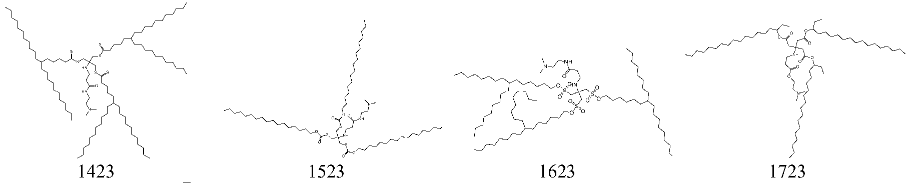

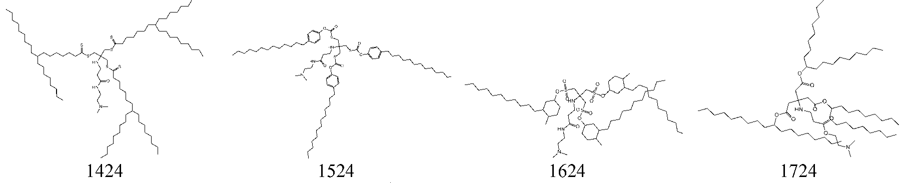

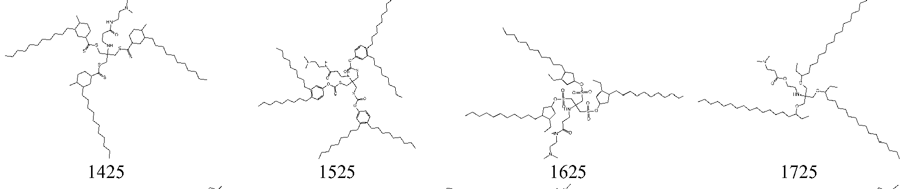

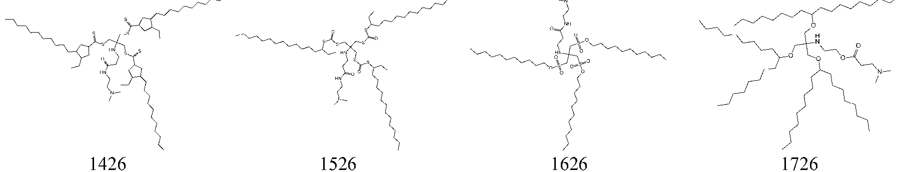

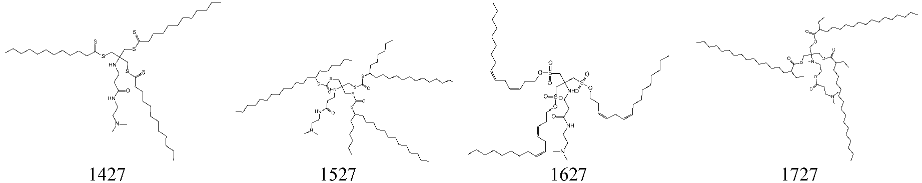

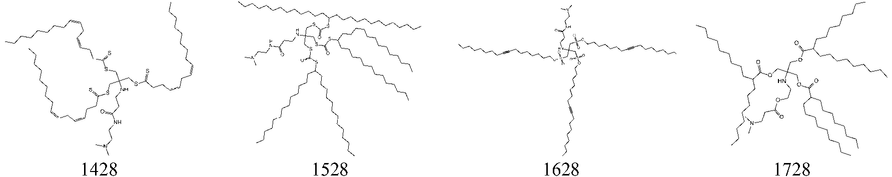

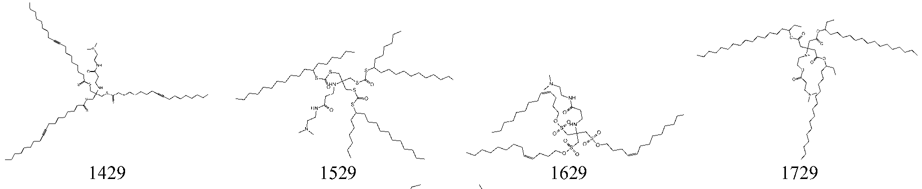

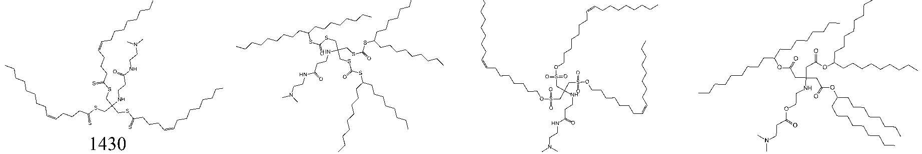

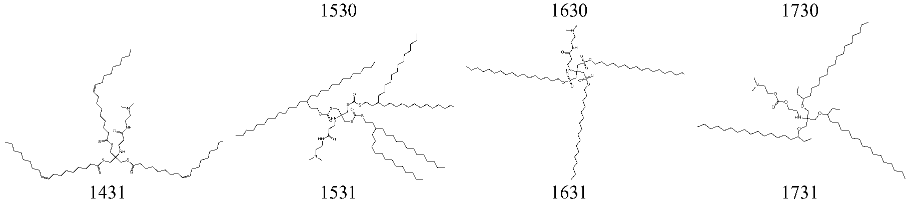

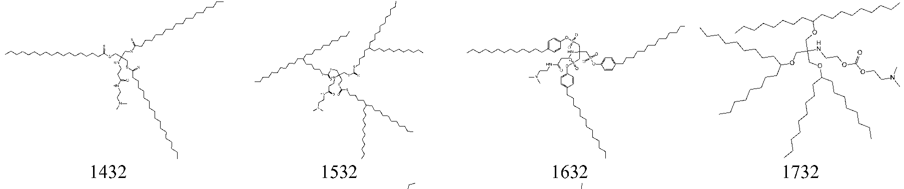

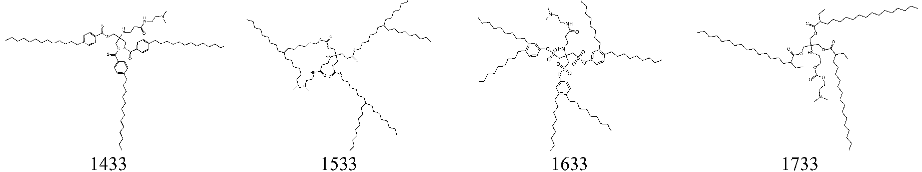

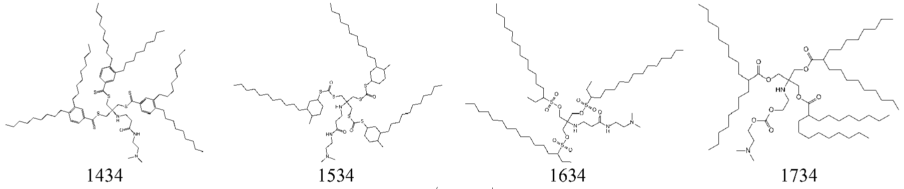

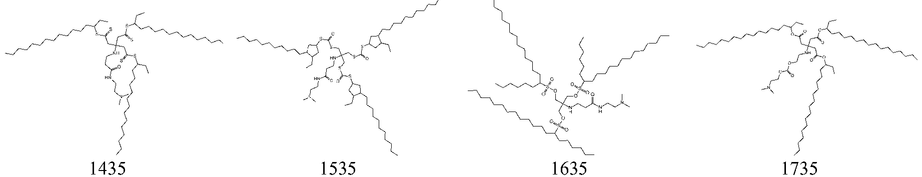

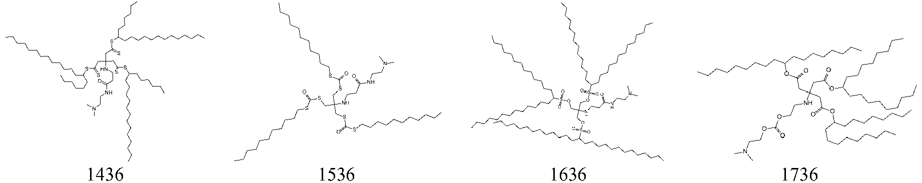

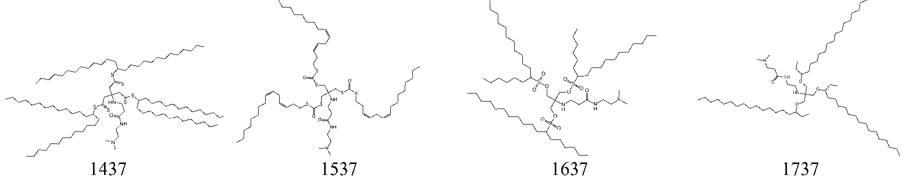

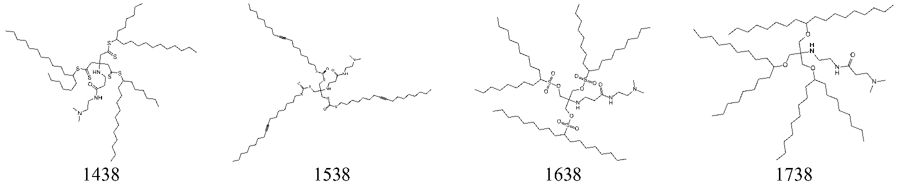

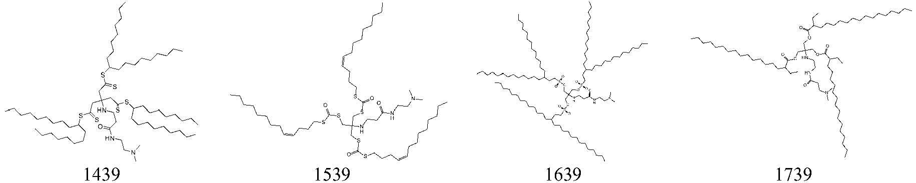

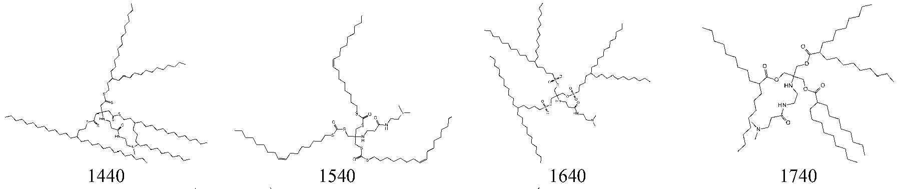

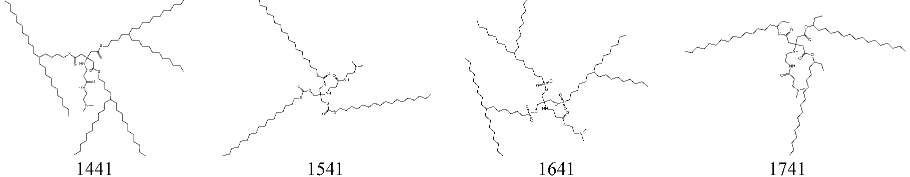

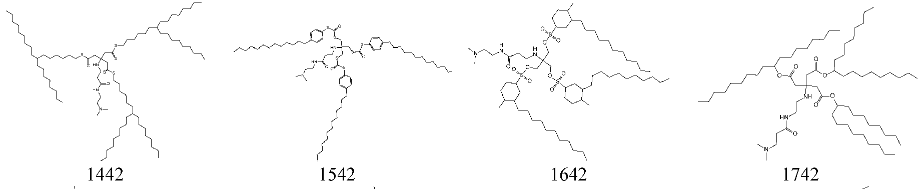

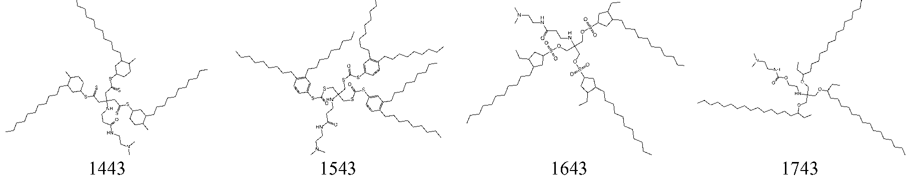

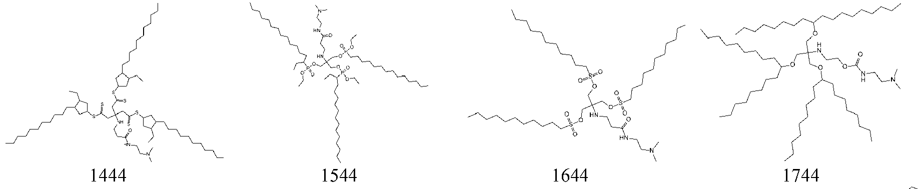

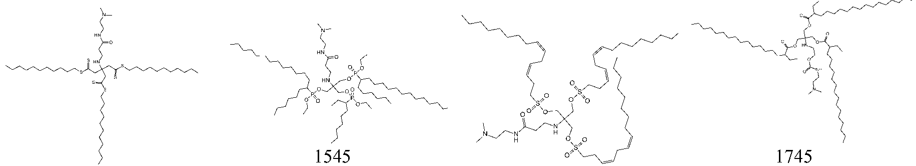

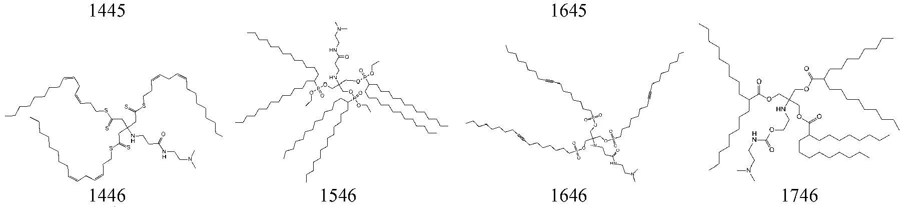

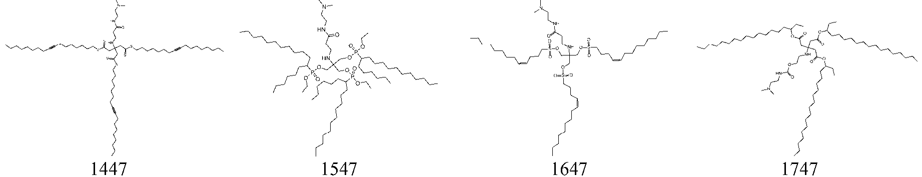

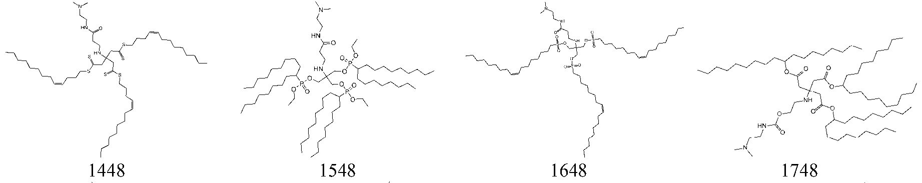

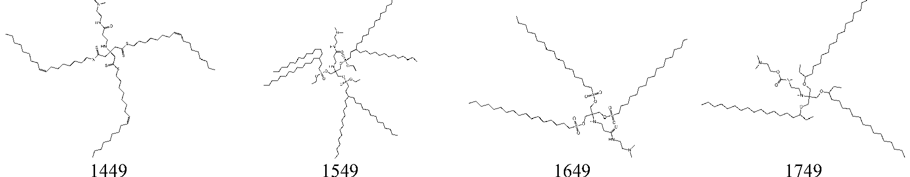

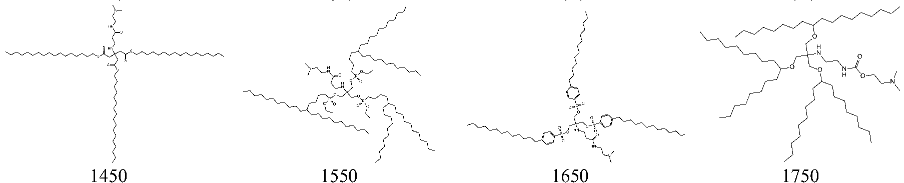

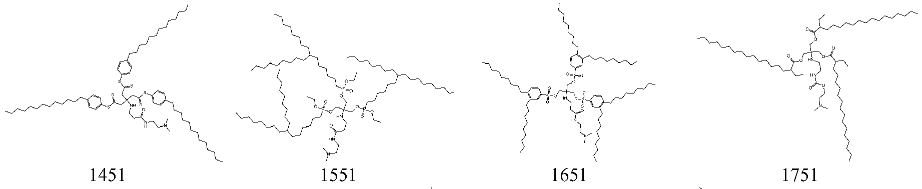

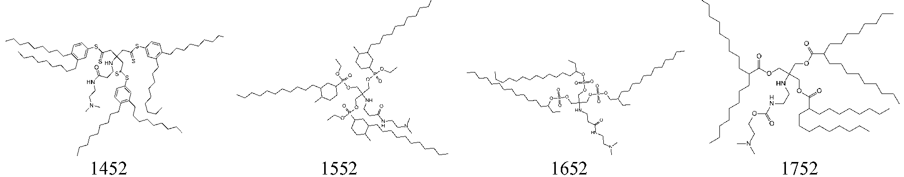

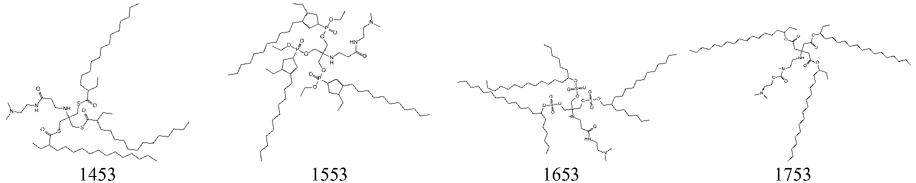

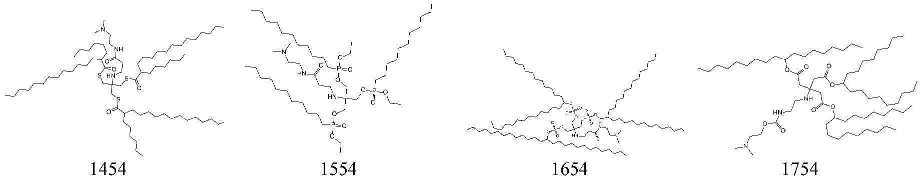

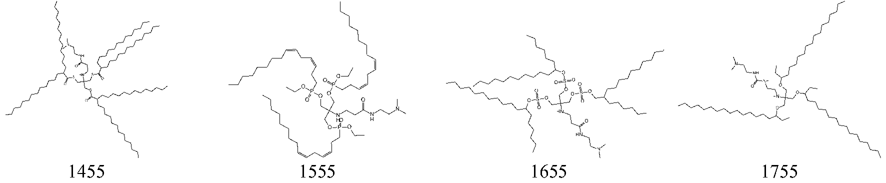

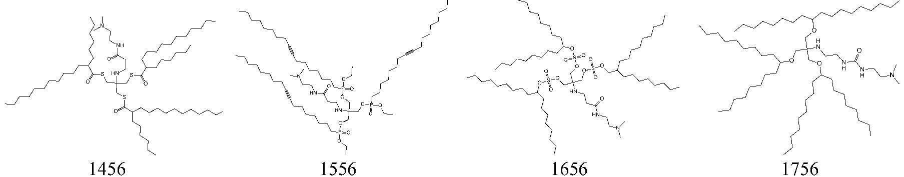

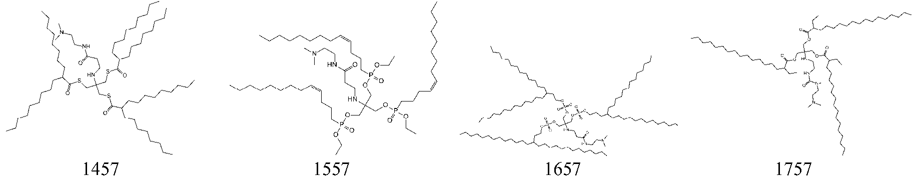

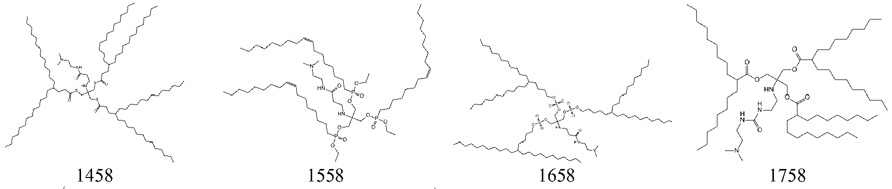

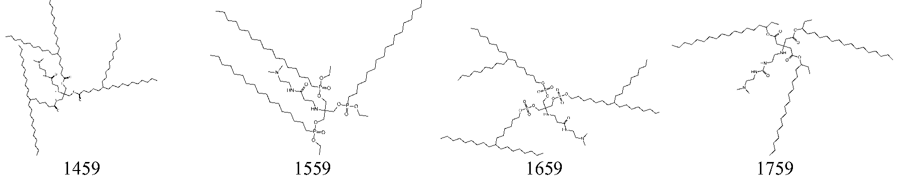

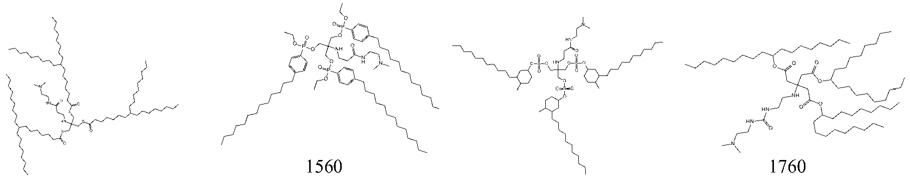

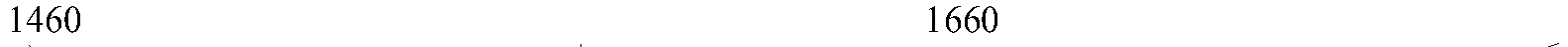

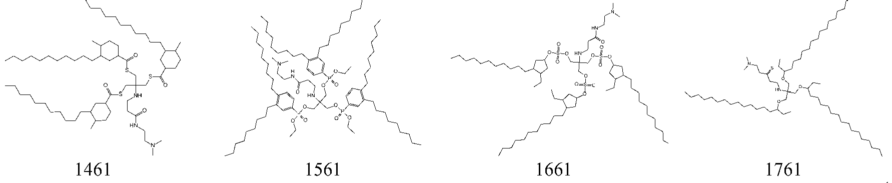

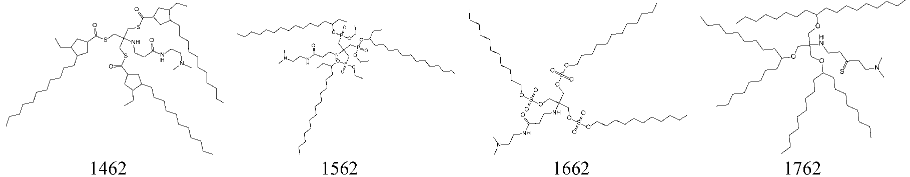

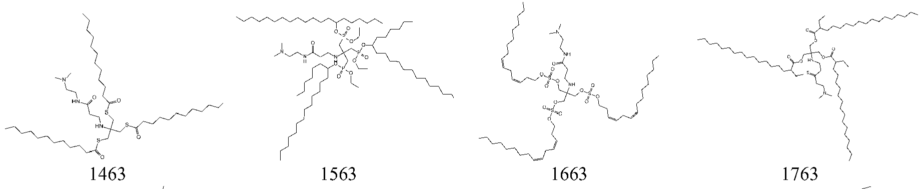

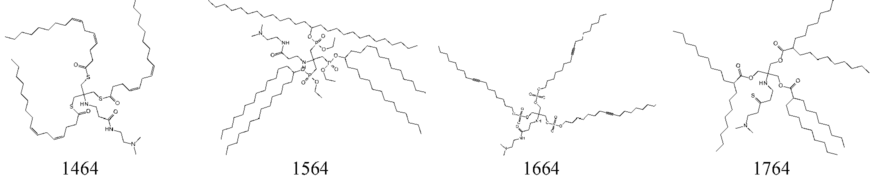

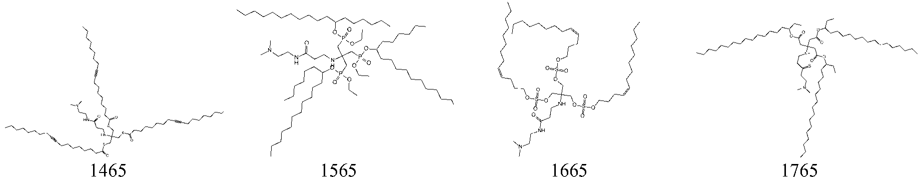

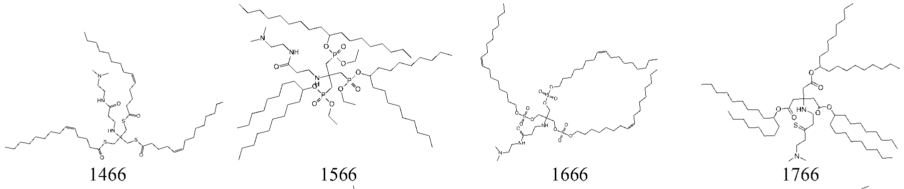

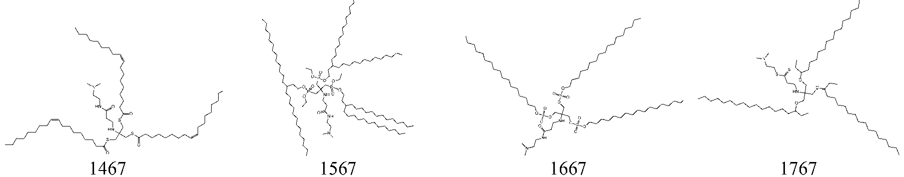

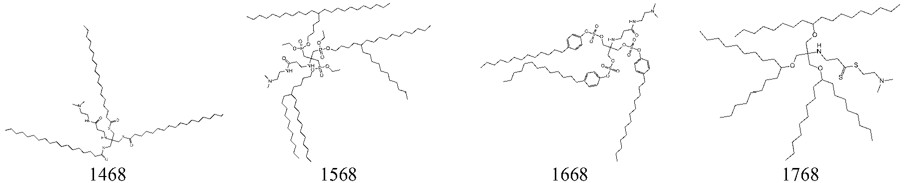

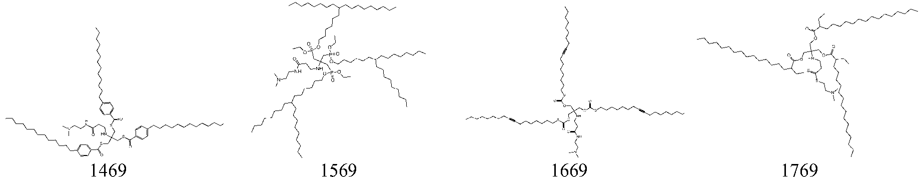

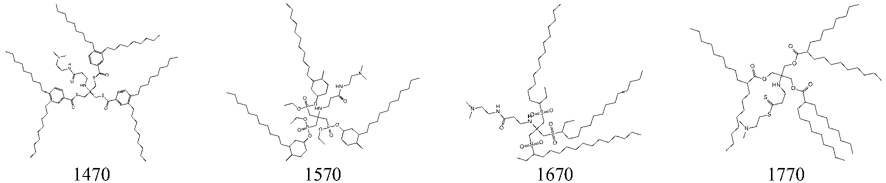

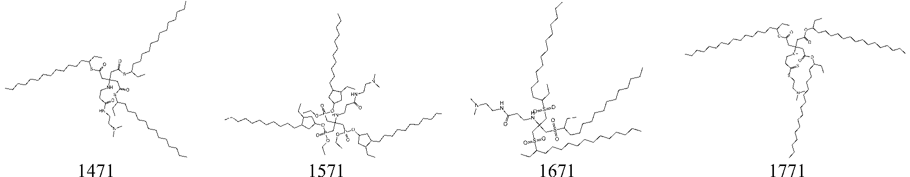

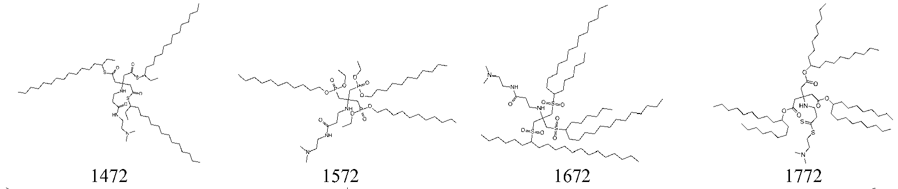

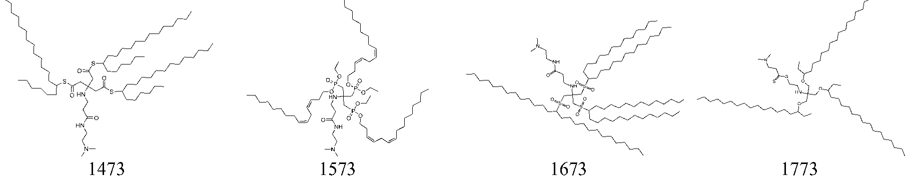

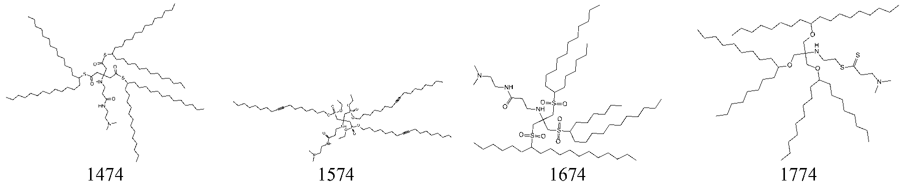

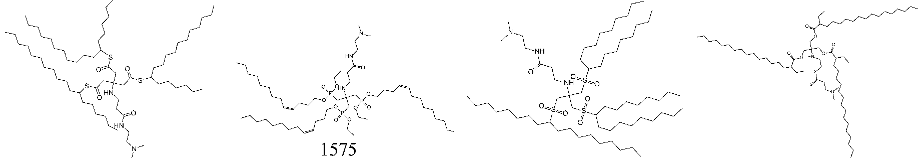

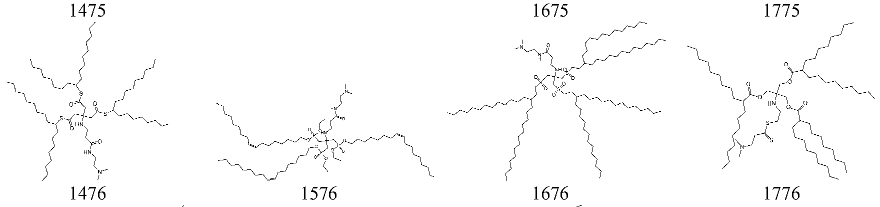

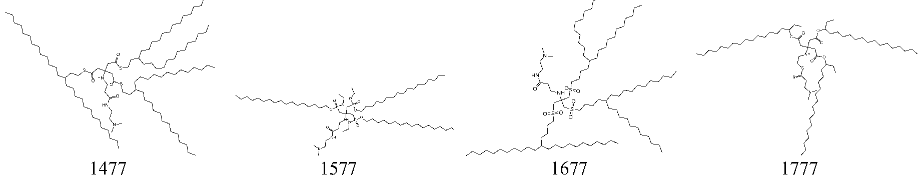

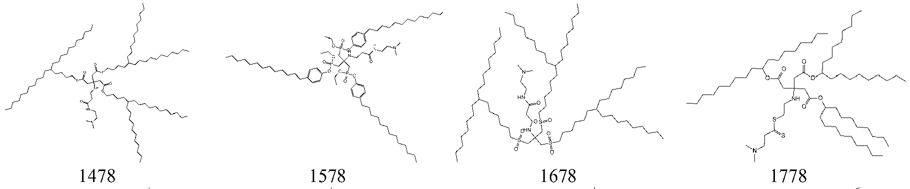

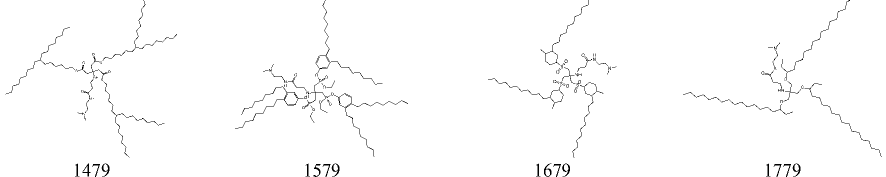

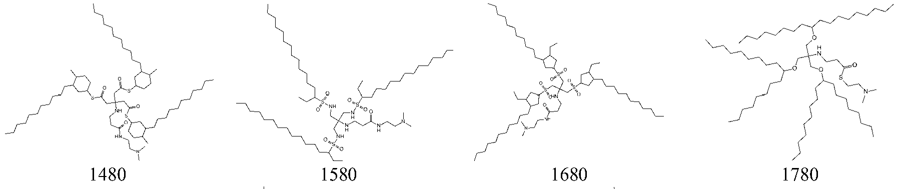

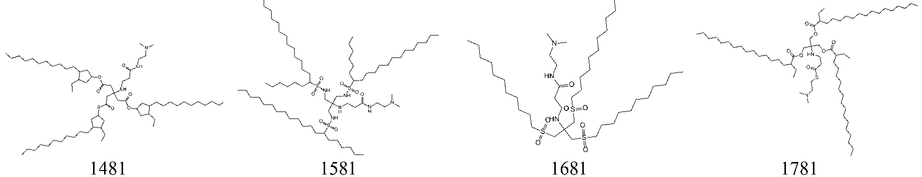

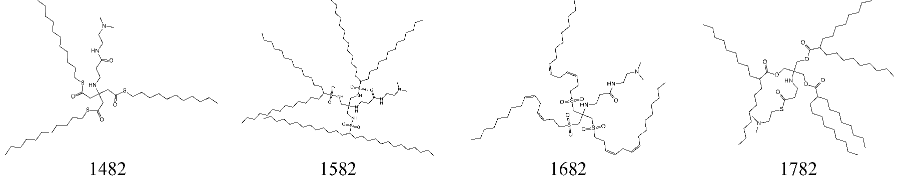

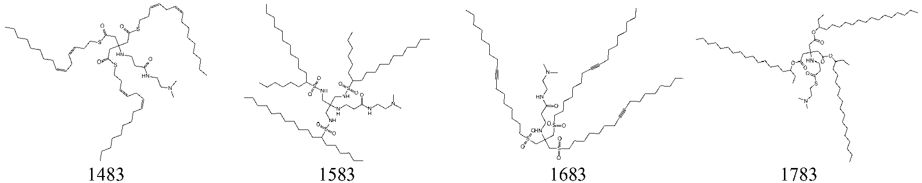

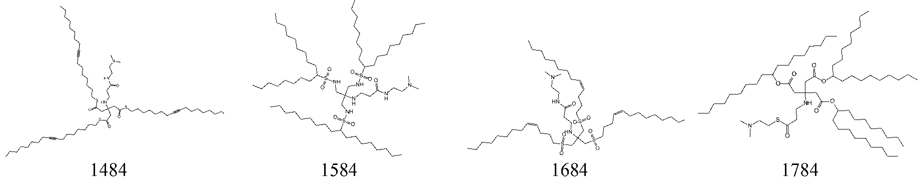

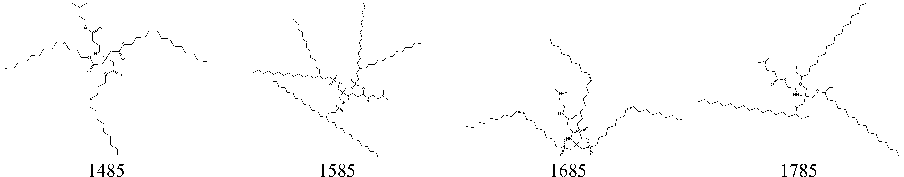

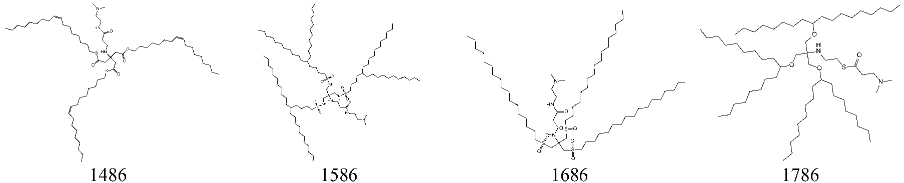

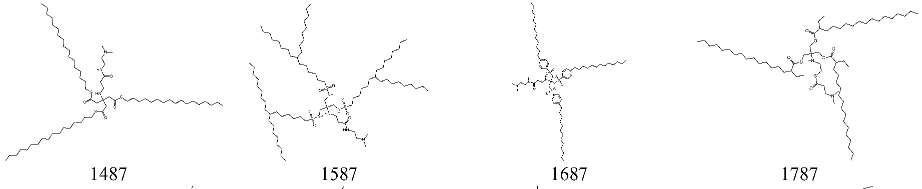

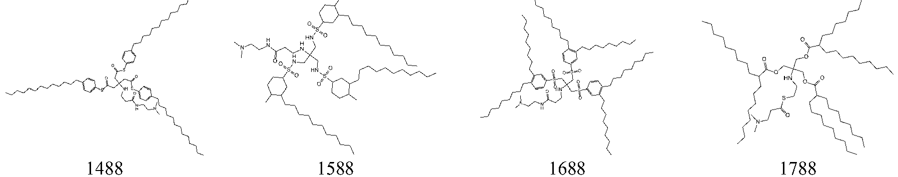

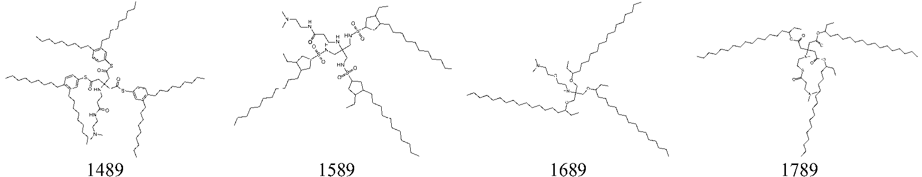

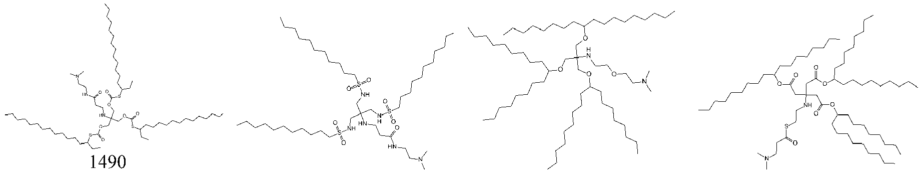

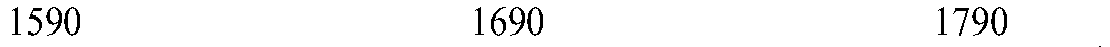

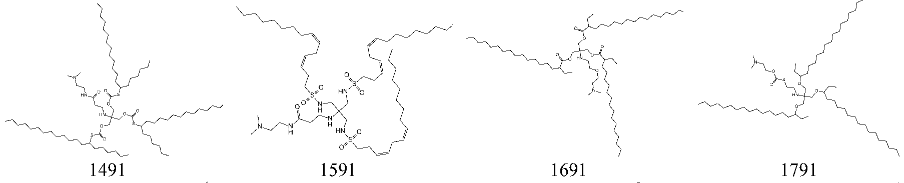

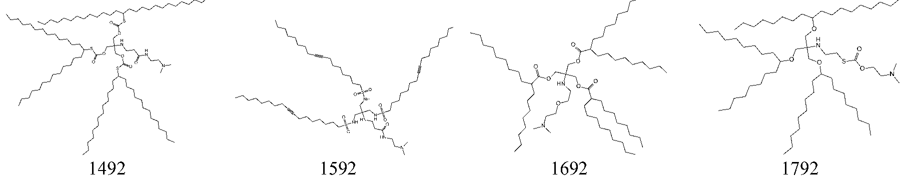

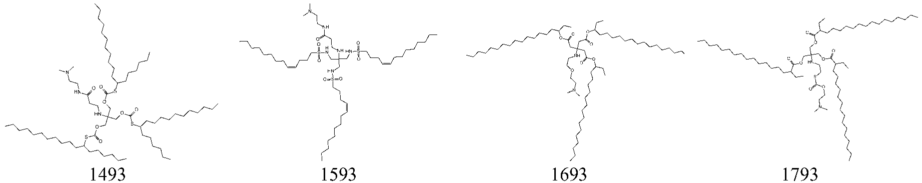

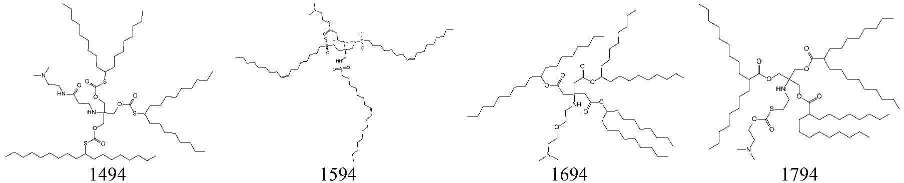

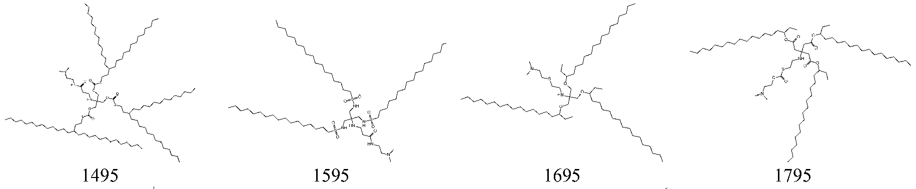

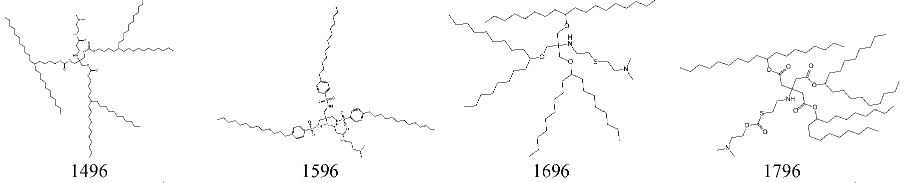

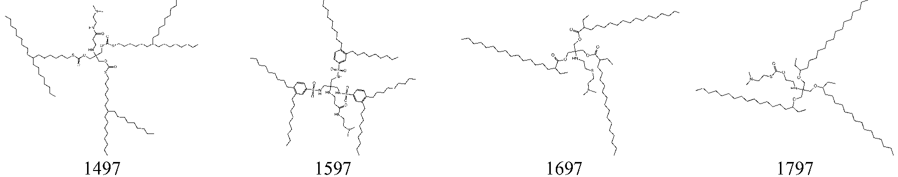

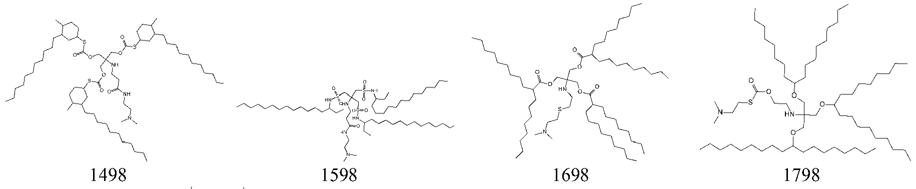

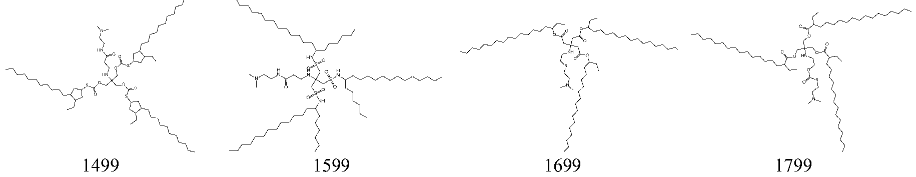

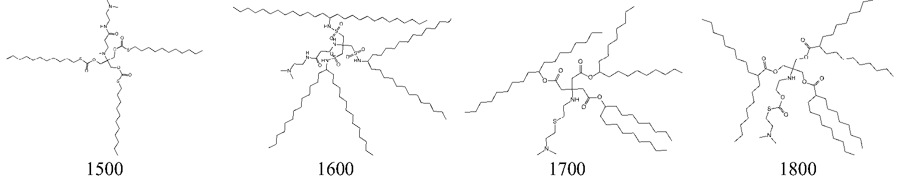

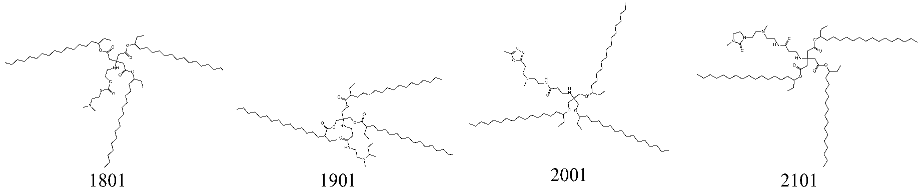

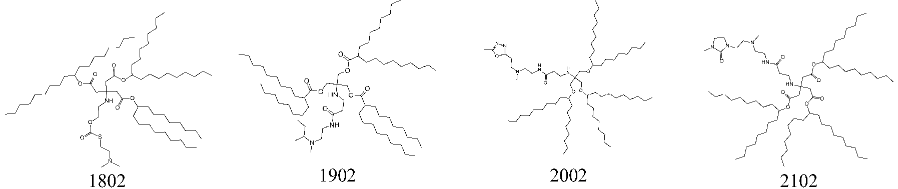

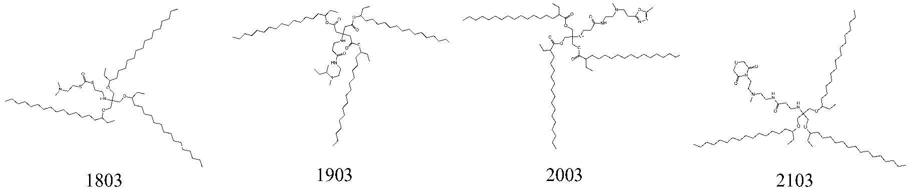

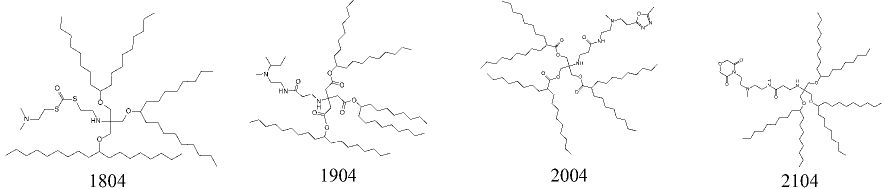

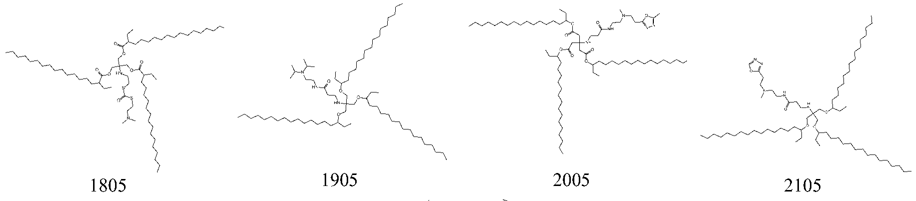

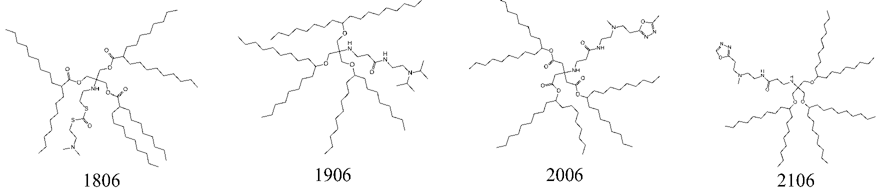

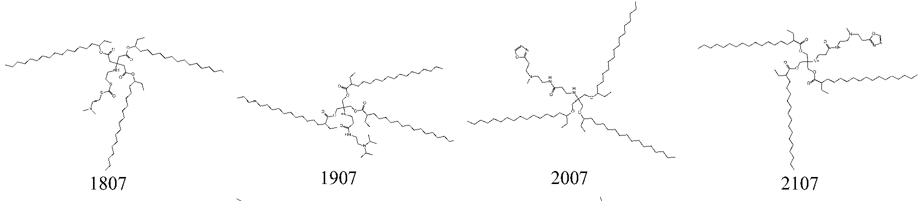

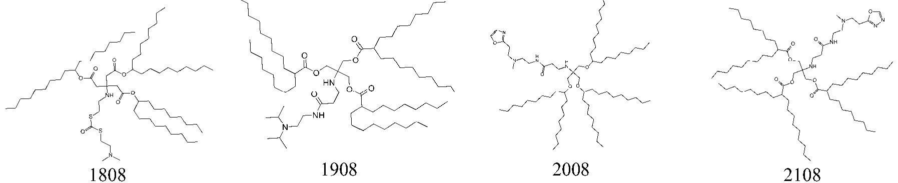

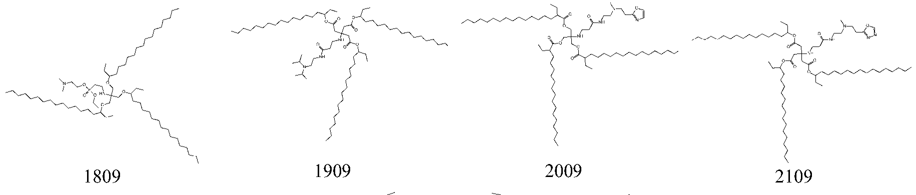

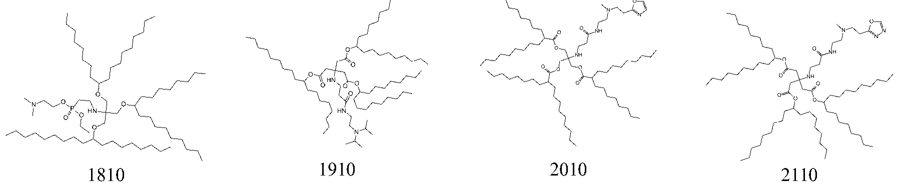

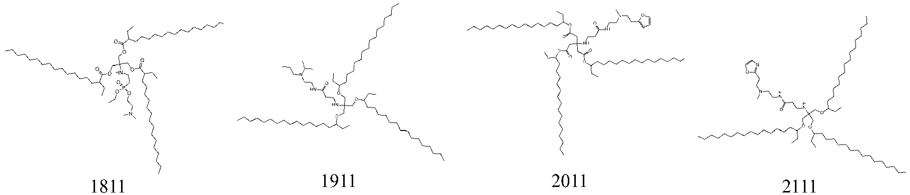

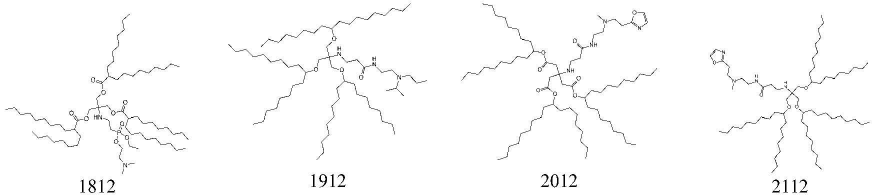

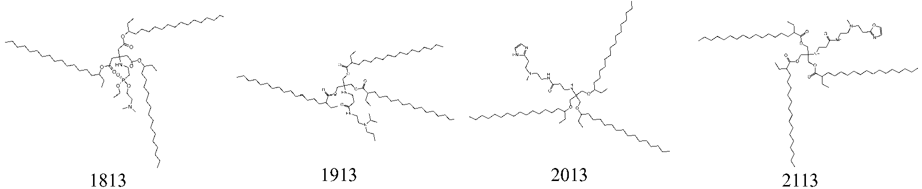

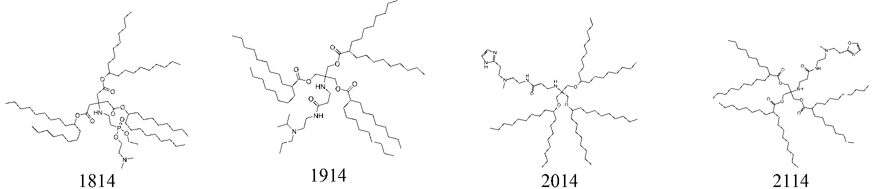

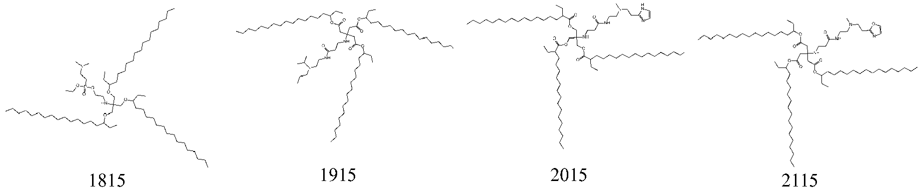

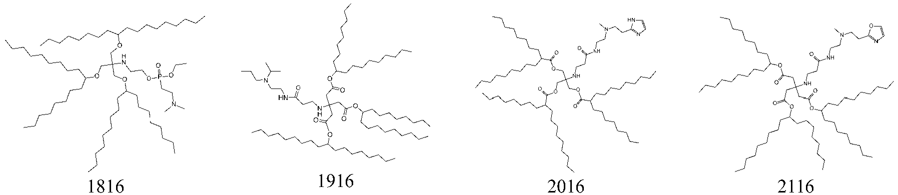

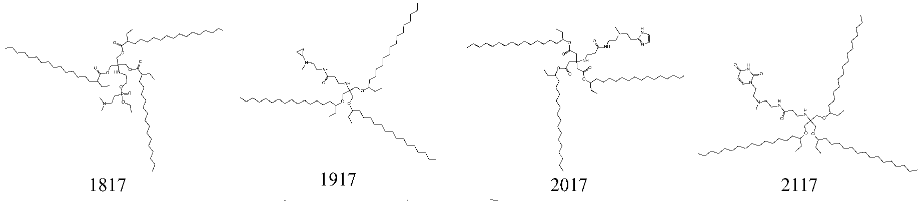

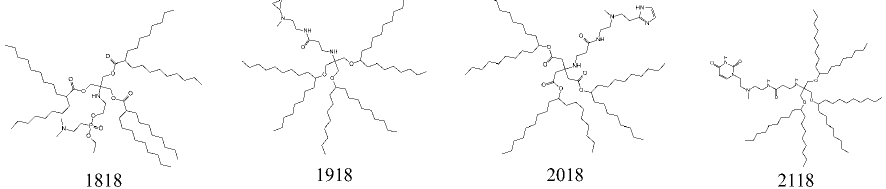

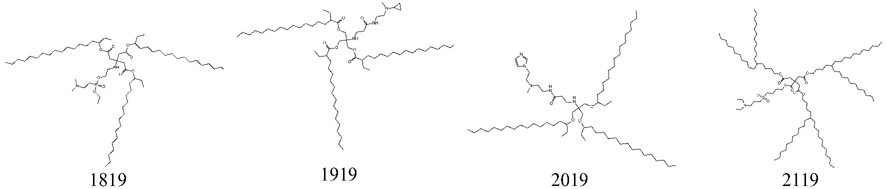

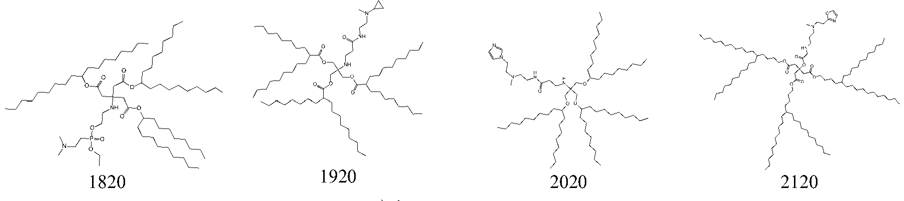

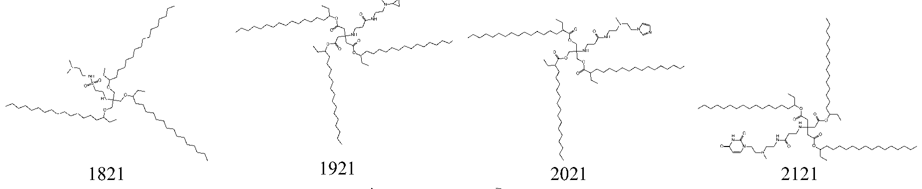

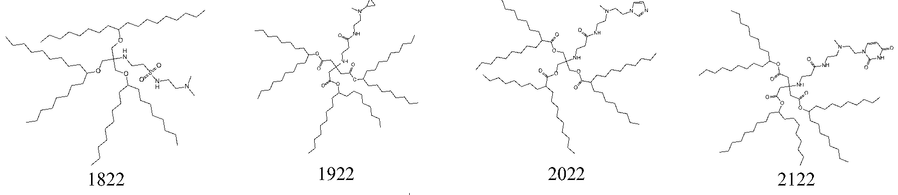

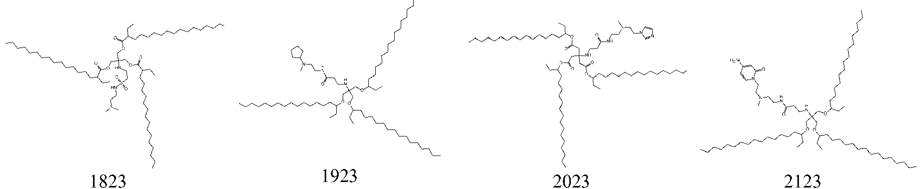

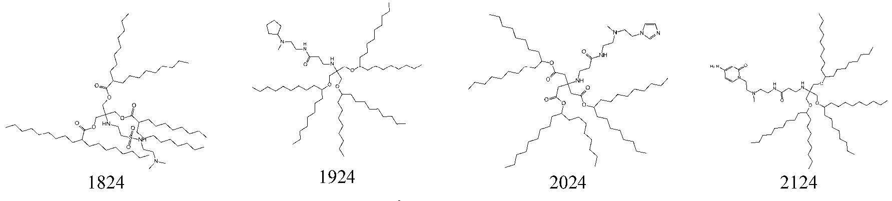

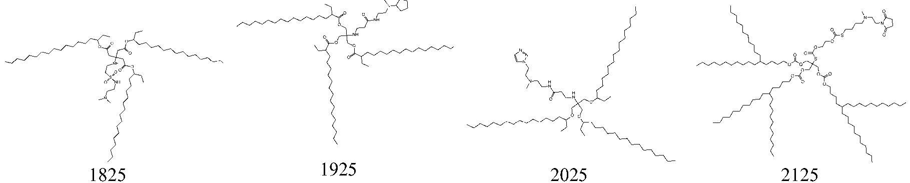

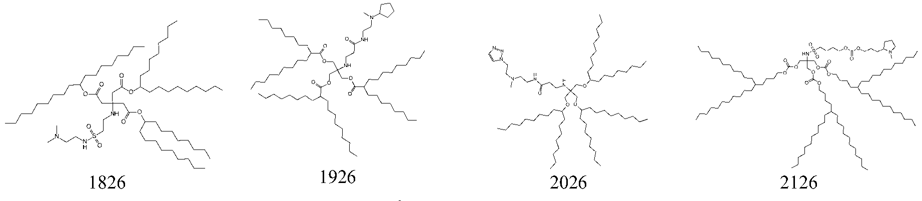

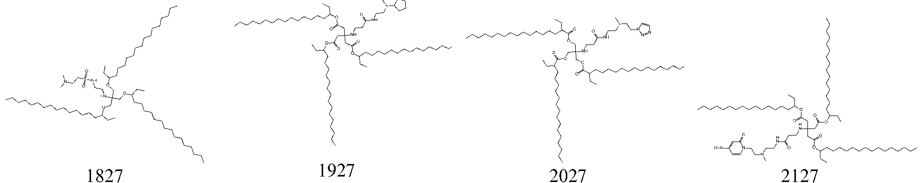

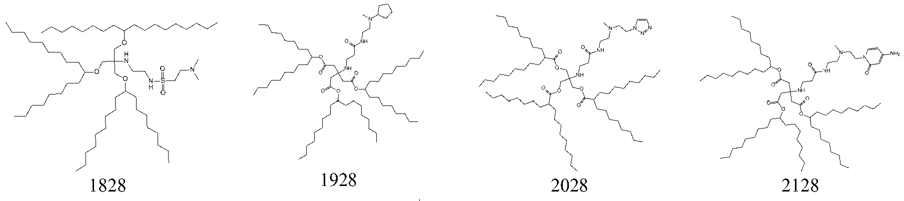

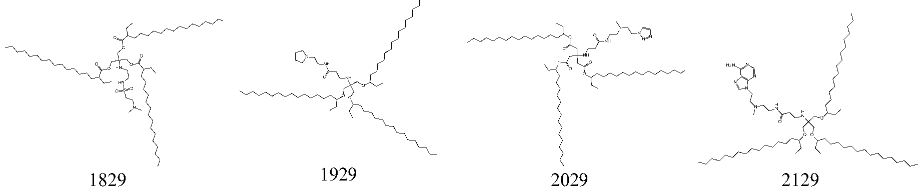

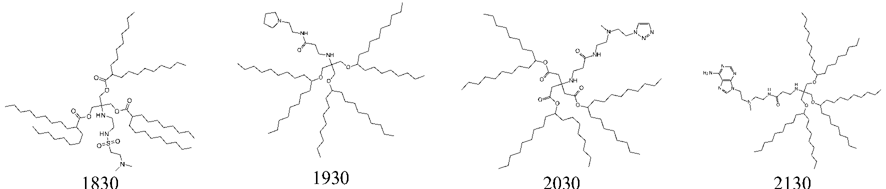

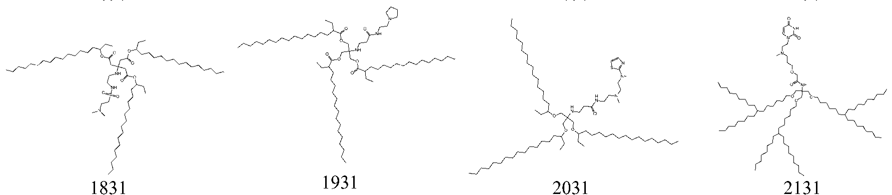

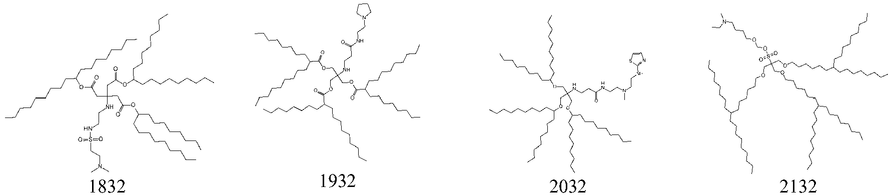

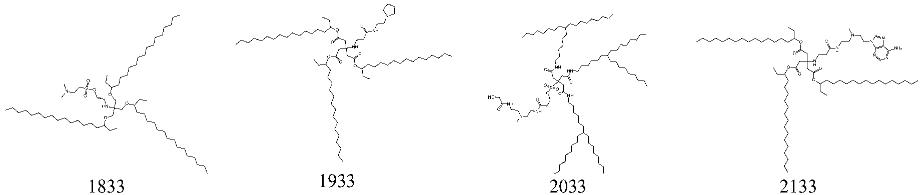

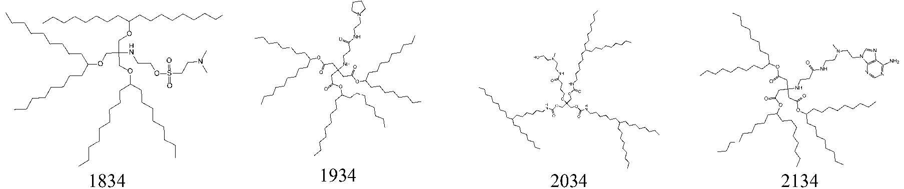

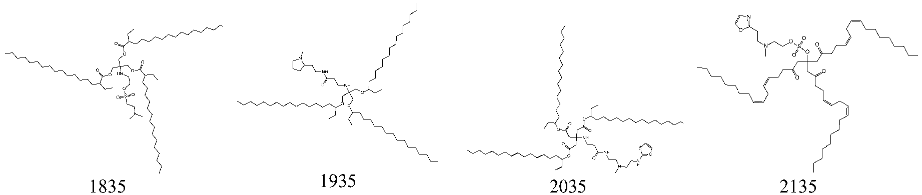

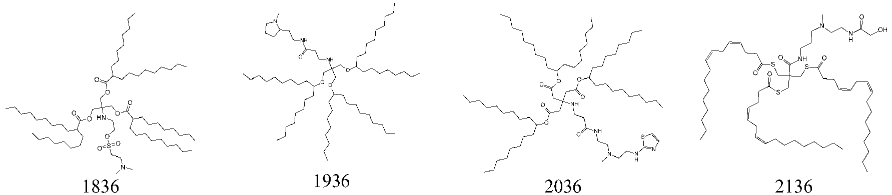

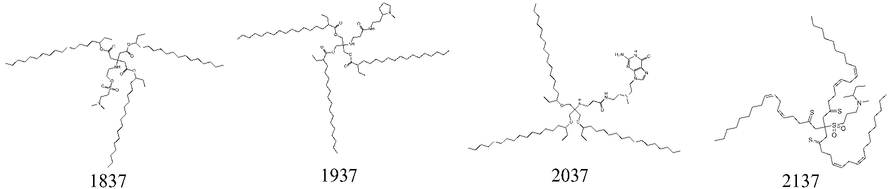

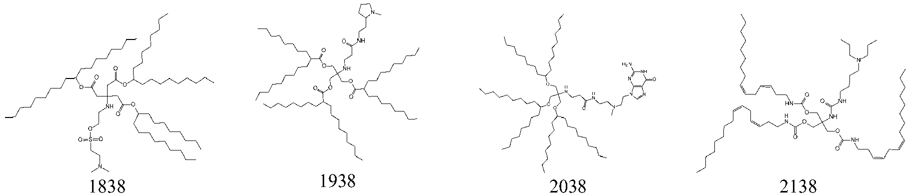

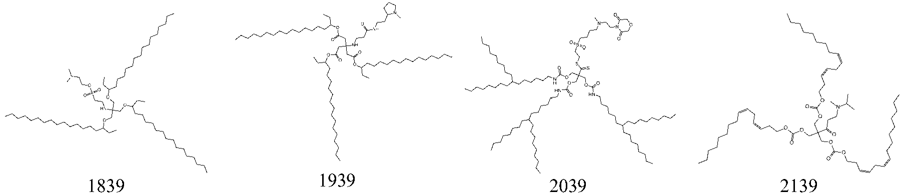

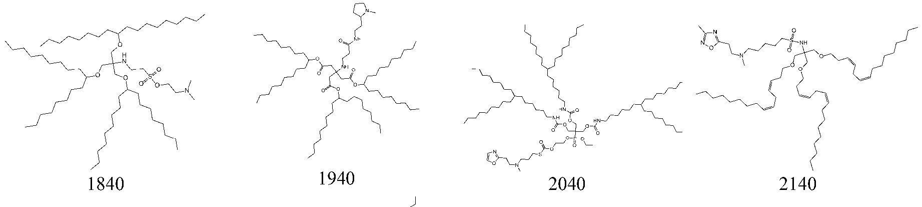

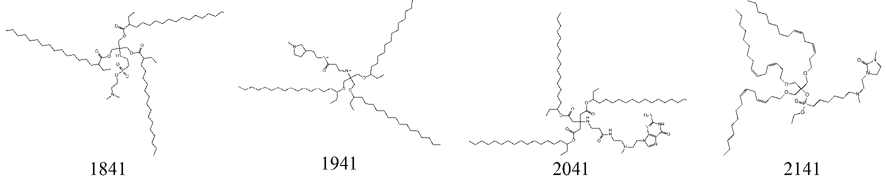

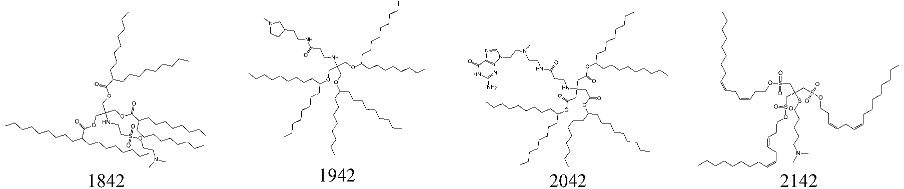

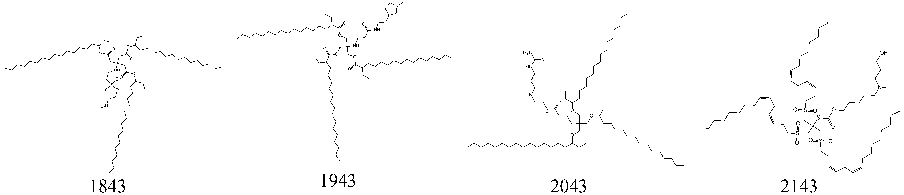

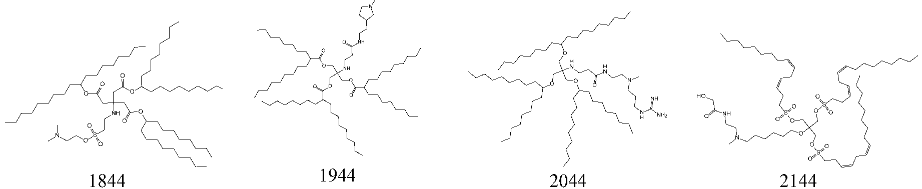

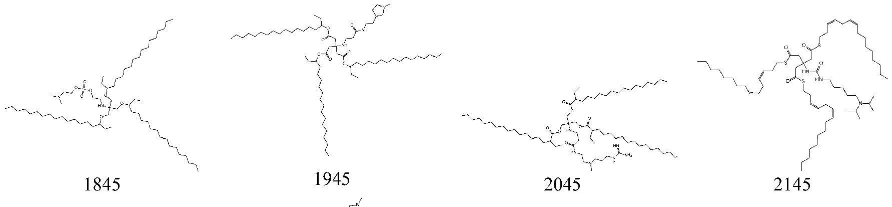

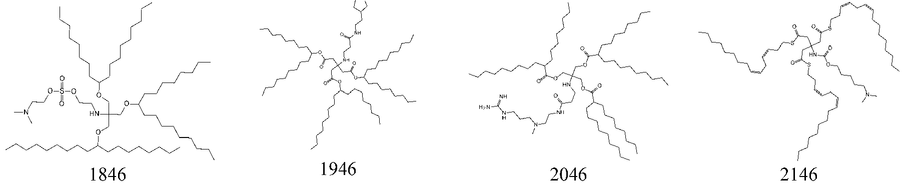

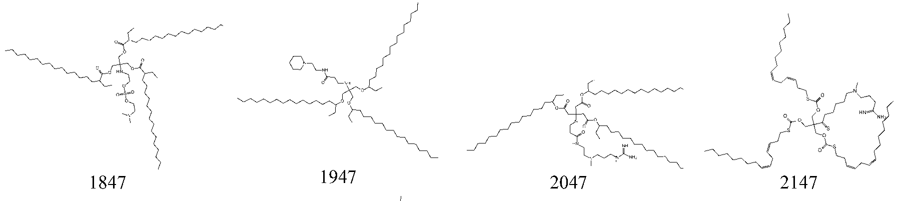

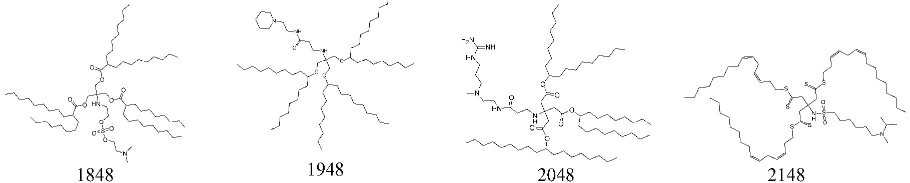

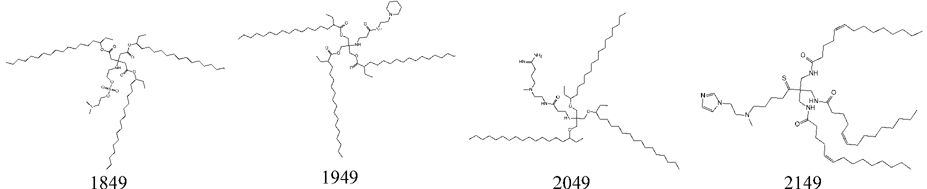

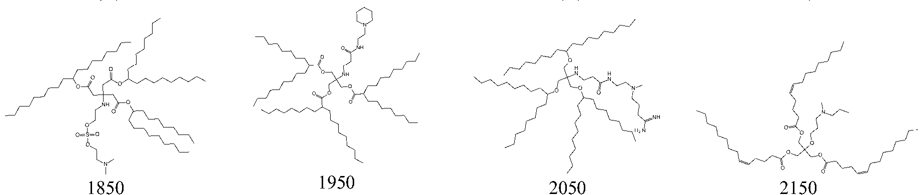

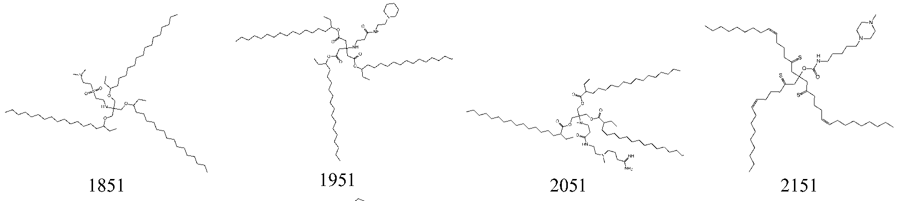

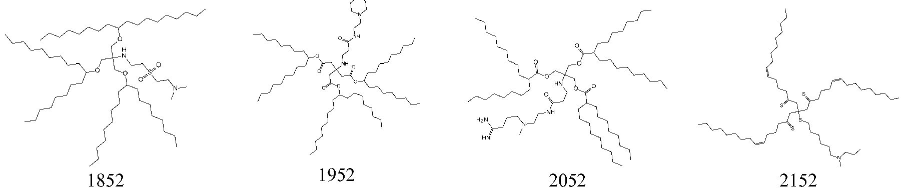

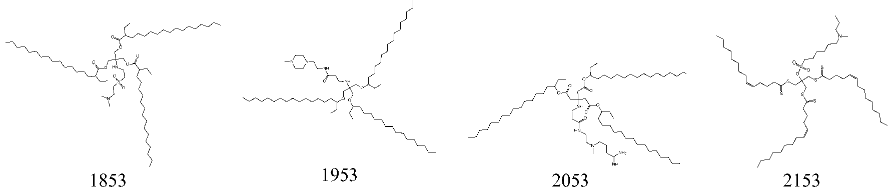

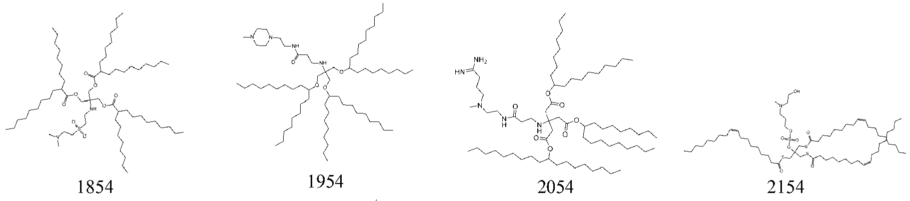

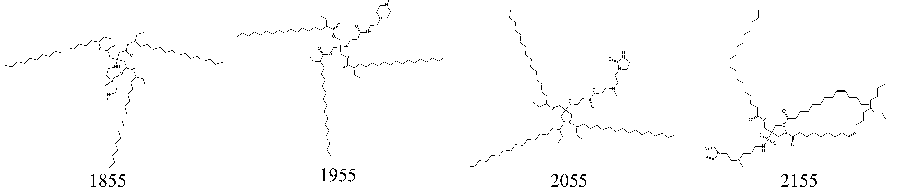

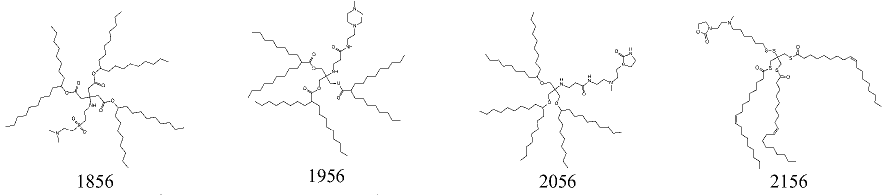

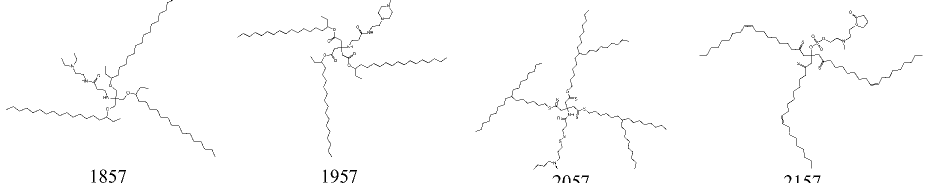

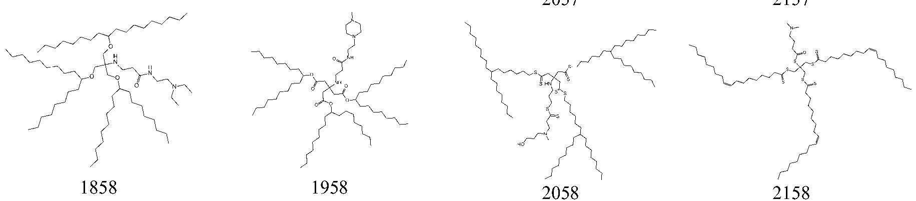

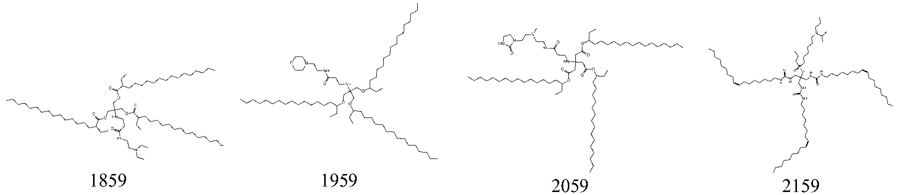

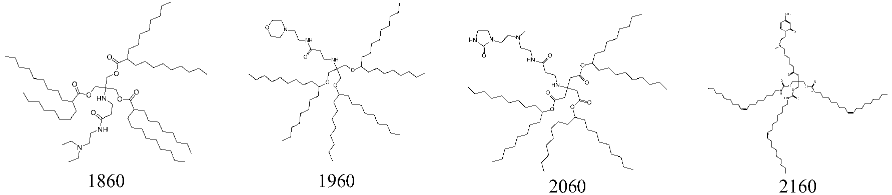

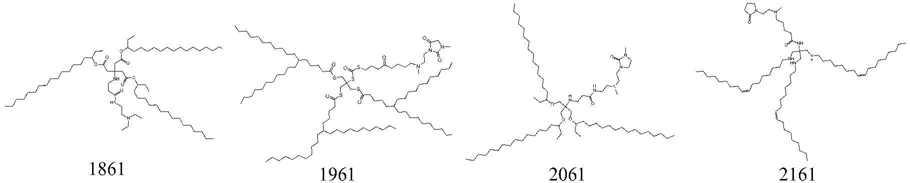

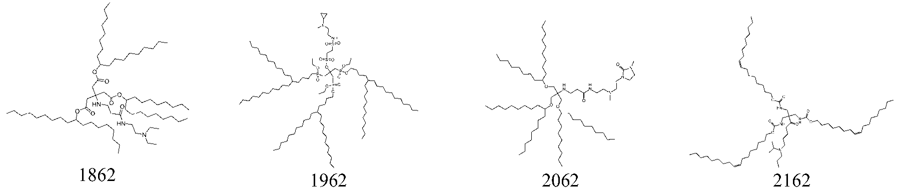

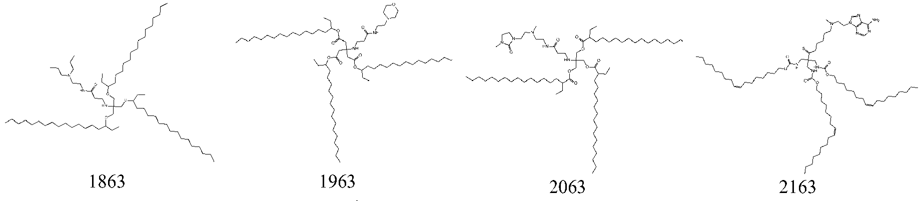

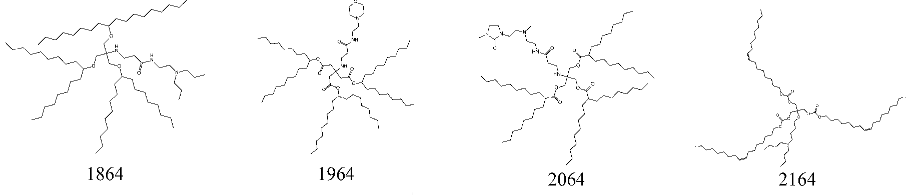

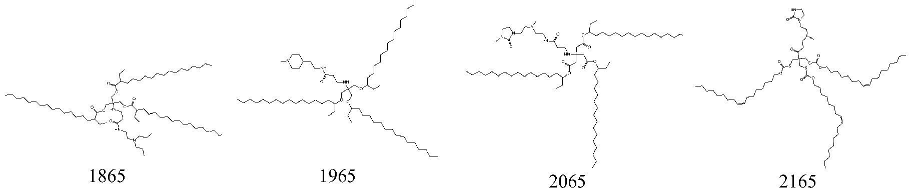

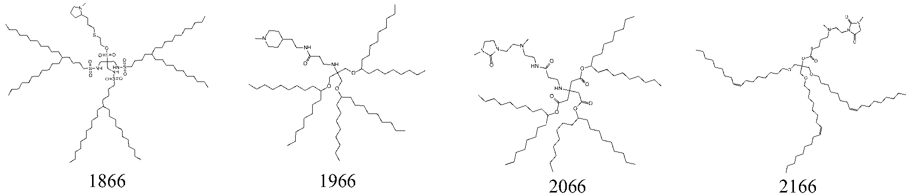

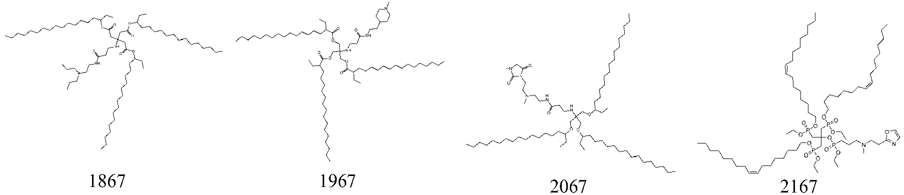

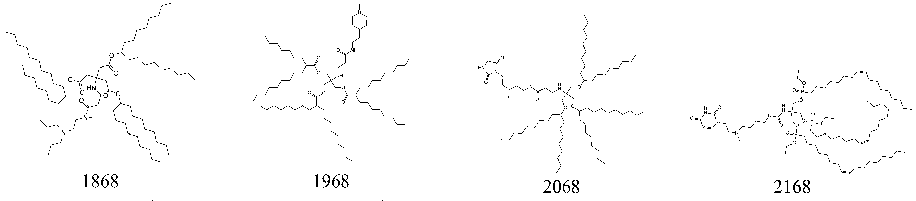

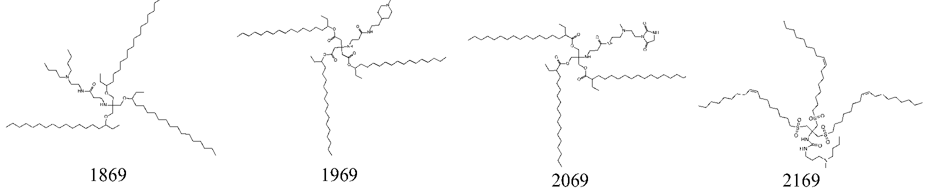

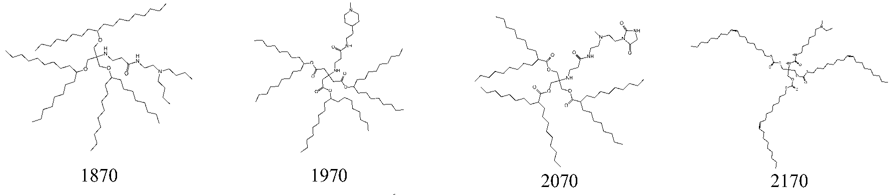

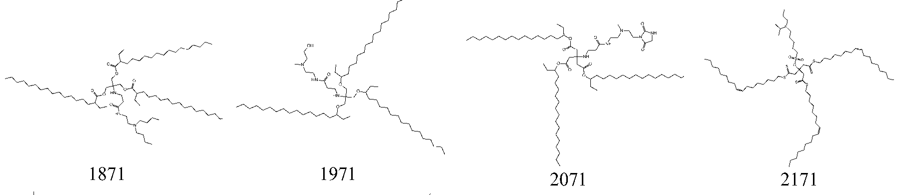

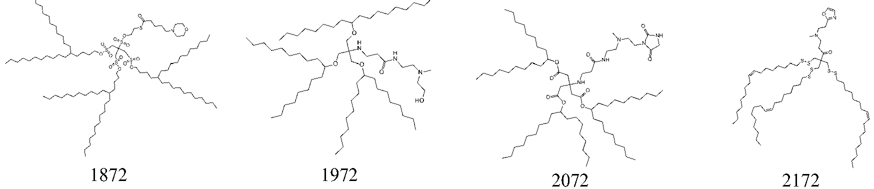

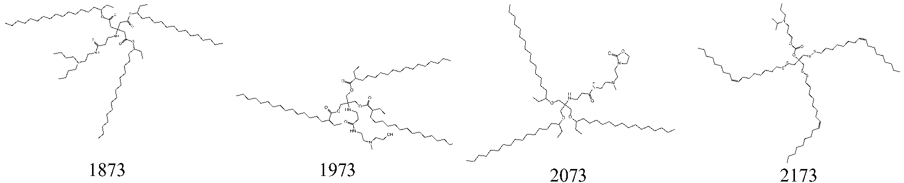

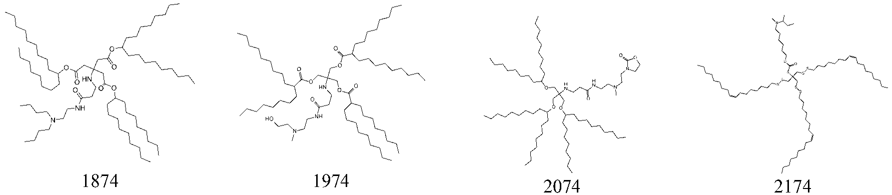

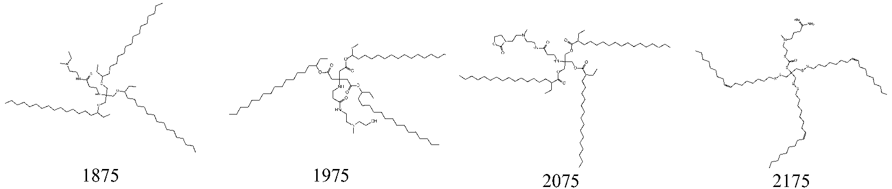

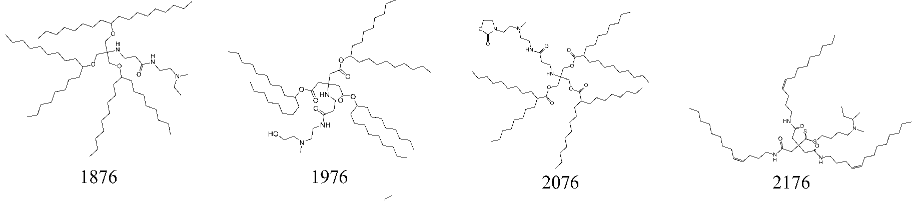

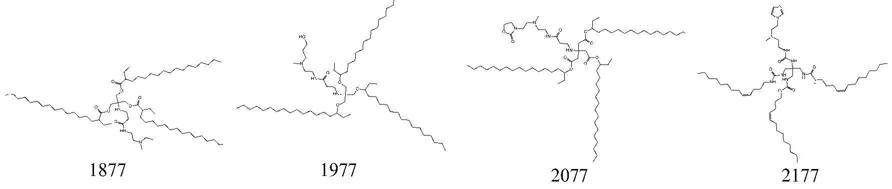

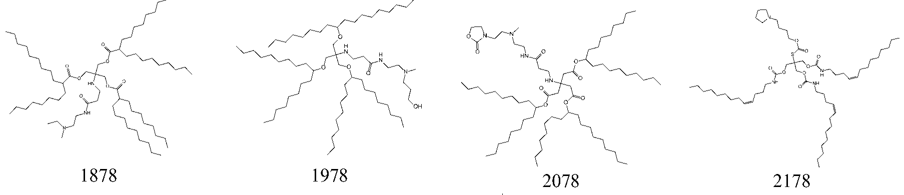

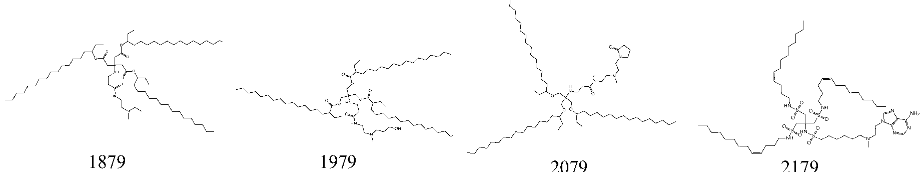

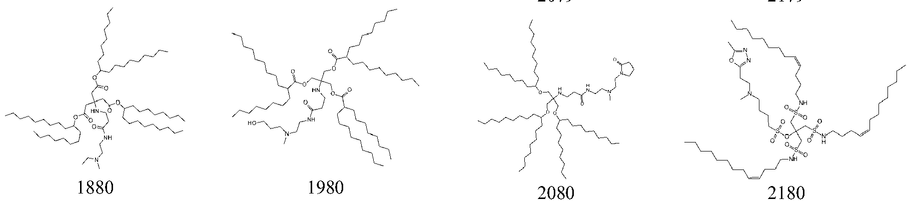

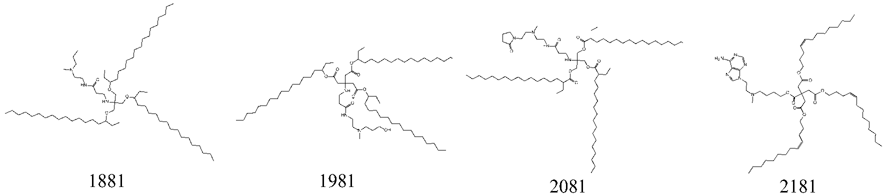

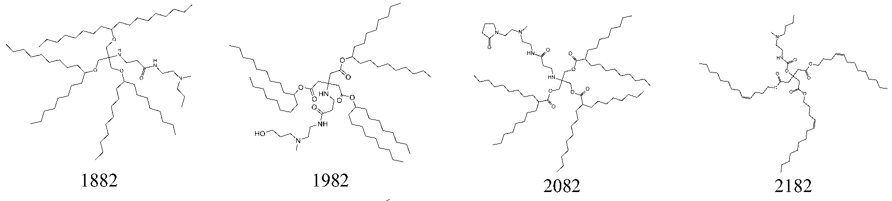

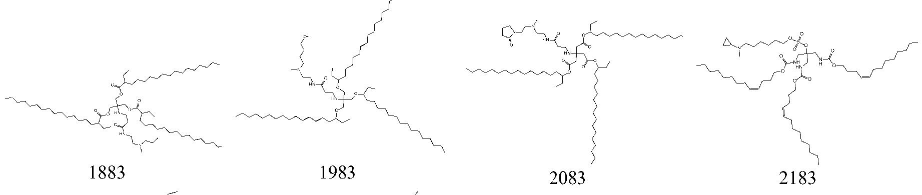

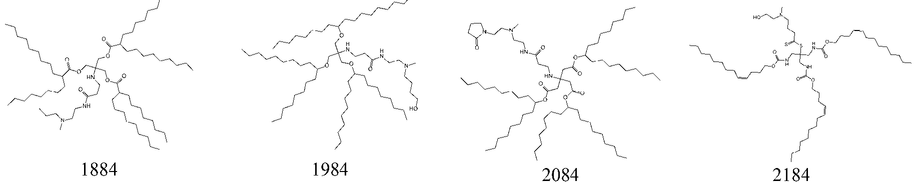

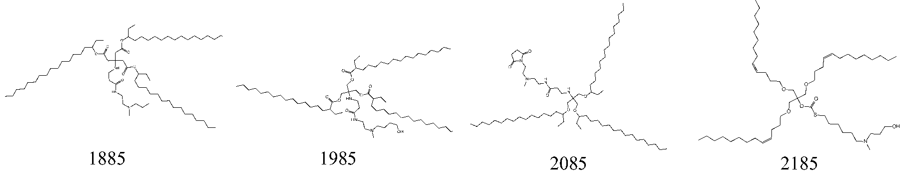

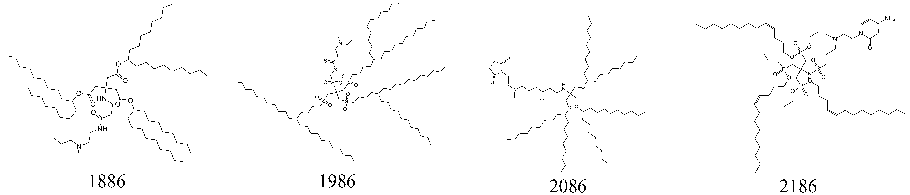

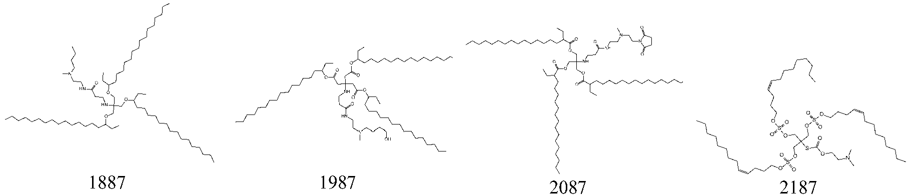

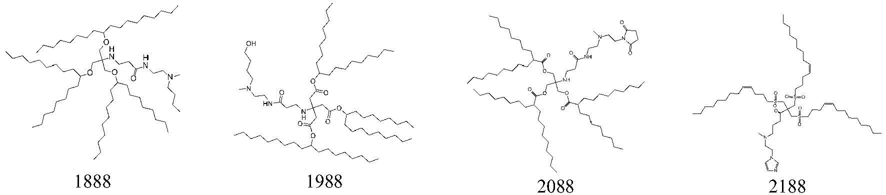

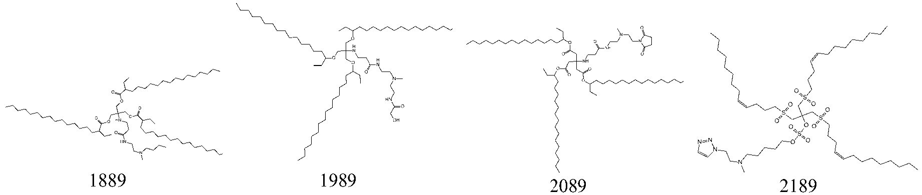

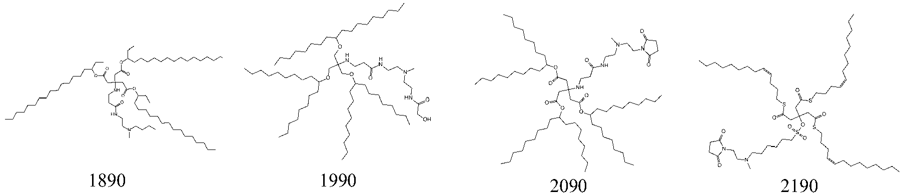

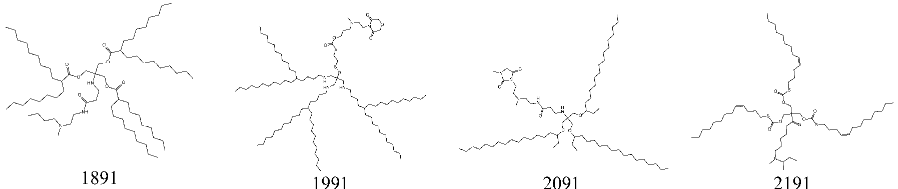

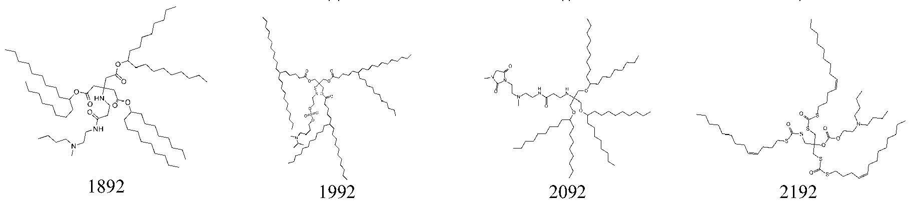

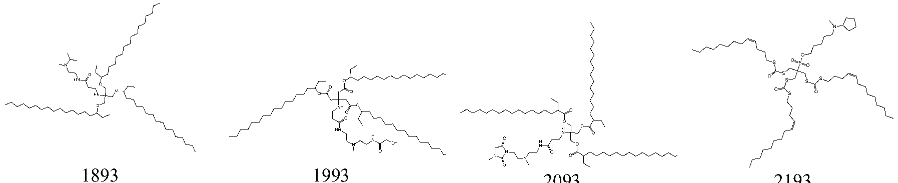

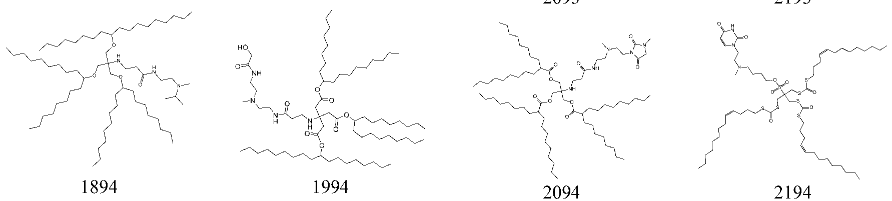

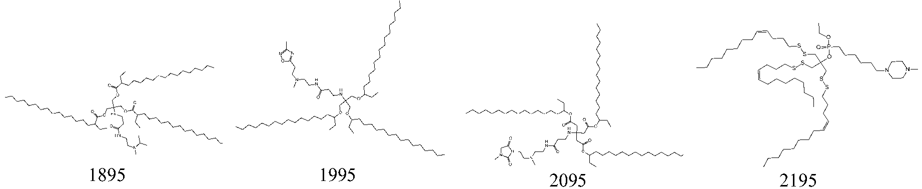

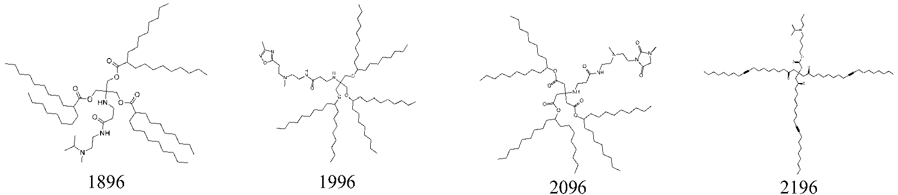

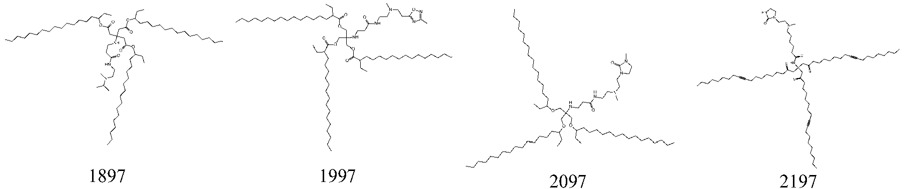

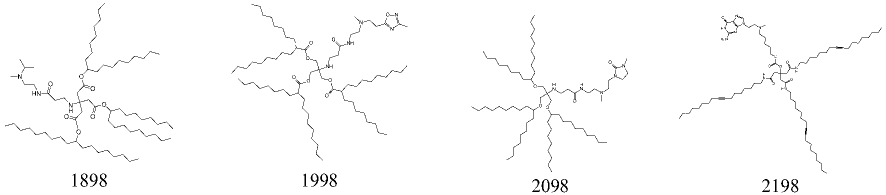

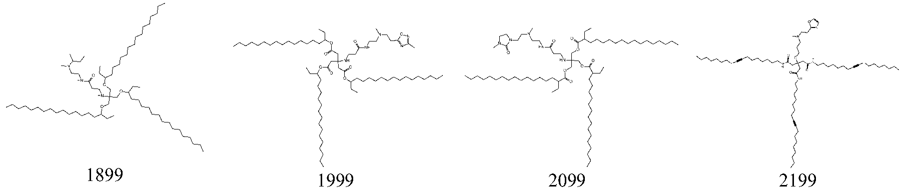

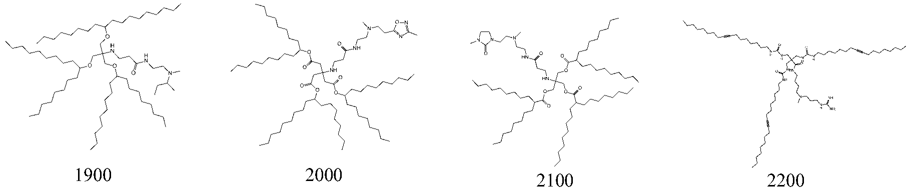

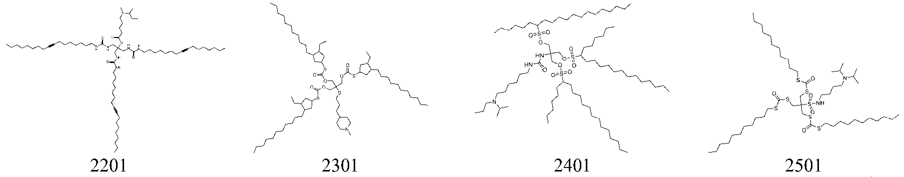

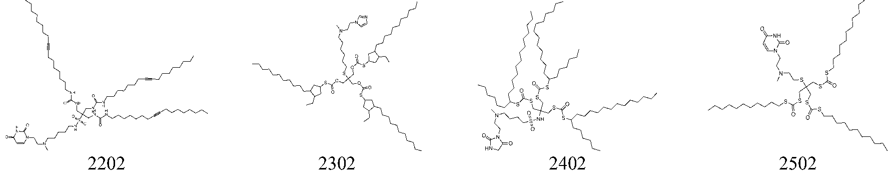

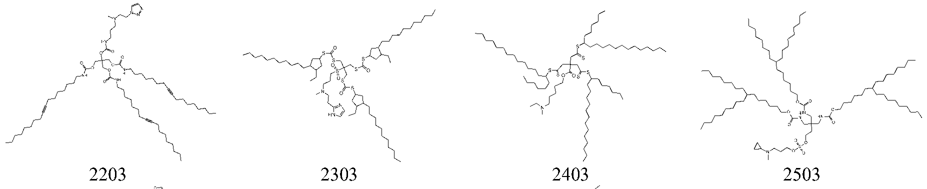

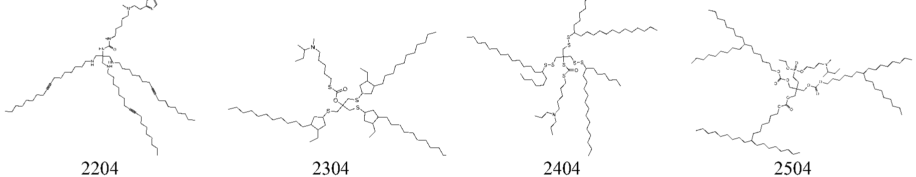

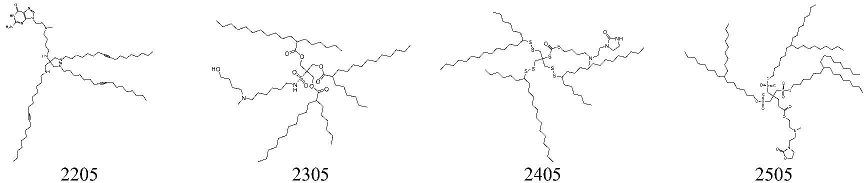

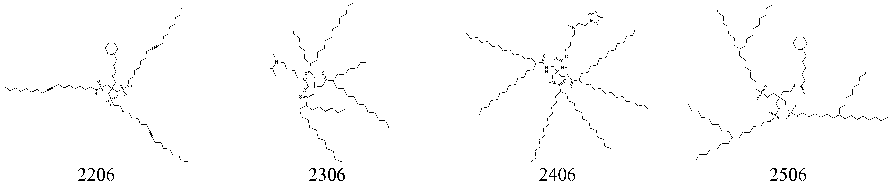

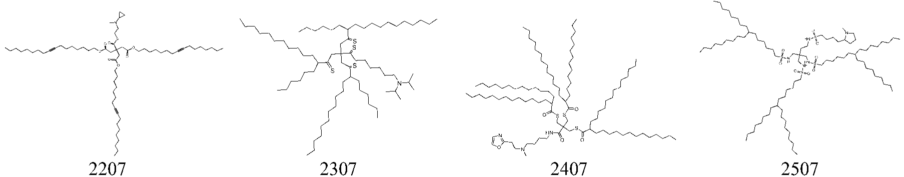

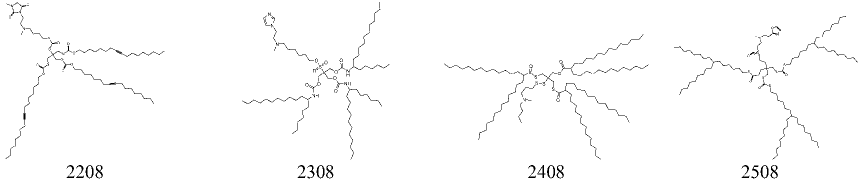

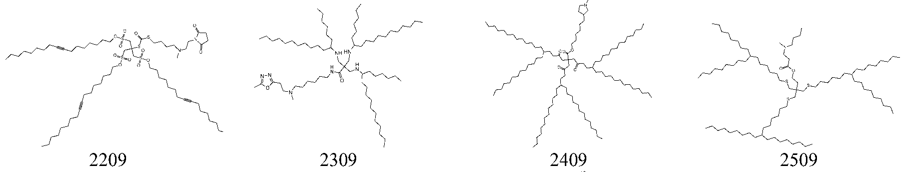

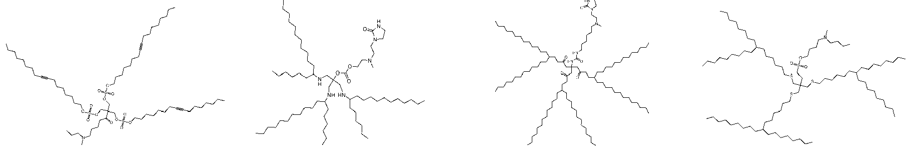

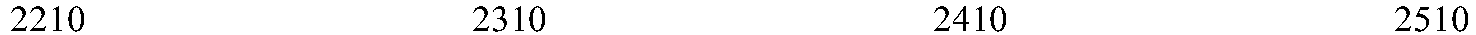

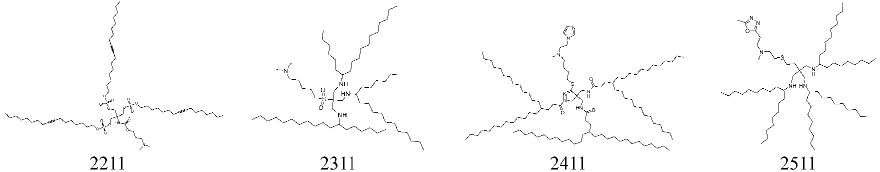

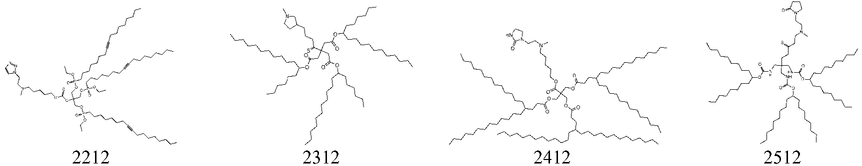

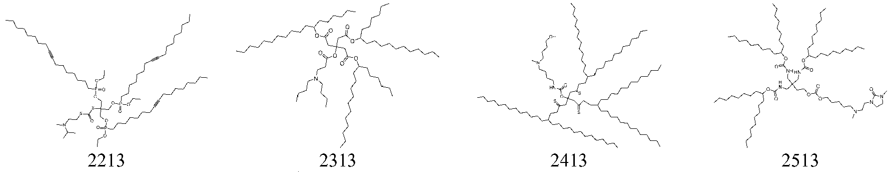

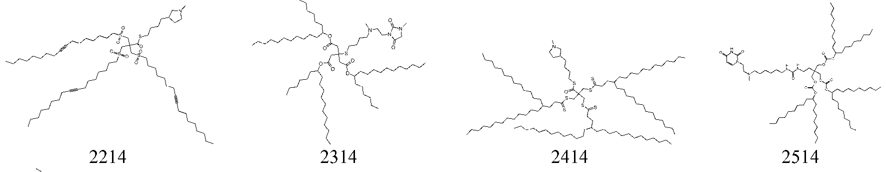

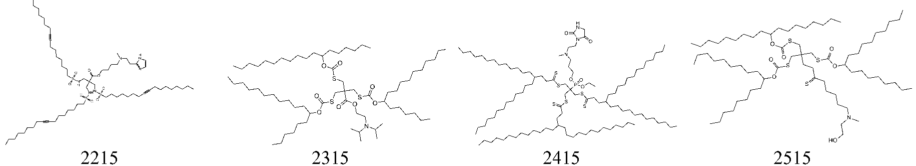

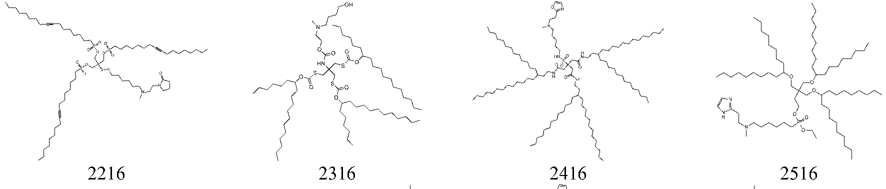

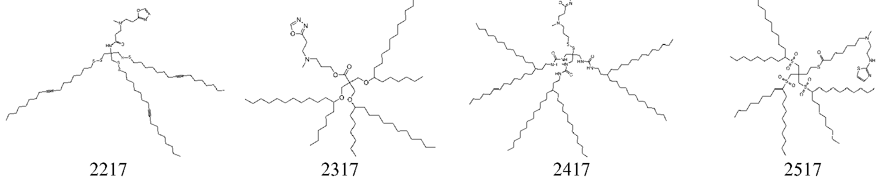

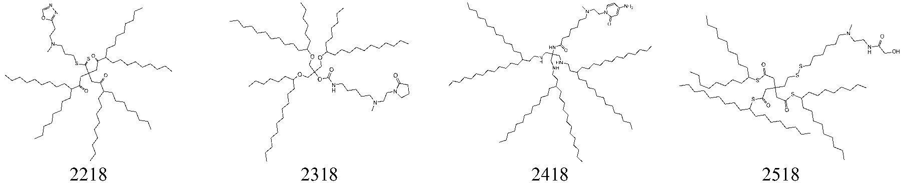

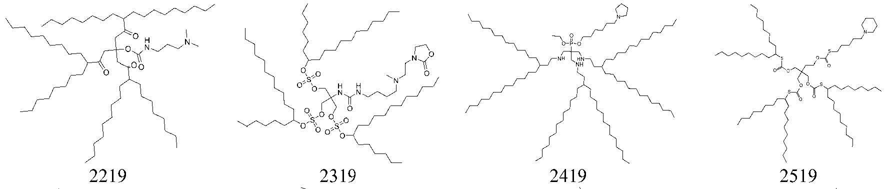

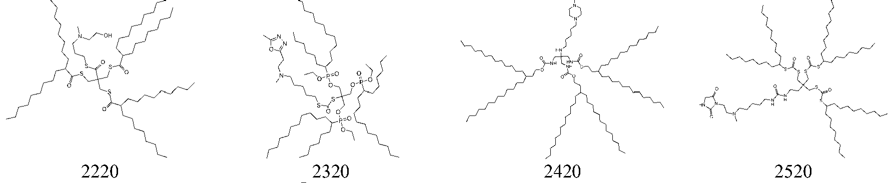

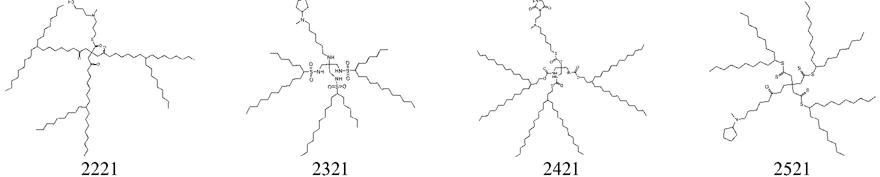

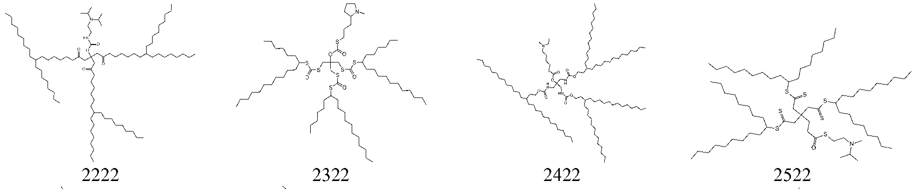

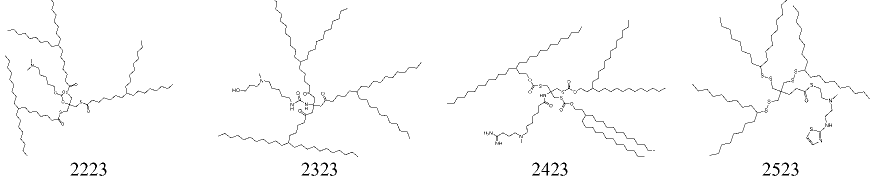

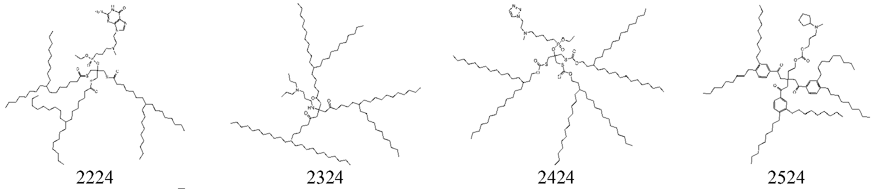

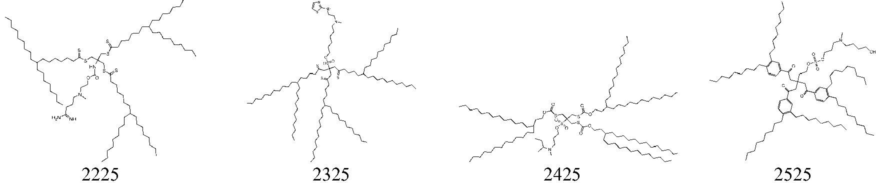

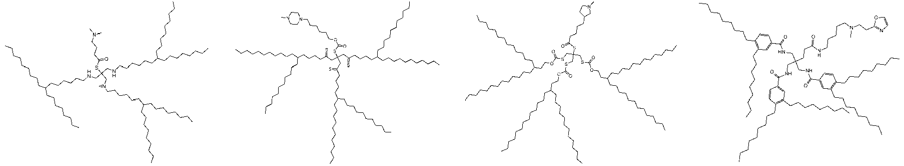

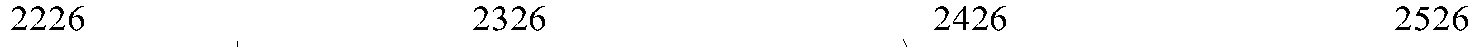

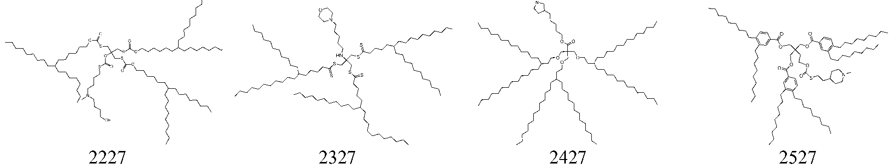

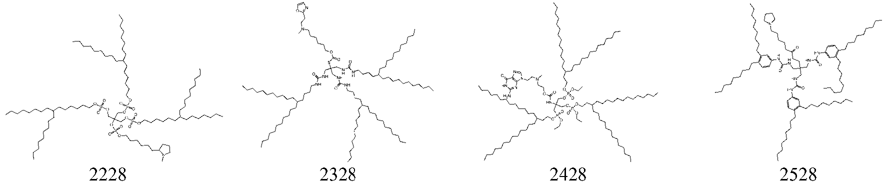

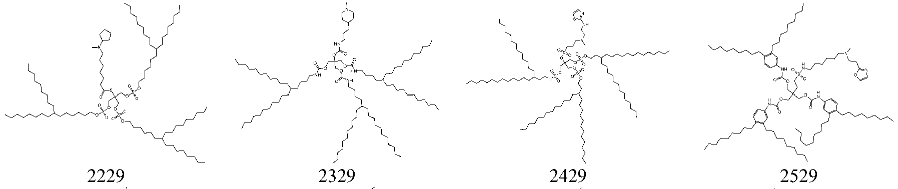

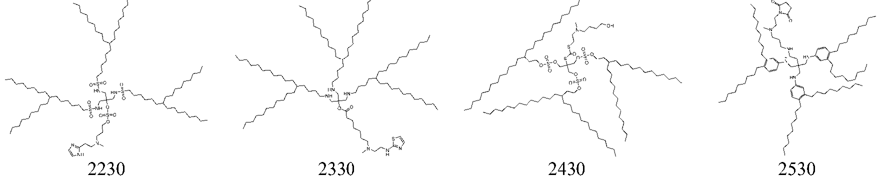

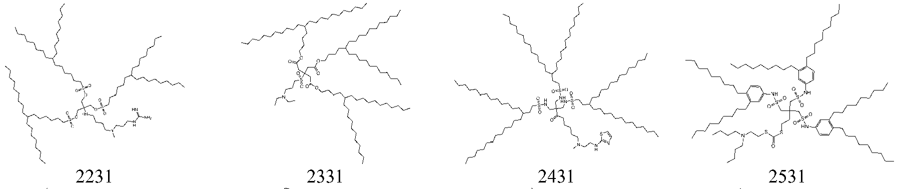

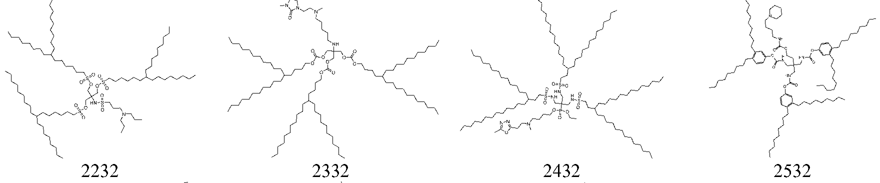

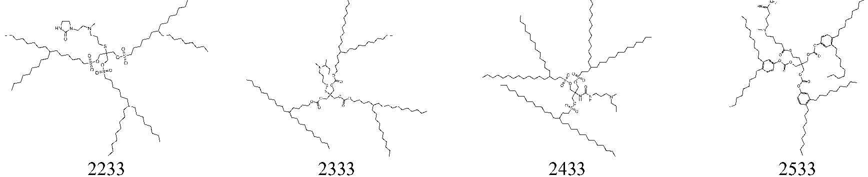

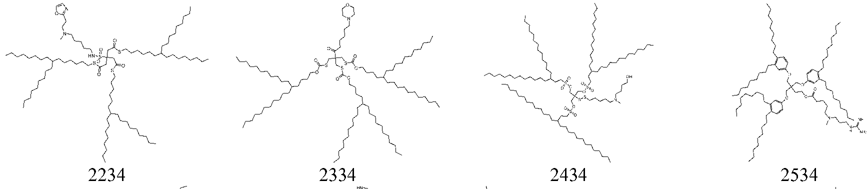

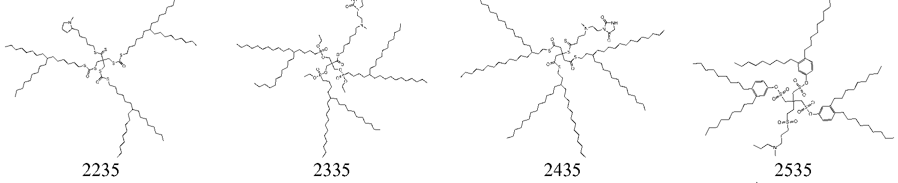

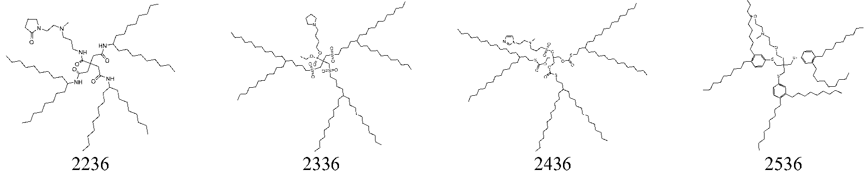

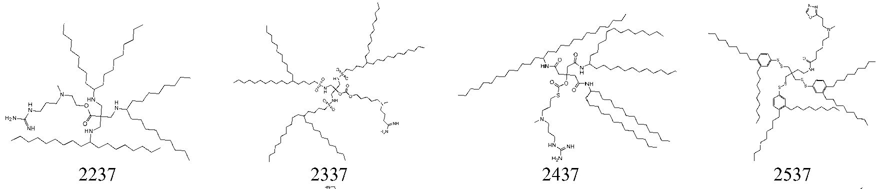

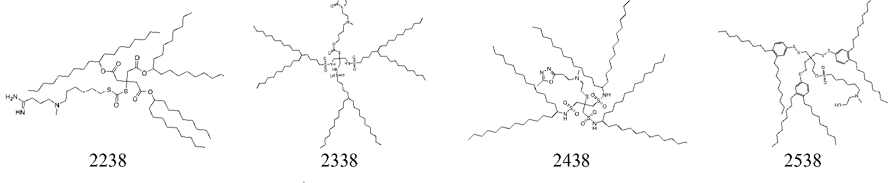

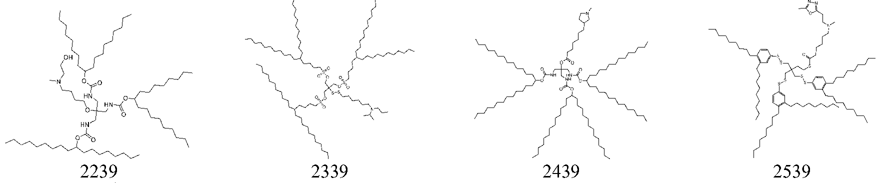

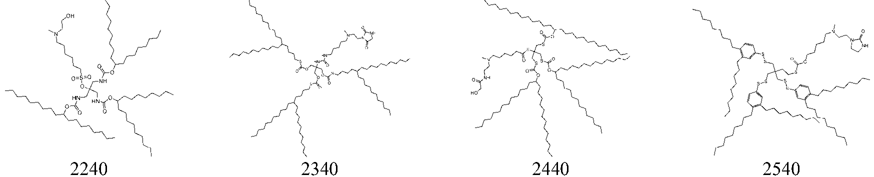

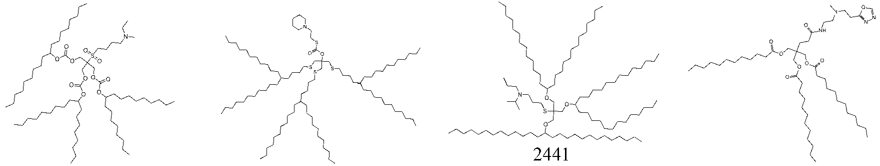

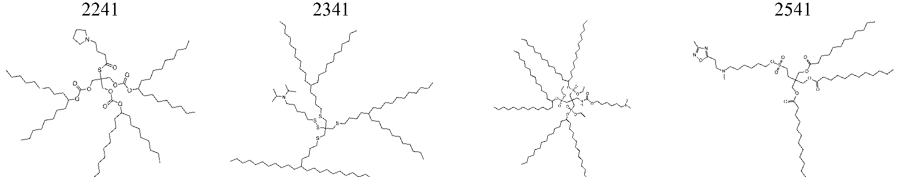

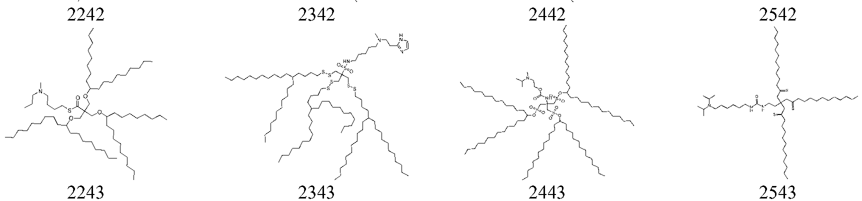

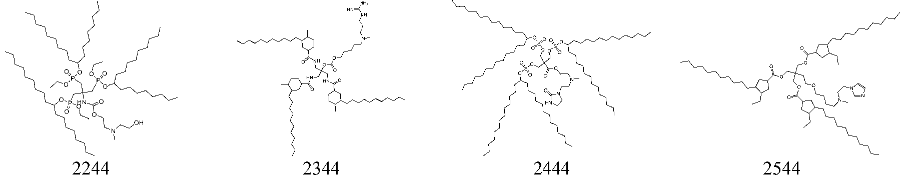

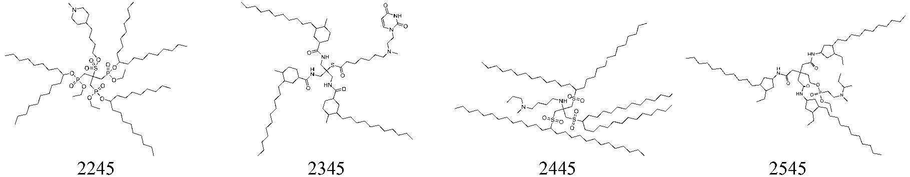

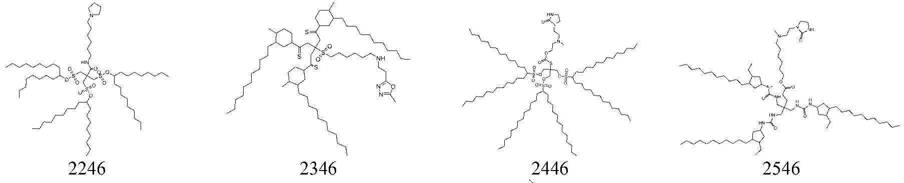

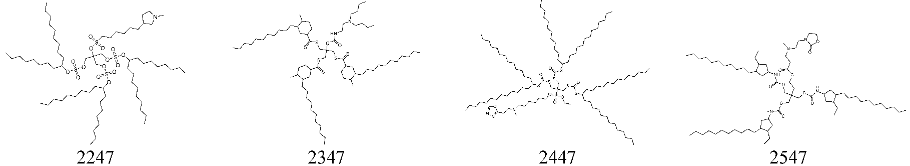

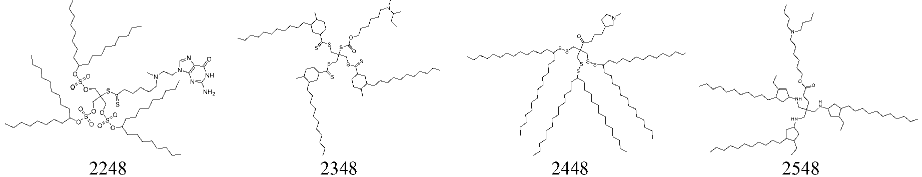

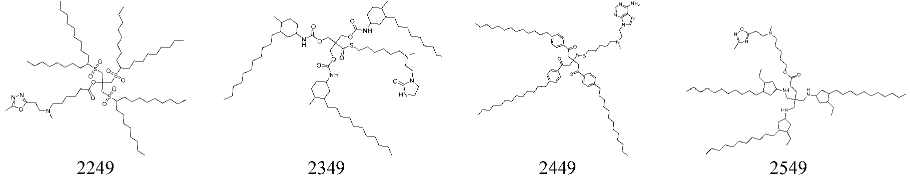

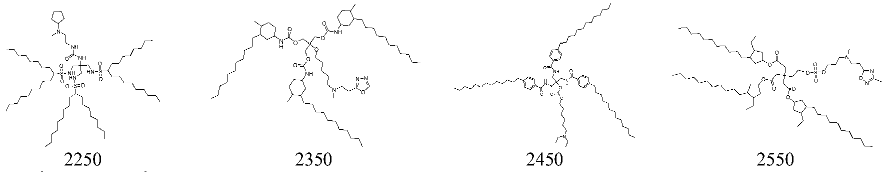

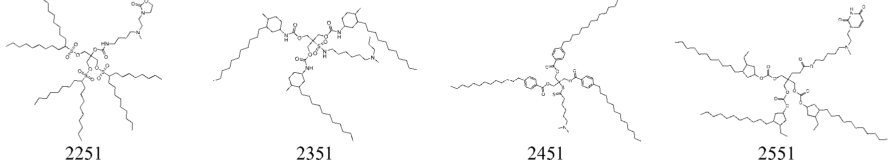

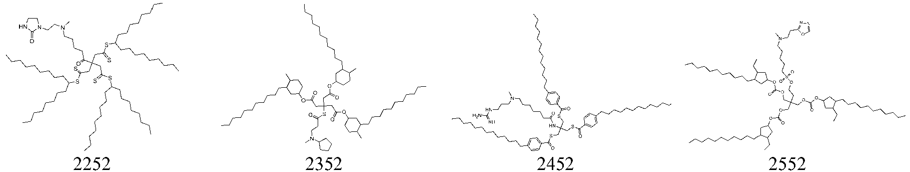

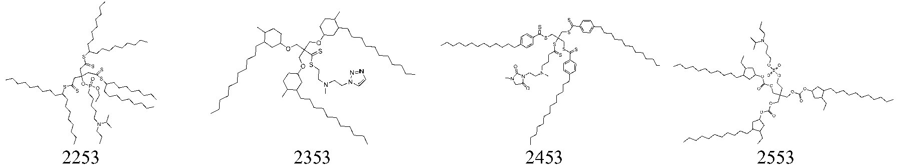

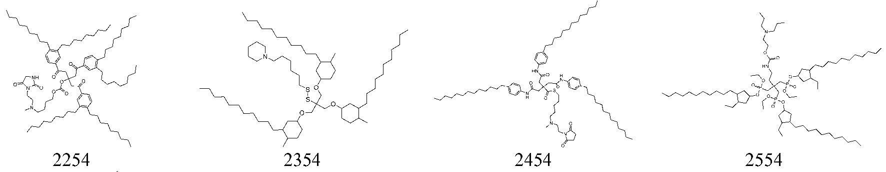

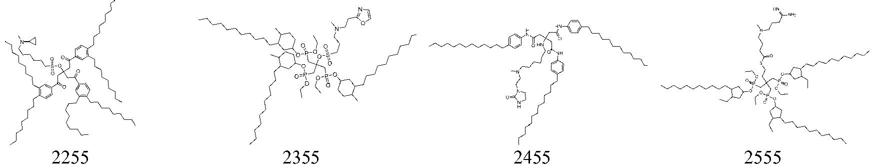

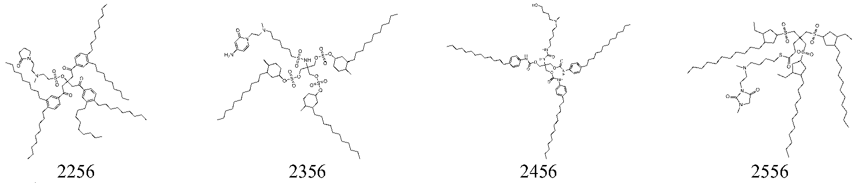

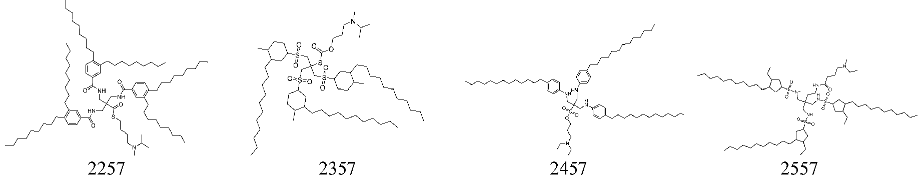

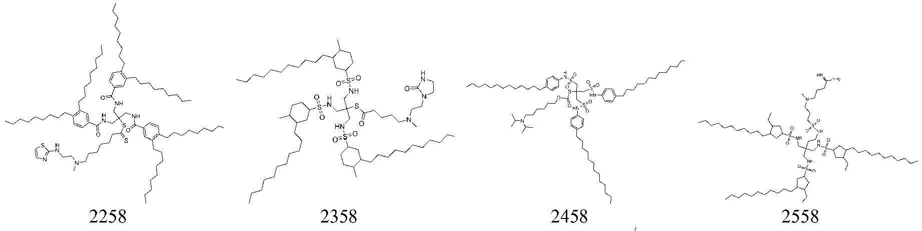

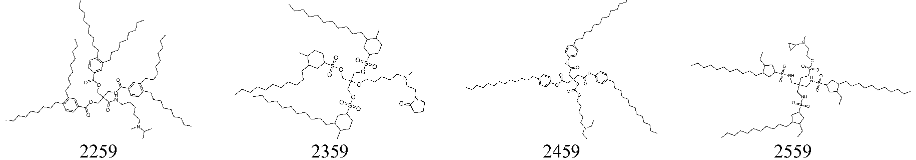

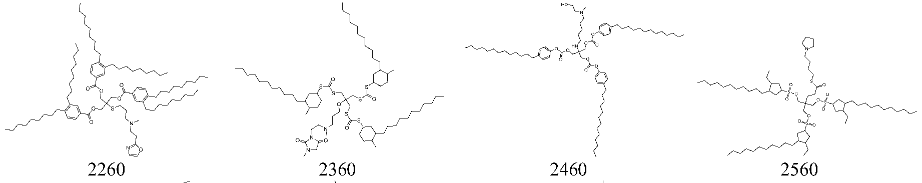

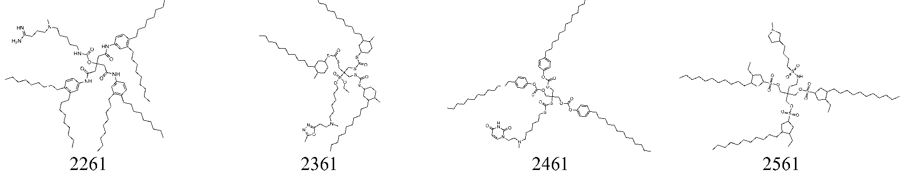

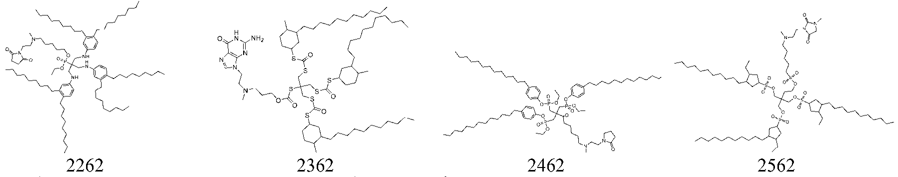

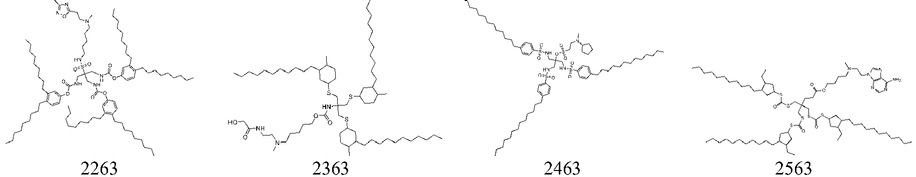

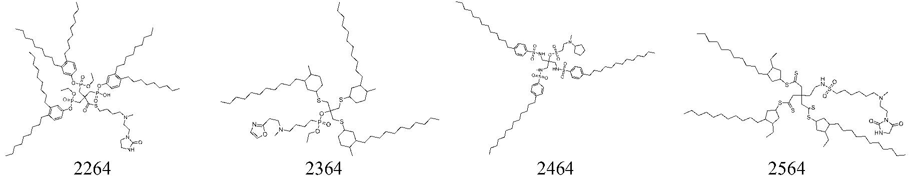

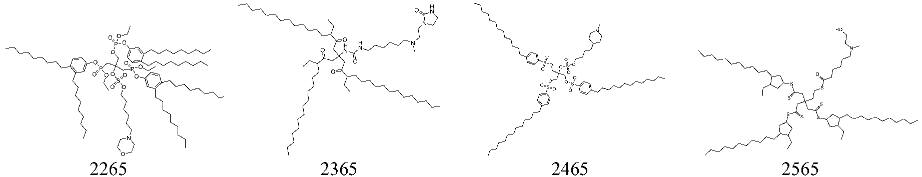

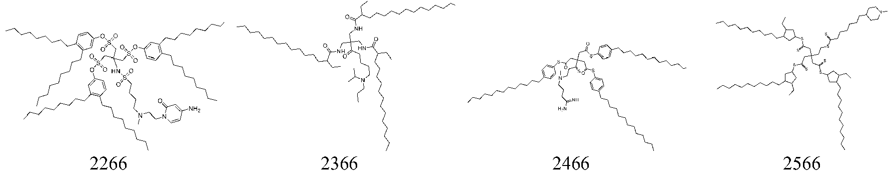

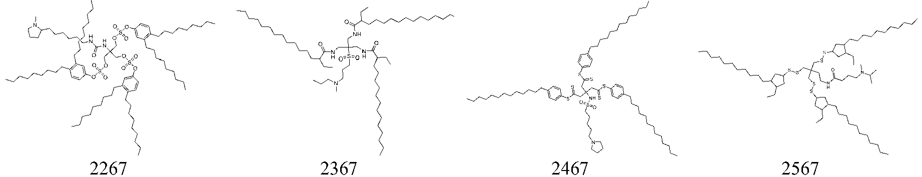

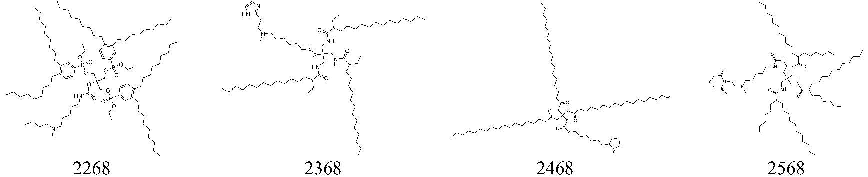

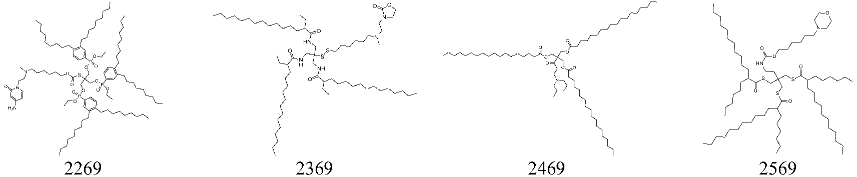

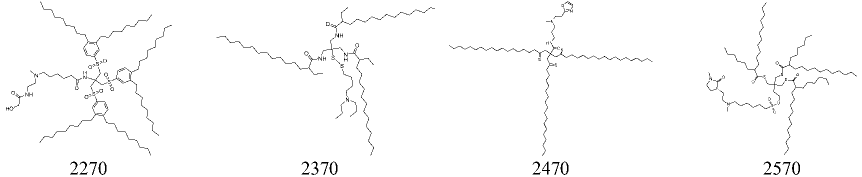

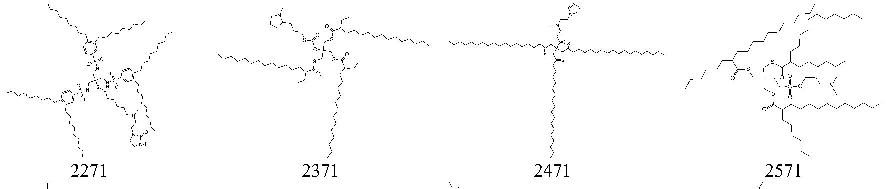

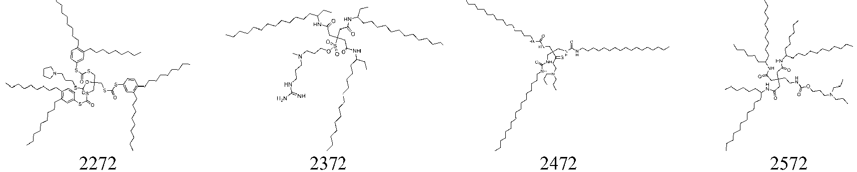

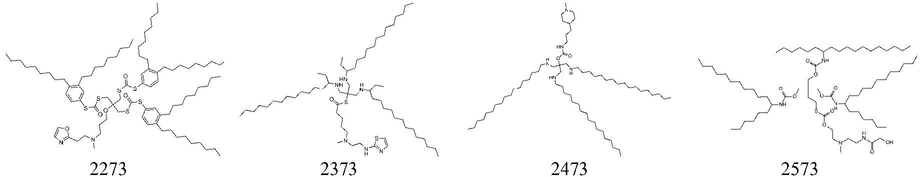

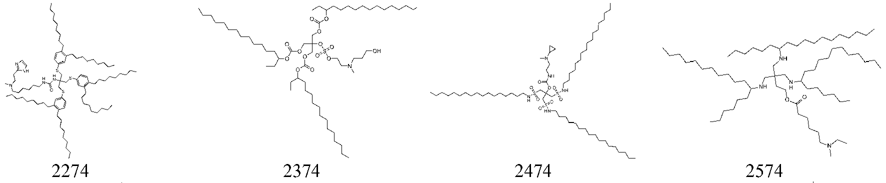

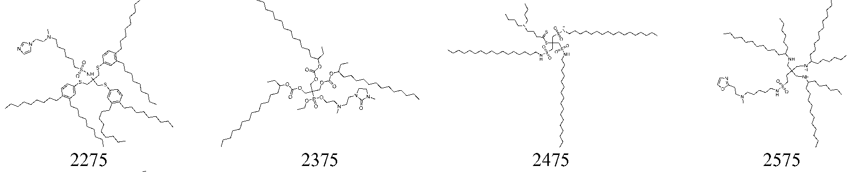

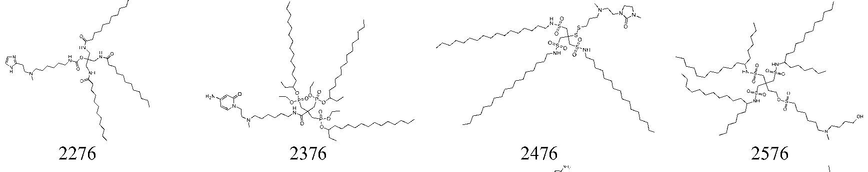

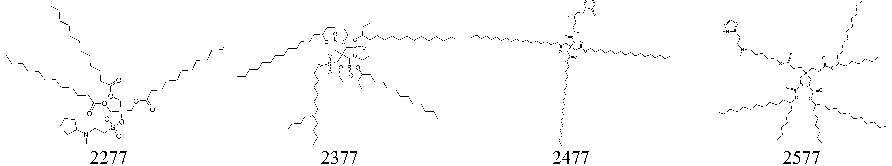

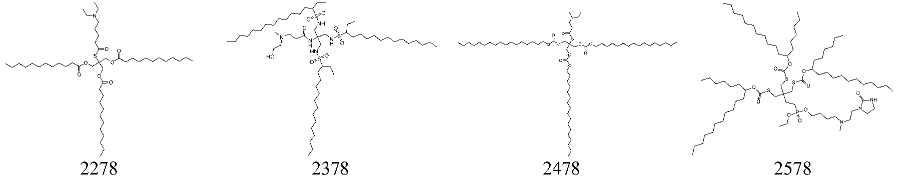

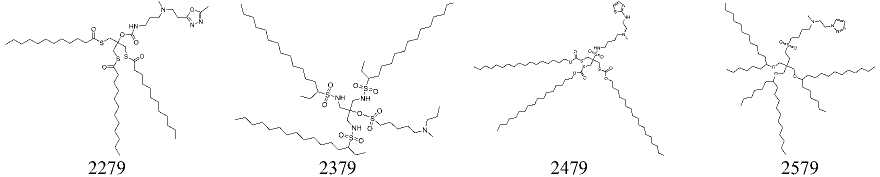

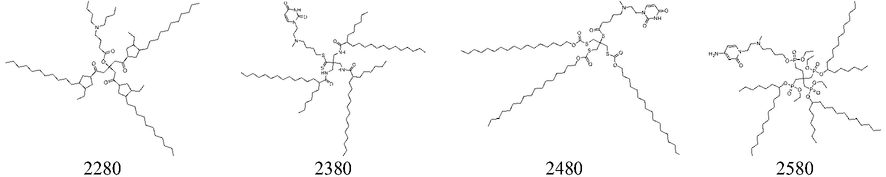

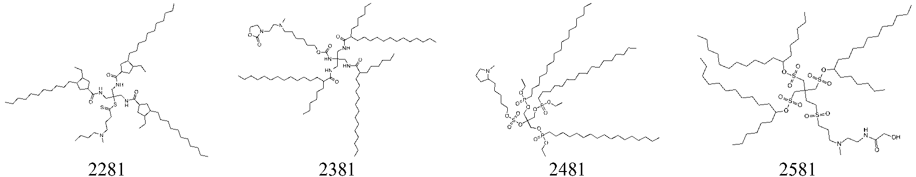

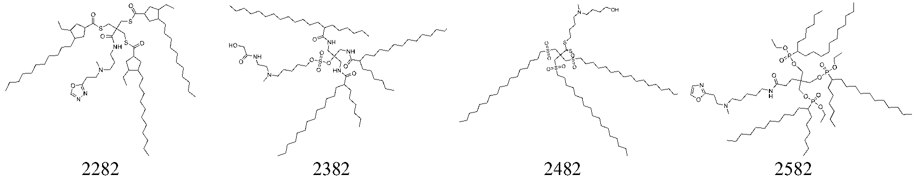

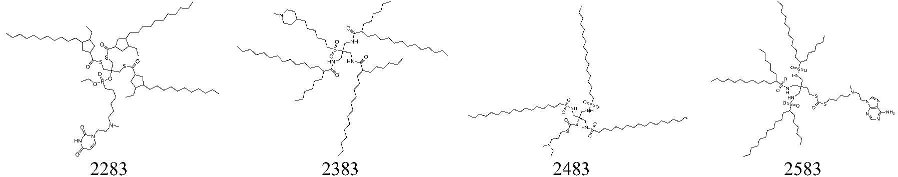

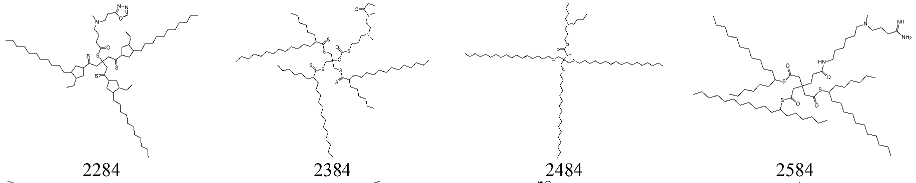

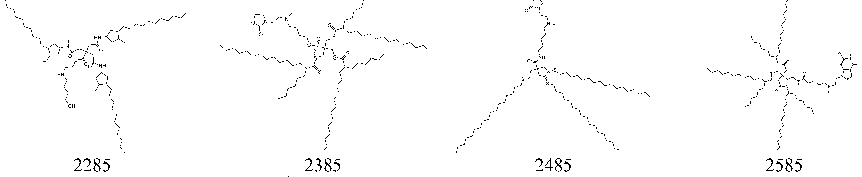

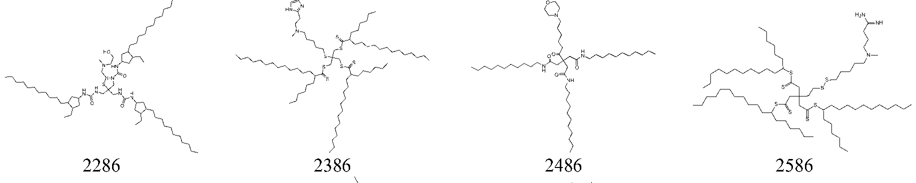

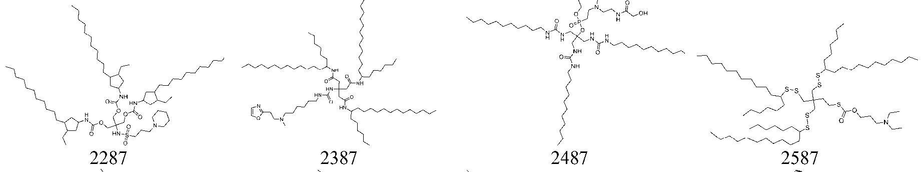

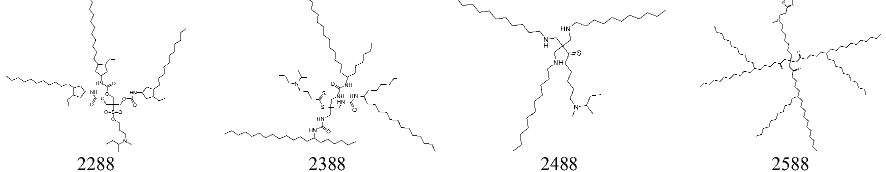

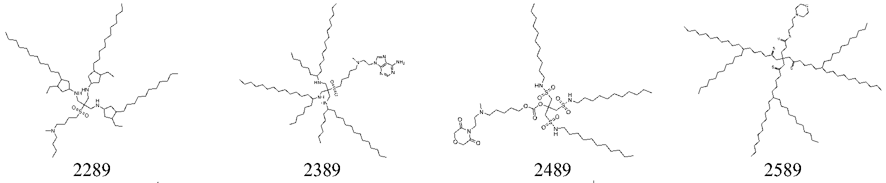

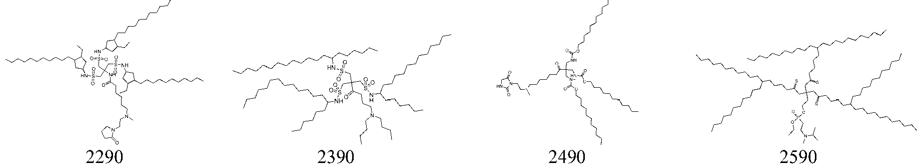

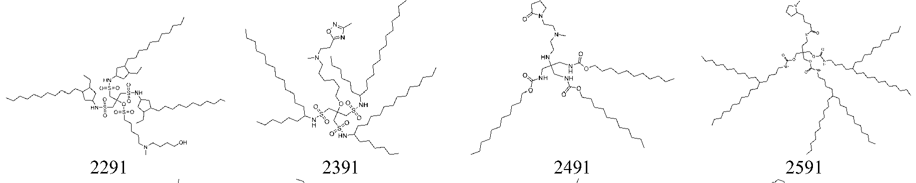

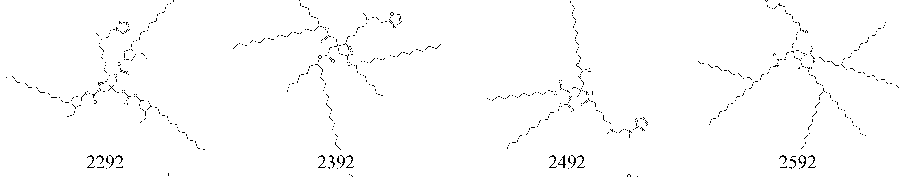

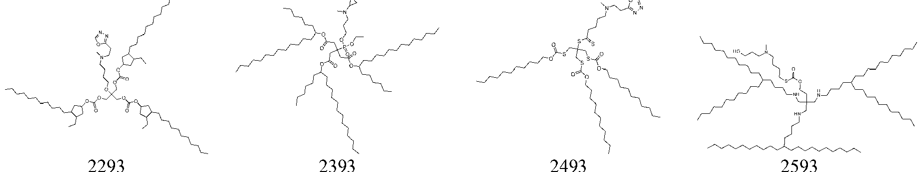

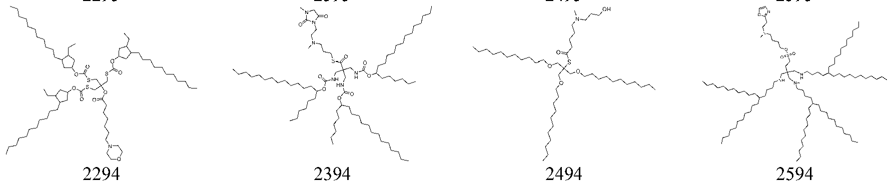

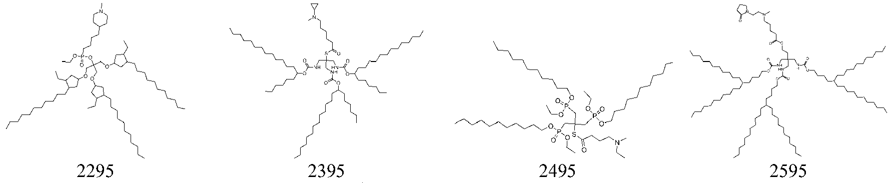

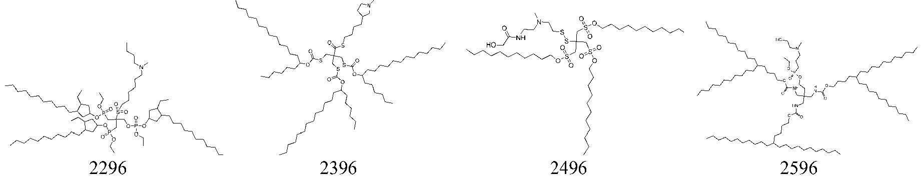

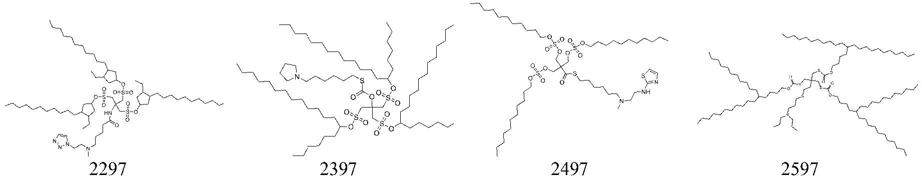

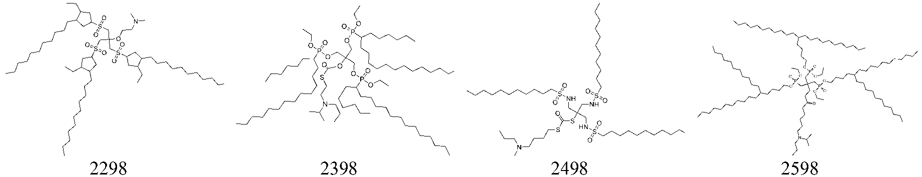

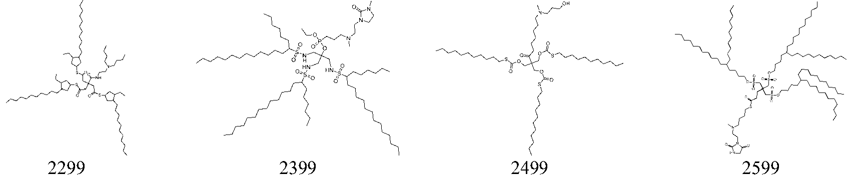

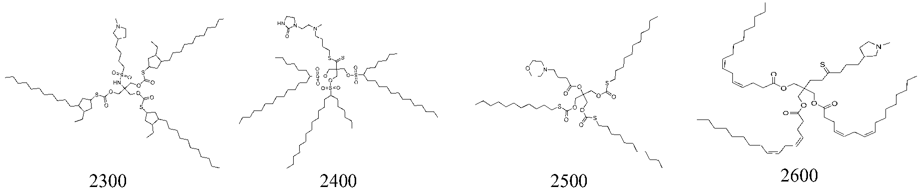

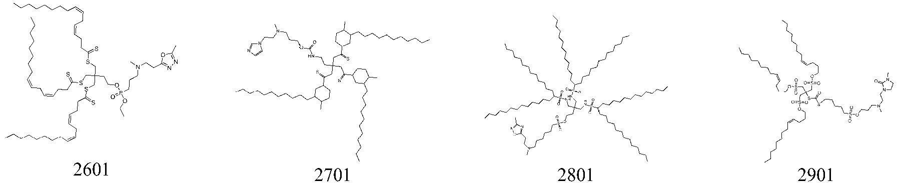

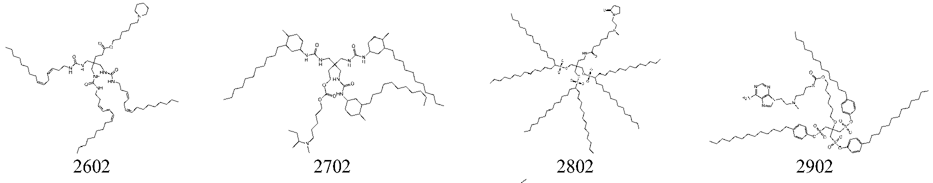

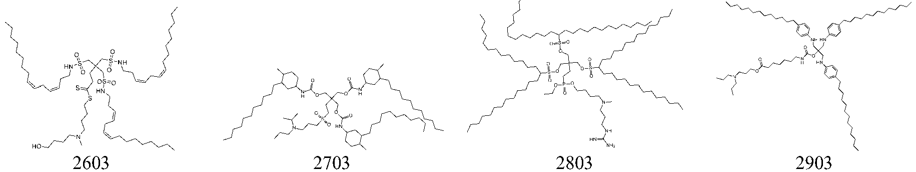

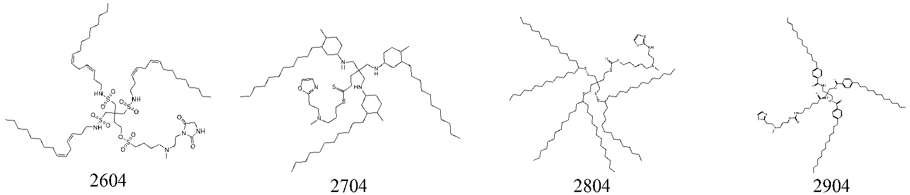

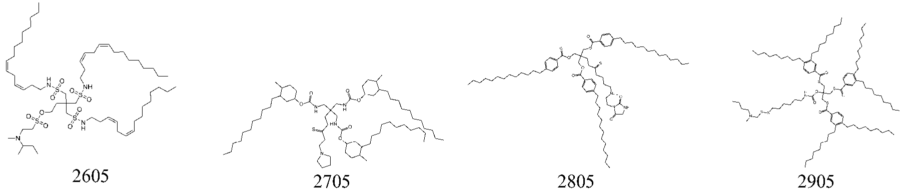

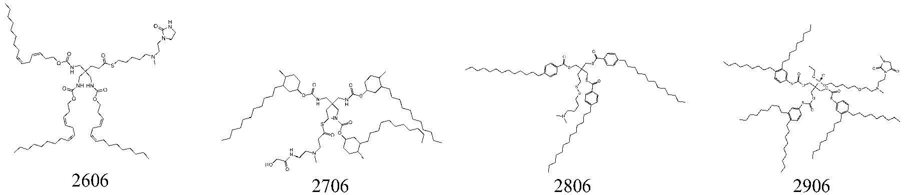

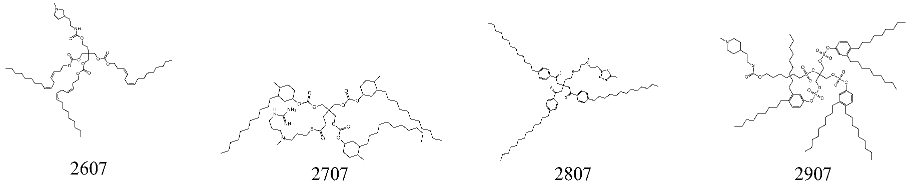

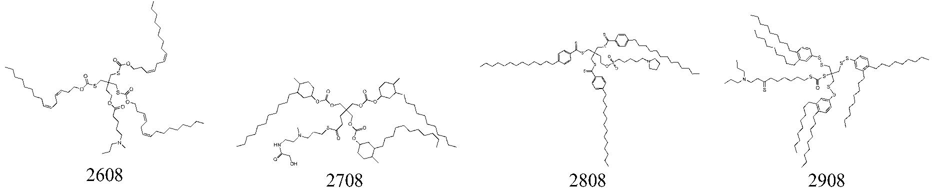

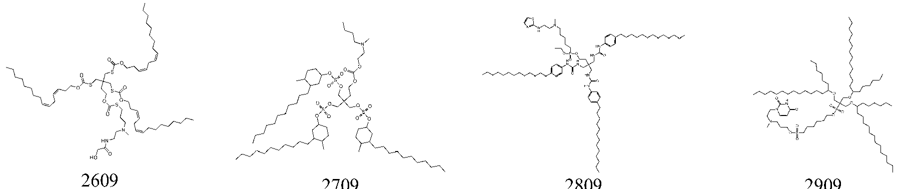

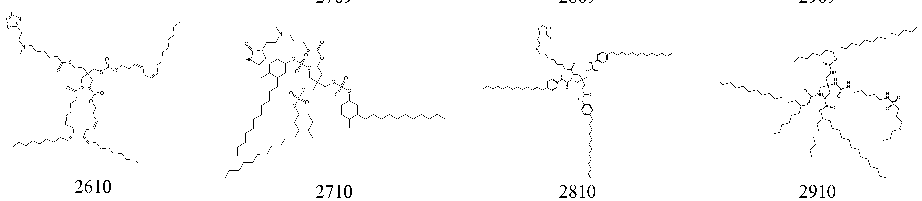

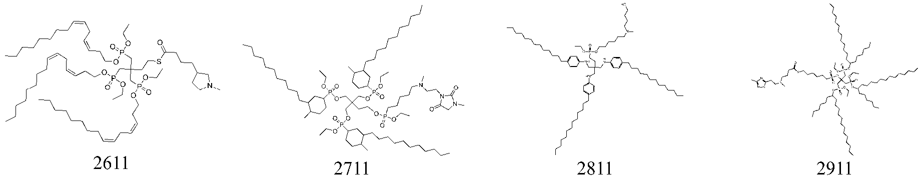

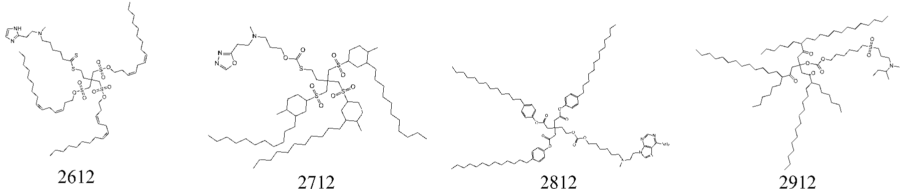

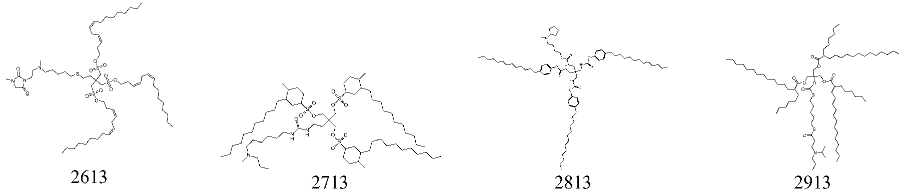

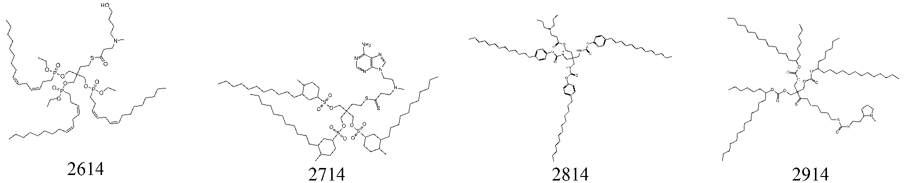

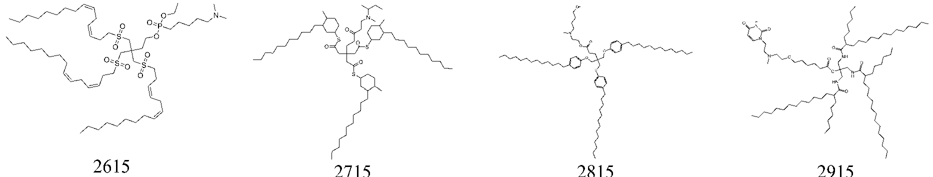

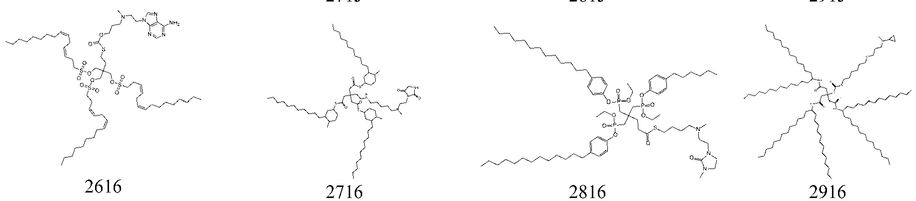

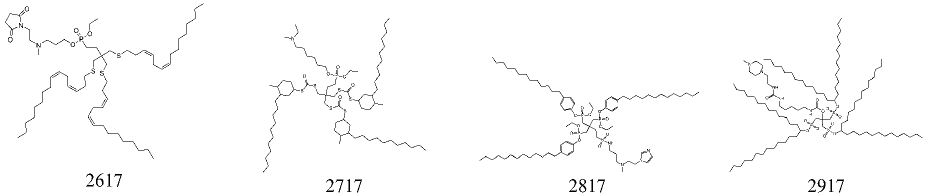

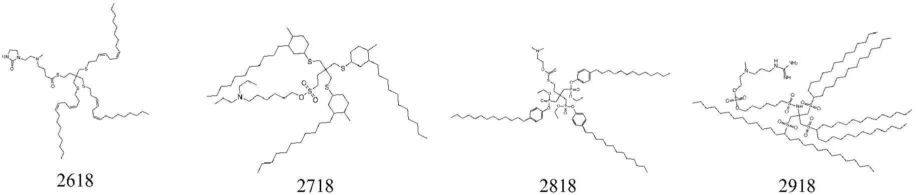

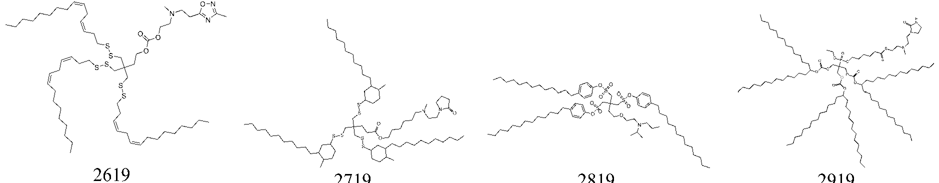

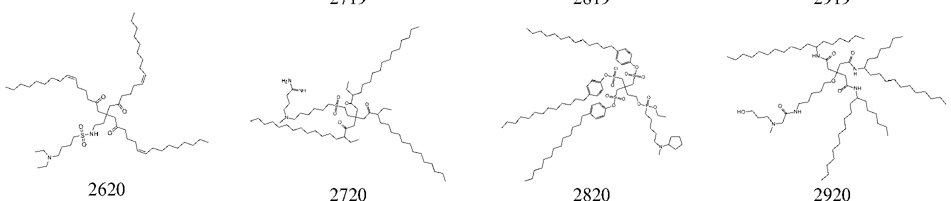

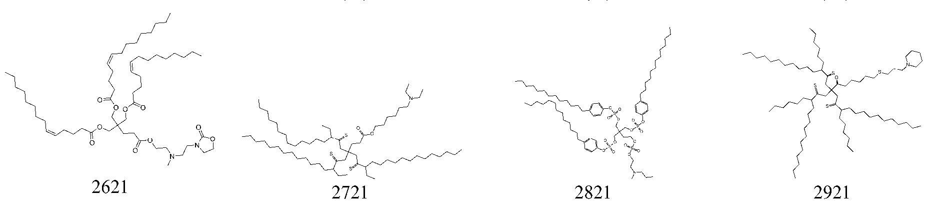

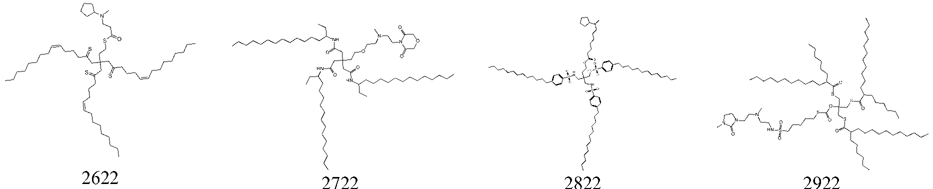

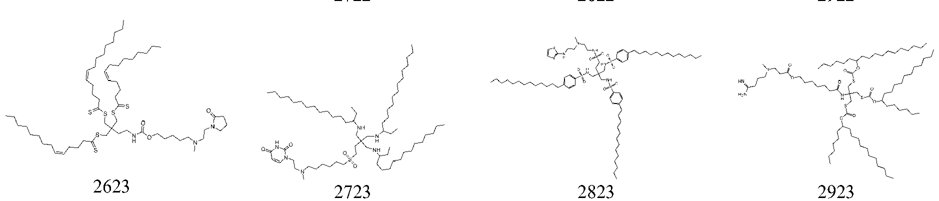

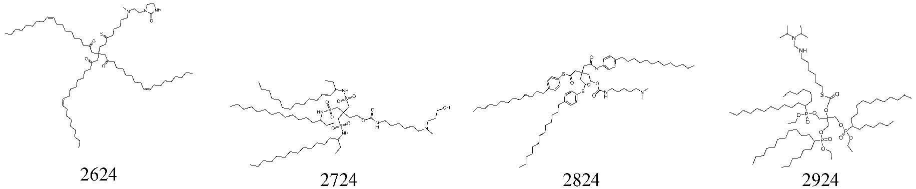

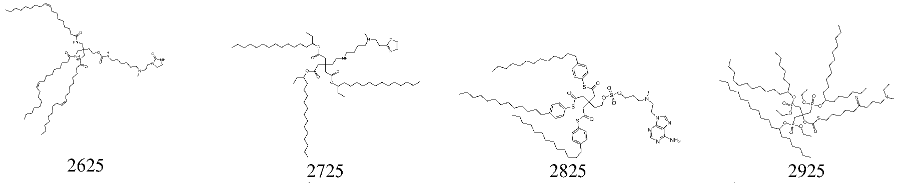

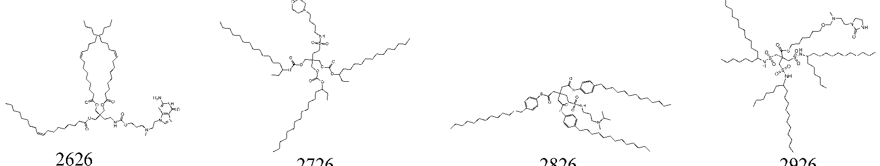

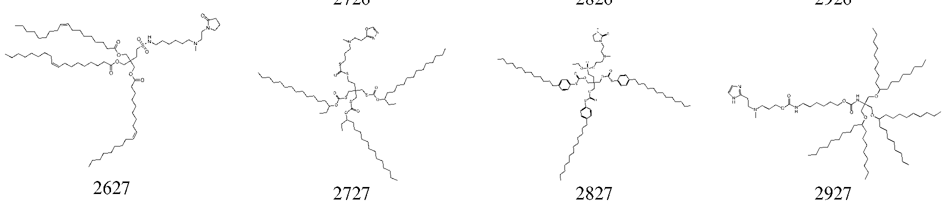

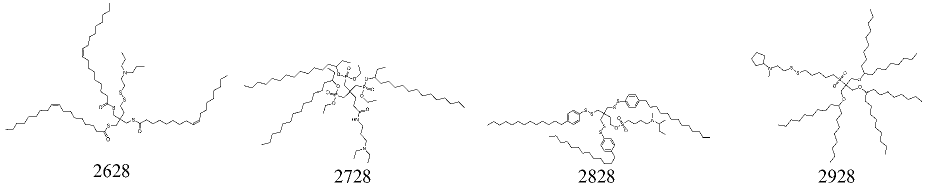

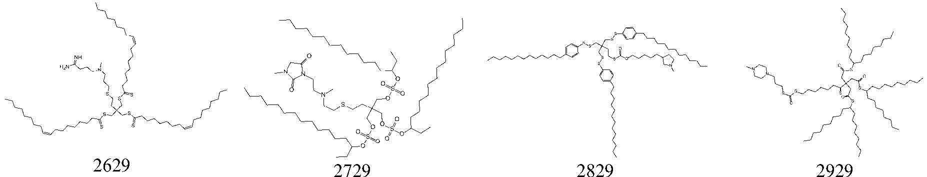

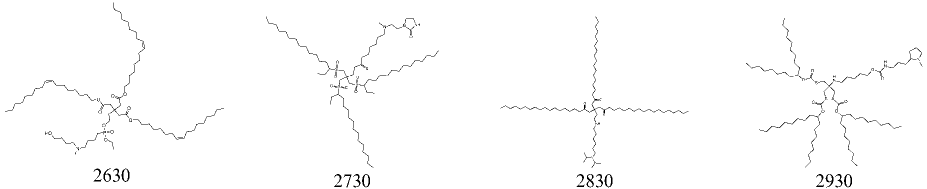

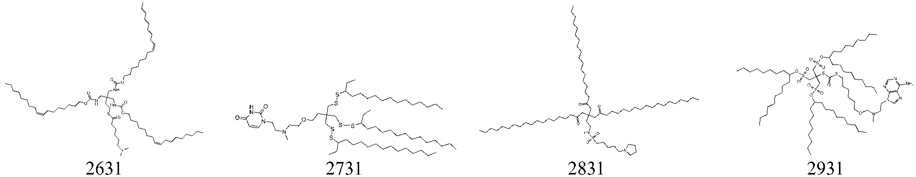

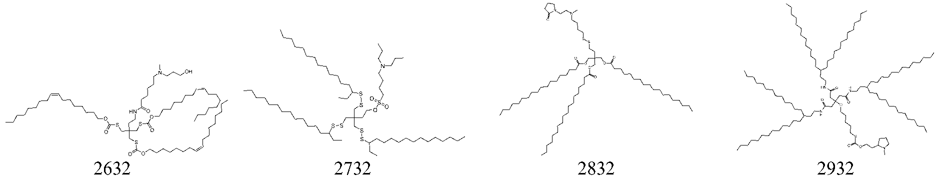

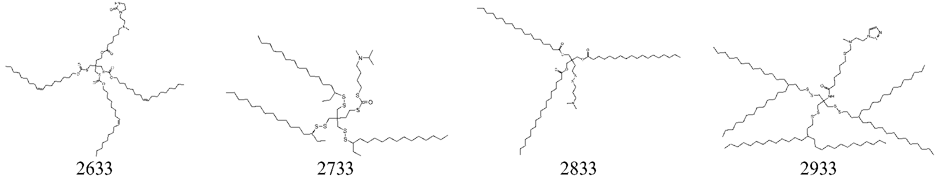

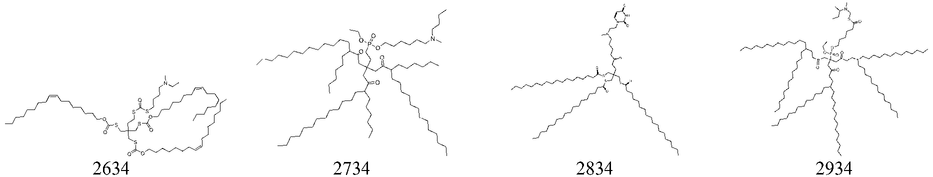

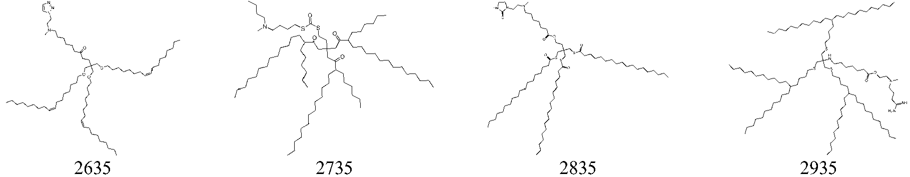

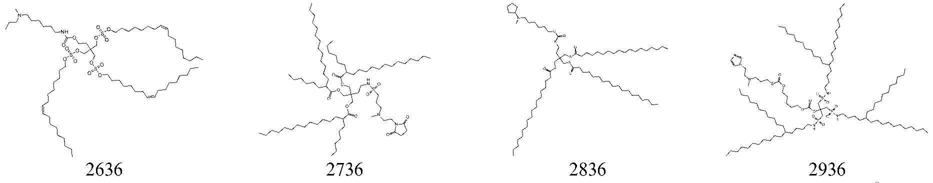

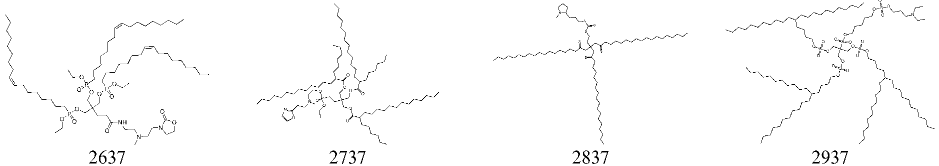

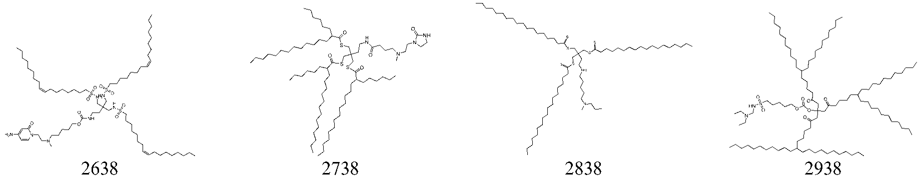

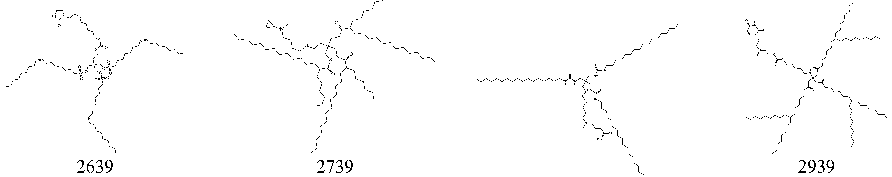

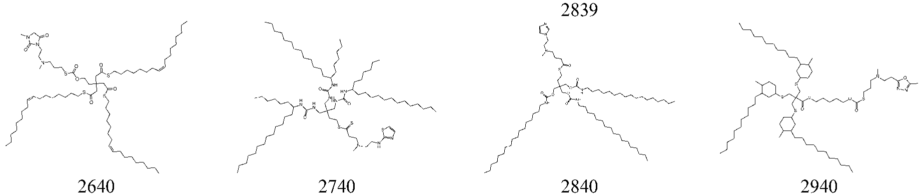

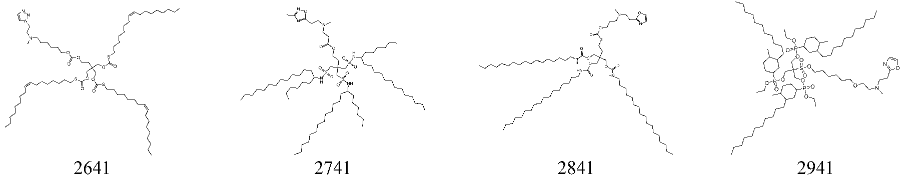

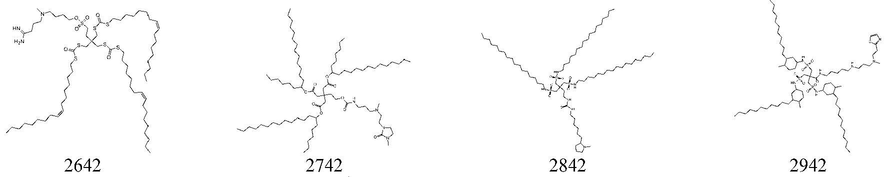

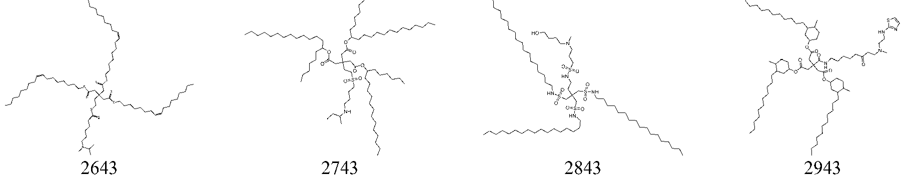

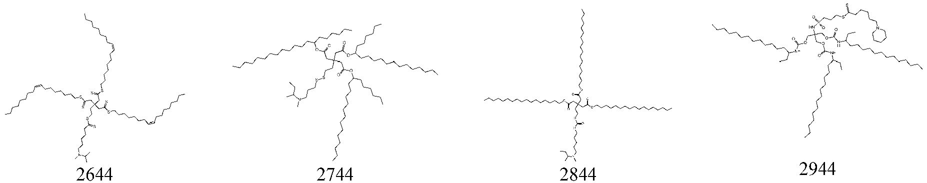

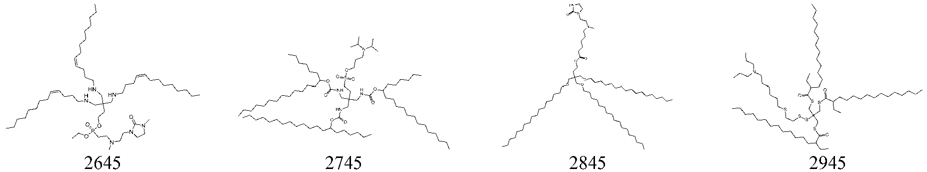

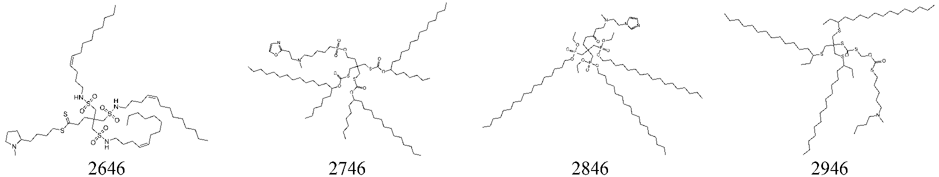

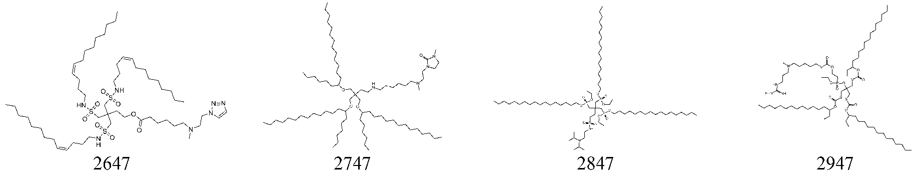

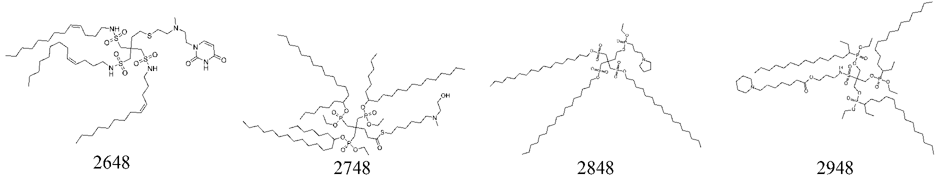

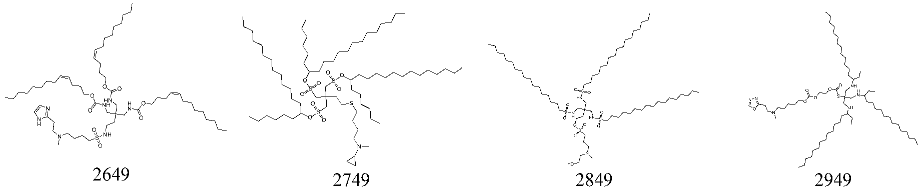

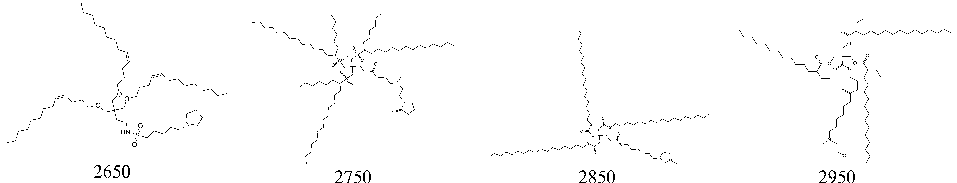

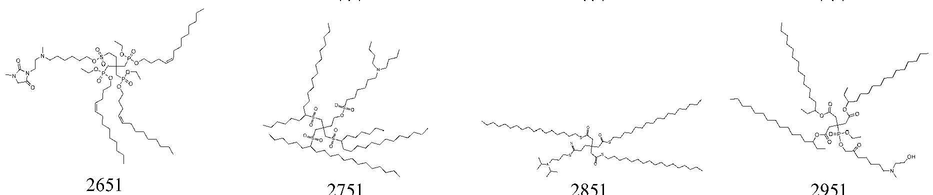

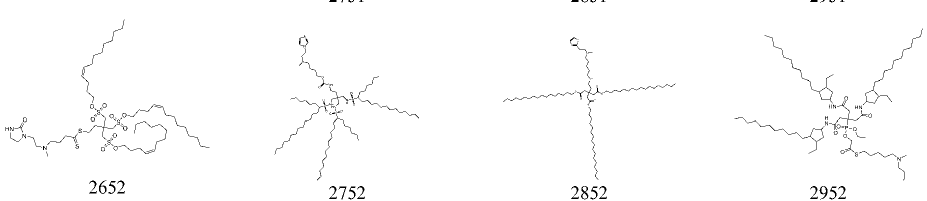

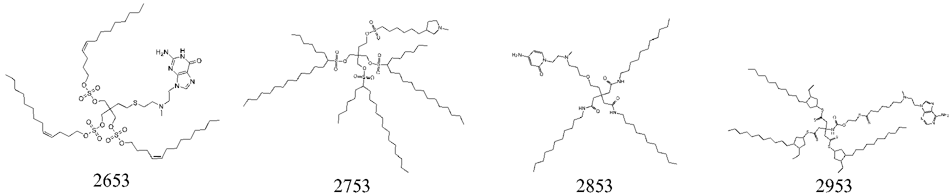

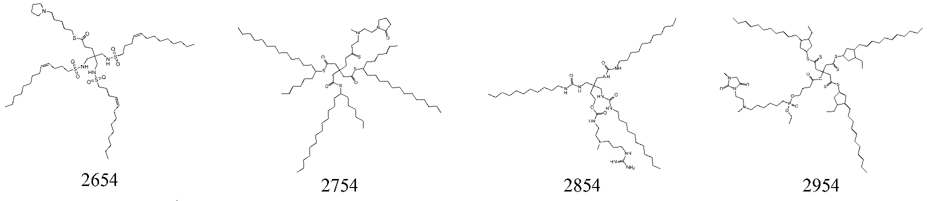

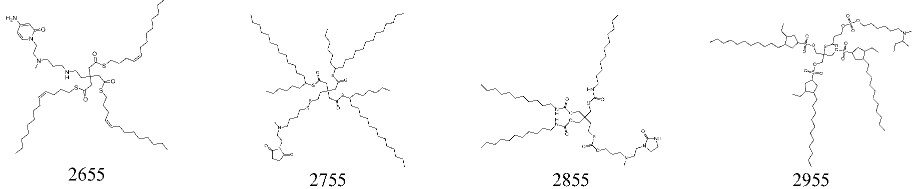

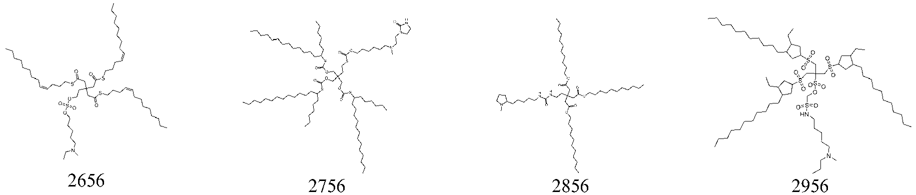

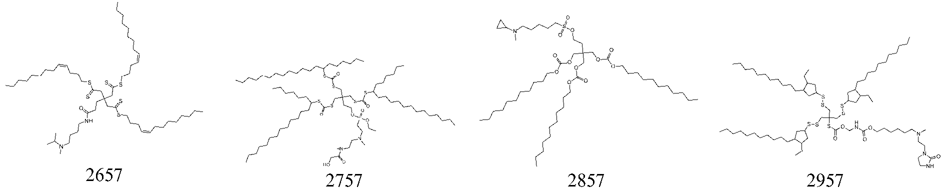

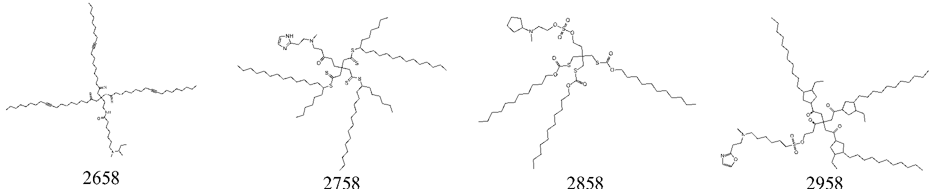

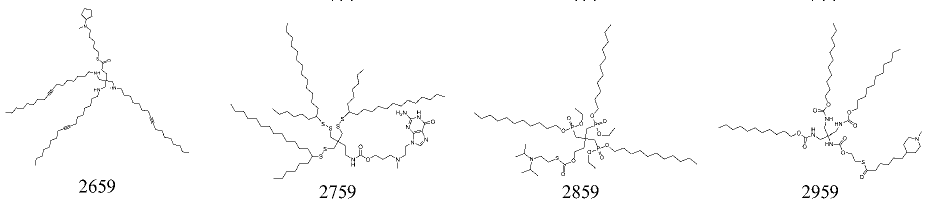

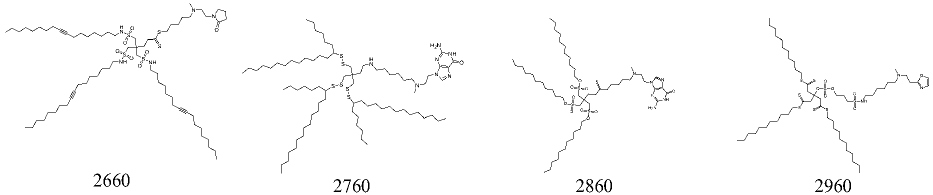

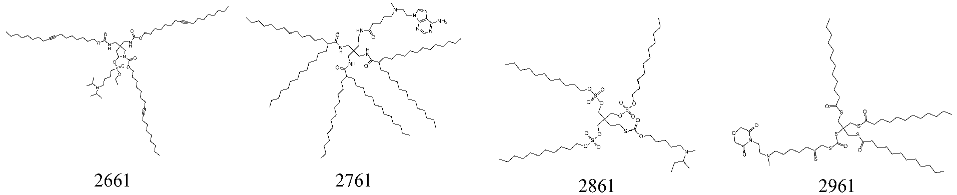

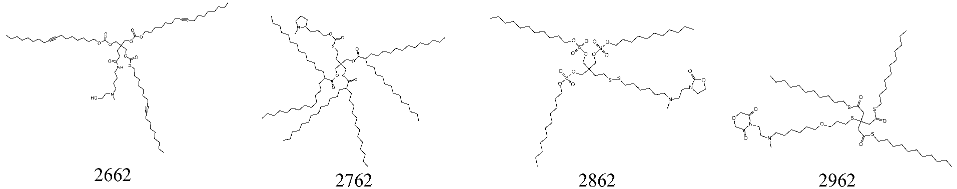

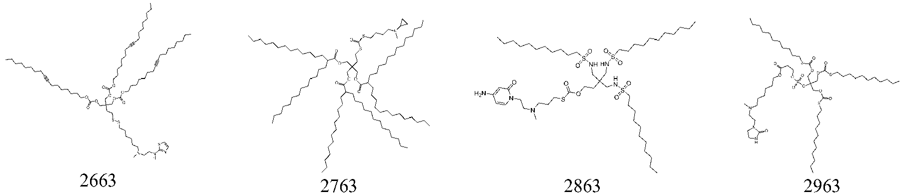

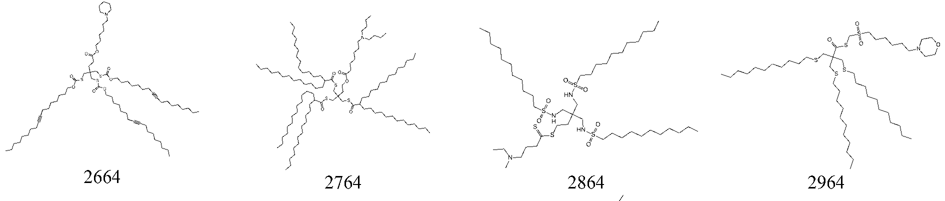

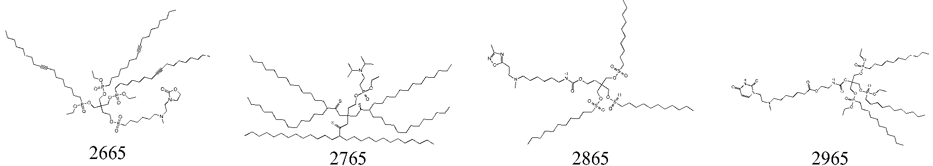

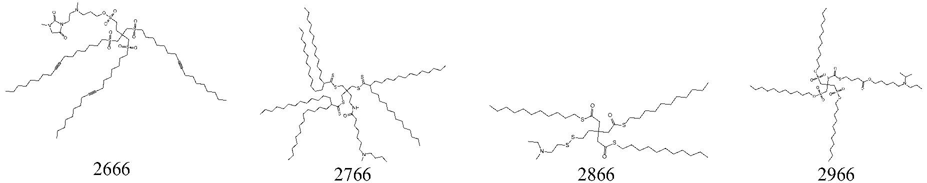

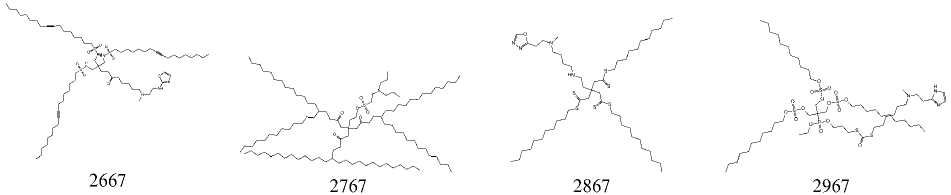

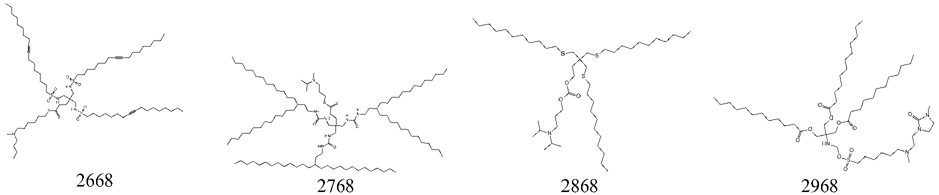

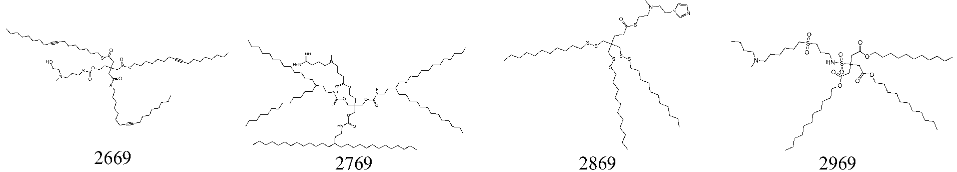

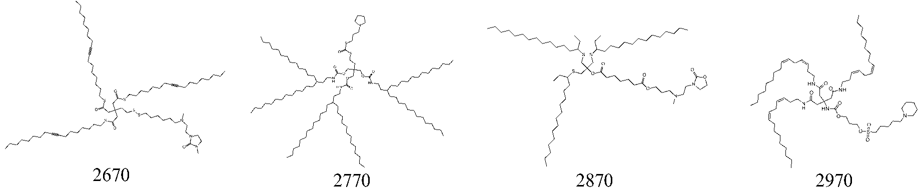

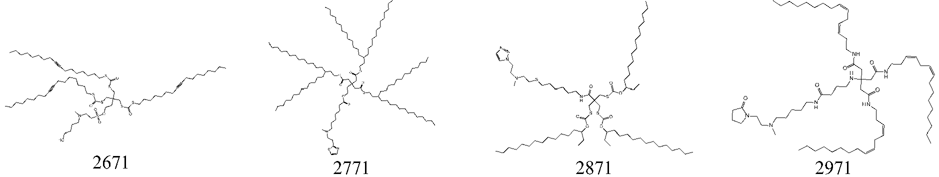

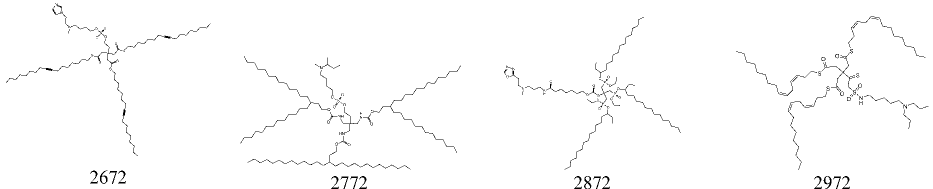

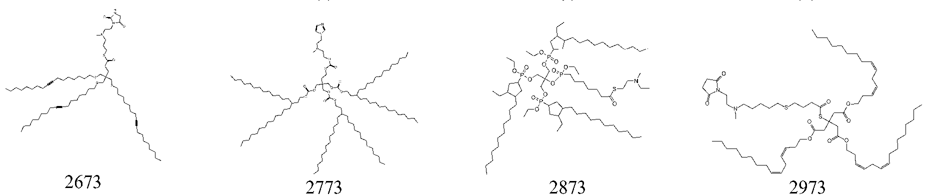

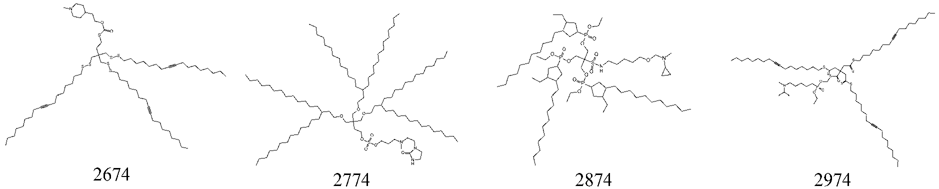

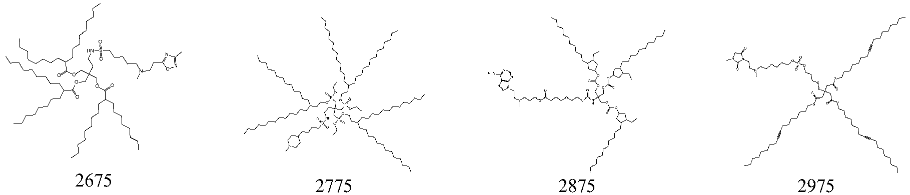

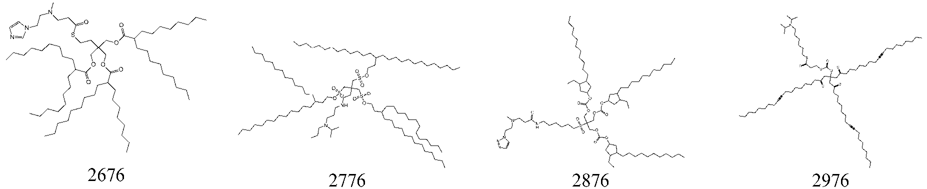

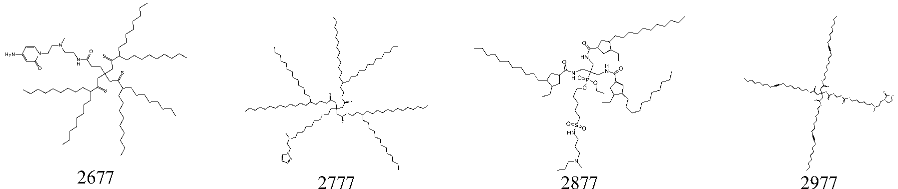

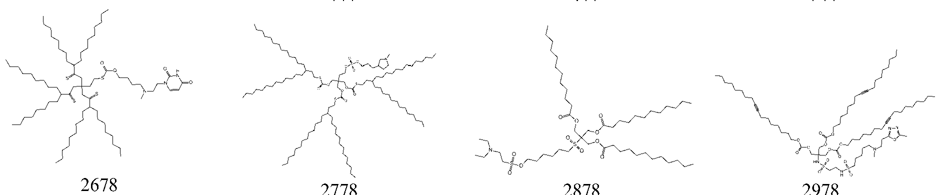

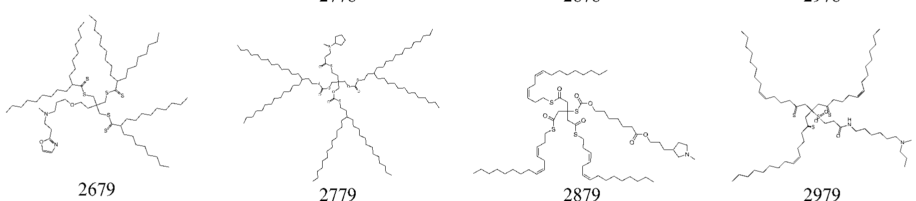

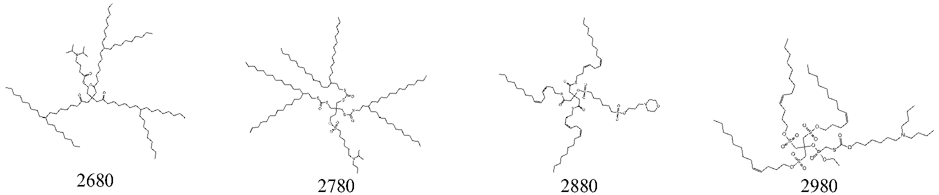

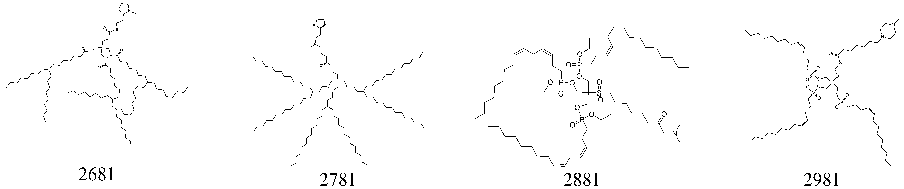

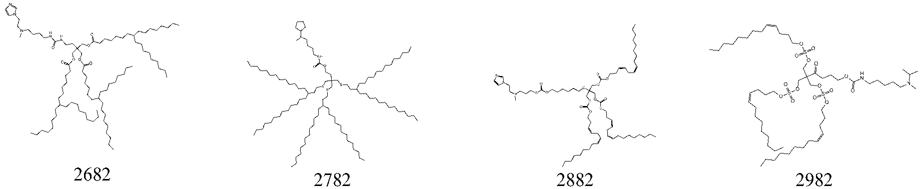

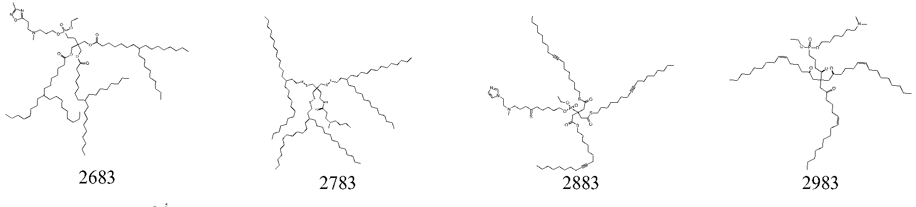

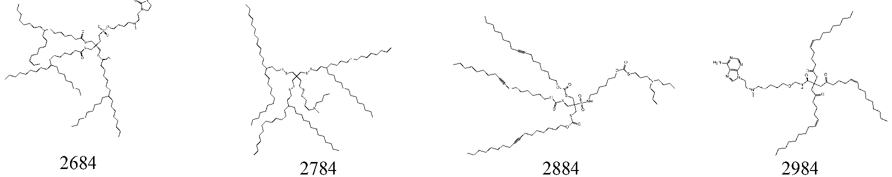

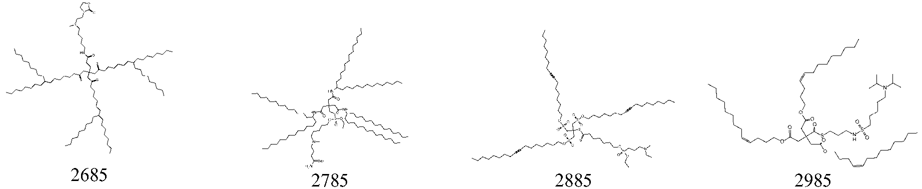

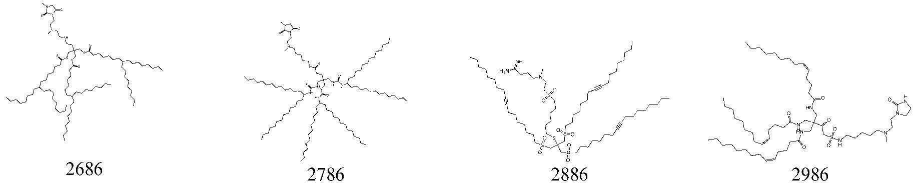

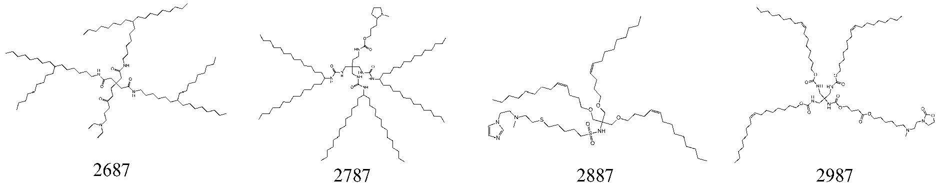

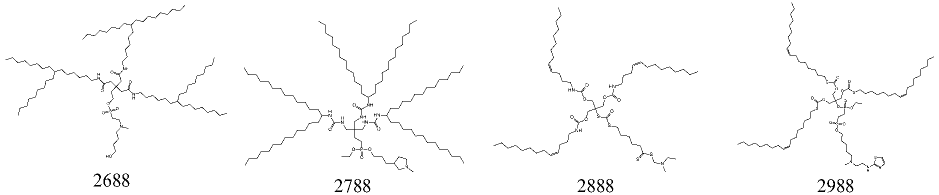

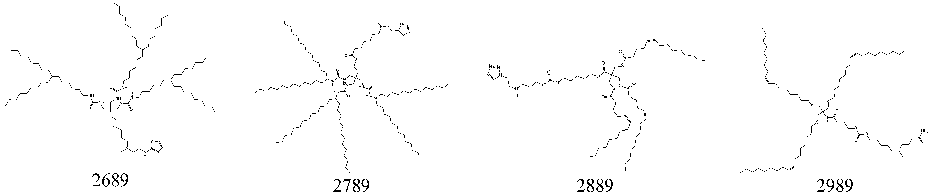

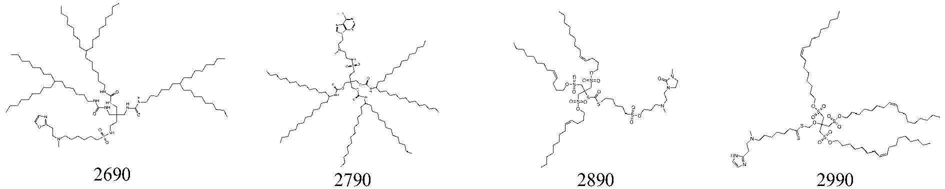

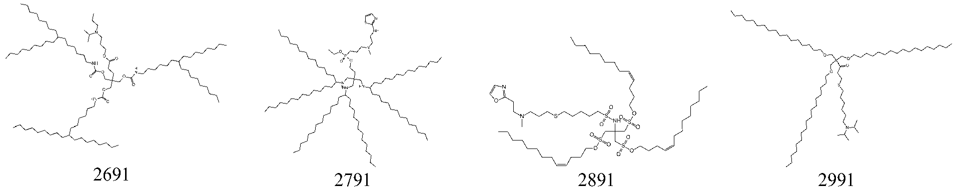

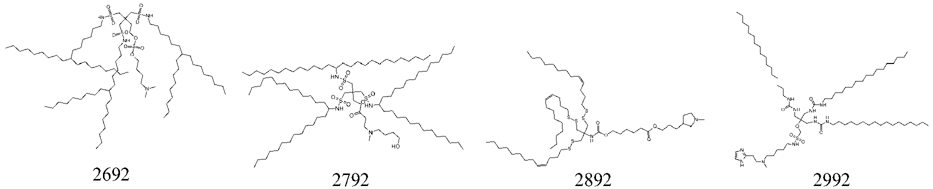

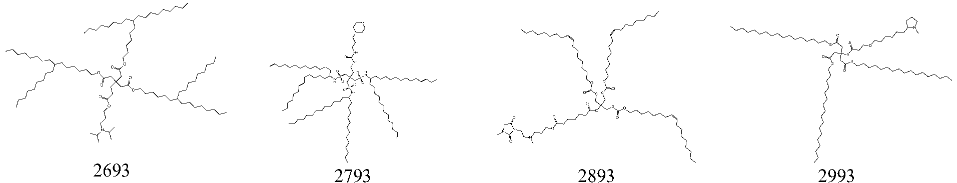

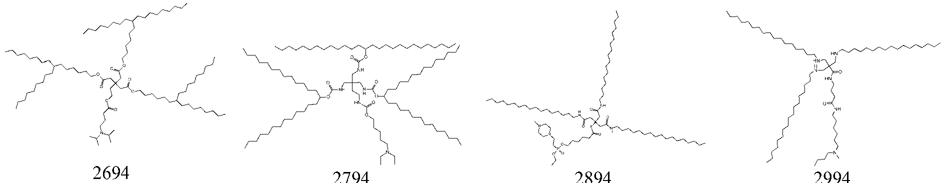

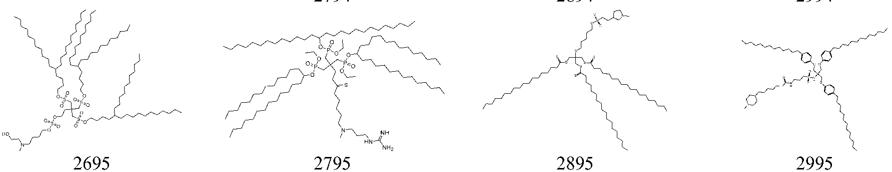

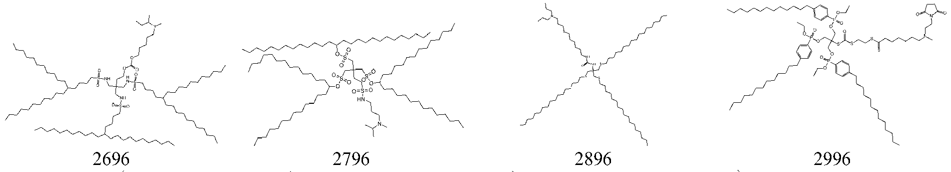

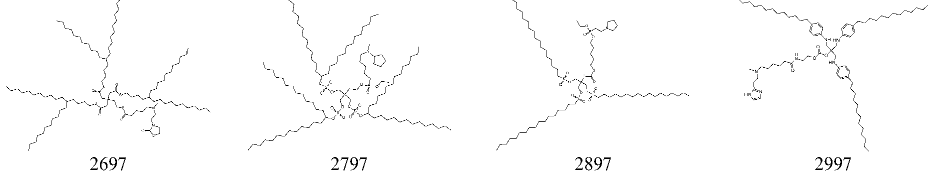

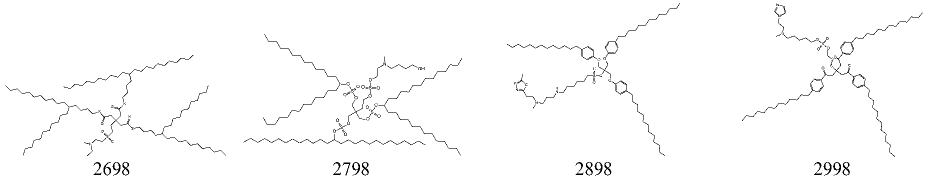

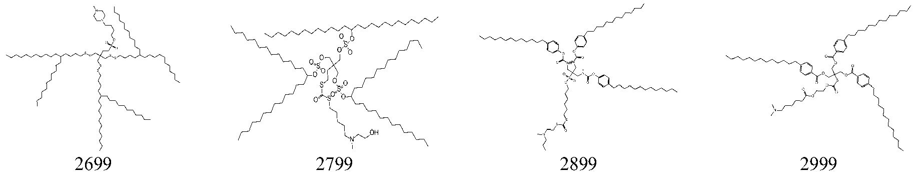

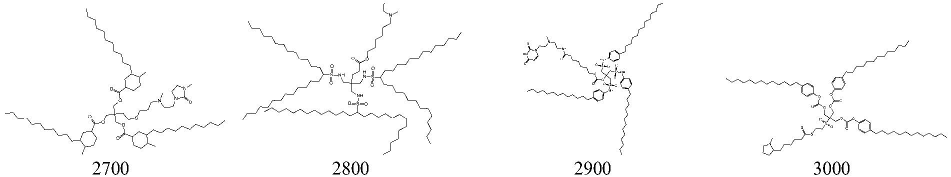

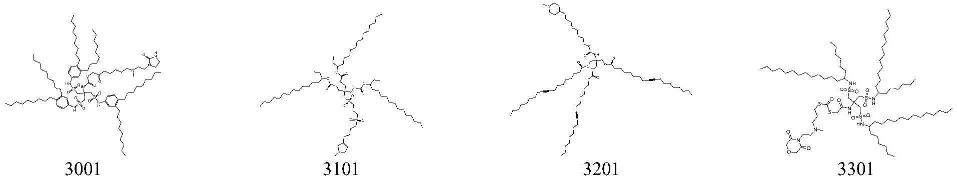

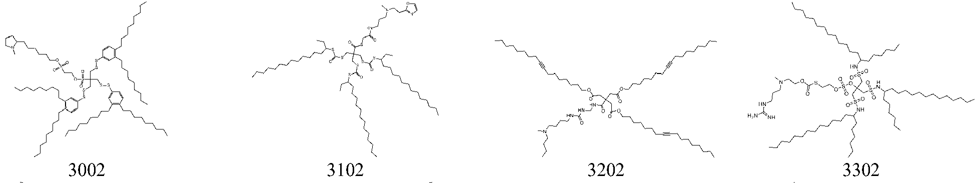

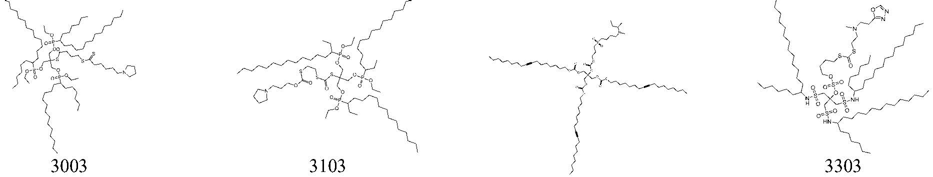

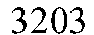

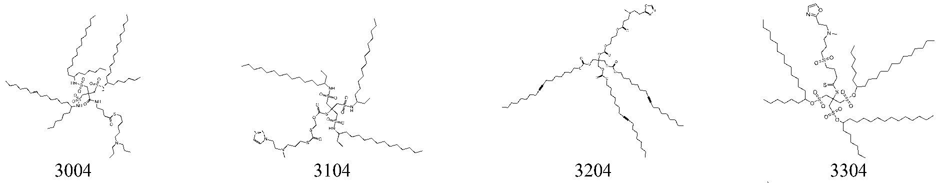

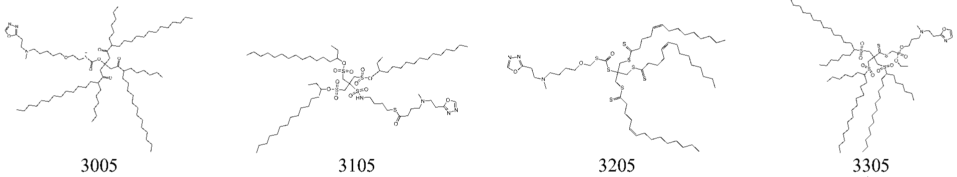

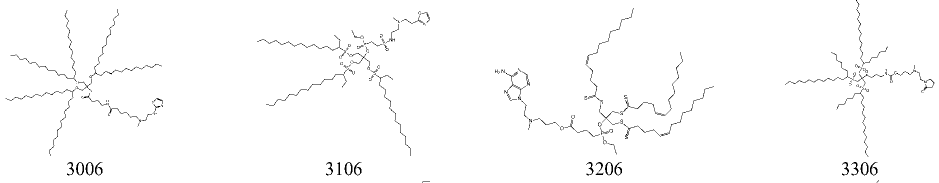

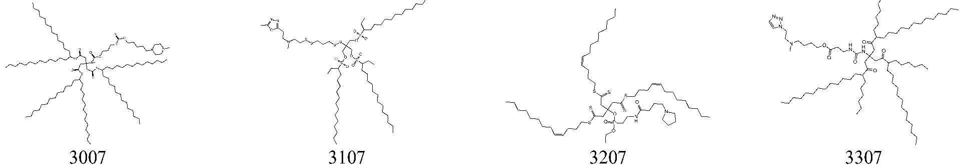

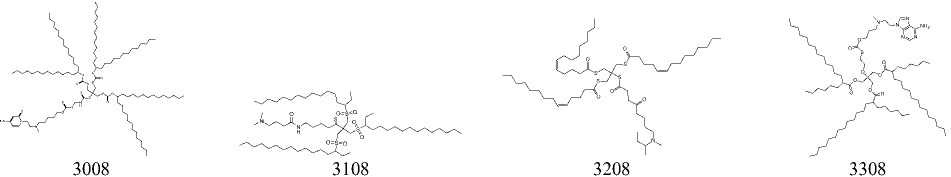

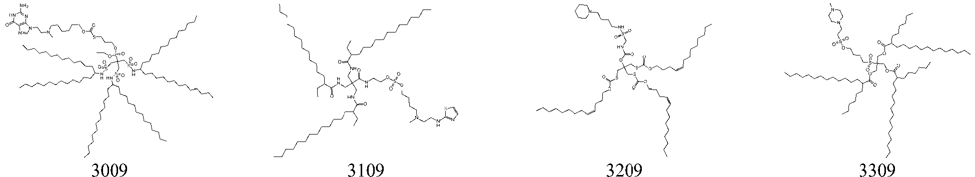

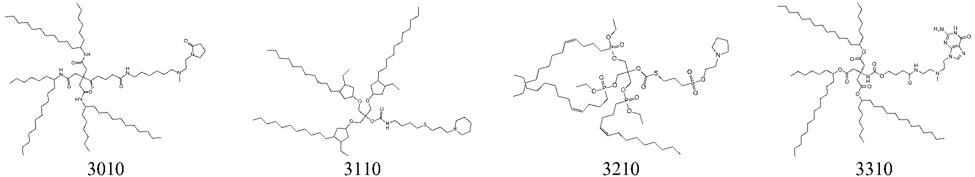

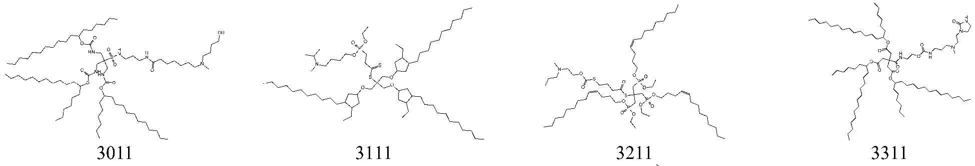

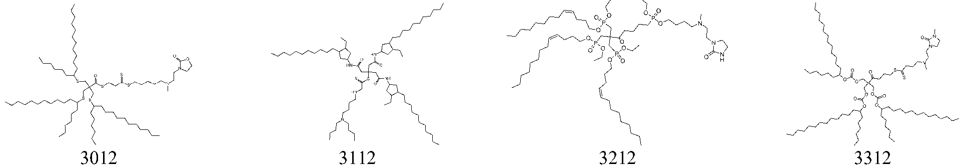

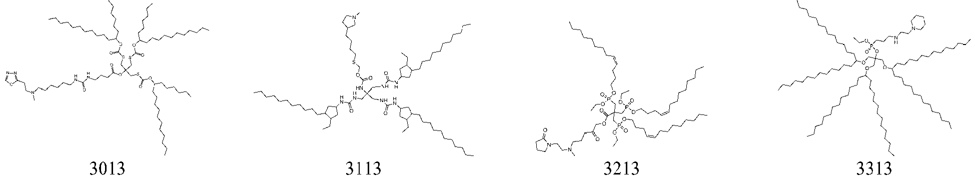

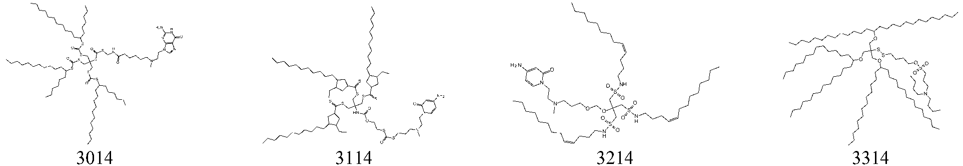

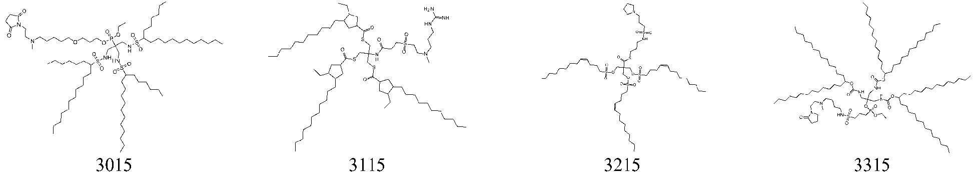

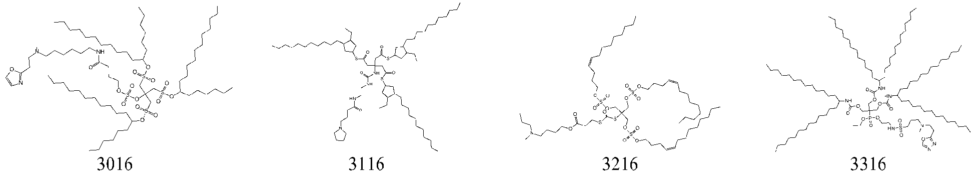

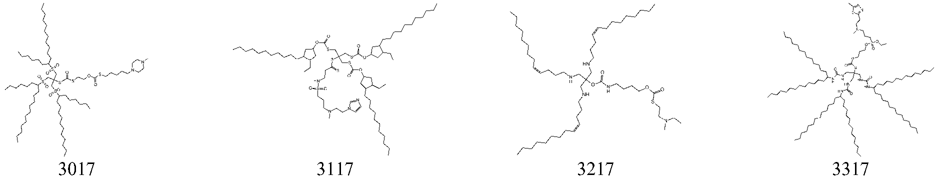

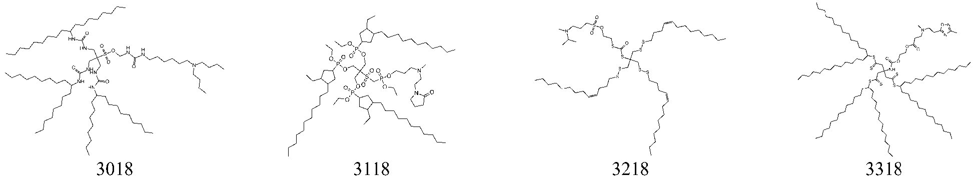

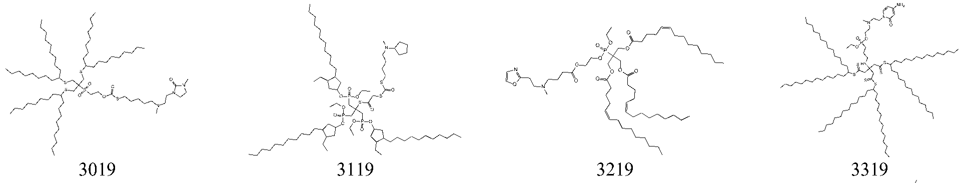

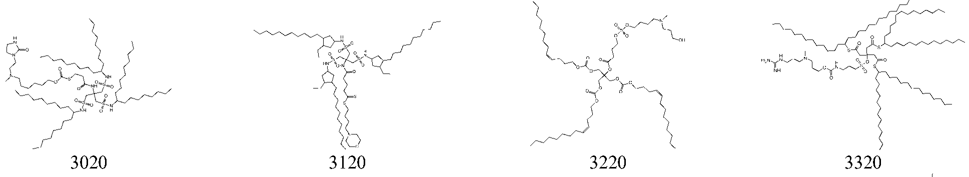

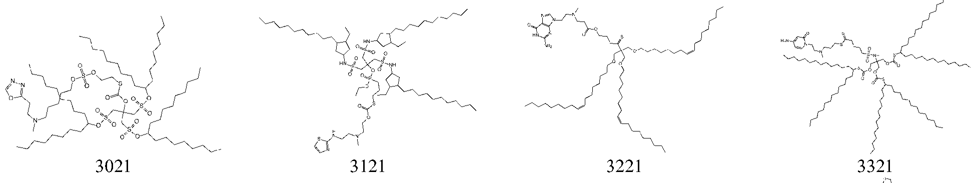

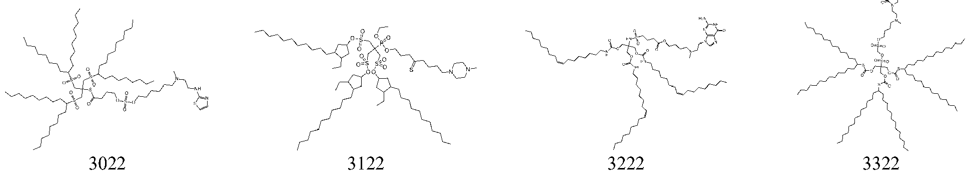

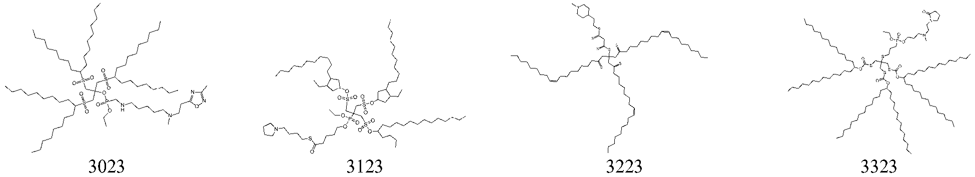

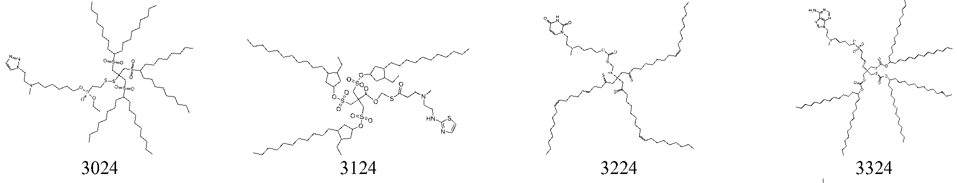

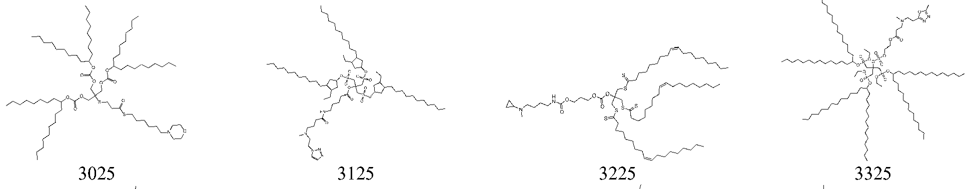

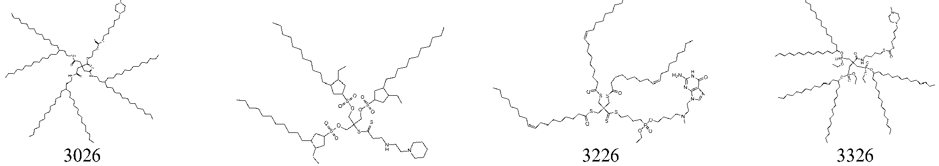

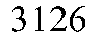

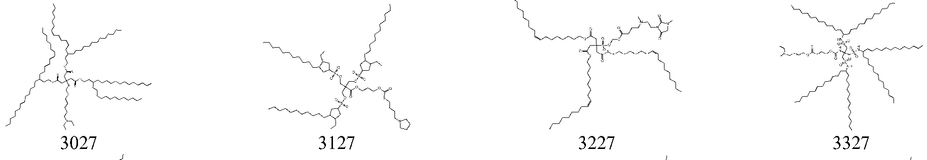

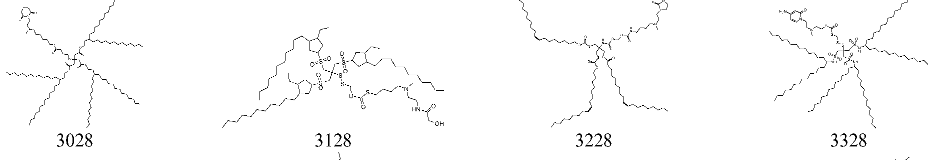

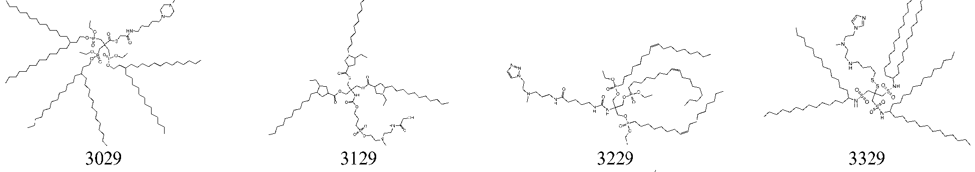

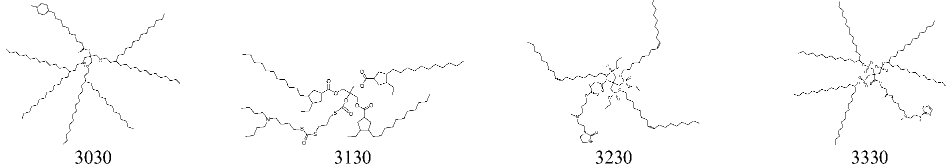

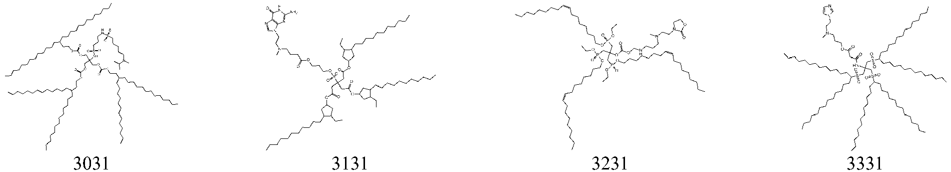

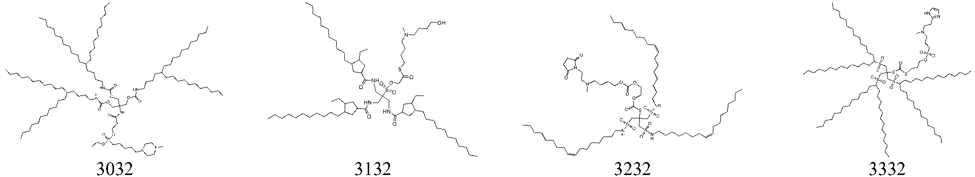

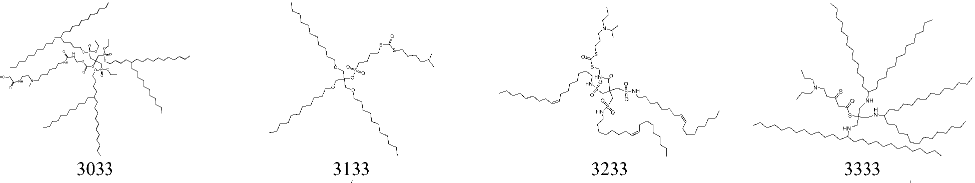

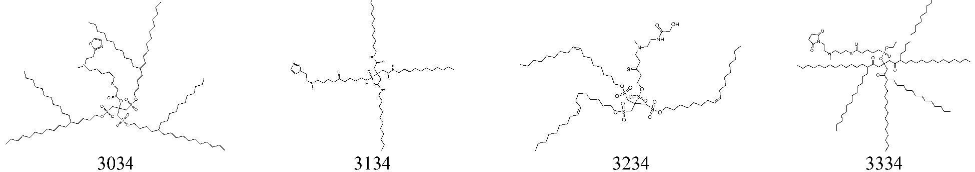

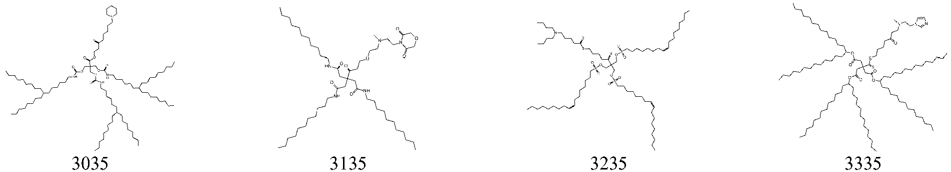

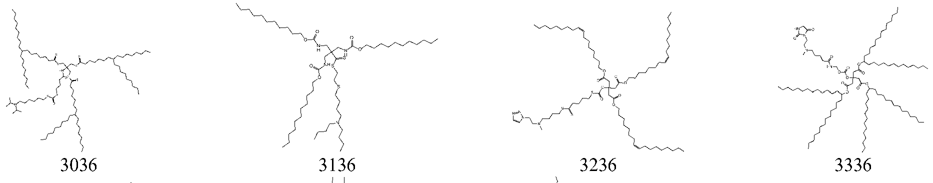

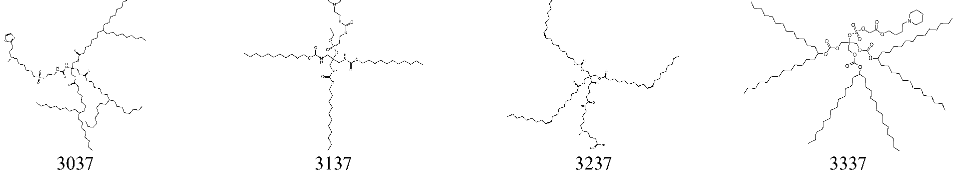

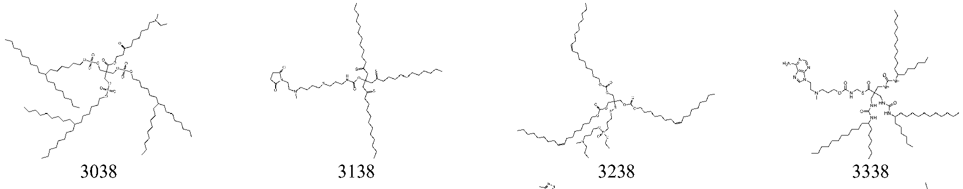

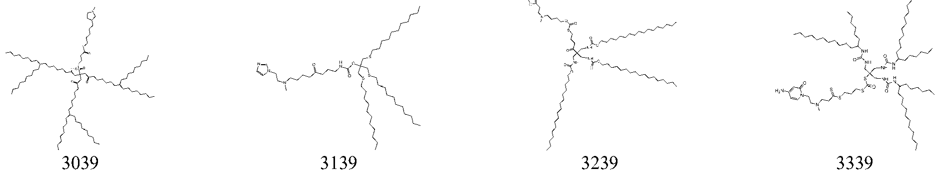

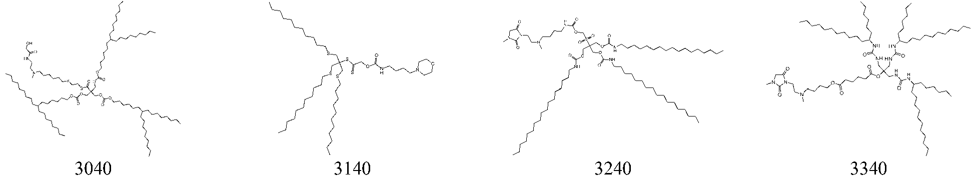

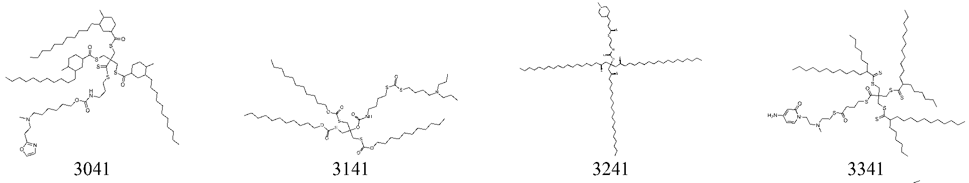

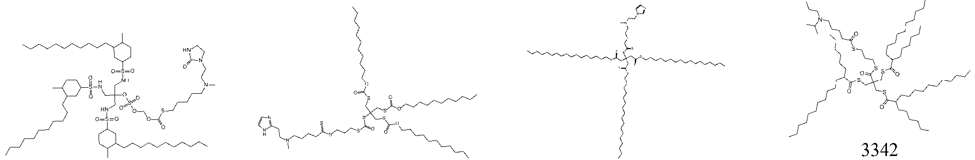

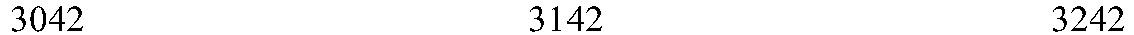

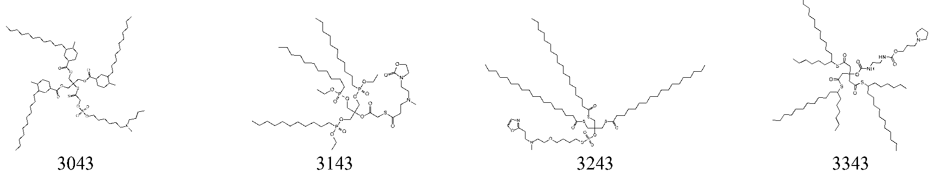

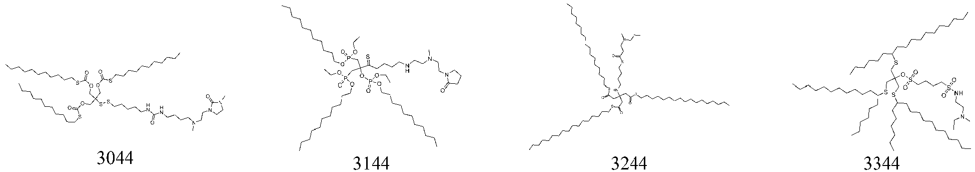

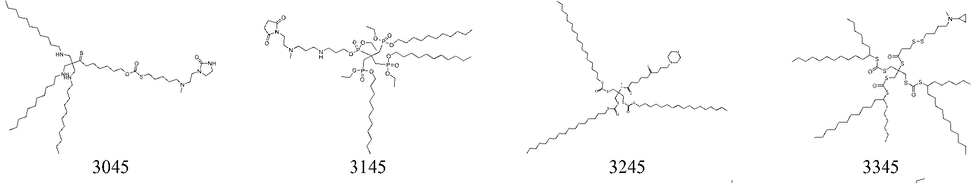

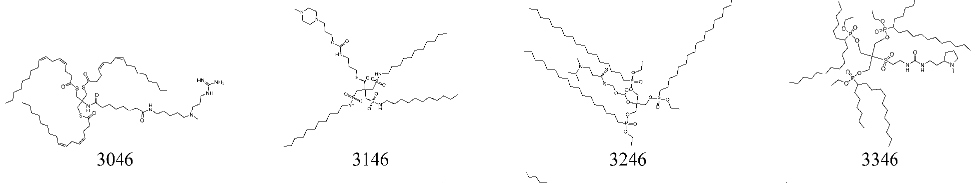

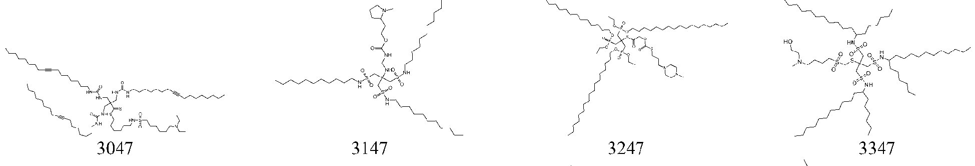

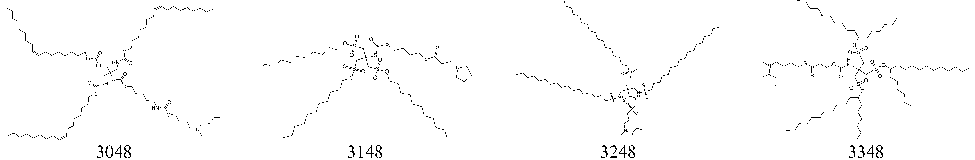

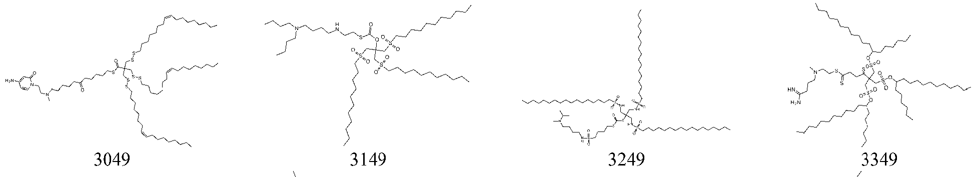

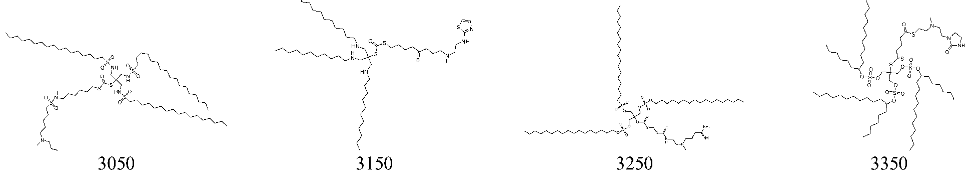

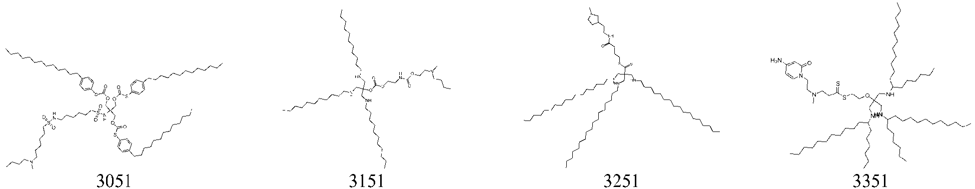

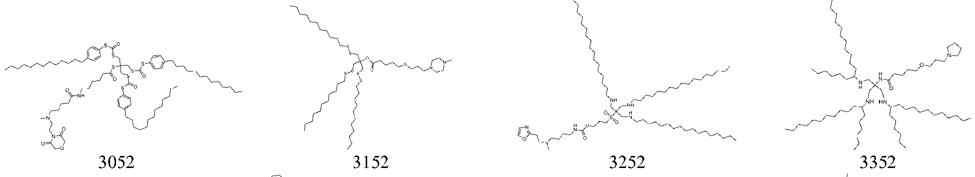

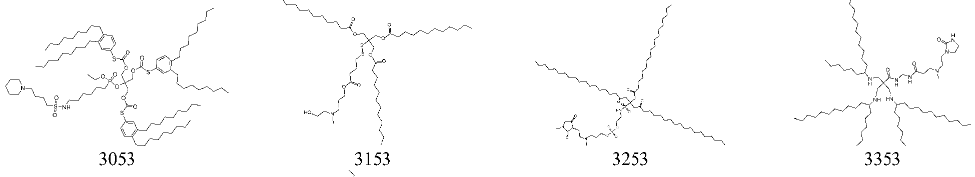

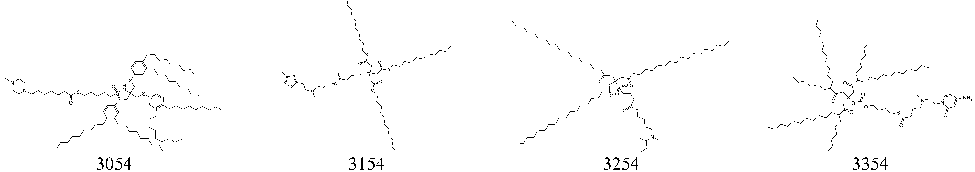

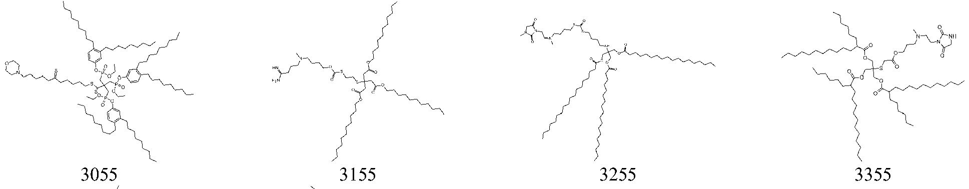

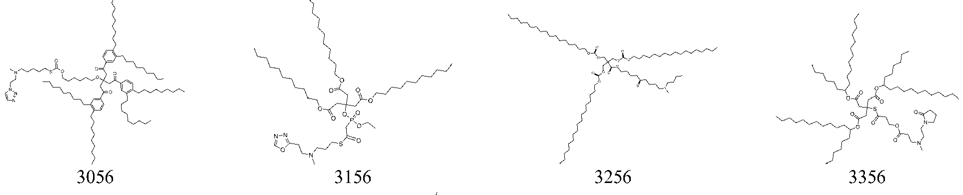

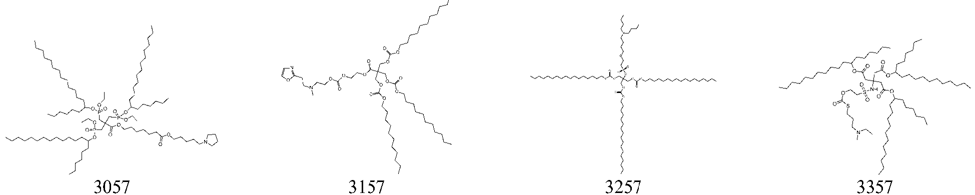

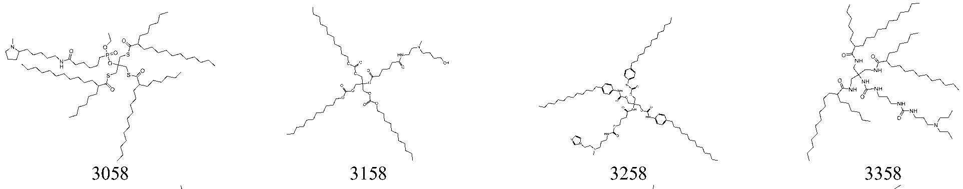

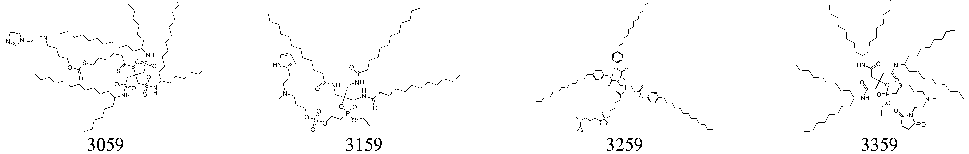

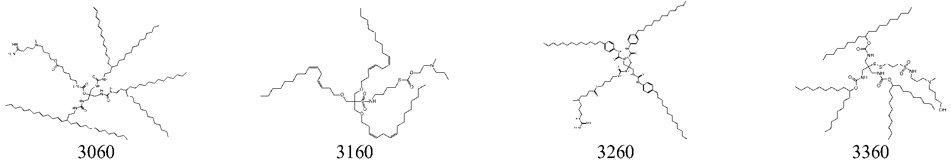

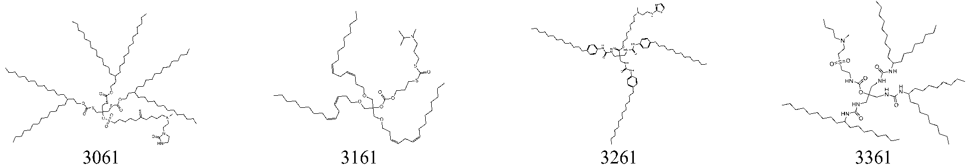

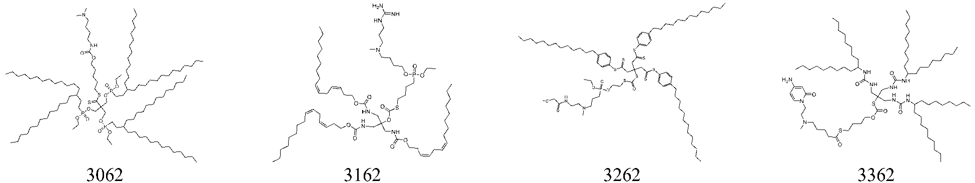

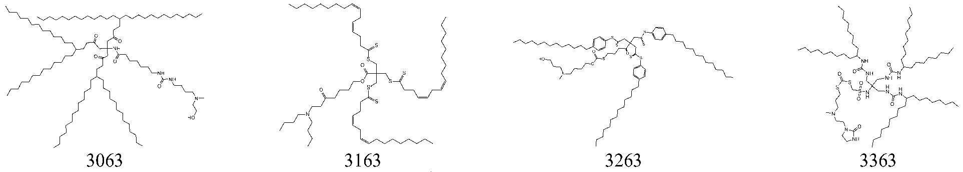

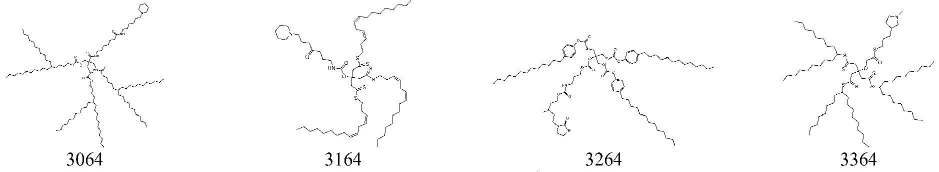

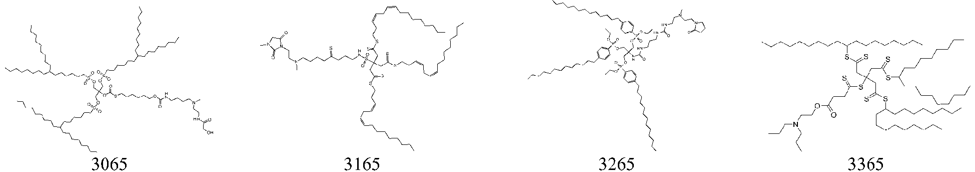

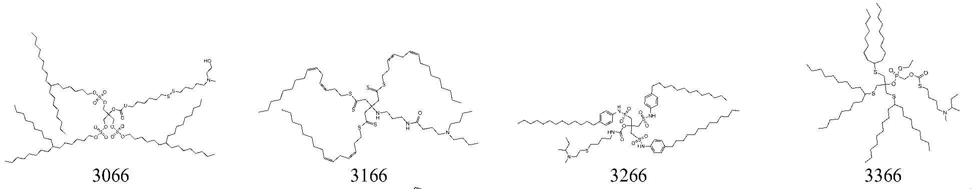

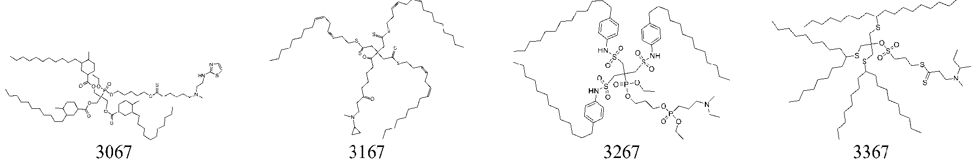

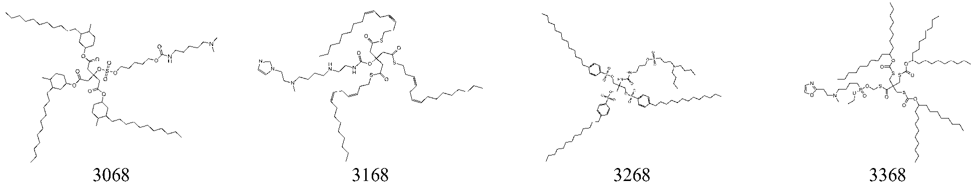

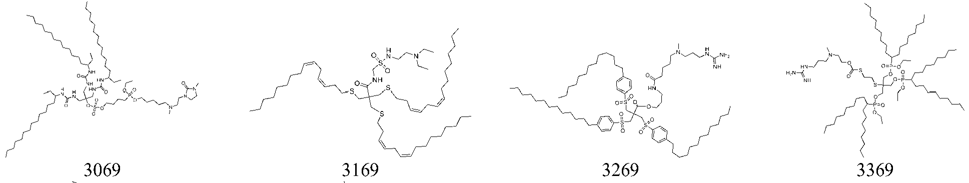

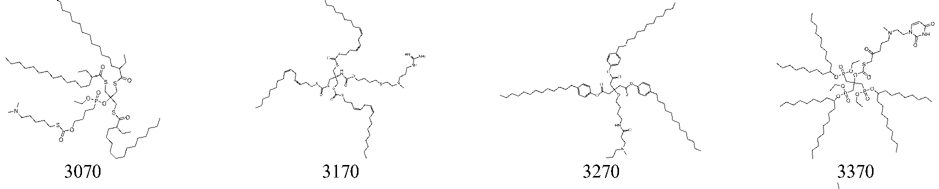

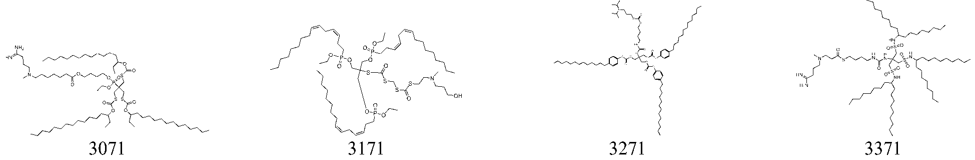

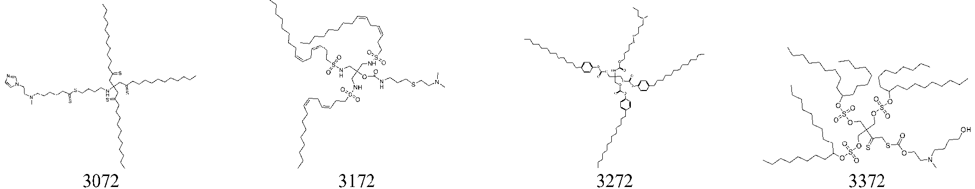

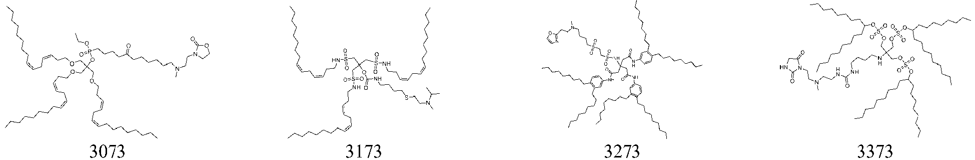

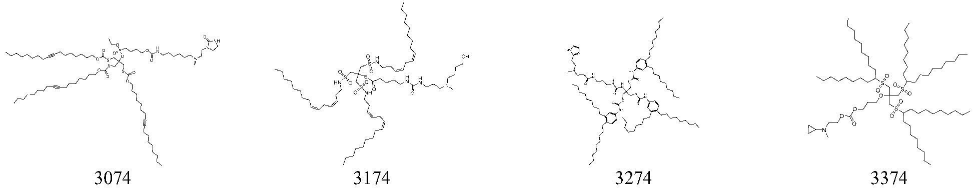

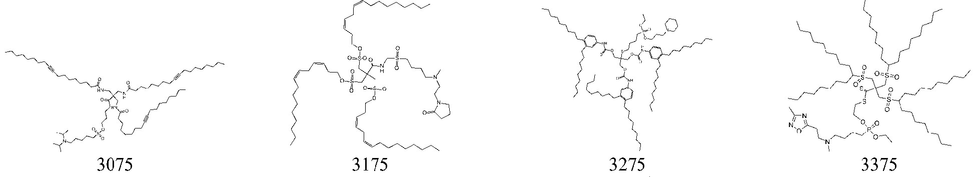

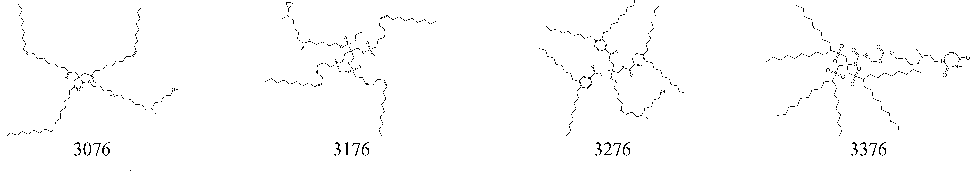

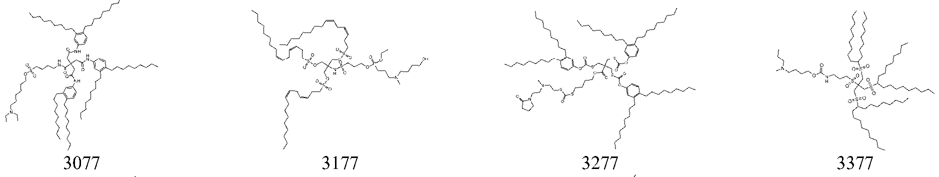

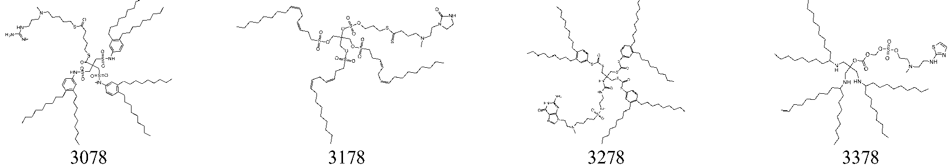

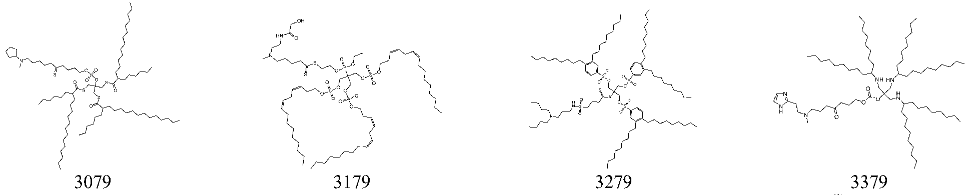

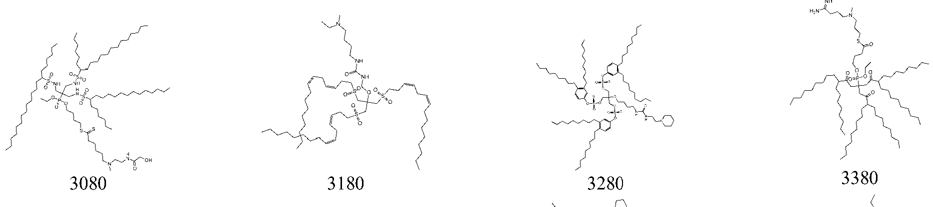

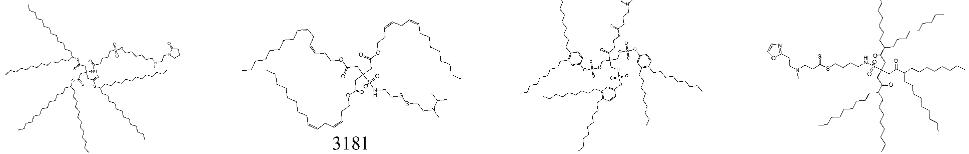

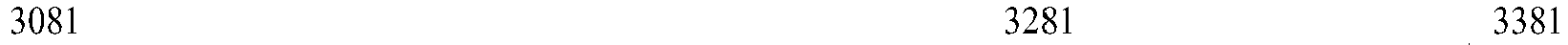

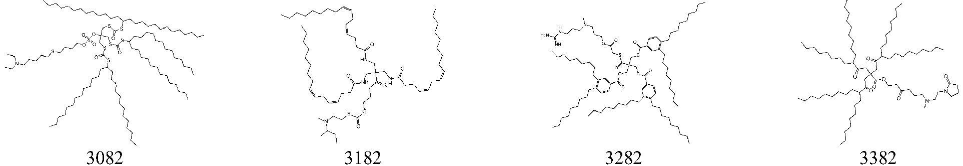

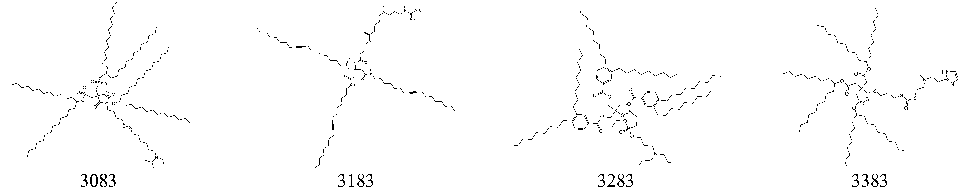

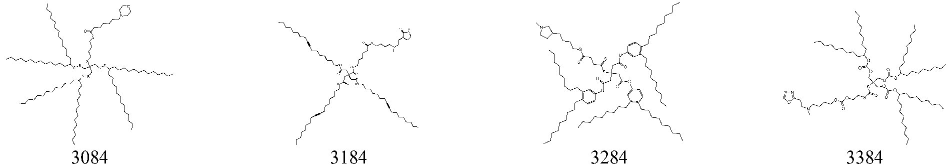

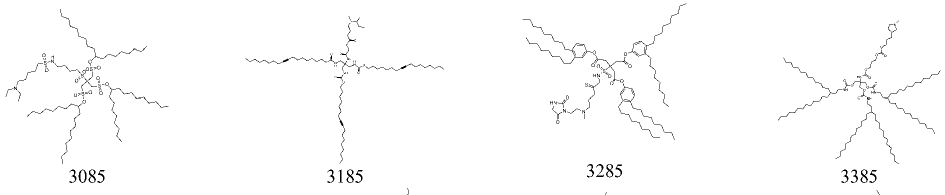

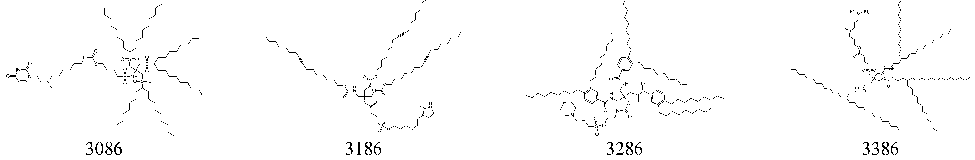

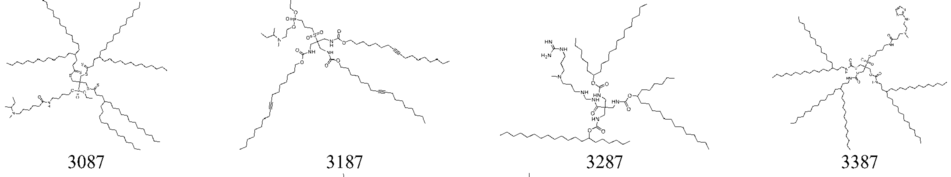

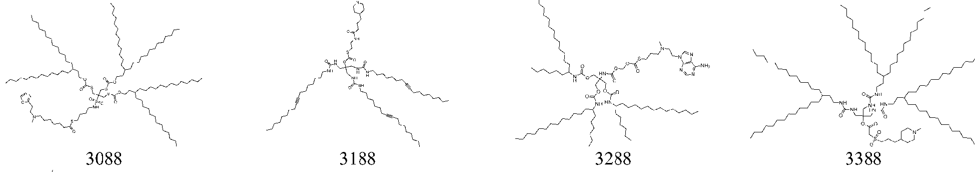

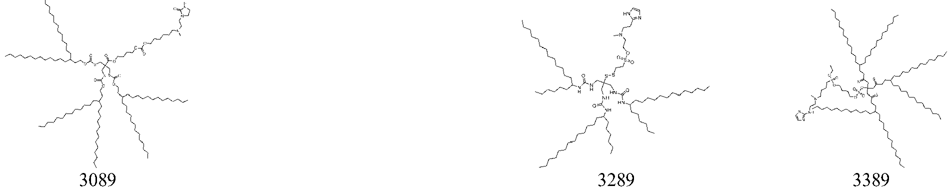

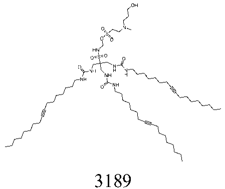

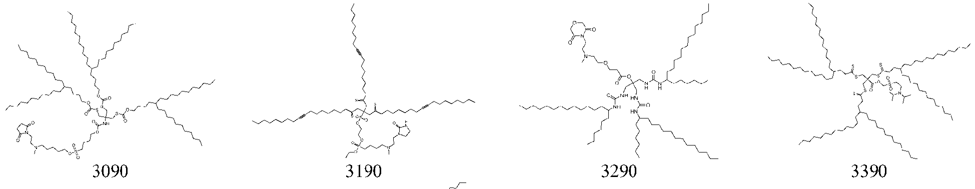

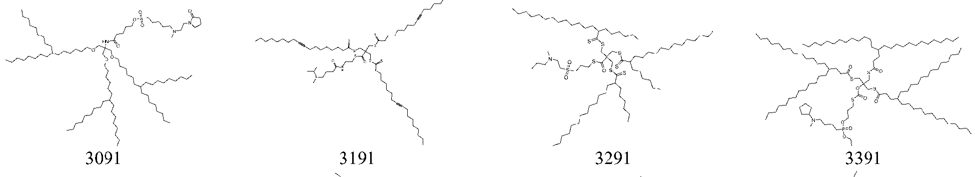

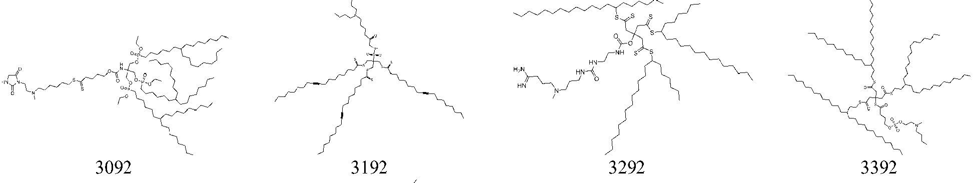

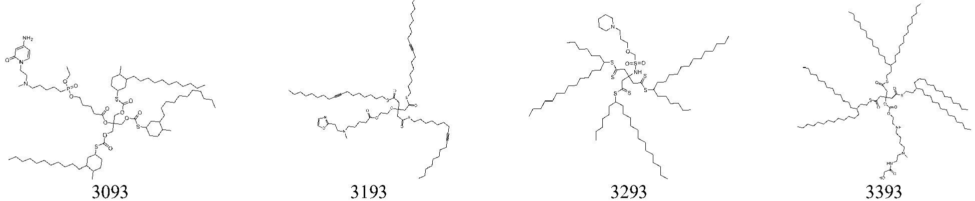

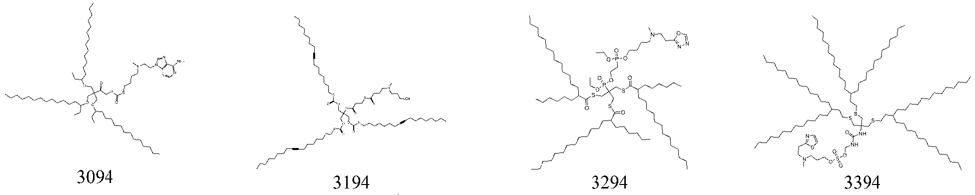

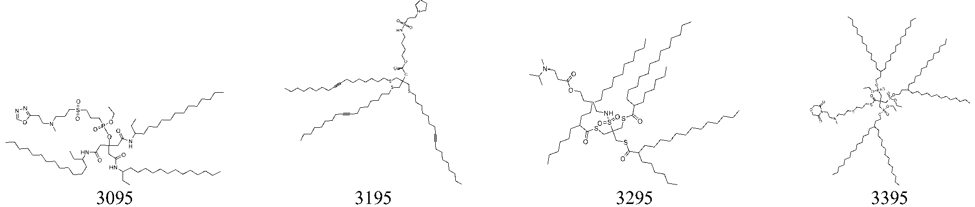

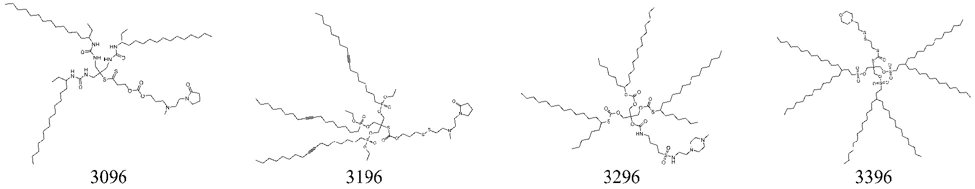

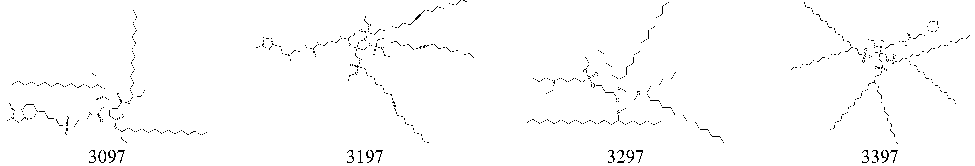

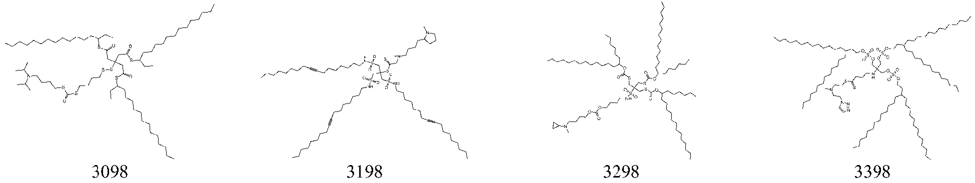

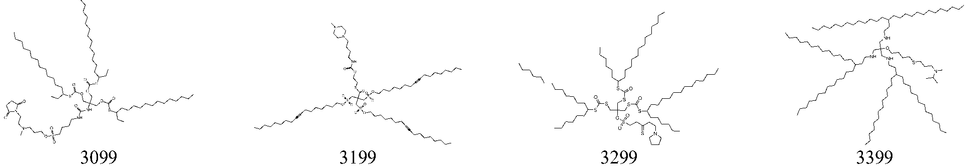

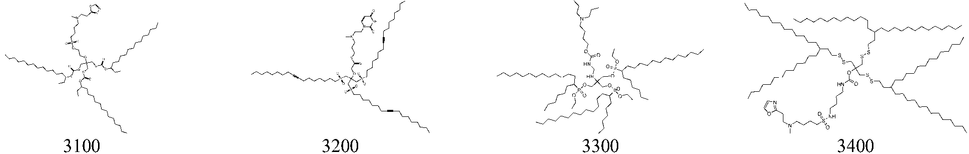

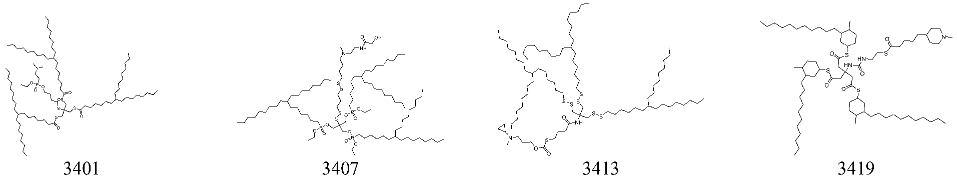

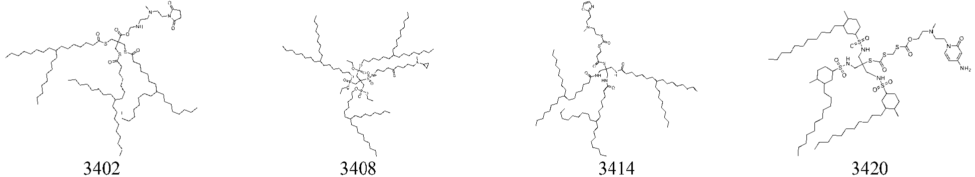

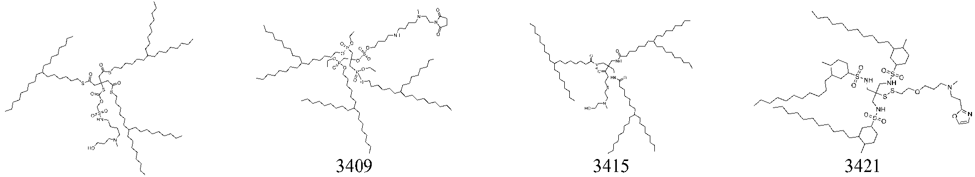

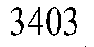

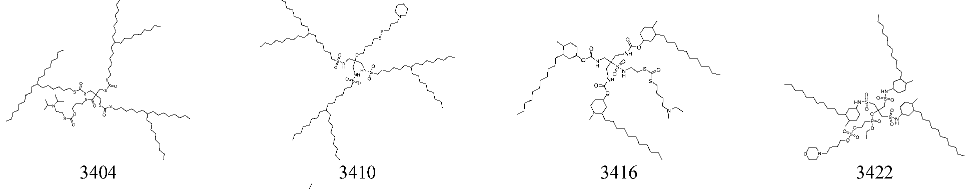

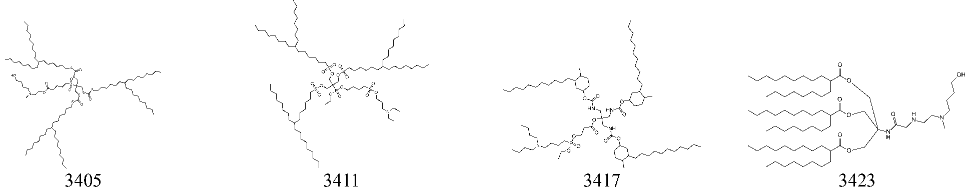

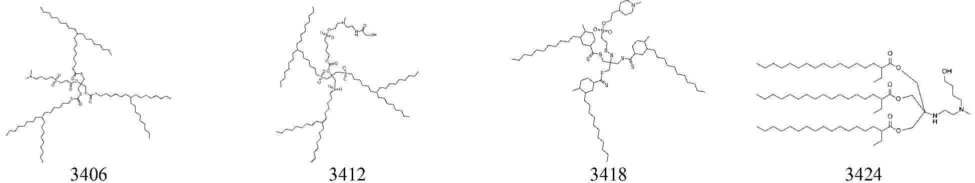

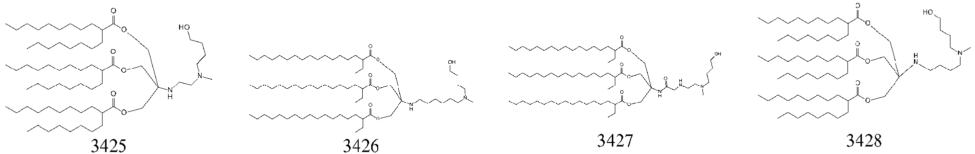

In a second aspect, the present invention provides a preparation method for the aforementioned ionizable cationic lipid compound, comprising a step of reacting a compound of Formula (II) with a compound of Formula (III): 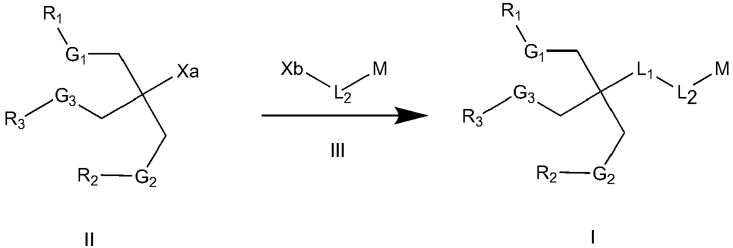 wherein Xₐ and X_{b} are each a group containing a leaving group or a nucleophilic group, and Xₐ and X_{b} form L₁ through a nucleophilic reaction or a condensation reaction.

In a third aspect, the present invention provides a preparation method for the aforementioned ionizable cationic lipid compound, comprising a step of reacting a compound of Formula (IV) with a compound of Formula (V): 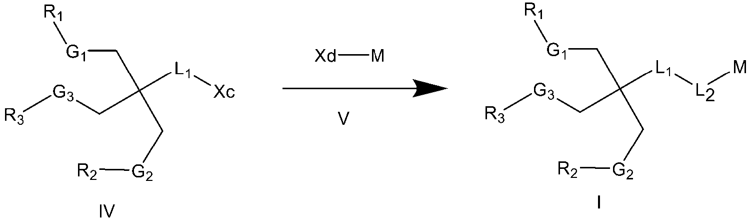 wherein X_{c} and X_{d} are each a group containing a leaving group or a nucleophilic group, and X_{c} and X_{d} form L₂ through a nucleophilic reaction or a condensation reaction.

In a fourth aspect, the present invention provides a preparation method for the aforementioned ionizable cationic lipid compound, comprising a step of reacting a compound of Formula (VI): 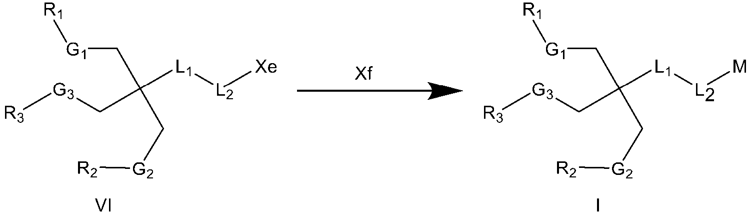 wherein X_{c} is a group containing a leaving group or a nucleophilic group, X_{f} is a compound containing a leaving group or a nucleophilic group, and Xₑ and X_{f} form M through a nucleophilic reaction or a condensation reaction.

In a fifth aspect, the present invention provides a preparation method for the aforementioned ionizable cationic lipid compound, comprising a step of reacting a compound of Formula (VII) sequentially with a compound of Formula (VIII), a compound of Formula (IX), and a compound of Formula (X): 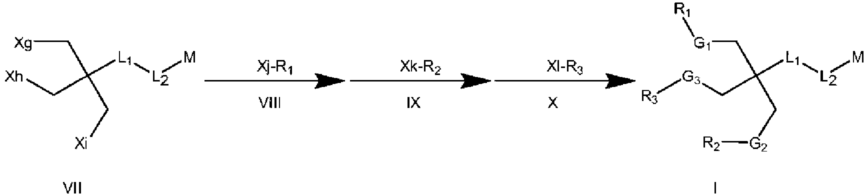 wherein X_{g}, Xₕ, Xᵢ, Xⱼ, Xₖ, and Xₗ are each a group containing a leaving group or a nucleophilic group, and X_{g} and Xⱼ form G₁ through a nucleophilic reaction or a condensation reaction, Xₕ and Xₖ form G₂ through a nucleophilic reaction or a condensation reaction, and Xᵢ and Xₗ form G₃ through a nucleophilic reaction or a condensation reaction.

In a sixth aspect, the present invention provides a preparation method for the aforementioned ionizable cationic lipid compound, comprising a step of reacting a compound of Formula (XI) with a compound of Formula (XII): 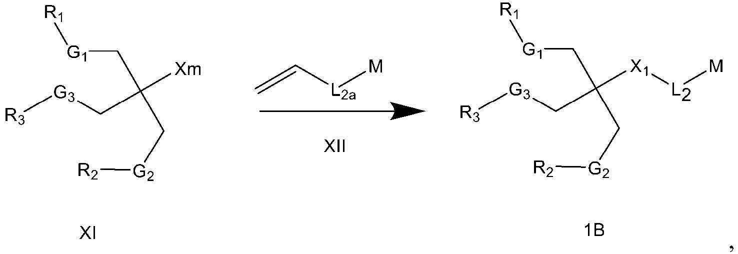 or
a step of reacting Formula (XIII) with a compound of Formula (XII): 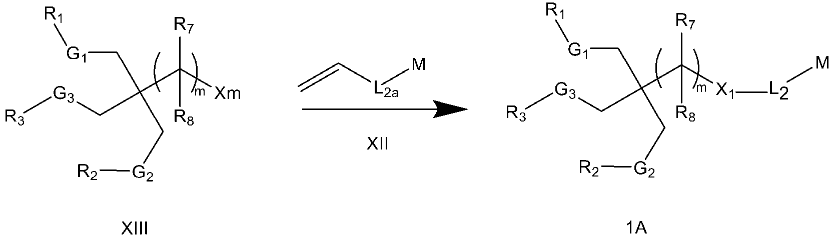
wherein Xₘ is a group containing a nucleophilic group, and Xₘ reacts with a compound of Formula (XII), C=C-L₂ₐ, through an addition reaction to form X₁-L₂.

In a preferred embodiment, R₁, R₂ and R₃ are each independently the following group: 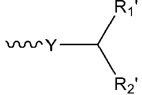
wherein Y, R₁' and R₂' are as defined above;
wherein further comprising a step of forming tail chains R₁, R₂ and R₃: 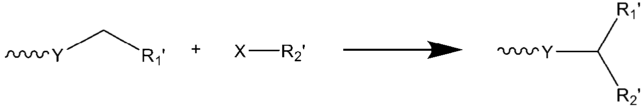
wherein X is a leaving group.

In a preferred embodiment, the raw materials used in the reaction further contain protective groups, and the reaction steps include steps of protection and/or deprotection.

Those of ordinary skill in the art will recognize that protective groups may be required to protect certain groups from the influence of reaction conditions. Protective groups may also be used to distinguish similar functional groups in a molecule. A list of protective groups and methods for their introduction and removal can be found in Wuts, P.G.M., Greene, T.W., Greene's Protective Groups in Organic Synthesis, 4th edition, John Wiley & Sons: New Jersey, 2007.

Preferred protective groups include but are not limited to protective groups for hydroxyl group, protective groups for amino group or an amine group, protective groups for carboxyl group, protective groups for aldehydes or ketones, protective groups for mercapto group, and protective groups for any combination of functional groups such as hydroxyl group, amino group or amine group, carboxyl group, aldehyde or ketone, mercapto groups, and the like.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, methyl and the substituted methyl group may be selected from: methoxymethyl, methylthiomethyl, (phenyldimethylsilyl)methoxymethyl, benzyloxymethyl, p-methoxybenzyloxymethyl, [(3,4-dimethoxybenzyl)oxy]methyl, p-nitrobenzyloxymethyl, o-nitrobenzyloxymethyl, [(R)-1-(2-nitrophenyl)ethoxy]methyl, (4-methoxyphenoxy)methyl, o-methoxyphenoxymethyl, p-phenylphenoxymethyl, t-butoxymethyl, 4-isopentyloxymethyl, siloxymethyl, 2-methoxyethoxymethyl, 2-cyanoethoxymethyl, bis(2-chloroethoxy)methyl, 2,2,2-trichloroethoxymethyl, 2-(trimethylsilyl)ethoxymethyl, menthyloxymethyl, O-bis(2-acetoxyethoxy)methyl, 2-tetrahydropyranyl, fluorotetrahydropyranyl, 3-bromo-2-tetrahydropyranyl, tetrahydrothiopyranyl, 1-methoxycyclohexyl, 4-methoxycyclohexyl, 4-methoxytetrahydropyran-4-yl, 4-methoxythiotetrahydropyran-4-yl, 4-methoxythiotetrahydropyran-4-yl, 1-[(2-chloro-4-methyl)phenyl]-4-methoxypiperidin-4-yl, 1-(2-fluorophenyl)-4-methoxypiperidin-4-yl, 1-(4-chlorophenyl)-4-methoxypiperidin-4-yl, 1,4-dioxan-2-yl, tetrahydrofuran-2-yl, thiotetrahydrofuran-2-yl, 2,3,3a,4,5,6,7,7a-octahydro-7,8,8-trimethyl-4,7-methofuran-2-yl.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, the substituted ethyl group may be selected from: 1-ethoxyethyl, 1-(2-chloroethoxy)ethyl, 2-hydroxyethyl, 2-bromoethyl, 1-[2-(trimethylsilyl)ethoxy]ethyl, 1-methyl-1-methoxyethyl, 1-methyl-1-benzyloxyethyl, 1-methyl-1-benzyloxy-2-fluoroethyl, 1-methyl-1-phenoxyethyl, 2,2,2-trichloroethyl, 1,1-di(p-methoxyphenyl)-2,2,2-trichloroethyl, 1,1,1,3,3,3-hexafluoro-2-phenylisopropyl, 1-(2-cyanoethoxy)ethyl, 2-trimethylsilylethyl, 2-(benzylthio)ethyl, 2-(phenylseleno)ethyl, t-butyl, cyclohexyl, 1-methyl-1'-cyclopropylmethyl, allyl, isopentenyl, cinnamyl, 2-styryl, propargyl, p-chlorophenyl, p-methoxyphenyl, p-nitrophenyl, 2,4-dinitrophenyl, 2,3,5,6-tetrafluoro-4-(trifluoromethyl)phenyl, or the like.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, benzyl and the methoxy-substituted benzyl group may be selected from: p-methoxybenzyl, 3,4-dimethoxybenzyl, 2,6-dimethoxybenzyl, o-nitrobenzyl, p-nitrobenzyl, pentadienylnitrobenzyl, pentadienylnitropiperonyl, 2,6-dichlorobenzyl, 2,4-dichlorobenzyl, 2,6-difluorobenzyl, p-cyanobenzyl, fluorobenzyl, 4-fluoroalkoxybenzyl, p-trimethylsilylmethylbenzyl, p-phenylbenzyl, 2-phenyl-2-propyl, p-acetamidobenzyl, p-4-azidobenzyl, 4-azido-3-chlorobenzyl, 2-trifluoromethylbenzyl, 4-trifluoromethylbenzyl, p-(methylsulfinyl)benzyl, p-(Si-methyl-cyclobutylsilyl)benzyl, 4-acetoxybenzyl, 4-(2-trimethylsilyl)ethoxymethoxybenzyl, 2-naphthylmethyl, 2-picolyl, 4-picolyl, 3-methyl-N-oxo-2-picolyl, 2-quinolinylmethyl, 6-methoxy-2-(4-methylphenyl)-4-quinolyl, 1-pyrenylmethyl, diphenylmethyl, 4-methoxydiphenylmethyl, 4-phenyldiphenylmethyl, bis(p-nitrophenyl)methyl, 5-dibenzosuberyl, triphenylmethyl, tris(4-t-butylphenyl)methyl, α-naphthyldiphenylmethyl, p-methoxyphenyldiphenylmethyl, bis(p-methoxyphenyl)phenylmethyl, tri(p-methoxyphenyl)methyl, 4-(4'-benzoyl)phenylphenyldiphenylmethyl, 4,4',4"-tris(4,5-dichlorophthalimidophenyl)methyl, 4,4',4"-tris(levulinoyloxyphenyl)methyl, 4,4',4"-tris(benzyloxyphenyl)methyl, 4,4'-dimethoxy-3"-[N-(imidazolylmethyl)]triphenylmethyl, 4,4'-dimethoxy-3"-[N-(imidazolylmethyl)aminocarbonyl]triphenylmethyl, bis(4-methoxyphenyl)-1'-pyrenylmethyl, 4-(17-tetrabenzo[a,c,g,i]fluorenylmethyl)-4,4"-dimethoxytriphenylmethyl, 9-anthryl, 9-(9-phenyl)xanthenyl, 9-phenylthioxanthenyl, 9-(9-phenyl-10-oxo)anthryl, 4,5-di(ethoxycarbonyl)-[1,3]-dioxolan-2-yl, benzisothiazole-S,S-dioxide, or the like.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, the silyl group may be selected from: trimethylsilyl, triethylsilyl, triisopropylsilyl, dimethylisopropylsilyl, diethylisopropylsilyl, dimethyl-n-hexylsilyl, 2-norbornanedimethylsilyl, t-butyldimethylsilyl, t-butyldiphenylsilyl, triphenylsilyl, tri(p-methylphenyl)silyl, triphenylmethylsilyl, diphenylmethyl, di-t-butylmethylsilyl, di(t-butyl)-1-pyrenylmethoxysilyl, tris(trimethylsilyl)silyl, (2-hydroxystyryl)dimethylsilyl, (2-hydroxystyryl)diisopropylsilyl, t-butylmethoxyphenylsilyl, t-butoxydiphenylsilyl, 1,1,3,3-tetraisopropyl-3-[2-(triphenylmethoxy)ethoxy]disiloxan-1-yl, fluoroalkylsilyl, or the like.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, the acyl group may form ester with an alcohol and may be: formate, benzyloxyformate, ethyl ester, chloroacetate, dichloroacetate, triacetate, trichloroacetimidate, trifluoroacetate, methoxyacetate, triphenylmethoxyacetate, phenoxyacetate, p-chlorophenoxyacetate, phenylacetate, p-polymer phenylacetate, diphenylacetate, 3-phenylpropionate, propionate having two fluorine-substituted alkyl chains, 4-pentenoate, levulinate, 4,4-(ethylenedithio)pentanoate, 5-[3-di(4-methoxyphenyl)hydroxymethylphenoxy]levulinate, pivalate, 1-adamantanecarboxylate, 2-butenoate, 4-methoxy-2-butenoate, benzoate, p-phenylbenzoate, 2,4,6-trimethylbenzoate, 4-bromobenzoate, 2,5-difluorobenzoate, p-nitrobenzoate, pyridine-2-carboxylate, nicotinate, 2-(azidomethyl)benzoate, 4-azidobutyrate, (2-azidomethyl)phenylacetate, 2-{[(triphenylmethylmercapto)oxy]methyl}benzoate, 2-{[(4-methoxytriphenylmethylmercapto)oxy]methyl}benzoate, 2-{[methyl(trityl)amino]methyl}benzoate, 2- { { [(4-methoxytriphenylmethyl)mercapto]methylamino } -methyl } benzoate, 2-(allyloxy)phenylacetate, 2-(isopentenyloxymethyl)benzoate, 6-(levulinyloxymethyl)-3-methoxy-2-nitrobenzoate, 6-(levulinyloxymethyl)-3-methoxy-4-nitrobenzoate, 4-benzyloxybutyrate, 4-triallylsiloxybutyrate, 4-acetoxy-2,2-dimethylbutyrate, 2,2-dimethyl-4-pentenoate, 2-iodobenzoate, 4-nitro-4-methylpentanoate, o-(dibromomethyl)benzoate, 2-formylbenzenesulfonate, 4-(methylthiomethoxy)butyrate, 2-(methylthiomethoxy)benzoate, 2-(chloroacetoxymethyl)benzoate, 2-[(2-chloroacetoxy)ethyl]benzoate, 2-[2-(benzyloxy)ethyl]benzoate, 2-[2-(4-methoxybenzyloxy)ethyl]benzoate, or the like.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, the sulfonate, the sulfinate or the sulfonate may be, for example: sulfate, allylsulfonate, methanesulfonate, benzylsulfonate, p-toluenesulfonate, 2-[(4-nitrophenyl)ethyl]sulfonate, 2-trifluoromethanesulfonate, 4-monomethoxytritylsulfenate, 2,4-dinitrobenzenesulfenate, 2,2,5,5-tetramethylpyrrolidin-3-one-1-sulfinate, or the like.

In a preferred embodiment, the protective group for hydroxyl group may be a boronate or a dimethyl phosphorothioate.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, the carbonate may be: methyl carbonate, methoxymethyl carbonate, 9-fluorenylmethyl carbonate, ethyl carbonate, bromoethyl carbonate, 2-(methylthiomethoxy)ethyl carbonate, 2,2,2-trichloroethyl carbonate, 1,1-dimethyl-2,2,2-trichloroethyl carbonate, 2-(trimethylsilyl)ethyl carbonate, 2-[dimethyl(2-naphthylmethyl)silyl]ethyl carbonate, 2-(benzenesulfonyl)ethyl carbonate, 2-(triphenylphosphonio)ethyl carbonate, cis-[4-[[(methoxytrityl)sulfenyl]oxy]tetrahydrofuran-3-yl]oxy carbonate, isobutyl carbonate, t-butyl carbonate, vinyl carbonate, allyl carbonate, cinnamyl carbonate, propargyl carbonate, p-chlorophenyl carbonate, p-nitrophenyl carbonate, 4-ethoxy-1-naphthyl carbonate, 6-bromo-7-hydroxycoumarin-4-ylmethyl carbonate, benzyl carbonate, o-nitrobenzyl carbonate, p-nitrobenzyl carbonate, p-methoxybenzyl carbonate, 3,4-dimethoxybenzyl carbonate, anthraquinon-2-ylmethyl carbonate, 2-dansylethyl carbonate, 2-(4-nitrophenyl)ethyl carbonate, 2-(2,4-dinitrophenyl)ethyl carbonate, 2-(2-nitrophenyl)propyl carbonate, 2-(3,4-methylenedioxy-6-nitrophenyl)propyl carbonate, 2-cyano-1-phenylethyl carbonate, 2-(2-pyridyl)amino-1-phenylethyl carbonate, 2-[N-methyl-N-(2-pyridyl)]amino-1-phenylethyl carbonate, phenacyl carbonate, 3',5'-dimethoxybenzoin carbonate, methyl dithiocarbonate, S-benzyl thiocarbonate, or the like.

In a preferred embodiment, the protective group for hydroxyl group may be a substituted methyl group, a substituted ethyl group, a substituted benzyl group, a silyl group, an acyl group, a sulfonyl group, a sulfinyl group, a sulfenyl group, a carbamoyl group, a carbonate group, a boronate group, and a phosphoryl group. Among them, the carbamate may be: N,N-dimethylthiocarbamate, N-phenylcarbamate, N-methyl-N-(o-nitrophenyl)carbamate, or the like.

In a preferred embodiment, 1,2-diol or 1,3-diol may be protected by forming a cyclic acetal or ketal, a cyclic orthoester, a silane derivative, a cyclic carbonate, or a cyclic boronate. Among them, the protective groups forming a cyclic acetal or ketal may be: methylene, ethylidene, t-butylmethylene, 1-t-butylethylidene, 1-phenylethylidene, 2-(methoxycarbonyl)ethylidene, 2-(t-butylcarbonyl)ethylidene, benzenesulfonylethylidene, 2,2,2-trichloroethylidene, 3-(benzyloxy)propylidene, acrolein acetal, acetonide (isopropylidene), cyclopentylidene, cyclohexylidene, cycloheptylidene, benzylidene, p-methoxybenzylidene, 1-(4-methoxyphenyl)ethylidene, 2,4-dimethoxybenzylidene, 3,4-dimethoxybenzylidene, p-acetoxybenzylidene, 4-(t-butyldimethylsilyloxy)benzylidene, 2-nitrobenzylidene, 4-nitrobenzylidene, mesitylene, 6-bromo-7-hydroxycoumarin-2-methylene, 1-naphthaldehyde acetal, 2-naphthaldehyde acetal, 9-anthracene acetal, benzophenone ketal, bis(p-anisyl)methylene ketal, xanthen-9-ylidene, 2,7-dimethylxanthen-9-ylidene.

In a preferred embodiment, 1,2-diol or 1,3-diol may be protected by forming a cyclic acetal or ketal, a cyclic orthoester, a silane derivative, a cyclic carbonate, or a cyclic boronate. Among them, the protective groups forming a cyclic orthoester may be: methoxymethylene, ethoxymethylene, 2-oxacyclopentylidene, dimethoxymethylene, 1-methoxyethylidene 1-ethoxyethylidene, methylene, phthalide, 1,2-dimethoxyethylidene, α-methoxybenzylidene, 1-(N,N-dimethylamino)ethylidene derivative, α-(N,N-dimethylamino)benzylidene derivative, butane-2,3-diacetal, cyclohexane-1,2-diacetal, dispiroketal, or the like.

In a preferred embodiment, 1,2-diol or 1,3-diol may be protected by forming a cyclic acetal or ketal, a cyclic orthoester, a silane derivative, a cyclic carbonate, or a cyclic boronate. Among them, the silane derivative protective group may be: di-t-butylsilylene, dialkylsilylene, 1,3-(1,1,3,3-tetraisopropyldisiloxane) derivative, 1,1,3,3-tetra-t-butoxydisiloxanylidene derivative, methylene-bis-(diisopropylsiloxanylidene)methylene, 1,1,4,4-tetraphenyl-1,4-disilanylene, o-xylylene ether, 3,3'-oxybis(dimethoxytrityl)ether, 1,2-vinylene-3,3-bis(4",4'-dimethoxytrityl)ether.

In a preferred embodiment, 1,2-diol or 1,3-diol may be protected by forming a cyclic acetal or ketal, a cyclic orthoester, a silane derivative, a cyclic carbonate, or a cyclic boronate. Among them, the protective group forming a cyclic carbonate may be formyl.

In a preferred embodiment, 1,2-diol or 1,3-diol may be protected by forming a cyclic acetal or ketal, a cyclic orthoester, a silane derivative, a cyclic carbonate, or a cyclic boronate. Among them, the protective group forming a cyclic boronate may be: methyl boronate, ethyl boronate, phenyl boronate, o-acetamidophenyl boronate, or the like.

In a preferred embodiment, the protective group for hydroxyl group is preferably selected from the following protective groups: tetrahydropyranyl (THP), methyl (Me), benzyl (Bn), methoxymethyl (MOM), allyl (All), triphenylmethyl (Trt), acetyl (Ac), pivaloyl (Piv), t-butyldimethylsilyl (TBDMS), t-butyldimethylsilyl (TBDPS), trimethylsilyl (TMS), triethylsilyl (TES), phthalimide (Phth), t-butoxycarbonate (Boc), benzyloxycarbonate (Cbz), 9-fluorenylmethoxycarbonate (Fmoc).

In a preferred embodiment, the protective group for carboxylic acid may be a substituted methyl ester, a substituted ethyl ester, a phenyl ester or substituted phenyl ester, a silyl ester, an amino group, or a hydrazino group. Among them, substituted methyl esters may be: 9-fluorenylmethyl ester, methoxymethyl ester, 2-methoxyethoxymethyl ester, methylthiomethyl ester, 2-tetrahydropyranyl ester, 2-tetrahydrofuranyl ester, 2-(trimethylsilyl)ethoxymethyl ester, benzyloxymethyl ester, triisopropylsilyloxymethyl ester, pivaloyloxymethyl ester, phenylacetoxymethyl ester, triisopropylsilylmethyl ester, cyanomethyl ester, acetol ester, phenacyl ester, p-bromophenacyl ester, α-methylphenacyl ester, p-methoxyphenacyl ester, 3,4,5-trimethoxyphenacyl ester, 2,5-dimethylphenacyl ester, desyl ester, aminoacylmethyl ester, p-azobenzoylmethyl ester, 6-bromo-7-hydroxycoumarin-4-ylmethyl ester, N-phthalimidomethyl ester, or the like.

In a preferred embodiment, the protective group for carboxylic acid is a substituted ethyl ester, for example: 2,2,2-trichloroethyl ester, 2-haloethyl ester, α-chloroalkyl ester, 2-(trimethylsilyl)ethyl ester, (2-methyl-2-trimethylsilyl)ethyl ester, (2-phenyl-2-trimethylsilyl)ethyl ester, 2-(methylthio)ethyl ester, 1,3-dithian-2-ylmethyl ester, 2-(p-nitrobenzenesulfinyl)ethyl ester, 2-(p-methylbenzenesulfonyl)ethyl ester, 2-(2'-pyridyl)ethyl ester, 2-(diphenylphosphino)ethyl ester, (p-methoxyphenyl)ethyl ester, 1-methyl-1-phenylethyl ester, 2-(4-acetyl-2-nitrophenyl)ethyl ester, 1-[2-(2-hydroxyalkyl)phenyl]ethanone, 2-cyanoethyl ester, t-butyl ester, 3-methyl-3-pentyl ester, dicyclopropylmethyl ester, 2,4-dimethyl-3-pentyl ester, cyclopentyl ester, cyclohexyl ester, allyl ester, methallyl ester, 2-methyl-3-buten-2-yl ester, 3-methyl-2-butenyl ester, 3-butenyl ester, 4-(trimethylsilyl)-2-buten-1-yl ester, cinnamyl ester, α-methylcinnamyl ester, propargyl ester, or the like.

In a preferred embodiment, the protective group for carboxylic acid is a phenyl ester or substituted phenyl ester, for example: phenyl ester, 2,6-dimethylphenyl ester, 2,6-diisopropylphenyl ester, 2,6-di-t-butyl-4-methylphenyl ester, 2,6-di-t-butyl-4-methoxyphenyl ester, p-(methylthio)phenyl ester, pentafluorophenyl ester, 2-(dimethylamino)-5-nitrophenyl ester, or the like.

In a preferred embodiment, the protective group for carboxylic acid is a benzyl ester or substituted phenyl ester, for example: benzyl ester, triphenylmethyl ester, 2-chlorophenyldiphenylmethyl ester, 2,3,4,4',4",5,6-heptafluorotriphenylmethyl ester, diphenylmethyl ester, bis(o-nitrophenyl)methyl ester, 9-anthrylmethyl ester, 2-(9,10-dioxo)anthrylmethyl ester, 5-dibenzosuberyl ester, 1-pyrenylmethyl ester, 2-(trifluoromethyl)-6-cromoglycoylmethyl ester, 2,4,6-trimethylbenzyl ester, p-bromobenzyl ester, o-nitrobenzyl ester, p-nitrobenzyl ester, p-methoxybenzyl ester, 2,6-dimethoxybenzyl ester, 4-(methylsulfinyl)benzyl ester, 4-sulfobenzyl ester, 4-azidomethoxybenzyl ester, 4- {N-[1-(4,4-dimethyl-2,6-dioxocyclohexylidene)-3-methylbutyl]amino}benzyl ester, piperonyl ester, 4-picolyl ester, p-polymer substituted benzyl ester, 2-naphthylmethyl ester, 3-nitro-2-naphthylmethyl ester, 4-quinolylmethyl ester, 8-bromo-7-hydroxyquinolin-2-ylmethyl ester, 2-nitro-4,5-dimethoxybenzyl ester, 1,2,3,4-tetrahydro-1-naphthyl ester, or the like.

In a preferred embodiment, the protective group for carboxylic acid may be silyl ester, for example: trimethylsilyl ester, triethylsilyl ester, t-butyldimethylsilyl ester, t-butyldiphenylsilyl ester, isopropyldimethylsilyl ester, phenyldimethylsilyl ester, di-t-butylmethylsilyl ester, triisopropylsilyl ester, tris(2,6-diphenylbenzyl)silyl ester, or the like.

In a preferred embodiment, the protective group for carboxylic acid may be an amino group or hydrazino group, for example: N,N-dimethylamino, pyrrolinyl, piperidyl, 5,6-dihydrophenanthridyl, o-nitroanilino, N-7-nitroindolyl, N-8-nitro-1,2,3,4-tetrahydroquinolyl, 2-(2-aminophenyl)acetaldehyde dimethyl acetal, p-polymer-benzenesulfonamido, N-phenylhydrazine, N,N'-dimethylhydrazine, N,N'-diisopropylhydrazine, or the like.

In a preferred embodiment, protection of a carboxylic acid may also be achieved by chemically converting the carboxylic acid group into the following groups: 2-alkyl-1,3-oxazoline, 4-alkyl-5-oxo-1,3-oxazoline, 2,2-bis(trifluoromethyl)-4-alkyl-5-oxo-1,3-oxazoline, 2,2-dimethyl-4-alkyl-2-sila-5-oxo-1,3-oxazoline, 2,2-difluoro-1,3,2-oxazaboran-5-one, 5-alkyl-4-oxo-1,3-dioxolane, dioxanone, orthoester, pentaamminecobalt(III) complex, or the like.

In a preferred embodiment, the protective group for carboxyl group is preferably selected from the following protective groups: t-butyl ester (t-Bu), benzyl ester (Bn), methyl ester (Me), ethyl ester (Et), allyl ester (All), isopropyl ester (i-Pr), isobutyl ester (i-Bu), triphenylmethyl ester (Trt), methoxymethyl ester (MOM), t-butyldimethylsilyl ester (TBDMS), and 9-fluorenylmethoxycarbonyl (Fmoc).

In a preferred embodiment, the protective group for amino group or amine group may be carbamate ROC(O)N(R')₂, wherein N(R')₂ is the protected group, and R may be methyl or substituted methyl, for example: methyl, 9-fluorenylmethyl, 2,6-di-t-butyl-9-fluorenylmethyl, 2,7-bis(trimethylsilyl)fluorenylmethyl, 9-(2-sulfo)fluorenylmethyl, 9-(2,7-dibromo)fluorenylmethyl, 17-tetrabenzo[a,c,g,i]fluorenylmethyl, 2-chloro-3-indenylmethyl, benzo[flinden-3-ylmethyl, 1,1-dioxidobenzo[b]thiophen-2-ylmethyl, 2-methanesulfonyl-3-phenyl-1-prop-2-enoyloxy, 2,7-di-t-butyl-[9-(10,10-dioxo-10,10,10,10-tetrahydrothioxanthonyl)]methyl, or the like.

In a preferred embodiment, the protective group for amino group or amine group may be carbamate ROC(O)N(R')₂, wherein N(R')₂ is the protected group, and R may be ethyl or substituted ethyl, for example: 2,2,2-trichloroethyl, 2-trimethylsilylethyl, (2-phenyl-2-trimethylsilyl)ethyl, 2-phenylethyl, 2-chloroethyl, 1,1-dimethyl-2-haloethyl, 1,1-dimethyl-2,2-dibromoethyl, 1,1-dimethyl-2,2,2-trichloroethyl, 2-(2'-pyridyl)ethyl, 2-(4'-pyridyl)ethyl, 2,2-bis(4'-nitrophenyl)ethyl, 2-[(2-nitrophenyl)dithio]-1-phenylethyl, 2-(N,N-dicyclohexylcarbamoyl)ethyl, t-butyl, fluorine-substituted Boc, 1-adamantyl, 2-adamantyl, 1-(1-adamantyl)-1-methylethyl, 1-methyl-1-(4-biphenyl)ethyl, 1-(3,5-di-t-butylphenyl)-1-methylethyl, triisopropylsilyl, vinyl, allyl, isopentenyl, 1-isopropylallyl, cinnamyl, 4-nitrocinnamyl, 3-(3'-pyridyl)prop-2-enyl, hexadienyl, propargyl, but-2-yne-1,4-diyl, 8-quinolyl, N-hydroxypiperidyl, alkyldithio, benzyl, 3,5-di-t-butylbenzyl, p-methoxybenzyl, p-nitrobenzyl, p-bromobenzyl, p-chlorobenzyl, 2,4-dichlorobenzyl, 4-methylsulfinylbenzyl, 4-trifluoromethylbenzyl, fluorobenzyl, 2-naphthylmethyl, 9-anthrylmethyl, diphenylmethyl, or the like.

In a preferred embodiment, the protective group for amino group or amine group may be carbamate ROC(O)N(R')₂, wherein N(R')₂ is the protected group, and R may be the following groups: 4-phenylacetylbenzyl, 4-azidobenzyl, 4-azidomethoxybenzyl, m-chloro-p-acetoxybenzyl, p-(dihydroxyboryl)benzyl, 5-benzisoxazolylmethyl, 2-(trifluoromethyl)-6-chromonylmethyl, 2-methylthioethyl, 2-methanesulfonylethyl, 2-(p-toluenesulfonyl)ethyl, 2-(4-nitrobenzenesulfonyl)ethyl, 2-(2,4-dinitrobenzenesulfonyl)ethyl, 2-(4-trifluoromethylbenzenesulfonyl)ethyl, [2-(1,3-dithianyl)]methyl, 2-alkylphosphinoethyl, 2-[phenyl(methyl)sulfonio]ethyl, 1-methyl-1-(triphenylphosphonio)ethyl, 1,1-dimethyl-2-cyanoethyl, 2-dansylethyl, 2-(4-nitrophenyl)ethyl, 4-methylthiophenyl, 2,4-dimethylthiophenyl, m-nitrophenyl, 3,5-dimethoxybenzyl, 1-methyl-1-(3,5-dimethoxyphenyl)ethyl, α-methylnitropiperonyl, nitrobenzyl, 3,4-dimethoxy-6-nitrobenzyl, 3,4-disubstituted-6-nitrobenzyl, phenyl(o-nitrophenyl)methyl, 2-nitrophenylethyl, 6-nitroveratryl, 4-methoxybenzoylmethyl, 3',5'-dimethoxybenzoin, 9-xanthenylmethyl, N-methyl-N-(o-nitrophenyl), N-(2-acetoxyethyl)amino, 1-methyl-1-cyclopropylmethyl, t-amyl, 1-methylcyclobutyl, 1-methylcyclohexyl, cyclobutyl, cyclopentyl, cyclohexyl, isobutyl, isobornyl, cyclopropylmethyl, p-decyloxybenzyl, diisopropylmethyl, 2,2-dimethoxycarbonylvinyl, o-(N,N-dimethylcarbamoyl)benzyl, 1,1-dimethyl-3-(N,N-dimethylcarbamoyl)propyl, butynyl, 1,1-dimethylpropynyl, 2-iodoethyl, 1-methyl-1-(4'-pyridyl)ethyl, 1-methyl-1-(p-phenylazophenyl)ethyl, p-(p-methoxyphenylazo)benzyl, p-(phenylazo)benzyl, 2,4,6-trimethylbenzyl, isonicotinyl, 4-(trimethylammonio)benzyl, p-cyanobenzyl, bis(2-pyridyl)methyl, 2-furylmethyl, phenyl, 2,4,6-tri-t-butylphenyl, 1-methyl-1-phenylethyl, benzyl thiocarbamate.

In a preferred embodiment, the protective group for amino group or amine group may be urea or thiourea derivative RN(R')₂, wherein N(R')₂ is the protected group, and R may be selected from the following groups: carbamoyl, phenothiazinyl-(10)-carbonyl derivative, N'-p-toluenesulfonylaminocarbonyl, N'-phenylaminothiocarbonyl, 4-hydroxyanilinocarbonyl, 3-hydroxytryptophylcarbonyl, N'-phenylaminothiocarbonyl, or the like.

In a preferred embodiment, the protective group for amino group or amine group may be an amide RN(R')₂, wherein N(R')₂ is the protected group, and R may be selected from the following groups: formyl, acetyl, chloroacetyl, trichloroacetyl, trifluoroacetyl, phenylacetyl, 3-phenylpropionyl, pent-4-enoyl, pyridylcarbonyl, 3-pyridylcarbonyl, N-benzoyl-phenylalanyl derivative, benzoyl, p-phenylbenzoyl, o-nitrophenylacetyl, 2,2-dimethyl-2-o-nitrophenylacetyl, o-nitrophenoxyacetyl, 3-(o-nitrophenyl)propionyl, 2-methyl-2-(o-nitrophenoxy)propionyl, 3-methyl-3-nitrobutyryl, o-nitrocinnamoyl, o-nitrobenzoyl, 3-(4-t-butyl-2,6-dinitrophenyl)-2,2-dimethylpropionyl, o-(benzoyloxymethyl)benzoyl, 2-(acetoxymethyl)benzoyl, 2-[(t-butyldiphenylsilyloxy)methyl]benzoyl, 3-(3',6'-dioxo-2',4',5-trimethylcyclohexa-1,4-dienyl)-3,3-dimethylpropionyl, o-hydroxy-trans-cinnamoyl, 2-methyl-2-(o-phenylazophenoxy)propionyl, 4-chlorobutyryl, acetoacetyl, 3-(p-hydroxyphenyl)propionyl, (N'-dithiobenzyloxycarbonylamino)acetyl, N-acetylmethionine derivative, or the like.

In a preferred embodiment, for primary amino group, a dual protection strategy may be adopted, and the protective group may be selected from the following groups: 4,5-diphenyl-3-oxazolin-2-one, N-phthaloyl, N-dichlorophthaloyl, N-tetrachlorophthaloyl, N-4-nitrophthaloyl, N-thiodiglycolyl, N-dithiosuccinyl, N-2,3-diphenylmaleoyl, N-2,3-dimethylmaleoyl, N-2,5-dimethylpyrrolyl, N-2,5-bis(triisopropylsiloxy)pyrrole, N-1,1,4,4-tetramethyl-1,4-disila-5-azacyclopentane, N-1,1,3,3-tetramethyl-1,3-disilaisoindoline, N-diphenylsilyldivinylene, N-5-substituted-1,3-dimethyl-1,3,5-triazacyclohexan-2-one, N-5-substituted-1,3-dibenzyl-1,3,5-triazacyclohexan-2-one, 1-substituted-3,5-dinitro-4-pyridone, 1,3,5-dioxazine.

In a preferred embodiment, for N-alkyl-substituted and N-aryl-substituted amino group, the protective group may be selected from the following groups: N-methyl, N-t-butyl, N-allyl, N-isoprenyl, N-cinnamyl, N-phenylallyl, N-propargyl, N-methoxymethyl, N-[2-(trimethylsilyl)ethoxy]methyl, N-3-acetoxypropyl, N-cyanomethyl, 2-azanorbornene, N-2,4-dinitrophenyl, o-methoxy- or p-methoxyphenyl, N-benzyl, N-4-methoxybenzyl, N-2,4-dimethoxybenzyl, N-2-hydroxybenzyl, N-9-phenylfluorenyl, N-fluorenyl, N-ferrocenylmethyl, N-2-picolylamine N'-oxide, N-7-methoxy-4-ylmethyl, N-diphenylmethyl, N-bis(4-methoxyphenylmethyl), N-5-dibenzophenethyl, N-triphenylmethyl, N-(4-methylphenyl)diphenylmethyl, N-(4-methoxyphenyl)diphenylmethyl, or the like.

In a preferred embodiment, protection of an amino group or amine group may include preparing an imine derivative, for example: N-1,1-dimethylthiomethylene, N-benzylidene, N-p-methoxybenzylidene, N-diphenylmethylene, N-[(2-pyridyl) m-xylyl]methylene, N-(N',N'-dimethylaminomethylene), N-(N',N'-dibenzylaminomethylene), N-(N'-t-butylaminomethylene), N,N'-isopropylidene, N-p-nitrobenzylidene, N-salicylidene, N-5-chlorosalicylidene, N-(5-chloro-2-hydroxyphenyl)phenylmethylene, N-cyclohexylidene, N-t-butylmethylene, or the like.

In a preferred embodiment, protection of an amino group or amine group may include preparing an enamine derivative, for example: N-(5,5-dimethyl-3-oxo-1-cyclohexenyl), N-2,7-dichloro-9-fluorenylmethylene, N-1-(4,4-dimethyl-2,6-dioxocyclohexylidene)ethyl, N-(1,3-dimethyl-2,4,6-(1H,3H,5H)-trioxopyrimidin-5-ylidene)methyl, N-4,4,4-trifluoro-3-oxo-1-butenyl, N-(1-isopropyl-4-nitro-2-oxo-3-pyrrolin-3-yl).

In a preferred embodiment, an amino group or amine group may be protected by derivatives formed by reaction of the N atom of the amino group or amine group with other heteroatoms (e.g., N, O, P, Si, or S), for example: N-nitro, N-nitroso, N-oxide, azide, triazacycloalkene, N-trimethylsilylmethyl-N-benzylhydrazine, N-diphenylphosphinamide, dimethyl and diphenyl thiophosphinamide, dialkyl phosphoramidite, dibenzyl and diphenyl phosphoramidite, iminotriphenylphosphorane, tritylsilyl, phenylthioamide, 2-nitrophenylthioamide, 2,4-dinitrophenylthioamide, pentachlorophenylthioamide, 2-nitro-4-methoxyphenylthioamide, triphenylmethylthioamide, 1-(2,2,2-trifluoro-1,1-diphenyl)ethylthioamide, N-3-nitro-2-pyridylthioamide, methanesulfonamide, trifluoromethanesulfonamide, t-butylsulfonamide, benzylsulfonamide, 2-(trimethylsilyl)ethylsulfonamide, p-toluenesulfonamide, benzenesulfonamide, anisolesulfonamide, 2- or 4-nitrobenzenesulfonamide, 2,4-dinitrobenzenesulfonamide, 2-naphthalenesulfonamide, 4-(4',8'-dimethoxynaphthylmethyl)benzenesulfonamide, 2-(4-methylphenyl)-6-methoxy-4-methylsulfonamide, 9-anthracenesulfonamide, pyridine-2-sulfonamide, benzothiazole-2-sulfonamide, phenacylsulfonamide, 2,3,6-trimethyl-4-methoxybenzenesulfonamide, 2,4,6-trimethoxybenzenesulfonamide, 2,6-dimethyl-4-methoxybenzenesulfonamide, pentamethylbenzenesulfonamide, 2,3,5,6-tetramethyl-4-methoxybenzenesulfonamide, 4-methoxybenzenesulfonamide, 2,4,6-trimethylbenzenesulfonamide, 2,6-dimethoxy-4-methylbenzenesulfonamide, 3-methoxy-4-t-butylbenzenesulfonamide, 2,2,5,7,8-pentamethylchromene-6-sulfonamide, or the like.

In a preferred embodiment, amino alcohol or amine alcohol may be protected by converting the amino group or amine group with hydroxyl group into a 2-oxazolidinone or oxazoline through reactions.

In a preferred embodiment, for nitrogen-containing aromatic heterocycles, the aforementioned protective groups of N-sulfonyl derivatives, carbamates, N-alkyl and N-aryl derivatives, N-trialkylsilyl, N-allyl, N-benzyl, amides, amino acetal derivatives, and amide and sulfonamide may be adopted.

In a preferred embodiment, the protective group for amine group is preferably selected from the following protective groups: 9-fluorenylmethoxycarbonyl (Fmoc), benzyloxycarbonyl (Cbz), t-butoxycarbonyl (Boc), trichloroethoxycarbonyl (Troc), allyloxycarbonyl (Alloc), methoxycarbonyl (Moc), acetyl (Ac), trifluoroacetyl (TFA), 2,4-dimethoxybenzyl (DMB), benzyl (Bn), trityl (Tr), benzyloxymethyl (Bom), p-toluenesulfonyl (Ts), 2,2,4,6,7-pentamethyldihydrobenzofuran-5-sulfonyl (Pbf), 4-methoxy-2,3,6-trimethylbenzenesulfonyl (Mtr), phthalimide (Phth), and benzylideneamine.

In a preferred embodiment, aldehyde or ketone group may be protected by forming acetal or ketal, dithio or monothio acetal or ketal, cyanohydrin, substituted hydrazone, oxime, 1,2-adduct, or by converting the carbonyl group into enolate anion, enol ester, enamine, or imine.

In a preferred embodiment, aldehyde or ketone group may be protected by forming acetal or ketal, and the protective group may be selected from: dimethyl, diisopropyl, bis(2,2,2-trichloroethyl), dibenzyl, bis(2-nitrobenzyl), diacetyl, 1,3-dioxane, 5-methylene-1,3-dioxane, 5-trimethylsilyl-1,3-dioxane, 5,5-dibromo-1,3-dioxane, 5-(2-pyridyl)-1,3-dioxane, salicylaldehyde acetal, 1,3-dioxolane, 4,4,5,5-tetramethyl-1,3-dioxolane, 4-bromomethyl-1,3-dioxolane, 4-benzenesulfonylmethyl-1,3-dioxolane, 4-(3-butenyl)-1,3-dioxolane, 4-phenyl-1,3-dioxolane, 4-(4-methoxyphenyl)-1,3-dioxolane, 4-(2-nitrophenyl)-1,3-dioxolane, 4-(4-nitrophenyl)-1,3-dioxolane, 4-fluoro-1,3-dioxolane, 4-[6-bromo-7-hydroxycoumarin-4-yl]-1,3-dioxane, 4-trimethylsilyl-1,3-dioxolane, O,O'-phenylenedioxy ketal, 1,3-dioxolane, 1,5-dihydro-3H-2,4-benzodioxepine, 7,7-dimethyl-1,2,4-trioxepane, 3,3-dialkyl-6-(1-phenylvinyl)-1,2,4-trioxane, (4R,5R)-diphenyl-1,3-dioxolane, 4,5-dimethyl-1,3-dioxolane, trans-1,2-cyclohexanediol ketal, trans-4,6-dimethyl-1,3-dioxane, 4,5-bis(dimethylaminocarbonyl)-1,3-dioxolane, 4,5-diformylmethoxy-1,3-dioxolane, 4,5-dimethoxymethyl-1,3-dioxolane, 2,2-dialkyl-4,5-bis(2-nitrophenyl)-1,3-dioxolane, 4,5-bis(2-nitro-4,5-dimethoxyphenyl)-1,3-dioxolane, or the like.

In a preferred embodiment, aldehyde or ketone group may be protected by forming a dithioacetal or a dithioketal, and the protective group may be selected from: S,S'-dimethyl, S,S'-diethyl, S,S'-dipropyl, S,S'-dibutyl, S,S'-dipentyl, S,S'-diphenyl, S,S'-dibenzyl, S,S'-diacetyl, 1,3-dithiane, 1,3-dithiolane, 1,5-dihydro-3H-2,4-dithiepin, or the like.

In a preferred embodiment, aldehyde or ketone group may be protected by forming a monothioacetal or a monothioketal, and the protective group may be selected from: O-trimethylsilyl-S-alkyl, O-alkyl-S-alkyl or -S-phenyl, O-methyl-S-2-methylthioethyl, 1,3-oxathiolane, or the like.

In a preferred embodiment, aldehyde or ketone group may be protected by forming an O-substituted cyanohydrin, and the protective group may be selected from: O-acetyl, O-methoxycarbonyl, O-trimethylsilyl, O-1-ethoxyethyl, O-tetrahydropyranyl, or the like.

In a preferred embodiment, aldehyde or ketone group may be protected by forming a substituted hydrazone, and the protective group may be selected from: N,N-dimethyl, phenyl, 2,4-dinitrophenyl, p-toluenesulfonyl, semicarbazone, diphenylmethyl, or the like.

In a preferred embodiment, aldehyde or ketone group may be protected by forming a substituted oxime, and the protective group may be selected from: O-methyl, O-benzyl, or O-phenylthiomethyl, or the like.

In a preferred embodiment, aldehyde or ketone group may be protected by forming a 1,2-adduct, and the protective group may be selected from: diethylamine adduct, N-methoxy-N-methylamine adduct, pyrrole adduct, 1-methyl-2-(1'-hydroxyalkyl)imidazole, O-silylimidazolyl amino acetal, sodium bisulfite adduct, O-carborane, aminobutyronitrile derivative, N,N'-dimethylimidazolidine, N,N'-diarylimidazolidine, 2,3-dihydro-1,3-benzothiazole.

In a preferred embodiment, aldehyde or ketone group may be protected by converting the carbonyl group to enolate anion, enol ester, enamine, or imine, for example: lithium diisopropylamide (LDA), trimethylsilyl enol ether, enamine, imine, substituted methylene derivative, methylaluminum bis(2,6-di-t-butyl-4-methylphenoxide) complex, or the like.

In a preferred embodiment, for compounds having two carbonyl groups, one of the carbonyl groups may be selectively protected. Among them, for α- or β-diketones, protection may be selected from: enamine, enol acetate, enol ether, methyl, ethyl, isobutyl, methoxyethoxymethyl, methoxymethyl, enamine derivative, 4-methyl-1,3-dioxolene enol acetate, pyrrolidine enamine, benzyl enol ether, butyl thioenol ether, or the like.

In a preferred embodiment, the protective group for aldehyde or ketone group is preferably selected from the following protective groups: O,O-dimethyl acetal (ketal), 1,3-dioxane, 1,3-dioxolane, S,S'-dimethyl thioacetal (thioketal), 1,3-dithiane, 1,3-dithiolane, N,N-dimethylhydrazone (DMH), 2,4-dinitrophenylhydrazone (DNPH), aminourea, oxime, trimethylsilyl (TMS), Tollens' reagent (silver ammonia solution), or the like.

In a preferred embodiment, thiol group may be protected by forming a thioether, thioester, unsymmetrical disulfide bond, or sulfinyl derivative. Among them, thioether protective groups may be: S-alkyl, S-benzyl, S-p-methoxybenzyl, S-o- or S-p-hydroxy or acetoxybenzyl, S-p- and S-o-nitrobenzyl, S-2,4,6-trimethylbenzyl, S-2,4,6-trimethoxybenzyl, S-4-pyridylmethyl, S-2-picoline N-oxide, S-2-quinolylmethyl, S-9-anthracenylmethyl, S-9-fluorenylmethyl, S-xanthenyl, S-ferrocenylmethyl, S-diphenylmethyl, S-bis(4-methoxyphenyl)methyl, S-5-dibenzosuberyl, S-triphenylmethyl, 4-methoxytrityl, S-diphenyl-4-pyridylmethyl, S-phenyl, S-2,4-dinitrophenyl, S-2-quinolyl, S-t-butyl, S-1-adamantyl, S-methoxymethyl, S-isobutoxymethyl, S-benzyloxymethyl, S-1-ethoxyethyl, S-2-tetrahydropyranyl, S-benzylthiomethyl, S-phenylthiomethyl, thiazolidine, S-acetamidomethyl, S-pivalamidomethyl, S-benzamidomethyl, S-allyloxycarbonylamidomethyl, N-[2,3,5,6-tetrafluoro-4-(N'-piperidyl)phenyl]-N-allyloxycarbonylamidomethyl, phthalimidomethyl, S-phenylacetamidomethyl, S-acetylmethyl, S-carboxymethyl, S-cyanomethyl, S-(2-nitro-1-phenyl)ethyl, S-2-(2,4-dinitrophenyl)ethyl, S-2-(4'-pyridyl)ethyl, S-2-cyanoethyl, S-2-(trimethylsilyl)ethyl, S-2,2-bis(carbonylethoxy)ethyl, S-(1-m-nitrophenyl-2-benzoyl)ethyl, S-2-benzenesulfonylethyl, S-1-(4-methylbenzenesulfonyl)-2-methylpropan-2-yl, S-p-hydroxyphenacyl.

In a preferred embodiment, thiol group may be protected by forming a thioether, thioester, unsymmetrical disulfide bond, or sulfinyl derivative. Among them, thioester protective groups may be: S-acetyl derivative, S-benzoyl derivative, S-2-methoxyisobutyryl, S-trifluoroacetyl derivative, S-N-[[(p-biphenylyl)isopropoxy]carbonyl]-N-methyl-y-aminothiobutyrate, S-N-(t-butoxycarbonyl)-N-methyl-γ-aminothiobutyrate, S-2,2,2-trichloroethoxycarbonyl, S-t-butoxycarbonyl, S-benzyloxycarbonyl, S-p-methoxybenzyloxycarbonyl, S-fluorenylmethylcarbonyl, S-n-ethylaminocarbonyl, S-(N-methoxymethyl)aminocarbonyl, or the like.

In a preferred embodiment, thiol group may be protected by forming a thioether, thioester, unsymmetrical disulfide bond, or sulfinyl derivative. Among them, protective groups forming unsymmetrical disulfide bonds may be: S-ethyl, S-t-butyl, substituted S-phenyl, or the like.

In a preferred embodiment, thiol group may be protected by forming a thioether, thioester, unsymmetrical disulfide bond, or sulfinyl derivative. Among them, protective groups forming sulfenyl derivatives may be: S-sulfonate derivative, S-thiosulfonate derivative, S-sulfenyl thiocarbonate, S-3-nitro-2-pyridylthio, S-tricarbonyl [1',2,3,4,5-η]-2,4-cyclohexadien-1-yl iron, oxathiolanone, or the like.

In a preferred embodiment, dithiol group may be protected by forming a dithio acetal or ketal, and the protective groups may be: S,S'-methylene, S,S'-isopropylidene, S,S'-benzylidene, S,S'-p-methoxybenzylidene, or the like.

In a preferred embodiment, when both amino and thiol groups are present, they may be protected simultaneously by forming thiazolidine or ninhydrin through reactions.

In a preferred embodiment, the protective group for thiol group is preferably selected from the following protective groups: diphenylmethyl (Dpm), triphenylmethyl (Trt), monomethoxytrityl (Mmt), benzyl (Bn), p-methylbenzyl, p-methoxybenzyl (Mob), 2,4,6-trimethoxybenzyl (Tmob), 2-nitrobenzyl (oNb), 2-nitro-4,5-dimethoxybenzyl (oNv), t-butyl (tBu), 1-adamantyl, fluorenylmethyl (Fm), 2,4-dinitrophenylethyl (Dnpe), fluorenylmethoxycarbonyl (Fmoc), allyloxycarbonyl (Alloc), acetamidomethyl (Acm), phenylacetamidomethyl (Phacm), S-t-butylthio (StBu), S-2-pyridylthio (S-Pyr), S-3-nitro-2-pyridylthio (Npys), tetrahydropyranyl (Thp), methanesulfonyl, p-toluenesulfonyl, or the like.

The leaving group described above refers to the moiety that departs in a nucleophilic reaction or condensation reaction, including but not limited to: H, OH, H₂O, halogen (e.g., F, Cl, Br, I), cyanate anion, inorganic acid (e.g., nitric acid, sulfuric acid, or phosphoric acid), carboxylic acid (e.g., acetic acid, trifluoroacetic acid, benzoic acid, etc.), sulfonic acid (e.g., methanesulfonic acid, trifluoromethanesulfonic acid, benzenesulfonic acid, p-toluenesulfonic acid, p-nitrobenzenesulfonic acid, etc.), carbon dioxide (CO₂), nitrogen (N₂), imidazole, alkoxy group (R-O⁻), amino group (-NHR, where R is H, alkyl or aryl to be removed), phenoxy group, tertiary carbocation (e.g., t-butyl cation), carbocation stabilized by an unsaturated system or heteroatom, or various protective groups described above.

The nucleophilic group described above refers to a molecule or ion that can donate an electron pair to form a new chemical bond in a chemical reaction. Common nucleophilic groups include: hydroxide (HO⁻), ammonia (NH₃), hydroxylamine (NH₂OH), hydrazine (NH₂-NH₂), substituted hydrazines, nucleophilic halogens (e.g., Cl⁻, Br⁻, I⁻), hydride (H⁻), azide (N₃⁻), cyanide (CN⁻), alcohol or alkoxide anion (e.g., alcohol deprotonated at the hydroxyl group), amine group (including primary, secondary, and tertiary amines) or amine anion, carbanion (e.g., carbanion in organometallic reagents such as Grignard reagents, organolithium reagents, Gilman reagents), thiol or thiolate anion, thioether, enol or enolate anion, enol ether, enamine, carboxylic acid or carboxylate anion, alkyl or aryl phosphine (e.g., triphenylphosphine), aromatic heterocycles with lone pairs (e.g., pyridine), or the like.

In a preferred embodiment, the synthesis route of the ionizable cationic lipid compound is as follows: 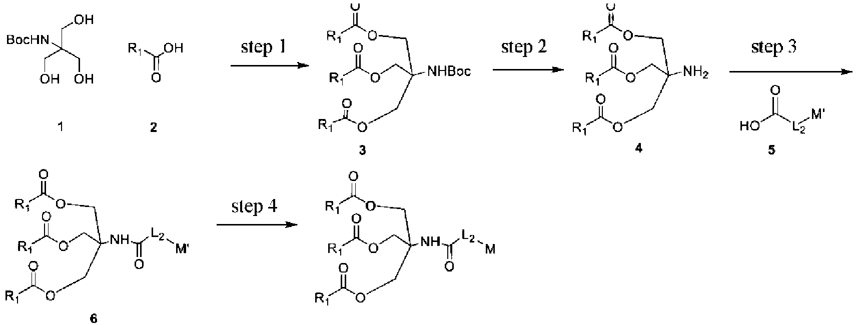 wherein M' is M or M containing a protective group.

In a preferred embodiment, the synthesis route of the ionizable cationic lipid compound is as follows: 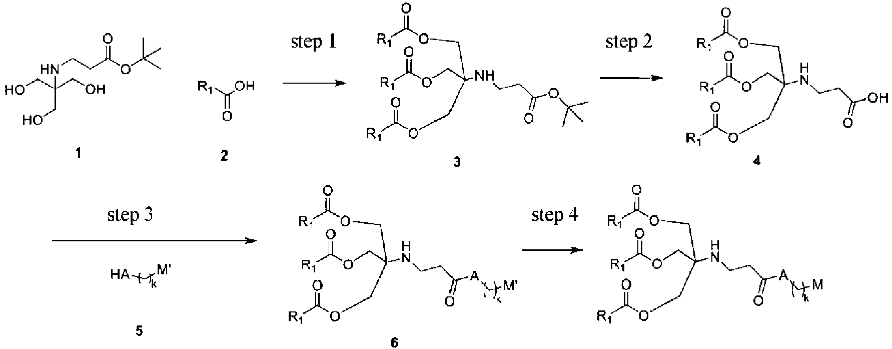 wherein M' is M or M containing a protective group; A is O, NH, or S.

In a preferred embodiment, the synthesis route of the ionizable cationic lipid compound is as follows: 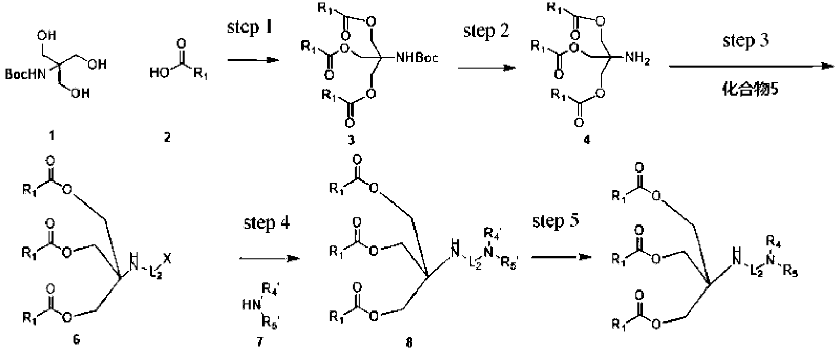 wherein compound 5 is 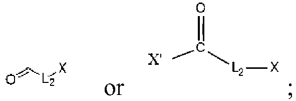 X' and X are halogens; R₄' is R₄ or R₄ containing a protective group; R₅' is R₅ or R₅ containing a protective group.

In a preferred embodiment, the synthesis route of the ionizable cationic lipid compound is as follows: 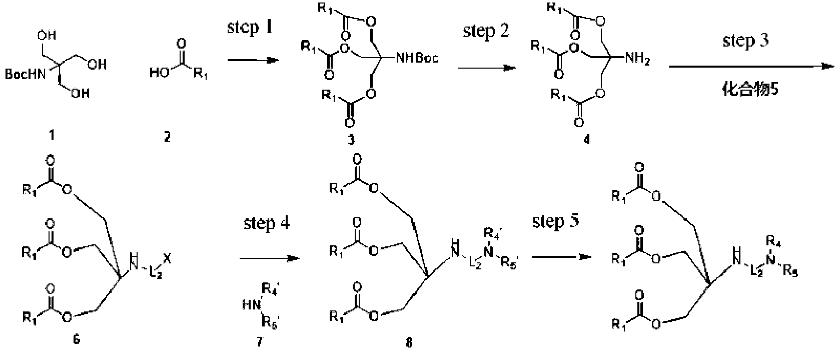 wherein compound 5 is 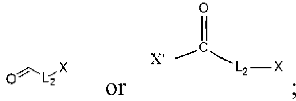 X' and X are halogens; R₄' is R₄ or R₄ containing a protective group; R₅' is R₅ or R₅ containing a protective group.

In a preferred embodiment, the synthesis route of the ionizable cationic lipid compound is as follows: 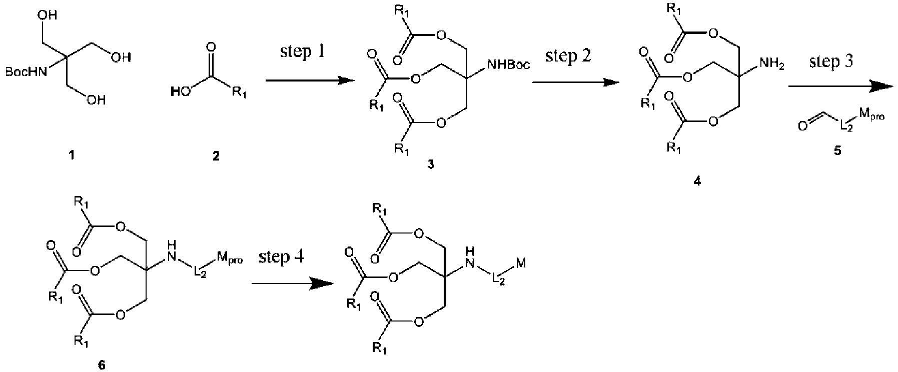 wherein Mₚᵣₒ is M or M containing a protective group.

In a seventh aspect, the present invention provides a use of the aforementioned ionizable cationic lipid compound as a surfactant.

In a preferred embodiment, the ionizable cationic lipid compound is used as emulsifier, suspending agent, dispersant, solubilizer, lubricant, thickener, bacteriostatic agent, or preservative.

In an eighth aspect, the present invention provides a use of the aforementioned ionizable cationic lipid compound in preparing a cosmetic composition.

In a ninth aspect, the present invention provides a use of the aforementioned ionizable cationic lipid compound in preparing a lipid composition, wherein the ionizable cationic lipid compound is used as a drug delivery vehicle.

In a tenth aspect, the present invention provides a lipid composition, wherein the lipid components of the lipid composition comprise the aforementioned ionizable cationic lipid compound.

In a preferred embodiment, the lipid components of the lipid composition further comprise additional lipids, including phospholipids, structural lipids, and PEG-conjugated lipids; the molar ratio of the ionizable cationic lipid compound to additional lipid components is 1:0.2-5; the active ingredient of the lipid nanoparticle composition comprises a therapeutic agent and/or prophylactic agent; the therapeutic agent and/or prophylactic agent is a vaccine or compound capable of eliciting an immune response; the mass ratio of the lipid components to the active ingredient is 10:1 to 60:1.

In a preferred embodiment, the phospholipid is selected from at least one of 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoyl phosphatidylethanolamine (DMPE), 1-stearoyl-2-oleoyl phosphatidylethanolamine (SOPE), 1-stearoyl-2-oleoyl phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine (LPE).

In a preferred embodiment, the structural lipid is selected from at least one of cholesterol, coprosterol, sitosterol, ergosterol, campesterol, stigmasterol, brassicasterol, tomatidine, ursolic acid, and α-tocopherol.

In a preferred embodiment, the PEG lipid is selected from PEG-modified phosphatidylethanolamine, PEG-modified phosphatidic acid, PEG-modified ceramide, PEG-modified dialkylamine, PEG-modified diacylglycerol, PEG-modified dialkylglycerol, and mixtures thereof.

In a preferred embodiment, the therapeutic agent and/or prophylactic agent is a nucleic acid; the nucleic acid is DNA or RNA.

In a preferred embodiment, the RNA is selected from at least one of small interfering RNA (siRNA), asymmetric interfering RNA (aiRNA), microRNA (miRNA), Dicer-substrate RNA (dsRNA), small hairpin RNA (shRNA), and messenger RNA (mRNA).

In an eleventh aspect, the present invention provides a use of the aforementioned lipid composition in a medicament for treating a disease in a mammal, wherein the disease is characterized by dysfunctional or abnormal protein or polypeptide activity; the disease is selected from infectious diseases, cancer, proliferative diseases, genetic diseases, autoimmune diseases, diabetes, neurodegenerative diseases, cardiovascular diseases, renovascular diseases, and metabolic diseases.

In a twelfth aspect, the present invention provides a pharmaceutical composition comprising the aforementioned lipid composition and a pharmaceutically acceptable carrier.

In a preferred embodiment, the pharmaceutical composition further comprises a pharmaceutically active compound selected from anti-inflammatory compounds, steroids, statins, estradiol, BTK inhibitors, S1P1 agonists, glucocorticoid receptor modulators, and antihistamines.

Compared with the prior art, the present invention has the following beneficial effects:
1. The ionizable cationic lipid compounds provided by the present invention are compounds of Formula (1), or a salt, a stereoisomer or a tautomer thereof. The ionizable cationic lipid compounds provided by the present invention are amphiphilic lipid compound constructed by using an ionizable cationic compound and analogs thereof as the ionizable lipid head group, lipids such as fatty acids as tail chains, and a tetrafunctional compound as a linker, where the ionizable lipid head group, tail chains and linker are connected by biodegradable chemical bonds (such as ester bonds or amide bonds, etc.).
2. The calculated c-pKa (molnetwork) of the ionizable cationic lipid compounds (exemplary compounds) provided by the present invention is 5-11. Within the common pH range (0-14), the ionization state of the molecule or the ratio of different ionization states varies with the environmental pH. c-LogP (cLogP driver) ranges from 5 to 55, exhibiting good lipophilicity. The ionizable cationic lipid compounds of the present invention are pH-dependent amphiphilic lipid compounds, indicating that they can serve as excellent surfactants, and are particularly suitable for preparing lipid nanoparticles for use as drug delivery vehicles.
3. The ionizable cationic lipid compounds of the present invention can be used to prepare lipid compositions. mRNA lipid compositions prepared using exemplary compounds have a Zeta potential of -40 mV to 40 mV, a dispersity index of 0.01-0.5, a particle size of 10-400 nm, and an Encapsulation Rate > 50%.
4. Lipid nanoparticles of mRNA and/or other nucleic acid substances (such as siRNA, microRNA, pDNA, etc.) prepared from the ionizable cationic lipid compounds of the present invention exhibit biological activity both *in vitro* in cells and *in vivo* in animals, such as expression of the protein encoded by the mRNA sequence. luciferase mRNA lipid nanoparticles prepared using exemplary compounds exhibit luciferase fluorescence intensity of > 10³ RLU in HEK 293T cells (dose: 100 ng, 96-well plate); the mean whole-body fluorescence signal is greater than 10⁴ p/s/cm²/sr in mice after administered by tail vein injection. This indicates that the luciferase mRNA lipid nanoparticles prepared using the ionizable cationic lipid compounds of the present invention exhibit biological activity both *in vitro* in cells and *in vivo* in animals, and have low toxicity. The lipid nanoparticles provided by the present invention have good safety and effectiveness as drug carriers.

### BRIEF DESCRIPTION OF THE DRAWINGS

Fig. 1 is the luciferase activity of luciferase mRNA lipid nanoparticles prepared from Compound 1005 in HEK 293T cells;
Fig. 2 is the whole-body live imaging at 3 h/6 h after intravenous administration of luciferase mRNA lipid nanoparticles prepared from Compound 1008 in mice.

### DESCRIPTION OF EMBODIMENTS

The synthesis processes of the present invention are compatible with a plurality of functional groups, thereby allowing the use of various substituted starting materials. These processes generally provide the desirable final compound at or near the end of the overall procedure, although in some cases, it may be necessary to further convert the compound into a pharmaceutically acceptable salt thereof. The compounds of the present invention can be prepared in a variety of ways using commercially available starting materials, compounds known in the literature, or readily prepared intermediates, by employing standard synthesis methods and procedures known to or readily apparent to those skilled in the art according to the teachings herein. Standard synthesis methods and procedures for the preparation of organic molecules, as well as functional group transformations and operations can be obtained from the relevant scientific literature or from standard textbooks in the field. The following description of synthesis methods is intended to illustrate, but not limit, the general procedures for preparing the compounds of the present invention.

The compounds having the various formulae described herein can be prepared from commercially available starting materials or starting materials preparable by processes described in literature according to the processes illustrated in the following general synthesis routes 1, 2, 3, 4 and 5. Variables (such as R₁, R₂, R₃ etc.) in each general synthesis route are as defined herein. Those of ordinary skill in the art will recognize that the order of certain steps, such as the introduction and removal of protective groups, can be varied in the reaction processes and synthesis schemes described herein.

A variety of stereoisomers may be produced in the reaction schemes described herein. When no particular stereoisomer is specified, this is understood to include all possible stereoisomers produced by the reaction. Those of ordinary skill in the art will recognize that the reaction can be optimized to obtain an isomer in priority, or new schemes can be designed to yield a single isomer. If a mixture is produced, the isomers can be separated using techniques such as preparative thin-layer chromatography, preparative HPLC, preparative chiral HPLC, or preparative SFC.

### General synthesis route 1

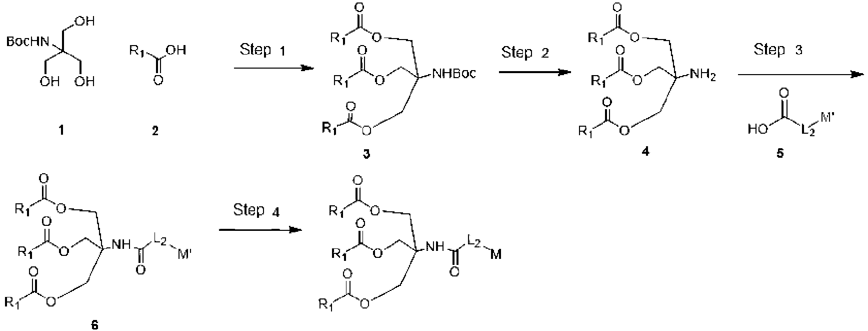

wherein M' is M or M containing a protective group.

As illustrated in the above General Synthesis Route 1, Boc-aminotris(hydroxymethyl)methane undergoes a condensation reaction with acid (compound 2) to form compound 3. Step 1 may be carried out in an organic solvent (e.g., dichloromethane (DCM)) in the presence of, for example, 1-ethyl-(3-dimethylaminopropyl)carbodiimide hydrochloride (EDCl) and 4-dimethylaminopyridine (DMAP). Step 1 may be carried out at room temperature for 24 hours.

Then, the Boc protective group of compound 3 is removed to form compound 4. Step 2 may be carried out in an organic solvent (e.g., DCM) under the catalysis of an acid (e.g., trifluoroacetic acid).

Subsequently, compound 4 undergoes a condensation reaction with compound 5 to form compound 6. Step 3 may be carried out in an organic solvent (e.g., DCM or DMF) under the catalysis of EDCl and DMAP or dicyclohexylcarbodiimide (DCC).

If the M' group of compound 6 contains the aforementioned protective group, the protective group is removed to obtain the target lipid compound. Step 4 is carried out under the deprotection reaction condition for the selected protective group.

### General synthesis route 2

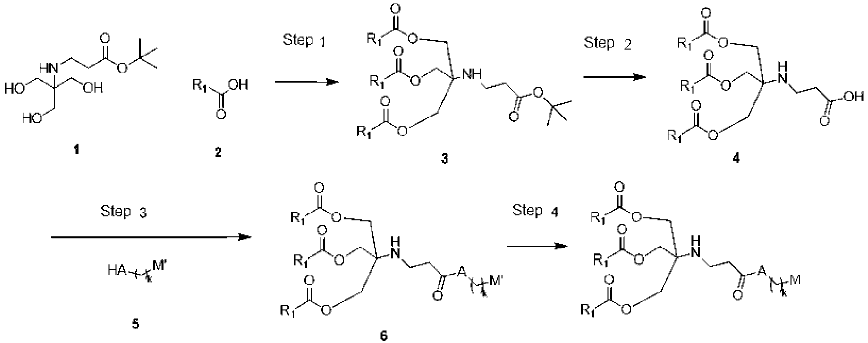

wherein M' is M or M containing a protective group; A is O, NH or S.

As illustrated in the above General Synthesis Route 2, compound 1 undergoes a condensation reaction with compound 2 to form compound 3. Step 1 may be carried out in an organic solvent (e.g., DCM) in the presence of EDCl and DMAP.

Then, the t-butoxy protective group of compound 3 is removed to form compound 4. Step 2 may be carried out in an organic solvent (e.g., DCM) in the presence of an acid (e.g., trifluoroacetic acid) and a cation scavenger (e.g., triisopropylsilane (TiPS)).

Subsequently, compound 4 undergoes a condensation reaction with compound 5 to form compound 6. Step 3 may be carried out in an organic solvent (e.g., DCM or DMF) in the presence of EDCl and DMAP or DCC.

If the M' group of compound 6 contains the aforementioned protective group, the protective group is removed to obtain the target lipid compound. Step 4 is carried out under the deprotection reaction condition for the selected protective group.

### General synthesis route 3

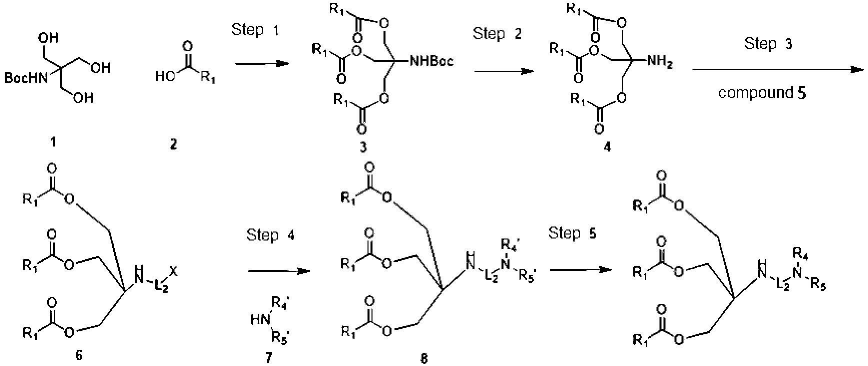

wherein compound 5 is 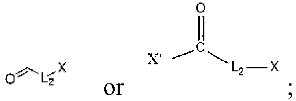 X' and X are halogens such as Cl, Br or I; R₄' is R₄ or R₄ containing a protective group; R₅' is R₅ or R₅ containing a protective group.

As illustrated in the above General Synthesis Route 3, Boc-aminotris(hydroxymethyl)methane undergoes a condensation reaction with compound 2 to form compound 3. Step 1 may be carried out in an organic solvent (e.g., DCM) in the presence of, for example, EDCl and DMAP. Step 1 may be carried out at room temperature for 24 hours.

Then, the Boc protective group of compound 3 is removed to form compound 4. Step 2 may be carried out in an organic solvent (e.g., DCM) under the catalysis of an acid (e.g., trifluoroacetic acid).

Subsequently, compound 4 undergoes a condensation and reduction reaction with a halogen-substituted aldehyde (compound 5) to form compound 6. Step 3 may be carried out in an organic solvent (e.g., 1,2-dichloroethane (DCE)) in the presence of a reducing agent (e.g., sodium triacetoxyborohydride (NaBH(OAc)₃)).

Subsequently, compound 6 undergoes a nucleophilic reaction with an amine (compound 7) to form compound 8. Step 4 may be carried out in an organic solvent (e.g., DMF) in the presence of a base (e.g., a non-nucleophilic organic base such as trimethylamine or iPr2EtN, or an inorganic base such as K₂CO₃) and a catalyst (KI or NaI).

If the R₄' and/or R₅' group of compound 8 contains the aforementioned protective group, the protective group is removed to obtain the target lipid compound. Step 5 is carried out under the deprotection reaction condition for the selected protective group.

### General synthesis route 4

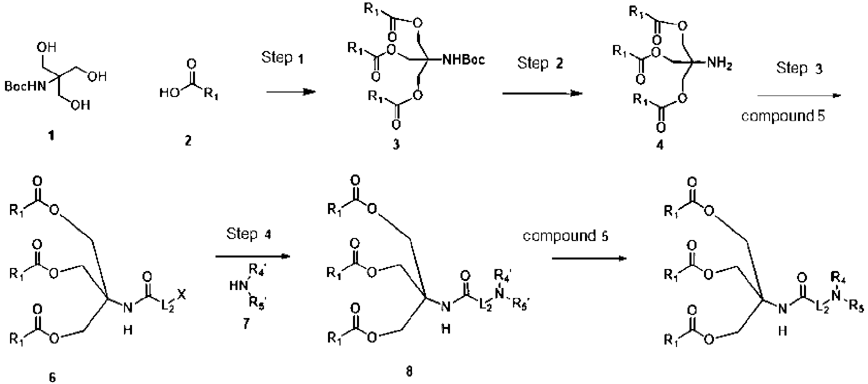

wherein compound 5 is 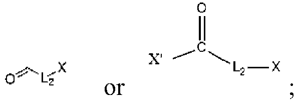 X' and X are halogens such as Cl, Br or I; R₄' is R₄ or R₄ containing a protective group; R₅' is R₅ or R₅ containing a protective group.

As illustrated in the above General Synthesis Route 4, Boc-aminotris(hydroxymethyl)methane undergoes a condensation reaction with compound 2 to form compound 3. Step 1 may be carried out in an organic solvent (e.g., DCM) in the presence of, for example, EDCl and DMAP. Step 1 may be carried out at room temperature for 24 hours.

Then, the Boc protective group of compound 3 is removed to form compound 4. Step 2 may be carried out in an organic solvent (e.g., DCM) under the catalysis of an acid (e.g., trifluoroacetic acid).

Subsequently, compound 4 undergoes a condensation reaction with a halogen-substituted compound 5 to form compound 6. Step 3 may be carried out in an organic solvent (e.g., DCM or DMF) under the catalysis of EDCl and DMAP or DCC.

Subsequently, compound 6 undergoes a nucleophilic reaction with an amine (compound 7) to form compound 8. Step 4 may be carried out in an organic solvent (e.g., DMF) in the presence of a base (e.g., a non-nucleophilic organic base such as trimethylamine or iPr2EtN, or an inorganic base such as K₂CO₃) and a catalyst (KI or NaI).

If the R₄' and/or R₅' group of compound 8 contains the aforementioned protective group, the protective group is removed to obtain the target lipid compound. Step 5 is carried out under the deprotection reaction condition for the selected protective group.

### General synthesis route 5

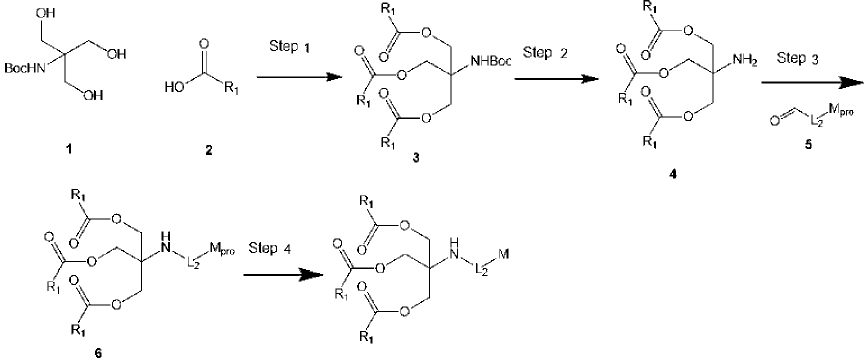

wherein Mₚᵣₒ is M or M containing a protective group.

As illustrated in the above General Synthesis Route 5, Boc-aminotris(hydroxymethyl)methane undergoes a condensation reaction with compound 2 to form compound 3. Step 1 may be carried out in an organic solvent (e.g., DCM) in the presence of, for example, EDCl and DMAP. Step 1 may be carried out at room temperature for 24 hours.

Then, the Boc protective group of compound 3 is removed to form compound 4. Step 2 may be carried out in an organic solvent (e.g., DCM) under the catalysis of an acid (e.g., trifluoroacetic acid).

Subsequently, compound 4 undergoes a condensation and reduction reaction with compound 5 to form compound 6. Step 3 may be carried out in an organic solvent (e.g., 1,2-dichloroethane (DCE)) in the presence of a reducing agent (e.g., sodium triacetoxyborohydride (NaBH(OAc)₃)).

If the Mₚᵣₒ group of compound 6 contains the aforementioned protective group, the protective group is removed to obtain the target lipid compound. Step 4 is carried out under the deprotection reaction condition for the selected protective group.

In addition, it should be understood that any specific embodiment of the present invention within the scope of the prior art may be explicitly excluded from any one or more claims. Since these embodiments are considered to be known to those of ordinary skill in the art, they may be excluded even if such exclusion is not explicitly stated herein.

All of the citation sources, such as references, publications, databases, database entries and technologies cited herein, are incorporated into the present application by reference, even if not explicitly stated in the citation. In the case of a conflict between the citation source and the statement in the present application, the statement in the present application shall prevail.

### Example 1: Synthesis of Compounds of Formulae (1), (1A), (1B), (1C), (1D), (1E), (1F), (1G), (1H), (11), (1J), (1K), (1L) or (1M)

### A. General Considerations

It should be noted that all raw materials used in the present invention are common commercially available products, and their sources are not specifically limited.

The process routes described below can be used to synthesize Compounds 1001-3422 of the present invention.

The following abbreviations are used herein:
THF: tetrahydrofuran
MeCN: acetonitrile
MeOH: methanol
PE: petroleum ether
EA: ethyl acetate
DMF: N,N-dimethylformamide
EDCl: 1-ethyl-(3-dimethylaminopropyl)carbodiimide hydrochloride
LAH: lithium aluminum hydride
DCM: dichloromethane
DMAP: 4-dimethylaminopyridine
LDA: lithium diisopropylamide
rt: room temperature
DCE: 1,2-dichloroethane
n-BuLi: n-butyllithium
i-Pr2EtN: N,N-diisopropylethylamine

### B. Synthesis of Intermediates

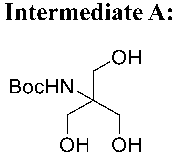

**Intermediate** A was obtained by the following synthesis process route:

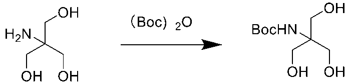

Tris(hydroxymethyl)aminomethane (50.0 g) and di-t-butyl dicarbonate (Boc₂O) (99.1 g) were dissolved in a mixed solvent of methanol (300 mL)/H₂O (30 mL), reacted at room temperature for 72 h, and purified by silica gel column chromatography (DCM:MeOH=20:1-10:1) to obtain Intermediate A (N-Boc-tris(hydroxymethyl)aminomethane) as a white solid (75.0 g). LCMS (ESI) calcd for C₉H₁₉NO₅, [M+H]⁺ m/z 222.13, found 222.25.

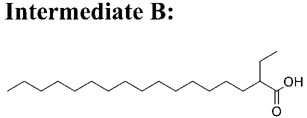

**Intermediate** B was obtained by the following synthesis process route:

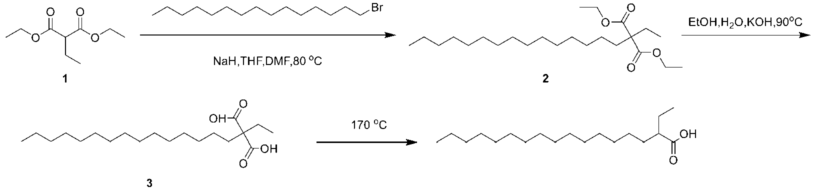

Diethyl 2-ethyl-1,3-malonate (29.0 g), THF (90 mL) and DMF (30 mL) were added to a single-neck flask, NaH (3.7 g) was added under an ice bath, stirred at room temperature for 30 min under nitrogen protection. Bromopentadecane (15.0 g) was added, reacted at 80°C for 2 h under nitrogen protection, and concentrated under reduced pressure to remove THF. The reaction solution was added dropwise to ice water, ethyl acetate was added and stirred, and filtered through celite. The organic phase was collected and subjected to liquid separation. The organic phase was washed with saturated brine. The organic phase was separated, and purified by silica gel column chromatography (PE:EA=100:1-50:1) to obtain diethyl 2-ethyl-2-pentadecylmaleate (16.5 g). EtOH (50 mL), H₂O (50 mL), and KOH (11.3 g) were added to diethyl 2-ethyl-2-pentadecylmaleate (8.0 g), reacted at 90°C for 12 h, and concentrated under reduced pressure to remove EtOH. Dilute hydrochloric acid was added to adjust the pH of the reaction system to 4-5. Water and ethyl acetate were added and stirred for liquid separation. The organic phase was washed with saturated brine. The organic phase was separated, and purified by column chromatography (PE:EA=5:1-1:1) to obtain 2-ethyl-2-pentadecylmaleic acid (5.2 g). 2-Ethyl-2-pentadecylmaleic acid (10.0 g) was reacted at 170°C for 6 h under open-vessel conditions, then cooled to room temperature. Water and ethyl acetate were added and stirred for liquid separation. The organic phase was washed with saturated brine. The organic phase was separated, concentrated, and purified by column chromatography (DCM:MeOH=20:1-10:1) to obtain Intermediate B (2-ethylheptadecanoic acid) (8.2 g). 1H NMR (400 MHz, CDCl₃) δ2.31 (tt, J=8.6, 5.3 Hz, 1H), 1.71-1.46 (m, 4H), 1.28 (s, 26H), 0.96 (t, J=7.4 Hz, 3H), 0.90 (t, J=6.7 Hz, 3H).

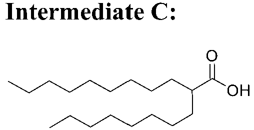

**Intermediate** C was obtained by the following synthesis process route:

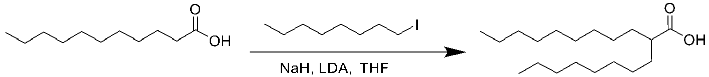

Decanoic acid (50 g) and THF solvent (500 mL) were added to a three-neck flask. The system was cooled to 0°C, then a reactant NaH (23.22 g) was slowly added, stirred at 0°C for 1 h under nitrogen protection, then LDA (62.19 g) was slowly added dropwise, and the stirring was continued at 0°C for 1 h under nitrogen protection. Finally, a reactant 1-iodononane (88.52 g) was added dropwise, then warmed to room temperature and stirred overnight. After that, the resultant was diluted with 1 L DCM, and washed with saturated NH₄Cl solution and water, respectively. The organic layer was dried over anhydrous Na₂SO₄, filtered and concentrated, then purified by silica gel column chromatography (PE:EA=50:1) to finally obtain Intermediate C (2-octylundecanoic acid) (11 g). 1H NMR (400 MHz, Chloroform-d) δ2.37 (tt, J=8.7, 5.1 Hz, 1H), 1.71-1.58 (m, 2H), 1.48 (dt, J=13.3, 6.7 Hz, 2H), 1.29 (d, J=9.4 Hz, 25H), 0.90 (t, J=6.8 Hz, 6H).

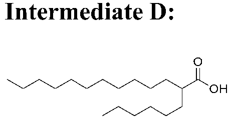

**Intermediate D** was obtained by the following synthesis process route:

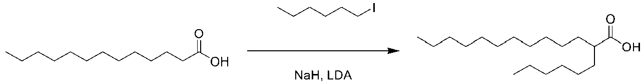

Tridecanoic acid (79.5 g) and THF solvent (800 mL) were added to a three-neck flask. The system was cooled to 0°C, then NaH (22.25) was slowly added. The reaction solution was stirred at 0°C for 1 h under nitrogen protection, then LDA (317.87 g) was slowly added dropwise to the reaction system. The reaction solution was stirred again at 0°C for 1 h under nitrogen protection. A reactant iodo-n-hexane (94.39) was added dropwise, then the mixture was warmed to room temperature and stirred overnight. The reaction solution was diluted with 1 L DCM, and washed with saturated NH₄Cl solution and water, respectively. The organic layer was dried over anhydrous Na₂SO₄, filtered and concentrated, then purified by silica gel column chromatography (PE:EA=50:1) to obtain Intermediate D (2-hexyltridecanoic acid) (26 g). 1H NMR (400 MHz, Chloroform-d) δ2.36 (tt, J=8.7, 5.4 Hz, 1H), 1.63 (ddd, J=14.3, 8.7, 5.5 Hz, 2H), 1.49 (dq, J=13.5, 6.6 Hz, 2H), 1.29 (d, J=10.1 Hz, 26H), 0.90 (t, J=6.6 Hz, 6H).

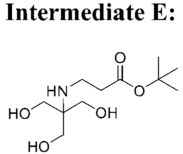

**Intermediate E** was obtained by the following synthesis process route:

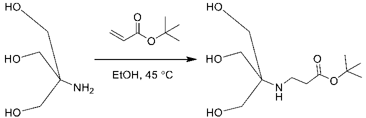

Tris(hydroxymethyl)aminomethane (10.0 g) and t-Butyl acrylate (21.1) were dissolved in EtOH (150 mL), reacted at 45°C for 30 h under nitrogen protection, then concentrated under reduced pressure to remove EtOH. 100 mL × 3 of (PE:EA=20:1) solvent was added for trituration, filtered to obtain Intermediate E as a white solid (11.5 g). 1H NMR (400 MHz, CDCl₃) δ3.60 (s,6H), 2.84 (t, J=5.9 Hz, 2H), 2.47 (t, J=5.8 Hz, 2H), 1.48 (s, 9H).

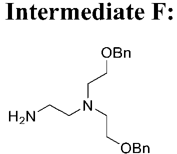

**Intermediate** F was obtained by the following synthesis process route:

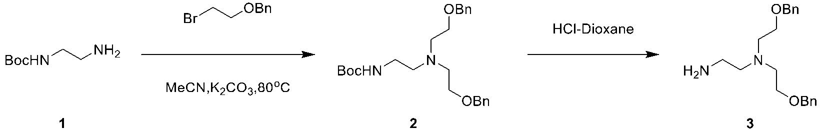

t-Butyl (2-aminoethyl)carbamate (2.7 g), MeCN (90 mL), Benzyl 2-bromoethyl ether (7.99 g) and K₂CO₃ (11.65 g) were added to a single-neck flask, reacted at 80°C overnight. Then, water and ethyl acetate were added and stirred for liquid separation. The organic phase was washed with saturated brine. The organic phase was separated, concentrated, and purified by silica gel column chromatography (PE:EA=10:1-5:1) to obtain Compound 2 (5.5 g). Dioxane (30 mL) and 4 M HCl in dioxane (30 mL) were added to Compound 2, stirred at room temperature for 3 h, then concentrated under reduced pressure to obtain Compound 3, that is, Intermediate F (6 g). LCMS (ESI) calcd for C₂₀H₂₈N₂O₂, [M+H]⁺ m/z 329.22, found 329.24.

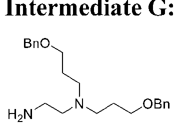

**Intermediate G** was obtained by the following synthesis process route:

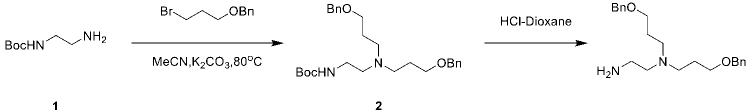

Compound 1 (3.0 g), MeCN (90 mL), 3-benzyloxy-1-bromopropane (9.45 g) and K₂CO₃ (12.94 g) were added to a single-neck flask, reacted at 80°C overnight. Then, water and ethyl acetate were added and stirred for liquid separation. The organic phase was washed with saturated brine. The organic phase was separated, concentrated, and purified by column chromatography (PE:EA=10:1-5:1) to obtain Compound 2 (6.9 g). Dioxane (30 mL) and 4 M HCl in dioxane (30 mL) were added to Compound 2 (6.9 g), stirred at room temperature for 3 h, then concentrated under reduced pressure to obtain Intermediate G (7.5 g). LCMS (ESI) calcd for C₂₂H₃₂N₂O₂, [M+H]⁺ m/z 357.25, found 357.51.

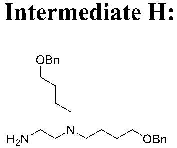

**Intermediate** H was obtained by the following synthesis process route:

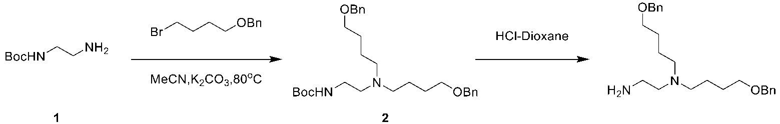

Compound 1 (1.5 g), MeCN (90 mL), Benzyl 4-bromobutyl ether (5.02 g) and K₂CO₃ (6.47 g) were added to a single-neck flask, reacted at 80°C overnight. Then, water and ethyl acetate were added and stirred for liquid separation. The organic phase was washed with saturated brine. The organic phase was separated, concentrated, and purified by column chromatography (PE:EA=20:1-10:1) to obtain Compound 2 (4.1 g). Dioxane (30 mL) and 4 M HCl in dioxane (30 mL) were added to Compound 2 (4.1 g), stirred at room temperature for 3 h, then concentrated under reduced pressure to obtain Intermediate H (4.9 g). LCMS (ESI) calcd for C₂₄H₃₆N₂O₂, [M+H]⁺ m/z 385.28, found 385.56.

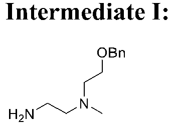

**Intermediate I** was obtained by the following synthesis process route:

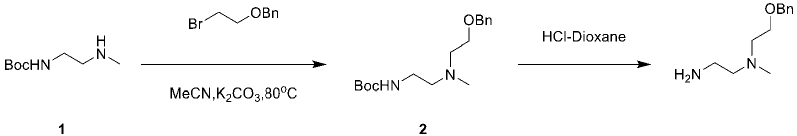

Compound 1 (3.9 g), MeCN (90 mL), Benzyl 2-bromoethyl ether (5.78 g) and K₂CO₃ (15.46 g) were added to a single-neck flask, reacted at 80°C overnight. Then, water and ethyl acetate were added and stirred for liquid separation. The organic phase was washed with saturated brine. The organic phase was separated, concentrated, and purified by column chromatography (PE:EA=20:1-10:1) to obtain Compound 2 (6.8 g). Dioxane (30 mL) and 4 M HCl in dioxane (30 mL) were added to Compound 2 (6.8 g), stirred at room temperature for 3 h, then concentrated under reduced pressure to obtain Intermediate I (6.5 g). LCMS (ESI) calcd for C₁₂H₂₀N₂O, [M+H]⁺ m/z 209.16, found 209.31.

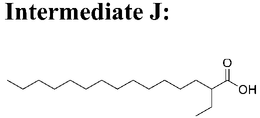

**Intermediate J** was obtained by the following synthesis process route:

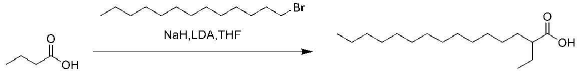

Butyric acid (5.0 g) and THF (100 mL) were added to a single-neck flask. NaH (2.73 g) was added at 0°C. LDA (56.8 ml) was slowly added dropwise, reacted at room temperature for 30 min, 1-Bromotridecane was added, continued to react at room temperature overnight. Then, ice water and ethyl acetate were added and stirred for liquid separation. The organic phase was washed with saturated brine. The organic phase was separated, concentrated, and purified by column chromatography (PE:EA=10:1-5:1) to obtain Intermediate J (5.0 g). LCMS (ESI) calcd for C₁₇H₃₄O₂, [M+H]⁺ m/z 271.26, found 271.46.

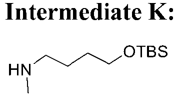

**Intermediate K** was obtained by the following synthesis process route:

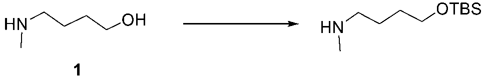

Reactant 1 (24 g), Imidazole (19.00 g) and DCM solvent (200 mL) were added to a three-neck flask, cooled to 0°C, then a reactant TBDMSCl (38.57 g) was slowly added, stirred at 25°C for 4 h, and diluted with 300 mL DCM. The organic phase was washed twice with 1 L water, then dried over anhydrous Na₂SO₄, filtered and concentrated. Silica gel was added, and column chromatograph was performed with DCM:MeOH=1:20. The sample was collected and concentrated to obtain Intermediate K (28.00 g). 1H NMR (400 MHz, CDCl₃) δ4.89 (s, 1H), 3.58 (t, J=6.1 Hz, 2H), 2.66 (t, J=7.3 Hz, 2H), 2.45 (s, 3H), 1.67-1.40 (m, 4H), 0.84 (s, 9H).

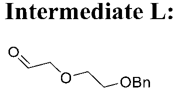

**Intermediate L** was obtained by the following synthesis process route:

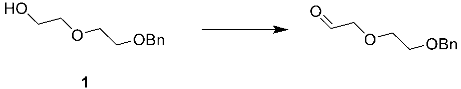

DMSO (3.58 g) and anhydrous DCM solvent (30 mL) were added to a three-neck flask. The system was cooled to -78°C, then oxalyl chloride (2.91 g) was slowly added. The reaction solution was stirred at -78°C for 10 min under N₂ protection, then Compound 1 (3 g) was slowly added dropwise. Stirring was continued at -78°C for 1 h under N₂ protection. Finally, TEA (9.28 g) was added dropwise. Stirring was continued at -78°C for 0.5 h under N₂ protection, then the resultant was diluted with 100 mL DCM, and washed with saturated NH₄Cl solution and water, respectively. The organic layer was dried over anhydrous Na₂SO₄, filtered and concentrated to obtain Intermediate L (3.0 g). LCMS (ESI) calcd for C₁₁H₁₄O₃, [M+H]⁺ m/z 195.09, found 195.23.

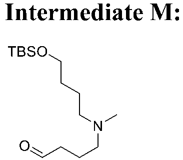

**Intermediate M** was obtained by the following synthesis process route:

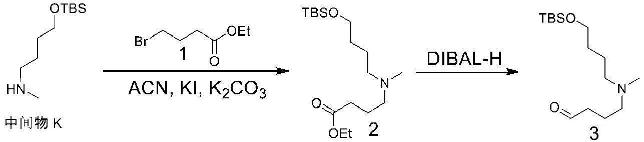

Intermediate K (2.0 g), Compound 1 (1.79 g) and ACN solvent (20 mL) were added to a sealed tube. Reactants K₂CO₃ (3.81 g) and KI (1.52 g) were added, stirred at 70°C overnight under N₂ protection, then diluted with 100 mL EA, and washed with saturated NH₄Cl solution and water, respectively. The organic layer was dried over anhydrous Na₂SO₄, filtered and concentrated. Silica gel was added, and column chromatograph was performed with DCM:MeOH=15:1. The sample was collected and concentrated to obtain Compound 2 (2.0 g). The reactant 2 (1.80 g) and DCM solvent (20 mL) were added to a three-neck flask. The system was purged with nitrogen and cooled to -78°C. After that, DIBAL-H (1.00 g) was added dropwise, stirred at -78°C for 4 h, then quenched by dropwise addition of methanol and sodium carbonate solution into the reaction solution, subsequently extracted with DCM (100 mL). The organic layer was dried over anhydrous sodium sulfate, filtered and concentrated to obtain Intermediate M (1.36 g). LCMS (ESI) calcd for C₁₅H₃₃NO₂Si, [M+H]⁺ m/z 288.52, found 288.23.

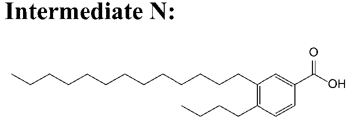

**Intermediate** N was obtained by the following synthesis process route:

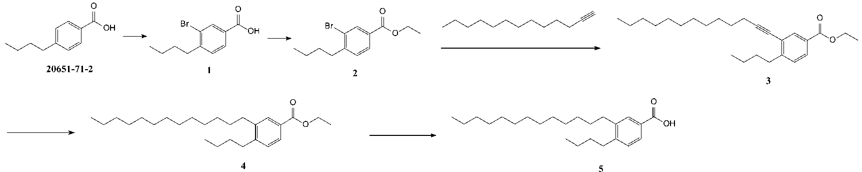

Reactant 20651-71-2 (20 g) was added to a single-neck flask. Concentrated sulfuric acid (350 mL) was added and stirred to dissolve. Then, NBS (22 g) was added and stirred to mix, reacted at room temperature overnight. The reaction solution was slowly poured into 1 L water to dilute. The aqueous phase was extracted twice with 1 L ethyl acetate, dried over anhydrous Na₂SO₄, filtered and concentrated to obtain Product 1 (28 g). 1H NMR (400 MHz, CDCl₃) δ8.29 (d, J=1.7 Hz, 1H), 7.98 (dd, J=8.0, 1.8 Hz, 1H), 7.34 (d, J=8.0 Hz, 1H), 2.87-2.78 (m, 2H), 1.64 (tt, J=7.8, 6.4 Hz, 2H), 1.43 (dp, J=13.4, 6.8 Hz, 2H), 0.99 (t, J=7.3 Hz, 3H).

Product 1 (28 g) was added to a single-neck flask. Ethanol (400 mL) was added and stirred to dissolve. Then, concentrated sulfuric acid (200 mL) was slowly added and stirred to mix. The reaction solution was stirred under reflux at 85°C for 18 h. The reaction solution was slowly poured into 1 L water to dilute. The aqueous phase was extracted twice with 1 L ethyl acetate, dried over anhydrous Na₂SO₄, filtered and concentrated to obtain Product 2 (30 g). 1H NMR (400 MHz, CDCl₃) δ8.22 (d, J=1.8 Hz, 1H), 7.91 (dd, J=7.9, 1.7 Hz, 1H), 7.30 (s, 1H), 4.39 (q, J=7.1 Hz, 2H), 2.85-2.74 (m, 2H), 1.62 (tt, J=7.9, 6.4 Hz, 2H), 1.42 (dt, J=9.7,7.3 Hz, 4H), 0.98 (t, J=7.3 Hz, 3H).

Product 2 (2.4 g) and triphenylphosphine (0.66 g) were added respectively to a 25 mL three-neck flask under nitrogen protection. Anhydrous DMF (20 mL) was added and stirred to dissolve. Then, palladium acetate (0.19 g) and copper(I) iodide (0.27 g) were added respectively and stirred to mix. Finally, triethylamine (4.26 g) and 26186-02-7 (1.82 g) were added. The reaction solution was heated to 90°C and reacted overnight, cooled to room temperature, slowly poured into 200 L water to dilute. The aqueous phase was extracted twice with 1 L ethyl acetate, dried over anhydrous Na₂SO₄, filtered and concentrated, and purified by silica gel column chromatography (PE:EA=50:1) to obtain Product 3 (2.5 g). Product 3 (3.8 g) and Pd/C (0.66 g) were added respectively to a three-neck flask under hydrogen protection. MeOH (20 mL) and EA (20 mL) were added and stirred to mix, then stirred at room temperature for 4 h, filtered and concentrated to obtain crude Compound 4 (3.7 g). Crude Compound 4 (3.9 g) was added to a single-neck flask. EtOH (20 mL) was added and stirred to dissolve. Then, 1M NaOH aqueous solution (20 mL) was added. The reaction solution was stirred at room temperature for 2 h. 1M dilute hydrochloric acid was slowly added dropwise to adjust the pH of the reaction solution to 6-7, then extracted twice with ethyl acetate, dried over anhydrous Na₂SO₄, filtered and concentrated, and purified by silica gel column chromatography (PE:EA=10:1) to obtain Intermediate N (1.5 g). 1H NMR (400 MHz, CDCl₃) δ7.93 (d, J=2.0 Hz, 1H), 7.89 (dd, J=7.9,1.8 Hz, 1H), 7.26 (d, J=8.0 Hz, 1H), 2.69 (q, J=7.3 Hz, 4H), 1.70-1.55 (m,4H), 1.45 (h,J=7.3 Hz, 4H), 1.30 (s,18H), 0.99 (t, J=7.3 Hz, 3H), 0.91 (t, J=6.7 Hz, 3H).

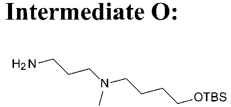

**Intermediate O** was obtained by the following synthesis process route:

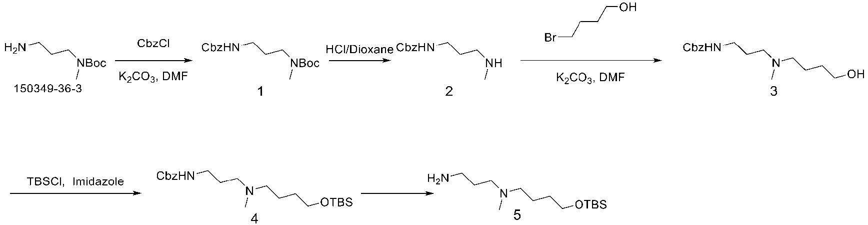

Reactant 150349-36-3 (3 g) was added to a single-neck flask. DMF (20 mL) was added and stirred to dissolve. Then, potassium carbonate (4.4 g) was added and stirred to mix. The reaction system was cooled to 0°C. Then, a reactant CbzCl (3.3 g) was added, warmed to room temperature and reacted overnight, and then diluted with 100 mL DCM. The organic phase was washed with water, dried, filtered and concentrated. Silica gel was added, and column chromatograph was performed with DCM:MeOH=20:1. The sample was collected and concentrated to obtain Product 1 (3.8 g). LCMS (ESI) calcd for C₁₇H₂₆N₂O₄, [M+Na]+m/z 345.18, found 345.22.

Reactant 1 (3.7 g) and DCM solvent (20 mL) were added to a three-neck flask, stirred to mix. Then, a reactant HCl in dioxane (4 M) (28.7 mL) was added and stirred at 25°C overnight. After diluted with DCM (100 mL), the organic phase was washed with saturated NaHCO₃ solution and water, then dried over anhydrous Na₂SO₄, filtered and concentrated to obtain Product 2 (2.4 g). LCMS (ESI) calcd for C₁₂H₁₈N₂O₂, [M+H]⁺ m/z 223.15, found 223.14+.

Product 2 (2.3 g) was added to a single-neck flask. DMF (20 mL) was added and stirred to dissolve. Then, potassium carbonate (2.9 g) was added and stirred to mix. Then, a reactant 4-bromo-1-butanol (2.1 g) was added, reacted at room temperature overnight. After diluted with 100 mL DCM, the organic phase was washed with water, dried over anhydrous Na₂SO₄, filtered and concentrated. Silica gel was added, and column chromatograph was performed with DCM:MeOH=20:1. The sample was collected and concentrated to obtain Product 3 (1.2 g). LCMS (ESI) calcd for C₁₆H₂₆N₂O₃, [M+H]⁺ m/z 295.20, found 295.20.

Product 3 (1.1 g) was added to a single-neck flask. DCM (20 mL) was added and stirred to dissolve. Imidazole (0.32 g) was added and stirred to mix. The reaction system was cooled to 0°C, then a reactant TBSCl (0.89 g) was added. The reaction solution was slowly warmed to room temperature, reacted for 4 h, and diluted with 100 mL DCM, then the organic phase was washed with water, dried over anhydrous Na₂SO₄, filtered and concentrated. Silica gel was added, and column chromatograph was performed with DCM:MeOH=20:1. The sample was collected and concentrated to obtain Product 4 (1.2 g). LCMS (ESI) calcd for C₂₂H₄₀N₂O₃Si, [M+H]⁺ m/z 409.29, found 409.30.

Product 4 (1.1 g) was added to a three-neck flask under hydrogen protection, MeOH (20 mL) was added and stirred to dissolve. Pd/C (0.89 g) was added and stirred to mix, reacted at room temperature overnight under hydrogen atmosphere and filtered. The filtrate was concentrated to obtain Intermediate O (0.8 g). LCMS (ESI) calcd for C₁₄H₃₄N₂OSi, [M+H]⁺ m/z 275.25, found 275.26+.

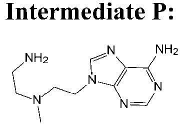

**Intermediate P** was obtained by the following synthesis process route:

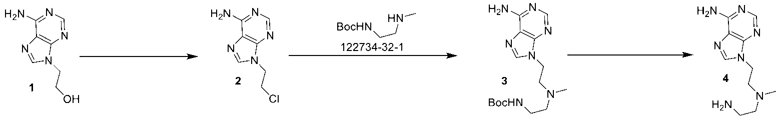

At room temperature, Compound 1 (5.3 g) was added to a three-neck flask under nitrogen protection. Anhydrous dioxane (100 mL) was added and stirred to mix. Then pyridine (0.24 mL) was added and stirred to mix. SOCl₂ (4.3 mL) was added, reacted at 100°C for 3 h, and cooled to room temperature. The solid was removed by filtration. The filtrate was rinsed twice with DCM, concentrated and purified to obtain Product 2 (5 g). LCMS (ESI) calcd for C₇H₈ClN₅, ⁺[M+H]⁺ m/z 198.06, found 198.1. Product 2 (0.8 g), 122734-32-1 (15.3 g) and K₂CO₃ (12.2 g) were added to a single-neck flask. Then, Dioxane (100 mL) was added and stirred to mix, reacted at 100°C overnight. The reaction solution was cooled to room temperature, filtered, concentrated and purified to obtain Product 3 (5 g). LCMS (ESI) calcd for C₁₅H₂₅N₇O₂, ⁺[M+H]⁺m/z 336.21, found 336.2. Product 3 (1 g) and 4 M HCl in dioxane (7.5 mL) were added to a single-neck flask and stirred to mix, reacted at room temperature overnight. The resultant was concentrated and purified to obtain Intermediate P (0.3 g). LCMS (ESI) calcd for C₁₀H₁₇N₇, ⁺[M+H]⁺ m/z 236.16, found 236.1.

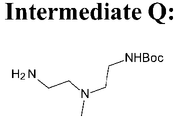

**Intermediate Q** was obtained by the following synthesis process route:

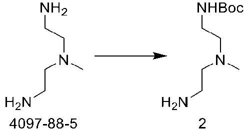

At 0°C, compound 4097-88-5 (5 g) was added to a single-neck flask. DCM (100 mL) was added and stirred to mix, then di-t-Butyl dicarbonate (0.24 mL) was added and stirred to mix, reacted at room temperature overnight. After diluted with DCM, water was added and stirred for liquid separation. The aqueous phase was extracted twice with DCM. The organic phase was dried over anhydrous sodium sulfate, concentrated and purified (DCM:MeOH=10:1) to obtain Intermediate Q (4 g). LCMS (ESI) calcd for C₁₀H₂₃N₃O₂, ⁺[M+H]⁺ m/z 218.19, found 218.2.

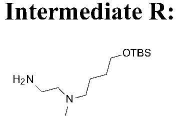

**Intermediate R** was obtained by the following synthesis process route:

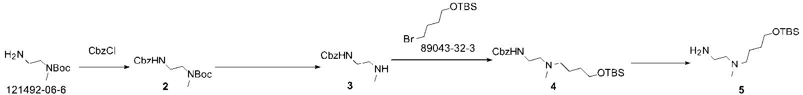

121492-06-6 (5.0 g), CbzCl (5.87 g) and DMF solvent (50 mL) were added to a single-neck flask and stirred to mix. Then, a reactant K₂CO₃ (5.95 g) was added, stirred at room temperature overnight. After that, the resultant diluted with 100 mL EA, and washed with saturated NH₄Cl solution and water, respectively. The organic layer was dried over anhydrous Na₂SO₄, filtered and concentrated. Silica gel was added, and column chromatograph was performed with DCM:MeOH=20:1. The sample was collected and concentrated to obtain Product 2 (5.1 g). Product 2 (5 g) and DCM (50 mL) were added to a single-neck flask and stirred to dissolve. Then, 4 M HCl in dioxane (40 mL) was added, reacted at room temperature overnight. The solvent was removed by concentration to obtain Product 3 (4 g). Product 3 (1.05 g), 89043-32-3 (0.9 g) and DMF solvent (10 mL) were added to a single-neck flask and stirred to mix. A reactant K₂CO₃ (2.3 g) was added, stirred at room temperature overnight. After that, the resultant was diluted with 100 mL EA, and washed with saturated NH₄Cl solution and water, respectively. The organic layer was dried over anhydrous Na₂SO₄, filtered and concentrated. Silica gel was added, and column chromatograph was performed with DCM:MeOH=20:1. The sample was collected and concentrated to obtain Product 4 (1.1 g). Product 4 (1 g) and MeOH (10 mL) were added to a single-neck flask and stirred to mix. Pd/C was added and stirred to mix. The reaction system was reacted for 2 h under hydrogen atmosphere, and filtered through celite. The filtrate was concentrated to obtain Intermediate R (600 mg).

### C. Compound 1003, Synthesized According to General Synthesis Route 1

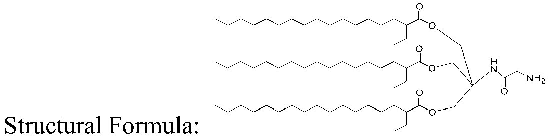

Molecular Formula: C₆₃H₁₂₂N₂O₇
Molecular Weight: 1019.68

### Step 1: Synthesis of Compound 3 in General Synthesis Route 1

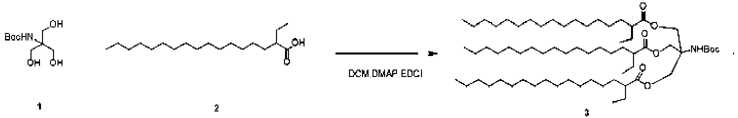

Compound 1 (Intermediate A) (1.0 g), DCM (20 mL), DMAP (2.2 g) and EDCI (3.4 g) were added to Compound 2 (Intermediate B) (4.3 g), stirred at room temperature for 12 h under nitrogen protection. Then, water and dichloromethane were added and stirred for liquid separation. The organic phase was washed with saturated brine and separated. The organic phase was dried over anhydrous Na₂SO₄, concentrated, and purified by silica gel column chromatography (PE:EA=40:1-30:1) to obtain Compound 3 (2.6 g).

### Step 2: Synthesis of Compound 4 in General Synthesis Route 1

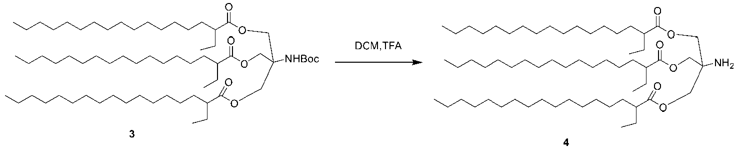

DCM (15 mL) and TFA (5 mL) were added to Compound 3 (2.6 g), stirred at room temperature for 3 h, then concentrated, and purified by silica gel column chromatography (PE:EA=10:1-5:1) to obtain Compound 4 (3.2 g).

### Step 3: Synthesis of Compound 6 in General Synthesis Route 1

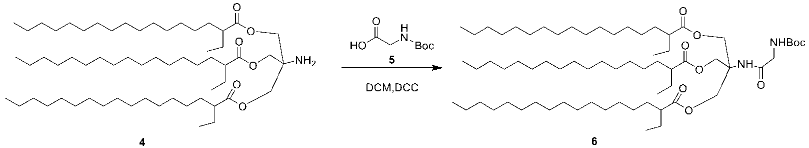

DCM (20 mL), Boc-glycine (Compound 5, 578 mg) and DCC (6.8 g) were added to Compound 4 (3.2 g), stirred at room temperature for 12 h under nitrogen protection. Then, water and ethyl acetate were added and stirred for liquid separation. The organic phase was washed with saturated brine and separated. The organic phase was dried over anhydrous Na₂SO₄, concentrated, and purified by column chromatography (PE:EA=5:1-2:1) to obtain Compound 6 (2.2 g).

### Step 4: Compound 1003

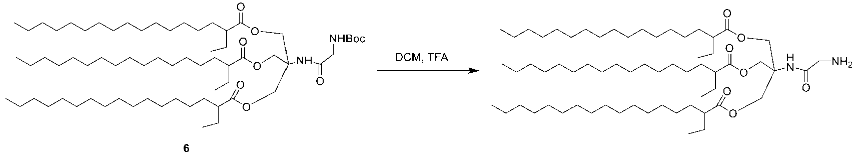

DCM (15 mL) and TFA (5 mL) were added to Compound 6 (2.2 g), stirred at room temperature for 12 h, then concentrated, and purified by column chromatography (DCM:MeOH=30:1-20:1) to obtain Compound 1003 (1.3 g). ¹H NMR (400 MHz, CDCl₃) δ4.49 (d, J=2.6 Hz, 6H), 3.28 (s, 2H), 2.32 (tt, J=8.6, 5.5 Hz, 3H), 1.62 (q, J=7.1 Hz, 12H), 1.27 (d, J=3.4 Hz, 78H), 0.94-0.86 (m, 18H).

### D. Compound 1002, Synthesized According to General Synthesis Route 1

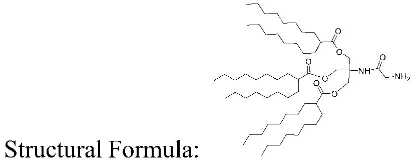

Molecular Formula: C₆₀H₁₁₆N₂O₇
Molecular Weight: 977.60

Synthesized according to General Synthesis Route 1. The synthesis process was similar to that of Compound 1003, except that Intermediate C was used as Compound 2 in General Synthesis Route 1. ¹H NMR (400 MHz, CDCl₃) δ4.48 (s, 6H), 3.27 (s, 2H), 2.37 (tt, J=8.5, 5.5 Hz, 3H), 1.64 (d, J=6.9 Hz, 6H), 1.50-1.42 (m, 6H), 1.27 (s, 72H), 0.90 (t, J=6.7 Hz, 18H).

### E. Compound 1004, Synthesized According to General Synthesis Route 1

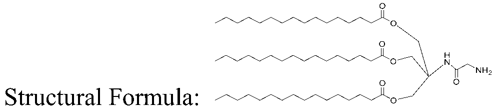

Molecular Formula: C₅₄H₁₀₄N₂O₇
Molecular Weight: 893.43

Synthesized according to General Synthesis Route 1. The synthesis process was similar to that of Compound 1003, except that palmitic acid was used as Compound 2 in General Synthesis Route 1. ¹H NMR (400 MHz, CDCl₃) δ7.55 (s, 1H), 4.45 (s, 6H), 3.74 (s, 2H), 2.35 (t, J=7.6 Hz, 6H), 1.60 (p, J=6.9 Hz, 6H), 1.27 (s, 72H), 0.90 (t, J=6.7 Hz, 9H).

### F. Compound 1001, Synthesized According to General Synthesis Route 1

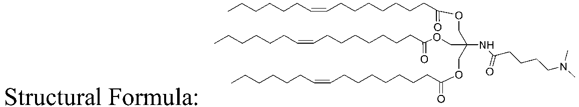

Molecular Formula: C₅₉H₁₀₈N₂O₇
Molecular Weight: 957.52

Synthesized according to General Synthesis Route 1. The synthesis process was similar to that of Compound 1003, except that (9Z)-9-hexadecenoic acid was used as Compound 2 in General Synthesis Route 1, and 5-(N,N-dimethylamino)pentanoic acid was used as Compound 5 in General Synthesis Route 1. ¹H NMR (400 MHz, CDCl₃) δ5.42-5.30 (m, 6H), 4.51-4.38 (m, 6H), 3.19 (dt, J=11.3, 5.6 Hz, 2H), 2.97 (t, J=5.9 Hz, 6H), 2.46 (t, J=6.6 Hz, 1H), 2.35 (td, J=7.6, 3.1 Hz, 5H), 2.20 (t, J=7.6 Hz, 2H), 2.03 (d, J=6.2 Hz, 12H), 1.89-1.81 (m, 3H), 1.74 (q, J=7.8 Hz, 2H), 1.67-1.51 (m, 6H), 1.31 (d, J=7.8 Hz, 48H), 0.90 (t, J=6.7 Hz, 9H).

### G. Compound 1014, Synthesized According to General Synthesis Route 2

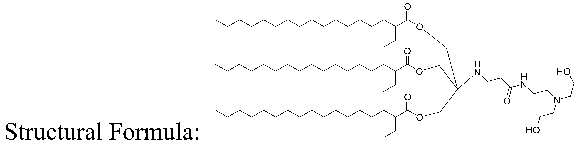

Molecular Formula: C₇₀H₁₃₇N₃O₉
Molecular Weight: 1164.88

### Step 1: Synthesis of Intermediate 3 in General Synthesis Route 2

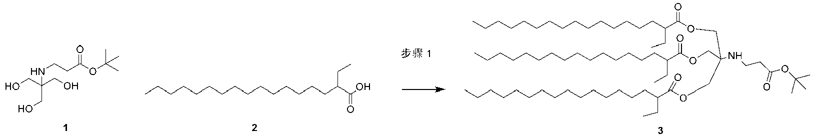

Intermediate 3 was synthesized according to the method shown in Step 1 of General Synthesis Route 1, except that Intermediate E was used as Compound 1 in General Synthesis Route 1.

### Step 2: Synthesis of Compound 4 in General Synthesis Route 2

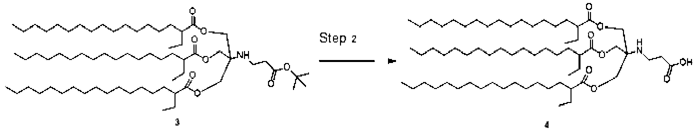

DCM (15 mL), TFA (12 mL) and TiPS (3 mL) were added to Compound 3 (4.8 g), reacted at room temperature for 12 h. Then, water and dichloromethane were added and stirred for liquid separation. The organic phase was washed with saturated brine. The organic phase was separated, concentrated, and purified by silica gel column chromatography (DCM:MeOH=20:1-10:1) to obtain Compound 4 (3.3 g).

### Step 3: Synthesis of Compound 6 in General Synthesis Route 2

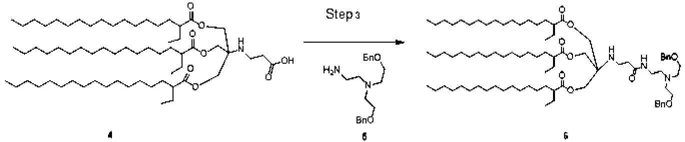

Compound 4 (4.0 g), DCM (80 mL), EDCI (1.1 g), 1-hydroxybenzotriazole (HOBt) (0.78 g) and DIEA (2.5 g) were added to Compound 5 (1.9 g), stirred at room temperature for 12 h. Then, water and dichloromethane were added and stirred for liquid separation. The organic phase was washed with saturated brine. The organic phase was separated, concentrated, and purified by silica gel column chromatography (DCM:MeOH=30:1-20:1) to obtain Compound 6 (4.0 g).

### Step 4: Synthesis of Compound 1014

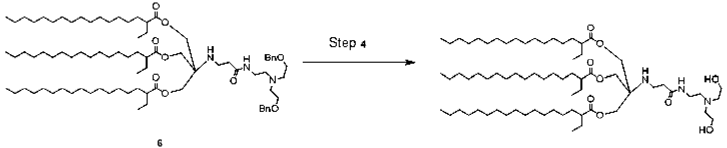

MeOH (30 mL), DCM (10 mL) and Pd/C (4.73 g (10%)) were added to Compound 6 (4.0 g), stirred at room temperature overnight under hydrogen atmosphere. Then, the resultant was filtered through celite, concentrated, and purified by silica gel column chromatography (DCM:MeOH=30:1-15:1) to obtain Compound 1014 (2 g). 1H (400 MHz, CDCl₃) δ7.38 (t, J=5.8 Hz, 1H), 4.14-4.05 (m, 6H), 3.58 (t, J=4.9 Hz, 4H), 3.31 (q, J=5.7 Hz, 2H), 2.89 (t, J=6.3 Hz, 2H), 2.64 (q, J=5.2 Hz, 6H), 2.29 (dq, J=10.8, 4.4 Hz, 6H), 1.66-1.37 (m, 12H), 1.24 (d, J=4.3 Hz, 78H), 0.87 (t, J=7.1 Hz, 18H).

### H. Compound 1015, Synthesized According to General Synthesis Route 2

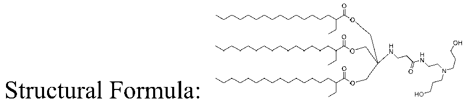

Molecular Formula: C₇₂H₁₄₁N₃O₉
Molecular Weight: 1192.93

Synthesized according to General Synthesis Route 2. The synthesis process was similar to that of Compound 1014, except that Intermediate G was used as Compound 5 in General Synthesis Route 2. 1H (400 MHz, CDCl₃) δ7.58 (t, J=5.7 Hz, 1H), 4.15-4.01 (m, 6H), 3.70 (t, J=5.4 Hz, 4H), 3.39 (q, J=5.7 Hz, 2H), 2.86 (t, J=6.2 Hz, 2H), 2.57 (dt, J=20.5, 5.8 Hz, 6H), 2.36-2.22 (m, 5H), 1.76-1.67 (m, 4H), 1.62-1.35 (m, 13H), 1.23 (d, J=4.6 Hz, 78H), 0.92-0.80 (m, 18H).

### I. Compound 1020, Synthesized According to General Synthesis Route 2

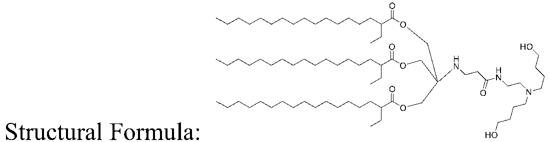

Molecular Formula: C₇₄H₁₄₅N₃O₉
Molecular Weight: 1220.99

Synthesized according to General Synthesis Route 2. The synthesis process was similar to that of Compound 1014, except that Intermediate H was used as Compound 5 in General Synthesis Route 2.

### J. Compound 1025, Synthesized According to General Synthesis Route 2

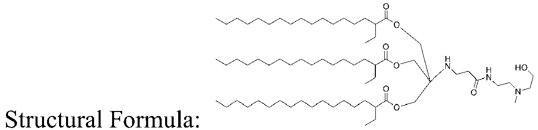

Molecular Formula: C₆₉H₁₃₅N₃O₈
Molecular Weight: 1134.85

Synthesized according to General Synthesis Route 2. The synthesis process was similar to that of Compound 1014, except that Intermediate I was used as Compound 5 in General Synthesis Route 2. 1H (400 MHz, CDCl₃) δ6.98 (t, J=5.7 Hz, 1H), 4.10 (m, J=3.8 Hz, 6H), 3.63 (t, J=5.2 Hz, 2H), 3.35 (q, J=5.8 Hz, 2H), 2.89 (t, J=6.1 Hz, 2H), 2.57 (q, J=5.3 Hz, 4H), 2.32 (s, 3H), 2.28 (q, J=4.1 Hz, 5H), 1.65-1.41 (m, 13H), 1.24 (d, J=4.4 Hz, 78H), 0.87 (t, J=7.1 Hz, 18H).

### K. Compound 1005, Synthesized According to General Synthesis Route 2

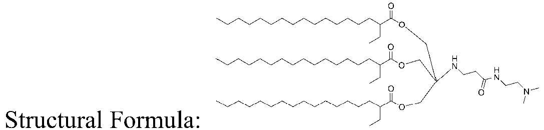

Molecular Formula:C₆₈H₁₃₃N₃O₇
Molecular Weight: 1104.83

Synthesized according to General Synthesis Route 2. The synthesis process was similar to that of Compound 1014, except that N,N-dimethylethylenediamine was used as Compound 5 in General Synthesis Route 2. ¹H NMR (400 MHz, CDCl₃) δ4.19-4.07 (m, 6H), 3.40 (d, J=5.7 Hz, 2H), 2.95-2.87 (m, 2H), 2.82 (s, 4H), 2.64-2.55 (m, 2H), 2.38 (s, 6H), 2.33-2.29 (m, 3H), 1.63-1.55 (m, 6H), 1.49-1.44 (m, 2H), 1.26 (d, J=5.4 Hz, 78H), 0.89 (td, J=7.0, 2.8 Hz, 18H).

### L. Compound 1006, Synthesized According to General Synthesis Route 2

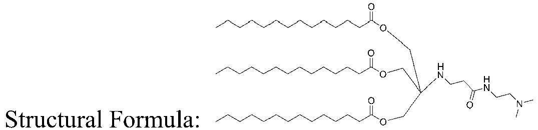

Molecular Formula: C₅₃H₁₀₃N₃O₇
Molecular Weight: 894.42

Synthesized according to General Synthesis Route 2. The synthesis process was similar to that of Compound 1014, except that tetradecanoic acid was used as Compound 2 in General Synthesis Route 2, and N,N-dimethylethylenediamine was used as Compound 5 in General Synthesis Route 2. ¹H NMR (400 MHz, CDCl₃) δ7.99 (t, J=5.8 Hz, 1H), 4.13 (s, 6H), 3.60 (q, J=5.3 Hz, 2H), 3.38-3.34 (m, 2H), 3.00 (s, 6H), 2.94 (t, J=5.8 Hz, 2H), 2.45 (t, J=5.8 Hz, 2H), 2.35 (t, J=7.6 Hz, 6H), 1.66-1.56 (m, 6H), 1.33-1.23 (m, 60H), 0.90 (t, J=6.8 Hz, 9H).

### M. Compound 1007, Synthesized According to General Synthesis Route 2

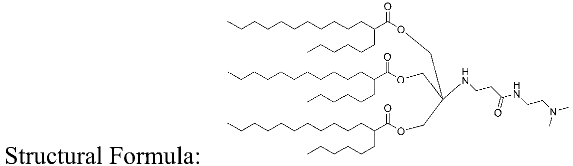

Molecular Formula: C₆₈H₁₃₃N₃O₇
Molecular Weight: 1104.83

Synthesized according to General Synthesis Route 2. The synthesis process was similar to that of Compound 1014, except that Intermediate D was used as Compound 2 in General Synthesis Route 2, and N,N-dimethylethylenediamine was used as Compound 5 in General Synthesis Route 2. ¹H NMR (400 MHz, Chloroform-d) δ7.13 (t, J=5.0 Hz, 1H), 4.13 (s, 6H), 3.33 (q, J=5.6 Hz, 2H), 2.92 (t, J=6.0 Hz, 2H), 2.47-2.28 (m, 7H), 2.24 (s, 6H), 1.60 (dq, J=14.7, 7.2 Hz, 6H), 1.47 (dd, J=14.2, 6.7 Hz, 6H), 1.27 (d, J=3.4 Hz, 78H), 0.89 (td, J=6.7, 2.5 Hz, 18H).

### N. Compound 1008, Synthesized According to General Synthesis Route 2

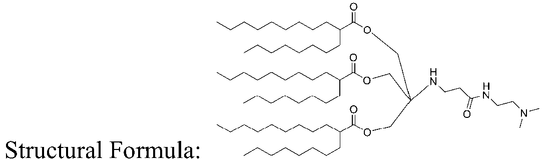

Molecular Formula: C₆₈H₁₃₃N₃O₇
Molecular Weight: 1104.01

Synthesized according to General Synthesis Route 2. The synthesis process was similar to that of Compound 1014, except that Intermediate C was used as Compound 2 in General Synthesis Route 2, and N,N-dimethylethylenediamine was used as Compound 5 in General Synthesis Route 2. ¹H NMR (400 MHz, Chloroform-d) δ4.13 (s, 6H), 3.33 (q, J=5.6 Hz, 2H), 2.92 (t, J=5.9 Hz, 2H), 2.51-2.26 (m, 7H), 2.24 (s, 6H), 1.59 (ddt, J=14.8, 10.9, 6.4 Hz, 6H), 1.47 (tq, J=11.0, 5.4 Hz, 6H), 1.26 (s, 77H), 0.89 (t, J=6.7 Hz, 18H).

### O. Compound 1009, Synthesized According to General Synthesis Route 2

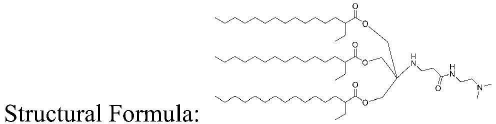

Molecular Formula: C₆₂H₁₂₁N₃O₇
Molecular Weight: 1020.66

Synthesized according to General Synthesis Route 2. The synthesis process was similar to that of Compound 1014, except that Intermediate J was used as Compound 2 in General Synthesis Route 2, and N,N-dimethylethylenediamine was used as Compound 5 in General Synthesis Route 2. 1H (400 MHz, CDCl₃) δ7.04 (t, J=5.0 Hz, 1H), 4.12 (m, J=3.3 Hz, 6H), 3.30 (q, J=5.6 Hz, 2H), 2.91 (t, J=6.0 Hz, 2H), 2.39 (t, J=6.0 Hz, 2H), 2.33-2.25 (m, 5H), 2.22 (s, 6H), 1.76-1.54 (m, 12H), 1.24 (m, J=3.7 Hz, 66H), 0.90-0.84 (m, 18H).

### P. Compound 1011, Synthesized According to General Synthesis Route 2

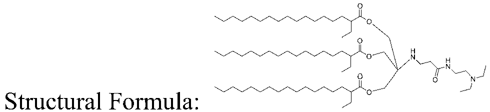

Molecular Formula: C₇₀H₁₃₇N₃O₇
Molecular Weight: 1132.88

Synthesized according to General Synthesis Route 2. The synthesis process was similar to that of Compound 1014, except that N,N-diethylethylenediamine was used as Compound 5 in General Synthesis Route 2. ¹H NMR (400 MHz, CDCl₃) δ4.19-4.08 (m, 6H), 3.31 (q, J=5.8 Hz, 2H), 2.92 (t, 2H), 2.57 (t, J=6.9 Hz, 6H), 2.37-2.26 (m, 5H), 1.62 (ddd, J=18.3, 9.0, 4.3 Hz, 12H), 1.27 (d, J=4.7 Hz, 78H), 1.04 (t, J=7.1 Hz, 6H), 0.94-0.86 (m, 18H).

### Q. Compound 1012, Synthesized According to General Synthesis Route 2

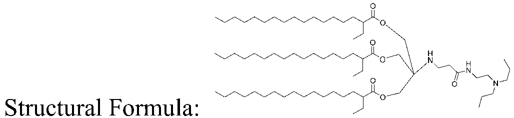

Molecular Formula: C₇₂H₁₄₁N₃O₇
Molecular Weight: 1160.93

Synthesized according to General Synthesis Route 2. The synthesis process was similar to that of Compound 1014, except that N,N-diethylethylenediamine was used as Compound 5 in General Synthesis Route 2. ¹H NMR (400 MHz, CDCl₃) δ6.59 (s, 1H), 4.18-4.07 (m, 6H), 3.29 (q, J=5.7 Hz, 2H), 2.91 (t, J=6.2 Hz, 2H), 2.54 (t, J=6.1 Hz, 2H), 2.40 (t, J=7.5 Hz, 4H), 2.35-2.27 (m, 5H), 1.63-1.42 (m, 16H), 1.27 (m, J=4.5 Hz, 78H), 0.93-0.85 (m, 24H).

### R. Compound 1013, Synthesized According to General Synthesis Route 2

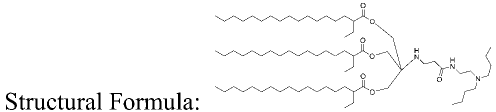

Molecular Formula: C₇₄H₁₄₅N₃O₇
Molecular Weight: 1188.99

Synthesized according to General Synthesis Route 2. The synthesis process was similar to that of Compound 1014, except that N,N-diethylethylenediamine was used as Compound 5 in General Synthesis Route 2. ¹H NMR (400 MHz, CDCl₃) δ4.10 (d, J=4.1 Hz, 6H), 3.26 (q, J=5.8 Hz, 2H), 2.89 (t, J=6.2 Hz, 2H), 2.52 (d, J=6.6 Hz, 2H), 2.41 (t, J=7.4 Hz, 4H), 2.33-2.25 (m, 5H), 1.60 (d, J=11.8 Hz, 16H), 1.24 (d, J=4.5 Hz, 82H), 0.96-0.79 (m, 24H).

### S. Compound 1029, Synthesized According to General Synthesis Route 2

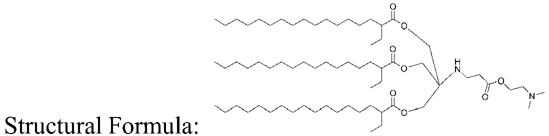

Molecular Formula: C₆₈H₁₃₂N₂O₈
Molecular Weight: 1105.81

Synthesized according to General Synthesis Route 2. The synthesis process was similar to that of Compound 1014, except that N,N-diethyl-2-hydroxyethylamine was used as Compound 5 in General Synthesis Route 2. ¹H NMR (400 MHz, CDCl₃) δ4.16 (t, J=5.8 Hz, 2H), 4.13-4.05 (m, 6H), 2.86 (t, J=6.3 Hz, 2H), 2.55 (t, J=5.8 Hz, 2H), 2.44 (t, J=6.3 Hz, 2H), 2.35-2.20 (m, 9H), 1.60-1.39 (m, 11H), 1.24 (d, J=4.5 Hz, 80H), 1.02-0.79 (m, 18H).

### T. Compound 1111, Synthesized According to General Synthesis Route 2

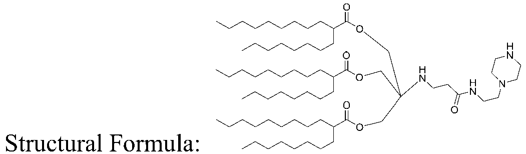

Molecular Formula: C₇₀H₁₃₆N₄O₇
Molecular Weight: 1145.88

Synthesized according to General Synthesis Route 2. The synthesis process was similar to that of Compound 1014, except that Intermediate C was used as Compound 2 in General Synthesis Route 2, and t-butyl 4-(2-aminoethyl)piperazine-1-carboxylate was used as Compound 5 in General Synthesis Route 2. ¹H NMR (400 MHz, Chloroform-d) δ4.28 (s, 6H), 3.41 (s, 2H), 3.31 (s, 4H), 3.22 (s, 3H), 2.97-2.80 (m, 5H), 2.70 (s, 5H), 2.46-2.32 (m, 4H), 1.60 (t, J=7.1 Hz, 6H), 1.54-1.40 (m, 6H), 1.27 (s, 78H), 0.89 (t, J=6.7 Hz, 18H).

### U. Compound 1118, Synthesized According to General Synthesis Route 2

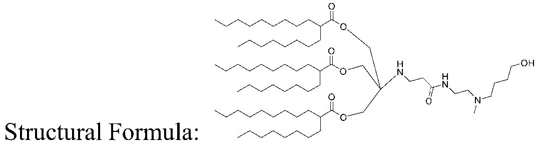

Molecular Formula: C₇₁H₁₃₉N₃O₈
Molecular Weight: 1162.91

Synthesized according to General Synthesis Route 2. The synthesis process was similar to that of Compound 1014, except that Intermediate C was used as Compound 2 in General Synthesis Route 2, and Intermediate K was used as Compound 5 in General Synthesis Route 2. ¹H NMR (400 MHz, Chloroform-d) δ4.11 (s, 6H), 3.61 (s, 2H), 3.42 (d, J=33.5 Hz, 2H), 2.91 (t, J=6.2 Hz, 3H), 2.36 (dd, J=9.2, 3.6 Hz, 6H), 1.47 (dd, J=13.9, 6.2 Hz, 14H), 1.27 (s, 71H), 0.90 (t, J=6.7 Hz, 18H).

### U1. Compound 1132, Synthesized According to General Synthesis Route 2

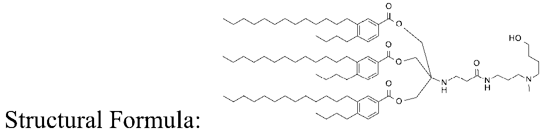

Molecular Weight: 1363.15

Synthesized according to General Synthesis Route 2. The synthesis process was similar to that of Compound 1014, except that Intermediate N was used as Compound 2 in General Synthesis Route 2, and Intermediate O was used as Compound 5 in General Synthesis Route 2. LCMS (ESI) calcd for: C₈₇H₁₄₇N₃O₈, [M+H]⁺=1363.13, found 682.4 (half peak). ¹H NMR (400 MHz, Chloroform-d) δ7.82 (d, J=1.9 Hz, 3H), 7.77 (dd, J=8.0, 1.9 Hz, 3H), 7.18 (d, J=8.0 Hz, 3H), 4.57 (s, 6H), 3.66 (t, J=5.4 Hz, 2H), 3.23 (q, J=6.1 Hz, 2H), 3.11 (t, J=6.0 Hz, 2H), 2.82 (d, J=17.6 Hz, 0H), 2.71-2.50 (m, 16H), 2.42 (t, J=5.9 Hz, 2H), 1.87 (p, J=7.2 Hz, 5H), 1.72-1.63 (m, 2H), 1.63-1.49 (m, 13H), 1.48-1.17 (m, 53H), 0.97 (t, J=7.3 Hz, 10H), 0.90 (t, J=6.7 Hz, 9H).

### U2. Compound 1139, Synthesized According to General Synthesis Route 2

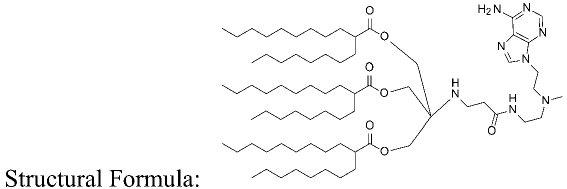

Molecular Weight: 1251.97

Synthesized according to General Synthesis Route 2. The synthesis process was similar to that of Compound 1014, except that Intermediate C was used as Compound 2 in General Synthesis Route 2, and Intermediate P was used as Compound 5 in General Synthesis Route 2. LCMS (ESI) calcd for C₇₄H₁₃₈N₈O₇, ⁺[M+H]⁺ m/z 1252.08, found 626.8 (half peak). ¹H NMR (400 MHz, Chloroform-d) δ8.38 (s, 1H), 7.90 (s, 1H), 6.25 (t, J=5.2 Hz, 1H), 5.71 (s, 2H), 4.31 (t, J=5.9 Hz, 2H), 4.15 (s, 6H), 3.20 (q, J=5.7 Hz, 2H), 2.86 (dt, J=15.6, 6.1 Hz, 4H), 2.51 (t, J=5.9 Hz, 2H), 2.37 (ddd, J=8.4, 5.7, 2.7 Hz, 3H), 2.32 (s, 3H), 2.20 (t, J=6.3 Hz, 2H), 1.60 (q, J=7.8 Hz, 6H), 1.51-1.43 (m, 6H), 1.26 (s, 78H), 0.92-0.86 (m, 18H).

### U3. Compound 1140, Synthesized According to General Synthesis Route 2

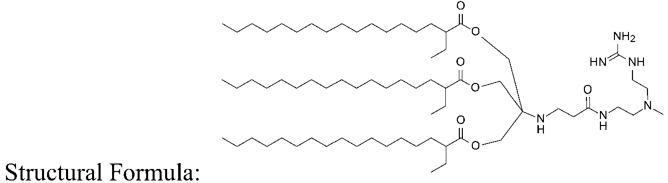

Molecular Weight: 1175.91

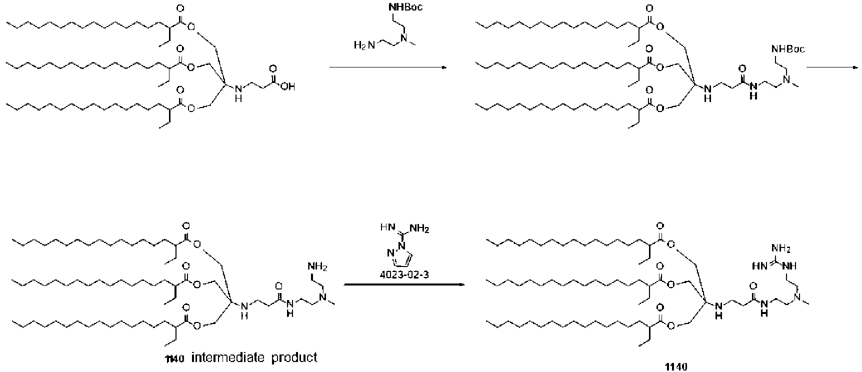

Synthesized according to General Synthesis Route 2. The synthesis process was similar to that of Compound 1014, except that Intermediate B was used as Compound 2 in General Synthesis Route 2, and Intermediate Q was used as Compound 5 in General Synthesis Route 2, and the intermediate product of 1140 was synthesized first. LCMS (ESI) calcd for C₆₉H₁₃₆N₄O₇, ⁺[M+H]⁺ m/z 1134.05, found 1134.25. Then, CH₃CN (10 mL), 4023-02-3 (101 mg) and DIPEA (355 mg) were added to the intermediate product of 1140 (520 mg) in a single-neck flask, reacted at room temperature for 12 h. Water and dichloromethane were added and stirred for liquid separation. The organic phase was washed with saturated brine. The organic phase was separated, concentrated and purified to obtain Compound 1140 (300 mg). LCMS (ESI) calcd for C₇₀H₁₃₈N₆O₇, ⁺[M+H]⁺ m/z 1176.07, found 1176.22. ¹H NMR (400 MHz, Chloroform-d) δ4.23-4.04 (m, 6H), 3.42-3.20 (m, 4H), 2.91 (t, J=6.1 Hz, 2H), 2.71-2.51 (m, 4H), 2.43 (t, J=6.0 Hz, 2H), 2.36-2.23 (m, 6H), 1.76-1.40 (m, 11H), 1.27 (d, J=4.0 Hz, 75H), 0.90 (t, J=7.1 Hz, 18H).

### V. Compound 1010, Synthesized According to General Synthesis Route 3

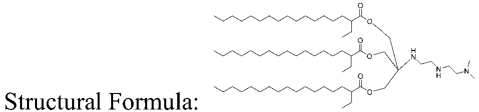

Molecular Formula: C₆₇H₁₃₃N₃O₆
Molecular Weight: 1076.82

### Step 1 and Step 2: Synthesis of Compound 3 and Compound 4 in General Synthesis Route 3

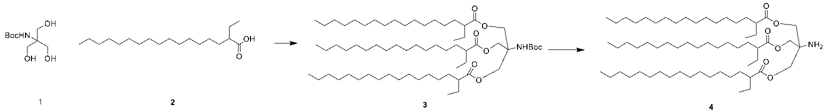

synthesized according to Step 1 and Step 2 of General Synthesis Route 1. The synthesis process was the same as that of Compound 1003.

### Step 3: Synthesis of Compound 6 in General Synthesis Route 3

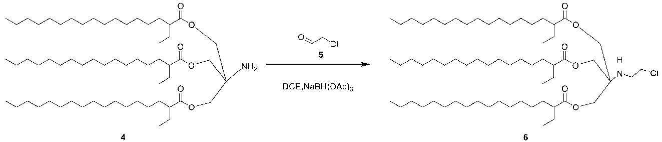

Compound 4 (600 mg), chloroacetaldehyde (Compound 5, 242 mg (40%)) and NaBH(OAc)₃ (394 mg) were added to DCE (60 mL), reacted at room temperature overnight. Then, water and dichloromethane were added and stirred for liquid separation. The organic phase was washed with saturated brine. The organic phase was separated, concentrated, and purified by silica gel column chromatography (DCM:MeOH=30:1-15:1) to obtain Compound 6 (270 mg).

### Step 4: Synthesis of Compound 1010

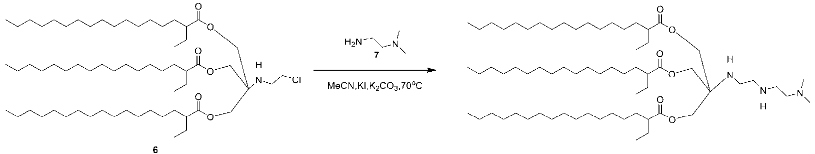

MeCN (20 mL), N,N-dimethylethylenediamine (Compound 7, 229 mg), KI (43 mg) and K₂CO₃ (179 mg) were added to Compound 6 (270 mg), reacted at 70°C overnight under nitrogen protection, then purified by silica gel column chromatography to obtain Compound 1010 (16 mg). 1H (400 MHz, CDCl₃) δ4.10 (m, J=2.9 Hz, 6H), 2.76-2.64 (m, 6H), 2.40 (t, J=6.2 Hz, 2H), 2.29 (ddd, J=8.5, 5.6, 2.9 Hz, 3H), 2.22 (s, 6H), 1.97 (s, 6H), 1.50-1.41 (m, 6H), 1.24 (d, J=4.2 Hz, 78H), 0.87 (t, J=7.1 Hz, 18H).

### W. Compound 1059, Synthesized According to General Synthesis Route 3

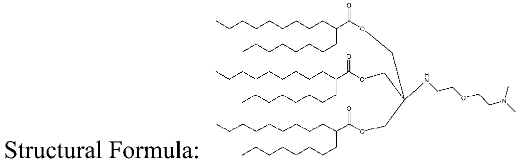

Molecular Formula: C₆₇H₁₃₂N₂O₇
Molecular Weight: 1077.80

Synthesized according to General Synthesis Route 3. The synthesis process was similar to that of Compound 1010, except that Intermediate C and Intermediate L were used as Compound 2 and Compound 5 in General Synthesis Route 3, respectively. In addition, Compound 6 in General Synthesis Route 3 was subjected to functional group transformation through the following process:

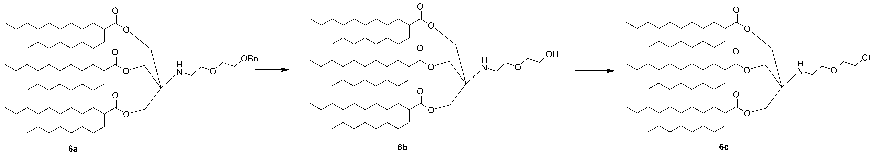

Solvent MeOH (5 mL) and THF (5 mL) were added to Intermediate 6a (1.5 g), then reactants Pd(OH)₂/C (10%) (0.46 g) and Pd/C (10%) (0.35 g) were added, reacted at 25°C overnight under H₂ atmosphere, then filtered through celite. The filtrate was concentrated, and purified by silica gel column chromatography (PE:EA=30:1 ~ 10:1) to obtain Intermediate 6b (520 mg). DCM (5 mL) was added to Intermediate 6b (470 mg). SOCl₂ (1064.26 mg, 8.946 mmol) was added dropwise at 0°C, the mixture was warmed to room temperature and reacted for 3 hours, then the reaction solution was extracted with water and dichloromethane, the organic phase was concentrated, and purified by silica gel column chromatography (PE:EA=20:1) to obtain Intermediate 6c (181 mg). Dimethylamine (143.39 mg), DMF (2 mL), K₂CO₃ (43.96 mg) and KI (26.39 mg) were added to Intermediate 6c (170 mg). The reaction solution was stirred at 70°C, then filtered, concentrated, and purified by silica gel column chromatography (DCM:MeOH=20:1) to obtain Compound 1059 (91 mg, yield 53.22%). ¹H NMR (400 MHz, Chloroform-d) δ4.11 (s, 6H), 3.51 (dt, J=18.3, 5.6 Hz, 4H), 2.81 (dd, J=10.3, 5.1 Hz, 2H), 2.50 (t, J=6.0 Hz, 2H), 2.42-2.32 (m, 3H), 2.28 (s, 6H), 2.08-1.98 (m, 1H), 1.53-1.38 (m, 7H), 1.27 (s, 77H), 0.90 (t, J=6.7 Hz, 18H).

### X. Compound 1112, Synthesized According to General Synthesis Route 3

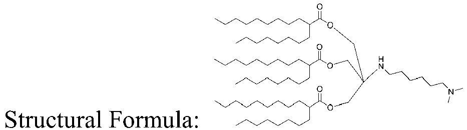

Molecular Formula:C₆₉H₁₃₆N₂O₆
Molecular Weight: 1089.85

Synthesized according to General Synthesis Route 3. The synthesis process was similar to that of Compound 1010, except that Intermediate C and 6-bromohexanal were used as Compound 2 and Compound 5 in General Synthesis Route 3, respectively. ¹H NMR (400 MHz, Chloroform-d) δ4.11 (s, 6H), 3.11 (s, 1H), 2.57 (t, J=6.8 Hz, 2H), 2.44-2.19 (m, 12H), 2.04 (q, J=6.5 Hz, 2H), 1.53-1.38 (m, 13H), 1.27 (s, 92H), 0.90 (t, J=6.7 Hz, 20H).

### X1. Compound 3424, Synthesized According to General Synthesis Route 3

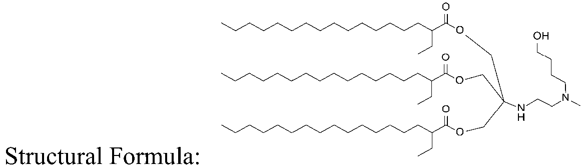

Molecular Weight: 1091.83

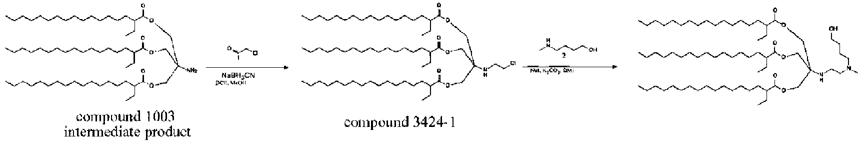

Synthesized according to General Synthesis Route 3. The synthesis process was similar to that of Compound 1010, except that Intermediate B, 2-chloroacetaldehyde and 4-(methylamino)butan-1-ol were used as Compound 2, Compound 5 and Compound 7 in General Synthesis Route 3, respectively. LCMS (ESI) calcd for: C₆₈H₁₃₄N₂O₇, [M+H]+=1091.02, found 1091.92+. ¹H NMR (400 MHz, CDCl₃) δ4.13 (d, J=2.1 Hz, 6H), 3.60 (d, J=5.4 Hz, 2H), 2.79 (s, 2H), 2.55-2.38 (m, 4H), 2.31 (ddt, J=16.3, 13.3, 6.6 Hz, 6H), 1.69-1.43 (m, 18H), 1.27 (d, J=4.0 Hz, 78H), 0.94-0.86 (m, 18H).

### X2. Compound 3425, Synthesized According to General Synthesis Route 3

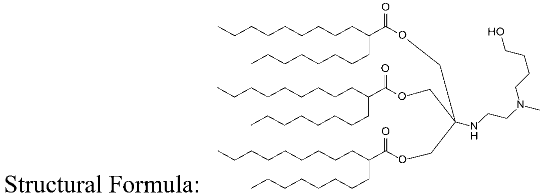

Molecular Weight: 1091.83

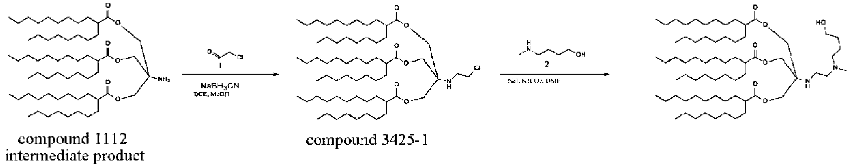

Synthesized according to General Synthesis Route 3. The synthesis process was similar to that of Compound 1010, except that Intermediate C, 2-chloroacetaldehyde and 4-(methylamino)butan-1-ol were used as Compound 2, Compound 5 and Compound 7 in General Synthesis Route 3, respectively. LCMS (ESI) calcd for: C₆₈H₁₃₄N₂O₇, [M+H]+=1091.02, found 1091.83+. ¹H NMR (400 MHz, CDCl₃) δ4.12 (s, 6H), 3.60 (d, J=5.2 Hz, 2H), 2.79 (s, 2H), 2.50 (s, 2H), 2.37 (tt, J=8.5, 5.6 Hz, 4H), 2.28 (s, 2H), 1.67 (t, J=3.7 Hz, 4H), 1.59 (p, J=7.2 Hz, 6H), 1.51-1.41 (m, 6H), 1.27 (s, 78H), 0.90 (t, J=6.7 Hz, 18H).

### X3. Compound 3426, Synthesized According to General Synthesis Route 3

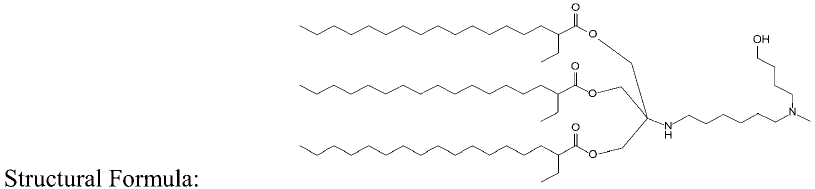

Molecular Weight: 1147.94

Synthesized according to General Synthesis Route 3. The synthesis process was similar to that of Compound 1010, except that Intermediate B, 6-bromohexanal and 4-(methylamino)butan-1-ol were used as Compound 2, Compound 5 and Compound 7 in General Synthesis Route 3, respectively. LCMS (ESI) calcd for: C₇₂H₁₄₂N₂O₇, [M+H]+=1148.08, found 1149.22+. ¹H NMR (400 MHz, CDCl₃) δ4.16-4.06 (m, 6H), 3.59 (dd, J=6.1, 3.4 Hz, 2H), 2.97 (dd, J=29.3, 22.5 Hz, 1H), 2.57 (t, J=6.9 Hz, 2H), 2.46 (dt, J=7.9, 5.2 Hz, 4H), 2.35-2.27 (m, 6H), 1.76-1.66 (m, 4H), 1.65-1.38 (m, 16H), 1.27 (d, J=4.1 Hz, 78H), 0.90 (t, J=7.1 Hz, 18H).

### Y. Compound 1113, Synthesized According to General Synthesis Route 4

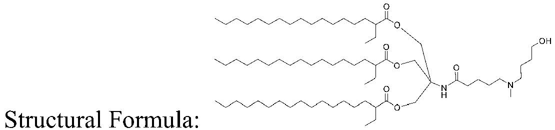

Molecular Formula: C₇₁H₁₃₈N₂O₈
Molecular Weight: 1147.89

### Step 1 and Step 2: Synthesis of Compound 3 and Compound 4 in General Synthesis Route 4

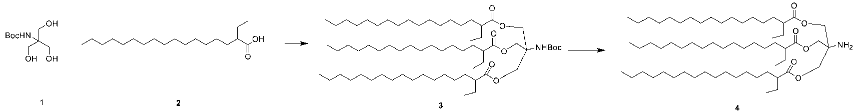

Synthesized according to Step 1 and Step 2 of General Synthesis Route 1. The synthesis process was the same as that of Compound 1003.

### Step 3: Synthesis of Compound 6 in General Synthesis Route 4

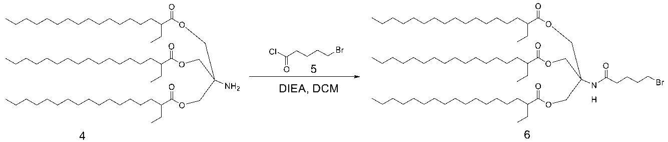

DIEA (0.30 g) and solvent DCM (20 mL) were added to Compound 4. The mixture was cooled to 0°C under nitrogen protection. Then, 5-bromovaleryl chloride (Compound 5, 0.27 g) was added, stirred at 25°C for 2 h, diluted with 100 mL DCM. After that, the organic phase was washed twice with 100 mL water. The organic phase was dried over anhydrous Na₂SO₄, filtered, concentrated, and purified by silica gel column chromatography (PE:EA=20:1) to obtain Compound 6 (800 mg).

### Step 4: Synthesis of Compound 8 in General Synthesis Route 4

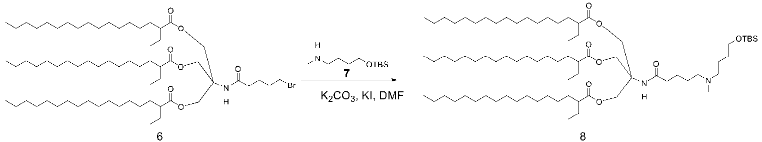

Compound 6 (750.00 mg), Intermediate K (Compound 7, 217.31 mg) and solvent DMF (20 mL) were added to a three-neck flask. Then, reactants K₂CO₃ (184.16 mg) and KI (110.60 mg) were added, stirred at 25°C for 16 h, then warmed to 100°C and stirred for 3 h. The reaction solution was diluted with 200 mL EA, then the organic phase was washed twice with 300 mL saturated brine. The organic phase was dried over anhydrous Na₂SO₄, filtered, concentrated, mixed with silica gel, and purified by column chromatography (DCM:MeOH=20:1), the sample was collected and concentrated to obtain Compound 8 (700.00 mg).

### Step 5: Synthesis of Compound 1113

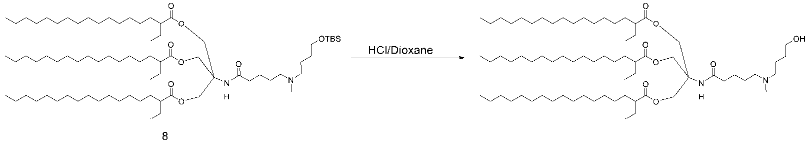

Compound 8 (700.00 mg) and solvent THF (5 mL) were added to a three-neck flask, then 4 M HCl in dioxane (5.55 mL) was added. The reaction solution was stirred at 25°C for 2 h, diluted with 50 mL EA. Then, the organic phase was washed with saturated NaHCO₃ solution and water, dried over anhydrous Na₂SO₄, filtered, concentrated, and purified by silica gel column chromatography (MeOH:DCM=1:20) to obtain Compound 1113 (201.1 mg, yield of 31.59%). ¹H NMR (400 MHz, CDCl₃) δ4.50-4.39 (m, 6H), 3.59 (t, J=4.6 Hz, 2H), 2.49 (s, 3H), 2.35-2.26 (m, 6H), 2.14 (t, J=7.5 Hz, 2H), 1.75-1.69 (m, 4H), 1.66-1.43 (m, 17H), 1.27 (d, J=3.7 Hz, 78H), 0.94-0.87 (m, 18H).

### Z. Compound 1115, Synthesized According to General Synthesis Route 4

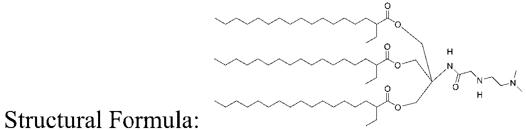

Molecular Formula: C₆₇H₁₃₁N₃O₇
Molecular Weight: 1090.80

Synthesized according to General Synthesis Route 4. The synthesis process was similar to that of Compound 1010, except that 2-bromoacetyl chloride and N,N-dimethylethylenediamine were used as Compound 5 and Compound 7 in General Synthesis Route 4, respectively. ¹H NMR (400 MHz, CDCl₃) δ7.42 (s, 1H), 4.48 (tt, J=11.9, 5.7 Hz, 6H), 3.27 (s, 2H), 3.13 (s, 2H), 3.01 (s, 2H), 2.83 (s, 6H), 2.38-2.25 (m, 3H), 1.55 (dtt, J=36.3, 14.1, 7.3 Hz, 12H), 1.27 (d, J=3.3 Hz, 78H), 0.90 (td, J=7.2, 3.4 Hz, 18H).

### Z1. Compound 3427, Synthesized According to General Synthesis Route 4

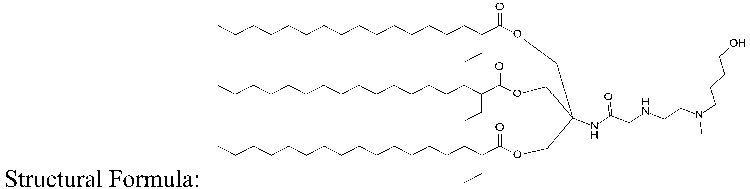

Molecular Weight: 1148.88

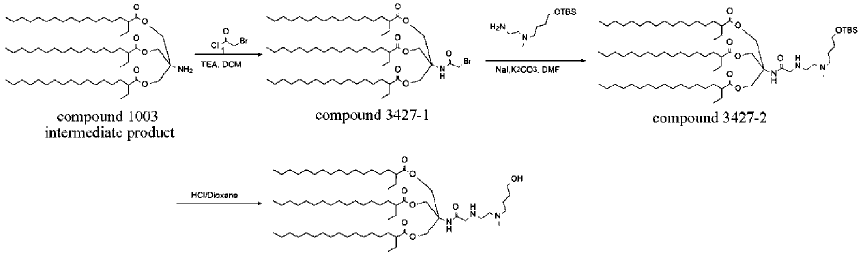

Synthesized according to General Synthesis Route 4. The synthesis process was similar to that of Compound 1010, except that 2-bromoacetyl chloride and Intermediate R were used as Compound 5 and Compound 7 in General Synthesis Route 4, respectively. LCMS (ESI) calcd for: C₇₀H₁₃₇N₃O₈, [M+H]+=1148.88, found 1148.97+. ¹H NMR (400 MHz, CDCl₃) δ7.74 (s, 1H), 4.53-4.40 (m, 6H), 3.60 (d, J=4.8 Hz, 2H), 3.20 (s, 2H), 2.72 (dd, J=6.6, 4.9 Hz, 2H), 2.54 (t, J=5.7 Hz, 2H), 2.43 (d, J=5.2 Hz, 2H), 2.34-2.23 (m, 6H), 1.70 (p, J=2.4 Hz, 4H), 1.66-1.40 (m, 12H), 1.26 (d, J=3.9 Hz, 78H), 0.95-0.84 (m, 18H).

### Z2. Compound 3423, Synthesized According to General Synthesis Route 4

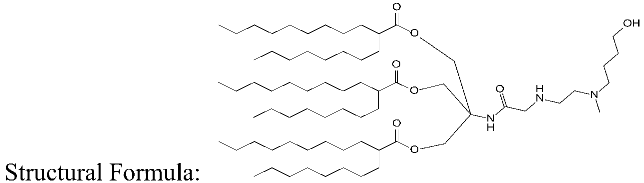

Molecular Weight: 1148.88

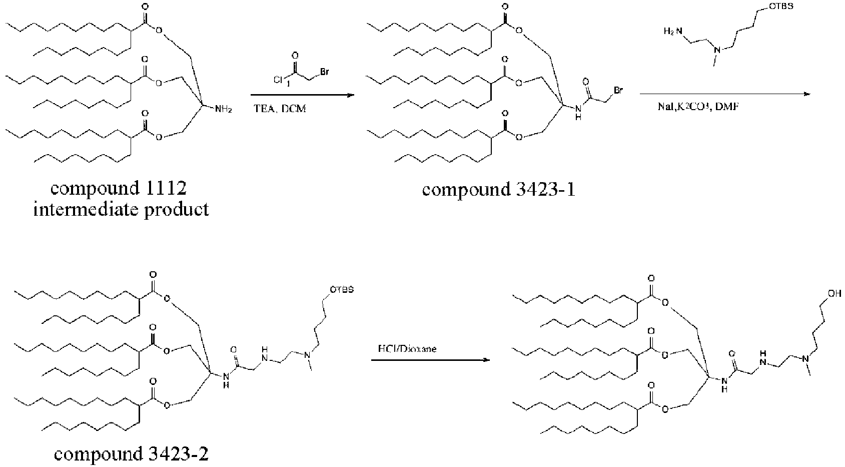

Synthesized according to General Synthesis Route 4. The synthesis process was similar to that of Compound 1010, except that Intermediate C, 2-bromoacetyl chloride and Intermediate R were used as Compound 2, Compound 5 and Compound 7 in General Synthesis Route 4, respectively. LCMS (ESI) calcd for: C₇₀H₁₃₇N₃O₈, [M+H]+=1148.88, found 1149.28+. ¹H NMR (400 MHz, CDCl₃) δ7.72 (s, 1H), 4.45 (s, 6H), 3.62 (d, J=5.2 Hz, 2H), 3.20 (s, 2H), 2.73 (d, J=6.3 Hz, 2H), 2.59 (s, 2H), 2.47 (s, 2H), 2.41-2.24 (m, 6H), 1.59 (dp, J=10.9, 6.4 Hz, 6H), 1.52-1.40 (m, 6H), 1.27 (s, 78H), 0.90 (t, J=6.7 Hz, 18H).

### AA. Compound 1114, Synthesized According to General Synthesis Route 5

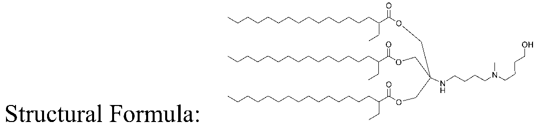

Molecular Formula: C₇₀H₁₃₈N₂O₇
Molecular Weight: 1119.88

### Step 1 and Step 2: Synthesis of Compound 3 and Compound 4 in General Synthesis Route 5

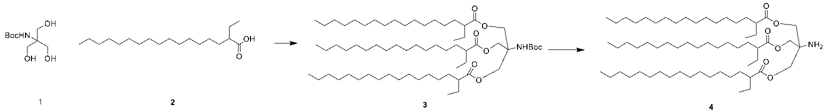

Synthesized according to Step 1 and Step 2 of General Synthesis Route 1. The synthesis process was the same as that of Compound 1003.

### Step 3: Synthesis of Compound 6 in General Synthesis Route 5

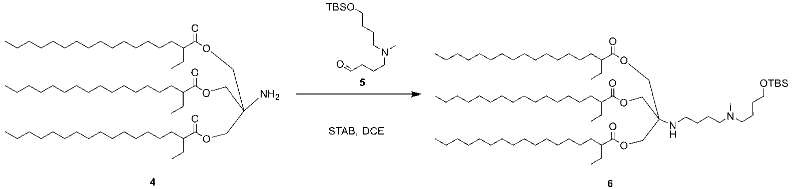

Compound 4 (500 mg), Intermediate M (Compound 5, 298.68 mg), a reactant STAB (328.68 mg) and DCE (10 mL) were added to a three-neck flask. The reaction solution was stirred at 25°C for 16 h under N₂ atmosphere. Then,100 mL water was added to the reaction solution, extracted twice with EA (100 mL). The organic phases were combined and concentrated. Silica gel was added, and column chromatograph was performed with DCM:MeOH=1:20. The sample was collected and concentrated to obtain Compound 6 (500 mg).

### Step 4: Synthesis of Compound 1114

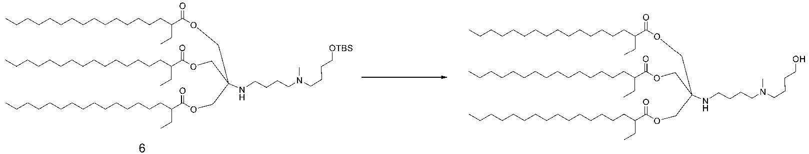

Compound 6 (450 mg) and solvent THF (5 mL) were added to a three-neck flask, then 4 M HCl in dioxane (3.65 mL) was added. The mixture was stirred at 25°C for 2 h, diluted with 50 mL EA. Then, the organic phase was washed with 50 mL saturated NaHCO₃ solution and water, dried over anhydrous Na₂SO₄, filtered, concentrated, and purified by silica gel column chromatography (MeOH:DCM=1:20) to obtain Compound 1114 (107 mg, yield 24.84%). ¹H NMR (400 MHz, CDCl₃) δ4.16-4.07 (m, 6H), 3.60 (t, J=4.8 Hz, 2H), 2.62 (t, J=7.0 Hz, 2H), 2.49 (s, 3H), 2.31 (tt, J=8.4, 5.5 Hz, 6H), 1.70 (d, J=4.6 Hz, 3H), 1.64-1.39 (m, 18H), 1.27 (d, J=4.0 Hz, 78H), 0.90 (t, J=7.1 Hz, 18H).

### AA-1. Compound 1120, Synthesized According to General Synthesis Route 5

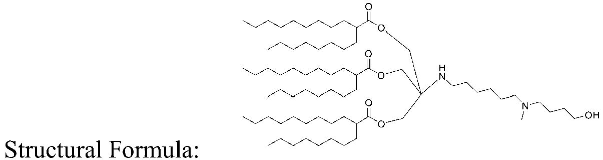

Molecular Formula: C₇₂H₁₄₂N₂O₇
Molecular Weight: 1147.90

Synthesized according to General Synthesis Route 5. The synthesis process was similar to that of Compound 1114, except that Intermediate C and 6-bromohexanal were used as Compound 2 and Compound 5 in General Synthesis Route 5, respectively. ¹H NMR (400 MHz, Chloroform-d) δ4.11 (s, 6H), 3.63 (s, 2H), 2.57 (t, J=6.8 Hz, 4H), 2.37 (ddd, J=8.5, 7.1, 4.3 Hz, 5H), 1.67-1.41 (m, 24H), 1.27 (s, 79H), 0.90 (t, J=6.7 Hz, 18H).

### AA-2. Compound 3428, Synthesized According to General Synthesis Route 5

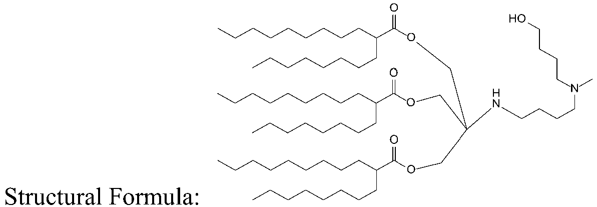

Molecular Weight: 1119.88

Synthesized according to General Synthesis Route 5. The synthesis process was similar to that of Compound 1114, except that Intermediate C was used as Compound 2 in General Synthesis Route 5.

LCMS (ESI) calcd for: C₇₀H₁₃₈N₂O₇, [M+H]+=1119.88, found 1119.98+.

¹H NMR (400 MHz, CDCl₃) δ4.10 (s, 6H), 3.59 (d, J=4.7 Hz, 2H), 2.61 (t, J=7.1 Hz, 2H), 2.45 (d, J=7.0 Hz, 4H), 2.36 (tt, J=8.5, 5.4 Hz, 4H), 2.28 (s, 3H), 1.69-1.42 (m, 18H), 1.27 (s, 79H), 0.90 (t, J=6.7 Hz, 18H).

### Example 2: Exemplary Compounds and Their Properties

The exemplary compounds of the present invention and their properties are listed in Table 1, which were synthesized according to the process routes in Example 1, respectively.

The calculated c-pKa (molnetwork) and LogP (cLogP driver) of the exemplary compounds of the present invention, c-pKa and cLogP values were generated by the ChemDraw module of Chemoffice.

**Table 1 Properties of Exemplary Compounds**

| No. | Molecular Formula | Molecular Weight | c-pKa | cLogP | No. | Molecular Formula | Molecular Weight | c-pKa | cLogP |
|---|---|---|---|---|---|---|---|---|---|
| 1001 | C₅₉H₁₀₈N₂O₇ | 957.52 | 9.83 | 21.43 | 1201 | C₇₁H₁₄₈N₆O | 1102.01 | 9.01 | 28.48 |
| 1002 | C₆₀H₁₁₆N₂O₇ | 977.60 | 6.97 | 24.49 | 1202 | C₉₂H₁₉₀N₆O | 1396.57 | 9.01 | 39.59 |
| 1003 | C₆₃H₁₂₂N₂O₇ | 1019.68 | 6.97 | 26.07 | 1203 | C₆₅H₁₃₆N₆O | 1017.84 | 9.01 | 25.31 |
| 1004 | C₅₄H₁₀₄N₂O₇ | 893.43 | 6.97 | 21.97 | 1204 | C₆₅H₁₃₆N₆O | 1017.84 | 9.01 | 25.31 |
| 1005 | C₆₈H₁₃₃N₃O₇ | 1104.83 | 9.01 | 26.86 | 1205 | C₉₈H₂₀₂N₆O | 1480.73 | 9.01 | 43.03 |
| 1006 | C₅₃H₁₀₃N₃O₇ | 894.42 | 9.01 | 19.58 | 1206 | C₉₂H₁₉₀N₆O | 1396.57 | 9.01 | 39.86 |
| 1007 | C₆₈H₁₃₃N₃O₇ | 1104.83 | 9.01 | 26.86 | 1207 | C₈₃H₁₇₂N₆O | 1270.33 | 9.01 | 35.10 |
| 1008 | C₆₈H₁₃₃N₃O₇ | 1104.01 | 9.01 | 26.86 | 1208 | C₆₅H₁₃₀N₆O | 1011.80 | 9.01 | 24.06 |
| 1009 | C₆₂H₁₂₁N₃O₇ | 1020.66 | 9.01 | 23.69 | 1209 | C₆₅H₁₃₀N₆O | 1011.80 | 9.01 | 23.97 |
| 1010 | C₆₇H₁₃₃N₃O₆ | 1076.82 | 9.78 | 27.13 | 1210 | C₄₄H₉₄N₆O | 723.28 | 9.01 | 14.86 |
| 1011 | C₇₀H₁₃₇N₃O₇ | 1132.88 | 9.07 | 27.92 | 1211 | C₅₆H₁₀₆N₆O | 879.50 | 9.01 | 18.30 |
| 1012 | C₇₂H₁₄₁N₃O₇ | 1160.93 | 9.10 | 28.98 | 1212 | C₆₂H₁₁₈N₆O | 963.67 | 9.01 | 20.30 |
| 1013 | C₇₄H₁₄₅N₃O₇ | 1188.99 | 9.12 | 30.03 | 1213 | C₅₀H₁₀₀N₆O | 801.39 | 9.01 | 16.58 |
| 1014 | C₇₀H₁₃₇N₃O₉ | 1164.88 | 7.40 | 26.03 | 1214 | C₆₂H₁₂₄N₆O | 969.71 | 9.01 | 22.93 |
| 1015 | C₇₂H₁₄₁N₃O₉ | 1192.93 | 8.33 | 26.61 | 1215 | C₆₂H₁₃₀N₆O | 975.76 | 9.01 | 24.38 |
| 1016 | C₆₅H₁₂₉N₃O₈ | 1080.80 | 9.11 | 23.70 | 1216 | C₆₈H₁₁₈N₆O | 1035.73 | 9.01 | 25.13 |
| 1017 | C₇₁H₁₄₁N₃O₈ | 1164.90 | 9.10 | 26.87 | 1217 | C₈₃H₁₄₈N₆O | 1246.14 | 9.01 | 32.83 |
| 1018 | C₇₂H₁₄₀N₂O₁₀ | 1193.92 | 8.15 | 27.21 | 1218 | C₅₉H₁₂₁N₃OS₆ | 1081.00 | 9.01 | 27.15 |
| 1019 | C₇₂H₁₄₀N₂O₁₀ | 1193.92 | 8.15 | 27.21 | 1219 | C₅₉H₁₂₁N₃OS₆ | 1081.00 | 9.01 | 27.15 |
| 1020 | C₇₄H₁₄₅N₃O₉ | 1220.99 | 8.74 | 26.06 | 1220 | C₇₁H₁₄₅N₃OS₆ | 1249.32 | 9.01 | 33.50 |
| 1021 | C₇₄H₁₄₅N₃O₉ | 1221.00 | 8.74 | 26.06 | 1221 | C₉₂H₁₈₇N₃OS₆ | 1543.89 | 9.01 | 44.61 |
| 1022 | C₇₃H₁₄₅N₃O₈ | 1193.00 | 9.51 | 26.33 | 1222 | C₆₅H₁₃₃N₃OS₆ | 1165.16 | 9.01 | 30.32 |
| 1023 | C₇₄H₁₄₄N₂O₁₀ | 1221.97 | 8.55 | 26.66 | 1223 | C₁₀₀H₁₉₈N₄O₁₀S₂ | 1680.82 | 8.90 | 43.24 |
| 1024 | C₇₄H₁₄₄N₂O₁₀ | 1221.97 | 8.55 | 26.66 | 1224 | C₉₈H₁₉₉N₃OS₆ | 1628.05 | 9.01 | 48.05 |
| 1025 | C₆₉H₁₃₅N₃O₈ | 1134.85 | 8.20 | 26.44 | 1225 | C₉₂H₁₈₇N₃OS₆ | 1543.89 | 9.01 | 44.88 |
| 1026 | C₆₉H₁₃₄N₂O₉ | 1135.84 | 8.02 | 27.05 | 1226 | C₈₃H₁₆₉N₃OS₆ | 1417.64 | 9.01 | 40.11 |
| 1027 | C₆₉H₁₃₄N₂O₉ | 1135.84 | 8.02 | 27.05 | 1227 | C₆₅H₁₂₇N₃OS₆ | 1159.11 | 9.01 | 29.08 |
| 1028 | C₆₄H₁₂₅N₃O₇ | 1048.70 | 9.07 | 24.74 | 1228 | C₆₅H₁₂₇N₃OS₆ | 1159.11 | 9.01 | 28.99 |
| 1029 | C₆₈H₁₃₂N₂O₈ | 1105.81 | 8.83 | 27.46 | 1229 | C₄₄H₉₁N₃OS₆ | 870.59 | 9.01 | 19.87 |
| 1030 | C₆₆H₁₂₉N₃O₇ | 1076.80 | 9.10 | 25.80 | 1230 | C₅₆H₁₀₃N₃OS₆ | 1026.82 | 9.01 | 23.32 |
| 1031 | C₆₅H₁₂₉N₃O₆ | 1048.80 | 9.86 | 26.07 | 1231 | C₆₂H₁₁₅N₃OS₆ | 1110.98 | 9.01 | 25.32 |
| 1032 | C₆₉H₁₃₅N₃O₇ | 1118.90 | 9.10 | 27.39 | 1232 | C₅₀H₉₇N₃OS₆ | 948.71 | 9.01 | 21.60 |
| 1033 | C₆₆H₁₂₈N₂O₈ | 1077.80 | 8.91 | 26.41 | 1233 | C₆₂H₁₂₁N₃OS₆ | 1117.03 | 9.01 | 27.94 |
| 1034 | C₆₈H₁₃₃N₃O₇ | 1104.83 | 9.12 | 26.86 | 1234 | C₆₂H₁₂₇N₃OS₆ | 1123.08 | 9.01 | 29.40 |
| 1035 | C₇₁H₁₃₉N₃O₇ | 1146.91 | 9.12 | 28.45 | 1235 | C₆₈H₁₁₅N₃OS₆ | 1183.05 | 9.01 | 27.86 |
| 1036 | C₆₈H₁₃₂N₂O₈ | 1105.80 | 8.93 | 27.46 | 1236 | C₈₃H₁₄₅N₃OS₆ | 1393.45 | 9.01 | 35.56 |
| 1037 | C₆₄H₁₂₅N₃O₉ | 1080.70 | 7.40 | 22.85 | 1237 | C₆₂H₁₂₁N₃O₄ | 972.67 | 9.01 | 23.48 |
| 1038 | C₆₇H₁₃₁N₃O₉ | 1122.80 | 7.40 | 24.44 | 1238 | C₇₄H₁₄₅N₃O₄ | 1140.99 | 9.01 | 29.83 |
| 1039 | C₆₄H₁₂₄N₂O₁₀ | 1081.70 | 7.21 | 23.46 | 1239 | C₉₅H₁₈₇N₃O₄ | 1435.56 | 9.01 | 40.94 |
| 1040 | C₆₉H₁₃₅N₃O₉ | 1150.90 | 8.33 | 25.02 | 1240 | C₆₈H₁₃₃N₃O₄ | 1056.83 | 9.01 | 26.65 |
| 1041 | C₆₆H₁₂₈N₂O₁₀ | 1109.80 | 8.15 | 24.04 | 1241 | C₆₈H₁₃₃N₃O₄ | 1056.83 | 9.01 | 26.65 |
| 1042 | C₆₈H₁₃₂N₂O₁₀ | 1137.80 | 8.55 | 23.49 | 1242 | C₁₀₁H₁₉₉N₃O₄ | 1519.72 | 9.01 | 43.72 |
| 1043 | C₇₀H₁₃₈N₄O₈ | 1163.89 | 9.40 | 25.69 | 1243 | C₉₅H₁₈₇N₃O₄ | 1435.56 | 9.01 | 40.55 |
| 1044 | C₆₄H₁₂₆N₄O₈ | 1079.73 | 9.40 | 22.52 | 1244 | C₈₆H₁₆₉N₃O₄ | 1309.32 | 9.01 | 35.79 |
| 1045 | C₇₀H₁₃₈N₄O₈ | 1163.89 | 9.40 | 25.69 | 1245 | C₆₈H₁₂₇N₃O₄ | 1050.78 | 9.01 | 24.75 |
| 1046 | C₇₀H₁₃₈N₄O₈ | 1163.89 | 9.40 | 25.69 | 1246 | C₆₈H₁₂₇N₃O₄ | 1050.78 | 9.01 | 24.66 |
| 1047 | C₆₇H₁₃₁N₃O₈ | 1106.80 | 8.95 | 25.14 | 1247 | C₄₇H₉₁N₃O₄ | 762.26 | 9.01 | 15.54 |
| 1048 | C₆₁H₁₁₉N₃O₈ | 1022.64 | 8.95 | 21.96 | 1248 | C₅₉H₁₀₃N₃O₄ | 918.49 | 9.01 | 18.99 |
| 1049 | C₆₇H₁₃₁N₃O₈ | 1106.80 | 8.95 | 25.14 | 1249 | C₆₅H₁₁₅N₃O₄ | 1002.65 | 9.01 | 20.99 |
| 1050 | C₇₂H₁₃₉NO₉ | 1162.90 | 9.10 | 28.52 | 1250 | C₅₃H₉₇N₃O₄ | 840.38 | 9.01 | 17.27 |
| 1051 | C₇₄H₁₃₉N₃O₁₃ | 1278.90 | 9.01 | 23.74 | 1251 | C₆₅H₁₂₁N₃O₄ | 1008.70 | 9.01 | 23.61 |
| 1052 | C₇₇H₁₅₀N₂O₇ | 1216.10 | 9.83 | 32.01 | 1252 | C₆₅H₁₂₇N₃O₄ | 1014.75 | 9.01 | 25.07 |
| 1053 | C₇₃H₁₃₇N₂O₆P | 1169.88 | 9.68 | 25.77 | 1253 | C₇₁H₁₁₅N₃O₄ | 1074.72 | 9.01 | 25.00 |
| 1054 | C₇₄H₁₃₈N₃O₇P | 1212.91 | 9.95 | 26.18 | 1254 | C₈₆H₁₄₅N₃O₄ | 1285.12 | 9.01 | 32.69 |
| 1055 | C₇₁H₁₃₆N₃O₃PS₉ | 1399.40 | 8.85 | 32.41 | 1255 | C₉₉H₁₉₅N₃O₃S₄ | 1603.91 | 8.68 | 43.96 |
| 1056 | C₆₈H₁₃₈N₂O₅ | 1063.90 | 9.70 | 26.03 | 1256 | C₇₄H₁₄₅N₃O₇ | 1188.99 | 9.01 | 30.03 |
| 1057 | C₇₀H₁₄₀N₄O₆S₂ | 1198.03 | 9.67 | 24.35 | 1257 | C₉₅H₁₈₇N₃O₇ | 1483.55 | 9.01 | 41.14 |
| 1058 | C₆₇H₁₃₂N₂O₇ | 1077.80 | 9.45 | 27.29 | 1258 | C₁₀₄H₂₀₀N₃O₇PS₄ | 1763.97 | 7.94 | 41.36 |
| 1059 | C₆₇H₁₃₂N₂O₇ | 1077.80 | 9.45 | 27.29 | 1259 | C₁₀₃H₂₀₀N₂OS₇ | 1707.17 | 10.09 | 46.14 |
| 1060 | C₆₇H₁₃₃N₃O₈S | 1140.87 | 8.74 | 26.96 | 1260 | C₁₀₁H₁₉₉N₃O₇ | 1567.72 | 9.01 | 44.59 |
| 1061 | C₇₃H₁₂₅N₃O₅ | 1124.82 | 9.77 | 23.41 | 1261 | C₉₅H₁₈₇N₃O₇ | 1483.55 | 9.01 | 41.41 |
| 1062 | C₆₇H₁₁₃N₃O₁₀S₃ | 1216.83 | 9.73 | 22.69 | 1262 | C₈₆H₁₆₉N₃O₇ | 1357.31 | 9.01 | 36.65 |
| 1063 | C₆₅H₁₀₉N₃O₁₀S₃ | 1188.78 | 9.01 | 21.48 | 1263 | C₆₈H₁₂₇N₃O₇ | 1098.78 | 9.01 | 25.62 |
| 1064 | C₆₈H₁₃₉N₃OS₆ | 1207.24 | 9.01 | 32.18 | 1264 | C₆₈H₁₂₇N₃O₇ | 1098.78 | 9.01 | 25.53 |
| 1065 | C₆₈H₁₃₉N₃OS₆ | 1207.24 | 9.01 | 32.18 | 1265 | C₄₇H₉₁N₃O₇ | 810.26 | 9.01 | 16.41 |
| 1066 | C₇₄H₁₅₄N₃O₁₃P₃ | 1386.98 | 9.01 | 26.51 | 1266 | C₅₉H₁₀₃N₃O₇ | 966.49 | 9.01 | 19.85 |
| 1067 | C₇₄H₁₅₄N₃O₁₃P₃ | 1386.98 | 9.01 | 26.51 | 1267 | C₆₅H₁₁₅N₃O₇ | 1050.65 | 9.01 | 21.86 |
| 1068 | C₇₁H₁₃₁N₃O₇ | 1138.84 | 5.07 | 26.81 | 1268 | C₅₃H₉₇N₃O₇ | 888.37 | 9.01 | 18.13 |
| 1069 | C₇₁H₁₃₁N₃O₇ | 1138.84 | 5.04 | 26.81 | 1269 | C₆₅H₁₂₁N₃O₇ | 1056.70 | 9.01 | 24.48 |
| 1070 | C₆₉H₁₃₀N₄O₇ | 1127.82 | 5.03 | 26.38 | 1270 | C₆₅H₁₂₇N₃O₇ | 1062.74 | 9.01 | 25.93 |
| 1071 | C₆₉H₁₃₀N₄O₇ | 1127.82 | 5.00 | 26.38 | 1271 | C₇₁H₁₁₅N₃O₇ | 1122.71 | 9.01 | 26.17 |
| 1072 | C₇₀H₁₃₅N₃O₈ | 1146.86 | 6.83 | 26.82 | 1272 | C₈₆H₁₄₅N₃O₇ | 1333.12 | 9.01 | 33.87 |
| 1073 | C₇₀H₁₃₅N₃O₈ | 1146.86 | 6.83 | 26.82 | 1273 | C₆₂H₁₂₁N₃O₇ | 1020.66 | 9.01 | 23.69 |
| 1074 | C₇₁H₁₃₈N₄O₈ | 1175.91 | 7.89 | 26.41 | 1274 | C₇₄H₁₄₅N₃O₇ | 1188.99 | 9.01 | 30.03 |
| 1075 | C₇₁H₁₃₈N₄O₈ | 1175.91 | 7.89 | 26.41 | 1275 | C₉₅H₁₈₇N₃O₇ | 1483.55 | 9.01 | 41.14 |
| 1076 | C₇₁H₁₃₈N₄O₉ | 1191.90 | 7.80 | 26.25 | 1276 | C₆₈H₁₃₃N₃O₇ | 1104.83 | 9.01 | 26.86 |
| 1077 | C₇₁H₁₃₈N₄O₉ | 1191.90 | 7.80 | 26.25 | 1277 | C₆₈H₁₃₃N₃O₇ | 1104.83 | 9.01 | 26.86 |
| 1078 | C₇₁H₁₃₈N₄O₉ | 1191.90 | 7.75 | 27.09 | 1278 | C₁₀₁H₁₉₉N₃O₇ | 1567.72 | 9.01 | 44.59 |
| 1079 | C₇₁H₁₃₈N₄O₉ | 1191.90 | 7.75 | 27.09 | 1279 | C₉₅H₁₈₇N₃O₇ | 1483.55 | 9.01 | 41.41 |
| 1080 | C₇₀H₁₃₈N₄O₉S | 1211.95 | 7.61 | 26.56 | 1280 | C₈₆H₁₆₉N₃O₇ | 1357.31 | 9.01 | 36.65 |
| 1081 | C₇₀H₁₃₈N₄O₉S | 1211.95 | 7.61 | 26.56 | 1281 | C₆₈H₁₂₇N₃O₇ | 1098.78 | 9.01 | 25.62 |
| 1082 | C₇₂H₁₃₉N₅O₈ | 1202.93 | 7.82 | 26.46 | 1282 | C₆₈H₁₂₇N₃O₇ | 1098.78 | 9.01 | 25.53 |
| 1083 | C₇₂H₁₃₉N₅O₈ | 1202.93 | 7.82 | 26.46 | 1283 | C₄₇H₉₁N₃O₇ | 810.26 | 9.01 | 16.41 |
| 1084 | C₇₂H₁₃₇N₅O₇S | 1216.98 | 7.95 | 28.08 | 1284 | C₅₉H₁₀₃N₃O₇ | 966.49 | 9.01 | 19.85 |
| 1085 | C₇₂H₁₃₇N₅O₇S | 1216.98 | 7.95 | 28.08 | 1285 | C₆₅H₁₁₅N₃O₇ | 1050.65 | 9.01 | 21.86 |
| 1086 | C₇₄H₁₃₈N₈O₇ | 1251.97 | 5.88 | 25.00 | 1286 | C₅₃H₉₇N₃O₇ | 888.37 | 9.01 | 18.13 |
| 1087 | C₇₄H₁₃₈N₈O₇ | 1251.97 | 5.88 | 25.00 | 1287 | C₆₅H₁₂₁N₃O₇ | 1056.70 | 9.01 | 24.48 |
| 1088 | C₇₄H₁₃₈N₈O₈ | 1267.97 | 7.59 | 25.58 | 1288 | C₆₅H₁₂₇N₃O₇ | 1062.74 | 9.01 | 25.93 |
| 1089 | C₇₄H₁₃₈N₈O₈ | 1267.97 | 7.59 | 25.58 | 1289 | C₇₁H₁₁₅N₃O₇ | 1122.71 | 9.01 | 24.80 |
| 1090 | C₇₃H₁₃₇N₅O₉ | 1228.93 | 7.07 | 26.15 | 1290 | C₈₆H₁₄₅N₃O₇ | 1333.12 | 9.01 | 32.49 |
| 1091 | C₇₃H₁₃₇N₅O₉ | 1228.93 | 7.07 | 26.15 | 1291 | C₆₂H₁₂₁N₃O₁₀ | 1068.66 | 9.01 | 23.51 |
| 1092 | C₇₄H₁₃₉N₅O₈ | 1226.95 | 7.47 | 26.45 | 1292 | C₇₄H₁₄₅N₃O₁₀ | 1236.98 | 9.01 | 29.86 |
| 1093 | C₇₄H₁₃₉N₅O₈ | 1226.95 | 7.47 | 26.45 | 1293 | C₉₅H₁₈₇N₃O₁₀ | 1531.55 | 9.01 | 40.96 |
| 1094 | C₇₀H₁₃₈N₆O₇ | 1175.91 | 8.12 | 25.90 | 1294 | C₆₈H₁₃₃N₃O₁₀ | 1152.82 | 9.01 | 26.68 |
| 1095 | C₇₀H₁₃₈N₆O₇ | 1175.91 | 8.12 | 25.90 | 1295 | C₆₈H₁₃₃N₃O₁₀ | 1152.82 | 9.01 | 26.68 |
| 1096 | C₇₂H₁₄₂N₆O₉S | 1268.02 | 7.63 | 26.40 | 1296 | C₁₀₁H₁₉₉N₃O₁₀ | 1615.71 | 9.01 | 44.41 |
| 1097 | C₇₂H₁₄₂N₆O₉S | 1268.02 | 7.63 | 26.40 | 1297 | C₉₅H₁₈₇N₃O₁₀ | 1531.55 | 9.01 | 41.23 |
| 1098 | C₇₃H₁₄₃N₅O₉S | 1267.03 | 7.51 | 27.09 | 1298 | C₈₆H₁₆₉N₃O₁₀ | 1405.31 | 9.01 | 36.47 |
| 1099 | C₇₃H₁₄₃N₅O₉S | 1267.03 | 7.51 | 27.09 | 1299 | C₆₈H₁₂₇N₃O₁₀ | 1146.77 | 9.01 | 25.44 |
| 1100 | C₇₄H₁₄₅N₅O₉S | 1281.06 | 8.31 | 27.25 | 1300 | C₆₈H₁₂₇N₃O₁₀ | 1146.77 | 9.01 | 25.35 |
| 1101 | C₇₃H₁₄₃N₅O₉S | 1267.03 | 7.51 | 27.09 | 1301 | C₄₇H₉₁N₃O₁₀ | 858.26 | 9.01 | 16.23 |
| 1102 | C₇₂H₁₃₉N₃O₇ | 1158.92 | 10.26 | 27.41 | 1302 | C₅₉H₁₀₃N₃O₁₀ | 1014.48 | 9.01 | 19.68 |
| 1103 | C₇₁H₁₃₈N₄O₇ | 1159.91 | 9.78 | 26.04 | 1303 | C₆₅H₁₁₅N₃O₁₀ | 1098.65 | 9.01 | 21.68 |
| 1104 | C₆₉H₁₃₃N₃O₇ | 1116.84 | 9.32 | 25.52 | 1304 | C₅₃H₉₇N₃O₁₀ | 936.37 | 9.01 | 17.95 |
| 1105 | C₆₉H₁₃₃N₃O₇ | 1116.84 | 9.32 | 25.52 | 1305 | C₆₅H₁₂₁N₃O₁₀ | 1104.69 | 9.01 | 24.30 |
| 1106 | C₆₉H₁₃₆N₆O₇ | 1161.88 | 7.46 | 24.33 | 1306 | C₆₅H₁₂₇N₃O₁₀ | 1110.74 | 9.01 | 25.75 |
| 1107 | C₆₃H₁₂₄N₆O₇ | 1077.72 | 7.46 | 21.15 | 1307 | C₇₁H₁₁₅N₃O₁₀ | 1170.71 | 9.01 | 24.31 |
| 1108 | C₆₉H₁₃₆N₆O₇ | 1161.88 | 7.46 | 24.33 | 1308 | C₈₆H₁₄₅N₃O₁₀ | 1381.12 | 9.01 | 32.00 |
| 1109 | C₆₉H₁₃₆N₆O₇ | 1161.88 | 7.46 | 24.33 | 1309 | C₆₂H₁₂₄N₆O₄ | 1017.71 | 9.01 | 20.89 |
| 1110 | C₇₀H₁₃₆N₄O₇ | 1145.88 | 8.82 | 26.41 | 1310 | C₇₄H₁₄₈N₆O₄ | 1186.04 | 9.01 | 27.24 |
| 1111 | C₇₀H₁₃₆N₄O₇ | 1145.88 | 8.82 | 26.41 | 1311 | C₉₅H₁₉₀N₆O₄ | 1480.60 | 9.01 | 38.35 |
| 1112 | C₆₉H₁₃₆N₂O₆ | 1089.85 | 10.19 | 28.16 | 1312 | C₆₈H₁₃₆N₆O₄ | 1101.87 | 9.01 | 24.06 |
| 1113 | C₇₁H₁₃₈N₂O₈ | 1147.89 | 9.69 | 26.58 | 1313 | C₆₈H₁₃₆N₆O₄ | 1101.87 | 9.01 | 24.06 |
| 1114 | C₇₀H₁₃₈N₂O₇ | 1119.88 | 9.84 | 26.71 | 1314 | C₁₀₁H₂₀₂N₆O₄ | 1564.76 | 9.01 | 41.79 |
| 1115 | C₆₇H₁₃₁N₃O₇ | 1090.80 | 9.50 | 26.91 | 1315 | C₉₅H₁₉₀N₆O₄ | 1480.60 | 9.01 | 38.62 |
| 1116 | C₆₉H₁₃₅N₃O₈ | 1134.85 | 9.52 | 27.58 | 1316 | C₈₆H₁₇₂N₆O₄ | 1354.36 | 9.01 | 33.85 |
| 1117 | C₇₁H₁₄₁N₃O₆ | 1132.92 | 9.88 | 29.03 | 1317 | C₆₈H₁₃₀N₆O₄ | 1095.83 | 9.01 | 22.82 |
| 1118 | C₇₁H₁₃₉N₃O₈ | 1162.91 | 8.87 | 26.46 | 1318 | C₆₈H₁₃₀N₆O₄ | 1095.83 | 9.01 | 22.73 |
| 1119 | C₇₁H₁₃₃NO₉S₅ | 1305.14 | 9.96 | 25.12 | 1319 | C₄₇H₉₄N₆O₄ | 807.31 | 9.01 | 13.61 |
| 1120 | C₇₂H₁₄₂N₂O₇ | 1147.90 | 10.05 | 27.76 | 1320 | C₅₉H₁₀₆N₆O₄ | 963.53 | 9.01 | 17.06 |
| 1121 | C₇₃H₁₃₉N₅O₁₅S₄ | 1455.17 | 7.80 | 23.62 | 1321 | C₅₉H₁₀₆N₆O₄ | 963.53 | 9.01 | 17.06 |
| 1122 | C₆₈H₁₂₉NO₁₃S₃ | 1264.95 | 8.49 | 25.91 | 1322 | C₅₃H₁₀₀N₆O₄ | 885.42 | 9.01 | 15.34 |
| 1123 | C₇₇H₁₅₂N₂O₇ | 1218.07 | 9.99 | 30.68 | 1323 | C₆₅H₁₂₄N₆O₄ | 1053.74 | 9.01 | 21.68 |
| 1124 | C₆₇H₁₃₃N₃O₈S | 1140.87 | 8.74 | 26.96 | 1324 | C₆₅H₁₃₀N₆O₄ | 1059.79 | 9.01 | 23.14 |
| 1125 | C₆₉H₁₃₆N₄O₇ | 1133.87 | 9.61 | 27.37 | 1325 | C₇₁H₁₁₈N₆O₄ | 1119.76 | 9.01 | 23.40 |
| 1126 | C₆₅H₁₂₁N₃O₇ | 1056.70 | 9.01 | 23.82 | 1326 | C₈₆H₁₄₈N₆O₄ | 1330.17 | 9.01 | 31.09 |
| 1127 | C₆₉H₁₃₆N₂O₉ | 1137.85 | 8.31 | 26.05 | 1327 | C₆₂H₁₂₄N₆O₄ | 1017.71 | 9.01 | 20.89 |
| 1128 | C₇₀H₁₃₉N₂O₁₁P | 1215.86 | 7.77 | 26.53 | 1328 | C₇₄H₁₄₈N₆O₄ | 1186.04 | 9.01 | 27.24 |
| 1129 | C₆₈H₁₃₄N₂O₇S₂ | 1155.95 | 8.84 | 27.80 | 1329 | C₉₅H₁₉₀N₆O₄ | 1480.60 | 9.01 | 38.35 |
| 1130 | C₇₄H₁₅₄N₃O₁₃P₃ | 1386.98 | 9.01 | 26.51 | 1330 | C₆₈H₁₃₆N₆O₄ | 1101.87 | 9.01 | 24.06 |
| 1131 | C₈₄H₁₅₉N₃O₈ | 1339.21 | 9.46 | 31.70 | 1331 | C₆₈H₁₃₆N₆O₄ | 1101.87 | 9.01 | 24.06 |
| 1132 | C₈₇H₁₄₇N₃O₈ | 1363.15 | 9.46 | 32.11 | 1332 | C₁₀₁H₂₀₁N₅O₅ | 1565.75 | 9.01 | 42.72 |
| 1133 | C₇₁H₁₃₁N₃O₇ | 1138.84 | 5.07 | 26.81 | 1333 | C₉₅H₁₈₉N₅O₅ | 1481.59 | 9.01 | 39.55 |
| 1134 | C₆₉H₁₃₀N₄O₇ | 1127.82 | 5.00 | 26.38 | 1334 | C₈₆H₁₇₁N₅O₅ | 1355.34 | 9.01 | 34.79 |
| 1135 | C₇₁H₁₃₅N₄O₈ | 1175.91 | 7.89 | 26.41 | 1335 | C₆₈H₁₃₀N₆O₄ | 1095.83 | 9.01 | 22.82 |
| 1136 | C₁₀₈H₂₀₄N₂O₉S₂ | 1738.94 | 8.56 | 46.05 | 1336 | C₆₈H₁₂₉N₅O₅ | 1096.81 | 9.01 | 23.66 |
| 1137 | C₉₉H₁₉₅NO₆S₃ | 1591.83 | 7.68 | 42.68 | 1337 | C₄₇H₉₃N₅O₅ | 808.29 | 9.01 | 14.55 |
| 1138 | C₇₂H₁₃₇N₅O₇S | 1216.98 | 7.95 | 28.08 | 1338 | C₅₉H₁₀₅N₅O₅ | 964.52 | 9.01 | 17.99 |
| 1139 | C₇₄H₁₃₈N₈O₇ | 1251.97 | 5.88 | 25.00 | 1339 | C₆₅H₁₁₇N₅O₅ | 1048.68 | 9.01 | 19.99 |
| 1140 | C₇₀H₁₃₈N₆O₇ | 1175.91 | 8.12 | 25.90 | 1340 | C₅₃H₉₉N₅O₅ | 886.40 | 9.01 | 16.27 |
| 1141 | C₇₃H₁₄₃N₅O₉S | 1267.03 | 7.51 | 27.09 | 1341 | C₆₅H₁₂₃N₅O₅ | 1054.73 | 9.01 | 22.62 |
| 1142 | C₇₀H₁₃₅N₃O₇ | 1130.86 | 9.98 | 26.32 | 1342 | C₆₅H₁₂₉N₅O₅ | 1060.78 | 9.01 | 24.07 |
| 1143 | C₆₅H₁₁₅N₃O₇ | 1050.60 | 9.01 | 21.20 | 1343 | C₇₁H₁₁₇N₅O₅ | 1120.75 | 9.01 | 24.31 |
| 1144 | C₇₅H₁₄₁N₃O₈ | 1212.97 | 9.18 | 28.86 | 1344 | C₈₆H₁₄₈N₆O₄ | 1330.17 | 9.01 | 31.76 |
| 1145 | C₇₀H₁₁₈N₂O₁₁S₃ | 1259.90 | 9.49 | 22.27 | 1345 | C₆₂H₁₂₄N₆O₇ | 1065.71 | 9.01 | 22.93 |
| 1146 | C₆₈H₁₃₄N₂O₇S₂ | 1155.95 | 9.14 | 27.39 | 1346 | C₇₄H₁₄₈N₆O₇ | 1234.03 | 9.01 | 29.28 |
| 1147 | C₈₆H₁₆₇N₃O₅S₂ | 1387.40 | 6.75 | 35.10 | 1347 | C₉₅H₁₉₀N₆O₇ | 1528.60 | 9.01 | 40.39 |
| 1148 | C₆₉H₁₂₀N₆O₂S₄ | 1194.00 | 8.63 | 24.08 | 1348 | C₆₈H₁₃₆N₆O₇ | 1149.87 | 9.01 | 26.10 |
| 1149 | C₇₆H₁₅₄N₆O₃S | 1232.20 | 7.65 | 30.39 | 1349 | C₆₈H₁₃₆N₆O₇ | 1149.87 | 9.01 | 26.10 |
| 1150 | C₉₁H₁₈₆N₄S₆ | 1528.90 | 9.77 | 43.45 | 1350 | C₁₀₁H₂₀₂N₆O₇ | 1612.76 | 9.01 | 43.83 |
| 1151 | C₇₃H₁₄₂N₆O₅ | 1244.00 | NA | 27.09 | 1351 | C₉₅H₁₉₀N₆O₇ | 1528.60 | 9.01 | 40.66 |
| 1152 | C₁₀₅H₁₈₁N₃O₁₂ | 1677.60 | 9.16 | 40.21 | 1352 | C₈₆H₁₇₂N₆O₇ | 1402.36 | 9.01 | 35.90 |
| 1153 | C₆₉H₁₃₁N₅O₅ | 1110.80 | 7.41 | 22.95 | 1353 | C₆₈H₁₃₀N₆O₇ | 1143.82 | 9.01 | 24.86 |
| 1154 | C₇₆H₁₃₉N₃S₃ | 1191.10 | 8.91 | 30.35 | 1354 | C₆₈H₁₃₀N₆O₇ | 1143.82 | 9.01 | 24.77 |
| 1155 | C₆₆H₁₃₂N₅O₁₀P | 1186.80 | 8.65 | 24.15 | 1355 | C₄₇H₉₄N₆O₇ | 855.30 | 9.01 | 15.66 |
| 1156 | C₇₀H₁₂₀N₇O₈P | 1218.70 | 9.55 | 21.96 | 1356 | C₅₉H₁₀₆N₆O₇ | 1011.53 | 9.01 | 19.10 |
| 1157 | C₇₂H₁₃₅N₅O₁₂S₃ | 1359.08 | 7.99 | 23.39 | 1357 | C₆₅H₁₁₈N₆O₇ | 1095.69 | 9.01 | 21.10 |
| 1158 | C₇₂H₁₃₀N₂O₉ | 1167.84 | 8.05 | 25.65 | 1358 | C₅₃H₁₀₀N₆O₇ | 933.42 | 9.01 | 17.38 |
| 1159 | C₇₁H₁₃₉N₃O₃S₆ | 1275.30 | 9.04 | 31.51 | 1359 | C₆₅H₁₂₄N₆O₇ | 1101.74 | 9.01 | 23.73 |
| 1160 | C₆₉H₁₃₈N₄O₉S₃ | 1264.10 | 7.48 | 28.35 | 1360 | C₆₅H₁₃₀N₆O₇ | 1107.79 | 9.01 | 25.18 |
| 1161 | C₇₃H₁₄₂N₂O₈ | 1175.90 | 9.39 | 27.24 | 1361 | C₇₁H₁₁₈N₆O₇ | 1167.76 | 9.01 | 24.18 |
| 1162 | C₈₅H₁₆₇N₅O₁₀ | 1419.30 | 9.49 | 27.91 | 1362 | C₈₆H₁₄₈N₆O₇ | 1378.16 | 9.01 | 31.87 |
| 1163 | C₅₉H₁₂₁N₃O₄ | 936.63 | 9.01 | 22.86 | 1363 | C₆₂H₁₂₄N₆O₇ | 1065.71 | 9.01 | 22.93 |
| 1164 | C₇₁H₁₄₅N₃O₄ | 1104.96 | 9.01 | 29.21 | 1364 | C₇₄H₁₄₈N₆O₇ | 1234.03 | 9.01 | 29.28 |
| 1165 | C₉₂H₁₈₇N₃O₄ | 1399.53 | 9.01 | 40.32 | 1365 | C₉₅H₁₉₀N₆O₇ | 1528.60 | 9.01 | 40.39 |
| 1166 | C₆₅H₁₃₃N₃O₄ | 1020.80 | 9.01 | 26.04 | 1366 | C₆₈H₁₃₆N₆O₇ | 1149.87 | 9.01 | 26.10 |
| 1167 | C₆₅H₁₃₃N₃O₄ | 1020.80 | 9.01 | 26.04 | 1367 | C₆₈H₁₃₆N₆O₇ | 1149.87 | 9.01 | 26.10 |
| 1168 | C₉₈H₁₉₉N₃O₄ | 1483.69 | 9.01 | 43.77 | 1368 | C₁₀₁H₂₀₂N₆O₇ | 1612.76 | 9.01 | 43.83 |
| 1169 | C₉₂H₁₈₇N₃O₄ | 1399.53 | 9.01 | 40.59 | 1369 | C₉₅H₁₉₀N₆O₇ | 1528.60 | 9.01 | 40.66 |
| 1170 | C₈₃H₁₆₉N₃O₄ | 1273.28 | 9.01 | 35.83 | 1370 | C₈₆H₁₇₂N₆O₇ | 1402.36 | 9.01 | 35.90 |
| 1171 | C₆₅H₁₂₇N₃O₄ | 1014.75 | 9.01 | 24.80 | 1371 | C₆₈H₁₃₀N₆O₇ | 1143.82 | 9.01 | 24.86 |
| 1172 | C₆₅H₁₂₇N₃O₄ | 1014.75 | 9.01 | 24.71 | 1372 | C₆₈H₁₃₀N₆O₇ | 1143.82 | 9.01 | 24.77 |
| 1173 | C₄₄H₉₁N₃O₄ | 726.23 | 9.01 | 15.59 | 1373 | C₄₇H₉₄N₆O₇ | 855.30 | 9.01 | 15.66 |
| 1174 | C₅₆H₁₀₃N₃O₄ | 882.46 | 9.01 | 19.03 | 1374 | C₅₉H₁₀₆N₆O₇ | 1011.53 | 9.01 | 19.10 |
| 1175 | C₆₂H₁₁₅N₃O₄ | 966.62 | 9.01 | 21.04 | 1375 | C₆₅H₁₁₈N₆O₇ | 1095.69 | 9.01 | 21.10 |
| 1176 | C₅₀H₉₇N₃O₄ | 804.34 | 9.01 | 17.31 | 1376 | C₅₃H₁₀₀N₆O₇ | 933.42 | 9.01 | 17.38 |
| 1177 | C₆₂H₁₂₁N₃O₄ | 972.67 | 9.01 | 23.66 | 1377 | C₆₅H₁₂₄N₆O₇ | 1101.74 | 9.01 | 23.73 |
| 1178 | C₆₂H₁₂₇N₃O₄ | 978.71 | 9.01 | 25.11 | 1378 | C₆₅H₁₃₀N₆O₇ | 1107.79 | 9.01 | 25.18 |
| 1179 | C₆₈H₁₁₅N₃O₄ | 1038.68 | 9.01 | 26.06 | 1379 | C₇₁H₁₁₈N₆O₇ | 1167.76 | 9.01 | 25.06 |
| 1180 | C₈₃H₁₄₅N₃O₄ | 1249.09 | 9.01 | 33.76 | 1380 | C₈₆H₁₄₈N₆O₇ | 1378.16 | 9.01 | 32.76 |
| 1181 | C₅₉H₁₂₁N₃OS₃ | 984.82 | 9.01 | 25.15 | 1381 | C₆₂H₁₂₇N₉O₄ | 1062.76 | 9.01 | 22.06 |
| 1182 | C₇₁H₁₄₅N₃OS₃ | 1153.14 | 9.01 | 31.50 | 1382 | C₇₄H₁₅₁N₉O₄ | 1231.08 | 9.01 | 28.41 |
| 1183 | C₉₂H₁₈₇N₃OS₃ | 1447.71 | 9.01 | 42.61 | 1383 | C₉₅H₁₉₃N₉O₄ | 1525.65 | 9.01 | 39.52 |
| 1184 | C₆₅H₁₃₃N₃OS₃ | 1068.98 | 9.01 | 28.32 | 1384 | C₆₈H₁₃₉N₉O₄ | 1146.92 | 9.01 | 25.24 |
| 1185 | C₆₅H₁₃₃N₃OS₃ | 1068.98 | 9.01 | 28.32 | 1385 | C₆₈H₁₃₉N₉O₄ | 1146.92 | 9.01 | 25.24 |
| 1186 | C₉₈H₁₉₉N₃OS₃ | 1531.87 | 9.01 | 46.05 | 1386 | C₁₀₁H₂₀₅N₉O₄ | 1609.81 | 9.01 | 42.97 |
| 1187 | C₉₂H₁₈₇N₃OS₃ | 1447.71 | 9.01 | 42.88 | 1387 | C₉₅H₁₉₃N₉O₄ | 1525.65 | 9.01 | 39.79 |
| 1188 | C₈₃H₁₆₉N₃OS₃ | 1321.46 | 9.01 | 38.12 | 1388 | C₈₆H₁₇₅N₉O₄ | 1399.41 | 9.01 | 35.03 |
| 1189 | C₆₅H₁₂₇N₃OS₃ | 1062.93 | 9.01 | 27.08 | 1389 | C₆₈H₁₃₃N₉O₄ | 1140.87 | 9.01 | 24.00 |
| 1190 | C₆₅H₁₂₇N₃OS₃ | 1062.93 | 9.01 | 26.99 | 1390 | C₆₈H₁₃₃N₉O₄ | 1140.87 | 9.01 | 23.91 |
| 1191 | C₄₄H₉₁N₃OS₃ | 774.41 | 9.01 | 17.88 | 1391 | C₄₇H₉₇N₉O₄ | 852.35 | 9.01 | 14.79 |
| 1192 | C₅₆H₁₀₃N₃OS₃ | 930.64 | 9.01 | 21.32 | 1392 | C₅₉H₁₀₉N₉O₄ | 1008.58 | 9.01 | 18.23 |
| 1193 | C₆₂H₁₁₅N₃OS₃ | 1014.80 | 9.01 | 23.32 | 1393 | C₆₅H₁₂₁N₉O₄ | 1092.74 | 9.01 | 20.24 |
| 1194 | C₅₀H₉₇N₃OS₃ | 852.53 | 9.01 | 19.60 | 1394 | C₅₃H₁₀₃N₉O₄ | 930.47 | 9.01 | 16.51 |
| 1195 | C₆₂H₁₂₁N₃OS₃ | 1020.85 | 9.01 | 25.95 | 1395 | C₆₅H₁₂₇N₉O₄ | 1098.79 | 9.01 | 22.86 |
| 1196 | C₆₂H₁₂₇N₃OS₃ | 1026.90 | 9.01 | 27.40 | 1396 | C₆₅H₁₃₃N₉O₄ | 1104.84 | 9.01 | 24.31 |
| 1197 | C₆₈H₁₁₅N₃OS₃ | 1086.87 | 9.01 | 27.48 | 1397 | C₇₁H₁₂₁N₉O₄ | 1164.81 | 9.01 | 23.93 |
| 1198 | C₈₃H_{14S}N₃OS₃ | 1297.27 | 9.01 | 35.18 | 1398 | C₈₆H₁₅₁N₉O₄ | 1375.21 | 9.01 | 31.63 |
| 1199 | C₅₉H₁₂₄N₆O | 933.68 | 9.01 | 22.13 | 1399 | C₆₂H₁₂₁N₃OS₃ | 1020.85 | 9.01 | 24.00 |
| 1200 | C₅₉H₁₂₄N₆O | 933.68 | 9.01 | 22.13 | 1400 | C₇₄H₁₄₅N₃OS₃ | 1189.17 | 9.01 | 30.34 |
| 1401 | C₉₅H₁₈₇N₃OS₃ | 1483.74 | 9.01 | 41.45 | 1601 | C₆₅H₁₃₆N₆O₇S₃ | 1210.04 | 9.01 | 24.53 |
| 1402 | C₆₈H₁₃₃N₃OS₃ | 1105.01 | 9.01 | 27.17 | 1602 | C₆₅H₁₃₆N₆O₇S₃ | 1210.04 | 9.01 | 24.53 |
| 1403 | C₆₈H₁₃₃N₃OS₃ | 1105.01 | 9.01 | 27.17 | 1603 | C₉₈H₂₀₂N₆O₇S₃ | 1672.93 | 9.01 | 42.26 |
| 1404 | C₁₀₁H₁₉₉N₃OS₃ | 1567.90 | 9.01 | 44.90 | 1604 | C₉₂H₁₉₀N₆O₇S₃ | 1588.77 | 9.01 | 39.08 |
| 1405 | C₉₅H₁₈₇N₃OS₃ | 1483.74 | 9.01 | 41.72 | 1605 | C₈₃H₁₇₂N₆O₇S₃ | 1462.52 | 9.01 | 34.32 |
| 1406 | C₈₆H₁₆₉N₃OS₃ | 1357.50 | 9.01 | 36.96 | 1606 | C₆₅H₁₃₀N₆O₇S₃ | 1203.99 | 9.01 | 23.29 |
| 1407 | C₆₈H₁₂₇N₃OS₃ | 1098.96 | 9.01 | 25.93 | 1607 | C₆₅H₁₃₀N₆O₇S₃ | 1203.99 | 9.01 | 23.20 |
| 1408 | C₆₈H₁₂₇N₃OS₃ | 1098.96 | 9.01 | 25.84 | 1608 | C₄₄H₉₄N₆O₇S₃ | 915.47 | 9.01 | 14.08 |
| 1409 | C₄₇H₉₁N₃OS₃ | 810.44 | 9.01 | 16.72 | 1609 | C₅₆H₁₀₆N₆O₇S₃ | 1071.70 | 9.01 | 17.52 |
| 1410 | C₅₉H₁₀₃N₃OS₃ | 966.67 | 9.01 | 20.17 | 1610 | C₆₂H₁₁₈N₆O₇S₃ | 1155.86 | 9.01 | 19.53 |
| 1411 | C₆₅H₁₁₅N₃OS₃ | 1050.83 | 9.01 | 22.17 | 1611 | C₅₀H₁₀₀N₆O₇5₃ | 993.59 | 9.01 | 15.80 |
| 1412 | C₅₃H₉₇N₃OS₃ | 888.56 | 9.01 | 18.44 | 1612 | C₆₂H₁₂₄N₆O₇S₃ | 1161.91 | 9.01 | 22.15 |
| 1413 | C₆₅H₁₂₁N₃OS₃ | 1056.88 | 9.01 | 24.79 | 1613 | C₆₂H₁₃₀N₆O₇S₃ | 1167.96 | 9.01 | 23.60 |
| 1414 | C₆₅H₁₂₇N₃OS₃ | 1062.93 | 9.01 | 26.24 | 1614 | C₆₈H₁₁₈N₆O₇S₃ | 1227.93 | 9.01 | 23.60 |
| 1415 | C₇₁H₁₁₅N₃OS₃ | 1122.90 | 9.01 | 25.38 | 1615 | C₈₃H₁₄₈N₆O₇S₃ | 1438.33 | 9.01 | 31.29 |
| 1416 | C₈₆H_{14S}N₃OS₃ | 1333.31 | 9.01 | 33.07 | 1616 | C₅₉H₁₂₁N₃O₁₀S₃ | 1128.83 | 9.01 | 22.22 |
| 1417 | C₆₂H₁₂₁N₃OS₆ | 1117.03 | 9.01 | 26.24 | 1617 | C₇₁H₁₄₅N₃O₁₀S₃ | 1297.15 | 9.01 | 28.57 |
| 1418 | C₇₄H₁₄₅N₃OS₆ | 1285.35 | 9.01 | 32.59 | 1618 | C₉₂H₁₈₇N₃O₁₀S₃ | 1591.72 | 9.01 | 39.68 |
| 1419 | C₉₅H₁₈₇N₃OS₆ | 1579.92 | 9.01 | 43.70 | 1619 | C₆₅H₁₃₃N₃O₁₀S₃ | 1212.99 | 9.01 | 25.39 |
| 1420 | C₆₈H₁₃₃N₃OS₆ | 1201.19 | 9.01 | 29.41 | 1620 | C₆₅H₁₃₃N₃O₁₀S₃ | 1212.99 | 9.01 | 25.39 |
| 1421 | C₆₈H₁₃₃N₃OS₆ | 1201.19 | 9.01 | 29.41 | 1621 | C₉₈H₁₉₉N₃O₁₀S₃ | 1675.88 | 9.01 | 43.12 |
| 1422 | C₁₀₁H₁₉₉N₃OS₆ | 1664.08 | 9.01 | 47.14 | 1622 | C₉₂H₁₈₇N₃O₁₀S₃ | 1591.72 | 9.01 | 39.95 |
| 1423 | C₉₅H₁₈₇N₃OS₆ | 1579.92 | 9.01 | 43.97 | 1623 | C₈₃H₁₆₉N₃O₁₀S₃ | 1465.48 | 9.01 | 35.19 |
| 1424 | C₈₆H₁₆₉N₃OS₆ | 1453.68 | 9.01 | 39.20 | 1624 | C₆₅H₁₂₇N₃O₁₀S₃ | 1206.94 | 9.01 | 24.15 |
| 1425 | C₆₈H₁₂₇N₃OS₆ | 1195.14 | 9.01 | 28.17 | 1625 | C₆₅H₁₂₇N₃O₁₀S₃ | 1206.94 | 9.01 | 24.06 |
| 1426 | C₆₈H₁₂₇N₃OS₆ | 1195.14 | 9.01 | 28.08 | 1626 | C₄₄H₉₁N₃O₁₀S₃ | 918.42 | 9.01 | 14.95 |
| 1427 | C₄₇H₉₁N₃OS₆ | 906.62 | 9.01 | 18.96 | 1627 | C₅₆H₁₀₃N₃O₁₀S₃ | 1074.65 | 9.01 | 18.39 |
| 1428 | C₅₉H₁₀₃N₃OS₆ | 1062.85 | 9.01 | 22.41 | 1628 | C₆₂H₁₁₅N₃O₁₀S₃ | 1158.81 | 9.01 | 20.39 |
| 1429 | C₆₅H₁₁₅N₃OS₆ | 1147.01 | 9.01 | 24.41 | 1629 | C₅₀H₉₇N₃O₁₀S₃ | 996.54 | 9.01 | 16.67 |
| 1430 | C₅₃H₉₇N₃OS₆ | 984.74 | 9.01 | 20.69 | 1630 | C₆₂H₁₂₁N₃O₁₀S₃ | 1164.86 | 9.01 | 23.02 |
| 1431 | C₆₅H₁₂₁N₃OS₆ | 1153.06 | 9.01 | 27.03 | 1631 | C₆₂H₁₂₇N₃O₁₀S₃ | 1170.91 | 9.01 | 24.47 |
| 1432 | C₆₅H₁₂₇N₃OS₆ | 1159.11 | 9.01 | 28.49 | 1632 | C₆₈H₁₁₅N₃O₁₀S₃ | 1230.88 | 9.01 | 24.27 |
| 1433 | C₇₁H₁₁₅N₃O₂S₅ | 1203.02 | 9.01 | 26.60 | 1633 | C₈₃H₁₄₅N₃O₁₀S₃ | 1441.28 | 9.01 | 31.96 |
| 1434 | C₈₆H₁₄₅N₃OS₆ | 1429.49 | 9.01 | 34.45 | 1634 | C₅₉H₁₂₁N₃O₁₀S₃ | 1128.83 | 9.01 | 22.22 |
| 1435 | C₆₂H₁₂₁N₃OS₆ | 1117.03 | 9.01 | 26.24 | 1635 | C₇₁H₁₄₅N₃O₁₀S₃ | 1297.15 | 9.01 | 28.57 |
| 1436 | C₇₄H₁₄₅N₃OS₆ | 1285.35 | 9.01 | 32.59 | 1636 | C₉₂H₁₈₇N₃O₁₀S₃ | 1591.72 | 9.01 | 39.68 |
| 1437 | C₉₅H₁₈₇N₃OS₆ | 1579.92 | 9.01 | 43.70 | 1637 | C₆₅H₁₃₃N₃O₁₀S₃ | 1212.99 | 9.01 | 25.39 |
| 1438 | C₆₈H₁₃₃N₃OS₆ | 1201.19 | 9.01 | 29.41 | 1638 | C₆₅H₁₃₃N₃O₁₀S₃ | 1212.99 | 9.01 | 25.39 |
| 1439 | C₆₈H₁₃₃N₃OS₆ | 1201.19 | 9.01 | 29.41 | 1639 | C₉₈H₁₉₉N₃O₁₀S₃ | 1675.88 | 9.01 | 43.12 |
| 1440 | C₁₀₁H₁₉₉N₃OS₆ | 1664.08 | 9.01 | 47.14 | 1640 | C₉₂H₁₈₇N₃O₁₀S₃ | 1591.72 | 9.01 | 39.95 |
| 1441 | C₉₅H₁₈₇N₃OS₆ | 1579.92 | 9.01 | 43.97 | 1641 | C₈₃H₁₆₉N₃O₁₀S₃ | 1465.48 | 9.01 | 35.19 |
| 1442 | C₈₆H₁₆₉N₃OS₆ | 1453.68 | 9.01 | 39.20 | 1642 | C₆₅H₁₂₇N₃O₁₀S₃ | 1206.94 | 9.01 | 24.15 |
| 1443 | C₆₈H₁₂₇N₃OS₆ | 1195.14 | 9.01 | 28.17 | 1643 | C₆₅H₁₂₇N₃O₁₀S₃ | 1206.94 | 9.01 | 24.06 |
| 1444 | C₆₈H₁₂₇N₃OS₆ | 1195.14 | 9.01 | 28.08 | 1644 | C₄₄H₉₁N₃O₁₀S₃ | 918.42 | 9.01 | 14.95 |
| 1445 | C₄₇H₉₁N₃OS₆ | 906.62 | 9.01 | 18.96 | 1645 | C₅₆H₁₀₃N₃O₁₀S₃ | 1074.65 | 9.01 | 18.39 |
| 1446 | C₅₉H₁₀₃N₃OS₆ | 1062.85 | 9.01 | 22.41 | 1646 | C₆₂H₁₁₅N₃O₁₀S₃ | 1158.81 | 9.01 | 20.39 |
| 1447 | C₆₅H₁₁₅N₃OS₆ | 1147.01 | 9.01 | 24.41 | 1647 | C₅₀H₉₇N₃O₁₀S₃ | 996.54 | 9.01 | 16.67 |
| 1448 | C₅₃H₉₇N₃OS₆ | 984.74 | 9.01 | 20.69 | 1648 | C₆₂H₁₂₁N₃O₁₀S₃ | 1164.86 | 9.01 | 23.02 |
| 1449 | C₆₅H₁₂₁N₃OS₆ | 1153.06 | 9.01 | 27.03 | 1649 | C₆₂H₁₂₇N₃O₁₀S₃ | 1170.91 | 9.01 | 24.47 |
| 1450 | C₆₅H₁₂₇N₃OS₆ | 1159.11 | 9.01 | 28.49 | 1650 | C₆₈H₁₁₅N₃O₁₀S₃ | 1230.88 | 9.01 | 23.31 |
| 1451 | C₇₁H₁₁₅N₃OS₆ | 1219.08 | 9.01 | 27.44 | 1651 | C₈₃H₁₄₅N₃O₁₀S₃ | 1441.28 | 9.01 | 31.01 |
| 1452 | C₈₆H₁₄₅N₃OS₆ | 1429.49 | 9.01 | 35.13 | 1652 | C₆₀H₁₂₃N₃O₁₃S₃ | 1190.85 | 9.01 | 23.97 |
| 1453 | C₆₂H₁₂₁N₃O₄S₃ | 1068.85 | 9.01 | 25.13 | 1653 | C₇₂H₁₄₇N₃O₁₃S₃ | 1359.18 | 9.01 | 30.32 |
| 1454 | C₇₄H₁₄₅N₃O₄S₃ | 1237.17 | 9.01 | 31.48 | 1654 | C₉₃H₁₈₉N₃O₁₃S₃ | 1653.74 | 9.01 | 41.43 |
| 1455 | C₉₅H₁₈₇N₃O₄S₃ | 1531.74 | 9.01 | 42.59 | 1655 | C₆₆H₁₃₅N₃O₁₃S₃ | 1275.01 | 9.01 | 27.15 |
| 1456 | C₆₈H₁₃₃N₃O₄S₃ | 1153.01 | 9.01 | 28.30 | 1656 | C₆₆H₁₃₅N₃O₁₃S₃ | 1275.01 | 9.01 | 27.15 |
| 1457 | C₆₈H₁₃₃N₃O₄S₃ | 1153.01 | 9.01 | 28.30 | 1657 | C₉₉H₂₀₁N₃O₁₃S₃ | 1737.91 | 9.01 | 44.78 |
| 1458 | C₁₀₁H₁₉₉N₃O₄S₃ | 1615.90 | 9.01 | 46.03 | 1658 | C₉₃H₁₈₉N₃O₁₃S₃ | 1653.74 | 9.01 | 41.61 |
| 1459 | C₉₅H₁₈₇N₃O₄S₃ | 1531.74 | 9.01 | 42.86 | 1659 | C₈₄H₁₇₁N₃O₁₃S₃ | 1527.50 | 9.01 | 36.85 |
| 1460 | C₈₆H₁₆₉N₃O₄S₃ | 1405.50 | 9.01 | 38.09 | 1660 | C₆₅H₁₂₇N₃O₁₃S₃ | 1254.94 | 9.01 | 25.28 |
| 1461 | C₆₈H₁₂₇N₃O₄S₃ | 1146.96 | 9.01 | 27.06 | 1661 | C₆₅H₁₂₇N₃O₁₃S₃ | 1254.94 | 9.01 | 25.19 |
| 1462 | C₆₈H₁₂₇N₃O₄S₃ | 1146.96 | 9.01 | 26.97 | 1662 | C₄₅H₉₃N₃O₁₃S₃ | 980.45 | 9.01 | 16.61 |
| 1463 | C₄₇H₉₁N₃O₄S₃ | 858.44 | 9.01 | 17.85 | 1663 | C₅₆H₁₀₃N₃O₁₃S₃ | 1122.65 | 9.01 | 19.52 |
| 1464 | C₅₉H₁₀₃N₃O₄S₃ | 1014.67 | 9.01 | 21.30 | 1664 | C₆₂H₁₁₅N₃O₁₃S₃ | 1206.81 | 9.01 | 21.53 |
| 1465 | C₆₅H₁₁₅N₃O₄S₃ | 1098.83 | 9.01 | 23.30 | 1665 | C₅₀H₉₇N₃O₁₃S₃ | 1044.53 | 9.01 | 17.80 |
| 1466 | C₅₃H₉₇N₃O₄S₃ | 936.56 | 9.01 | 19.58 | 1666 | C₆₂H₁₂₁N₃O₁₃S₃ | 1212.86 | 9.01 | 24.15 |
| 1467 | C₆₅H₁₂₁N₃O₄S₃ | 1104.88 | 9.01 | 25.92 | 1667 | C₆₂H₁₂₇N₃O₁₃S₃ | 1218.91 | 9.01 | 25.60 |
| 1468 | C₆₅H₁₂₇N₃O₄S₃ | 1110.93 | 9.01 | 27.38 | 1668 | C₆₈H₁₁₅N₃O₁₃S₃ | 1278.88 | 9.01 | 25.80 |
| 1469 | C₇₁H₁₁₅N₃O₄S₃ | 1170.90 | 9.01 | 26.31 | 1669 | C₈₃H₁₄₅N₃O₁₃S₃ | 1489.28 | 9.01 | 33.49 |
| 1470 | C₈₆H₁₄₅N₃O₄S₃ | 1381.30 | 9.01 | 34.00 | 1670 | C₅₉H₁₂₁N₃O₇S₃ | 1080.83 | 9.01 | 19.96 |
| 1471 | C₆₂H₁₂₁N₃O₄S₃ | 1068.85 | 9.01 | 25.13 | 1671 | C₅₉H₁₂₁N₃O₇S₃ | 1080.83 | 9.01 | 19.96 |
| 1472 | C₆₂H₁₂₁N₃O₄S₃ | 1068.85 | 9.01 | 25.13 | 1672 | C₇₁H₁₄₅N₃O₇S₃ | 1249.16 | 9.01 | 26.30 |
| 1473 | C₇₄H₁₄₅N₃O₄S₃ | 1237.17 | 9.01 | 31.48 | 1673 | C₉₂H₁₈₇N₃O₇S₃ | 1543.72 | 9.01 | 37.41 |
| 1474 | C₉₅H₁₈₇N₃O₄S₃ | 1531.74 | 9.01 | 42.59 | 1674 | C₆₅H₁₃₃N₃O₇S₃ | 1164.99 | 9.01 | 23.13 |
| 1475 | C₆₈H₁₃₃N₃O₄S₃ | 1153.01 | 9.01 | 28.30 | 1675 | C₆₅H₁₃₃N₃O₇S₃ | 1164.99 | 9.01 | 23.13 |
| 1476 | C₆₈H₁₃₃N₃O₄S₃ | 1153.01 | 9.01 | 28.30 | 1676 | C₉₈H₁₉₉N₃O₇S₃ | 1627.88 | 9.01 | 40.86 |
| 1477 | C₁₀₁H₁₉₉N₃O₄S₃ | 1615.90 | 9.01 | 46.03 | 1677 | C₉₂H₁₈₇N₃O₇S₃ | 1543.72 | 9.01 | 37.68 |
| 1478 | C₉₅H₁₈₇N₃O₄S₃ | 1531.74 | 9.01 | 42.86 | 1678 | C₈₃H₁₆₉N₃O₇S₃ | 1417.48 | 9.01 | 32.92 |
| 1479 | C₈₆H₁₆₉N₃O₄S₃ | 1405.50 | 9.01 | 38.09 | 1679 | C₆₅H₁₂₇N₃O₇S₃ | 1158.95 | 9.01 | 21.89 |
| 1480 | C₆₈H₁₂₇N₃O₄S₃ | 1146.96 | 9.01 | 27.06 | 1680 | C₆₅H₁₂₇N₃O₇S₃ | 1158.95 | 9.01 | 21.80 |
| 1481 | C₆₈H₁₂₇N₃O₆S | 1114.84 | 9.01 | 26.01 | 1681 | C₄₄H₉₁N₃O₇S₃ | 870.43 | 9.01 | 12.68 |
| 1482 | C₄₇H₉₁N₃O₄S₃ | 858.46 | 9.01 | 17.85 | 1682 | C₅₆H₁₀₃N₃O₇S₃ | 1026.65 | 9.01 | 16.12 |
| 1483 | C₅₉H₁₀₃N₃O₄S₃ | 1014.69 | 9.01 | 21.30 | 1683 | C₆₂H₁₁₅N₃O₇S₃ | 1110.82 | 9.01 | 18.13 |
| 1484 | C₆₅H₁₁₅N₃O₄S₃ | 1098.85 | 9.01 | 23.30 | 1684 | C₅₀H₉₇N₃O₇S₃ | 948.54 | 9.01 | 14.40 |
| 1485 | C₅₃H₉₇N₃O₄S₃ | 936.58 | 9.01 | 19.58 | 1685 | C₆₂H₁₂₁N₃O₇S₃ | 1116.86 | 9.01 | 20.75 |
| 1486 | C₆₅H₁₂₁N₃O₄S₃ | 1104.90 | 9.01 | 25.92 | 1686 | C₆₂H₁₂₇N₃O₇S₃ | 1122.91 | 9.01 | 22.20 |
| 1487 | C₆₅H₁₂₇N₃O₄S₃ | 1110.95 | 9.01 | 27.38 | 1687 | C₆₈H₁₁₅N₃O₇S₃ | 1182.88 | 9.01 | 22.60 |
| 1488 | C₇₁H₁₁₅N₃O₄S₃ | 1170.92 | 9.01 | 26.44 | 1688 | C₈₃H₁₄₅N₃O₇S₃ | 1393.29 | 9.01 | 30.30 |
| 1489 | C₈₆H₁₄₅N₃O₄S₃ | 1381.32 | 9.01 | 34.14 | 1689 | C₆₄H₁₃₂N₂O₄ | 993.77 | 9.45 | 26.47 |
| 1490 | C₆₂H₁₂₁N₃O₇S₃ | 1116.86 | 9.01 | 26.05 | 1690 | C₆₄H₁₃₂N₂O₄ | 993.77 | 9.45 | 26.47 |
| 1491 | C₇₄H₁₄₅N₃O₇S₃ | 1285.19 | 9.01 | 32.39 | 1691 | C₆₇H₁₃₂N₂O₇ | 1077.80 | 9.45 | 27.29 |
| 1492 | C₉₅H₁₈₇N₃O₇S₃ | 1579.76 | 9.01 | 43.50 | 1692 | C₆₇H₁₃₂N₂O₇ | 1077.80 | 9.45 | 27.29 |
| 1493 | C₆₈H₁₃₃N₃O₇S₃ | 1201.03 | 9.01 | 29.22 | 1693 | C₆₇H₁₃₂N₂O₇ | 1077.80 | 9.46 | 27.29 |
| 1494 | C₆₈H₁₃₃N₃O₇S₃ | 1201.03 | 9.01 | 29.22 | 1694 | C₆₇H₁₃₂N₂O₇ | 1077.80 | 9.46 | 27.29 |
| 1495 | C₁₀₁H₁₉₉N₃O₇S₃ | 1663.92 | 9.01 | 46.95 | 1695 | C₆₄H₁₃₂N₂O₃S | 1009.83 | 9.80 | 26.96 |
| 1496 | C₉₅H₁₈₇N₃O₇S₃ | 1579.76 | 9.01 | 43.77 | 1696 | C₆₄H₁₃₂N₂O₃S | 1009.83 | 9.80 | 26.96 |
| 1497 | C₈₆H₁₆₉N₃O₇S₃ | 1453.51 | 9.01 | 39.01 | 1697 | C₆₇H₁₃₂N₂O₆S | 1093.86 | 9.79 | 27.78 |
| 1498 | C₆₈H₁₂₇N₃O₇S₃ | 1194.98 | 9.01 | 27.98 | 1698 | C₆₇H₁₃₂N₂O₆S | 1093.86 | 9.79 | 27.78 |
| 1499 | C₆₈H₁₂₇N₃O₇S₃ | 1194.98 | 9.01 | 27.89 | 1699 | C₆₇H₁₃₂N₂O₆S | 1093.86 | 9.81 | 27.78 |
| 1500 | C₄₇H₉₁N₃O₇S₃ | 906.46 | 9.01 | 18.77 | 1700 | C₆₇H₁₃₂N₂O₆S | 1093.86 | 9.81 | 27.78 |
| 1501 | C₅₉H₁₀₃N₃O₇S₃ | 1062.69 | 9.01 | 22.21 | 1701 | C₆₄H₁₃₃N₃O₃ | 992.79 | 9.78 | 26.31 |
| 1502 | C₆₅H₁₁₅N₃O₇S₃ | 1146.85 | 9.01 | 24.22 | 1702 | C₆₄H₁₃₃N₃O₃ | 992.79 | 9.78 | 26.31 |
| 1503 | C₅₃H₉₇N₃O₇S₃ | 984.57 | 9.01 | 20.49 | 1703 | C₆₇H₁₃₃N₃O₆ | 1076.82 | 9.78 | 27.13 |
| 1504 | C₆₅H₁₂₁N₃O₇S₃ | 1152.90 | 9.01 | 26.84 | 1704 | C₆₇H₁₃₃N₃O₆ | 1076.82 | 9.78 | 27.13 |
| 1505 | C₆₅H₁₂₇N₃O₇S₃ | 1158.95 | 9.01 | 28.29 | 1705 | C₆₇H₁₃₃N₃O₆ | 1076.82 | 9.79 | 27.13 |
| 1506 | C₇₁H₁₁₅N₃O₇S₃ | 1218.92 | 9.01 | 27.44 | 1706 | C₆₇H₁₃₃N₃O₆ | 1076.82 | 9.79 | 27.13 |
| 1507 | C₈₆H₁₄₅N₃O₇S₃ | 1429.32 | 9.01 | 35.13 | 1707 | C₆₄H₁₃₂N₂O₃S₂ | 1041.89 | 9.65 | 27.39 |
| 1508 | C₆₂H₁₂₁N₃O₇S₃ | 1116.86 | 9.01 | 26.05 | 1708 | C₆₄H₁₃₂N₂O₃S₂ | 1041.89 | 9.65 | 27.39 |
| 1509 | C₇₄H₁₄₅N₃O₇S₃ | 1285.19 | 9.01 | 32.39 | 1709 | C₆₇H₁₃₂N₂O₆S₂ | 1125.92 | 9.65 | 28.21 |
| 1510 | C₉₅H₁₈₇N₃O₇S₃ | 1579.76 | 9.01 | 43.50 | 1710 | C₆₇H₁₃₂N₂O₆S₂ | 1125.92 | 9.65 | 28.21 |
| 1511 | C₆₈H₁₃₃N₃O₇S₃ | 1201.03 | 9.01 | 29.22 | 1711 | C₆₇H₁₃₂N₂O₆S₂ | 1125.92 | 9.65 | 28.21 |
| 1512 | C₆₈H₁₃₃N₃O₇S₃ | 1201.03 | 9.01 | 29.22 | 1712 | C₆₇H₁₃₂N₂O₆S₂ | 1125.92 | 9.65 | 28.21 |
| 1513 | C₁₀₁H₁₉₉N₃O₇S₃ | 1663.92 | 9.01 | 46.95 | 1713 | C₆₅H₁₃₂N₂O₄ | 1005.78 | 8.95 | 26.46 |
| 1514 | C₉₅H₁₈₇N₃O₇S₃ | 1579.76 | 9.01 | 43.77 | 1714 | C₆₅H₁₃₂N₂O₄ | 1005.78 | 8.95 | 26.46 |
| 1515 | C₈₆H₁₆₉N₃O₇S₃ | 1453.51 | 9.01 | 39.01 | 1715 | C₆₈H₁₃₂N₂O₇ | 1089.81 | 8.95 | 27.28 |
| 1516 | C₆₈H₁₂₇N₃O₇S₃ | 1194.98 | 9.01 | 27.98 | 1716 | C₆₈H₁₃₂N₂O₇ | 1089.81 | 8.95 | 27.28 |
| 1517 | C₆₈H₁₂₇N₃O₇S₃ | 1194.98 | 9.01 | 27.89 | 1717 | C₆₈H₁₃₂N₂O₇ | 1089.81 | 8.96 | 27.28 |
| 1518 | C₄₇H₉₁N₃O₇S₃ | 906.46 | 9.01 | 18.77 | 1718 | C₆₈H₁₃₂N₂O₇ | 1089.81 | 8.96 | 27.28 |
| 1519 | C₅₉H₁₀₃N₃O₇S₃ | 1062.69 | 9.01 | 22.21 | 1719 | C₆₅H₁₃₂N₂O₅ | 1021.78 | 8.83 | 26.64 |
| 1520 | C₆₅H₁₁₅N₃O₇S₃ | 1146.85 | 9.01 | 24.22 | 1720 | C₆₅H₁₃₂N₂O₅ | 1021.78 | 8.83 | 26.64 |
| 1521 | C₅₃H₉₇N₃O₇S₃ | 984.57 | 9.01 | 20.49 | 1721 | C₆₈H₁₃₂N₂O₈ | 1105.81 | 8.83 | 27.46 |
| 1522 | C₆₅H₁₂₁N₃O₇S₃ | 1152.90 | 9.01 | 26.84 | 1722 | C₆₈H₁₃₂N₂O₈ | 1105.81 | 8.83 | 27.46 |
| 1523 | C₆₅H₁₂₇N₃O₇S₃ | 1158.95 | 9.01 | 28.29 | 1723 | C₆₈H₁₃₂N₂O₈ | 1105.81 | 8.83 | 27.46 |
| 1524 | C₇₁H₁₁₅N₃O₇S₃ | 1218.92 | 9.01 | 25.64 | 1724 | C₆₈H₁₃₂N₂O₈ | 1105.81 | 8.83 | 27.46 |
| 1525 | C₈₆H₁₄₅N₃O₇S₃ | 1429.32 | 9.01 | 33.34 | 1725 | C₆₅H₁₃₂N₂O₅ | 1021.78 | 8.67 | 26.64 |
| 1526 | C₆₂H₁₂₁N₃O₄S₆ | 1165.07 | 9.01 | 27.16 | 1726 | C₆₅H₁₃₂N₂O₅ | 1021.78 | 8.67 | 26.64 |
| 1527 | C₇₄H₁₄₅N₃O₄S₆ | 1333.39 | 9.01 | 33.50 | 1727 | C₆₈H₁₃₂N₂O₈ | 1105.81 | 8.67 | 27.46 |
| 1528 | C₉₅H₁₈₇N₃O₄S₆ | 1627.96 | 9.01 | 44.61 | 1728 | C₆₈H₁₃₂N₂O₈ | 1105.81 | 8.67 | 27.46 |
| 1529 | C₆₈H₁₃₃N₃O₄S₆ | 1249.23 | 9.01 | 30.33 | 1729 | C₆₈H₁₃₂N₂O₈ | 1105.81 | 8.67 | 27.46 |
| 1530 | C₆₈H₁₃₃N₃O₄S₆ | 1249.23 | 9.01 | 30.33 | 1730 | C₆₈H₁₃₂N₂O₈ | 1105.81 | 8.67 | 27.46 |
| 1531 | C₁₀₁H₁₉₉N₃O₄S₆ | 1712.12 | 9.01 | 48.06 | 1731 | C₆₅H₁₃₂N₂O₆ | 1037.78 | 8.66 | 26.60 |
| 1532 | C₉₅H₁₈₇N₃O₄S₆ | 1627.96 | 9.01 | 44.88 | 1732 | C₆₅H₁₃₂N₂O₆ | 1037.78 | 8.66 | 26.60 |
| 1533 | C₈₆H₁₆₉N₃O₄S₆ | 1501.72 | 9.01 | 40.12 | 1733 | C₆₈H₁₃₂N₂O₉ | 1121.81 | 8.66 | 27.43 |
| 1534 | C₆₈H₁₂₇N₃O₄S₆ | 1243.18 | 9.01 | 29.09 | 1734 | C₆₈H₁₃₂N₂O₉ | 1121.81 | 8.66 | 27.43 |
| 1535 | C₆₈H₁₂₇N₃O₄S₆ | 1243.18 | 9.01 | 29.00 | 1735 | C₆₈H₁₃₂N₂O₉ | 1121.81 | 8.66 | 27.43 |
| 1536 | C₄₇H₉₁N₃O₄S₆ | 954.66 | 9.01 | 19.88 | 1736 | C₆₈H₁₃₂N₂O₉ | 1121.81 | 8.66 | 27.43 |
| 1537 | C₅₉H₁₀₃N₃O₄S₆ | 1110.89 | 9.01 | 23.32 | 1737 | C₆₅H₁₃₃N₃O₄ | 1020.80 | 8.84 | 26.04 |
| 1538 | C₆₅H₁₁₅N₃O₄S₆ | 1195.05 | 9.01 | 25.33 | 1738 | C₆₅H₁₃₃N₃O₄ | 1020.80 | 8.84 | 26.04 |
| 1539 | C₅₃H₉₇N₃O₄S₆ | 1032.78 | 9.01 | 21.60 | 1739 | C₆₈H₁₃₃N₃O₇ | 1104.83 | 8.83 | 26.86 |
| 1540 | C₆₅H₁₂₁N₃O₄S₆ | 1201.10 | 9.01 | 27.95 | 1740 | C₆₈H₁₃₃N₃O₇ | 1104.83 | 8.84 | 26.86 |
| 1541 | C₆₅H₁₂₇N₃O₄S₆ | 1207.15 | 9.01 | 29.40 | 1741 | C₆₈H₁₃₃N₃O₇ | 1104.83 | 8.84 | 26.86 |
| 1542 | C₇₁H₁₁₅N₃O₄S₆ | 1267.12 | 9.01 | 28.55 | 1742 | C₆₈H₁₃₃N₃O₇ | 1104.83 | 8.84 | 26.86 |
| 1543 | C₈₆H₁₄₅N₃O₄S₆ | 1477.52 | 9.01 | 36.24 | 1743 | C₆₅H₁₃₃N₃O₅ | 1036.79 | 8.85 | 26.48 |
| 1544 | C₆₅H₁₃₆N₃O₁₀P₃ | 1212.74 | 9.01 | 21.98 | 1744 | C₆₅H₁₃₃N₃O₅ | 1036.79 | 8.85 | 26.48 |
| 1545 | C₇₇H₁₆₀N₃O₁₀P₃ | 1381.06 | 9.01 | 28.33 | 1745 | C₆₈H₁₃₃N₃O₈ | 1120.82 | 8.85 | 27.30 |
| 1546 | C₉₈H₂₀₂N₃O₁₀P₃ | 1675.63 | 9.01 | 39.44 | 1746 | C₆₈H₁₃₃N₃O₈ | 1120.82 | 8.85 | 27.30 |
| 1547 | C₇₁H₁₄₈N₃O₁₀P₃ | 1296.90 | 9.01 | 25.16 | 1747 | C₆₈H₁₃₃N₃O₈ | 1120.82 | 8.85 | 27.30 |
| 1548 | C₇₁H₁₄₈N₃O₁₀P₃ | 1296.90 | 9.01 | 25.16 | 1748 | C₆₈H₁₃₃N₃O₈ | 1120.82 | 8.85 | 27.30 |
| 1549 | C₁₀₄H₂₁₄N₃O₁₀P₃ | 1759.79 | 9.01 | 42.88 | 1749 | C₆₅H₁₃₃N₃O₅ | 1036.79 | 8.75 | 26.48 |
| 1550 | C₉₈H₂₀₂N₃O₁₀P₃ | 1675.63 | 9.01 | 39.71 | 1750 | C₆₅H₁₃₃N₃O₅ | 1036.79 | 8.75 | 26.48 |
| 1551 | C₈₉H₁₈₄N₃O₁₀P₃ | 1549.38 | 9.01 | 34.95 | 1751 | C₆₈H₁₃₃N₃O₈ | 1120.82 | 8.75 | 27.30 |
| 1552 | C₇₁H₁₄₂N₃O₁₀P₃ | 1290.85 | 9.01 | 23.91 | 1752 | C₆₈H₁₃₃N₃O₈ | 1120.82 | 8.75 | 27.30 |
| 1553 | C₇₁H₁₄₂N₃O₁₀P₃ | 1290.85 | 9.01 | 23.82 | 1753 | C₆₈H₁₃₃N₃O₈ | 1120.82 | 8.75 | 27.30 |
| 1554 | C₅₀H₁₀₆N₃O₁₀P₃ | 1002.33 | 9.01 | 14.71 | 1754 | C₆₈H₁₃₃N₃O₈ | 1120.82 | 8.75 | 27.30 |
| 1555 | C₆₂H₁₁₈N₃O₁₀P₃ | 1158.56 | 9.01 | 18.15 | 1755 | C₆₅H₁₃₄N₄O₄ | 1035.81 | 8.95 | 26.29 |
| 1556 | C₆₈H₁₃₀N₃O₁₀P₃ | 1242.72 | 9.01 | 20.16 | 1756 | C₆₅H₁₃₄N₄O₄ | 1035.81 | 8.95 | 26.29 |
| 1557 | C₅₆H₁₁₂N₃O₁₀P₃ | 1080.44 | 9.01 | 16.43 | 1757 | C₆₈H₁₃₄N₄O₇ | 1119.84 | 8.95 | 27.11 |
| 1558 | C₆₈H₁₃₆N₃O₁₀P₃ | 1248.77 | 9.01 | 22.78 | 1758 | C₆₈H₁₃₄N₄O₇ | 1119.84 | 8.95 | 27.11 |
| 1559 | C₆₈H₁₄₂N₃O₁₀P₃ | 1254.82 | 9.01 | 24.23 | 1759 | C₆₈H₁₃₄N₄O₇ | 1119.84 | 8.95 | 27.11 |
| 1560 | C₇₄H₁₃₀N₃O₁₀P₃ | 1314.79 | 9.01 | 23.74 | 1760 | C₆₈H₁₃₄N₄O₇ | 1119.84 | 8.95 | 27.11 |
| 1561 | C₈₉H₁₆₀N₃O₁₀P₃ | 1525.19 | 9.01 | 31.43 | 1761 | C₆₅H₁₃₂N₂O₃S | 1021.84 | 9.29 | 26.71 |
| 1562 | C₆₅H₁₃₆N₃O₁₀P₃ | 1212.74 | 9.01 | 21.98 | 1762 | C₆₅H₁₃₂N₂O₃S | 1021.84 | 9.29 | 26.71 |
| 1563 | C₇₇H₁₆₀N₃O₁₀P₃ | 1381.06 | 9.01 | 28.33 | 1763 | C₆₈H₁₃₂N₂O₆S | 1105.87 | 9.29 | 27.53 |
| 1564 | C₉₈H₂₀₂N₃O₁₀P₃ | 1675.63 | 9.01 | 39.44 | 1764 | C₆₈H₁₃₂N₂O₆S | 1105.87 | 9.29 | 27.53 |
| 1565 | C₇₁H₁₄₈N₃O₁₀P₃ | 1296.90 | 9.01 | 25.16 | 1765 | C₆₈H₁₃₂N₂O₆S | 1105.87 | 9.31 | 27.53 |
| 1566 | C₇₁H₁₄₈N₃O₁₀P₃ | 1296.90 | 9.01 | 25.16 | 1766 | C₆₈H₁₃₂N₂O₆S | 1105.87 | 9.31 | 27.53 |
| 1567 | C₁₀₄H₂₁₄N₃O₁₀P₃ | 1759.79 | 9.01 | 42.88 | 1767 | C₆₅H₁₃₂N₂O₃S₂ | 1053.90 | 9.35 | 27.19 |
| 1568 | C₉₈H₂₀₂N₃O₁₀P₃ | 1675.63 | 9.01 | 39.71 | 1768 | C₆₅H₁₃₂N₂O₃S₂ | 1053.90 | 9.35 | 27.19 |
| 1569 | C₈₉H₁₈₄N₃O₁₀P₃ | 1549.38 | 9.01 | 34.95 | 1769 | C₆₈H₁₃₂N₂O₆S₂ | 1137.93 | 9.35 | 28.02 |
| 1570 | C₇₁H₁₄₂N₃O₁₀P₃ | 1290.85 | 9.01 | 23.91 | 1770 | C₆₈H₁₃₂N₂O₆S₂ | 1137.93 | 9.35 | 28.02 |
| 1571 | C₇₁H₁₄₂N₃O₁₀P₃ | 1290.85 | 9.01 | 23.82 | 1771 | C₆₈H₁₃₂N₂O₆S₂ | 1137.93 | 9.35 | 28.02 |
| 1572 | C₅₀H₁₀₆N₃O₁₀P₃ | 1002.33 | 9.01 | 14.71 | 1772 | C₆₈H₁₃₂N₂O₆S₂ | 1137.93 | 9.35 | 28.02 |
| 1573 | C₆₂H₁₁₈N₃O₁₀P₃ | 1158.56 | 9.01 | 18.15 | 1773 | C₆₅H₁₃₂N₂O₃S₂ | 1053.90 | 9.13 | 27.19 |
| 1574 | C₆₈H₁₃₀N₃O₁₀P₃ | 1242.72 | 9.01 | 20.16 | 1774 | C₆₅H₁₃₂N₂O₃S₂ | 1053.90 | 9.13 | 27.19 |
| 1575 | C₅₆H₁₁₂N₃O₁₀P₃ | 1080.44 | 9.01 | 16.43 | 1775 | C₆₈H₁₃₂N₂O₆S₂ | 1137.93 | 9.13 | 28.02 |
| 1576 | C₆₈H₁₃₆N₃O₁₀P₃ | 1248.77 | 9.01 | 22.78 | 1776 | C₆₈H₁₃₂N₂O₆S₂ | 1137.93 | 9.13 | 28.02 |
| 1577 | C₆₈H₁₄₂N₃O₁₀P₃ | 1254.82 | 9.01 | 24.23 | 1777 | C₆₈H₁₃₂N₂O₆S₂ | 1137.93 | 9.14 | 28.02 |
| 1578 | C₇₄H₁₃₀N₃O₁₀P₃ | 1314.79 | 9.01 | 24.34 | 1778 | C₆₈H₁₃₂N₂O₆S₂ | 1137.93 | 9.14 | 28.02 |
| 1579 | C₈₉H₁₆₀N₃O₁₀P₃ | 1525.19 | 9.01 | 32.03 | 1779 | C₆₅H₁₃₂N₂O₄S | 1037.84 | 9.20 | 26.95 |
| 1580 | C₅₉H₁₂₄N₆O₇S₃ | 1125.88 | 9.01 | 21.35 | 1780 | C₆₅H₁₃₂N₂O₄S | 1037.84 | 9.20 | 26.95 |
| 1581 | C₇₁H₁₄₈N₆O₇S₃ | 1294.20 | 9.01 | 27.70 | 1781 | C₆₈H₁₃₂N₂O₇S | 1121.87 | 9.20 | 27.78 |
| 1582 | C₉₂H₁₉₀N₆O₇S₃ | 1588.77 | 9.01 | 38.81 | 1782 | C₆₈H₁₃₂N₂O₇S | 1121.87 | 9.20 | 27.78 |
| 1583 | C₆₅H₁₃₆N₆O₇S₃ | 1210.04 | 9.01 | 24.53 | 1783 | C₆₈H₁₃₂N₂O₇S | 1121.87 | 9.20 | 27.78 |
| 1584 | C₆₅H₁₃₆N₆O₇S₃ | 1210.04 | 9.01 | 24.53 | 1784 | C₆₈H₁₃₂N₂O₇S | 1121.87 | 9.20 | 27.78 |
| 1585 | C₉₈H₂₀₂N₆O₇S₃ | 1672.93 | 9.01 | 42.26 | 1785 | C₆₅H₁₃₂N₂O₄S | 1037.84 | 8.80 | 26.95 |
| 1586 | C₉₂H₁₉₀N₆O₇S₃ | 1588.77 | 9.01 | 39.08 | 1786 | C₆₅H₁₃₂N₂O₄S | 1037.84 | 8.80 | 26.95 |
| 1587 | C₈₃H₁₇₂N₆O₇S₃ | 1462.52 | 9.01 | 34.32 | 1787 | C₆₈H₁₃₂N₂O₇S | 1121.87 | 8.80 | 27.78 |
| 1588 | C₆₅H₁₃₀N₆O₇S₃ | 1203.99 | 9.01 | 23.29 | 1788 | C₆₈H₁₃₂N₂O₇S | 1121.87 | 8.80 | 27.78 |
| 1589 | C₆₅H₁₃₀N₆O₇S₃ | 1203.99 | 9.01 | 23.20 | 1789 | C₆₈H₁₃₂N₂O₇S | 1121.87 | 8.80 | 27.78 |
| 1590 | C₄₄H₉₄N₆O₇S₃ | 915.47 | 9.01 | 14.08 | 1790 | C₆₈H₁₃₂N₂O₇S | 1121.87 | 8.80 | 27.78 |
| 1591 | C₅₆H₁₀₆N₆O₇S₃ | 1071.70 | 9.01 | 17.52 | 1791 | C₆₅H₁₃₂N₂O₅S | 1053.84 | 8.70 | 27.15 |
| 1592 | C₆₂H₁₁₈N₆O₇S₃ | 1155.86 | 9.01 | 19.53 | 1792 | C₆₅H₁₃₂N₂O₅S | 1053.84 | 8.70 | 27.15 |
| 1593 | C₅₀H₁₀₀N₆O₇S₃ | 993.59 | 9.01 | 15.80 | 1793 | C₆₈H₁₃₂N₂O₈S | 1137.87 | 8.70 | 27.97 |
| 1594 | C₆₂H₁₂₄N₆O₇S₃ | 1161.91 | 9.01 | 22.15 | 1794 | C₆₈H₁₃₂N₂O₈S | 1137.87 | 8.70 | 27.97 |
| 1595 | C₆₂H₁₃₀N₆O₇S₃ | 1167.96 | 9.01 | 23.60 | 1795 | C₆₈H₁₃₂N₂O₈S | 1137.87 | 8.70 | 27.97 |
| 1596 | C₆₈H₁₁₈N₆O₇S₃ | 1227.93 | 9.01 | 23.53 | 1796 | C₆₈H₁₃₂N₂O₈S | 1137.87 | 8.70 | 27.97 |
| 1597 | C₈₃H₁₄₈N₆O₇S₃ | 1438.33 | 9.01 | 31.23 | 1797 | C₆₅H₁₃₂N₂O₅S | 1053.84 | 9.00 | 27.15 |
| 1598 | C₅₉H₁₂₄N₆O₇S₃ | 1125.88 | 9.01 | 21.35 | 1798 | C₆₅H₁₃₂N₂O₅S | 1053.84 | 9.00 | 27.15 |
| 1599 | C₇₁H₁₄₈N₆O₇S₃ | 1294.20 | 9.01 | 27.70 | 1799 | C₆₈H₁₃₂N₂O₈S | 1137.87 | 9.00 | 27.97 |
| 1600 | C₉₂H₁₉₀N₆O₇S₃ | 1588.77 | 9.01 | 38.81 | 1800 | C₆₈H₁₃₂N₂O₈S | 1137.87 | 9.00 | 27.97 |
| 1801 | C₆₈H₁₃₂N₂O₈S | 1137.87 | 9.00 | 27.97 | 2001 | C₆₉H₁₃₇N₅O₅ | 1116.88 | 7.65 | 24.63 |
| 1802 | C₆₈H₁₃₂N₂O₈S | 1137.87 | 9.00 | 27.97 | 2002 | C₆₉H₁₃₇N₅O₅ | 1116.88 | 7.65 | 24.63 |
| 1803 | C₆₅H₁₃₂N₂O₄S₂ | 1069.90 | 9.06 | 27.39 | 2003 | C₇₂H₁₃₇N₅O₈ | 1200.91 | 7.65 | 25.45 |
| 1804 | C₆₅H₁₃₂N₂O₄S₂ | 1069.90 | 9.06 | 27.39 | 2004 | C₇₂H₁₃₇N₅O₈ | 1200.91 | 7.65 | 25.45 |
| 1805 | C₆₈H₁₃₂N₂O₇S₂ | 1153.93 | 9.06 | 28.21 | 2005 | C₇₂H₁₃₇N₅O₈ | 1200.91 | 7.65 | 25.45 |
| 1806 | C₆₈H₁₃₂N₂O₇S₂ | 1153.93 | 9.06 | 28.21 | 2006 | C₇₂H₁₃₇N₅O₈ | 1200.91 | 7.65 | 25.45 |
| 1807 | C₆₈H₁₃₂N₂O₇S₂ | 1153.93 | 9.06 | 28.21 | 2007 | C₆₉H₁₃₆N₄O₅ | 1101.87 | 7.90 | 25.58 |
| 1808 | C₆₈H₁₃₂N₂O₇S₂ | 1153.93 | 9.06 | 28.21 | 2008 | C₆₉H₁₃₆N₄O₅ | 1101.87 | 7.90 | 25.58 |
| 1809 | C₆₆H₁₃₇N₂O₆P | 1085.80 | 8.79 | 26.39 | 2009 | C₇₂H₁₃₆N₄O₈ | 1185.90 | 7.90 | 26.41 |
| 1810 | C₆₆H₁₃₇N₂O₆P | 1085.80 | 8.79 | 26.39 | 2010 | C₇₂H₁₃₆N₄O₈ | 1185.90 | 7.90 | 26.41 |
| 1811 | C₆₉H₁₃₇N₂O₉P | 1169.83 | 8.79 | 27.21 | 2011 | C₇₂H₁₃₆N₄O₈ | 1185.90 | 7.90 | 26.41 |
| 1812 | C₆₉H₁₃₇N₂O₉P | 1169.83 | 8.79 | 27.21 | 2012 | C₇₂H₁₃₆N₄O₈ | 1185.90 | 7.90 | 26.41 |
| 1813 | C₆₉H₁₃₇N₂O₉P | 1169.83 | 8.79 | 27.21 | 2013 | C₆₉H₁₃₇N₅O₄ | 1100.89 | 8.23 | 25.73 |
| 1814 | C₆₉H₁₃₇N₂O₉P | 1169.83 | 8.79 | 27.21 | 2014 | C₆₉H₁₃₇N₅O₄ | 1100.89 | 8.23 | 25.73 |
| 1815 | C₆₆H₁₃₇N₂O₆P | 1085.80 | 8.41 | 26.39 | 2015 | C₇₂H₁₃₇N₅O₇ | 1184.92 | 8.23 | 26.56 |
| 1816 | C₆₆H₁₃₇N₂O₆P | 1085.80 | 8.41 | 26.39 | 2016 | C₇₂H₁₃₇N₅O₇ | 1184.92 | 8.23 | 26.56 |
| 1817 | C₆₉H₁₃₇N₂O₉P | 1169.83 | 8.41 | 27.21 | 2017 | C₇₂H₁₃₇N₅O₇ | 1184.92 | 8.23 | 26.56 |
| 1818 | C₆₉H₁₃₇N₂O₉P | 1169.83 | 8.41 | 27.21 | 2018 | C₇₂H₁₃₇N₅O₇ | 1184.92 | 8.23 | 26.56 |
| 1819 | C₆₉H₁₃₇N₂O₉P | 1169.83 | 8.41 | 27.21 | 2019 | C₆₉H₁₃₇N₅O₄ | 1100.89 | 7.75 | 26.13 |
| 1820 | C₆₉H₁₃₇N₂O₉P | 1169.83 | 8.41 | 27.21 | 2020 | C₆₉H₁₃₇N₅O₄ | 1100.89 | 7.75 | 26.13 |
| 1821 | C₆₄H₁₃₃N₃O₅S | 1056.84 | 8.74 | 26.14 | 2021 | C₇₂H₁₃₇N₅O₇ | 1184.92 | 7.75 | 26.95 |
| 1822 | C₆₄H₁₃₃N₃O₅S | 1056.84 | 8.74 | 26.14 | 2022 | C₇₂H₁₃₇N₅O₇ | 1184.92 | 7.75 | 26.95 |
| 1823 | C₆₇H₁₃₃N₃O₈S | 1140.87 | 8.74 | 26.96 | 2023 | C₇₂H₁₃₇N₅O₇ | 1184.92 | 7.75 | 26.95 |
| 1824 | C₆₇H₁₃₃N₃O₈S | 1140.87 | 8.74 | 26.96 | 2024 | C₇₂H₁₃₇N₅O₇ | 1184.92 | 7.75 | 26.95 |
| 1825 | C₆₇H₁₃₃N₃O₈S | 1140.87 | 8.74 | 26.96 | 2025 | C₆₈H₁₃₆N₆O₄ | 1101.87 | 7.19 | 25.85 |
| 1826 | C₆₇H₁₃₃N₃O₈S | 1140.87 | 8.74 | 26.96 | 2026 | C₆₈H₁₃₆N₆O₄ | 1101.87 | 7.19 | 25.85 |
| 1827 | C₆₄H₁₃₃N₃O₅S | 1056.84 | 7.78 | 26.14 | 2027 | C₇₁H₁₃₆N₆O₇ | 1185.90 | 7.19 | 26.67 |
| 1828 | C₆₄H₁₃₃N₃O₅S | 1056.84 | 7.78 | 26.14 | 2028 | C₇₁H₁₃₆N₆O₇ | 1185.90 | 7.19 | 26.67 |
| 1829 | C₆₇H₁₃₃N₃O₈S | 1140.87 | 7.78 | 26.96 | 2029 | C₇₁H₁₃₆N₆O₇ | 1185.90 | 7.19 | 26.67 |
| 1830 | C₆₇H₁₃₃N₃O₈S | 1140.87 | 7.78 | 26.96 | 2030 | C₇₁H₁₃₆N₆O₇ | 1185.90 | 7.19 | 26.67 |
| 1831 | C₆₇H₁₃₃N₃O₈S | 1140.87 | 7.78 | 26.96 | 2031 | C₆₉H₁₃₇N₅O₄S | 1132.95 | 7.95 | 27.26 |
| 1832 | C₆₇H₁₃₃N₃O₈S | 1140.87 | 7.78 | 26.96 | 2032 | C₆₉H₁₃₇N₅O₄S | 1132.95 | 7.95 | 27.26 |
| 1833 | C₆₄H₁₃₂N₂O₆S | 1057.83 | 7.60 | 26.33 | 2033 | C₈₉H₁₇₆N₆O₉S | 1506.48 | 7.74 | 33.24 |
| 1834 | C₆₄H₁₃₂N₂O₆S | 1057.83 | 7.60 | 26.33 | 2034 | C₈₈H₁₇₅N₅O₉ | 1447.39 | 8.17 | 35.73 |
| 1835 | C₆₇H₁₃₂N₂O₉S | 1141.86 | 7.60 | 27.15 | 2035 | C₇₂H₁₃₇N₅O₇S | 1216.98 | 7.96 | 28.08 |
| 1836 | C₆₇H₁₃₂N₂O₉S | 1141.86 | 7.60 | 27.15 | 2036 | C₇₂H₁₃₇N₅O₇S | 1216.98 | 7.96 | 28.08 |
| 1837 | C₆₇H₁₃₂N₂O₉S | 1141.86 | 7.60 | 27.15 | 2037 | C₇₁H₁₃₈N₈O₅ | 1183.94 | 7.59 | 24.76 |
| 1838 | C₆₇H₁₃₂N₂O₉S | 1141.86 | 7.60 | 27.15 | 2038 | C₇₁H₁₃₈N₈O₅ | 1183.94 | 7.59 | 24.76 |
| 1839 | C₆₄H₁₃₂N₂O₆S | 1057.83 | 8.32 | 26.33 | 2039 | C₉₃H₁₇₉N₅O₁₁S₃ | 1639.66 | 7.56 | 35.27 |
| 1840 | C₆₄H₁₃₂N₂O₆S | 1057.83 | 8.32 | 26.33 | 2040 | C₉₃H₁₈₀N₅O₁₂PS | 1623.52 | 8.52 | 37.28 |
| 1841 | C₆₇H₁₃₂N₂O₉S | 1141.86 | 8.32 | 27.15 | 2041 | C₇₄H₁₃₈N₈O₈ | 1267.97 | 7.59 | 25.58 |
| 1842 | C₆₇H₁₃₂N₂O₉S | 1141.86 | 8.32 | 27.15 | 2042 | C₇₄H₁₃₈N₈O₈ | 1267.97 | 7.59 | 25.58 |
| 1843 | C₆₇H₁₃₂N₂O₉S | 1141.86 | 8.32 | 27.15 | 2043 | C₆₈H₁₄₀N₆O₄ | 1105.91 | 8.62 | 25.32 |
| 1844 | C₆₇H₁₃₂N₂O₉S | 1141.86 | 8.32 | 27.15 | 2044 | C₆₈H₁₄₀N₆O₄ | 1105.91 | 8.62 | 25.32 |
| 1845 | C₆₄H₁₃₂N₂O₇S | 1073.83 | 8.15 | 26.57 | 2045 | C₇₁H₁₄₀N₆O₇ | 1189.94 | 8.62 | 26.14 |
| 1846 | C₆₄H₁₃₂N₂O₇S | 1073.83 | 8.15 | 26.57 | 2046 | C₇₁H₁₄₀N₆O₇ | 1189.94 | 8.62 | 26.14 |
| 1847 | C₆₇H₁₃₂N₂O₁₀S | 1157.86 | 8.15 | 27.39 | 2047 | C₇₁H₁₄₀N₆O₇ | 1189.94 | 8.62 | 26.14 |
| 1848 | C₆₇H₁₃₂N₂O₁₀S | 1157.86 | 8.15 | 27.39 | 2048 | C₇₁H₁₄₀N₆O₇ | 1189.94 | 8.62 | 26.14 |
| 1849 | C₆₇H₁₃₂N₂O₁₀S | 1157.86 | 8.15 | 27.39 | 2049 | C₆₈H₁₃₉N₅O₄ | 1090.89 | 8.61 | 25.54 |
| 1850 | C₆₇H₁₃₂N₂O₁₀S | 1157.86 | 8.15 | 27.39 | 2050 | C₆₈H₁₃₉N₅O₄ | 1090.89 | 8.61 | 25.54 |
| 1851 | C₆₄H₁₃₂N₂O₅S | 1041.83 | 7.83 | 25.84 | 2051 | C₇₁H₁₃₉N₅O₇ | 1174.92 | 8.61 | 26.36 |
| 1852 | C₆₄H₁₃₂N₂O₅S | 1041.83 | 7.83 | 25.84 | 2052 | C₇₁H₁₃₉N₅O₇ | 1174.92 | 8.61 | 26.36 |
| 1853 | C₆₇H₁₃₂N₂O₈S | 1125.86 | 7.83 | 26.66 | 2053 | C₇₁H₁₃₉N₅O₇ | 1174.92 | 8.61 | 26.36 |
| 1854 | C₆₇H₁₃₂N₂O₈S | 1125.86 | 7.83 | 26.66 | 2054 | C₇₁H₁₃₉N₅O₇ | 1174.92 | 8.61 | 26.36 |
| 1855 | C₆₇H₁₃₂N₂O₈S | 1125.86 | 7.84 | 26.66 | 2055 | C₆₉H₁₃₉N₅O₅ | 1118.90 | 7.82 | 25.64 |
| 1856 | C₆₇H₁₃₂N₂O₈S | 1125.86 | 7.84 | 26.66 | 2056 | C₆₉H₁₃₉N₅O₅ | 1118.90 | 7.82 | 25.64 |
| 1857 | C67H137N3O4 | 1048.85 | 9.07 | 27.10 | 2057 | C₉₀H₁₇₆N₂OS₈ | 1558.89 | 9.96 | 42.56 |
| 1858 | C₆₇H₁₃₇N₃O₄ | 1048.85 | 9.07 | 7.10 | 2058 | C₈₉H₁₇₄N₂OS₈ | 1544.86 | 8.77 | 40.62 |
| 1859 | C₇₀H₁₃₇N₃O₇ | 1132.88 | 9.07 | 27.92 | 2059 | C₇₂H₁₃₉N₅O₈ | 1202.93 | 7.82 | 26.46 |
| 1860 | C₇₀H₁₃₇N₃O₇ | 1132.88 | 9.07 | 27.92 | 2060 | C72H139N5O8 | 1202.93 | 7.82 | 26.46 |
| 1861 | C₇₀H₁₃₇N₃O₇ | 1132.88 | 9.07 | 27.92 | 2061 | C₇₀H₁₄₁N₅O₅ | 1132.93 | 7.84 | 26.35 |
| 1862 | C₇₀H₁₃₇N₃O₇ | 1132.88 | 9.07 | 27.92 | 2062 | C₇₀H₁₄₁N₅O₅ | 1132.93 | 7.84 | 26.35 |
| 1863 | C₆₉H₁₄₁N₃O₄ | 1076.90 | 9.10 | 28.15 | 2063 | C₇₃H₁₄₁N₅O₈ | 1216.96 | 7.84 | 27.17 |
| 1864 | C₆₉H₁₄₁N₃O₄ | 1076.90 | 9.09 | 28.15 | 2064 | C₇₃H₁₄₁N₅O₈ | 1216.96 | 7.84 | 27.17 |
| 1865 | C₇₂H₁₄₁N₃O₇ | 1160.93 | 9.10 | 28.98 | 2065 | C₇₃H₁₄₁N₅O₈ | 1216.96 | 7.84 | 27.17 |
| 1866 | C₉₅H₁₉₄N₄O₉S₅ | 1696.92 | 9.86 | 40.86 | 2066 | C₇₃H₁₄₁N₅O₈ | 1216.96 | 7.84 | 27.17 |
| 1867 | C₇₂H₁₄₁N₃O₇ | 1160.93 | 9.10 | 28.98 | 2067 | C₆₉H₁₃₇N₅O₆ | 1132.88 | 7.19 | 25.38 |
| 1868 | C₇₂H₁₄₁N₃O₇ | 1160.93 | 9.10 | 28.98 | 2068 | C₆₉H₁₃₇N₅O₆ | 1132.88 | 7.19 | 25.38 |
| 1869 | C₇₁H₁₄₅N₃O₄ | 1104.96 | 9.12 | 29.21 | 2069 | C₇₂H₁₃₇N₅O₉ | 1216.91 | 7.06 | 26.20 |
| 1870 | C₇₁H₁₄₅N₃O₄ | 1104.96 | 9.12 | 29.21 | 2070 | C₇₂H₁₃₇N₅O₉ | 1216.91 | 7.06 | 26.20 |
| 1871 | C₇₄H₁₄₅N₃O₇ | 1188.99 | 9.12 | 30.03 | 2071 | C₇₂H₁₃₇N₅O₉ | 1216.91 | 7.06 | 26.20 |
| 1872 | C₉₆H₁₉₁NO₁₄S₅ | 1743.88 | 7.67 | 41.25 | 2072 | C₇₂H₁₃₇N₅O₉ | 1216.91 | 7.06 | 26.20 |
| 1873 | C₇₄H₁₄₅N₃O₇ | 1188.99 | 9.12 | 30.03 | 2073 | C₆₉H₁₃₈N₄O₆ | 1119.88 | 7.66 | 26.29 |
| 1874 | C₇₄H₁₄₅N₃O₇ | 1188.99 | 9.12 | 30.03 | 2074 | C₆₉H₁₃₈N₄O₆ | 1119.88 | 7.66 | 26.29 |
| 1875 | C₆₆H₁₃₅N₃O₄ | 1034.82 | 9.04 | 26.57 | 2075 | C₇₂H₁₃₈N₄O₉ | 1203.91 | 7.66 | 27.11 |
| 1876 | C₆₆H₁₃₅N₃O₄ | 1034.82 | 9.04 | 26.57 | 2076 | C₇₂H₁₃₈N₄O₉ | 1203.91 | 7.66 | 27.11 |
| 1877 | C₆₉H₁₃₅N₃O₇ | 1118.85 | 9.04 | 27.39 | 2077 | C₇₂H₁₃₈N₄O₉ | 1203.91 | 7.66 | 27.11 |
| 1878 | C₆₉H₁₃₅N₃O₇ | 1118.85 | 9.04 | 27.39 | 2078 | C₇₂H₁₃₈N₄O₉ | 1203.91 | 7.66 | 27.11 |
| 1879 | C₆₉H₁₃₅N₃O₇ | 1118.85 | 9.04 | 27.39 | 2079 | C₇₀H₁₄₀N₄O₅ | 1117.91 | 7.94 | 26.15 |
| 1880 | C₆₉H₁₃₅N₃O₇ | 1118.85 | 9.04 | 27.39 | 2080 | C₇₀H₁₄₀N₄O₅ | 1117.91 | 7.93 | 26.15 |
| 1881 | C₆₇H₁₃₇N₃O₄ | 1048.85 | 9.05 | 27.10 | 2081 | C₇₃H₁₄₀N₄O₈ | 1201.94 | 7.94 | 26.97 |
| 1882 | C₆₇H₁₃₇N₃O₄ | 1048.85 | 9.05 | 27.10 | 2082 | C₇₃H₁₄₀N₄O₈ | 1201.94 | 7.93 | 26.97 |
| 1883 | C₇₀H₁₃₇N₃O₇ | 1132.88 | 9.05 | 27.92 | 2083 | C₇₃H₁₄₀N₄O₈ | 1201.94 | 7.94 | 26.97 |
| 1884 | C₇₀H₁₃₇N₃O₇ | 1132.88 | 9.05 | 27.92 | 2084 | C₇₃H₁₄₀N₄O₈ | 1201.94 | 7.94 | 26.97 |
| 1885 | C₇₀H₁₃₇N₃O₇ | 1132.88 | 9.05 | 27.92 | 2085 | C₇₀H₁₃₈N₄O₆ | 1131.90 | 7.19 | 25.85 |
| 1886 | C₇₀H₁₃₇N₃O₇ | 1132.88 | 9.05 | 27.92 | 2086 | C₇₀H₁₃₈N₄O₆ | 1131.90 | 7.19 | 25.85 |
| 1887 | C₆₈H₁₃₉N₃O₄ | 1062.88 | 9.06 | 27.63 | 2087 | C₇₃H₁₃₈N₄O₉ | 1215.93 | 7.17 | 26.67 |
| 1888 | C₆₈H₁₃₉N₃O₄ | 1062.88 | 9.06 | 27.63 | 2088 | C₇₃H₁₃₈N₄O₉ | 1215.93 | 7.17 | 26.67 |
| 1889 | C₇₁H₁₃₉N₃O₇ | 1146.91 | 9.06 | 28.45 | 2089 | C₇₃H₁₃₈N₄O₉ | 1215.93 | 7.17 | 26.67 |
| 1890 | C₇₁H₁₃₉N₃O₇ | 1146.91 | 9.06 | 28.45 | 2090 | C₇₃H₁₃₈N₄O₉ | 1215.93 | 7.17 | 26.67 |
| 1891 | C₇₁H₁₃₉N₃O₇ | 1146.91 | 9.06 | 28.45 | 2091 | C₇₀H₁₃₉N₅O₆ | 1146.91 | 7.19 | 25.65 |
| 1892 | C₇₁H₁₃₉N₃O₇ | 1146.91 | 9.06 | 28.45 | 2092 | C₇₀H₁₃₉N₅O₆ | 1146.91 | 7.19 | 25.65 |
| 1893 | C₆₇H₁₃₇N₃O₄ | 1048.85 | 9.11 | 26.88 | 2093 | C₇₃H₁₃₉N₅O₉ | 1230.94 | 7.08 | 26.47 |
| 1894 | C₆₇H₁₃₇N₃O₄ | 1048.85 | 9.11 | 26.88 | 2094 | C₇₃H₁₃₉N₅O₉ | 1230.94 | 7.08 | 26.47 |
| 1895 | C₇₀H₁₃₇N₃O₇ | 1132.88 | 9.11 | 27.70 | 2095 | C₇₃H₁₃₉N₅O₉ | 1230.94 | 7.08 | 26.47 |
| 1896 | C₇₀H₁₃₇N₃O₇ | 1132.88 | 9.11 | 27.70 | 2096 | C₇₃H₁₃₉N₅O₉ | 1230.94 | 7.08 | 26.47 |
| 1897 | C₇₀H₁₃₇N₃O₇ | 1132.88 | 9.11 | 27.70 | 2097 | C₇₀H₁₄₁N₅O₅ | 1132.93 | 7.84 | 26.35 |
| 1898 | C₇₀H₁₃₇N₃O₇ | 1132.88 | 9.11 | 27.70 | 2098 | C₇₀H₁₄₁N₅O₅ | 1132.93 | 7.84 | 26.35 |
| 1899 | C₆₈H₁₃₉N₃O₄ | 1062.88 | 9.12 | 27.41 | 2099 | C₇₃H₁₄₁N₅O₈ | 1216.96 | 7.84 | 27.17 |
| 1900 | C₆₈H₁₃₉N₃O₄ | 1062.88 | 9.12 | 27.41 | 2100 | C₇₃H₁₄₁N₅O₈ | 1216.96 | 7.84 | 27.17 |
| 1901 | C₇₁H₁₃₉N₃O₇ | 1146.91 | 9.12 | 28.23 | 2101 | C₇₃H₁₄₁N₅O₈ | 1216.96 | 7.84 | 27.17 |
| 1902 | C₇₁H₁₃₉N₃O₇ | 1146.91 | 9.12 | 28.23 | 2102 | C₇₃H₁₄₁N₅O₈ | 1216.96 | 7.84 | 27.17 |
| 1903 | C₇₁H₁₃₉N₃O₇ | 1146.91 | 9.12 | 28.23 | 2103 | C₇₀H₁₃₈N₄O₇ | 1147.89 | 7.19 | 26.06 |
| 1904 | C₇₁H₁₃₉N₃O₇ | 1146.91 | 9.12 | 28.23 | 2104 | C₇₀H₁₃₈N₄O₇ | 1147.89 | 7.19 | 26.06 |
| 1905 | C₆₉H₁₄₁N₃O₄ | 1076.90 | 9.20 | 27.71 | 2105 | C₆₈H₁₃₅N₅O₅ | 1102.86 | 7.65 | 24.36 |
| 1906 | C₆₉H₁₄₁N₃O₄ | 1076.90 | 9.20 | 27.71 | 2106 | C₆₈H₁₃₅N₅O₅ | 1102.86 | 7.65 | 24.36 |
| 1907 | C₇₂H₁₄₁N₃O₇ | 1160.93 | 9.20 | 28.54 | 2107 | C₇₁H₁₃₅N₅O₈ | 1186.89 | 7.65 | 25.18 |
| 1908 | C₇₂H₁₄₁N₃O₇ | 1160.93 | 9.20 | 28.54 | 2108 | C₇₁H₁₃₅N₅O₈ | 1186.89 | 7.65 | 25.18 |
| 1909 | C₇₂H₁₄₁N₃O₇ | 1160.93 | 9.20 | 28.54 | 2109 | C₇₁H₁₃₅N₅O₈ | 1186.89 | 7.65 | 25.18 |
| 1910 | C₇₂H₁₄₁N₃O₇ | 1160.93 | 9.20 | 28.54 | 2110 | C₇₁H₁₃₅N₅O₈ | 1186.89 | 7.65 | 25.18 |
| 1911 | C₆₉H₁₄₁N₃O₄ | 1076.90 | 9.15 | 27.93 | 2111 | C₆₉H₁₃₆N₄O₅ | 1101.87 | 7.90 | 25.58 |
| 1912 | C₆₉H₁₄₁N₃O₄ | 1076.90 | 9.15 | 27.93 | 2112 | C₆₉H₁₃₆N₄O₅ | 1101.87 | 7.90 | 25.58 |
| 1913 | C₇₂H₁₄₁N₃O₇ | 1160.93 | 9.15 | 28.76 | 2113 | C₇₂H₁₃₆N₄O₈ | 1185.90 | 7.90 | 26.41 |
| 1914 | C₇₂H₁₄₁N₃O₇ | 1160.93 | 9.15 | 28.76 | 2114 | C₇₂H₁₃₆N₄O₈ | 1185.90 | 7.90 | 26.41 |
| 1915 | C₇₂H₁₄₁N₃O₇ | 1160.93 | 9.15 | 28.76 | 2115 | C₇₂H₁₃₆N₄O₈ | 1185.90 | 7.90 | 26.41 |
| 1916 | C₇₂H₁₄₁N₃O₇ | 1160.93 | 9.15 | 28.76 | 2116 | C₇₂H₁₃₆N₄O₈ | 1185.90 | 7.90 | 26.41 |
| 1917 | C₆₇H₁₃₅N₃O₄ | 1046.83 | 8.42 | 26.62 | 2117 | C₇₀H₁₃₇N₅O₆ | 1144.89 | 7.19 | 25.33 |
| 1918 | C₆₇H₁₃₅N₃O₄ | 1046.83 | 8.42 | 26.62 | 2118 | C₇₀H₁₃₇N₅O₆ | 1144.89 | 7.19 | 25.33 |
| 1919 | C₇₀H₁₃₅N₃O₇ | 1130.86 | 8.42 | 27.44 | 2119 | C₁₀₀H₁₉₅NO₉S₂ | 1619.78 | 9.12 | 42.45 |
| 1920 | C₇₀H₁₃₅N₃O₇ | 1130.86 | 8.42 | 27.44 | 2120 | C₉₉H₁₈₉N₃O₉ | 1565.61 | 8.77 | 41.14 |
| 1921 | C₇₀H₁₃₅N₃O₇ | 1130.86 | 8.42 | 27.44 | 2121 | C₇₃H₁₃₇N₅O₉ | 1228.92 | 7.07 | 26.15 |
| 1922 | C₇₀H₁₃₅N₃O₇ | 1130.86 | 8.42 | 27.44 | 2122 | C₇₃H₁₃₇N₅O₉ | 1228.92 | 7.07 | 26.15 |
| 1923 | C₆₉H₁₃₉N₃O₄ | 1074.89 | 8.99 | 27.51 | 2123 | C₇₁H₁₃₉N₅O₅ | 1142.92 | 7.47 | 25.63 |
| 1924 | C₆₉H₁₃₉N₃O₄ | 1074.89 | 8.99 | 27.51 | 2124 | C₇₁H₁₃₉N₅O₅ | 1142.92 | 7.47 | 25.63 |
| 1925 | C₇₂H₁₃₉N₃O₇ | 1158.92 | 8.99 | 28.33 | 2125 | C₁₀₃H₁₉₄N₂O₁₅S₂ | 1764.80 | 8.09 | 42.63 |
| 1926 | C₇₂H₁₃₉N₃O₇ | 1158.92 | 8.99 | 28.33 | 2126 | C₁₀₁H₁₉₆N₂O₁₄S | 1694.74 | 9.65 | 42.49 |
| 1927 | C₇₂H₁₃₉N₃O₇ | 1158.92 | 9.00 | 28.33 | 2127 | C₇₄H₁₃₉N₅O₈ | 1226.95 | 7.47 | 26.45 |
| 1928 | C₇₂H₁₃₉N₃O₇ | 1158.92 | 9.00 | 28.33 | 2128 | C₇₄H₁₃₉N₅O₈ | 1226.95 | 7.47 | 26.45 |
| 1929 | C₆₇H₁₃₅N₃O₄ | 1046.83 | 8.77 | 26.65 | 2129 | C₇₁H₁₃₈N₈O₄ | 1167.94 | 7.58 | 25.74 |
| 1930 | C₆₇H₁₃₅N₃O₄ | 1046.83 | 8.77 | 26.65 | 2130 | C₇₁H₁₃₈N₈O₄ | 1167.94 | 7.58 | 25.74 |
| 1931 | C₇₀H₁₃₅N₃O₇ | 1130.86 | 8.77 | 27.47 | 2131 | C₈₈H₁₇₂N₄O₇ | 1398.36 | 7.81 | 35.59 |
| 1932 | C₇₀H₁₃₅N₃O₇ | 1130.86 | 8.77 | 27.47 | 2132 | C₈₄H₁₇₁NO₇S | 1339.35 | 10.02 | 36.46 |
| 1933 | C₇₀H₁₃₅N₃O₇ | 1130.86 | 8.77 | 27.47 | 2133 | C₇₄H₁₃₈N₈O₇ | 1251.97 | 7.58 | 26.56 |
| 1934 | C₇₀H₁₃₅N₃O₇ | 1130.86 | 8.77 | 27.47 | 2134 | C₇₄H₁₃₈N₈O₇ | 1251.97 | 7.58 | 26.56 |
| 1935 | C₆₈H₁₃₇N₃O₄ | 1060.86 | 9.40 | 26.63 | 2135 | C₆₀H₁₀₀N₂O₈S | 1009.53 | 7.01 | 18.88 |
| 1936 | C₆₈H₁₃₇N₃O₄ | 1060.86 | 9.40 | 26.63 | 2136 | C₆₁H₁₀₅N₃O₆S₃ | 1072.71 | 8.34 | 21.30 |
| 1937 | C₇₁H₁₃₇N₃O₇ | 1144.89 | 9.40 | 27.45 | 2137 | C₆₀H₁₀₅NO₂S₄ | 1000.75 | 8.96 | 22.13 |
| 1938 | C₇₁H₁₃₇N₃O₇ | 1144.89 | 9.40 | 27.45 | 2138 | C₆₃H₁₁₄N₆O₇ | 1067.64 | 10.00 | 21.45 |
| 1939 | C₇₁H₁₃₇N₃O₇ | 1144.89 | 9.40 | 27.45 | 2139 | C₅₉H₁₀₁NO₁₀ | 984.45 | 8.35 | 21.09 |
| 1940 | C₇₁H₁₃₇N₃O₇ | 1144.89 | 9.40 | 27.45 | 2140 | C₆₀H₁₀₈N₄O₆S | 1013.61 | 8.40 | 19.22 |
| 1941 | C₆₈H₁₃₇N₃O₄ | 1060.86 | 9.62 | 25.87 | 2141 | C₆₄H₁₁₈N₃O₇P | 1072.64 | 8.79 | 20.87 |
| 1942 | C₆₈H₁₃₇N₃O₄ | 1060.86 | 9.62 | 25.87 | 2142 | C₅₆H₁₀₃NO₉S₄ | 1062.68 | 10.13 | 19.46 |
| 1943 | C₇₁H₁₃₇N₃O₇ | 1144.89 | 9.62 | 26.69 | 2143 | C₅₉H₁₀₇NO₉S₄ | 1102.74 | 9.62 | 17.16 |
| 1944 | C₇₁H₁₃₇N₃O₇ | 1144.89 | 9.62 | 26.69 | 2144 | C₆₀H₁₁₀N₂O₁₂S₃ | 1147.72 | 8.95 | 19.15 |
| 1945 | C₇₁H₁₃₇N₃O₇ | 1144.89 | 9.62 | 26.69 | 2145 | C₆₄H₁₁₃N₃O₄S₃ | 1084.80 | 10.17 | 23.74 |
| 1946 | C₇₁H₁₃₇N₃O₇ | 1144.89 | 9.62 | 26.69 | 2146 | C₆₀H₁₀₄N₂O₅S₃ | 1029.68 | 9.96 | 22.15 |
| 1947 | C₆₈H₁₃₇N₃O₄ | 1060.86 | 8.87 | 27.21 | 2147 | C₆₃H₁₀₉N₃O₆S₄ | 1132.82 | 9.65 | 22.68 |
| 1948 | C₆₈H₁₃₇N₃O₄ | 1060.86 | 8.87 | 27.21 | 2148 | C₆₂H₁₁₀N₂O₂S₇ | 1139.99 | 10.03 | 24.47 |
| 1949 | C₇₁H₁₃₇N₃O₇ | 1144.89 | 8.87 | 28.03 | 2149 | C₅₇H₁₀₂N₆O₃S | 951.54 | 8.64 | 15.85 |
| 1950 | C₇₁H₁₃₇N₃O₇ | 1144.89 | 8.87 | 28.03 | 2150 | C₅₃H₉₇NO₇ | 860.36 | 9.79 | 19.82 |
| 1951 | C₇₁H₁₃₇N₃O₇ | 1144.89 | 8.87 | 28.03 | 2151 | C₅₇H₁₀₃N₃O₂S₃ | 958.65 | 8.96 | 18.52 |
| 1952 | C₇₁H₁₃₇N₃O₇ | 1144.89 | 8.87 | 28.03 | 2152 | C₅₆H₁₀₃NS₄ | 918.69 | 10.21 | 21.10 |
| 1953 | C₆₈H₁₃₈N₄O₄ | 1075.88 | 8.77 | 25.07 | 2153 | C₅₆H₁₀₃NO₃S₇ | 1062.86 | 9.96 | 23.06 |
| 1954 | C₆₈H₁₃₈N₄O₄ | 1075.88 | 8.77 | 25.07 | 2154 | C₆₄H₁₁₉NO₈S₄ | 1158.89 | 8.40 | 26.14 |
| 1955 | C₇₁H₁₃₈N₄O₇ | 1159.91 | 8.77 | 25.89 | 2155 | C₆₇H₁₂₂N₄O₅S₄ | 1191.98 | 8.15 | 26.63 |
| 1956 | C₇₁H₁₃₈N₄O₇ | 1159.91 | 8.77 | 25.89 | 2156 | C₇₀H₁₂₈N₂O₅S₅ | 1238.10 | 8.80 | 28.64 |
| 1957 | C₇₁H₁₃₈N₄O₇ | 1159.91 | 8.77 | 25.89 | 2157 | C₆₇H₁₂₂N₂O₅S₄ | 1163.96 | 7.06 | 25.24 |
| 1958 | C₇₁H₁₃₈N₄O₇ | 1159.91 | 8.77 | 25.89 | 2158 | C₆₄H₁₁₇NOS₇ | 1141.07 | 9.43 | 28.02 |
| 1959 | C₆₇H₁₃₅N₃O₅ | 1062.83 | 7.19 | 26.00 | 2159 | C₇₂H₁₄₂N₇O₆P | 1232.94 | 10.10 | 26.28 |
| 1960 | C₆₇H₁₃₅N₃O₅ | 1062.83 | 7.19 | 26.00 | 2160 | C₇₂H₁₃₀N₆O₇S₂ | 1255.99 | 8.52 | 24.50 |
| 1961 | C₁₀₄H₁₉₇N₃O₇S₅ | 1762.03 | 7.97 | 43.84 | 2161 | C₆₇H₁₃₀N₆O₂ | 1051.82 | 8.75 | 23.16 |
| 1962 | C₉₉H₂₀₃N₂O₁₄P₃S₂ | 1802.75 | 7.87 | 40.39 | 2162 | C₆₉H₁₃₀N₄O₇ | 1127.82 | 9.95 | 26.16 |
| 1963 | C₇₀H₁₃₅N₃O₈ | 1146.86 | 6.84 | 26.82 | 2163 | C₇₂H₁₂₉N₉O₆S | 1248.94 | 8.63 | 24.39 |
| 1964 | C₇₀H₁₃₅N₃O₈ | 1146.86 | 6.83 | 26.82 | 2164 | C₇₀H₁₃₁NO₁₀ | 1146.82 | 10.01 | 27.80 |
| 1965 | C₆₉H₁₃₉N₃O₄ | 1074.89 | 9.80 | 26.43 | 2165 | C₆₇H₁₂₁N₃O₈S₃ | 1192.90 | 7.17 | 27.01 |
| 1966 | C₆₉H₁₃₉N₃O₄ | 1074.89 | 9.80 | 26.43 | 2166 | C₆₆H₁₂₃N₃O₇S | 1102.78 | 7.67 | 23.92 |
| 1967 | C₇₂H₁₃₉N₃O₇ | 1158.92 | 9.80 | 27.25 | 2167 | C₇₂H₁₄₀N₂O₁₃P₄ | 1365.81 | 8.13 | 23.27 |
| 1968 | C₇₂H₁₃₉N₃O₇ | 1158.92 | 9.80 | 27.25 | 2168 | C₇₃H₁₃₉N₄O₁₃P₃ | 1373.85 | 7.94 | 22.39 |
| 1969 | C₇₂H₁₃₉N₃O₇₊₊ | 1158.92 | 9.80 | 27.25 | 2169 | C₆₄H₁₂₅N₃O₇S₃ | 1144.90 | 9.63 | 22.96 |
| 1970 | C₇₂H₁₃₉N₃O₇ | 1158.92 | 9.80 | 27.25 | 2170 | C₆₈H₁₂₇N₃O₇S₃ | 1194.96 | 10.18 | 28.67 |
| 1971 | C₆₆H₁₃₅N₃O₅ | 1050.82 | 8.20 | 25.62 | 2171 | C₆₉H₁₂₉NO₃S₇ | 1245.21 | 10.02 | 29.71 |
| 1972 | C₆₆H₁₃₅N₃O₅ | 1050.82 | 8.20 | 25.62 | 2172 | C₆₄H₁₁₈N₂O₂S₆ | 1140.02 | 7.72 | 28.06 |
| 1973 | C₆₉H₁₃₅N₃O₈ | 1134.85 | 8.20 | 26.44 | 2173 | C₆₅H₁₂₅NO₃S₆ | 1161.08 | 9.59 | 30.57 |
| 1974 | C₆₉H₁₃₅N₃O₈ | 1134.85 | 8.20 | 26.44 | 2174 | C₆₇H₁₂₉NO₂S₇ | 1205.19 | 10.25 | 31.99 |
| 1975 | C₆₉H₁₃₅N₃O₈ | 1134.85 | 8.20 | 26.44 | 2175 | C₆₃H₁₂₁N₃OS₈ | 1193.16 | 8.62 | 29.09 |
| 1976 | C₆₉H₁₃₅N₃O₈ | 1134.85 | 8.20 | 26.44 | 2176 | C₅₆H₁₀₄N₄O₃S₂ | 945.59 | 10.08 | 17.35 |
| 1977 | C₆₇H₁₃₇N₃O₅ | 1064.85 | 8.67 | 25.91 | 2177 | C₅₅H₁₀₃N₁₁O₄ | 982.50 | 7.63 | 16.96 |
| 1978 | C₆₇H₁₃₇N₃O₅ | 1064.85 | 8.67 | 25.91 | 2178 | C₅₅H₁₀₀N₄O₈S | 977.48 | 9.61 | 18.82 |
| 1979 | C₇₀H₁₃₇N₃O₈ | 1148.88 | 8.67 | 26.73 | 2179 | C₅₇H₁₀₈N₁₀O₈S₄ | 1189.79 | 8.50 | 16.73 |
| 1980 | C₇₀H₁₃₇N₃O₈ | 1148.88 | 8.67 | 26.73 | 2180 | C₅₃H₁₀₂N₆O₁₀S₄ | 1111.67 | 8.18 | 14.64 |
| 1981 | C₇₀H₁₃₇N₃O₈ | 1148.88 | 8.67 | 26.73 | 2181 | C₅₉H₁₀₀N₆O₈ | 1021.48 | 8.42 | 18.23 |
| 1982 | C₇₀H₁₃₇N₃O₈ | 1148.88 | 8.67 | 26.73 | 2182 | C₅₄H₉₈N₂O₈ | 903.38 | 8.85 | 20.07 |
| 1983 | C₆₈H₁₃₉N₃O₅ | 1078.88 | 8.87 | 25.64 | 2183 | C₅₆H₁₀₄N₄O₁₀S | 1025.53 | 9.53 | 19.39 |
| 1984 | C₆₈H₁₃₉N₃O₅ | 1078.88 | 8.87 | 25.64 | 2184 | C₅₃H₉₈N₄O₇S₂ | 967.51 | 8.78 | 17.62 |
| 1985 | C₇₁H₁₃₉N₃O₈ | 1162.91 | 8.87 | 26.46 | 2185 | C₅₄H₁₀₃NO₆S | 894.48 | 9.81 | 18.88 |
| 1986 | C₉₃H₁₈₇NO₈S₆ | 1639.88 | 8.87 | 39.61 | 2186 | C₆₀H₁₁₅N₄O₁₂P₃S | 1209.58 | 7.38 | 16.63 |
| 1987 | C₇₁H₁₃₉N₃O₈ | 1162.91 | 8.87 | 26.46 | 2187 | C₄₈H₉₁NO₁₄S₄ | 1034.49 | 8.57 | 18.09 |
| 1988 | C₇₁H₁₃₉N₃O₈ | 1162.91 | 8.87 | 26.46 | 2188 | C₅₃H₉₇N₃O₇S₃ | 984.55 | 7.65 | 15.34 |
| 1989 | C₆₈H₁₃₈N₄O₆ | 1107.87 | 7.80 | 25.43 | 2189 | C₅₃H₁₀₀N₄O₁₀S₄ | 1081.64 | 8.02 | 15.11 |
| 1990 | C₆₈H₁₃₈N₄O₆ | 1107.87 | 7.80 | 25.43 | 2190 | C₅₉H₁₀₄N₂O₈S₄ | 1097.73 | 8.07 | 20.70 |
| 1991 | C₉₈H₁₉₅N₅O₅S₃ | 1619.85 | 7.45 | 41.60 | 2191 | C₅₇H₁₀₃NO₆S₄ | 1026.69 | 10.16 | 22.82 |
| 1992 | C₉₈H₁₉₃NO₁₀S₃ | 1641.80 | 9.82 | 42.89 | 2192 | C₅₇H₁₀₃NO₆S₆ | 1090.81 | 8.72 | 25.21 |
| 1993 | C₇₁H₁₃₈N₄O₉ | 1191.90 | 7.80 | 26.25 | 2193 | C₅₇H₁₀₃NO₆S₇ | 1122.87 | 9.87 | 23.86 |
| 1994 | C₇₁H₁₃₈N₄O₉ | 1191.90 | 7.80 | 26.25 | 2194 | C₅₇H₉₉N₃O₈S₇ | 1178.85 | 7.78 | 21.15 |
| 1995 | C₆₉H₁₃₇N₅O₅ | 1116.88 | 7.49 | 25.53 | 2195 | C₅₆H₁₀₉N₂O₃PS₆ | 1081.83 | 8.96 | 22.34 |
| 1996 | C₆₉H₁₃₇N₅O₅ | 1116.88 | 7.49 | 25.53 | 2196 | C₆₉H₁₂₁NO₅S | 1076.79 | 9.98 | 23.52 |
| 1997 | C₇₂H₁₃₇N₅O₈ | 1200.91 | 7.49 | 26.35 | 2197 | C₇₀H₁₂₂N₄O₂S | 1147.95 | 8.85 | 22.42 |
| 1998 | C₇₂H₁₃₇N₅O₈ | 1200.91 | 7.49 | 26.35 | 2198 | C₇₂H₁₂₃N₉O₇ | 1226.83 | 8.52 | 18.69 |
| 1999 | C₇₂H₁₃₇N₅O₈ | 1200.91 | 7.49 | 26.35 | 2199 | C₆₇H₁₁₇N₅O₅ | 1072.70 | 8.36 | 19.23 |
| 2000 | C₇₂H₁₃₇N₅O₈ | 1200.91 | 7.49 | 26.35 | 2200 | C₆₈H₁₂₇N₁₁O₄ | 1162.84 | 9.49 | 19.64 |
| 2201 | C₆₈H₁₂₅N₇O₅ | 1120.79 | 9.91 | 22.00 | 2401 | C₇₇H₁₅₇N₃O₁₀S₃ | 1381.31 | 10.29 | 31.76 |
| 2202 | C₇₀H₁₂₆N₁₀O₇S | 1251.90 | 7.99 | 20.23 | 2402 | C₇₇H₁₄₈N₄O₇S₇ | 1466.52 | 7.62 | 33.01 |
| 2203 | C₆₇H₁₁₈N₈O₈ | 1163.73 | 7.71 | 21.54 | 2403 | C₇₆H₁₄₇NO₂S₆ | 1299.42 | 10.02 | 34.04 |
| 2204 | C₆₇H₁₂₃N₇O₂ | 1058.77 | 8.87 | 20.62 | 2404 | C₇₆H₁₅₃NOS₈ | 1353.60 | 10.09 | 38.26 |
| 2205 | C₆₇H₁₂₂N₁₀O | 1083.78 | 8.58 | 19.27 | 2405 | C₇₅H₁₄₉N₃O₂S₈ | 1381.57 | 8.73 | 35.21 |
| 2206 | C₆₃H₁₁₈N₄O₆S₅ | 1187.96 | 9.68 | 21.42 | 2406 | C₉₉H₁₉₃N₇O₆ | 1577.68 | 8.36 | 38.16 |
| 2207 | C₆₅H₁₁₁NO₇S | 1050.66 | 8.10 | 22.75 | 2407 | C₉₉H₁₈₉N₃O₅S₃ | 1597.82 | 8.77 | 42.25 |
| 2208 | C₇₀H₁₁₉N₃O₉S₅ | 1307.03 | 7.99 | 25.56 | 2408 | C₉₆H₁₈₉NO₃S₅ | 1565.91 | 9.91 | 45.50 |
| 2209 | C₆₇H₁₁₈N₂O₁₂S₅ | 1303.98 | 8.08 | 20.95 | 2409 | C₁₀₆H₂₀₅NO₅ | 1573.81 | 9.90 | 46.33 |
| 2210 | C₆₄H₁₁₇NO₁₃S₃ | 1204.81 | 9.78 | 23.12 | 2410 | C₁₀₇H₂₀₉N₅O₅ | 1645.88 | 8.96 | 44.62 |
| 2211 | C₆₁H₁₁₂N₂O₁₄S₃ | 1193.75 | 9.49 | 22.15 | 2411 | C₁₀₅H₂₀₄N₆O₃S₂ | 1662.96 | 8.70 | 42.53 |
| 2212 | C₇₂H₁₃₃N₄O₁₂P₃ | 1339.79 | 8.12 | 20.87 | 2412 | C₁₀₆H₂₀₅N₃O₉ | 1665.82 | 8.80 | 45.11 |
| 2213 | C₆₈H₁₂₈NO₁₀P₃S₂ | 1276.81 | 9.04 | 21.27 | 2413 | C₁₀₄H₂₀₄N₂O₃S₃ | 1626.99 | 9.75 | 44.82 |
| 2214 | C₆₅H₁₁₇NO₇S₄ | 1152.89 | 9.86 | 19.82 | 2414 | C₁₀₄H₂₀₁NOS₇ | 1706.23 | 9.86 | 49.18 |
| 2215 | C₆₆H₁₂₀N₆O₈S₃ | 1221.90 | 9.07 | 19.62 | 2415 | C₁₀₅H₂₀₄N₃O₅PS₆ | 1812.18 | 7.51 | 47.43 |
| 2216 | C₆₈H₁₂₄N₂O₁₀S₅ | 1290.04 | 9.08 | 22.31 | 2416 | C₁₀₅H₂₀₆N₆O₆S | 1680.91 | 8.82 | 42.03 |
| 2217 | C₆₃H₁₁₂N₄O₂S₆ | 1149.97 | 7.37 | 24.03 | 2417 | C₁₀₃H₂₀₄N₈O₄S₂ | 1682.95 | 8.74 | 43.18 |
| 2218 | C₇₀H₁₃₁N₃O₄S₂ | 1142.95 | 8.36 | 25.63 | 2418 | C₁₀₆H₂₁₁N₇O₂ | 1615.90 | 8.51 | 43.80 |
| 2219 | C₆₇H₁₃₀N₂O₅ | 1043.79 | 9.54 | 27.28 | 2419 | C₁₀₃H₂₁₁N₄O₃P | 1584.82 | 9.91 | 45.36 |
| 2220 | C₆₈H₁₃₁NO₅S₄ | 1171.04 | 8.83 | 28.53 | 2420 | C₁₀₄H₂₀₈N₆O₆ | 1638.84 | 8.96 | 43.83 |
| 2221 | C₈₇H₁₆₉NO₅S | 1341.37 | 9.31 | 36.31 | 2421 | C₁₀₇H₂₀₈N₆O₁₀S | 1770.94 | 8.19 | 44.86 |
| 2222 | C₈₈H₁₇₂N₂O₅ | 1338.35 | 9.01 | 37.81 | 2422 | C₁₀₂H₂₀₂N₄O₈S | 1644.82 | 9.95 | 44.99 |
| 2223 | C₈₇H₁₆₉NO₆S₃ | 1421.49 | 9.94 | 39.01 | 2423 | C₁₀₆H₂₀₈N₄O₇S₃ | 1747.05 | 9.64 | 47.63 |
| 2224 | C₉₂H₁₇₅N₆O₇PS₃ | 1604.60 | 7.83 | 37.76 | 2424 | C₁₀₆H₂₀₇N₄O₉PS₃ | 1809.02 | 8.18 | 47.75 |
| 2225 | C₈₇H₁₇₀N₄O₂S₆ | 1496.70 | 8.30 | 39.06 | 2425 | C₁₀₂H₂₀₁NO₉S₄ | 1714.00 | 9.33 | 48.93 |
| 2226 | C₈₂H₁₆₈N₄OS | 1258.33 | 9.43 | 35.75 | 2426 | C₁₀₄H₂₀₁NO₇S₄ | 1706.02 | 9.76 | 48.92 |
| 2227 | C₈₉H₁₇₃NO₉S₄ | 1529.60 | 9.78 | 39.84 | 2427 | C₁₀₂H₂₀₃NO₅ | 1523.75 | 9.86 | 45.85 |
| 2228 | C₈₇H₁₇₅NO₁₆S₄ | 1619.59 | 9.94 | 38.90 | 2428 | C₁₀₈H₂₁₄N₇O₁₂P₃ | 1895.86 | 7.23 | 41.61 |
| 2229 | C₈₉H₁₇₇NO₁₃S₄ | 1597.63 | 10.04 | 39.39 | 2429 | C₁₀₀H₁₉₉N₃O₁₅S₅ | 1844.03 | 7.71 | 46.10 |
| 2230 | C₈₉H₁₇₂N₆O₁₀S₄ | 1566.58 | 8.43 | 34.65 | 2430 | C₉₉H₁₉₉NO₁₄S₅ | 1788.01 | 8.79 | 44.17 |
| 2231 | C₈₅H₁₇₅N₅O₉S₃ | 1507.54 | 9.58 | 34.71 | 2431 | C₁₀₃H₂₀₆N₆O₇S₄ | 1769.08 | 8.96 | 44.54 |
| 2232 | C₈₅H₁₇₄N₂O₁₁S₄ | 1528.60 | 8.94 | 37.92 | 2432 | C₁₀₃H₂₀₉N₆O₁₀PS₃ | 1819.01 | 8.49 | 41.31 |
| 2233 | C₈₅H₁₇₁N₃O₁₀S₄ | 1523.58 | 8.67 | 35.43 | 2433 | C₉₉H₂₀₁N₃O₁₀S₃ | 1689.91 | 9.61 | 44.80 |
| 2234 | C₉₀H₁₇₃N₃O₆S₄ | 1521.66 | 8.82 | 38.34 | 2434 | C₁₀₁H₂₀₅NO₁₀S₅ | 1754.08 | 9.99 | 43.99 |
| 2235 | C₉₀H₁₇₃NO₃S₈ | 1573.91 | 9.95 | 43.51 | 2435 | C₁₀₄H₁₉₉N₃O₅S₅ | 1732.09 | 7.64 | 46.37 |
| 2236 | C₇₂H₁₄₀N₆O₅ | 1169.95 | 8.47 | 24.78 | 2436 | C₁₀₃H₁₉₉N₃O₉S₄ | 1752.00 | 7.51 | 47.66 |
| 2237 | C₆₆H₁₃₇N₇O₂ | 1060.87 | 8.33 | 24.92 | 2437 | C₉₇H₁₉₃N₇O₅S | 1569.72 | 9.22 | 38.28 |
| 2238 | C₇₃H₁₄₁N₃O₇S₂ | 1237.08 | 9.74 | 28.32 | 2438 | C₉₃H₁₈₈N₆O₇S₄ | 1630.83 | 7.70 | 37.71 |
| 2239 | C₆₈H₁₃₆N₄O₈ | 1137.86 | 9.16 | 26.01 | 2439 | C₁₀₀H₁₉₆N₄O₈ | 1582.69 | 9.91 | 43.00 |
| 2240 | C₇₀H₁₄₀N₄O₁₀S | 1229.97 | 9.11 | 27.00 | 2440 | C₉₉H₁₉₂N₂O₉S₄ | 1682.90 | 8.81 | 44.24 |
| 2241 | C₆₈H₁₃₃NO₁₁S | 1172.87 | 9.51 | 27.24 | 2441 | C₉₄H₁₉₁NO₃S | 1415.63 | 10.10 | 42.86 |
| 2242 | C₆₉H₁₃₁NO₁₀S | 1166.87 | 9.12 | 27.95 | 2442 | C₁₀₀H₂₀₅N₂O₁₁P₃ | 1704.66 | 10.14 | 43.84 |
| 2243 | C₆₇H₁₃₅NO₄S | 1050.89 | 9.96 | 27.64 | 2443 | C₉₂H₁₈₆N₂O₁₁S₃ | 1592.70 | 8.75 | 40.86 |
| 2244 | C₇₀H₁₄₅N₂O₁₂P₃ | 1299.85 | 7.89 | 25.09 | 2444 | C₉₄H₁₈₇N₃O₁₅S₃ | 1695.74 | 7.43 | 40.74 |
| 2245 | C₇₄H₁₅₂NO₁₂P₃S | 1373.01 | 9.94 | 27.12 | 2445 | C₉₃H₁₉₀N₂O₆S₃ | 1528.75 | 10.06 | 38.72 |
| 2246 | C₆₉H₁₃₈N₂O₁₀S₃ | 1252.07 | 9.92 | 27.38 | 2446 | C₉₄H₁₈₇N₃O₁₂S₄ | 1679.81 | 7.37 | 39.57 |
| 2247 | C₆₉H₁₃₉NO₁₅S₄ | 1351.13 | 9.90 | 28.99 | 2447 | C₁₀₁H₁₉₆N₃O₇PS₆ | 1788.07 | 8.79 | 44.46 |
| 2248 | C₇₂H₁₃₈N₆O₁₃S₅ | 1456.26 | 8.64 | 26.43 | 2448 | C₉₄H₁₈₇NOS₆ | 1539.94 | 9.73 | 45.76 |
| 2249 | C₇₀H₁₃₇N₃O₉S₃ | 1261.08 | 8.66 | 22.65 | 2449 | C₇₇H₁₂₀N₆O₃S₂ | 1241.98 | 8.70 | 26.41 |
| 2250 | C₆₇H₁₃₈N₆O₇S₃ | 1236.08 | 8.87 | 26.36 | 2450 | C₇₅H₁₂₄N₄O₆ | 1177.84 | 10.20 | 25.90 |
| 2251 | C₆₉H₁₃₇N₃O₁₃S₃ | 1313.06 | 8.54 | 25.97 | 2451 | C₇₂H₁₁₅NO₆S₂ | 1154.85 | 10.06 | 27.89 |
| 2252 | C₇₂H₁₃₇N₃O₂S₆ | 1269.31 | 8.65 | 29.55 | 2452 | C₇₆H₁₂₅N₅O₄S₃ | 1269.07 | 9.66 | 26.77 |
| 2253 | C₇₄H₁₄₆NO₃PS₆ | 1321.36 | 10.13 | 32.30 | 2453 | C₇₅H₁₁₇N₃O₂S₈ | 1349.32 | 7.66 | 27.75 |
| 2254 | C₉₀H₁₄₇N₃O₈ | 1399.18 | 7.90 | 32.42 | 2454 | C₇₇H₁₂₃N₅O₆S | 1246.93 | 8.19 | 24.95 |
| 2255 | C₈₉H₁₄₉NO₆S | 1361.24 | 9.32 | 34.59 | 2455 | C₇₆H₁₂₇N₇O₄ | 1202.90 | 9.00 | 24.97 |
| 2256 | C₈₈H₁₄₆N₂O₇S | 1376.21 | 6.19 | 33.17 | 2456 | C₇₅H₁₂₆N₆O₈ | 1239.87 | 9.84 | 25.43 |
| 2257 | C₈₈H₁₅₀N₄O₄S | 1360.26 | 10.02 | 32.47 | 2457 | C₆₈H₁₁₈N₄O₃S | 1071.78 | 9.27 | 26.81 |
| 2258 | C₉₂H₁₅₄N₆O₃S₃ | 1488.48 | 9.03 | 34.03 | 2458 | C₇₄H₁₂₈N₄O₈S₄ | 1330.13 | 10.33 | 26.97 |
| 2259 | C₈₉H₁₅₀N₂O₇ | 1360.19 | 10.09 | 35.36 | 2459 | C₇₄H₁₁₉NO₉ | 1166.76 | 10.00 | 26.77 |
| 2260 | C₈₉H₁₄₄N₂O₇S | 1374.19 | 8.78 | 34.21 | 2460 | C₇₂H₁₁₈N₂O₁₀ | 1171.74 | 9.32 | 24.67 |
| 2261 | C₉₀H₁₅₅N₇O₅ | 1415.27 | 9.54 | 32.12 | 2461 | C₇₈H₁₂₁N₃O₁₂S₂ | 1356.97 | 8.21 | 25.60 |
| 2262 | C₉₀H₁₅₈N₅O₅P | 1421.26 | 8.10 | 33.64 | 2462 | C₈₀H₁₃₇N₂O₁₁P₃ | 1395.90 | 8.95 | 25.51 |
| 2263 | C₉₀H₁₅₃N₇O₉S | 1509.32 | 8.41 | 32.06 | 2463 | C₇₇H₁₃₅N₄O₁₀P₃S | 1401.93 | 7.50 | 25.37 |
| 2264 | C₉₃H₁₆₄N₃O₁₁P₃S | 1625.33 | 8.74 | 32.17 | 2464 | C₆₉H₁₁₈N₄O₉S₄ | 1275.99 | 7.50 | 25.37 |
| 2265 | C₉₂H₁₆₄NO₁₄P₃S | 1633.31 | 7.95 | 33.25 | 2465 | C₇₁H₁₁₉NO₁₃S₄ | 1322.99 | 9.94 | 25.39 |
| 2266 | C₈₈H₁₅₀N₄O₁₂S₄ | 1584.45 | 8.03 | 31.72 | 2466 | C₇₂H₁₁₅N₃O₃S₄ | 1199.00 | 8.42 | 26.27 |
| 2267 | C₈₈H₁₅₃N₃O₁₃S₃ | 1557.40 | 9.96 | 35.94 | 2467 | C₇₃H₁₁₈N₂O₂S₇ | 1280.23 | 9.68 | 28.94 |
| 2268 | C₉₃H₁₆₇N₂O₁₁P₃ | 1582.28 | 10.00 | 33.87 | 2468 | C₇₀H₁₃₃NO₄S₂ | 1116.97 | 9.97 | 28.02 |
| 2269 | C₉₇H₁₆₈N₃O₁₂P₃S | 1693.41 | 8.60 | 32.71 | 2469 | C₆₇H₁₂₉NO₇ | 1060.77 | 8.51 | 28.62 |
| 2270 | C₈₇H₁₅₁N₃O₉S₃ | 1479.38 | 8.83 | 30.34 | 2470 | C₆₈H₁₂₇N₃O₂S₃ | 1114.98 | 8.46 | 26.40 |
| 2271 | C₈₇H₁₅₄N₆O₇S₅ | 1556.56 | 8.94 | 32.10 | 2471 | C₆₆H₁₂₄N₄S₄ | 1102.01 | 7.24 | 26.38 |
| 2272 | C₈₇H₁₄₃NO₅S₇ | 1507.57 | 9.36 | 38.83 | 2472 | C₆₇H₁₃₅N₇O₃S | 1118.93 | 8.80 | 26.76 |
| 2273 | C₈₈H₁₄₄N₂O₅S₆ | 1502.53 | 8.66 | 36.59 | 2473 | C₆₅H₁₃₃N₅O₂ | 1016.81 | 9.94 | 25.89 |
| 2274 | C₈₉H₁₅₃N₅OS₃ | 1405.44 | 9.38 | 36.17 | 2474 | C₆₂H₁₂₇N₅O₈S₃ | 1166.93 | 8.18 | 24.53 |
| 2275 | C₈₈H₁₅₂N₄O₂S₄ | 1426.48 | 8.67 | 36.49 | 2475 | C₆₇H₁₃₈N₄O₆S₅ | 1256.20 | 9.66 | 27.43 |
| 2276 | C₅₃H₁₀₁N₇O₅ | 916.43 | 9.37 | 14.69 | 2476 | C₆₅H₁₃₄N₆O₇S₅ | 1272.16 | 8.65 | 24.58 |
| 2277 | C₄₈H₉₁NO₉S | 858.32 | 7.45 | 18.24 | 2477 | C₆₉H₁₂₉N₅O₈ | 1156.82 | 7.35 | 25.88 |
| 2278 | C₄₉H₉₃NO₇S | 840.35 | 9.87 | 18.01 | 2478 | C₆₄H₁₂₃NO₁₀S | 1098.75 | 8.66 | 26.59 |
| 2279 | C₅₀H₉₂N₄O₆S₃ | 941.51 | 8.18 | 17.07 | 2479 | C₆₈H₁₃₀N₄O₈S₅ | 1292.14 | 8.76 | 29.68 |
| 2280 | C₇₃H₁₃₅NO₅ | 1106.88 | 9.49 | 28.47 | 2480 | C₇₀H₁₂₉N₃O₉S₄ | 1285.08 | 7.88 | 28.40 |
| 2281 | C₇₀H₁₃₂N₄O₃S₂ | 1141.98 | 9.64 | 24.97 | 2481 | C₇₁H₁₄₆NO₁₃P₃S | 1346.93 | 9.92 | 26.28 |
| 2282 | C₇₀H₁₂₆N₄O₅S₃ | 1200.00 | 8.18 | 25.90 | 2482 | C₆₅H₁₃₁NO₇S₅ | 1199.10 | 9.82 | 22.46 |
| 2283 | C₇₆H₁₃₈N₃O₈PS₃ | 1349.13 | 8.02 | 27.79 | 2483 | C₆₂H₁₂₈N₄O₇S₅ | 1202.06 | 9.66 | 24.80 |
| 2284 | C₇₀H₁₂₅N₃O₂S₄ | 1169.06 | 8.09 | 24.80 | 2484 | C₆₇H₁₃₆N₂O₂S₃ | 1098.04 | 9.60 | 31.20 |
| 2285 | C₆₉H₁₃₀N₄O₅S | 1127.89 | 8.62 | 22.64 | 2485 | C₆₇H₁₃₄N₄O₂S₆ | 1220.24 | 8.81 | 29.65 |
| 2286 | C₆₆H₁₂₉N₇O₄S | 1116.87 | 8.42 | 23.80 | 2486 | C₅₀H₉₆N₄O₅ | 833.34 | 7.85 | 14.83 |
| 2287 | C₆₉H₁₃₁N₅O₈S | 1190.90 | 8.72 | 26.48 | 2487 | C₄₉H₁₀₁N₈O₈P | 961.37 | 7.10 | 14.90 |
| 2288 | C₆₉H₁₃₂N₄O₉S | 1193.90 | 9.26 | 26.76 | 2488 | C₄₈H₁₀₀N₄S | 765.42 | 10.17 | 17.18 |
| 2289 | C₆₇H₁₃₄N₄O₂S | 1059.90 | 9.61 | 25.59 | 2489 | C₅₀H₉₉N₅O₁₂S₃ | 1058.57 | 7.89 | 15.01 |
| 2290 | C₇₁H₁₃₈N₆O₈S₃ | 1300.12 | 8.97 | 23.96 | 2490 | C₅₃H₁₀₀N₆O₉ | 965.42 | 8.13 | 16.59 |
| 2291 | C₆₈H₁₃₆N₄O₁₀S₄ | 1298.12 | 9.75 | 23.58 | 2491 | C₄₉H₉₆N₆O₇ | 881.34 | 8.08 | 16.13 |
| 2292 | C₇₂H₁₃₀N₄O₉S₂ | 1259.98 | 8.25 | 26.34 | 2492 | C₅₂H₉₆N₄O₇S₄ | 1017.63 | 8.99 | 20.64 |
| 2293 | C₇₀H₁₂₇N₃O₁₁ | 1186.80 | 8.54 | 23.99 | 2493 | C₅₀H₉₁N₃O₇S₅ | 1006.63 | 8.54 | 18.41 |
| 2294 | C₇₂H₁₃₁NO₉S₃ | 1251.04 | 7.85 | 29.49 | 2494 | C₄₆H₉₃NO₅S | 772.32 | 9.48 | 16.00 |
| 2295 | C₇₀H₁₃₆NO₆P | 1118.83 | 9.96 | 26.88 | 2495 | C₄₉H₁₀₂NO₁₀P₃S₂ | 1022.41 | 9.45 | 15.88 |
| 2296 | C₇₅H₁₅₀NO₁₁P₃S | 1367.01 | 10.01 | 26.33 | 2496 | C₄₄H₉₀N₂O₁₁S₅ | 983.54 | 7.61 | 14.61 |
| 2297 | C₆₉H₁₂₉N₅O₁₀S₃ | 1273.01 | 8.02 | 23.99 | 2497 | C₅₀H₉₇N₃O₁₃S₅ | 1108.67 | 9.10 | 18.90 |
| 2298 | C₆₂H₁₂₁NO₇S₃ | 1088.85 | 8.69 | 22.15 | 2498 | C₄₆H₉₆N₄O₇S₅ | 977.63 | 9.97 | 15.88 |
| 2299 | C₇₂H₁₃₄N₂O₄S₃ | 1188.07 | 8.69 | 30.53 | 2499 | C₅₁H₉₇NO₈S₃ | 948.54 | 9.74 | 20.24 |
| 2300 | C₇₀H₁₃₀N₂O₈S₄ | 1256.08 | 9.65 | 29.21 | 2500 | C₄₈H₈₉NO₉S₃ | 920.44 | 7.12 | 19.34 |
| 2301 | C₇₀H₁₂₉NO₆S₄ | 1209.07 | 9.97 | 29.90 | 2501 | C₅₀H₉₈N₂O₅S₇ | 1031.81 | 10.01 | 21.72 |
| 2302 | C₇₃H₁₃₃N₃O₆S₄ | 1277.15 | 8.91 | 29.86 | 2502 | C₄₉H₈₉N₃O₅S₇ | 1024.74 | 7.38 | 20.15 |
| 2303 | C₇₀H₁₂₇N₃O₅S₇ | 1315.27 | 8.15 | 29.29 | 2503 | C₈₈H₁₇₄N₄O₁₀S | 1480.44 | 8.64 | 37.76 |
| 2304 | C₆₉H₁₃₃NO₂S₄ | 1137.10 | 10.08 | 29.80 | 2504 | C₉₁H₁₈₀NO₁₂P | 1511.41 | 9.64 | 38.49 |
| 2305 | C₇₂H₁₄₂N₂O₉S | 1212.00 | 10.03 | 27.69 | 2505 | C₈₇H₁₇₂N₂O₁₃S₃ | 1550.51 | 7.44 | 36.68 |
| 2306 | C₇₀H₁₃₅NO₂S₃ | 1119.06 | 9.94 | 28.41 | 2506 | C₈₉H₁₇₇NO₁₃S₄ | 1597.63 | 9.89 | 39.95 |
| 2307 | C₇₄H₁₄₃NS₄ | 1175.23 | 10.31 | 30.33 | 2507 | C₈₇H₁₇₉N₅O₈S₄ | 1551.66 | 9.72 | 35.89 |
| 2308 | C₇₃H₁₄₂N₆O₉S | 1280.04 | 8.87 | 27.51 | 2508 | C₉₁H₁₇₃N₃O₅S₇ | 1613.82 | 8.49 | 39.46 |
| 2309 | C₇₁H₁₄₃N₇O₂ | 1126.97 | 8.80 | 25.07 | 2509 | C₈₆H₁₇₃NO₂S₃ | 1349.52 | 8.69 | 40.39 |
| 2310 | C₆₇H₁₃₆N₆O₄ | 1089.86 | 7.38 | 25.24 | 2510 | C₈₇H₁₇₇NO₃S₄ | 1413.62 | 9.80 | 39.79 |
| 2311 | C₆₅H₁₃₆N₄O₂S | 1037.90 | 9.89 | 25.86 | 2511 | C₆₈H₁₃₈N₆OS | 1087.95 | 8.34 | 25.64 |
| 2312 | C₇₀H₁₃₃NO₆S | 1116.90 | 9.78 | 27.91 | 2512 | C₇₃H₁₄₁N₅O₇S | 1233.02 | 8.19 | 27.42 |
| 2313 | C₇₂H₁₃₉NO₈ | 1146.90 | 8.29 | 30.37 | 2513 | C₇₆H₁₄₈N₆O₁₀ | 1306.05 | 8.77 | 28.04 |
| 2314 | C₇₂H₁₃₇N₃O₈S | 1204.97 | 8.10 | 27.01 | 2514 | C₇₇H₁₄₅N₅O₁₂ | 1333.03 | 8.22 | 27.36 |
| 2315 | C₇₀H₁₃₅NO₈S₃ | 1215.05 | 8.87 | 31.23 | 2515 | C₇₂H₁₃₉NO₇S₄ | 1259.14 | 9.28 | 30.05 |
| 2316 | C₆₉H₁₃₄N₂O₉S₃ | 1232.04 | 8.56 | 29.17 | 2516 | C₇₄H₁₄₈N₃O₆P | 1206.99 | 9.19 | 27.09 |
| 2317 | C₆₇H₁₃₁N₃O₆ | 1074.80 | 8.05 | 24.99 | 2517 | C₇₃H₁₄₃N₃O₇S₅ | 1335.26 | 9.00 | 27.79 |
| 2318 | C₇₁H₁₄₁N₃O₆ | 1132.92 | 8.87 | 27.00 | 2518 | C₇₄H₁₄₄N₂O₅S₅ | 1302.27 | 8.94 | 31.06 |
| 2319 | C₆₉H₁₃₈N₄O₁₅S₃ | 1360.08 | 8.57 | 27.02 | 2519 | C₇₄H₁₄₁NO₈S₄ | 1301.18 | 9.84 | 32.75 |
| 2320 | C₇₆H₁₅₂N₃O₁₁P₃S₂ | 1441.12 | 8.65 | 25.02 | 2520 | C₇₅H₁₄₃N₅O₆S₆ | 1403.36 | 8.02 | 29.84 |
| 2321 | C₇₀H₁₄₅N₅O₆S₃ | 1249.16 | 10.17 | 27.30 | 2521 | C₇₅H₁₄₃NOS₆ | 1267.34 | 10.03 | 31.57 |
| 2322 | C₇₀H₁₃₃NO₅S₇ | 1293.30 | 9.72 | 32.57 | 2522 | C₆₉H₁₃₅N₃OS₈ | 1231.28 | 9.26 | 31.21 |
| 2323 | C₉₈H₁₉₃N₃O₅ | 1493.64 | 9.64 | 41.19 | 2523 | C₆₉H₁₃₅N₃OS₈ | 1279.34 | 8.11 | 32.33 |
| 2324 | C₉₇H₁₉₂N₂O₃ | 1434.61 | 9.81 | 43.28 | 2524 | C₉₁H₁₅₁NO₆ | 1355.21 | 9.47 | 35.21 |
| 2325 | C₁₀₀H₁₉₃N₃O₃S₅ | 1646.00 | 9.08 | 44.26 | 2525 | C₈₉H₁₅₁NO₈S | 1395.25 | 9.10 | 33.28 |
| 2326 | C₁₀₀H₁₉₄N₂O₂S₄ | 1584.93 | 8.96 | 42.63 | 2526 | C₉₃H₁₅₆N₆O₅ | 1438.31 | 8.90 | 31.09 |
| 2327 | C₉₇H₁₉₀N₂OS₆ | 1593.00 | 7.95 | 44.89 | 2527 | C₉₀H₁₄₉NO₈S | 1405.24 | 9.77 | 36.24 |
| 2328 | C₁₀₁H₁₉₈N₈O₆S | 1652.81 | 9.03 | 41.03 | 2528 | C₉₁H₁₅₇N₇O₄ | 1413.30 | 9.81 | 33.65 |
| 2329 | C₉₈H₁₉₃N₅O₈ | 1569.65 | 9.94 | 42.17 | 2529 | C₉₃H₁₅₈N₆O₉S | 1536.38 | 9.06 | 33.18 |
| 2330 | C₉₈H₁₉₆N₆O₂S | 1522.75 | 8.97 | 42.54 | 2530 | C₈₈H₁₅₄N₆O₂ | 1328.24 | 9.69 | 33.16 |
| 2331 | C₉₅H₁₈₇NO₈S | 1503.61 | 8.62 | 43.07 | 2531 | C₈₉H₁₅₈N₄O₇S₅ | 1556.56 | 9.19 | 36.70 |
| 2332 | C₁₀₀H₁₉₆N₄O₁₀ | 1614.69 | 8.95 | 42.32 | 2532 | C₉₁H₁₅₅N₅O₈ | 1447.27 | 9.75 | 33.92 |
| 2333 | C₉₆H₁₈₉NO₉S₂ | 1565.70 | 9.90 | 43.36 | 2533 | C₉₁H₁₅₃N₃O₁₀S | 1481.30 | 9.45 | 33.16 |
| 2334 | C₉₈H₁₈₉NO₈S₃ | 1605.79 | 7.84 | 44.98 | 2534 | C₈₈H₁₅₄N₄O₅ | 1348.22 | 9.44 | 33.71 |
| 2335 | C₁₀₄H₂₁₀N₃O₁₂P₃ | 1787.75 | 8.95 | 40.77 | 2535 | C₈₅H₁₄₉NO₁₁S₄ | 1489.36 | 9.07 | 32.92 |
| 2336 | C₉₅H₁₉₂NO₉PS₃ | 1619.75 | 9.61 | 38.95 | 2536 | C₈₃H₁₄₅NO₂S₃ | 1285.26 | 8.63 | 34.83 |
| 2337 | C₉₆H₁₉₆N₆O₉S₃ | 1674.86 | 9.53 | 39.50 | 2537 | C₈₉H₁₅₂N₄O₂S₆ | 1502.58 | 8.69 | 33.48 |
| 2338 | C₉₆H₁₉₄N₆O₉S₄ | 1704.91 | 8.66 | 39.31 | 2538 | C₈₇H₁₅₃NO₄S₇ | 1501.60 | 9.11 | 35.87 |
| 2339 | C₉₇H₁₉₇NO₉S₅ | 1681.98 | 10.29 | 43.64 | 2539 | C₉₀H₁₅₃N₃O₂S₇ | 1533.65 | 8.67 | 35.66 |
| 2340 | C₁₀₀H₁₉₃N₅O₉S₃ | 1705.87 | 8.02 | 43.88 | 2540 | C₉₀H₁₅₅N₃O₃S₇ | 1551.67 | 8.78 | 37.08 |
| 2341 | C₉₃H₁₈₅NO₂S₄ | 1477.78 | 8.83 | 44.69 | 2541 | C₅₀H₉₂N₄O₈ | 877.31 | 7.61 | 14.76 |
| 2342 | C₉₆H₁₉₅NS₅ | 1523.96 | 10.32 | 46.50 | 2542 | C₅₄H₁₀₁N₃O₁₀S | 984.47 | 8.61 | 17.32 |
| 2343 | C₉₆H₁₉₂N₄O₂S₇ | 1659.09 | 9.15 | 45.27 | 2543 | C₅₅H₁₀₇N₃OS₃ | 922.66 | 10.35 | 19.69 |
| 2344 | C₇₂H₁₃₇N₇O₆ | 1196.93 | 9.55 | 23.01 | 2544 | C₇₃H₁₃₃N₃O₇ | 1164.88 | 8.78 | 26.27 |
| 2345 | C₇₅H₁₃₆N₆O₆S | 1250.02 | 8.12 | 23.71 | 2545 | C₇₂H₁₃₉N₄O₆P | 1187.90 | 8.50 | 25.02 |
| 2346 | C₇₃H₁₃₃N₃O₃S₄ | 1229.15 | 8.60 | 25.60 | 2546 | C₇₆H₁₄₅N₉O₆ | 1281.05 | 8.95 | 25.90 |
| 2347 | C₇₂H₁₃₄N₂O₂S₆ | 1252.28 | 8.91 | 31.69 | 2547 | C₇₂H₁₃₃N₅O₁₀ | 1228.88 | 7.28 | 26.13 |
| 2348 | C₇₃H₁₃₅NO₂S₇ | 1283.36 | 10.25 | 31.89 | 2548 | C₇₅H₁₄₈N₄O₂ | 1138.04 | 10.25 | 29.53 |
| 2349 | C₇₄H₁₃₈N₆O₈S | 1272.02 | 8.96 | 26.06 | 2549 | C₇₂H₁₃₈N₆O₃ | 1135.93 | 8.47 | 25.31 |
| 2350 | C₇₁H₁₃₂N₆O₈ | 1197.87 | 8.73 | 24.03 | 2550 | C₇₂H₁₃₁N₃O₁₁S | 1246.91 | 7.71 | 26.16 |
| 2351 | C₇₁H₁₃₇N₅O₈S | 1220.97 | 10.21 | 27.15 | 2551 | C₇₆H₁₃₅N₃O₁₃ | 1298.92 | 8.06 | 26.23 |
| 2352 | C₇₀H₁₂₇NO₇S | 1126.85 | 8.28 | 27.40 | 2552 | C₇₃H₁₃₃N₃O₁₃S | 1292.93 | 8.53 | 24.60 |
| 2353 | C₆₆H₁₂₄N₄O₃S₂ | 1085.88 | 7.45 | 24.89 | 2553 | C₇₂H₁₃₅NO₁₃S | 1254.93 | 9.37 | 28.42 |
| 2354 | C₆₉H₁₃₃NO₃S₂ | 1088.96 | 10.01 | 27.96 | 2554 | C₇₅H₁₄₉N₂O₁₁P₃ | 1347.94 | 8.85 | 27.16 |
| 2355 | C₇₃H₁₄₁N₂O₁₃P₃S | 1379.92 | 7.73 | 24.08 | 2555 | C₇₅H₁₄₈N₃O₁₀P₃S | 377.00 | 9.09 | 25.62 |
| 2356 | C₇₂H₁₃₆N₄O₁₅S₄ | 1426.16 | 8.39 | 26.10 | 2556 | C₇₂H₁₃₅N₃O₉S₄ | 1315.12 | 8.02 | 23.79 |
| 2357 | C₆₆H₁₂₇NO₈S₄ | 1191.01 | 9.56 | 23.38 | 2557 | C₆₇H₁₃₃N₅O₇S₃ | 1217.01 | 9.55 | 24.42 |
| 2358 | C₆₉H₁₃₄N₆O₈S₄ | 1304.13 | 8.63 | 23.43 | 2558 | C₆₈H₁₃₇N₇O₈S₄ | 1309.12 | 8.59 | 23.56 |
| 2359 | C₇₀H₁₃₄N₂O₁₁S₃ | 1276.05 | 9.01 | 25.11 | 2559 | C₆₆H₁₃₀N₄O₉S₄ | 1252.03 | 7.70 | 24.69 |
| 2360 | C₇₁H₁₂₉N₃O₆S₆ | 1313.23 | 7.83 | 29.32 | 2560 | C₆₉H₁₃₁NO₁₀S₄ | 1263.04 | 9.72 | 26.82 |
| 2361 | C₇₅H₁₃₈N₃O₇PS₆ | 1417.32 | 8.79 | 29.20 | 2561 | C₆₉H₁₃₄N₂O₁₁S₄ | 1296.07 | 9.65 | 25.47 |
| 2362 | C₇₃H₁₂₈N₆O₆S₇ | 1410.33 | 8.05 | 29.45 | 2562 | C₇₂H₁₃₇N₃O₁₄S₄ | 1397.13 | 7.96 | 24.84 |
| 2363 | C₆₉H₁₃₃N₃O₄S₃ | 1165.04 | 8.75 | 27.33 | 2563 | C₇₆H₁₃₄N₆O₅S₆ | 1404.30 | 8.44 | 29.07 |
| 2364 | C₇₀H₁₃₃N₂O₄PS₃ | 1194.02 | 8.64 | 27.09 | 2564 | C₇₅H₁₃₈N₄O₄S₇ | 1384.37 | 7.99 | 27.97 |
| 2365 | C₆₈H₁₃₁N₅O₅ | 1098.83 | 8.96 | 24.38 | 2565 | C₇₂H₁₃₃NO₂S₇ | 1269.28 | 9.22 | 29.39 |
| 2366 | C₆₆H₁₃₀N₄O₄ | 1043.79 | 9.95 | 23.32 | 2566 | C₇₆H₁₃₉NS₈ | 1323.43 | 9.99 | 32.54 |
| 2367 | C₆₂H₁₂₄N₄O₅S | 1037.76 | 8.99 | 22.55 | 2567 | C₆₈H₁₃₂N₂OS₆ | 1186.18 | 9.61 | 29.43 |
| 2368 | C₆₇H₁₃₀N₆O₃S₂ | 1131.95 | 9.38 | 22.39 | 2568 | C₇₆H₁₄₆N₆O₈ | 1272.04 | 7.91 | 25.83 |
| 2369 | C₆₇H₁₃₁N₅O₅S₂ | 1150.95 | 8.80 | 22.94 | 2569 | C₇₄H₁₄₂N₂O₆S₃ | 1252.14 | 7.97 | 29.92 |
| 2370 | C₆₅H₁₃₀N₄O₃S₂ | 1079.91 | 10.12 | 23.75 | 2570 | C₇₆H₁₄₇N₃O₇S₄ | 1343.27 | 8.74 | 29.77 |
| 2371 | C₆₄H₁₂₁NO₅S₄ | 1112.94 | 9.72 | 26.66 | 2571 | C₆₈H₁₃₃NO₆S₄ | 1189.05 | 9.29 | 28.76 |
| 2372 | C₆₃H₁₂₇N₇O₆S | 1110.82 | 8.83 | 20.79 | 2572 | C₇₃H₁₄₅N₅O₅ | 1172.99 | 9.61 | 27.70 |
| 2373 | C₆₃H₁₂₆N₆OS₂ | 1047.88 | 8.76 | 23.90 | 2573 | C₇₁H₁₃₉N₅O₁₀S | 1254.98 | 7.51 | 27.38 |
| 2374 | C₆₁H₁₁₉NO₁₄S | 1122.68 | 7.69 | 23.72 | 2574 | C₆₉H₁₄₂N₄O₂ | 1059.92 | 10.05 | 27.68 |
| 2375 | C₆₆H₁₂₈N₃O₁₃P | 1202.73 | 7.46 | 24.54 | 2575 | C₇₁H₁₄₄N₆O₃S | 1162.03 | 8.83 | 25.65 |
| 2376 | C₇₃H₁₄₅N₄O₁₁P₃ | 1347.90 | 8.61 | 22.75 | 2576 | C₇₁H₁₄₈N₄O₁₀S₄ | 1346.22 | 9.78 | 25.99 |
| 2377 | C₇₂H₁₅₀NO₁₃P₃S | 1362.97 | 10.23 | 26.58 | 2577 | C₇₅H₁₄₁N₃O₉S₂ | 1293.09 | 9.29 | 29.12 |
| 2378 | C₅₈H₁₂₁N₅O₈S₃ | 1112.83 | 7.95 | 21.32 | 2578 | C₇₅H₁₄₆N₃O₁₀PS₃ | 1377.16 | 8.69 | 28.51 |
| 2379 | C₆₁H₁₂₈N₄O₉S₄ | 1189.98 | 9.93 | 23.20 | 2579 | C₆₉H₁₃₈N₄O₅S | 1135.95 | 7.81 | 25.35 |
| 2380 | C₇₉H₁₅₀N₆O₅S₂ | 1328.24 | 8.02 | 27.18 | 2580 | C₈₀H₁₆₁N₃O₁₃P₄ | 1497.07 | 8.34 | 25.40 |
| 2381 | C₈₀H₁₅₆N₆O₇ | 1314.16 | 8.79 | 28.87 | 2581 | C₆₈H₁₃₈N₂O₁₃S₄ | 1320.09 | 7.81 | 24.98 |
| 2382 | C₇₇H₁₅₃N₅O₉S | 1325.16 | 8.63 | 27.52 | 2582 | C₇₃H₁₅₆N₃O₁₁P₃ | 1405.04 | 8.90 | 25.51 |
| 2383 | C₇₉H₁₅₆N₄O₅S | 1274.21 | 9.99 | 30.04 | 2583 | C₇₃H₁₄₃N₉O₇S₅ | 1419.30 | 8.50 | 27.26 |
| 2384 | C₇₈H₁₄₈N₂O₃S₇ | 1386.52 | 8.53 | 34.02 | 2584 | C₇₅H₁₄₆N₄O₄S₃ | 1264.20 | 9.75 | 28.36 |
| 2385 | C₇₇H₁₄₈N₂O₅S₇ | 1406.51 | 8.37 | 33.09 | 2585 | C₇₆H₁₄₁N₇O₅S₃ | 1329.19 | 8.44 | 6.51 |
| 2386 | C₇₈H₁₄₉N₃S₇ | 1353.54 | 9.35 | 33.71 | 2586 | C₇₃H₁₄₃N₃S₈ | 1319.45 | 9.73 | 31.55 |
| 2387 | C₈₀H₁₅₅N₇O₅ | 1295.16 | 9.05 | 28.08 | 2587 | C₆₈H₁₃₇NO₂S₇ | 1225.27 | 9.56 | 32.84 |
| 2388 | C₇₇H₁₅₅N₇O₃S₂ | 1291.27 | 9.72 | 30.97 | 2588 | C₁₀₀H₁₉₂N₂O₄S | 1518.71 | 9.01 | 41.94 |
| 2389 | C₇₇H₁₅₃N₉O₂S | 1269.20 | 8.46 | 28.74 | 2589 | C₉₈H₁₈₉NOS₅ | 1557.90 | 7.61 | 43.84 |
| 2390 | C₇₆H₁₅₆N₄O₇S₃ | 1334.31 | 8.94 | 31.72 | 2590 | C₉₈H₁₉₄NO₃PS₃ | 1561.79 | 8.48 | 42.90 |
| 2391 | C₇₆H₁₅₄N₆O₈S₃ | 1376.30 | 8.64 | 28.57 | 2591 | C₉₉H₁₉₄N₄O₇S | 1584.72 | 9.65 | 43.10 |
| 2392 | C₇₈H₁₄₆N₂O₈ | 1240.03 | 8.71 | 30.11 | 2592 | C₉₉H₁₉₄N₄O₅S₂ | 1632.78 | 7.76 | 43.54 |
| 2393 | C₇₆H₁₄₈NO₉P | 1250.99 | 8.86 | 31.56 | 2593 | C₉₆H₁₉₆N₄O₃S | 1486.71 | 9.59 | 42.11 |
| 2394 | C₇₈H₁₅₀N₆O₉S | 1348.16 | 7.69 | 29.53 | 2594 | C₉₇H₁₉₅N₅O₄S | 1527.72 | 8.65 | 40.06 |
| 2395 | C₇₇H₁₅₀N₄O₇S | 1276.13 | 9.43 | 30.93 | 2595 | C₁₀₂H₁₉₉N₅O₉ | 1639.74 | 8.75 | 41.94 |
| 2396 | C₇₇H₁₄₇NO₇S₄ | 1327.29 | 9.86 | 34.36 | 2596 | C₉₈H₁₉₇N₄O₁₀P | 1622.65 | 8.51 | 41.00 |
| 2397 | C₇₅H₁₄₉NO₁₁S₄ | 1369.28 | 9.91 | 31.06 | 2597 | C₉₉H₁₉₅NO₆S₄ | 1623.89 | 10.09 | 47.23 |
| 2398 | C₇₉H₁₆₂NO₁₁P₃S | 1427.15 | 9.02 | 30.52 | 2598 | C₁₀₅H₂₁₄NO₁₀P₃ | 1743.79 | 10.19 | 43.28 |
| 2399 | C₇₆H₁₅₇N₆O₁₀PS₃ | 1442.30 | 8.06 | 28.96 | 2599 | C₉₈H₁₉₃N₃O₁₂S₄ | 1733.87 | 8.00 | 41.24 |
| 2400 | C₇₅H₁₄₉N₃O₁₀S₅ | 1413.36 | 8.76 | 28.82 | 2600 | C₆₃H₁₀₇NO₆S | 1006.62 | 9.80 | 21.64 |
| 2601 | C₆₅H₁₁₂N₃O₄PS₆ | 1223.00 | 7.95 | 21.13 | 2801 | C₉₉H₂₀₀N₆O₁₀S₄ | 1762.96 | 8.40 | 40.16 |
| 2602 | C₆₆H₁₁₉N₇O₅ | 1090.72 | 10.00 | 21.99 | 2802 | C₁₀₀H₁₉₉N₃O₁₁S₃ | 1715.88 | 8.98 | 40.36 |
| 2603 | C₆₁H₁₁₄N₄O₇S₅ | 1175.94 | 9.86 | 19.74 | 2803 | C₉₈H₂₀₁N₄O₁₂PS₃ | 1754.86 | 9.49 | 39.38 |
| 2604 | C₆₁H₁₁₂N₆O₁₁S₄ | 1233.87 | 7.61 | 17.67 | 2804 | C₉₉H₁₉₅N₃OS₉ | 1732.21 | 8.95 | 48.29 |
| 2605 | C₅₈H₁₁₀N₄O₉S₄ | 1135.80 | 7.67 | 19.80 | 2805 | C₇₇H₁₂₁N₃O₈S | 1248.89 | 8.03 | 26.31 |
| 2606 | C₆₆H₁₁₆N₆O₈S | 1153.75 | 8.85 | 21.05 | 2806 | C₇₂H₁₁₇NO₃S₅ | 1205.03 | 10.07 | 28.49 |
| 2607 | C₆₂H₁₀₆N₂O₁₁ | 1055.53 | 9.60 | 20.49 | 2807 | C₇₄H₁₁₇N₃OS₄ | 1193.01 | 8.45 | 25.19 |
| 2608 | C₆₃H₁₀₉NO₈S₃ | 1104.76 | 9.87 | 23.85 | 2808 | C₇₅H₁₂₁NO₃S₇ | 1309.22 | 9.65 | 27.97 |
| 2609 | C₆₃H₁₀₈N₂O₁₀S₄ | 1181.83 | 8.42 | 23.27 | 2809 | C₇₉H₁₃₄N₉O₆PS | 1369.03 | 8.95 | 25.87 |
| 2610 | C₆₅H₁₀₉N₃O₇S₅ | 1204.94 | 8.71 | 22.79 | 2810 | C₇₉H₁₃₀N₆O₉ | 1307.94 | 8.95 | 26.58 |
| 2611 | C₆₆H₁₂₂NO₁₀P₃S | 1214.69 | 9.74 | 20.96 | 2811 | C₇₆H₁₃₅N₄O₄P | 1199.91 | 10.01 | 25.75 |
| 2612 | C₆₃H₁₁₁N₃O₉S₅ | 1214.93 | 9.29 | 21.07 | 2812 | C₈₁H₁₂₆N₆O₉ | 1327.93 | 8.71 | 26.39 |
| 2613 | C₆₃H₁₁₃N₃O₁₁S₄ | 1216.87 | 8.22 | 20.49 | 2813 | C₇₇H₁₂₆N₄O₆S₂ | 1268.00 | 9.98 | 27.83 |
| 2614 | C₆₅H₁₂₂NO₁₁P₃S | 1218.68 | 8.62 | 19.71 | 2814 | C₇₅H₁₂₄N₄O₈ | 1209.84 | 8.73 | 27.26 |
| 2615 | C₆₀H₁₁₂NO₉PS₃ | 1118.73 | 10.00 | 17.50 | 2815 | C₇₁H₁₁₉NO₆ | 1082.73 | 8.65 | 26.27 |
| 2616 | C₆₃H₁₀₈N₆O₁₁S₄ | 1253.86 | 8.07 | 20.48 | 2816 | C₈₁H₁₃₈N₃O₁₂P₃S | 1470.99 | 8.02 | 25.79 |
| 2617 | C₆₃H₁₁₃N₂O₅PS₃ | 1105.78 | 7.69 | 21.48 | 2817 | C₇₈H₁₃₅N₄O₁₁P₃S | 1429.94 | 8.22 | 25.01 |
| 2618 | C₆₁H₁₀₉N₃O₂S₄ | 1044.83 | 8.31 | 22.42 | 2818 | C₇₄H₁₂₈NO₁₁P₃S | 1332.82 | 8.72 | 26.25 |
| 2619 | C₆₀H₁₀₅N₃O₄S₆ | 1124.92 | 7.15 | 23.05 | 2819 | C₇₁H₁₂₁NO₁₀S₃ | 1244.93 | 9.53 | 26.84 |
| 2620 | C₅₆H₁₀₄N₂O_{S}S | 917.52 | 9.66 | 18.46 | 2820 | C₇₆H₁₃₀NO₁₂PS₃ | 1377.02 | 9.99 | 26.39 |
| 2621 | C₅₇H₁₀₀N₂O₁₀ | 973.43 | 7.44 | 19.35 | 2821 | C₆₉H₁₁₇NO₁₆S₄ | 1344.93 | 8.21 | 27.40 |
| 2622 | C₅₇H₁₀₁NOS₄ | 944.71 | 8.76 | 21.30 | 2822 | C₇₆H₁₃₀N₄O₈S₃ | 1324.08 | 10.01 | 27.21 |
| 2623 | C₆₁H₁₀₉N₃O₃S₆ | 1124.96 | 8.89 | 21.53 | 2823 | C₇₁H₁₂₁N₇O₈S₅ | 1361.09 | 7.65 | 25.06 |
| 2624 | C₇₂H₁₃₁N₃O₄S | 1134.92 | 8.90 | 25.07 | 2824 | C₇₄H₁₂₀N₂O₅S₃ | 1213.96 | 9.95 | 27.23 |
| 2625 | C₇₃H₁₃₇N₇O₆ | 1208.94 | 8.96 | 23.56 | 2825 | C₇₇H₁₂₀N₆O₇S₄ | 1370.08 | 7.80 | 26.86 |
| 2626 | C₇₂H₁₂₇N₇O₉ | 1234.85 | 8.10 | 24.19 | 2826 | C₇₃H₁₂₀N₂O₅S₄ | 1234.01 | 9.58 | 27.28 |
| 2627 | C₇₃H₁₃₅N₃O₉S | 1230.96 | 9.09 | 25.63 | 2827 | C₇₆H₁₂₄N₃O₁₀PS₃ | 1366.99 | 7.57 | 26.82 |
| 2628 | C₆₈H₁₂₇NO₃S₅ | 1167.10 | 9.70 | 29.88 | 2828 | C₇₂H₁₂₃NO₃S₇ | 1275.20 | 9.67 | 28.90 |
| 2629 | C₆₈H₁₂₇N₃S₇ | 1211.25 | 9.60 | 27.84 | 2829 | C₇₄H₁₂₃NO₂S₇ | 1283.22 | 9.86 | 30.94 |
| 2630 | C₇₁H₁₃₄NO₁₀P | 1192.82 | 9.64 | 24.22 | 2830 | C₇₂H₁₄₂N₂O₃ | 1083.94 | 9.36 | 28.29 |
| 2631 | C₆₈H₁₂₈N₄O₆S₂ | 1161.93 | 10.07 | 26.57 | 2831 | C₆₉H₁₃₄N₂O₅S | 1103.90 | 9.68 | 26.54 |
| 2632 | C₇₀H₁₃₀N₂O₈S₃ | 1224.02 | 9.71 | 26.56 | 2832 | C₇₁H₁₃₆N₂O₇S₂ | 1194.00 | 8.99 | 28.63 |
| 2633 | C₇₂H₁₃₁N₃O₉S₃ | 1279.05 | 8.83 | 27.55 | 2833 | C₆₅H₁₂₇NO₆S₂ | 1082.85 | 9.93 | 28.44 |
| 2634 | C₆₈H₁₂₅NO₇S₅ | 1229.08 | 9.96 | 29.51 | 2834 | C₇₂H₁₃₃N₃O₆S₃ | 1233.05 | 7.96 | 27.03 |
| 2635 | C₆₉H₁₃₀N₄O₄ | 1079.82 | 8.27 | 25.32 | 2835 | C₇₃H₁₃₉N₃O₆S₃ | 1251.11 | 8.84 | 28.79 |
| 2636 | C₆₈H₁₃₂N₂O₁₄S₃ | 1298.00 | 10.19 | 27.01 | 2836 | C₇₃H₁₃₉NO₅S₄ | 1239.16 | 10.13 | 31.72 |
| 2637 | C₇₂H₁₄₀N₃O₁₂P₃ | 1332.84 | 7.62 | 23.57 | 2837 | C₆₉H₁₃₁NOS₅ | 1151.11 | 9.73 | 29.40 |
| 2638 | C₇₁H₁₃₅N₇O₉S₃ | 1327.10 | 8.42 | 23.39 | 2838 | C₆₉H₁₃₆N₂S₆ | 1186.22 | 10.20 | 29.92 |
| 2639 | C₆₉H₁₃₁N₃O₁₂S₄ | 1323.08 | 8.78 | 25.51 | 2839 | C₆₈H₁₃₉N₉O₃S₂ | 1195.04 | 9.53 | 26.63 |
| 2640 | C₇₁H₁₂₇N₃O₇S₄ | 1263.08 | 7.70 | 27.37 | 2840 | C₇₀H₁₃₄N₆O₇S | 1203.94 | 8.24 | 27.19 |
| 2641 | C₇₂H₁₃₀N₄O₉S₃ | 1292.05 | 8.32 | 28.21 | 2841 | C₇₀H₁₃₃N₅O₉S | 1220.92 | 8.39 | 26.98 |
| 2642 | C₆₉H₁₂₉N₃O₆S₇ | 1321.27 | 9.35 | 27.16 | 2842 | C₆₉H₁₄₂N₆O₇S₃ | 1264.11 | 9.97 | 26.55 |
| 2643 | C₇₀H₁₂₉NS₈ | 1241.29 | 10.16 | 30.09 | 2843 | C₆₅H₁₃₇N₅O₉S₄ | 1261.08 | 8.86 | 24.06 |
| 2644 | C₅₈H₁₀₈N₄O₈S | 1021.59 | 10.16 | 30.09 | 2844 | C₇₁H₁₃₇NO₉ | 1148.88 | 10.05 | 28.77 |
| 2645 | C₅₆H₁₁₁N₆O₄P | 963.52 | 7.19 | 17.58 | 2845 | C₆₉H₁₃₇N₃O₆ | 1104.87 | 8.83 | 26.42 |
| 2646 | C₅₅H₁₀₆N₄O₆S₅ | 1079.81 | 9.87 | 19.57 | 2846 | C₇₂H₁₄₄N₃O₁₀P₃ | 1304.88 | 7.67 | 25.50 |
| 2647 | C₅₆H₁₀₇N₇O₈S₃ | 1102.71 | 8.21 | 17.61 | 2847 | C₇₁H₁₄₉N₂O₁₁P₃S | 1331.96 | 8.90 | 26.60 |
| 2648 | C₅₄H₁₀₂N₆O₈S₄ | 1091.71 | 7.81 | 16.98 | 2848 | C₆₆H₁₃₄NO₁₂PS₃ | 1260.95 | 9.07 | 25.89 |
| 2649 | C₅₈H₁₀₇N₇O₈S | 1062.60 | 8.83 | 17.19 | 2849 | C₆₃H₁₃₂N₄O₁₀S₄ | 1234.01 | 8.10 | 24.36 |
| 2650 | C₅₄H₁₀₄N₂O₅S | 893.50 | 9.68 | 18.78 | 2850 | C₇₂H₁₃₇NO₃S₅ | 1225.19 | 9.90 | 31.49 |
| 2651 | C₆₄H₁₂₄N₃O₁₄P₃S | 1284.69 | 8.20 | 17.97 | 2851 | C₇₀H₁₃₅NO₃S₅ | 1199.15 | 9.97 | 30.96 |
| 2652 | C₅₅H₁₀₃N₃O₁₀S₅ | 1126.77 | 8.47 | 18.72 | 2852 | C₇₁H₁₃₅N₃OS₆ | 1239.24 | 9.56 | 28.40 |
| 2653 | C₅₅H₁₀₀N₆O₁₃S₄ | 1181.70 | 8.28 | 18.03 | 2853 | C₅₃H₁₀₀N₆O₅ | 901.42 | 8.32 | 14.69 |
| 2654 | C₅₅H₁₀₆N₄O₇S₄ | 1063.74 | 9.80 | 19.25 | 2854 | C₅₀H₁₀₃N₁₁O₅ | 938.45 | 8.48 | 15.09 |
| 2655 | C₅₉H₁₀₄N₄O₄S₃ | 1029.71 | 9.13 | 19.59 | 2855 | C₅₂H₁₀₀N₆O₉S | 985.46 | 8.31 | 17.51 |
| 2656 | C₅₆H₁₀₃NO₇S₄ | 1030.71 | 9.87 | 21.24 | 2856 | C₅₃H₁₀₁N₃O₇ | 892.40 | 9.95 | 18.39 |
| 2657 | C₅₇H₁₀₄N₂OS₆ | 1025.87 | 10.01 | 20.81 | 2857 | C₅₁H₉₇NO₁₂S | 948.39 | 9.30 | 17.75 |
| 2658 | C₇₂H₁₂₈N₂OS₃ | 1134.03 | 10.16 | 24.30 | 2858 | C₅₀H₉₅NO₁₀S₄ | 998.55 | 8.15 | 20.65 |
| 2659 | C₆₉H₁₂₈N₄OS | 1061.88 | 10.02 | 24.28 | 2859 | C₅₄H₁₁₂NO₁₁P₃S | 1076.47 | 9.21 | 18.64 |
| 2660 | C₇₀H₁₂₉N₅O₇S₅ | 1313.16 | 8.99 | 22.78 | 2860 | C₅₃H₁₀₀N₆O₁₀S₄ | 1109.65 | 8.67 | 15.63 |
| 2661 | C₇₂H₁₃₃N₄O₉P | 1229.85 | 9.96 | 23.06 | 2861 | C₅₀H₁₀₁NO₁₄S₄ | 1068.59 | 10.07 | 20.13 |
| 2662 | C₆₈H₁₂₀N₂O₁₁ | 1141.71 | 9.11 | 21.06 | 2862 | C₅₁H₁₀₂N₂O₁₄S₅ | 1127.68 | 8.80 | 20.04 |
| 2663 | C₇₂H₁₂₅N₃O₉S₃ | 1273.00 | 9.10 | 26.58 | 2863 | C₅₁H₁₀₀N₆O₉S₄ | 1069.63 | 8.09 | 16.22 |
| 2664 | C₇₂H₁₂₅NO₈S₃ | 1228.99 | 10.00 | 27.22 | 2864 | C₄₆H₉₆N₄O₆S₅ | 961.60 | 9.69 | 17.23 |
| 2665 | C₇₅H₁₄₁N₂O₁₄P₃S | 1419.94 | 8.57 | 22.34 | 2865 | C₅₂H₁₀₂N₄O₁₂S₃ | 1071.58 | 8.63 | 16.45 |
| 2666 | C₆₇H₁₂₁N₃O₁₁S₄ | 1272.95 | 7.36 | 19.18 | 2866 | C₄₇H₉₁NO₃S₅ | 878.55 | 9.65 | 20.22 |
| 2667 | C₆₉H₁₂₆N₆O₇S₄ | 1280.04 | 9.00 | 22.36 | 2867 | C₅₁H₉₆N₄OS₆ | 973.72 | 9.50 | 17.13 |
| 2668 | C₆₉H₁₃₀N₄O₆S₅ | 1272.12 | 10.21 | 24.79 | 2868 | C₄₉H₉₉NO₃S₃ | 846.51 | 9.67 | 20.47 |
| 2669 | C₆₇H₁₁₇NO₆S₄ | 1160.91 | 8.83 | 24.72 | 2869 | C₄₈H₉₃N₃OS₇ | 952.71 | 7.90 | 20.75 |
| 2670 | C₇₃H₁₂₉N₃O₄S₅ | 1273.15 | 8.98 | 26.99 | 2870 | C₆₈H₁₃₂N₂O₆S₃ | 1169.99 | 8.20 | 27.11 |
| 2671 | C₆₇H₁₁₉NO₇S₇ | 1275.11 | 7.43 | 24.74 | 2871 | C₆₉H₁₃₁N₅O₇S₄ | 1271.07 | 8.05 | 28.00 |
| 2672 | C₇₀H₁₂₁N₃O₄S₇ | 1293.17 | 8.47 | 24.89 | 2872 | C₇₄H₁₄₇N₄O₁₃P₃S | 1425.99 | 8.26 | 21.77 |
| 2673 | C₆₇H₁₁₉N₃O₃S₄ | 1142.95 | 7.75 | 24.17 | 2873 | C₇₈H₁₅₅NO₁₃P₄S | 1471.05 | 9.23 | 26.84 |
| 2674 | C₆₆H₁₁₉NO₂S₇ | 1183.10 | 9.76 | 27.41 | 2874 | C₇₄H₁₄₇N₂O₁₂P₃S | 1381.97 | 7.99 | 25.04 |
| 2675 | C₇₄H₁₄₂N₄O₉S | 1264.03 | 8.40 | 26.85 | 2875 | C₈₀H₁₄₁N₇O₁₂S | 1425.10 | 8.28 | 27.74 |
| 2676 | C₇₂H₁₃₅N₃O₇S | 1186.95 | 7.50 | 28.39 | 2876 | C₇₅H₁₃₇N₅O₁₂S | 1333.00 | 7.02 | 25.80 |
| 2677 | C₇₄H₁₃₈N₄O₂S₃ | 1212.12 | 7.44 | 26.73 | 2877 | C₇₅H₁₄₆N₅O₈PS | 1309.05 | 9.53 | 24.72 |
| 2678 | C₇₅H₁₃₉N₃O₄S₄ | 1275.19 | 7.91 | 28.49 | 2878 | C₅₂H₁₀₁NO₁₁S₂ | 980.50 | 7.66 | 18.55 |
| 2679 | C₇₂H₁₃₆N₂O₂S₆ | 1254.25 | 8.75 | 29.44 | 2879 | C₆₈H₁₁₅NO₇S₄ | 1186.91 | 9.71 | 24.43 |
| 2680 | C₉₁H₁₁₇NO₄S | 1381.48 | 9.69 | 39.41 | 2880 | C₆₅H₁₁₃NO₁₀S₅ | 1228.92 | 7.12 | 22.76 |
| 2681 | C₈₉H₁₇₂N₂O₇ | 1382.36 | 9.42 | 37.27 | 2881 | C₆₅H₁₂₂NO₁₂P₃S | 1234.67 | 7.76 | 19.16 |
| 2682 | C₉₁H₁₇₆N₆O₇ | 1466.44 | 8.11 | 36.76 | 2882 | C₆₈H₁₁₅N₃O₁₂S | 1198.74 | 8.37 | 22.77 |
| 2683 | C₉₂H₁₇₈N₃O₁₀P | 1517.42 | 8.01 | 36.76 | 2883 | C₇₅H₁₃₀N₃O₆PS₄ | 1329.09 | 8.51 | 25.28 |
| 2684 | C₉₁H₁₇₇N₃O₇S₄ | 1553.67 | 8.56 | 37.79 | 2884 | C₇₆H₁₃₆N₂O₁₀S₅ | 1398.23 | 9.74 | 30.25 |
| 2685 | C₉₃H₁₇₉N₃O₃S₃ | 1483.65 | 8.65 | 37.45 | 2885 | C₇₀H₁₃₀NO₁₂PS₅ | 1369.08 | 9.35 | 22.77 |
| 2686 | C₉₀H₁₇₄N₄O₂S₆ | 1536.77 | 9.13 | 38.77 | 2886 | C₆₈H₁₂₇N₃O₈S₅ | 1275.08 | 7.49 | 18.47 |
| 2687 | C₈₉H₁₇₆N₄O₄ | 1366.41 | 9.65 | 36.39 | 2887 | C₅₆H₁₀₆N₄O₅S₂ | 979.61 | 8.60 | 19.07 |
| 2688 | C₈₈H₁₇₆N₄O₇S | 1434.46 | 7.44 | 34.46 | 2888 | C₅₇H₁₀₄N₄O₇S₄ | 1085.72 | 8.19 | 20.14 |
| 2689 | C₉₀H₁₈₀N₁₀O₃S | 1482.56 | 9.39 | 37.26 | 2889 | C₆₁H₁₀₆N₄O₈S₃ | 1119.72 | 7.67 | 21.03 |
| 2690 | C₉₃H₁₈₅N₉O₆S | 1557.62 | 8.83 | 35.92 | 2890 | C₅₉H₁₁₁N₃O₁₄S₆ | 1278.90 | 8.13 | 18.51 |
| 2691 | C₉₁H₁₈₀N₄O₈ | 1458.46 | 9.68 | 39.20 | 2891 | C₅₇H₁₀₇N₃O₁₂S₅ | 1186.79 | 8.93 | 18.22 |
| 2692 | C₈₄H₁₇₄N₄O₁₀S₄ | 1528.57 | 9.73 | 35.64 | 2892 | C₅₈H₁₀₈N₂O₄S₆ | 1089.87 | 9.71 | 23.62 |
| 2693 | C₉₁H₁₇₇NO₈ | 1413.42 | 9.74 | 39.59 | 2893 | C₇₅H₁₃₃N₃O₁₂S₃ | 1365.08 | 7.62 | 28.16 |
| 2694 | C₉₁H₁₇₇NO₈ | 1413.42 | 9.63 | 39.59 | 2894 | C₇₃H₁₄₄N₅O₇PS | 1267.02 | 8.77 | 23.15 |
| 2695 | C₉₄H₁₉₁NO₁₄S₄ | 1687.80 | 8.92 | 40.68 | 2895 | C₇₀H₁₃₅NO₃S₈ | 1295.33 | 9.28 | 29.89 |
| 2696 | C₉₈H₂₀₀N₄O₉S₃ | 1674.88 | 10.05 | 42.36 | 2896 | C₇₁H₁₄₅N₃OS₄ | 1185.20 | 10.13 | 32.12 |
| 2697 | C₁₀₁H₁₉₄N₂O₆S₄ | 1660.91 | 8.50 | 44.51 | 2897 | C₆₉H₁₃₈NO₁₃PS₅ | 1381.13 | 8.17 | 26.27 |
| 2698 | C₉₅H₁₈₇NO₂S₇ | 1599.97 | 7.87 | 43.73 | 2898 | C₇₆H₁₂₈N₄O₇S | 1241.94 | 9.76 | 26.18 |
| 2699 | C₉₆H₁₉₄N₂O₃S₇ | 1649.04 | 8.91 | 43.81 | 2899 | C₇₇H₁₂₈N₄O₁₀S₃ | 1366.07 | 9.13 | 28.18 |
| 2700 | C₇₃H₁₃₅N₃O₇S | 1198.96 | 8.83 | 27.72 | 2900 | C₇₈H₁₂₉N₇O₁₁S₄ | 1469.17 | 7.08 | 24.13 |
| 2701 | C₇₃H₁₃₂N₄O₂S₃ | 1194.07 | 8.26 | 26.95 | 2901 | C₇₆H₁₃₀N₆O₁₀S₆ | 1480.27 | 8.32 | 26.25 |
| 2702 | C₇₄H₁₄₃N₇O₆ | 1227.00 | 10.05 | 27.15 | 2902 | C₇₉H₁₂₈N₆O₁₂S₄ | 1482.16 | 8.21 | 26.97 |
| 2703 | C₇₂H₁₃₈N₄O₈S | 1219.98 | 9.16 | 26.82 | 2903 | C₇₈H₁₃₅N₅O₄ | 1206.97 | 9.64 | 29.41 |
| 2704 | C₇₀H₁₃₃N₅OS₂ | 1124.99 | 8.67 | 26.08 | 2904 | C₈₀H₁₂₉N₇O₇ | 1300.95 | 8.42 | 24.11 |
| 2705 | C₇₀H₁₃₀N₄O₆S | 1155.89 | 9.03 | 26.72 | 2905 | C₉₂H₁₅₆N₂O₅S₅ | 1530.57 | 8.87 | 38.21 |
| 2706 | C₇₁H₁₃₃N₅O₉S | 1232.93 | 7.56 | 25.84 | 2906 | C₉₅H₁₆₀N₃O₁₂PS₃ | 1663.49 | 7.57 | 35.94 |
| 2707 | C₇₂H₁₃₄N₄O₁₀S | 1247.94 | 9.32 | 26.54 | 2907 | C₉₁H₁₅₇NO₁₇S₅ | 1697.55 | 9.77 | 37.07 |
| 2708 | C₇₂H₁₃₂N₂O₁₂S | 1249.91 | 8.50 | 26.64 | 2908 | C₉₂H₁₅₉NOS₉ | 1583.83 | 9.44 | 41.50 |
| 2709 | C₆₈H₁₃₁NO₁₅S₃ | 1298.97 | 8.73 | 27.84 | 2909 | C₈₀H₁₅₇N₃O₁₁S₂ | 1401.27 | 7.36 | 30.07 |
| 2710 | C₇₀H₁₃₃N₃O₁₅S₄ | 1385.08 | 8.44 | 27.06 | 2910 | C₈₀H₁₆₁N₇O₉S | 1397.27 | 9.05 | 31.05 |
| 2711 | C₈₀H₁₅₇N₃O₁₄P₄ | 1509.04 | 8.07 | 24.70 | 2911 | C₈₆H₁₇₂N₃O₁₄P₃S₂ | 1629.37 | 7.98 | 30.43 |
| 2712 | C₆₉H₁₂₉N₃O₉S₄ | 1273.04 | 8.14 | 23.02 | 2912 | C₈₂H₁₅₉NO₈S | 1319.23 | 9.17 | 32.07 |
| 2713 | C₇₁H₁₃₉N₃O₁₀S₃ | 1291.08 | 10.20 | 26.84 | 2913 | C₈₂H₁₅₇NO₉S₂ | 1365.28 | 8.94 | 34.05 |
| 2714 | C₇₁H₁₃₀N₆O₉S₅ | 1372.15 | 7.66 | 25.94 | 2914 | C₈₂H₁₅₅NO₁₀S₃ | 1411.32 | 9.46 | 33.89 |
| 2715 | C₇₁H₁₃₁NO₄S₃ | 1159.01 | 9.05 | 28.83 | 2915 | C₈₂H₁₅₆N₆O₈ | 1354.18 | 7.58 | 27.67 |
| 2716 | C₇₄H₁₃₆N₄O₅S₃ | 1258.11 | 9.28 | 26.98 | 2916 | C₁₀₂H₂₀₀N₄O₆S | 1610.80 | 9.30 | 41.11 |
| 2717 | C₇₄H₁₄₀NO₆PS₆ | 1363.27 | 10.18 | 30.16 | 2917 | C₉₉H₁₉₉N₅O₁₂S₃ | 1747.88 | 8.74 | 39.52 |
| 2718 | C₇₂H₁₄₁NO₃S₄ | 1197.16 | 10.22 | 30.35 | 2918 | C₉₈H₂₀₁N₅O₁₂S₅ | 1802.01 | 7.78 | 38.29 |
| 2719 | C₇₄H₁₄₀N₂O₃S₆ | 1298.30 | 9.07 | 30.62 | 2919 | C₁₀₄H₂₀₂N₃O₁₃PS₂ | 1797.86 | 8.16 | 42.89 |
| 2720 | C₆₈H₁₃₃N₃O₅S | 1104.89 | 9.56 | 23.55 | 2920 | C₇₂H₁₄₃N₅O₆ | 1174.96 | 7.02 | 24.11 |
| 2721 | C₆₈H₁₃₁NO₂S₃ | 1090.98 | 10.20 | 27.08 | 2921 | C₇₅H₁₄₃NOS₄ | 1203.22 | 9.90 | 30.72 |
| 2722 | C₆₆H₁₂₇N₅O₇ | 1102.77 | 7.40 | 22.13 | 2922 | C₇₆H₁₄₆N₄O₈S₅ | 1404.32 | 7.57 | 29.76 |
| 2723 | C₆₇H₁₃₄N₆O₄S | 1119.91 | 8.03 | 22.12 | 2923 | C₇₆H₁₄₆N₄O₉S₃ | 1356.20 | 8.27 | 30.39 |
| 2724 | C₆₅H₁₃₅N₅O₉S₃ | 1227.00 | 9.81 | 23.41 | 2924 | C₇₈H₁₆₁N₂O₁₁P₃S | 1428.13 | 9.86 | 28.23 |
| 2725 | C₆₇H₁₂₇N₃O₇ | 1086.77 | 9.14 | 23.56 | 2925 | C₇₇H₁₅₆NO₁₁P₃S₂ | 1429.13 | 9.80 | 27.74 |
| 2726 | C₆₅H₁₂₆N₂O₁₂S | 1159.78 | 7.67 | 23.67 | 2926 | C₇₂H₁₄₆N₆O₉S₄ | 1368.23 | 7.44 | 25.66 |
| 2727 | C₆₆H₁₂₃N₃O₈S₅ | 1247.02 | 8.30 | 26.43 | 2927 | C₇₅H₁₄₇N₅O₇ | 1231.03 | 8.75 | 27.63 |
| 2728 | C₆₈H₁₄₁N₂O₁₀P₃ | 1239.80 | 9.68 | 23.81 | 2928 | C₇₁H₁₄₃NO₅S₃ | 1187.11 | 9.63 | 29.70 |
| 2729 | C₆₃H₁₂₅N₃O₁₄S₄ | 1276.94 | 7.77 | 24.47 | 2929 | C₇₇H₁₄₆N₂O₄S₆ | 1356.38 | 8.92 | 30.90 |
| 2730 | C₆₇H₁₃₃N₃O₇S₄ | 1221.05 | 8.93 | 22.48 | 2930 | C₇₅H₁₄₅N₃O₈S₃ | 1313.18 | 9.69 | 30.82 |
| 2731 | C₆₃H₁₂₃N₃O₃S₆ | 1163.05 | 7.51 | 26.47 | 2931 | C₇₄H₁₄₂N₆O₁₁S₅ | 1452.28 | 7.20 | 26.83 |
| 2732 | C₆₄H₁₃₁NO₃S₇ | 1187.18 | 9.65 | 28.98 | 2932 | C₁₀₇H₂₁₀N₄O₆S | 1680.94 | 9.30 | 44.75 |
| 2733 | C₆₃H₁₂₇NOS₈ | 1171.19 | 10.03 | 29.92 | 2933 | C₁₀₃H₂₀₅N₅OS₇ | 1754.23 | 7.43 | 49.14 |
| 2734 | C₈₂H₁₆₂NO₆P | 1289.17 | 10.20 | 32.78 | 2934 | C₁₀₈H₂₁₂NO₇PS | 1699.92 | 7.96 | 46.71 |
| 2735 | C₇₉H₁₅₃NO₄S₂ | 1245.22 | 9.98 | 34.17 | 2935 | C₉₉H₂₀₀N₄O₂S₃ | 1574.89 | 9.31 | 44.17 |
| 2736 | C₇₉H₁₅₁N₃O₁₀S | 1335.15 | 7.16 | 30.27 | 2936 | C₁₀₀H₁₉₉N₇O₁₁S₄ | 1803.97 | 7.82 | 41.43 |
| 2737 | C₇₉H₁₅₁N₂O₁₀P | 1320.05 | 7.65 | 29.97 | 2937 | C₉₇H₁₉₇NO₁₉S₅ | 1841.93 | 9.29 | 43.58 |
| 2738 | C₇₉H₁₅₂N₄O₅S₃ | 1334.29 | 8.33 | 31.06 | 2938 | C₉₉H₁₉₄N₂O₈S | 1572.71 | 7.31 | 41.98 |
| 2739 | C₇₇H₁₄₉NO₄S₃ | 1249.22 | 9.44 | 32.44 | 2939 | C₉₄H₁₇₈N₄O₅S₃ | 1540.66 | 7.70 | 37.45 |
| 2740 | C₇₈H₁₅₃N₉O₃S₃ | 1361.32 | 8.05 | 31.75 | 2940 | C₇₄H₁₃₇N₃O₄S₅ | 1293.23 | 8.28 | 28.37 |
| 2741 | C₇₅H₁₅₀N₆O₉S₃ | 1376.24 | 7.11 | 28.59 | 2941 | C₇₃H₁₅₁N₂O₁₄P₃S | 1466.05 | 8.41 | 24.98 |
| 2742 | C₈₀H₁₅₄N₄O₉ | 1316.13 | 8.39 | 31.34 | 2942 | C₇₃H₁₄₁N₇O₈S₃ | 1341.15 | 9.86 | 23.70 |
| 2743 | C₇₈H₁₅₃NO₈S | 1265.14 | 9.73 | 30.75 | 2943 | C₇₆H₁₃₆N₄O₈S | 1266.00 | 7.96 | 27.68 |
| 2744 | C₇₇H₁₅₁NO₆S₂ | 1251.18 | 10.04 | 33.91 | 2944 | C₆₉H₁₃₅N₅O₈S₃ | 1259.05 | 9.93 | 26.72 |
| 2745 | C₇₈H₁₅₆N₄O₉S | 1326.18 | 9.48 | 31.83 | 2945 | C₆₉H₁₃₅NO₃S₆ | 1219.20 | 10.26 | 30.26 |
| 2746 | C₈₀H₁₅₂N₂O₁₀S₄ | 1430.34 | 8.79 | 32.39 | 2946 | C₆₅H₁₂₉NO₃S₆ | 1165.11 | 10.03 | 29.30 |
| 2747 | C₇₉H₁₆₀N₄O₄ | 1230.17 | 9.90 | 31.06 | 2947 | C₇₀H₁₃₇N₄O₁₁PS₄ | 1370.10 | 9.59 | 27.67 |
| 2748 | C₈₂H₁₆₈NO₁₁P₃S | 1469.22 | 9.35 | 31.16 | 2948 | C₇₃H₁₄₉N₂O₁₃P₃S | 1387.98 | 9.89 | 25.38 |
| 2749 | C₇₄H₁₄₉NO₉S₄ | 1325.24 | 9.52 | 31.12 | 2949 | C₆₆H₁₃₀N₆O₆S | 1135.86 | 8.58 | 21.52 |
| 2750 | C₇₆H₁₅₁N₃O₉S₃ | 1347.24 | 7.62 | 28.28 | 2950 | C₆₉H₁₃₂N₂O₈S | 1149.88 | 9.31 | 25.41 |
| 2751 | C₈₀H₁₆₃NO₉S₄ | 1411.42 | 10.02 | 31.61 | 2951 | C₆₇H₁₃₀NO₁₁P | 1156.75 | 9.20 | 24.28 |
| 2752 | C₇₇H₁₅₅N₇O₈S₃ | 1403.31 | 8.62 | 28.92 | 2952 | C₇₄H₁₄₁N₄O₇PS | 1262.00 | 10.07 | 25.50 |
| 2753 | C₇₇H₁₅₅NO₁₂S₄ | 1415.32 | 9.90 | 31.42 | 2953 | C₇₉H₁₃₉N₇O₂S₈ | 1475.51 | 8.66 | 30.35 |
| 2754 | C₇₉H₁₅₀N₂O₄S₄ | 1320.32 | 8.20 | 32.38 | 2954 | C₈₀H₁₄₆N₃O₇PS₆ | 1485.40 | 8.03 | 29.10 |
| 2755 | C₈₀H₁₅₂N₂O₅S₅ | 1382.40 | 8.23 | 33.60 | 2955 | C₇₂H₁₃₉NO₁₄S₅ | 1403.20 | 10.24 | 26.99 |
| 2756 | C₈₂H₁₅₇N₃O₉S₄ | 1457.41 | 8.95 | 34.69 | 2956 | C₆₈H₁₃₄N₂O₁₁S₅ | 1316.12 | 9.97 | 23.02 |
| 2757 | C₇₈H₁₅₃N₂O₈PS₆ | 1470.42 | 7.17 | 32.63 | 2957 | C₇₃H₁₃₈N₄O₅S₇ | 1376.35 | 8.95 | 30.58 |
| 2758 | C₇₈H₁₄₇N₃OS₆ | 1335.41 | 8.18 | 32.47 | 2958 | C₇₆H₁₃₆N₂O₈S | 1237.99 | 8.81 | 25.65 |
| 2759 | C₇₈H₁₅₁N₇O₃S₆ | 1427.47 | 8.10 | 32.55 | 2959 | C₅₅H₁₀₅N₅O₉S | 1012.53 | 9.99 | 19.03 |
| 2760 | C₈₀H₁₅₇N₇OS₆ | 1425.54 | 9.93 | 32.94 | 2960 | C₅₅H₁₀₃N₃O₇S₈ | 1174.92 | 9.06 | 19.72 |
| 2761 | C₁₀₃H₁₉₈N₁₀O₄ | 1640.78 | 8.43 | 38.34 | 2961 | C₅₅H₉₈N₂O₇S₆ | 1091.76 | 8.02 | 19.51 |
| 2762 | C₉₉H₁₉₁NO₈S | 1555.68 | 9.65 | 43.86 | 2962 | C₅₆H₁₀₄N₂O₇S₄ | 1045.70 | 8.13 | 19.65 |
| 2763 | C₉₉H₁₉₁NO₇S₂ | 1571.74 | 9.34 | 44.32 | 2963 | C₅₅H₁₀₃N₃O₁₃S₄ | 1142.68 | 8.95 | 20.08 |
| 2764 | C₁₀₅H₂₀₅NO₅S₃ | 1657.98 | 10.13 | 47.57 | 2964 | C₄₉H₉₇NO₄S₅ | 924.62 | 7.78 | 18.16 |
| 2765 | C₁₀₀H₁₉₈NO₃PS₃ | 1589.84 | 8.58 | 43.46 | 2965 | C₆₀H₁₁₇N₄O₁₅P₃ | 1227.53 | 8.09 | 15.71 |
| 2766 | C₁₀₂H₂₀₀N₂OS₆ | 1663.09 | 10.10 | 45.62 | 2966 | C₅₃H₁₀₅NO₁₂S₅ | 1108.72 | 10.13 | 18.40 |
| 2767 | C₁₀₄H₂₀₅NO₆S | 1597.85 | 7.66 | 46.47 | 2967 | C₅₅H₁₀₈N₃O₁₆PS₅ | 1258.75 | 9.37 | 19.39 |
| 2768 | C₁₀₄H₂₀₉N₇O₃S₂ | 1669.98 | 9.88 | 46.29 | 2968 | C₅₄H₁₀₄N₄O₁₀S | 1001.50 | 8.74 | 17.69 |
| 2769 | C₁₀₅H₂₀₈N₆O₈ | 1682.85 | 9.03 | 44.74 | 2969 | C₅₄H₁₀₆N₂O₁₀S₂ | 1007.57 | 10.02 | 18.69 |
| 2770 | C₁₀₄H₂₀₄N₄O₇S₂ | 1686.92 | 9.42 | 47.15 | 2970 | C₆₆H₁₁₇N₅O₈S | 1140.75 | 9.75 | 19.41 |
| 2771 | C₁₀₉H₂₀₈N₂O₉ | 1690.87 | 8.92 | 46.22 | 2971 | C₆₈H₁₂₁N₇O₅ | 1116.76 | 8.93 | 17.67 |
| 2772 | C₁₀₅H₂₁₀N₄O₁₀S | 1720.91 | 9.84 | 46.22 | 2972 | C₆₅H₁₁₄N₂O₅S₅ | 1163.94 | 10.02 | 23.50 |
| 2773 | C₁₀₅H₂₀₁N₃O₁₁S | 1713.83 | 7.46 | 46.28 | 2973 | C₆₈H₁₁₄N₂O₉S₂ | 1167.78 | 8.32 | 21.74 |
| 2774 | C₁₀₂H₂₀₅N₃O₈S | 1633.83 | 8.04 | 44.66 | 2974 | C₇₁H₁₂₆NO₄PS₇ | 1313.19 | 10.09 | 25.85 |
| 2775 | C₁₀₉H₂₂₃N₂O₁₁P₃S | 1862.97 | 9.95 | 45.08 | 2975 | C₇₄H₁₂₉N₃O₆S₉ | 1445.40 | 8.19 | 27.04 |
| 2776 | C₁₀₂H₂₀₈N₂O₉S₃ | 1702.97 | 10.14 | 46.00 | 2976 | C₇₄H₁₂₉NO₆S₂ | 1192.97 | 10.35 | 24.90 |
| 2777 | C₁₀₇H₂₀₈N₄O₃S₅ | 1759.17 | 8.34 | 49.21 | 2977 | C₇₃H₁₂₅N₃O₁₁S₂ | 1284.93 | 8.96 | 23.77 |
| 2778 | C₁₀₄H₂₀₃NO₁₀S₄ | 1756.01 | 9.55 | 48.00 | 2978 | C₇₁H₁₂₅N₅O₁₄S₂ | 1336.92 | 8.57 | 20.70 |
| 2779 | C₁₀₅H₂₀₃NO₇S₄ | 1720.02 | 8.74 | 49.41 | 2979 | C₅₉H₁₀₈N₂O₃S₄ | 1021.76 | 10.20 | 20.34 |
| 2780 | C₁₀₈H₂₁₃NO₆S₇ | 1846.31 | 10.05 | 50.72 | 2980 | C₆₁H₁₁₈NO₁₄PS₄ | 1248.82 | 10.25 | 22.37 |
| 2781 | C₁₀₃H₂₀₃N₃O₂S₃ | 1611.96 | 8.66 | 46.73 | 2981 | C₅₆H₁₀₆N₂O₁₁S₄ | 1111.71 | 8.96 | 17.71 |
| 2782 | C₁₀₄H₂₀₈N₂O₂S₃ | 1615.00 | 9.88 | 47.93 | 2982 | C₅₇H₁₀₈N₂O₁₅S₃ | 1157.67 | 10.05 | 20.10 |
| 2783 | C₁₀₁H₂₀₄N₂OS₆ | 1655.12 | 8.86 | 49.01 | 2983 | C₆₀H₁₁₀NO₇P | 988.51 | 10.15 | 18.89 |
| 2784 | C₁₀₁H₂₀₅NS₈ | 1690.24 | 9.98 | 51.49 | 2984 | C₆₁H₁₀₅N₇O₅ | 1016.55 | 8.67 | 17.66 |
| 2785 | C₁₀₂H₂₀₅N₆O₆P | 1642.77 | 9.54 | 39.05 | 2985 | C₆₁H₁₁₂N₂O₉S₂ | 1081.69 | 10.11 | 20.51 |
| 2786 | C₁₀₃H₂₀₃N₉O₅S₂ | 1711.93 | 8.13 | 42.14 | 2986 | C₅₉H₁₀₉N₇O₇S | 1060.62 | 8.74 | 15.29 |
| 2787 | C₉₉H₁₉₈N₈O₅ | 1580.72 | 9.68 | 41.14 | 2987 | C₇₅H₁₃₈N₆O₁₂ | 1315.96 | 8.80 | 26.03 |
| 2788 | C₁₀₀H₂₀₂N₇O₆P | 1629.73 | 9.66 | 40.59 | 2988 | C₇₃H₁₃₄N₃O₁₂PS₅ | 1437.16 | 8.84 | 29.13 |
| 2789 | C₁₀₃H₂₀₃N₉O₅S | 1679.88 | 8.70 | 41.15 | 2989 | C₇₀H₁₃₄N₄O₄S₃ | 1192.05 | 9.54 | 26.89 |
| 2790 | C₁₀₀H₁₉₄N₁₀O₈S | 1696.77 | 7.27 | 40.42 | 2990 | C₆₉H₁₂₉N₃O₁₀S₅ | 1321.10 | 9.31 | 24.87 |
| 2791 | C₉₈H₁₉₉N₆O₃PS | 1572.74 | 8.26 | 41.72 | 2991 | C₇₀H₁₄₁NO₄S | 1092.96 | 10.37 | 29.21 |
| 2792 | C₉₅H₁₉₄N₄O₈S₃ | 1616.80 | 8.77 | 39.52 | 2992 | C₇₀H₁₄₀N₁₀O₆S | 1250.01 | 9.15 | 24.15 |
| 2793 | C₉₅H₁₉₄N₆O₈S₃ | 1644.81 | 7.43 | 39.83 | 2993 | C₇₂H₁₃₇NO₄S₅ | 1241.19 | 9.95 | 30.25 |
| 2794 | C₁₀₀H₁₉₉N₅O₈ | 1599.72 | 10.02 | 42.80 | 2994 | C₇₁H₁₄₆N₆O₂ | 1115.99 | 10.21 | 26.99 |
| 2795 | C₁₀₄H₂₁₃N₄O₉P₃S | 1788.85 | 9.70 | 40.93 | 2995 | C₇₇H₁₃₁N₃O₆S | 1226.97 | 9.99 | 28.80 |
| 2796 | C₉₄H₁₉₂N₂O₁₁S₄ | 1654.81 | 9.58 | 41.21 | 2996 | C₈₄H₁₄₁N₂O₁₂P₃S₄ | 1592.22 | 8.25 | 26.39 |
| 2797 | C₁₀₀H₂₀₂NO₁₅PS₃ | 1785.86 | 9.99 | 43.01 | 2997 | C₇₆H₁₂₇N₇O₄ | 1202.90 | 9.28 | 25.07 |
| 2798 | C₉₄H₁₉₁NO₁₇S₄ | 1735.79 | 8.03 | 41.32 | 2998 | C₇₆H₁₂₁N₃O₇S₃ | 1285.00 | 8.58 | 26.20 |
| 2799 | C₉₆H₁₉₃NO₁₄S₅ | 1745.89 | 9.20 | 43.57 | 2999 | C₇₅H₁₁₉NO₁₀S | 1226.83 | 10.03 | 27.76 |
| 2800 | C₉₇H₁₉₈N₄O₈S₃ | 1644.85 | 10.17 | 41.87 | 3000 | C₇₇H₁₂₃NO₁₁S₃ | 1335.01 | 9.89 | 27.55 |
| 3001 | C₉₀H₁₅₇N₇O₁₀S₃ | 1593.47 | 8.81 | 31.48 | 3201 | C₇₂H₁₂₆N₂O₉ | 1163.81 | 9.97 | 23.54 |
| 3002 | C₈₉H₁₅₅NO₆S₈ | 1591.70 | 9.94 | 37.95 | 3202 | C₇₀H₁₂₄N₄O₈ | 1149.78 | 9.97 | 23.36 |
| 3003 | C₈₄H₁₆₈NO₁₀P₃S₃ | 1541.37 | 9.83 | 31.59 | 3203 | C₇₁H₁₂₅NO₁₄S | 1248.83 | 9.73 | 22.70 |
| 3004 | C₈₀H₁₆₃N₅O₈S₄ | 1451.45 | 10.12 | 31.84 | 3204 | C₇₀H₁₁₇N₃O₁₅ | 1240.71 | 7.26 | 20.93 |
| 3005 | C₈₀H₁₅₂N₄O₇ | 1282.12 | 8.76 | 29.06 | 3205 | C₅₇H₉₉N₃O₃S₈ | 1130.92 | 8.63 | 19.99 |
| 3006 | C₁₀₁H₁₉₈N₄O₅S₂ | 1612.84 | 9.00 | 42.07 | 3206 | C₆₃H₁₀₉N₆O₅PS₆ | 1253.94 | 8.12 | 21.93 |
| 3007 | C₁₀₃H₁₉₉N₃O₇S₄ | 1719.98 | 8.96 | 44.17 | 3207 | C₅₈H₁₀₅N₂O₄PS₆ | 1117.82 | 9.26 | 21.79 |
| 3008 | C₁₀₄H₁₉₆N₄O₁₂S₃ | 1790.91 | 8.40 | 43.89 | 3208 | C₅₉H₁₀₅NO₅S₄ | 1036.73 | 9.99 | 21.27 |
| 3009 | C₁₀₅H₂₀₈N₉O₁₂PS₄ | 1948.08 | 8.72 | 40.74 | 3209 | C₅₇H₁₀₃N₃O₁₀S₄ | 1118.70 | 9.68 | 21.46 |
| 3010 | C₇₉H₁₅₂N₆O₅S | 1298.18 | 9.08 | 25.22 | 3210 | C₅₉H₁₁₄NO₁₄P₃S₂ | 1218.60 | 8.06 | 17.66 |
| 3011 | C₇₆H₁₅₂N₆O₁₀S | 1342.14 | 9.91 | 26.73 | 3211 | C₆₀H₁₁₆NO₁₂P₃S₂ | 1200.62 | 8.76 | 19.01 |
| 3012 | C₇₃H₁₄₂N₂O₃S₆ | 1288.31 | 8.72 | 30.69 | 3212 | C₆₆H₁₂₉N₃O₁₄P₄ | 1312.66 | 8.69 | 16.22 |
| 3013 | C₇₇H₁₄₅N₅O₁₀S₃ | 1397.21 | 8.79 | 29.01 | 3213 | C₆₂H₁₁₇N₂O₁₃P₃ | 1191.54 | 8.34 | 16.88 |
| 3014 | C₇₇H₁₄₁N₇O₆S₈ | 1517.50 | 8.62 | 30.23 | 3214 | C₅₅H₁₀₄N₆O₉S₃ | 1089.65 | 8.26 | 15.77 |
| 3015 | C₇₅H₁₅₂N₅O₁₂PS₃ | 1443.22 | 8.29 | 25.88 | 3215 | C₅₄H₁₀₂N₂O₁₂S₅ | 1131.71 | 8.76 | 17.82 |
| 3016 | C₇₄H₁₄₅N₃O₁₅S₄ | 1445.22 | 9.05 | 25.98 | 3216 | C₅₅H₁₀₃NO₁₅S₅ | 1178.72 | 9.92 | 19.53 |
| 3017 | C₇₂H₁₄₂N₂O₉S₆ | 1372.29 | 8.96 | 24.57 | 3217 | C₅₄H₁₀₅N₅O₄S | 920.52 | 9.05 | 18.70 |
| 3018 | C₇₇H₁₅₇N₉O₇S | 1353.22 | 10.26 | 29.33 | 3218 | C₅₃H₁₀₁NO₄S₉ | 1104.93 | 8.98 | 24.27 |
| 3019 | C₇₃H₁₄₅N₃O₅S₅ | 1305.28 | 8.80 | 30.66 | 3219 | C₆₀H₁₀₅N₂O₁₂P | 1077.48 | 8.29 | 18.94 |
| 3020 | C₇₄H₁₄₉N₇O₁₀S₄ | 1425.28 | 8.95 | 26.66 | 3220 | C₅₈H₁₀₅NO₁₆S | 1104.53 | 9.39 | 18.70 |
| 3021 | C₇₁H₁₃₉N₃O₁₆S₅ | 1451.20 | 8.48 | 24.73 | 3221 | C₇₀H₁₂₆N₆O₆S | 1179.87 | 7.23 | 23.31 |
| 3022 | C₇₄H₁₄₅N₃O₁₁S₆ | 1445.34 | 9.07 | 26.30 | 3222 | C₇₄H₁₃₄N₁₀O₁₁S | 1372.00 | 8.45 | 23.42 |
| 3023 | C₇₂H₁₄₅N₄O₁₀PS₃ | 1354.12 | 8.61 | 23.41 | 3223 | C₆₉H₁₂₃NS₇ | 1191.17 | 9.86 | 26.81 |
| 3024 | C₇₃H₁₄₇N₄O₉PS₅ | 1416.27 | 8.33 | 24.01 | 3224 | C₇₁H₁₂₆N₄O₄S₄ | 1228.05 | 7.91 | 25.16 |
| 3025 | C₇₄H₁₄₁NO₁₁S₂ | 1285.06 | 7.98 | 28.99 | 3225 | C₇₁H₁₂₈N₂O₅S₆ | 1282.17 | 9.30 | 28.34 |
| 3026 | C₁₀₉H₂₁₃N₅O₈ | 1721.93 | 8.96 | 42.65 | 3226 | C₇₇H₁₃₇N₆O₇PS₅ | 1450.25 | 8.46 | 25.30 |
| 3027 | C₁₀₄H₂₀₅NOS₇ | 1710.21 | 10.17 | 49.05 | 3227 | C₇₀H₁₂₅N₃O₁₀S₄ | 1297.02 | 7.45 | 26.12 |
| 3028 | C₁₁₁H₂₁₃N₃O₃S₈ | 1894.42 | 8.11 | 50.12 | 3228 | C₇₂H₁₃₁N₅O₁₁S₃ | 1339.04 | 8.71 | 27.24 |
| 3029 | C₁₁₀H₂₂₂N₃O₁₁P₃S | 1887.98 | 8.96 | 42.00 | 3229 | C₇₅H₁₄₆N₇O₁₁P₃ | 1414.95 | 7.81 | 22.60 |
| 3030 | C₁₁₀H₂₂₂N₃O₁₁P₃S | 1887.98 | 8.96 | 42.00 | 3230 | C₇₃H₁₄₀N₃O₁₄P₃S | 1408.91 | 8.27 | 22.79 |
| 3031 | C₁₀₆H₂₁₁N₃O₁₃S₂ | 1799.98 | 10.02 | 45.91 | 3231 | C₇₂H₁₄₀N₃O₁₄P₃ | 1364.84 | 8.46 | 23.22 |
| 3032 | C₁₀₃H₂₀₅N₆O₁₀P | 1718.78 | 8.96 | 40.53 | 3232 | C₇₀H₁₃₁N₅O₁₃S₄ | 1379.08 | 8.01 | 22.50 |
| 3033 | C₁₀₅H₂₁₃N₄O₁₃P₃S₂ | 1896.92 | 8.94 | 39.79 | 3233 | C₆₇H₁₃₁N₅O₈S₅ | 1295.11 | 9.77 | 25.44 |
| 3034 | C₉₈H₁₉₂N₂O₁₂S₄ | 1718.86 | 9.02 | 40.76 | 3234 | C₆₅H₁₂₄N₂O₁₄S₅ | 1318.01 | 7.85 | 23.07 |
| 3035 | C₉₃H₁₈₀N₄O₈S | 1514.54 | 9.95 | 38.53 | 3235 | C₇₃H₁₃₉NO₉S₆ | 1367.27 | 10.04 | 28.32 |
| 3036 | C₉₆H₁₈₆N₂O₂S₈ | 1657.04 | 10.34 | 44.16 | 3236 | C₇₃H₁₃₀N₄O₈S₃ | 1288.04 | 8.18 | 26.62 |
| 3037 | C₉₄H₁₈₀N₄O₅S₇ | 1670.92 | 8.81 | 39.99 | 3237 | C₇₂H₁₃₂N₄O₁₀S | 1245.93 | 9.51 | 23.80 |
| 3038 | C₈₉H₁₇₇NO₁₅S₃ | 1597.57 | 10.12 | 38.26 | 3238 | C₇₀H₁₃₂NO₁₂PS₂ | 1274.91 | 9.58 | 26.32 |
| 3039 | C₉₂H₁₇₇NO₆S₃ | 1489.61 | 9.88 | 38.99 | 3239 | C₇₃H₁₃₈N₆O₁₀S | 1292.00 | 8.50 | 25.82 |
| 3040 | C₉₃H₁₈₀N₂O₁₂S₂ | 1582.59 | 8.97 | 38.02 | 3240 | C₇₁H₁₃₇N₇O₁₂S | 1312.97 | 7.95 | 25.01 |
| 3041 | C₇₈H₁₃₉N₃O₆S₅ | 1375.28 | 9.03 | 28.75 | 3241 | C₇₀H₁₃₂N₂O₂S₅ | 1194.14 | 9.86 | 28.40 |
| 3042 | C₇₁H₁₃₈N₆O₁₃S₅ | 1444.21 | 8.79 | 24.44 | 3242 | C₆₈H₁₂₇N₃S₉ | 1275.32 | 8.31 | 29.94 |
| 3043 | C₇₄H₁₃₇NO₁₀S₃ | 1297.09 | 10.18 | 28.33 | 3243 | C₇₀H₁₃₂N₂O₉S₄ | 1274.07 | 8.35 | 27.50 |
| 3044 | C₅₇H₁₀₉N₅O₈S₅ | 1152.83 | 8.75 | 21.16 | 3244 | C₆₉H₁₃₄N₂O₄S₄ | 1184.08 | 8.84 | 29.64 |
| 3045 | C₅₆H₁₁₂N₆O₃S₂ | 981.67 | 8.79 | 18.08 | 3245 | C₇₁H₁₃₄N₂O₅S₇ | 1320.28 | 7.60 | 30.36 |
| 3046 | C₇₀H₁₂₄N₆O₅S₃ | 1225.98 | 9.62 | 21.57 | 3246 | C₇₀H₁₄₂NO₁₂P₃S₂ | 1346.94 | 8.97 | 25.79 |
| 3047 | C₇₅H₁₄₀N₈O₅S₃ | 1330.18 | 10.00 | 23.56 | 3247 | C₇₂H₁₄₅N₂O₁₂P₃S₂ | 1388.00 | 8.91 | 24.47 |
| 3048 | C₇₄H₁₃₉N₅O₁₁ | 1274.95 | 9.94 | 26.43 | 3248 | C₆₅H₁₃₄N₄O₉S₅ | 1276.11 | 8.95 | 25.16 |
| 3049 | C₇₅H₁₃₇N₃O₃S₇ | 1353.36 | 8.51 | 29.95 | 3249 | C₆₈H₁₄₁N₅O₉S₆ | 1365.26 | 9.94 | 24.68 |
| 3050 | C₇₁H₁₄₇N₅O₉S₆ | 1407.34 | 9.97 | 26.49 | 3250 | C₆₅H₁₃₀N₄O₁₆S₄ | 1352.01 | 8.42 | 25.08 |
| 3051 | C₈₁H₁₃₇N₃O₁₀S₅ | 1473.30 | 9.99 | 30.81 | 3251 | C₆₇H₁₃₅N₅OS₂ | 1090.97 | 9.62 | 24.49 |
| 3052 | C₈₂H₁₂₉N₃O₈S₇ | 1509.37 | 7.82 | 29.90 | 3252 | C₆₉H₁₃₈N₆O₅S | 1163.96 | 8.79 | 24.62 |
| 3053 | C₉₆H₁₆₅N₂O₁₁PS₄ | 1682.59 | 9.47 | 37.52 | 3253 | C₇₀H₁₃₄N₄O₁₁S₂ | 1271.98 | 7.29 | 25.51 |
| 3054 | C₉₃H₁₆₃N₃O₃S₅ | 1531.64 | 8.96 | 37.35 | 3254 | C₇₁H₁₃₇NO₆S₂ | 1165.00 | 10.07 | 27.92 |
| 3055 | C₉₈H₁₇₂NO₁₀P₃S₃ | 1713.55 | 7.92 | 34.58 | 3255 | C₇₄H₁₄₀N₄O₉S₂ | 1294.07 | 8.00 | 27.90 |
| 3056 | C₉₆H₁₆₀N₄O₆S | 1498.42 | 8.16 | 36.02 | 3256 | C₇₃H₁₃₉NO₁₀S₂ | 1255.03 | 10.04 | 28.57 |
| 3057 | C₈₇H₁₇₄NO₁₃P₃ | 1535.26 | 9.76 | 31.67 | 3257 | C₇₂H₁₄₁NO₁₃S | 1260.97 | 10.11 | 29.32 |
| 3058 | C₇₉H₁₅₃N₂O₇PS₃ | 1370.25 | 9.95 | 30.27 | 3258 | C₇₈H₁₂₆N₈O₉ | 1319.91 | 8.30 | 26.16 |
| 3059 | C₇₅H₁₄₈N₆O₈S₆ | 1454.40 | 8.59 | 27.41 | 3259 | C₇₆H₁₂₅N₅O₁₀S₂ | 1332.98 | 8.90 | 26.29 |
| 3060 | C₁₁₀H₂₂₀N₁₀O₇ | 1795.03 | 9.47 | 43.87 | 3260 | C₇₈H₁₃₄N₁₂O₄S₂ | 1368.13 | 9.28 | 25.15 |
| 3061 | C₁₁₀H₂₁₃N₃O₁₀S₅ | 1898.22 | 8.79 | 48.01 | 3261 | C₇₇H₁₂₇N₉O₅S₂ | 1323.04 | 8.94 | 26.78 |
| 3062 | C₁₁₀H₂₂₃N₂O₁₁P₃S₂ | 1907.04 | 9.90 | 44.27 | 3262 | C₇₇H₁₂₅N₂O₆PS₈ | 1462.31 | 8.08 | 28.61 |
| 3063 | C₁₀₉H₂₁₄N₄O₆ | 1676.93 | 9.12 | 44.30 | 3263 | C₇₇H₁₂₃NO₄S₇ | 1351.25 | 9.51 | 29.37 |
| 3064 | C₁₀₇H₂₁₀N₆O₈ | 1708.89 | 10.02 | 43.55 | 3264 | C₇₇H₁₂₂N₄O₁₁S₄ | 1408.08 | 7.58 | 26.28 |
| 3065 | C₉₃H₁₈₅N₃O₁₅S₄ | 1713.75 | 8.68 | 36.47 | 3265 | C₈₁H₁₄₁N₆O₁₂P₃ | 1483.97 | 7.90 | 24.37 |
| 3066 | C₉₁H₁₈₃NO₁₆S₅ | 1707.76 | 9.33 | 39.98 | 3266 | C₇₃H₁₂₇N₅O₈S₄ | 1331.09 | 9.94 | 26.26 |
| 3067 | C₈₀H₁₄₆N₃O₉PS₃ | 1421.21 | 9.01 | 30.06 | 3267 | C₇₄H₁₃₂N₄O₁₂P₂S₃ | 1428.01 | 9.34 | 25.27 |
| 3068 | C₇₄H₁₃₈N₂O₁₂S | 1279.98 | 9.95 | 27.25 | 3268 | C₇₆H₁₃₁N₃O₁₃S₄ | 1423.13 | 9.02 | 27.31 |
| 3069 | C₇₃H₁₄₈N₉O₁₁PS | 1391.07 | 8.77 | 22.93 | 3269 | C₇₅H₁₂₇N₅O₉S₃ | 1339.05 | 9.48 | 21.87 |
| 3070 | C₆₉H₁₃₄NO₈PS₄ | 1265.04 | 9.98 | 27.73 | 3270 | C₇₅H₁₂₂N₂O₈ | 1179.81 | 8.88 | 25.72 |
| 3071 | C₇₃H₁₄₂N₃O₁₁PS₃ | 1365.10 | 9.62 | 27.62 | 3271 | C₇₉H₁₂₈N₂O₁₂S₂ | 1362.01 | 9.85 | 28.34 |
| 3072 | C₅₆H₁₀₄N₄S₅ | 993.78 | 8.81 | 19.28 | 3272 | C₇₆H₁₂₄N₂O₁₁S₂ | 1305.95 | 9.98 | 27.72 |
| 3073 | C₆₈H₁₂₃N₂O₉P | 1143.71 | 8.74 | 20.97 | 3273 | C₉₁H₁₅₄N₆O₈S₂ | 1524.39 | 8.48 | 30.75 |
| 3074 | C₇₇H₁₃₇N₄O₁₂PS₃ | 1438.11 | 8.96 | 25.55 | 3274 | C₉₂H₁₅₅N₉O₈ | 1515.31 | 8.03 | 32.69 |
| 3075 | C₇₄H₁₃₄N₄O₇S | 1223.97 | 10.08 | 21.69 | 3275 | C₉₃H₁₆₁N₄O₉PS | 1542.36 | 9.40 | 34.53 |
| 3076 | C₇₄H₁₃₈N₂O₆ | 1151.93 | 10.06 | 24.52 | 3276 | C₉₀H₁₅₃NO₄S₇ | 1537.64 | 9.51 | 36.88 |
| 3077 | C₉₄H₁₆₃N₅O₇S | 1507.43 | 10.19 | 33.88 | 3277 | C₉₄H₁₅₄N₂O₁₀S₅ | 1632.57 | 8.01 | 35.49 |
| 3078 | C₉₂H₁₆₃N₇O₈S₅ | 1655.66 | 9.65 | 32.84 | 3278 | C₉₄H₁₅₅N₉O₇S₇ | 1747.75 | 8.18 | 34.85 |
| 3079 | C₈₃H₁₅₉NO₇S₅ | 1443.48 | 10.08 | 34.53 | 3279 | C₉₁H₁₆₀N₂O₁₁S₆ | 1650.64 | 9.59 | 34.80 |
| 3080 | C₈₁H₁₆₆N₅O₁₁PS₅ | 1577.52 | 8.86 | 29.78 | 3280 | C₈₈H₁₅₃N₃O₁₄S₃ | 1573.38 | 8.83 | 34.94 |
| 3081 | C₁₀₆H₂₀₅N₃O₅S₇ | 1826.24 | 9.06 | 44.87 | 3281 | C₈₇H₁₄₇NO₁₄S₄ | 1559.37 | 9.24 | 35.58 |
| 3082 | C₁₀₁H₁₉₉NO₇S₈ | 1796.18 | 10.22 | 47.81 | 3282 | C₉₀H₁₅₀N₄O₉S | 1464.27 | 9.12 | 33.75 |
| 3083 | C₁₀₂H₂₀₅NO₁₁S₅ | 1782.06 | 10.35 | 44.92 | 3283 | C₉₂H₁₅₈NO₉PS₂ | 1517.37 | 9.60 | 37.32 |
| 3084 | C₁₀₀H₁₉₉NO₂S₈ | 1704.18 | 7.88 | 48.22 | 3284 | C₉₂H₁₅₁NO₆S₄ | 1495.46 | 9.87 | 35.38 |
| 3085 | C₇₁H₁₄₆N₂O₁₃S₅ | 1396.25 | 9.98 | 26.51 | 3285 | C₉₀H₁₄₈N₄O₁₀S₂ | 1510.31 | 7.57 | 32.53 |
| 3086 | C₇₆H₁₄₈N₄O₁₂S₅ | 1470.34 | 8.20 | 25.26 | 3286 | C₉₀H₁₅₅N₅O₈S | 1467.32 | 8.94 | 33.22 |
| 3087 | C₁₁H₂₁₉N₂O₄PS₆ | 1869.32 | 10.16 | 48.79 | 3287 | C₇₇H₁₅₅N₆O₇ | 1319.14 | 9.48 | 28.87 |
| 3088 | C₁₁₁H₂₁₃N₃O₁₀S₅ | 1910.24 | 8.95 | 48.91 | 3288 | C₈₁H₁₅₂N₁₀O₁₀S | 1458.23 | 8.16 | 30.05 |
| 3089 | C₁₁₂H₂₁₅N₃O₁₂S₃ | 1892.14 | 8.95 | 48.47 | 3289 | C₇₇H₁₅₃N₉O₆S₃ | 1397.31 | 7.51 | 28.31 |
| 3090 | C₁₀₅H₂₀₁N₃O₁₃S₄ | 1842.01 | 8.05 | 44.33 | 3290 | C₇₉H₁₅₄N₈O₉ | 1360.15 | 7.29 | 28.84 |
| 3091 | C₉₃H₁₈₅N₃O₈S | 1505.58 | 8.82 | 36.48 | 3291 | C₇₇H₁₄₉NO₃S₈ | 1393.52 | 7.90 | 34.02 |
| 3092 | C₁₀₀H₁₉₉N₄O₁₃P₃S₂ | 1822.75 | 8.21 | 37.37 | 3292 | C₇₉H₁₅₄N₆O₃S₆ | 1428.50 | 9.20 | 32.59 |
| 3093 | C₈₁H₁₄₆N₃O₁₂PS₃ | 1481.22 | 8.46 | 28.15 | 3293 | C₇₆H₁₄₈N₂O₃S₇ | 1362.45 | 9.46 | 34.16 |
| 3094 | C₆₇H₁₂₆N₆O₅S₂ | 1159.90 | 8.50 | 24.22 | 3294 | C₈₂H₁₅₉N₃O₁₀P₂S₃ | 1505.31 | 8.51 | 30.25 |
| 3095 | C₆₈H₁₃₃N₆O₉PS | 1241.88 | 7.67 | 19.74 | 3295 | C₇₈H₁₅₂N₂O₇S₄ | 1358.32 | 8.76 | 32.96 |
| 3096 | C₆₉H₁₃₄N₈O₇S₂ | 1252.00 | 8.41 | 22.96 | 3296 | C₇₉H₁₅₄N₄O₁₀S₄ | 1448.36 | 8.70 | 31.46 |
| 3097 | C₇₀H₁₃₁N₃O₆S₈ | 1367.31 | 7.69 | 26.18 | 3297 | C₇₉H₁₆₂NO₃PS₄ | 1333.38 | 9.86 | 33.98 |
| 3098 | C₇₀H₁₃₅NO₄S₇ | 1279.27 | 10.12 | 30.32 | 3298 | C₈₀H₁₅₄N₂O₁₁S₄ | 1448.35 | 9.26 | 33.61 |
| 3099 | C₇₀H₁₃₂N₄O₁₂S₄ | 1350.08 | 7.46 | 26.35 | 3299 | C₇₆H₁₄₅NO₆S₈ | 1425.48 | 8.81 | 34.83 |
| 3100 | C₆₆H₁₂₅N₃O₁₁S₄ | 1264.98 | 8.20 | 24.53 | 3300 | C₈₂H₁₇₀N₃O₁₁P₃ | 1467.19 | 9.97 | 30.57 |
| 3101 | C₆₇H₁₂₉NO₁₁S₅ | 1285.06 | 9.67 | 26.84 | 3301 | C₇₇H₁₅₂N₆O₁₁S₅ | 1498.39 | 7.59 | 29.12 |
| 3102 | C₆₇H₁₂₃N₃O₆S₇ | 1291.16 | 8.47 | 27.22 | 3302 | C₇₄H₁₅₃N₇O₁₂S₅ | 1493.38 | 8.28 | 28.08 |
| 3103 | C₆₉H₁₃₈NO₁₂P₃S₃ | 1362.96 | 9.26 | 24.34 | 3303 | C₇₆H₁₅₂N₆O₁₂S₆ | 1534.44 | 7.73 | 28.03 |
| 3104 | C₆₃H₁₂₅N₇O₉S₆ | 1317.09 | 7.80 | 22.84 | 3304 | C₇₇H₁₅₀N₂O₁₂S₆ | 1488.41 | 7.93 | 28.36 |
| 3105 | C₆₅H₁₂₈N₄O₁₃S₅ | 1334.05 | 8.14 | 22.01 | 3305 | C₇₇H₁₅₁N₂O₁₀PS₅ | 1456.33 | 8.39 | 26.01 |
| 3106 | C₆₄H₁₂₈N₃O₁₅PS₄ | 1338.95 | 7.40 | 22.22 | 3306 | C₇₉H₁₅₅N₃O₁₀S₃ | 1403.30 | 8.45 | 27.66 |
| 3107 | C₆₃H₁₂₅N₃O₁₀S₇ | 1309.12 | 8.27 | 22.61 | 3307 | C₈₀H₁₅₂N₆O₆ | 1294.13 | 8.06 | 30.31 |
| 3108 | C₆₃H₁₂₆N₂O₇S₄ | 1151.95 | 9.53 | 20.16 | 3308 | C₈₁H₁₅₀N₆O₉S | 1384.18 | 8.07 | 31.20 |
| 3109 | C₆₈H₁₃₁N₇O₈S₂ | 1238.96 | 8.67 | 22.69 | 3309 | C₇₈H₁₅₂N₂O₁₁S₂ | 1358.20 | 8.66 | 29.10 |
| 3110 | C₇₁H₁₃₆N₂O₅S | 1129.94 | 9.90 | 27.52 | 3310 | C₈₂H₁₅₂N₈O₁₀ | 1410.16 | 7.54 | 29.00 |
| 3111 | C₇₁H₁₃₈NO₆PS₂ | 1196.98 | 9.97 | 25.57 | 3311 | C₈₀H₁₅₃N₅O₁₀ | 1345.13 | 8.37 | 30.38 |
| 3112 | C₇₅H₁₄₃N₅O₃S₂ | 1227.12 | 10.12 | 25.60 | 3312 | C₈₂H₁₅₅N₃O₁₁S₂ | 1423.27 | 8.49 | 32.30 |
| 3113 | C₇₂H₁₃₈N₈O₅S | 1228.01 | 9.88 | 25.83 | 3313 | C₉₇H₁₉₇N₂O₆P | 1518.62 | 9.48 | 42.37 |
| 3114 | C₇₆H₁₃₄N₄O₄S₈ | 1424.41 | 8.11 | 29.40 | 3314 | C₁₀₀H₂₀₃NO₆S₃ | 1611.90 | 9.02 | 44.49 |
| 3115 | C₇₁H₁₃₃N₅O₆S₄ | 1281.11 | 7.36 | 26.07 | 3315 | C₁₀₄H₂₀₇N₆O₁₂PS | 1796.86 | 8.77 | 40.97 |
| 3116 | C₇₂H₁₃₂N₄O₅S₃ | 1230.05 | 9.26 | 27.72 | 3316 | C₁₀₀H₁₉₈N₇O₁₂PS | 1753.75 | 7.59 | 39.40 |
| 3117 | C₇₃H₁₃₂N₄O₈S₆ | 1386.24 | 7.72 | 28.90 | 3317 | C₁₀₃H₂₀₄N₉O₉PS | 1775.85 | 7.96 | 38.97 |
| 3118 | C₇₇H₁₅₂N₂O₁₅P₄S | 1502.02 | 8.23 | 24.37 | 3318 | C₁₀₀H₁₉₀N₄O₅S₆ | 1721.00 | 7.09 | 43.77 |
| 3119 | C₇₇H₁₄₈NO₁₁P₃S₃ | 1453.13 | 9.91 | 26.96 | 3319 | C₁₀₃H₁₉₉N₄O₄PS₆ | 1781.08 | 7.23 | 43.88 |
| 3120 | C₇₁H₁₃₇N₅O₈S₅ | 1349.20 | 7.82 | 25.24 | 3320 | C₁₀₀H₁₉₇N₅O₈S₄ | 1725.94 | 9.14 | 42.88 |
| 3121 | C₇₂H₁₃₉N₆O₁₁PS₅ | 1456.21 | 7.67 | 26.24 | 3321 | C₁₀₄H₁₉₈N₄O₉S₆ | 1841.11 | 8.25 | 44.96 |
| 3122 | C₇₃H₁₄₁N₂O₁₂PS₄ | 1398.15 | 8.95 | 24.70 | 3322 | C₉₉H₁₉₂N₂O₁₄S₅ | 1794.92 | 8.35 | 43.66 |
| 3123 | C₇₃H₁₄₀NO₁₃PS₄ | 1399.13 | 9.72 | 26.53 | 3323 | C₁₀₃H₂₀₁N₂O₁₀PS₄ | 1786.96 | 8.46 | 44.74 |
| 3124 | C₆₉H₁₂₇N₃O₁₂S₅ | 1351.08 | 7.54 | 25.78 | 3324 | C₁₀₂H₁₉₅N₇O₈S₆ | 1840.08 | 8.41 | 43.99 |
| 3125 | C₇₂H₁₃₅N₅O₁₂S₃ | 1359.08 | 7.71 | 24.04 | 3325 | C₁₀₂H₂₀₄N₃O₁₆P₃S | 1853.74 | 7.25 | 38.61 |
| 3126 | C₆₈H₁₃₀N₂O₉S₅ | 1280.09 | 9.58 | 25.45 | 3326 | C₁₀₆H₂₁₄N₃O₁₁P₃S₂ | 1863.93 | 8.94 | 40.89 |
| 3127 | C₇₁H₁₃₃NO₁₃S₄ | 1337.08 | 9.69 | 26.71 | 3327 | C₉₇H₁₉₇N₅O₁₀S₃ | 1689.85 | 9.91 | 40.33 |
| 3128 | C₆₉H₁₃₂N₂O₁₀S₆ | 1342.18 | 8.71 | 22.55 | 3328 | C₉₇H₁₉₂N₆O₈S₇ | 1795.06 | 7.50 | 39.79 |
| 3129 | C₇₂H₁₃₃N₃O₁₃S | 1280.92 | 7.06 | 25.48 | 3329 | C₉₇H₁₉₇N₇O₆S₅ | 1717.99 | 9.70 | 39.61 |
| 3130 | C₇₈H₁₄₃NO₉S₃ | 1335.18 | 10.04 | 30.97 | 3330 | C₉₉H₁₉₃N₃O₁₀S₇ | 1810.06 | 8.90 | 42.09 |
| 3131 | C₇₆H₁₃₄N₆O₁₂S | 1356.00 | 8.01 | 25.46 | 3331 | C₉₇H₁₉₀N₄O₉S₃ | 1652.79 | 8.14 | 38.15 |
| 3132 | C₇₂H₁₃₆N₄O₈S₂ | 1250.02 | 9.77 | 23.12 | 3332 | C₉₈H₁₉₃N₃O₁₀S₆ | 1765.99 | 8.13 | 37.98 |
| 3133 | C₄₈H₉₇NO₇S₃ | 896.48 | 9.93 | 17.95 | 3333 | C₉₆H₁₉₄N₄OS₂ | 1484.76 | 9.09 | 41.92 |
| 3134 | C₅₅H₁₀₅N₇O₆S | 992.55 | 8.64 | 14.03 | 3334 | C₁₀₅H₂₀₁N₂O₉PS | 1698.80 | 7.87 | 42.52 |
| 3135 | C₅₂H₉₇N₅O₈ | 920.37 | 7.30 | 14.27 | 3335 | C₁₀₂H₁₉₃N₃O₇S₂ | 1637.80 | 7.73 | 42.08 |
| 3136 | C₅₅H₁₀₈N₄O₆S₃ | 1017.67 | 10.20 | 20.03 | 3336 | C₁₀₁H₁₉₂N₄O₁₂ | 1654.66 | 7.87 | 39.92 |
| 3137 | C₅₁H₁₀₁N₄O₁₀PS₂ | 1025.48 | 9.12 | 18.42 | 3337 | C₉₉H₁₉₁NO₁₅S | 1667.67 | 9.69 | 42.63 |
| 3138 | C₅₅H₁₀₁N₃O₄S₄ | 996.67 | 8.27 | 18.05 | 3338 | C₇₅H₁₄₃N₁₃O₆S | 1355.11 | 8.07 | 25.23 |
| 3139 | C₅₂H₁₀₀N₄O₃S₃ | 925.58 | 8.64 | 18.64 | 3339 | C₇₆H₁₄₅N₉O₅S₄ | 1393.29 | 7.57 | 26.41 |
| 3140 | C₄₈H₉₄N₂O₃S₅ | 907.59 | 7.71 | 18.31 | 3340 | C₇₈H₁₅₁N₉O₉ | 1359.12 | 7.94 | 25.79 |
| 3141 | C₅₆H₁₀₆N₂O₉S₅ | 1111.77 | 10.02 | 23.32 | 3341 | C₇₆H₁₃₉N₃O₃S₈ | 1399.45 | 7.63 | 30.64 |
| 3142 | C₅₅H₉₉N₃O₇S₇ | 1138.83 | 9.16 | 21.40 | 3342 | C₇₆H₁₄₅NO₅S₅ | 1313.30 | 9.99 | 31.73 |
| 3143 | C₅₄H₁₀₇N₂O₁₄P₃S | 1133.43 | 7.27 | 15.46 | 3343 | C₇₂H₁₃₇N₃O₇S₃ | 1253.08 | 9.28 | 29.58 |
| 3144 | C₅₇H₁₁₆N₃O₁₀P₃S | 1128.55 | 9.57 | 15.21 | 3344 | C₆₇H₁₃₈N₂O₅S₅ | 1212.15 | 8.77 | 28.81 |
| 3145 | C₅₈H₁₁₉N₃O₁₄P₄ | 1206.49 | 9.37 | 15.64 | 3345 | C₇₂H₁₃₇NO₄S₉ | 1369.43 | 9.47 | 33.57 |
| 3146 | C₅₀H₁₀₂N₆O₉S₄ | 1059.64 | 8.91 | 14.11 | 3346 | C₇₄H₁₅₂N₃O₁₂P₃S | 1401.02 | 9.40 | 26.10 |
| 3147 | C₄₆H₉₆N₆O₈S₃ | 957.49 | 9.30 | 14.92 | 3347 | C₆₆H₁₃₈N₄O₉S₅ | 1292.15 | 8.80 | 23.19 |
| 3148 | C₄₉H₉₅NO₁₀S₇ | 1082.72 | 8.89 | 17.71 | 3348 | C₇₁H₁₄₂N₂O₁₁S₅ | 1360.22 | 10.10 | 28.27 |
| 3149 | C₅₂H₁₀₆N₂O₈S₄ | 1015.67 | 10.20 | 15.94 | 3349 | C₆₉H₁₃₇N₃O₉S₆ | 1345.23 | 8.87 | 26.02 |
| 3150 | C₅₂H₁₀₂N₆OS₄ | 955.67 | 8.71 | 17.72 | 3350 | C₇₁H₁₃₉N₃O₁₄S₆ | 1451.26 | 7.99 | 27.43 |
| 3151 | C₄₈H₉₉N₅O₅ | 826.35 | 8.78 | 16.86 | 3351 | C₇₁H₁₄₀N₆O₂S₂ | 1174.06 | 7.56 | 26.04 |
| 3152 | C₅₀H₁₀₀N₂O₂S₇ | 985.78 | 8.94 | 20.77 | 3352 | C₇₀H₁₄₃N₅O₂ | 1086.95 | 9.65 | 26.48 |
| 3153 | C₅₀H₉₅NO₉S₂ | 918.43 | 8.74 | 17.41 | 3353 | C₆₉H₁₄₀N₈O₃ | 1129.93 | 7.47 | 24.72 |
| 3154 | C₅₃H₉₇N₃O₁₀ | 936.37 | 8.02 | 17.12 | 3354 | C₇₇H₁₄₁N₃O₈S₂ | 1301.11 | 7.55 | 28.26 |
| 3155 | C₅₂H₉₉N₃O₈S₂ | 958.50 | 9.45 | 17.41 | 3355 | C₇₂H₁₃₅N₃O₁₀S | 1234.94 | 7.57 | 26.66 |
| 3156 | C₅₂H₉₆N₃O₁₁PS | 1002.38 | 8.12 | 16.02 | 3356 | C₇₄H₁₃₈N₂O₁₀S | 1247.98 | 7.46 | 27.52 |
| 3157 | C₅₂H₉₂N₂O₁₅ | 985.31 | 7.52 | 16.32 | 3357 | C₇₀H₁₃₆N₂O₁₀S₂ | 1229.98 | 9.66 | 28.88 |
| 3158 | C₅₃H₁₀₀N₂O₁₃ | 973.38 | 8.88 | 15.78 | 3358 | C₇₅H₁₅₀N₈O₅ | 1244.08 | 9.70 | 26.96 |
| 3159 | C₅₃H₁₀₃N₆O₁₀PS | 1047.47 | 8.46 | 14.49 | 3359 | C₇₄H₁₄₄N₅O₈PS | 1295.03 | 8.00 | 25.17 |
| 3160 | C₆₀H₁₁₀N₂O₇S₂ | 1035.67 | 8.77 | 21.53 | 3360 | C₇₂H₁₄₅N₅O₉S₃ | 1321.16 | 9.35 | 26.34 |
| 3161 | C₆₁H₁₀₉NO₇S₂ | 1032.66 | 9.76 | 22.04 | 3361 | C₇₁H₁₄₄N₈O₇S | 1254.04 | 7.70 | 26.61 |
| 3162 | C₆₈H₁₂₄N₇O₁₁PS | 1278.81 | 9.49 | 18.68 | 3362 | C₇₉H₁₅₁N₉O₇S₂ | 1403.25 | 8.31 | 26.66 |
| 3163 | C₆₈H₁₁₇NO₃S₆ | 1189.05 | 9.07 | 26.17 | 3363 | C₇₂H₁₄₄N₁₀O₇S₃ | 1358.19 | 8.43 | 26.21 |
| 3164 | C₆₅H₁₁₀N₂O₃S₆ | 1159.97 | 9.44 | 24.53 | 3364 | C₇₁H₁₃₅NO₂S₇ | 1259.29 | 9.74 | 30.80 |
| 3165 | C₆₇H₁₁₄N₄O₄S₈ | 1296.15 | 8.05 | 23.10 | 3365 | C₇₃H₁₃₉NO₂S₈ | 1319.40 | 8.81 | 32.79 |
| 3166 | C₆₇H₁₁₉N₃OS₆ | 1175.07 | 9.63 | 26.04 | 3366 | C₇₁H₁₄₄NO₅PS₄ | 1251.15 | 10.03 | 31.16 |
| 3167 | C₆₄H₁₀₇NO₂S₆ | 1114.92 | 8.40 | 23.72 | 3367 | C₆₉H₁₃₉NO₃S₆ | 1223.23 | 9.21 | 31.23 |
| 3168 | C₆₅H₁₁₁N₅O₅S₃ | 1138.81 | 8.82 | 21.52 | 3368 | C₇₅H₁₄₁N₂O₁₁PS₄ | 1406.17 | 8.64 | 28.77 |
| 3169 | C₅₇H₁₀₅N₃O₃S₄ | 1008.72 | 8.60 | 22.28 | 3369 | C₇₄H₁₅₃N₄O₁₁P₃S₂ | 1432.10 | 8.31 | 25.59 |
| 3170 | C₆₄H₁₁₃N₅O₅S₇ | 1257.06 | 9.42 | 23.90 | 3370 | C₇₇H₁₅₀N₃O₁₄P₃S | 1467.04 | 7.45 | 24.68 |
| 3171 | C₆₄H₁₁₈NO₁₂P₃S₄ | 1314.80 | 8.70 | 19.42 | 3371 | C₇₁H₁₄₆N₈O₈S₄ | 1368.24 | 8.39 | 25.23 |
| 3172 | C₅₇H₁₀₇N₅O₈S₄ | 1118.75 | 9.82 | 18.80 | 3372 | C₆₈H₁₃₅NO₁₅S₅ | 1367.12 | 8.51 | 26.84 |
| 3173 | C₆₀H₁₁₃N₅O₈S₄ | 1160.83 | 9.93 | 19.66 | 3373 | C₇₁H₁₄₂N₆O₁₅S₃ | 1416.12 | 7.02 | 26.05 |
| 3174 | C₆₃H₁₁₈N₆O₉S₄ | 1231.91 | 9.47 | 17.86 | 3374 | C₆₈H₁₃₅NO₁₀S₃ | 1223.00 | 8.08 | 24.60 |
| 3175 | C₆₂H₁₁₁N₃O₁₃S₄ | 1234.82 | 8.51 | 17.77 | 3375 | C₇₃H₁₄₄N₃O₁₀PS₅ | 1415.24 | 8.25 | 22.65 |
| 3176 | C₆₃H₁₁₄NO₁₃PS₅ | 1284.87 | 9.07 | 21.47 | 3376 | C₇₂H₁₃₇N₃O₁₁S₆ | 1413.26 | 7.91 | 23.79 |
| 3177 | C₆₂H₁₁₇N₂O₁₅PS₄ | 1289.83 | 9.18 | 18.80 | 3377 | C₇₀H₁₄₂N₂O₁₀S₄ | 1300.15 | 9.93 | 24.88 |
| 3178 | C₆₂H₁₁₁N₃O₁₄S₆ | 1314.94 | 8.47 | 19.91 | 3378 | C₆₈H₁₃₆N₆O₇S₂ | 1213.99 | 7.07 | 26.69 |
| 3179 | C₆₃H₁₁₅N₂O₁₇PS₅ | 1363.88 | 8.72 | 20.79 | 3379 | C₇₂H₁₄₂N₆O₄ | 1155.97 | 8.79 | 26.00 |
| 3180 | C₅₈H₁₀₇N₃O₈S₃ | 1070.69 | 9.95 | 16.48 | 3380 | C₇₅H₁₄₆N₃O₇PS | 1265.04 | 9.30 | 28.09 |
| 3181 | C₆₀H₁₀₆N₂O₈S₃ | 1079.69 | 9.71 | 22.06 | 3381 | C₇₄H₁₃₉N₃O₆S₃ | 1263.12 | 8.03 | 27.70 |
| 3182 | C₆₄H₁₁₂N₄O₅S₂ | 1081.74 | 9.13 | 19.93 | 3382 | C₇₅H₁₄₀N₂O₇ | 1181.95 | 8.49 | 27.43 |
| 3183 | C₇₂H₁₂₉N₇O₆S | 1220.93 | 9.47 | 19.86 | 3383 | C₇₄H₁₃₇N₃O₇S₄ | 1309.16 | 8.31 | 28.35 |
| 3184 | C₇₄H₁₃₁N₇O₆S₂ | 1279.03 | 8.74 | 20.89 | 3384 | C₇₄H₁₃₇N₃O₁₄S₂ | 1357.04 | 8.49 | 25.65 |
| 3185 | C₆₉H₁₂₃N₅O₈S | 1182.83 | 9.47 | 23.43 | 3385 | C₁₀₇H₂₀₉N₅O₁₀S | 1757.93 | 9.69 | 46.30 |
| 3186 | C₇₁H₁₂₆N₆O₁₀S₃ | 1320.00 | 8.10 | 21.56 | 3386 | C₁₀₅H₂₀₈N₆O₁₂S | 1778.91 | 9.08 | 44.20 |
| 3187 | C₇₀H₁₂₉N₄O₁₁PS | 1265.85 | 8.87 | 23.05 | 3387 | C₁₀₇H₂₀₉N₇O₁₀S₂ | 1818.01 | 7.78 | 44.86 |
| 3188 | C₇₁H₁₂₅N₈O₆S | 1221.91 | 9.84 | 21.09 | 3388 | C₁₀₅H₂₀₉N₇O₇S | 1713.93 | 9.76 | 43.29 |
| 3189 | C₆₅H₁₂₂N₈O₉S₂ | 1223.86 | 7.01 | 20.16 | 3389 | C₁₁₀H₂₁₄N₃O₇PS₅ | 1882.21 | 8.72 | 46.15 |
| 3190 | C₇₂H₁₂₈N₃O₇PS₄ | 1307.04 | 8.58 | 21.87 | 3390 | C₁₀₄H₂₀₃NO₄S₇ | 1756.19 | 7.73 | 48.33 |
| 3191 | C₆₉H₁₂₀N₂O₂S₇ | 1234.15 | 9.59 | 25.76 | 3391 | C₁₁₀H₂₁₄NO₈PS₄ | 1838.13 | 9.76 | 48.15 |
| 3192 | C₇₂H₁₂₈N₂O₃S₇ | 1294.25 | 9.98 | 27.71 | 3392 | C₁₀₅H₂₀₅NO₈S₅ | 1770.09 | 8.22 | 48.48 |
| 3193 | C₇₁H₁₂₀N₂O₇S₃ | 1209.93 | 8.72 | 23.50 | 3393 | C₁₀₆H₂₀₇N₃O₈S₃ | 1748.01 | 8.86 | 45.55 |
| 3194 | C₆₉H₁₁₉NO₇S₇ | 1299.13 | 9.31 | 26.45 | 3394 | C₁₀₂H₂₀₂N₄O₆S₄ | 1709.00 | 8.10 | 46.12 |
| 3195 | C₆₆H₁₂₀N₂O₅S₄ | 1149.93 | 7.54 | 24.03 | 3395 | C₁₁₁H₂₂₁N₂O₁₃P₃S₂ | 1949.03 | 7.97 | 44.55 |
| 3196 | C₇₄H₁₃₇N₂O₁₂P₃S₃ | 1436.01 | 8.52 | 22.06 | 3396 | C₁₀₀H₁₉₉NO₁₁S₇ | 1816.11 | 7.43 | 44.88 |
| 3197 | C₇₄H₁₃₆N₅O₁₂P₃S₂ | 1444.97 | 7.61 | 19.31 | 3397 | C₁₀₅H₂₁₂N₃O₁₃PS₃ | 1852.01 | 8.82 | 42.85 |
| 3198 | C₆₆H₁₂₃N₅O₆S₅ | 1243.04 | 9.94 | 20.38 | 3398 | C₁₀₂H₂₀₃N₅O₁₂S₅ | 1852.07 | 7.51 | 45.60 |
| 3199 | C₆₈H₁₂₃N₃O₁₂S₄ | 1302.98 | 8.93 | 19.98 | 3399 | C₁₀₂H₂₁₀N₄OS | 1540.89 | 10.13 | 45.50 |
| 3200 | C₆₇H₁₁₇N₃O₉S₅ | 1268.98 | 7.82 | 18.53 | 3400 | C₁₀₆H₂₁₀N₄O₅S₇ | 1845.29 | 8.50 | 47.42 |
| 3401 | C₈₉H₁₇₄NO₇PS₄ | 1529.58 | 8.39 | 38.72 | 3415 | C₈₇H₁₇₂N₄O₅S | 1386.42 | 8.88 | 34.57 |
| 3402 | C₉₀H₁₇₁N₃O₇S₃ | 1503.55 | 7.71 | 37.84 | 3416 | C₇₁H₁₃₅N₅O₉S₃ | 1299.07 | 9.96 | 27.23 |
| 3403 | C₈₉H₁₇₄N₂O₈S₅ | 1560.68 | 9.46 | 37.71 | 3417 | C₇₈H₁₄₉N₄O₁₁P | 1350.04 | 9.88 | 28.11 |
| 3404 | C₉₂H₁₇₇NO₆S₈ | 1649.91 | 9.21 | 43.98 | 3418 | C₇₁H₁₃₁NO₃S₉ | 1335.37 | 9.68 | 29.97 |
| 3405 | C₉₁H₁₇₇NO₈S₇ | 1637.84 | 8.69 | 40.23 | 3419 | C₇₅H₁₃₇N₃O₅S₄ | 1289.18 | 9.96 | 29.92 |
| 3406 | C₈₈H₁₇₁NO₇S₇ | 1579.76 | 9.60 | 39.68 | 3420 | C₇₁H₁₃₂N₆O₁₀S₆ | 1422.23 | 7.13 | 23.90 |
| 3407 | C₉₄H₁₉₃N₂O₁₁P₃S₄ | 1748.74 | 8.86 | 36.61 | 3421 | C₆₉H₁₃₃N₅O₈S₅ | 1321.15 | 8.74 | 23.45 |
| 3408 | C₉₄H₁₉₁N₂O₁₁P₃S₂ | 1682.61 | 9.39 | 36.62 | 3422 | C₇₀H₁₃₉N₄O₁₄PS₄ | 1420.11 | 7.55 | 24.35 |
| 3409 | C₉₇H₁₉₆N₃O₁₅P₃S | 1769.62 | 9.74 | 34.98 | 3423 | C₇₀H₁₃₇N₃O₈ | 1148.88 | 9.36 | 26.51 |
| 3410 | C₈₈H₁₈₀N₄O₇S₅ | 1566.73 | 9.79 | 37.89 | 3424 | C₆₈H₁₃₄N₂O₇ | 1091.83 | 9.15 | 26.82 |
| 3411 | C₈₉H₁₈₂NO₁₅PS₄ | 1665.64 | 9.36 | 36.96 | 3425 | C₆₈H₁₃₄N₂O₇ | 1091.83 | 9.15 | 26.82 |
| 3412 | C₈₈H₁₇₆N₂O₁₄S₄ | 1614.62 | 7.11 | 32.69 | 3426 | C₇₂H₁₄₂N₂O₇ | 1147.94 | 10.05 | 27.76 |
| 3413 | C₈₈H₁₇₄N₂O₃S₇ | 1532.79 | 8.91 | 42.54 | 3427 | C₇₀H₁₃₇N₃O₈ | 1148.88 | 9.36 | 26.51 |
| 3414 | C₉₁H₁₇₃N₅O₆S₃ | 1529.59 | 7.96 | 34.92 | 3428 | C₇₀H₁₃₈N₂O₇ | 1119.88 | 9.84 | 26.70 |

The above properties of the exemplary compounds of the present invention indicate that they can serve as excellent surfactants, and are particularly suitable for preparing lipid nanoparticles for use as drug delivery vehicles.

### Example 3: Preparation of Nanoparticles

Luciferase mRNA was diluted in a 10-100 mM sodium citrate buffer solution at pH 4.0, with the concentration of the mRNA solution being 135 µg/ml. A mixed lipid ethanol solution was prepared at a molar ratio of ionizable cationic lipid compound (each exemplary compound) : DSPC: cholesterol : 2-[(polyethylene glycol)-2000]-N,N-ditetradecylacetamide (ALC-0159) of 50:10:38.5:1.5. The mRNA solution and the mixed lipid solution were mixed at a volume ratio of 3:1 in a nanodrug preparation device, followed by ultrafiltration to obtain luciferase mRNA lipid nanoparticles.

### Example 4: Determination of Apparent pKa

The apparent pKa of lipid nanoparticles is correlated with the efficacy of lipid nanoparticles in delivering nucleic acids (see Jayaraman et al., Angewandte Chemie, International Edition (2012), 51(34), 8529-8533; Semple et al., Nature Biotechnology 28, 172-176 (2010)). The apparent pKa of nanoparticles prepared from different lipid compounds was determined using fluorescence based on 2-(p-toluidino)-6-naphthalenesulfonic acid (TNS). The fluorescent probe has no fluorescence in water but slightly emits fluorescence when bound to lipid membranes. Blank lipid nanoparticles were prepared at a molar ratio of ionizable cationic lipid compound (each exemplary compound) : DSPC : cholesterol : 2-[(polyethylene glycol)-2000]-N,N-ditetradecylacetamide (ALC-0159) of 50:10:38.5:1.5. They were diluted in different pH buffer solutions having a pH ranging from 2 to 11. An aqueous TNS solution was added and mixed. Then, the mixtures were added to a 96-well plate (black bottom), respectively. Detection was performed with excitation wavelength at 325 nm and the emission wavelength at 435 nm, with top detection. Measurement was performed 10 times for each well, and the fluorescence intensity was recorded. A pH-fluorescence intensity curve was plotted to determine the apparent pKa. The apparent pKa data of lipid nanoparticles prepared from each exemplary compound are shown in Table 2.

### Example 5: Determination of Particle Size, Zeta Potential and Encapsulation Rate of Nanoparticles

1. Determination of particle size and polydispersity index (PDI): The average particle size and PDI of the nanoparticle sample solutions in the examples were determined by dynamic light scattering using Malvern ZetaSizer Nano ZS90. The measurement angle was 90°, the refractive index of the dispersant was 1.330, and the test temperature was 25°C.
2. Zeta potential: The Zeta potential of the nanoparticle sample solutions in the examples was determined by electrophoretic light scattering (ELS) using Malvern ZetaSizer Nano ZS90. Refractive index of the dispersant: 1.330, Test temperature: 25°C.
3. Encapsulation Rate: The Encapsulation Rate of luciferase mRNA in lipid nanoparticles was determined using a Quant-it Ribogreen RNA quantitation kit (ThermoFisher Scientific, UK) in accordance with the manufacturer's instructions.

The average particle size, PDI, Zeta potential and Encapsulation Rate data of lipid nanoparticles prepared from each exemplary compound are shown in Table 2.

**Table 2 Summary of Apparent pKa, Particle Size, PDI, Zeta Potential and Encapsulation Rate of Lipid Nanoparticles Prepared from the Compounds**

| **Compound No.** | **Apparent pKa** | **Particle Size (nm)** | **Polydispersity Index (PDI)** | **Zeta Potential (mV)** | **Encapsulation Rate (%)** |
|---|---|---|---|---|---|
| 1002 | / | 76.23 | 0.111 | -13.3 | / |
| 1003 | / | 62.59 | 0.127 | -3.64 | 95.4% |
| 1005 | 6.10 | 55.44 | 0.076 | -0.105 | 91.7% |
| 1007 | 5.13 | 85.78 | 0.068 | -4.98 | 95.9% |
| 1008 | 6.14 | 73.49 | 0.019 | -6.06 | 95.1% |
| 1009 | 5.23 | 67.63 | 0.126 | 2.60 | 92.7% |
| 1011 | 6.30 | 60.38 | 0.193 | 2.31 | 91.8% |
| 1012 | 6.05 | 80.07 | 0.051 | 0.811 | 87.4% |
| 1013 | 5.75 | 82.41 | 0.123 | -2.23 | 90.7% |
| 1014 | / | 85.4 | 0.111 | 3.06 | 70.3% |
| 1015 | / | 102.3 | 0.136 | 4.27 | 80.3% |
| 1025 | / | 77.74 | 0.072 | 2.84 | 64.5% |
| 1029 | 5.41 | 77.94 | 0.057 | -3.71 | 90.6% |
| 1059 | 5.18 | 72.71 | 0.067 | -10.0 | 93.0% |
| 1111 | / | 110.3 | 0.103 | -0.902 | 83.2% |
| 1113 | 5.91 | 58.00 | 0.082 | 4.57 | 100.3% |
| 1118 | 5.11 | 108.1 | 0.051 | -3.96 | 95.9% |
| 1120 | 6.14 | 98.18 | 0.049 | -6.05 | 97.2% |
| 1132 | / | 109.2 | 0.127 | 0.0908 | 90.1% |
| 1139 | / | 74.13 | 0.064 | -7.8 | 91.1% |
| 1140 | / | 63.21 | 0.127 | 4.39 | 92.4% |
| 3423 | / | 87.5 | 0.086 | -18.6 | 96.0% |
| 3424 | / | 94.71 | 0.115 | -0.327 | 88.9% |
| 3425 | / | 82.37 | 0.021 | -21.2 | 89.4% |
| 3426 | / | 85.51 | 0.104 | 2.23 | 95.5% |
| 3427 | / | 70.81 | 0.126 | 3.8 | 96.1% |
| 3428 | / | 83.84 | 0.046 | -17.8 | 96.6% |

### Example 6: In Vitro Cell Activity Evaluation of Lipid Nanoparticles

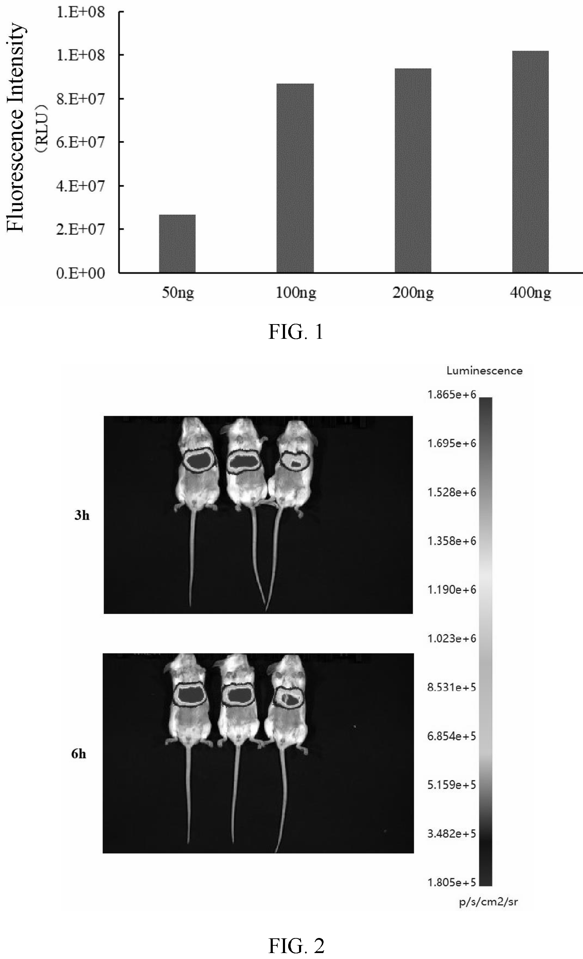

HEK-293T cells were used to evaluate the *in vitro* transfection efficiency of lipid nanoparticle compositions prepared from each exemplary compound. HEK293T cells were routinely cultured in DMEM + 10% FBS medium to ensure the cells were in the logarithmic growth phase. One day before transfection, the cells were seeded into a 96-well culture plate at an appropriate cell density and grown overnight. At the time of transfection, the cell confluency needed to reach 70-90%. The lipid nanoparticle compositions prepared from each exemplary compound were diluted into 4 different dose concentrations with DMEM and added to the 96-well cell culture plate, resulting in final concentrations of 400 ng, 200 ng, 100 ng and 50 ng per well, respectively. luciferase plasmid transfected using Lipofectamine 2000 was used as the positive control (PC). After incubation for 24 h in a 5% CO₂ incubator at 37°C, the substrate was added to the wells, and the luciferase activity was determined using a microplate reader. The results are shown in Table 3. It can be seen that the fluorescence intensity of luciferase expressed by the luciferase mRNA lipid nanoparticles prepared using the exemplary compounds in HEK 293T cells (dose: 100 ng, 96-well plate) was >10³ RLU, indicating that the luciferase mRNA lipid nanoparticles prepared using the ionizable cationic lipid compounds of the present invention exhibited biological activity *in vitro* in cells and had low cytotoxicity. The luciferase activity of the luciferase mRNA lipid nanoparticles prepared from Compound 1005 in HEK293T cells is shown in Fig. 1. It can be seen that at different doses (50-400 ng), the fluorescence intensity of luciferase expressed by the prepared luciferase mRNA lipid nanoparticles in HEK 293T cells was >10⁷ RLU, and the fluorescence intensity increased with the increase of dose, indicating that the expression level of luciferase increased correspondingly with the increase of dose, and the prepared lipid nanoparticles had good mRNA delivery capability and safety.

### Example 7: in Vivo Activity Evaluation of Lipid Nanoparticles

6-8 week-old female Balb/c mice were used to evaluate the *in vivo* transfection efficiency and safety of lipid nanoparticle compositions prepared from each exemplary compound. The lipid nanoparticles prepared from each exemplary compound were administered by tail vein injection at a single dose of 0.3 mpk. At specific time points after administration (e.g., 3 h, 6 h), live imaging of the animals was performed using a PerkinElmer small animal imaging system to determine the bioluminescent signal. The results are shown in Table 3. The whole-body live imaging of mice at 3 h/6 h after intravenous administration of luciferase mRNA lipid nanoparticles prepared from Compound 1008 is shown in Fig. 2. It is evident from Table 3 that 6 h after the administration of lipid nanoparticles prepared from each exemplary compound, the average whole-body fluorescence signal of the mice was greater than 10⁴ p/s/cm²/sr, indicating that the luciferase mRNA lipid nanoparticles containing the exemplary compounds induced an effective *in vivo* expression of luciferase. Meanwhile, the mice exhibited no toxic reactions such as abnormal activity after the administration of lipid nanoparticles prepared from each exemplary compound. This indicated that the luciferase mRNA lipid nanoparticles prepared using the ionizable cationic lipid compounds of the present invention exhibited biological activity *in vivo* in animals and had low toxicity.

**Table 3 Summary of in vitro/in vivo Activity Evaluation of Lipid Nanoparticles Prepared from Compounds**

| **Compound No.** | **HEK 293T Cell Luciferase Activity (100 ng dose; RLU)** | **Mouse Whole-Body Average Fluorescence Signal (p/s/cm²/sr)** |
|---|---|---|
| 1005 | 1.01×10⁸ | 1.21×10⁶ |
| 1007 | 1.60×10⁵ | 1.34×10⁶ |
| 1008 | 1.86×10⁶ | 7.66×10⁵ |
| 1009 | 6.15×10⁷ | 5.99×10⁴ |
| 1011 | 8.22×10⁷ | 1.29×10⁵ |
| 1012 | 6.61×10⁵ | 2.02×10⁵ |
| 1013 | 9.87×10⁴ | 1.69×10⁴ |
| 1014 | 2.86×10⁶ | 3.55×10⁴ |
| 1015 | 6.27×10⁷ | 1.43×10⁴ |
| 1025 | / | 4.20×10⁵ |
| 1029 | 8.23×10⁴ | 3.27×10⁴ |
| 1059 | 1.32×10⁴ | 4.94×10⁵ |
| 1111 | 3.29×10⁴ | 3.10×10⁵ |
| 1113 | 3.43×10⁴ | 3.56×10⁷ |
| 1118 | 1.48×10⁴ | 1.77×10⁸ |
| 1120 | 2.35×10⁴ | 3.52×10⁸ |
| 1132 | 7.28×10⁴ | 2.45×10⁵ |
| 1139 | 4.23×10⁵ | 3.87×10⁵ |
| 1140 | 2.13×10⁴ | 6.21×10⁴ |
| 3423 | 3.97×10⁵ | 5.53×10⁵ |
| 3424 | 1.81×10⁴ | 9.34×10⁴ |
| 3425 | 6.86×10⁶ | 1.56×10⁷ |
| 3426 | 3.01×10⁵ | 5.67×10⁶ |
| 3427 | 5.18×10⁵ | 8.24×10⁶ |
| 3428 | 3.23×10⁵ | 2.04×10⁵ |

The solution of the present invention is not limited to the technical means disclosed in the above technical means, but also includes technical solutions composed of any combination of the above technical features. The above are detailed embodiments of the present invention. It should be noted that for those of ordinary skill in the art, several improvements and modifications can be made without departing from the principle of the present invention, and these improvements and modifications are also regarded as the protection scope of the present invention.

## Claims

1. An ionizable cationic lipid compound, wherein the ionizable cationic lipid compound is a compound of Formula (1), or a salt, a stereoisomer or a tautomer thereof: 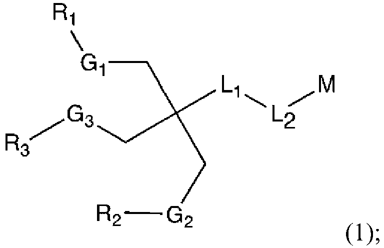
wherein R₁, R₂ and R₃ are each independently H, a C₅₋₄₀ linear or branched alkyl group, a C₅₋₄₀ linear or branched alkenyl group, a C₅₋₄₀ linear or branched alkynyl group, a 3-6 membered saturated or partially unsaturated cyclic hydrocarbon group containing 1-3 side chains, or a 6-10 membered aromatic group containing 1-3 side chains; wherein the side chains are each independently selected from a C₁₀₋₃₀ linear or branched alkyl group, a C₁₀₋₃₀ linear or branched alkenyl group, and a C₁₀₋₃₀ linear or branched alkynyl group; with the proviso that at most one of R₁, R₂ and R₃ is H;
M is selected from -NR₄R₅, a saturated or partially unsaturated 3-6 membered heterocyclic group containing at least one nitrogen atom, and a 6-10 membered heteroaryl group containing at least one nitrogen atom, wherein the heterocyclic group and the heteroaryl group are each unsubstituted or substituted with one or more of -OH, carboxyl group, amino group, oxo group, or halogen;
R₄ and R₅ are each independently H, a C₁₋₆ linear or branched alkyl group, a C₂₋₆ linear or branched alkenyl group, or a C₂₋₆ linear or branched alkynyl group, wherein the C₁₋₆ linear or branched alkyl group, the C₂₋₆ linear or branched alkenyl group or the C₂₋₆ linear or branched alkynyl group is unsubstituted or substituted with one or more of -OH, carboxyl group, amino group, amido group, amidino group, guanidino group and halogen;
G₁, G₂ and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)-, or -OP(=O)(OR₆)O-; wherein R₆ and R₁₃ are each independently selected from H, hydroxyl group, a C₁₋₃₀ linear or branched alkyl or cycloalkyl group, and a C₂₋₃₀ linear or branched alkenyl group;
L₁ is selected from -X₁- or -(CR₇R₈)ₘ-X₁-, wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)-, or -OP(=O)(OR₁₄)O-; m is an integer of 2-6; R₇ and R₈ are each independently H, hydroxyl group, halogen, a C₁₋₆ linear or branched alkyl or cycloalkyl group, or a C₂₋₆ linear or branched alkenyl group; R₁₄ and R₁₅ are each independently selected from H, a C₁₋₃₀ linear or branched alkyl or cycloalkyl group, and a C₂₋₃₀ linear or branched alkenyl group;
L₂ is -(CR₉R₁₀)ₙ- or -(CR₉R₁₀)ₙ-X₂-(CR₁₁R₁₂)ₖ-, wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, NR₁₆-S(=O)₂-, -P(=O)(OR₁₆)O-, -OP(=O)(OR₁₆)-, or -OP(=O)(OR₁₆)O-; n is an integer of 1-6; k is an integer of 1-6; R₉, R₁₀, R₁₁ and R₁₂ are each independently H, hydroxyl group, halogen, a C₁₋₆ linear or branched alkyl or cycloalkyl group, or a C₂₋₆ linear or branched alkenyl group; R₁₆ and R₁₇ are each independently selected from H, a C₁₋₃₀ linear or branched alkyl or cycloalkyl group, and a C₂₋₃₀ linear or branched alkenyl group;
wherein the alkyl, cycloalkyl and alkenyl groups in R₄ to R₁₇ are unsubstituted or substituted with one or more groups selected from hydroxyl group, mercapto group, amino group, substituted amino group, and halogen; and
wherein the salt does not include a quaternary ammonium salt.

2. The ionizable cationic lipid compound according to claim 1, wherein R₁, R₂ and R₃ are each independently the following group: 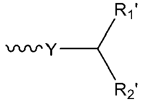 wherein Y is absent, or is a C₁₋₃₀ linear or branched alkyl or cycloalkyl group, a C₂₋₂₀ linear or branched alkenyl group, or a C₂₋₂₀ linear or branched alkynyl group; R₁' and R₂' are each independently H, a C₁₋₃₀ linear or branched alkyl group, a C₂₋₃₀ linear or branched alkenyl group, or a C₂₋₃₀ linear or branched alkynyl group; and the total length of the carbon chains of Y, R₁' and R₂' is 8-40.

3. The ionizable cationic lipid compound according to claim 1, wherein R₁, R₂ and R₃ are each independently selected from the following groups: 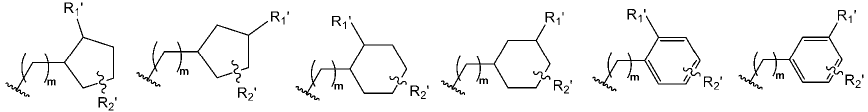 wherein R₁' and R₂' are each independently H, a C₁₋₃₀ linear or branched alkyl group, a C₂₋₃₀ linear or branched alkenyl group, or a C₂₋₃₀ linear or branched alkynyl group, and the total length of the carbon chains of R₁' and R₂' is 8-30.

4. The ionizable cationic lipid compound according to claim 1, wherein R₁, R₂ and R₃ are each independently any one of the following groups:

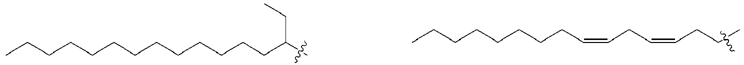

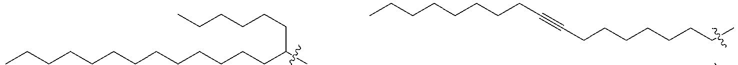

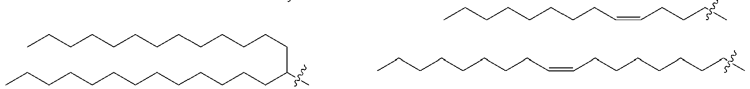

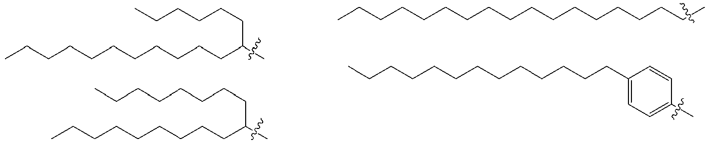

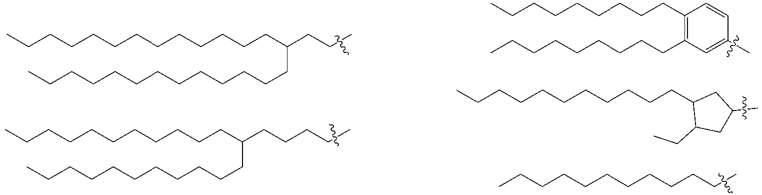

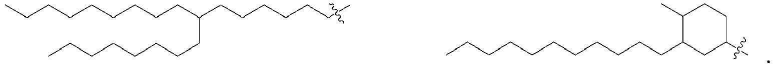

5. The ionizable cationic lipid compound according to claim 1, wherein G₁, G₂ and G₃ are each independently -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)-, or -OP(=O)(OR₆)O-.

6. The ionizable cationic lipid compound according to claim 1, wherein L₁ is selected from -(CR₇R₈)ₘ-X₁-, wherein X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)-, or -OP(=O)(OR₁₄)O-.

7. The ionizable cationic lipid compound according to claim 1, wherein L₂ is -(CR₉R₁₀)ₙ-X₂-(CR₁₁R₁₂)ₖ-, wherein X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)-, or -OP(=O)(OR₁₆)O-.

8. The ionizable cationic lipid compound according to claim 1, wherein M is selected from the following structures: 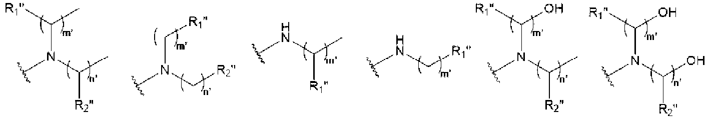 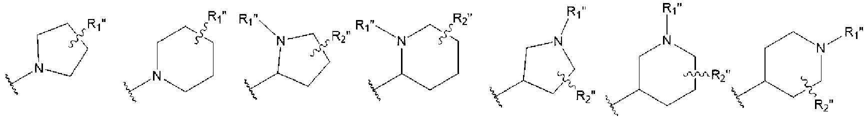 wherein m' and n' are each independently an integer of 0-6; R₁" and R₂" are each independently H, a C₁₋₆ alkyl group, a C₂₋₆ alkenyl group, guanidino group, amidino group, amido group, aliphatic amine group, or a 3-10 membered nitrogen-containing heterocycle; wherein the nitrogen-containing heterocycle is selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine and pyrimidine, and tautomers thereof, which are unsubstituted or optionally substituted with one or more organic groups selected from hydroxyl group, mercapto group, amino group, substituted amino group, halogen, C₁₋₆ alkyl group, C₂₋₆ alkenyl group, C₂₋₆ alkynyl group and C₆₋₁₄ aryl group.

9. The ionizable cationic lipid compound according to claim 1, wherein the compound of Formula (1) is selected from compounds represented by Formula (1A):

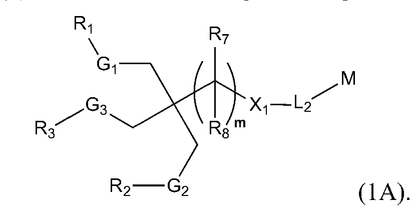

10. The ionizable cationic lipid compound according to claim 1, wherein the compound of Formula (1) is selected from compounds represented by Formula (1B):

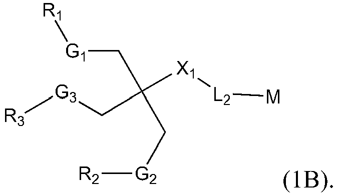

11. The ionizable cationic lipid compound according to claim 1, wherein the compound of Formula (1) is selected from compounds represented by Formula (1C):

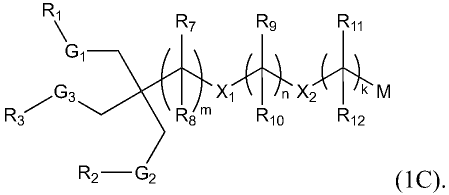

12. The ionizable cationic lipid compound according to claim 10, wherein the compound of Formula (1) is selected from compounds represented by Formula (1D):

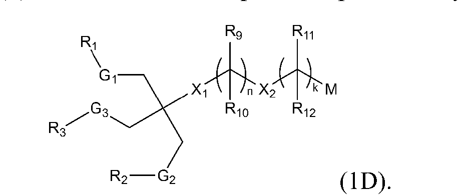

13. The ionizable cationic lipid compound according to claim 12, wherein the compound of Formula (1) is selected from compounds represented by Formula (1E):

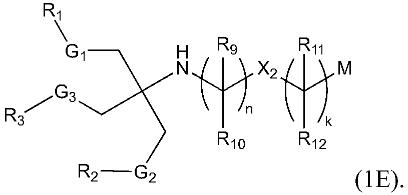

14. The ionizable cationic lipid compound according to claim 13, wherein the compound of Formula (1) is selected from compounds represented by Formula (1F):

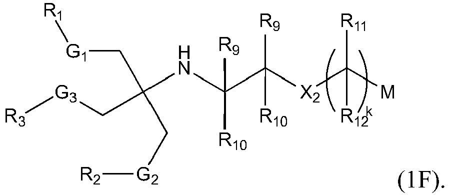

15. The ionizable cationic lipid compound according to claim 14, wherein the compound of Formula (1) is selected from compounds represented by Formula (1G):

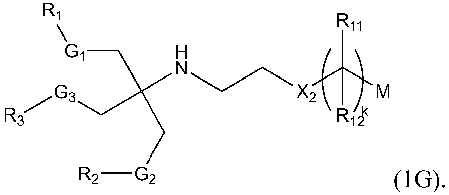

16. The ionizable cationic lipid compound according to claim 14, wherein the compound of Formula (1) is selected from compounds represented by Formula (1H):

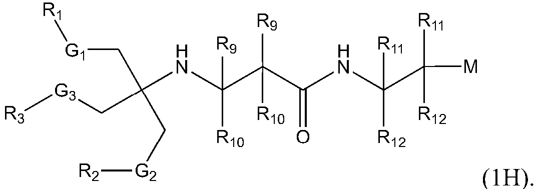

17. The ionizable cationic lipid compound according to claim 1, wherein the ionizable cationic lipid compound is a compound represented by Formula (11):

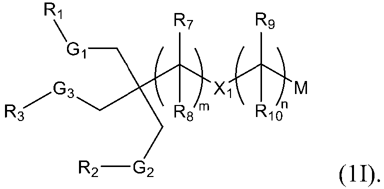

18. The ionizable cationic lipid compound according to claim 10, wherein the compound of Formula (1) is selected from compounds represented by Formula (1J):

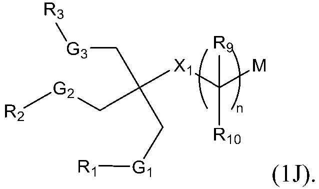

19. The ionizable cationic lipid compound according to claim 17, wherein the compound of Formula (1) is selected from compounds represented by Formula (1K):

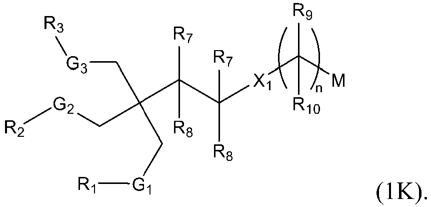

20. The ionizable cationic lipid compound according to claim 2, wherein Y is absent, and the compound of Formula (1) is selected from compounds represented by Formula (1L): 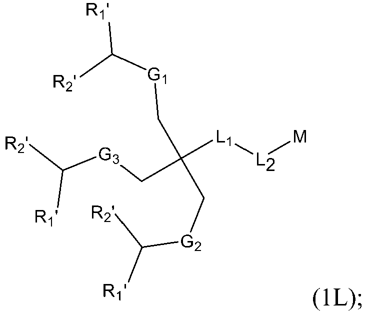 wherein R₁' and R₂' are each independently selected from H, a C₁₋₃₀ linear or branched alkyl group, a C₂₋₃₀ linear or branched alkenyl group, or a C₂₋₃₀ linear or branched alkynyl group, and the total length of the carbon chains of R₁' and R₂' is 8-40.

21. The ionizable cationic lipid compound according to claim 2, wherein the compound of Formula (1) is selected from compounds represented by Formula (1M):

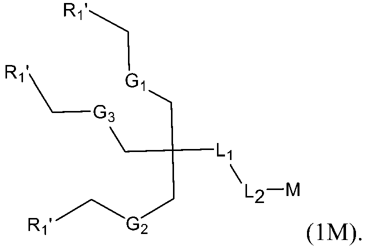

22. The ionizable cationic lipid compound according to claim 1, wherein the compound of Formula (1) is selected from:

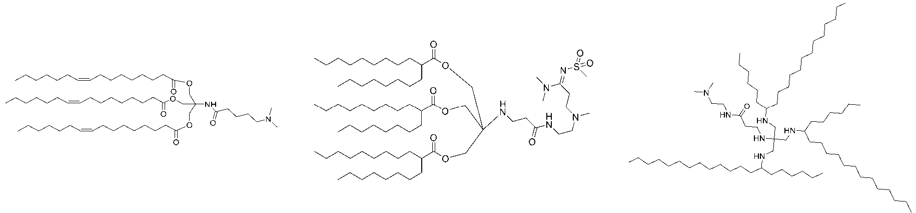

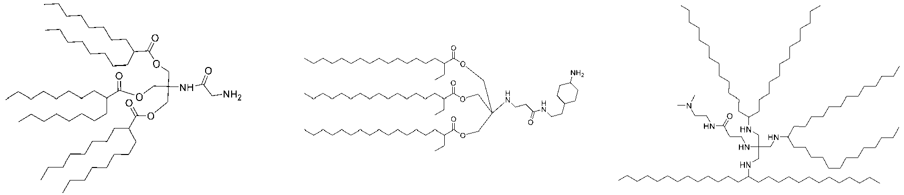

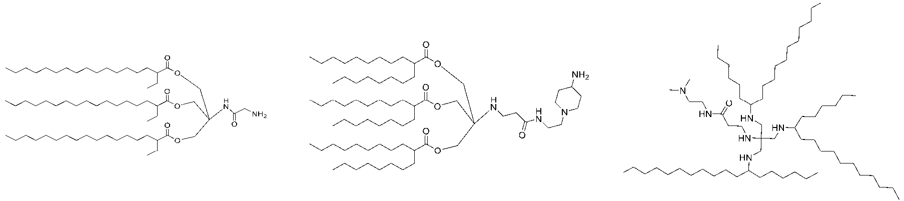

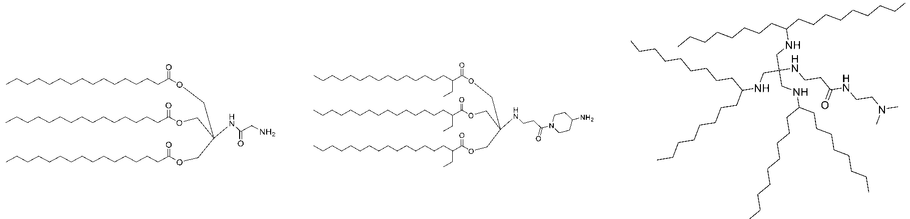

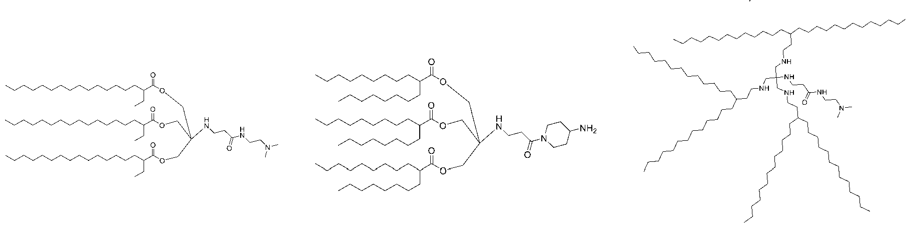

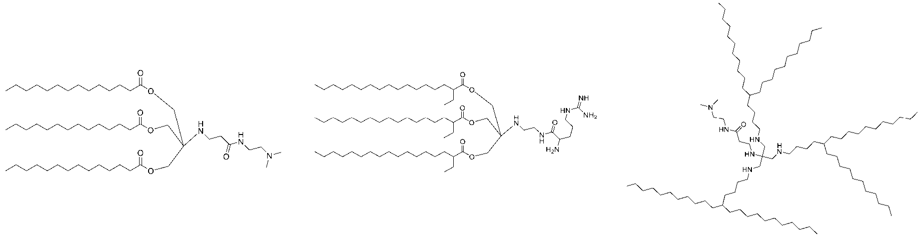

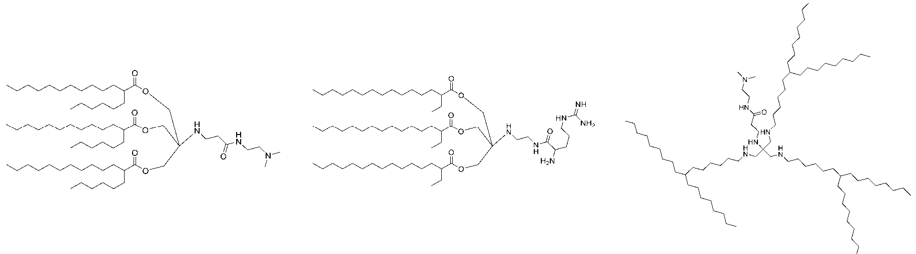

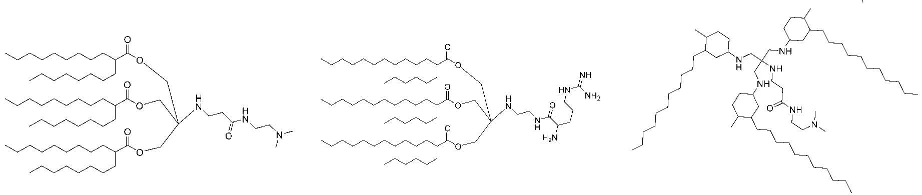

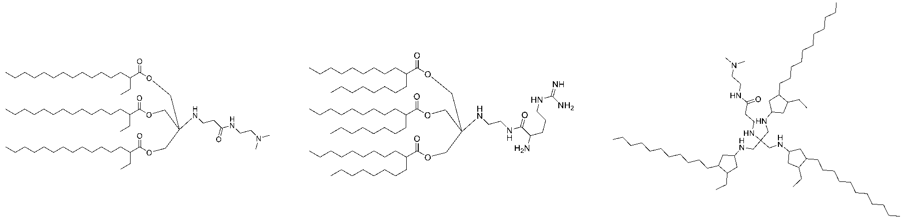

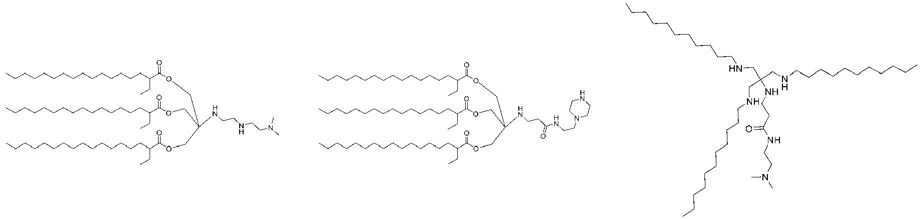

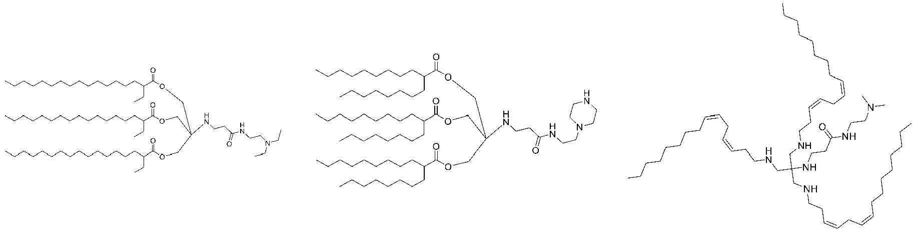

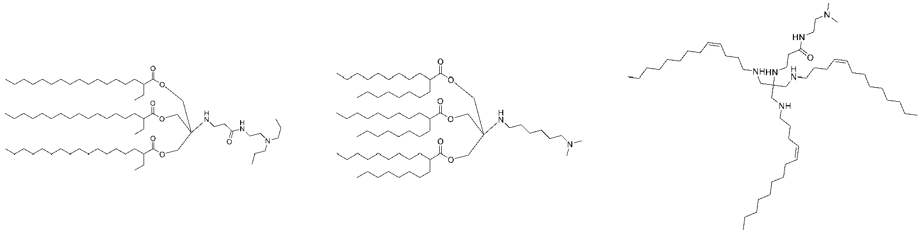

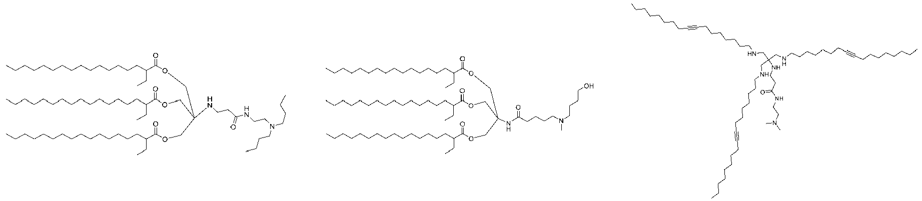

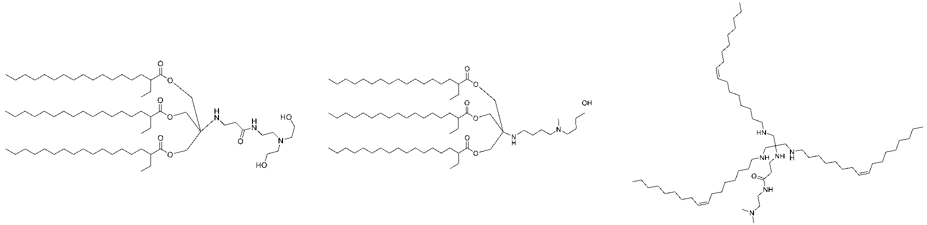

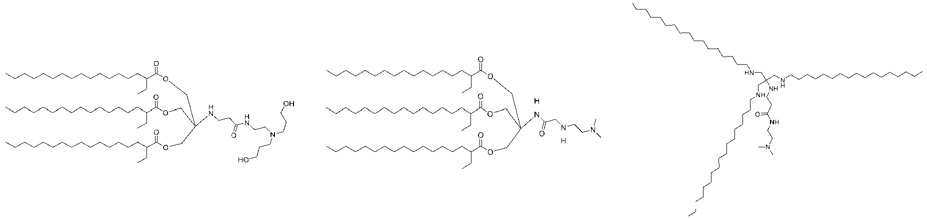

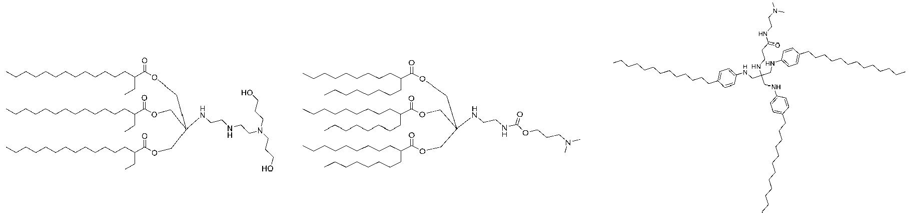

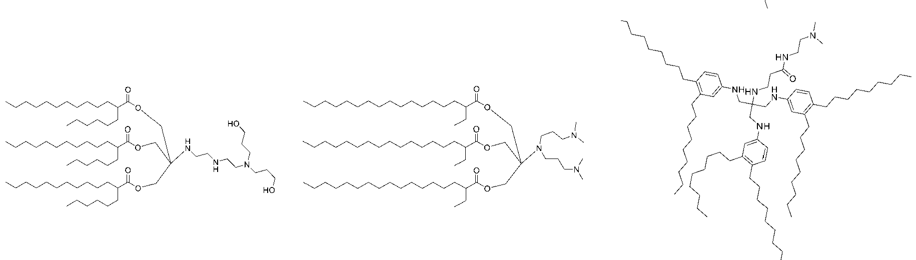

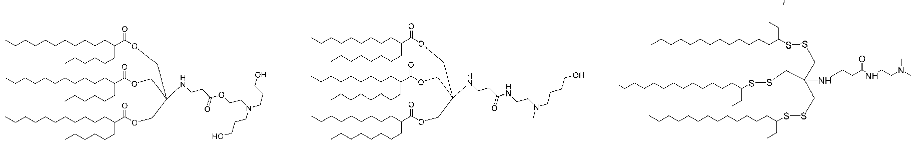

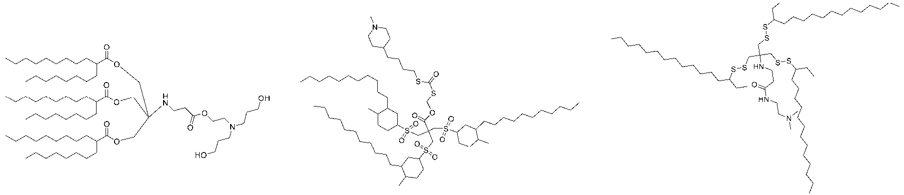

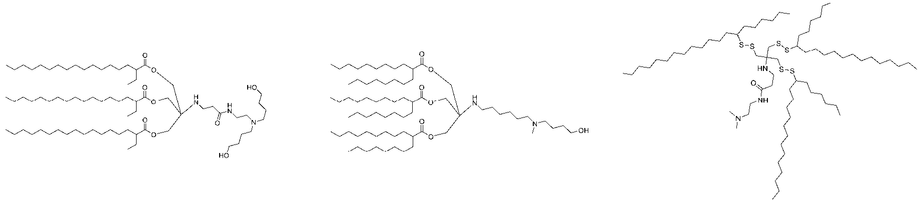

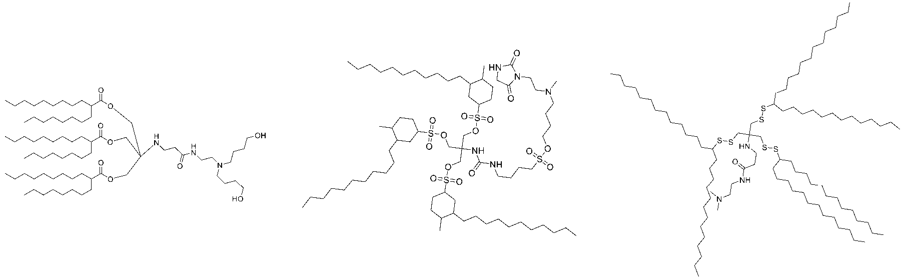

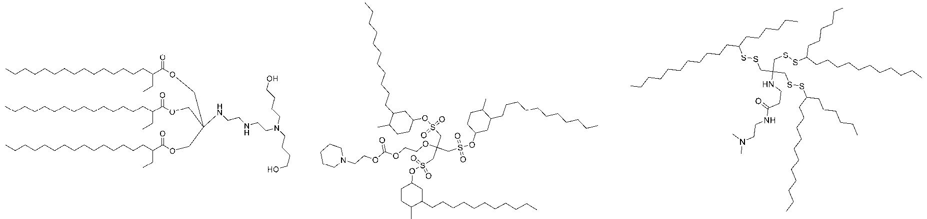

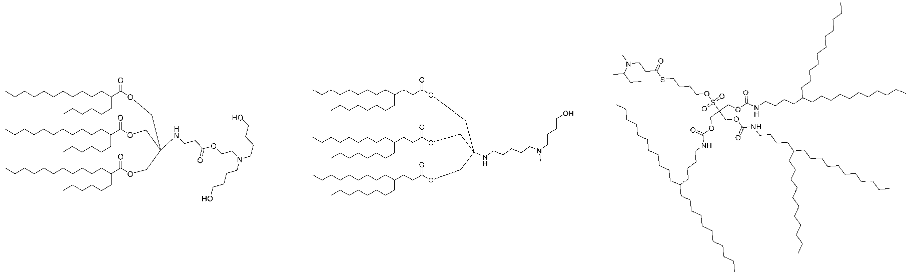

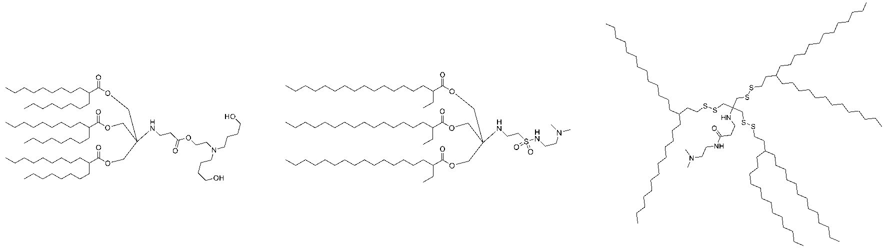

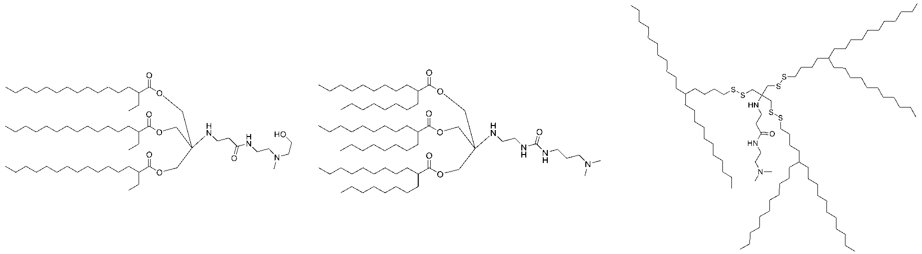

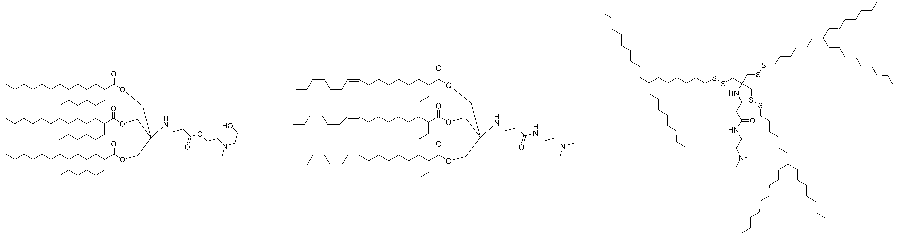

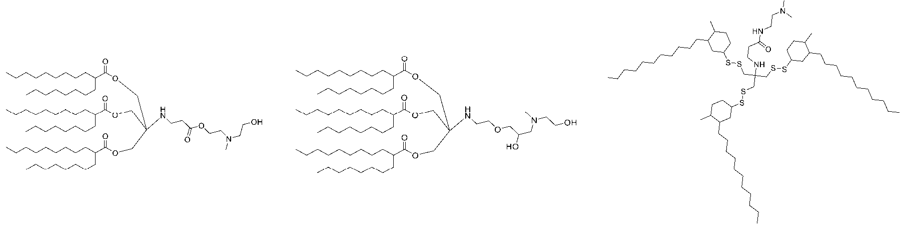

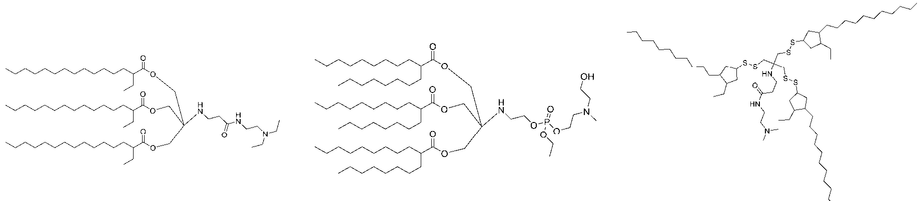

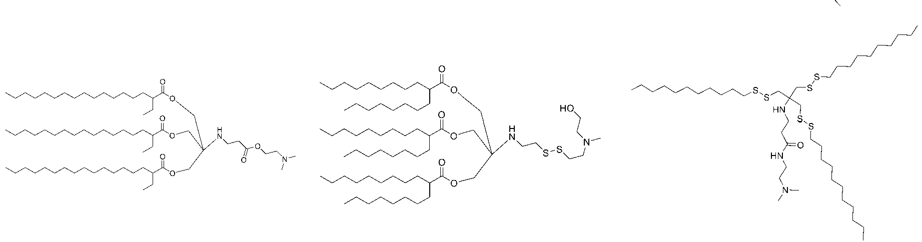

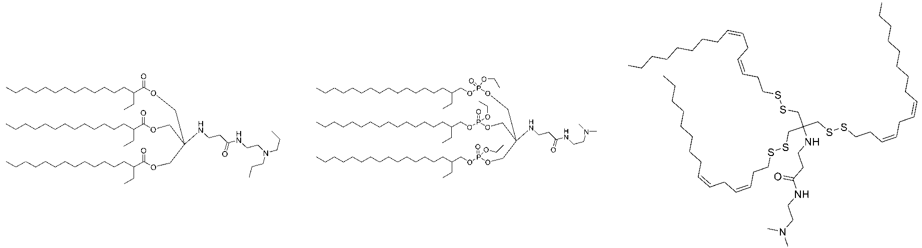

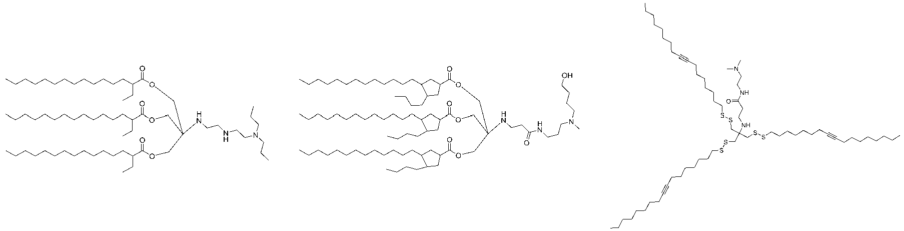

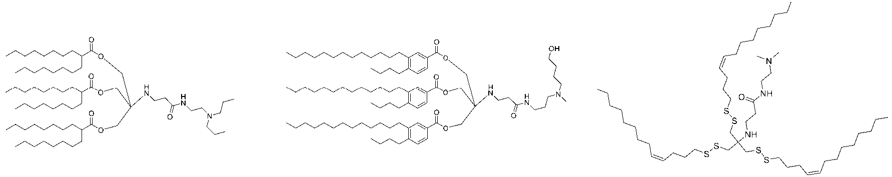

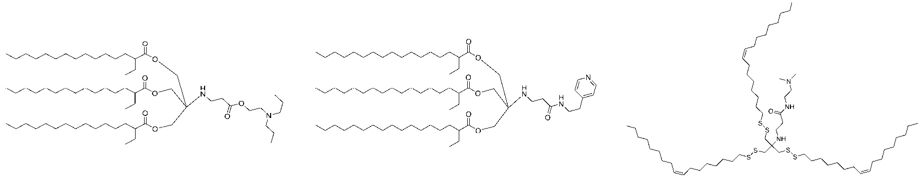

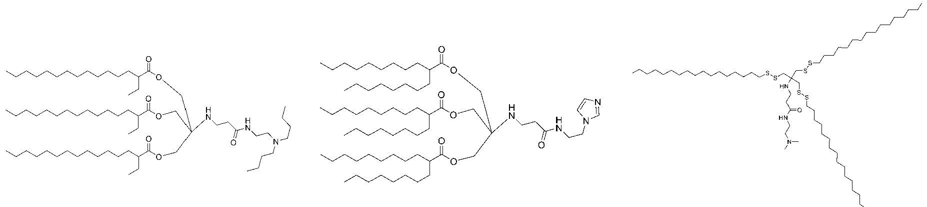

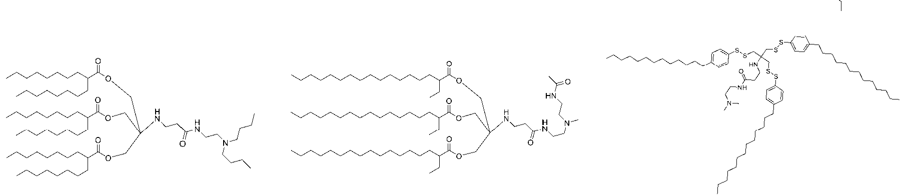

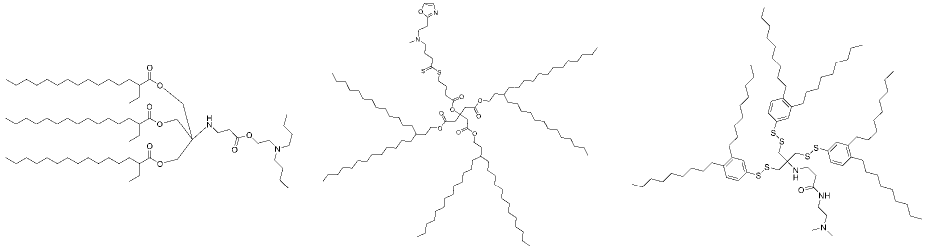

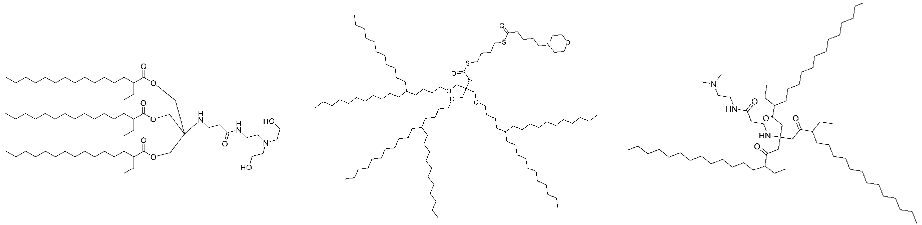

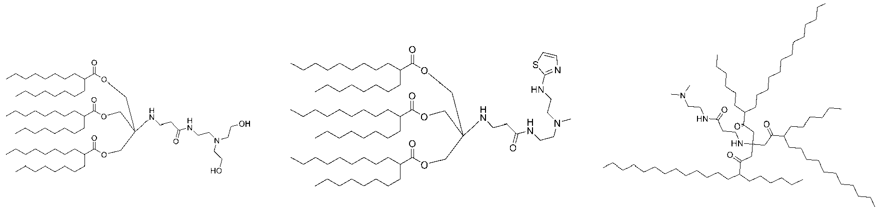

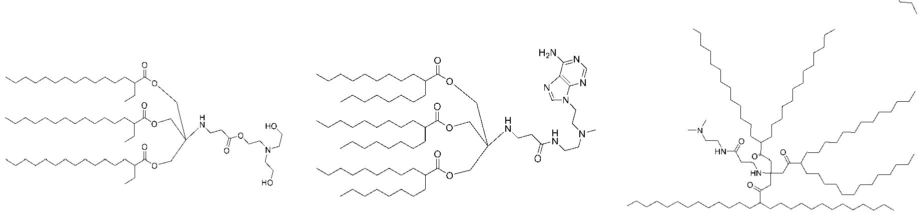

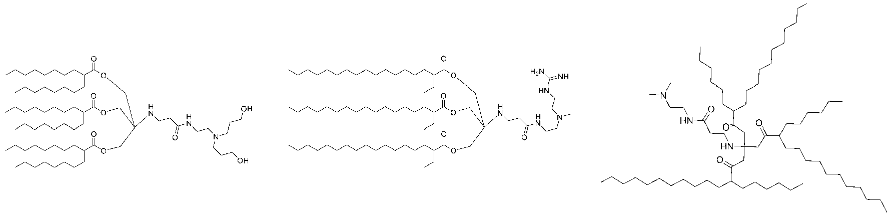

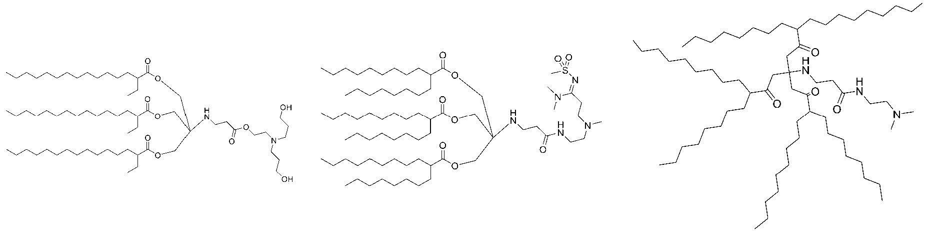

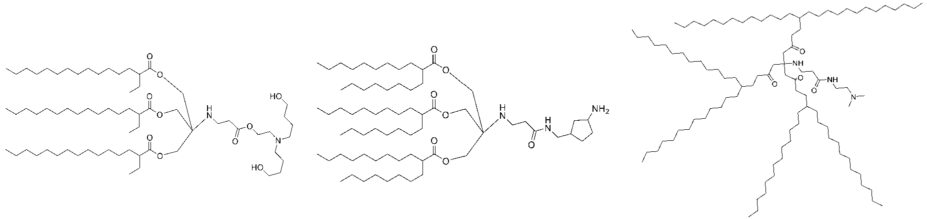

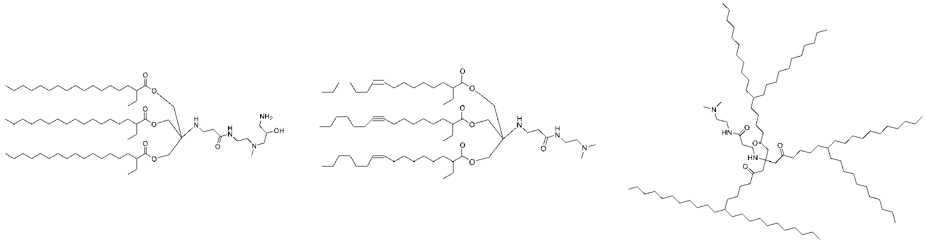

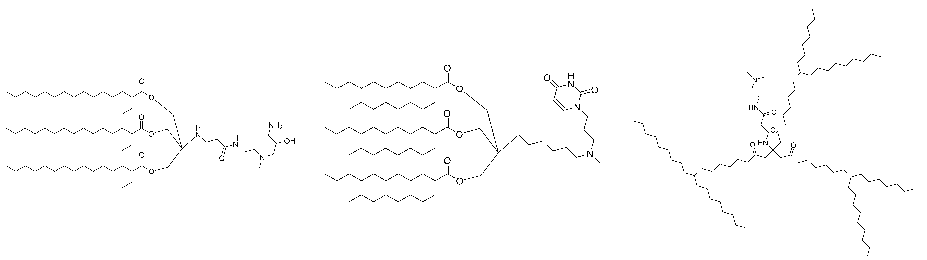

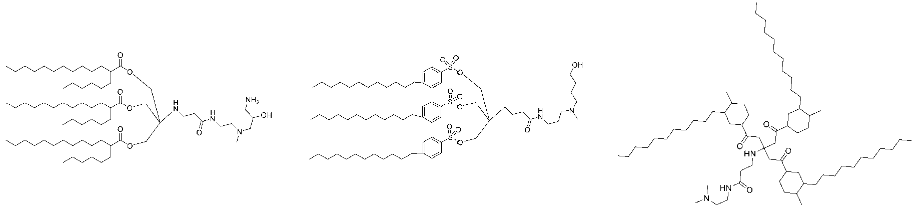

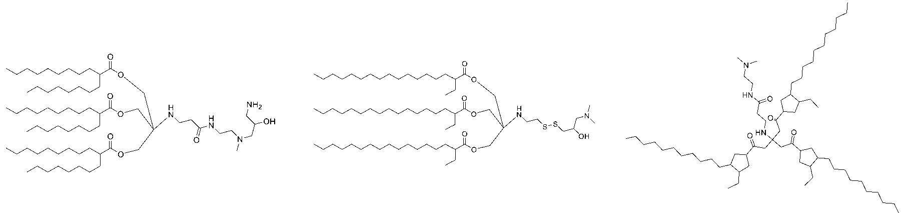

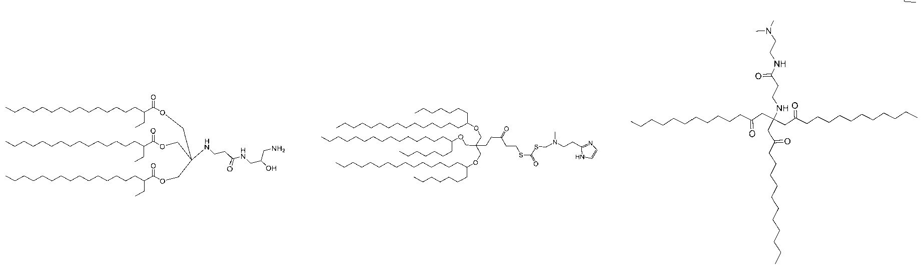

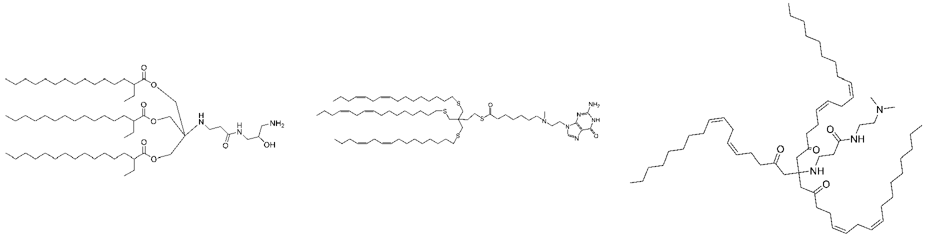

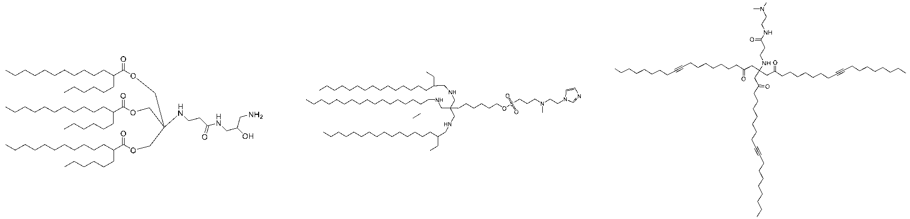

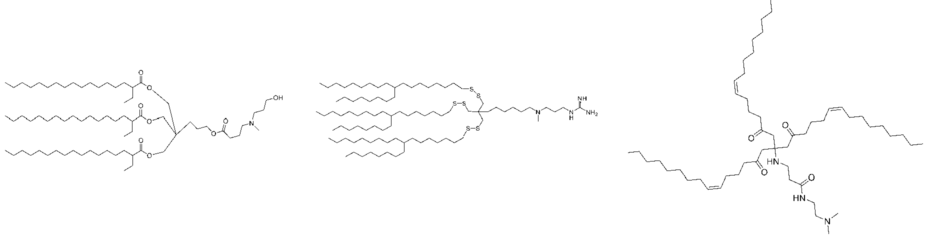

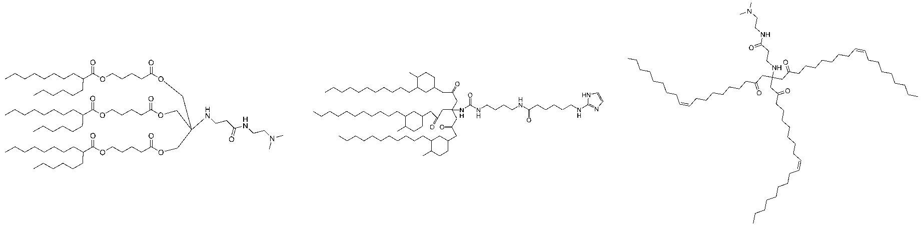

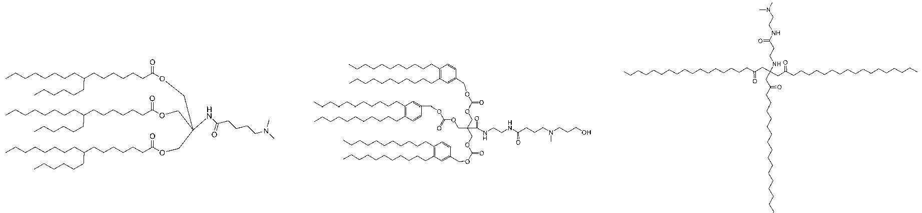

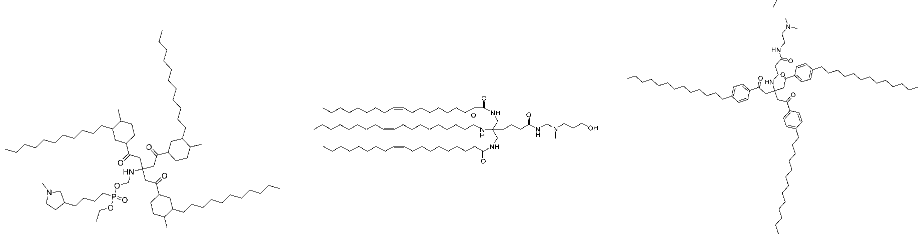

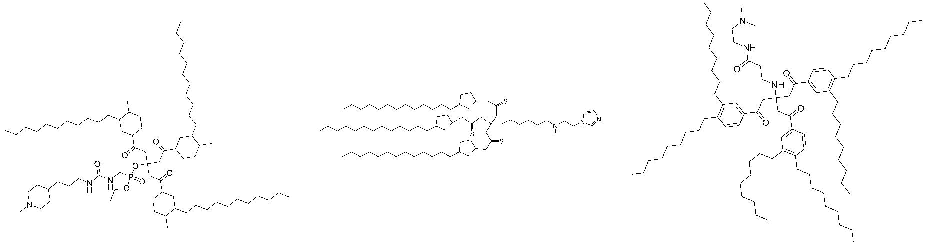

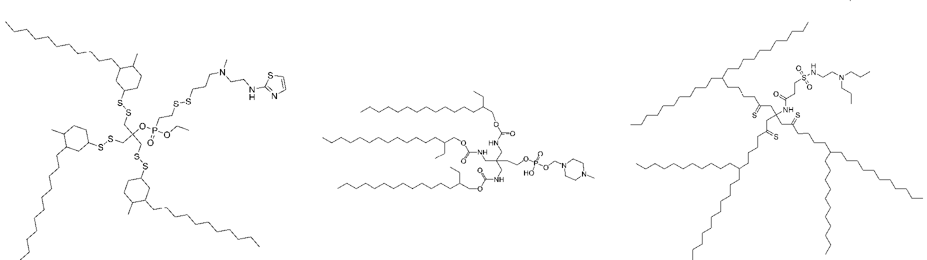

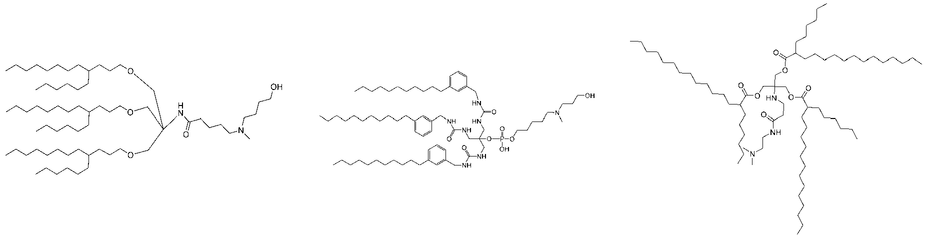

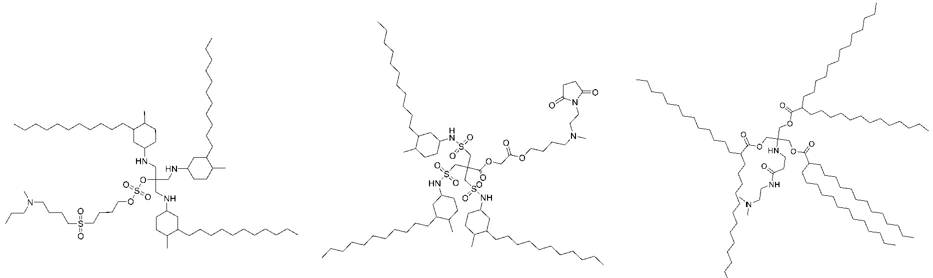

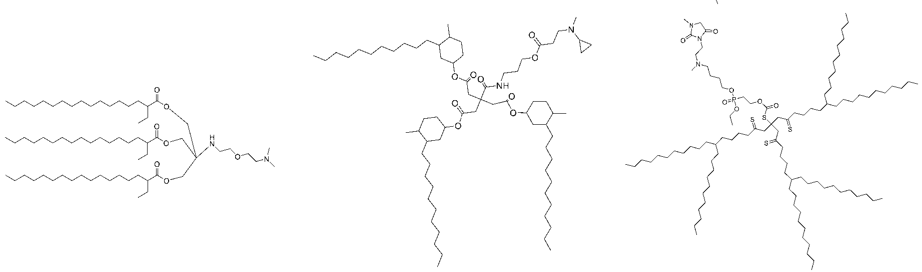

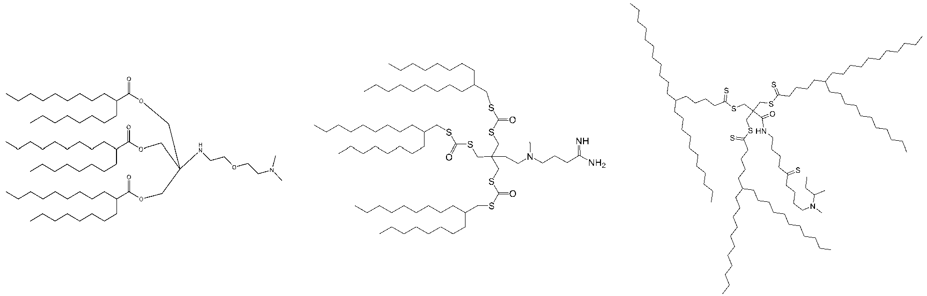

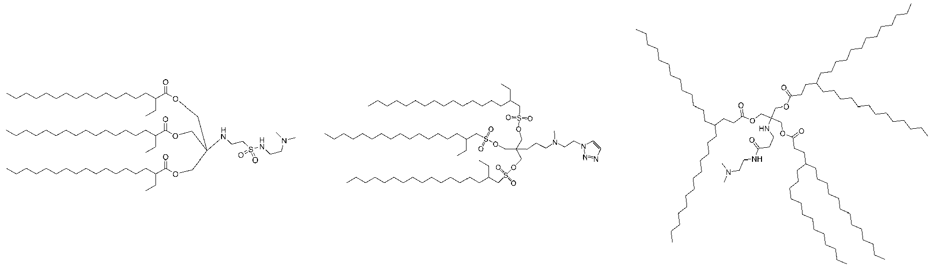

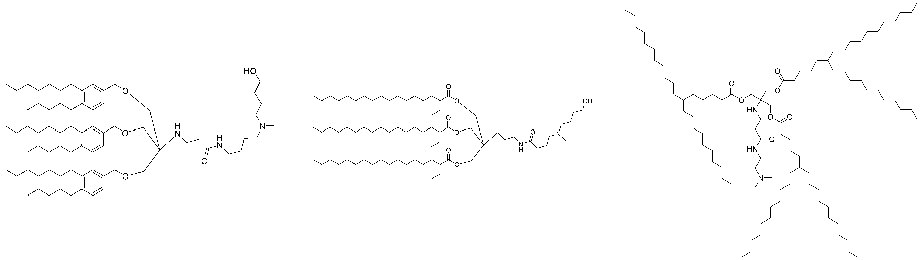

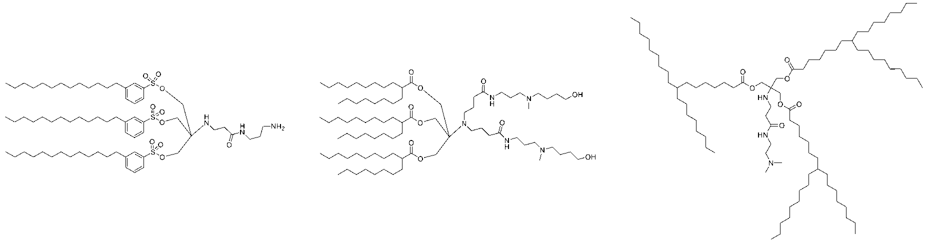

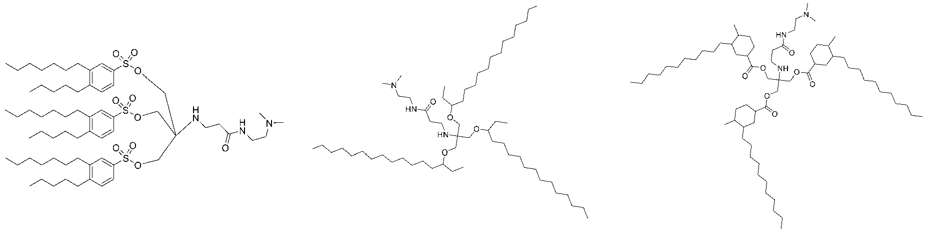

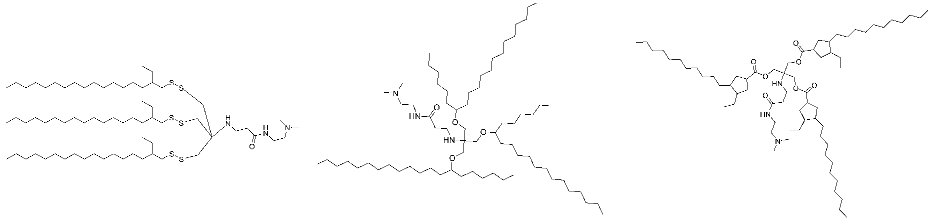

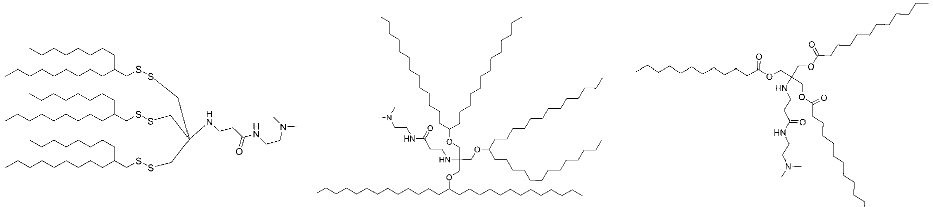

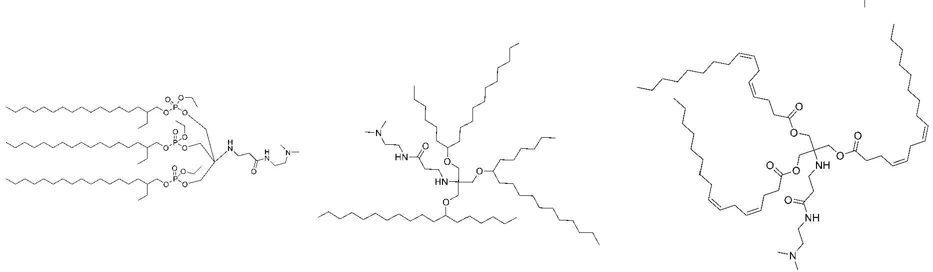

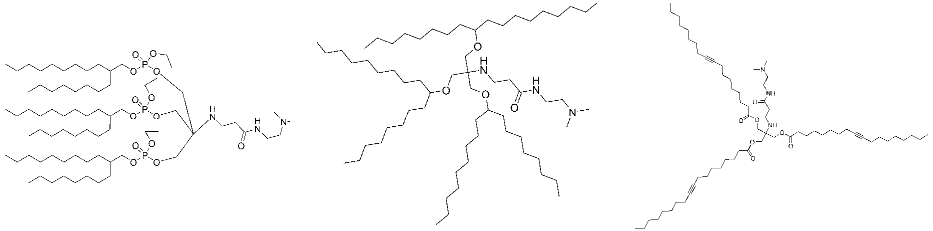

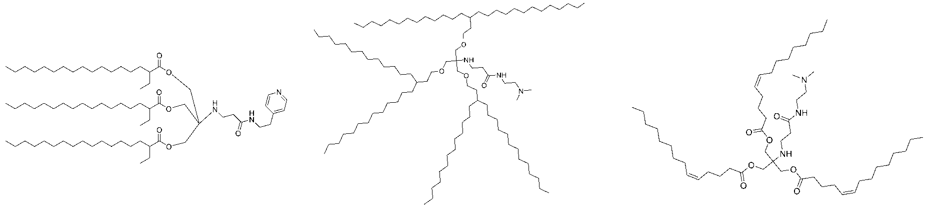

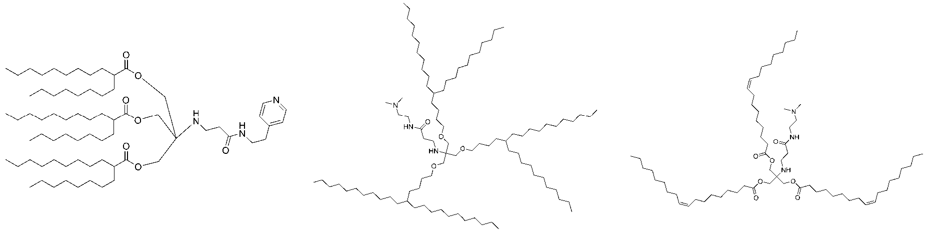

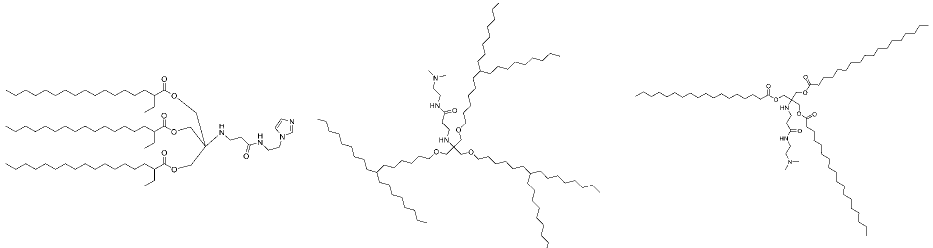

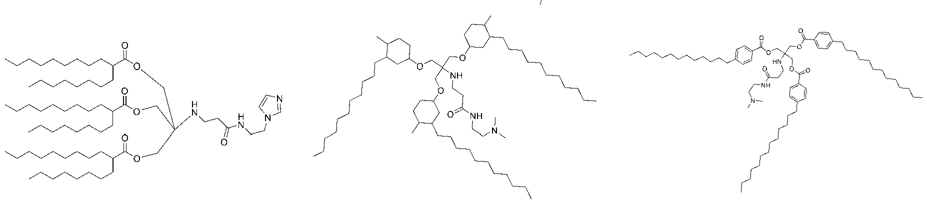

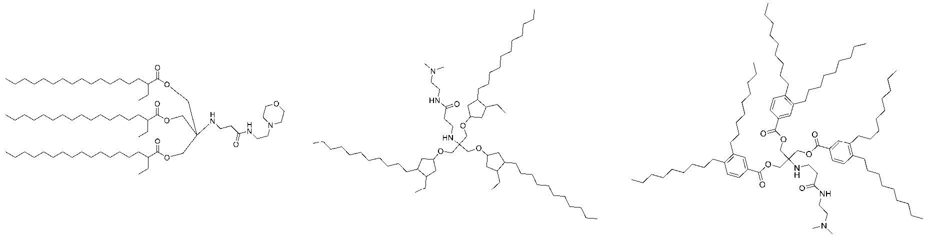

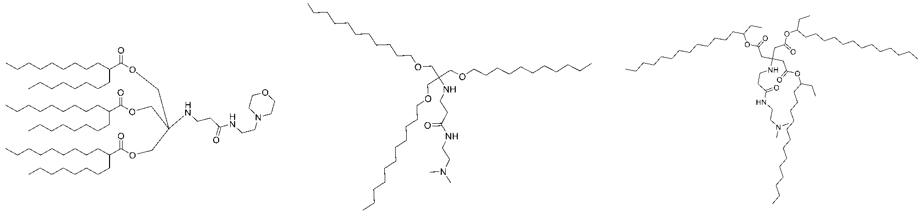

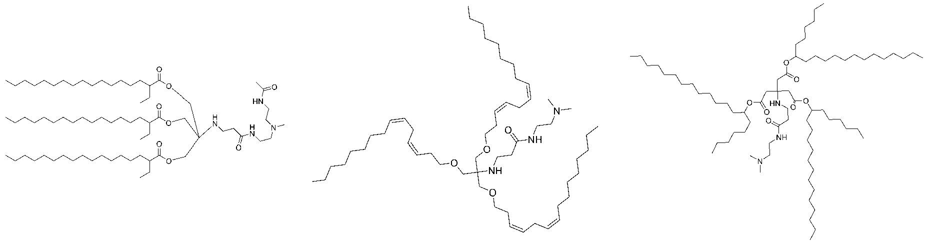

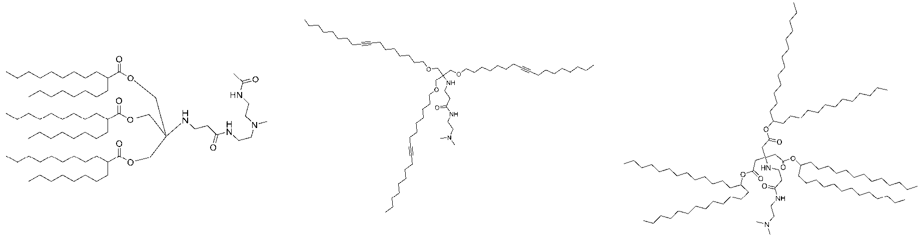

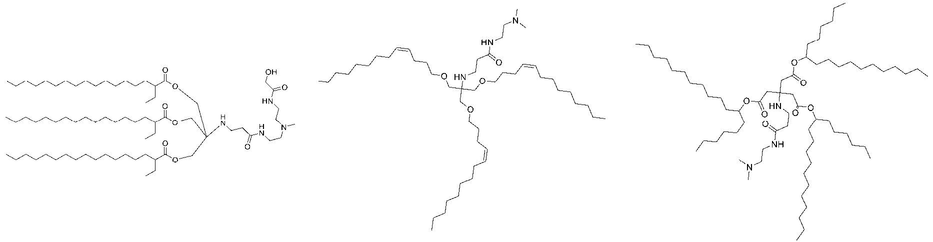

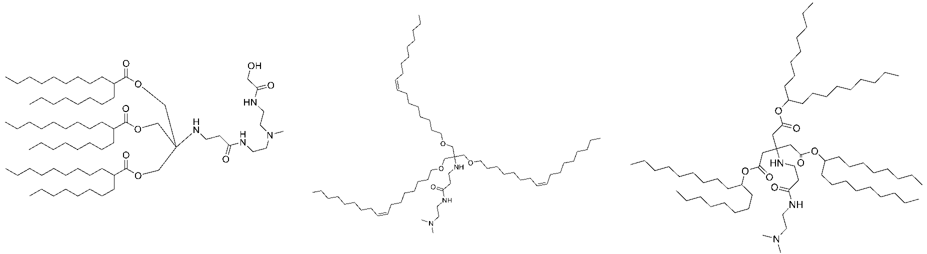

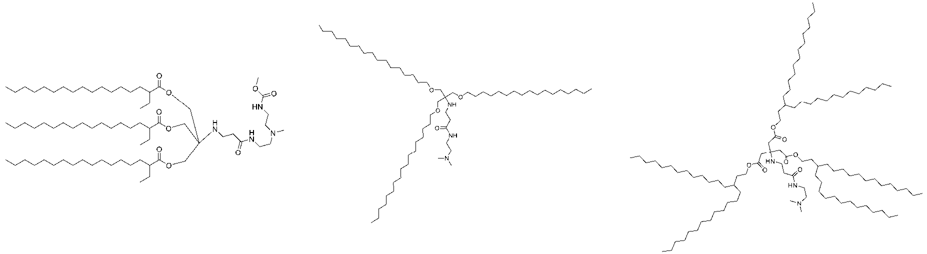

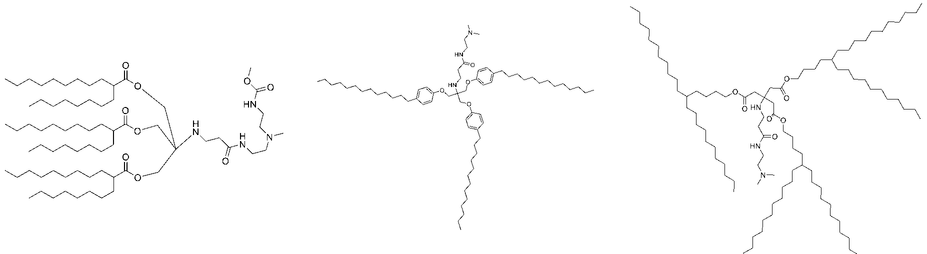

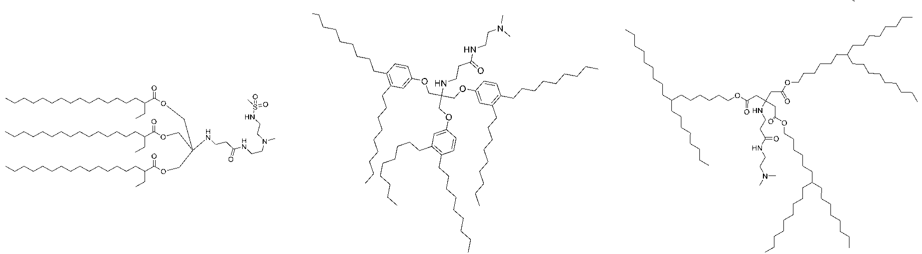

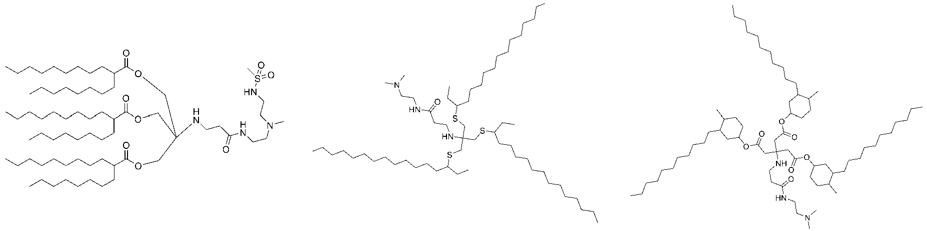

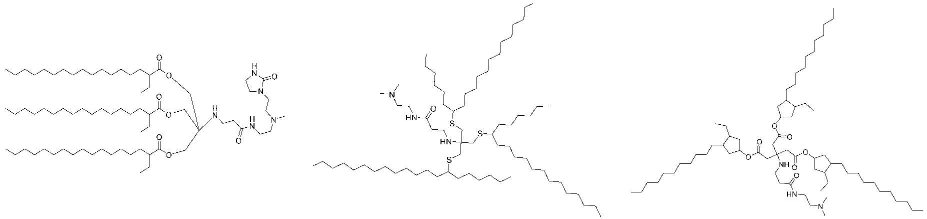

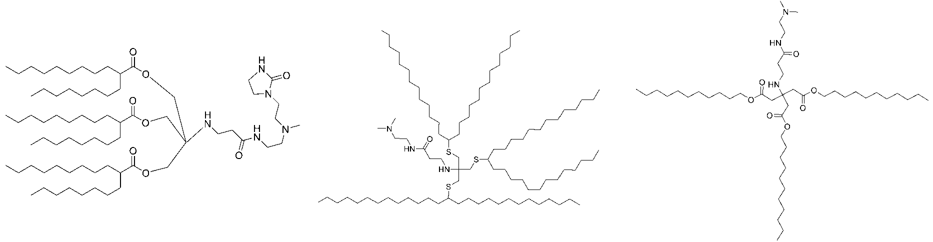

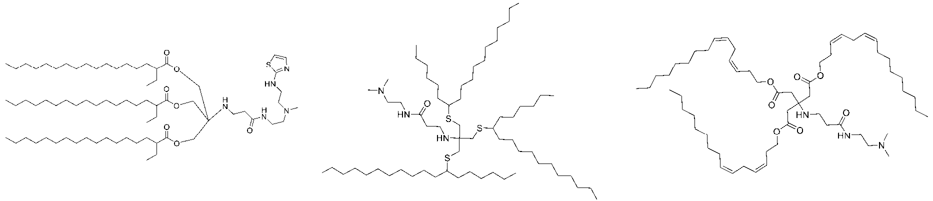

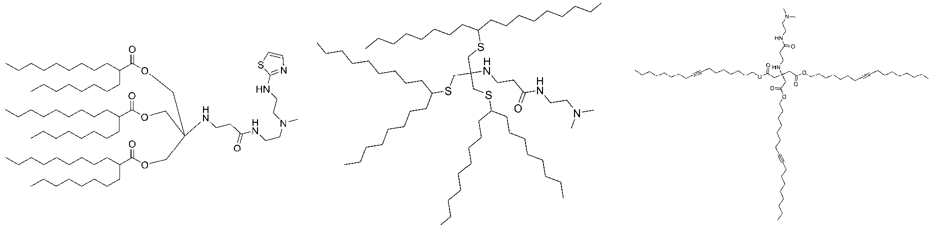

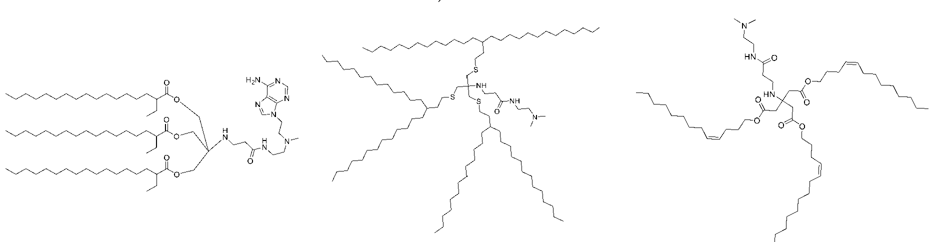

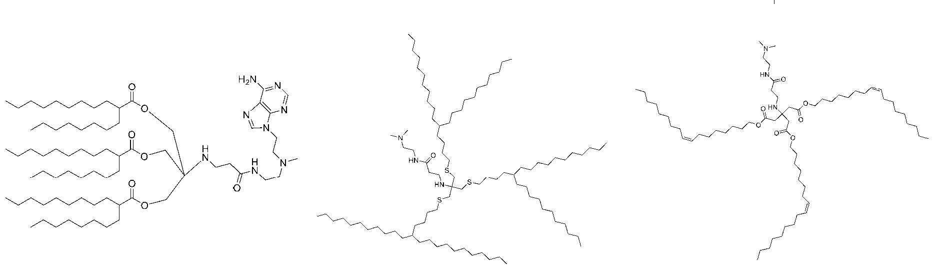

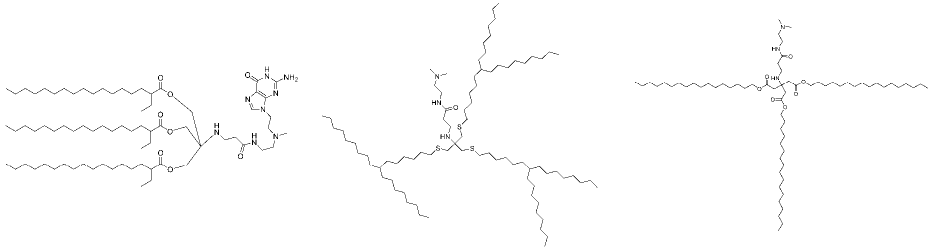

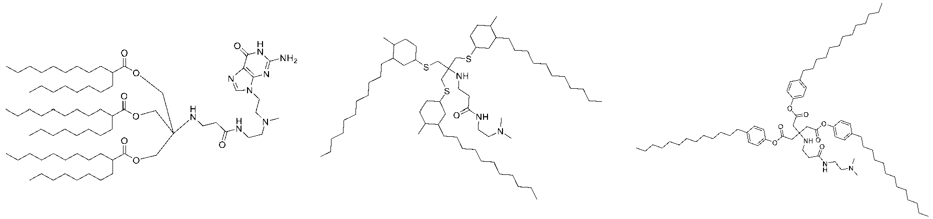

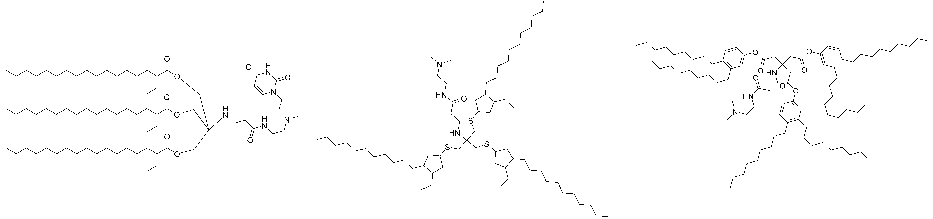

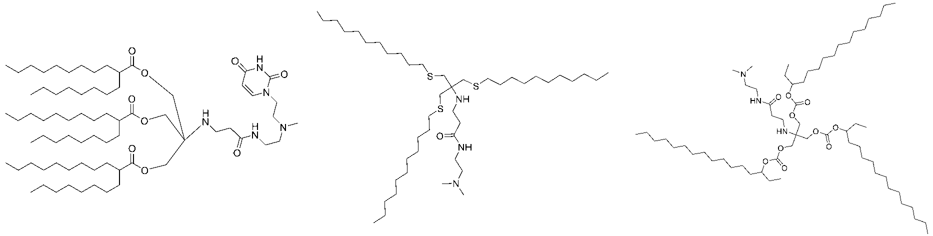

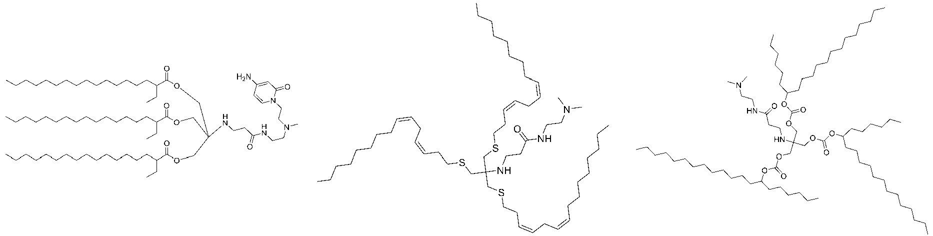

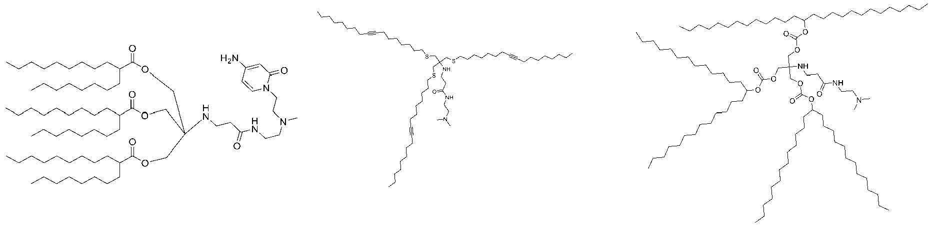

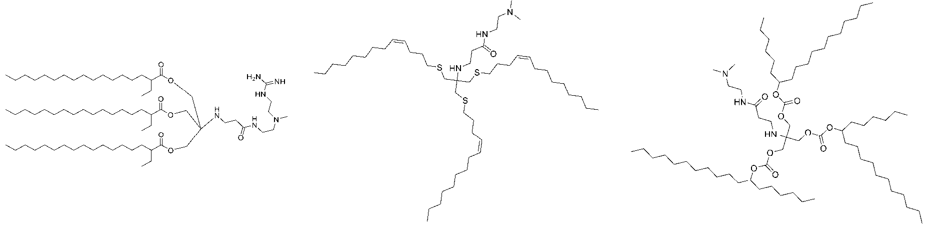

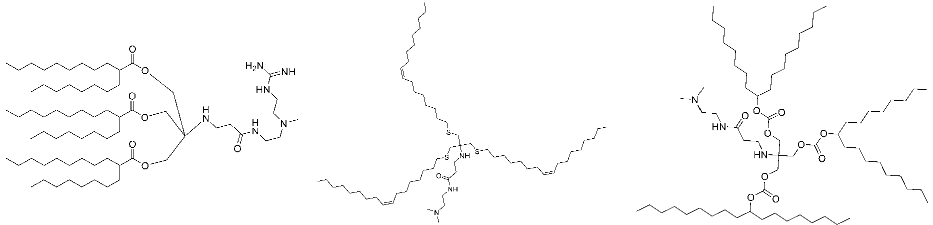

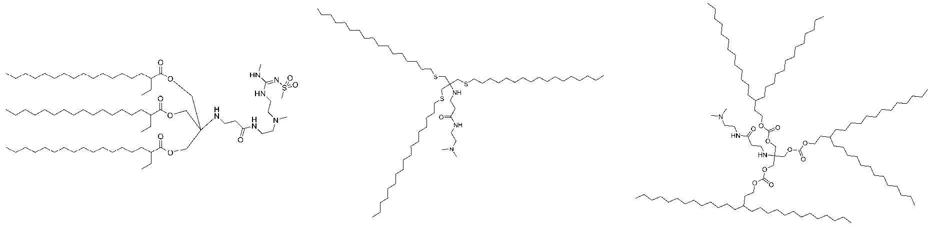

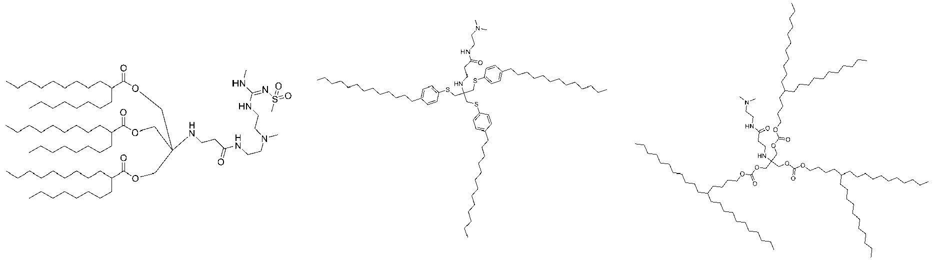

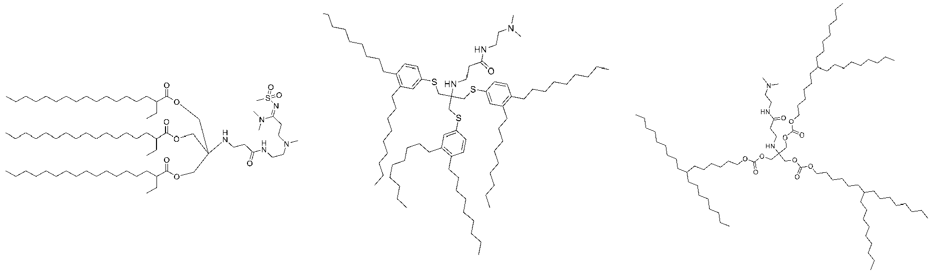

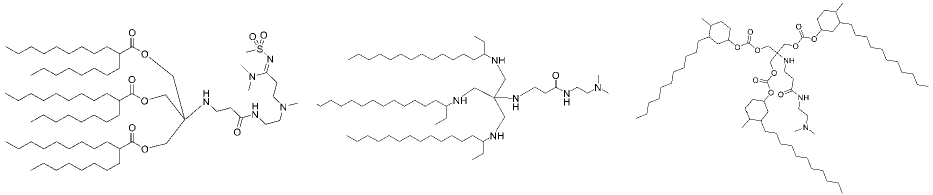

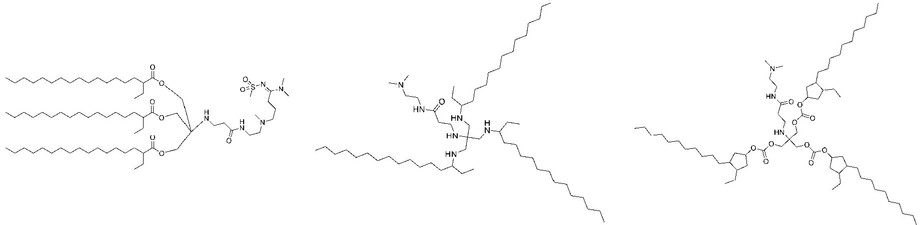

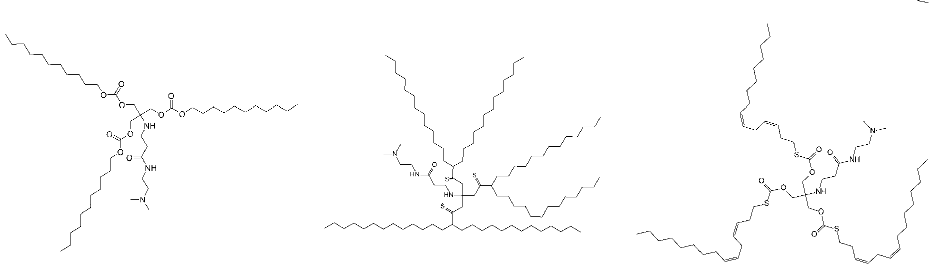

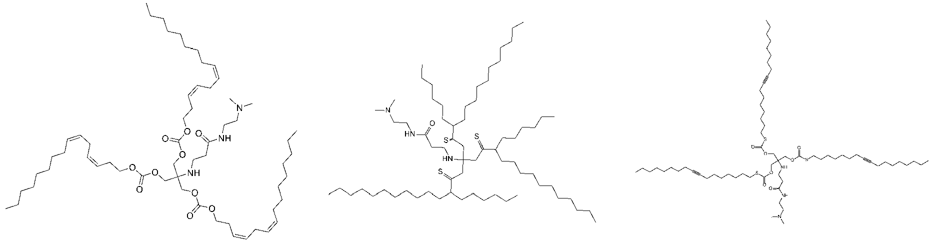

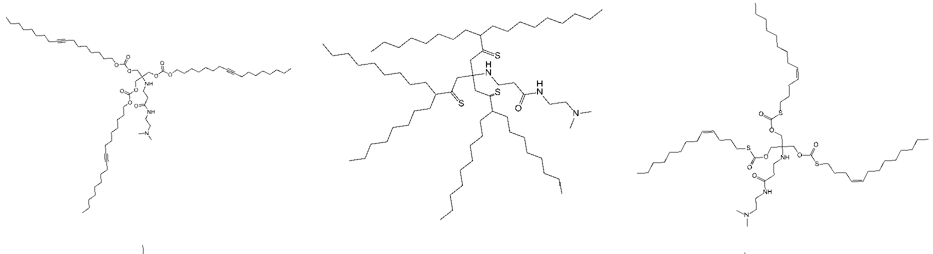

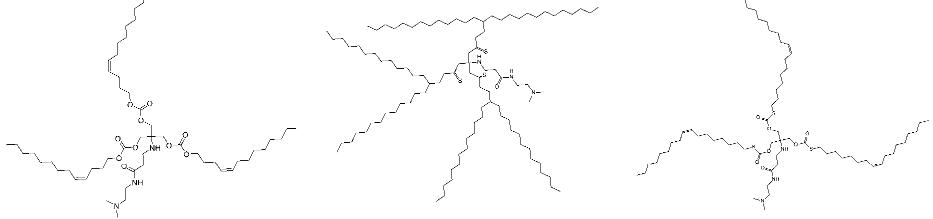

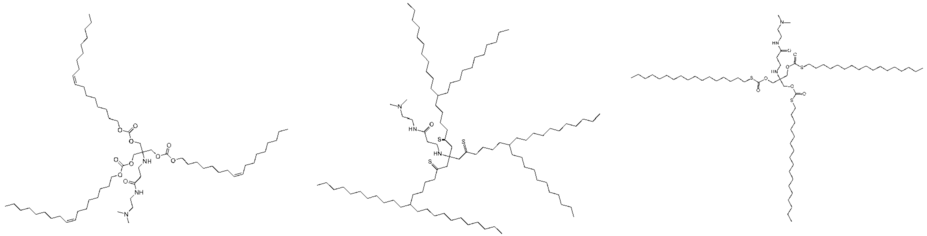

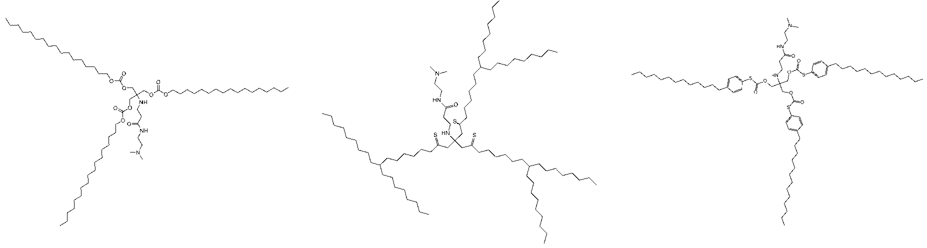

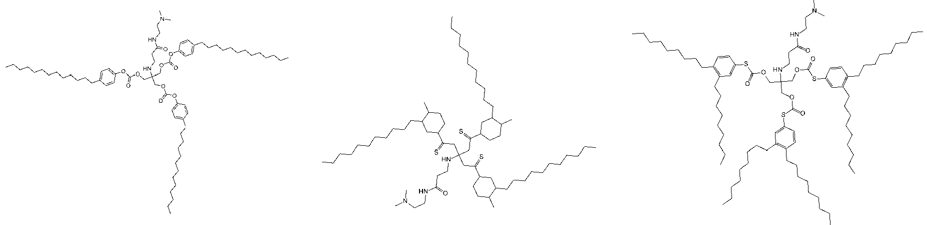

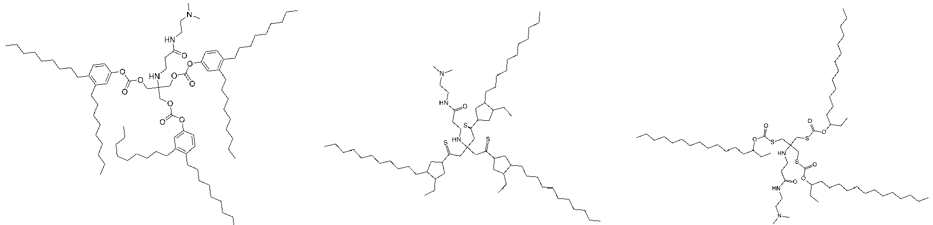

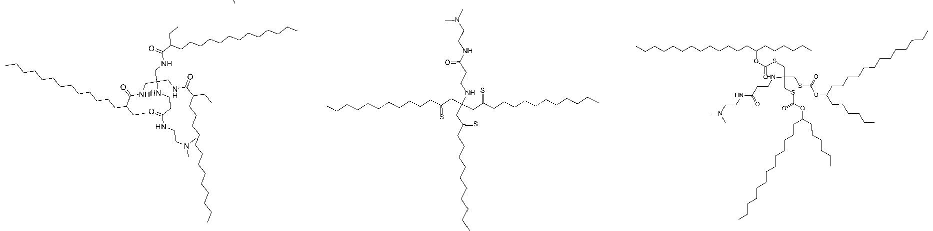

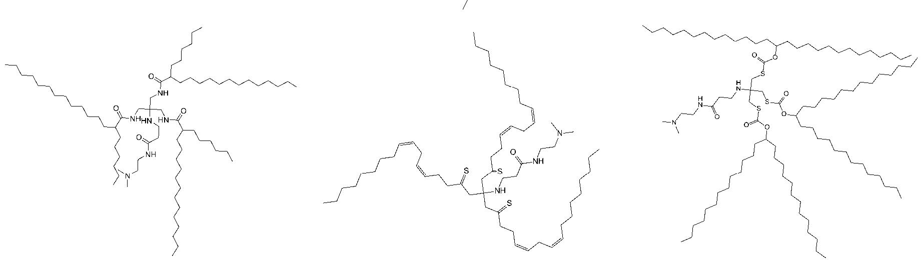

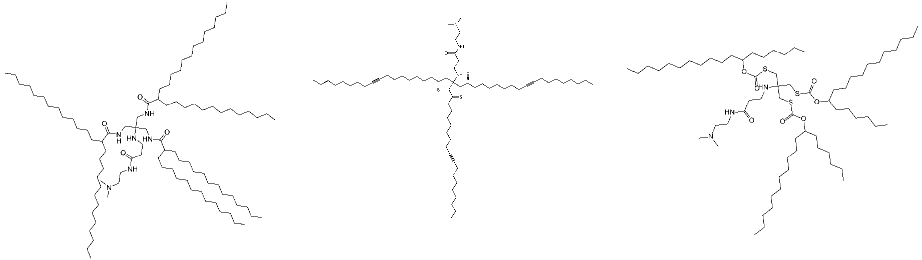

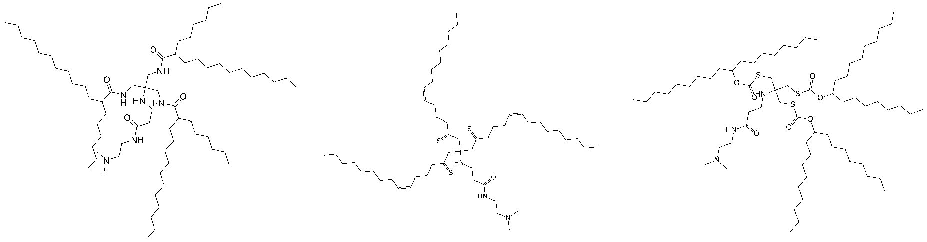

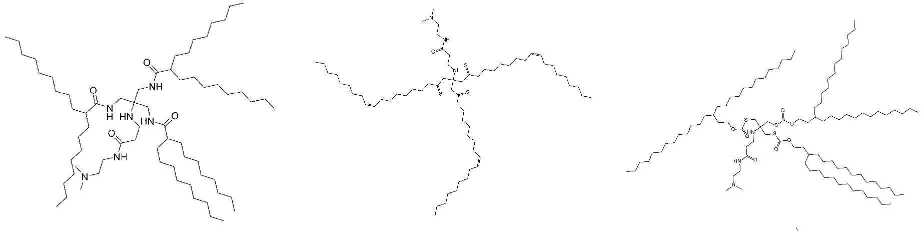

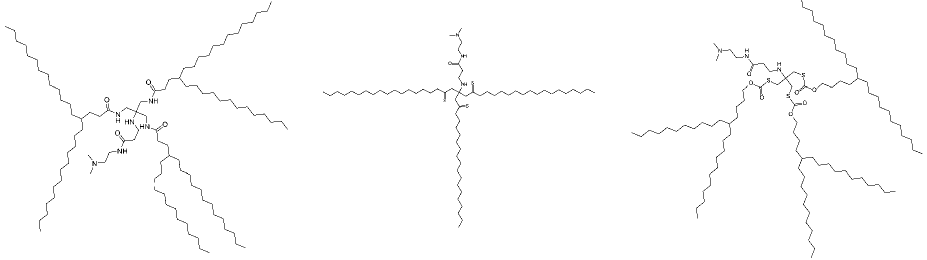

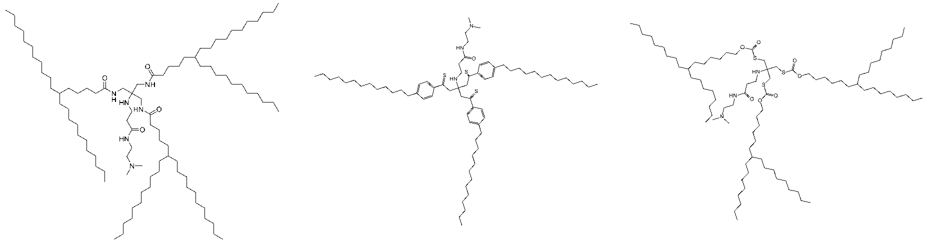

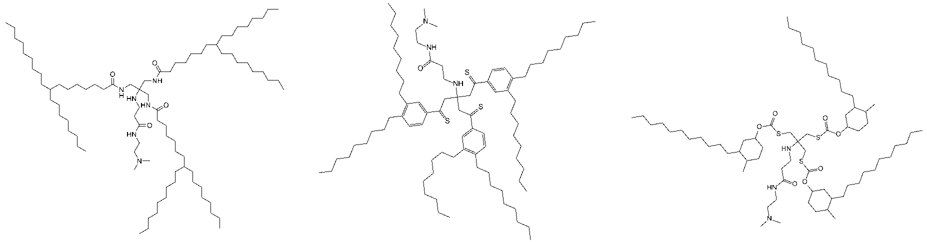

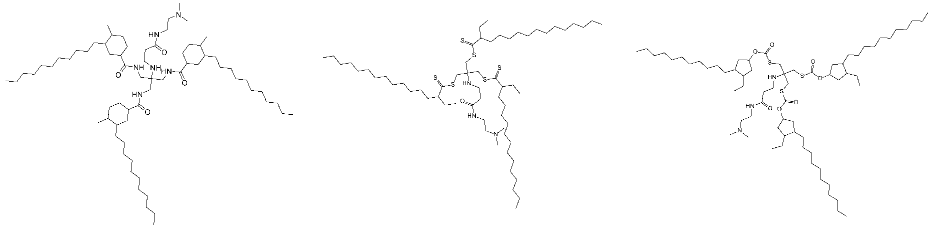

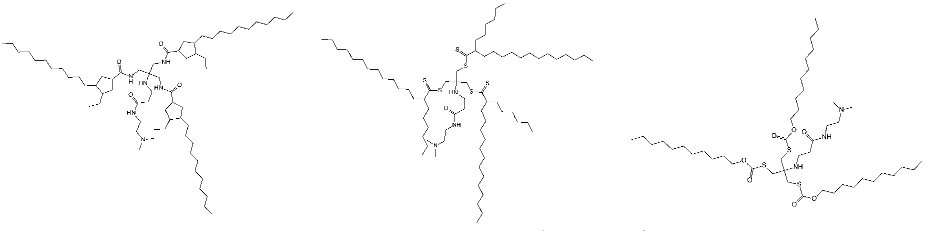

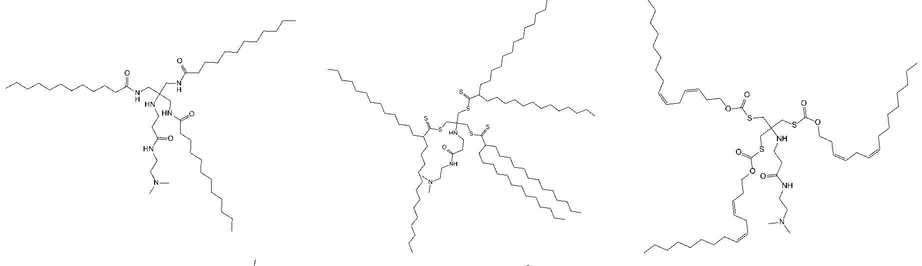

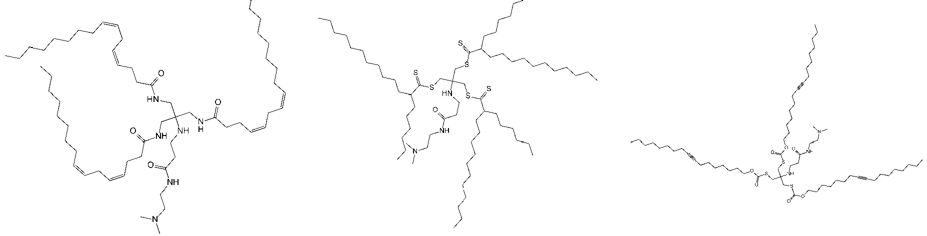

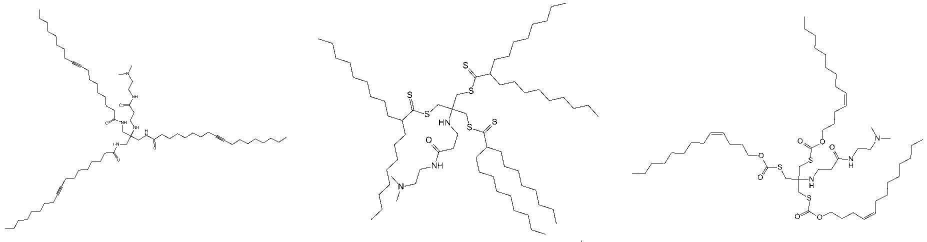

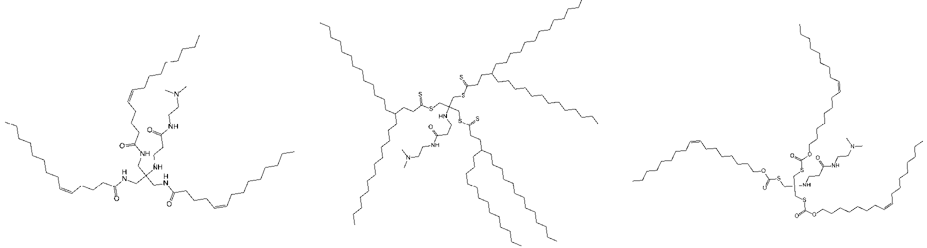

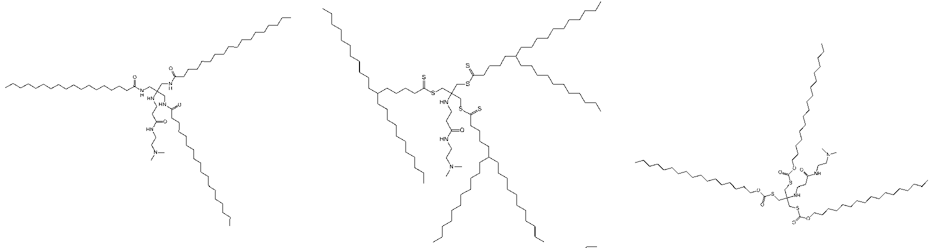

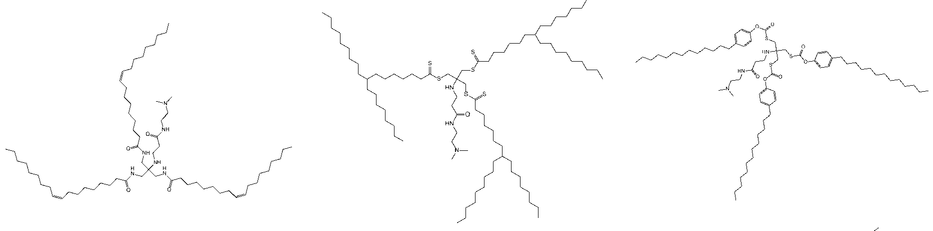

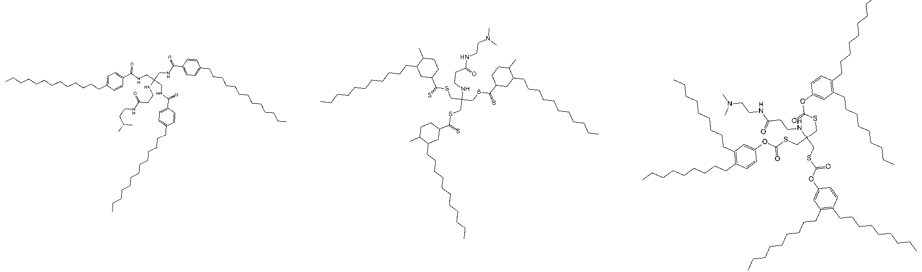

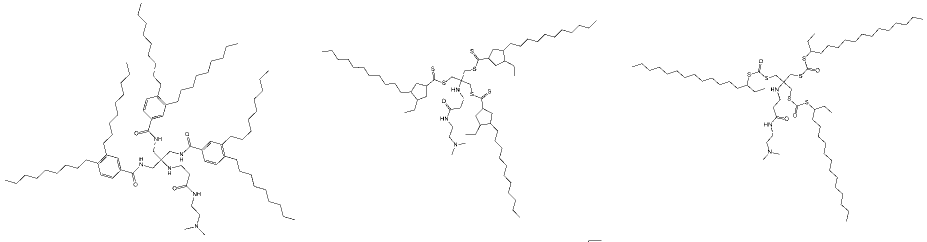

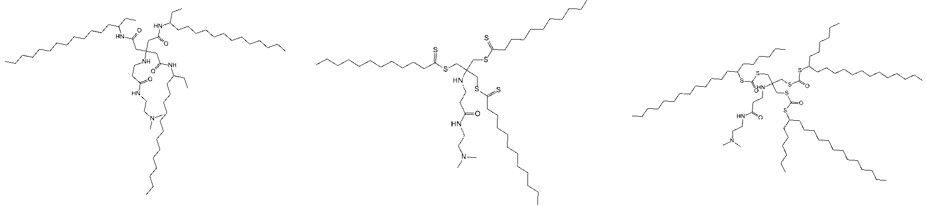

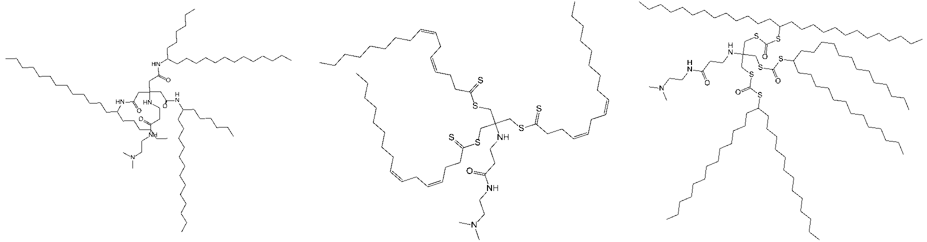

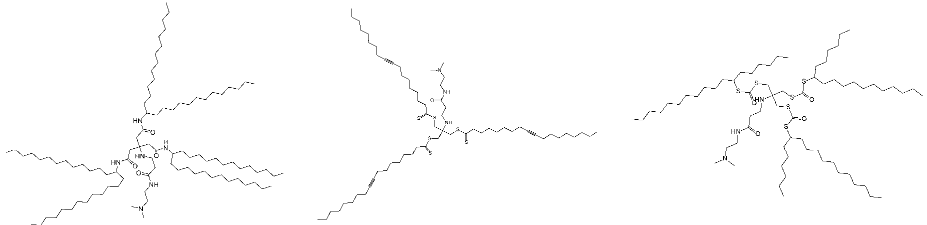

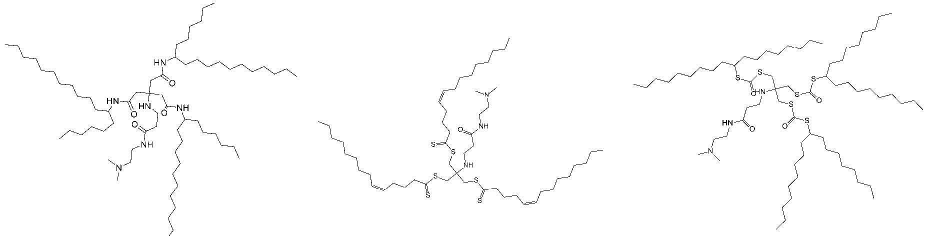

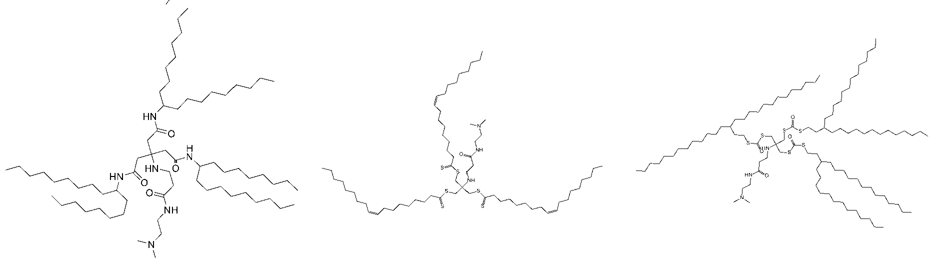

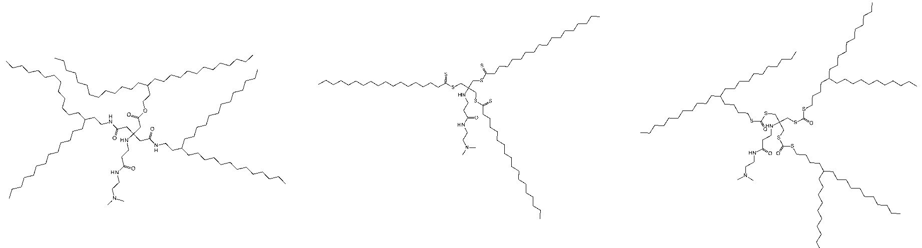

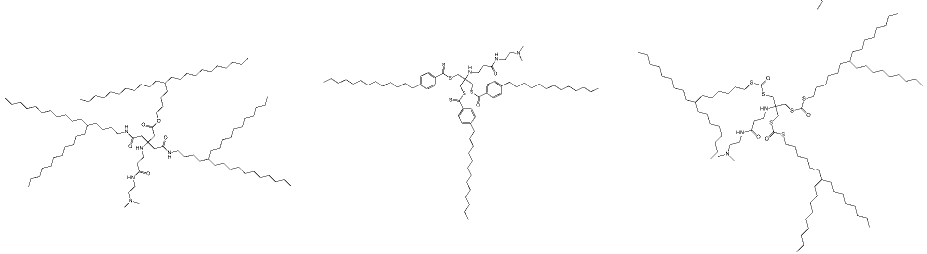

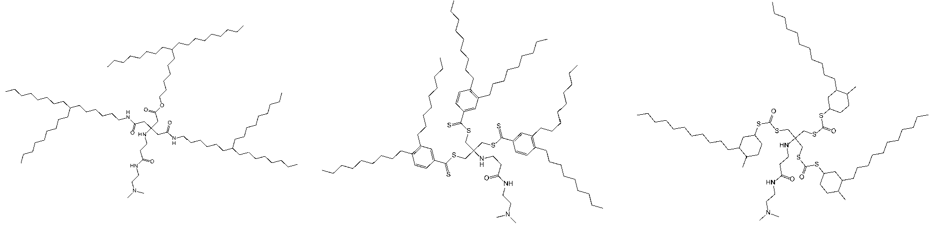

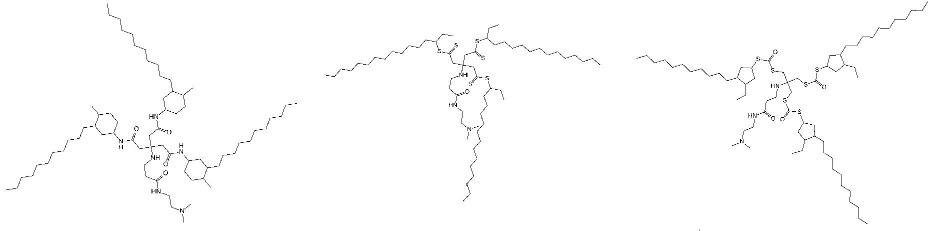

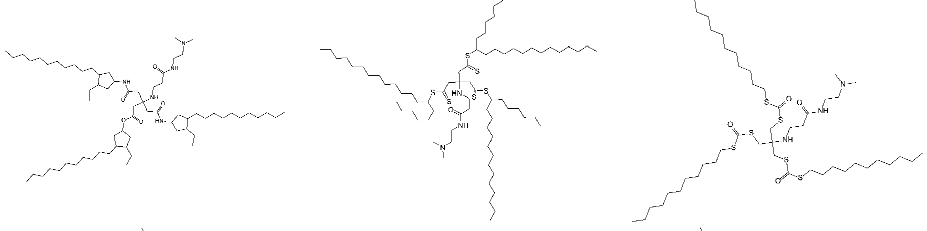

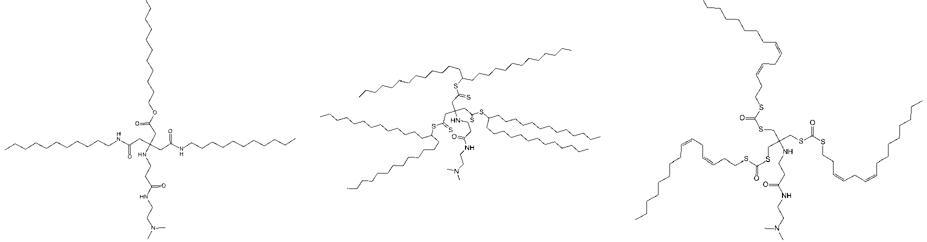

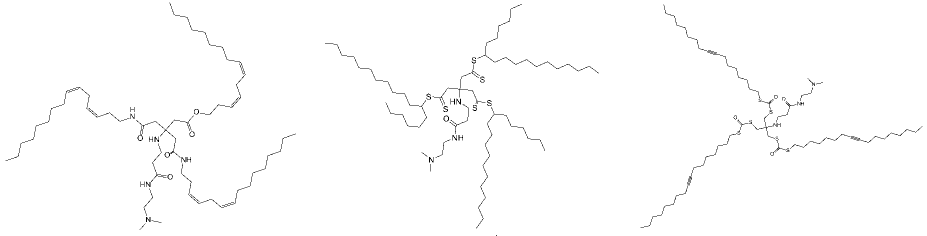

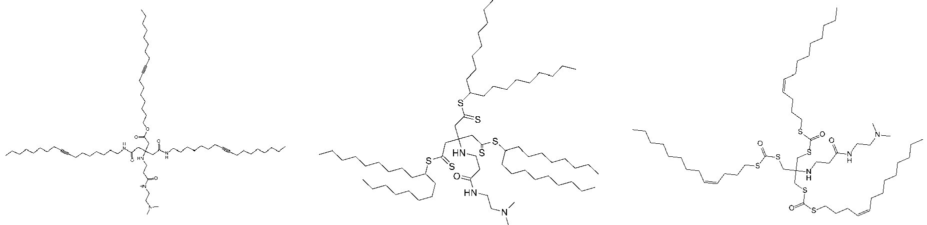

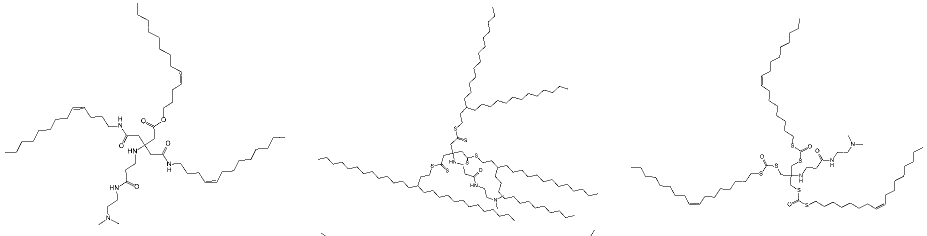

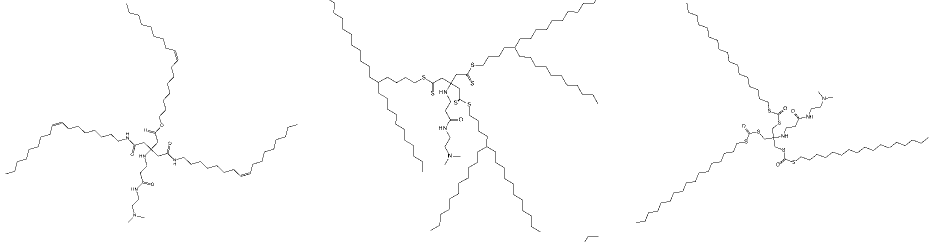

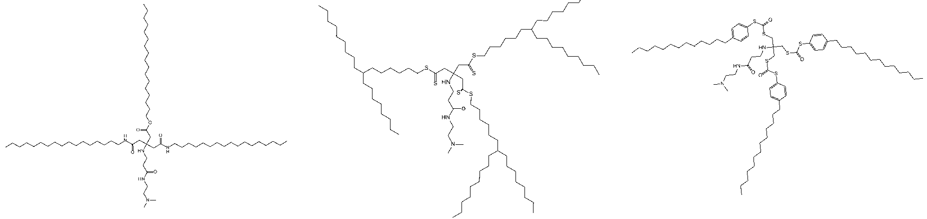

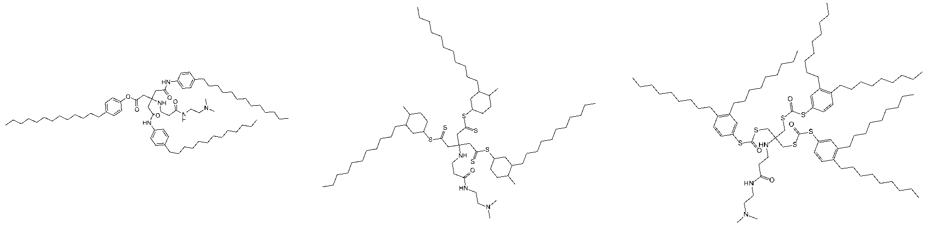

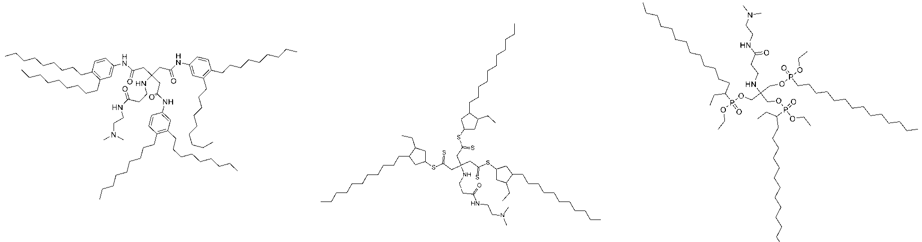

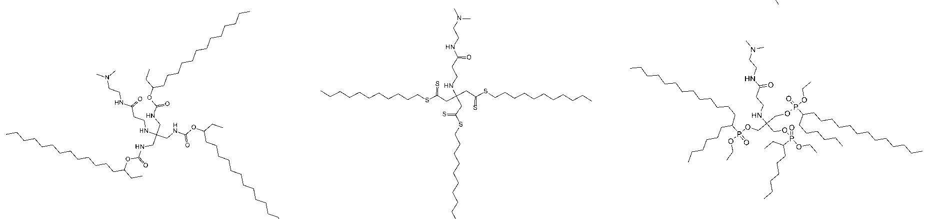

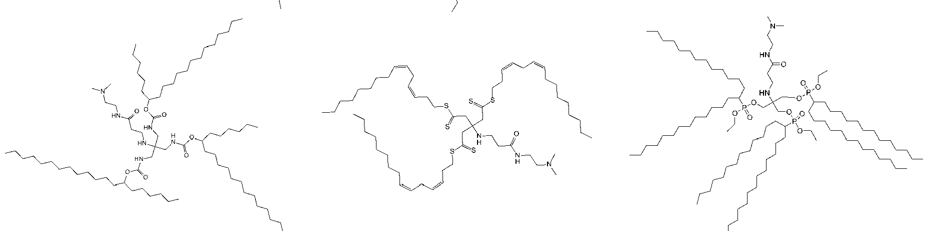

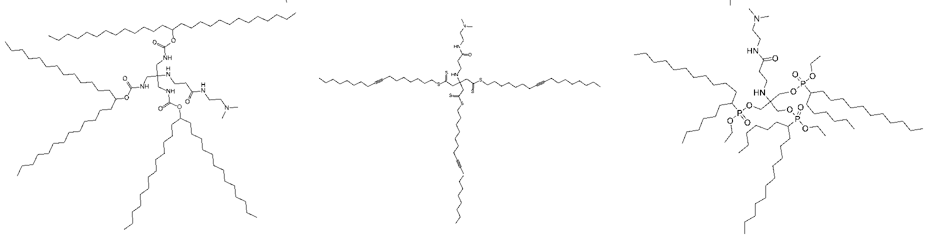

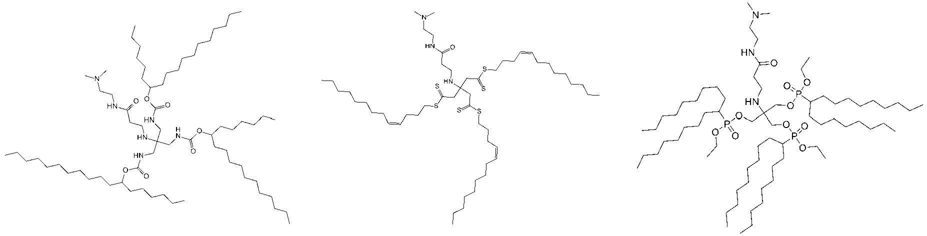

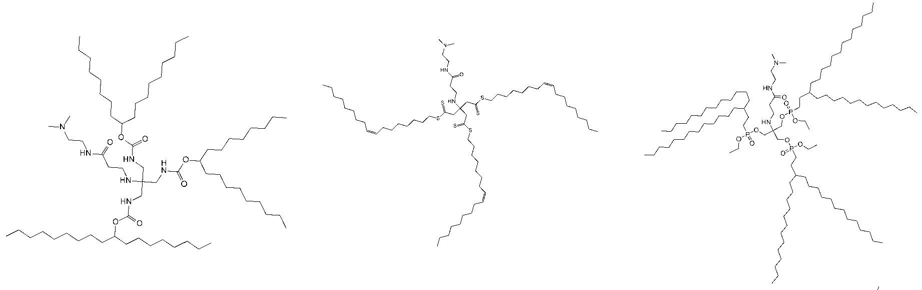

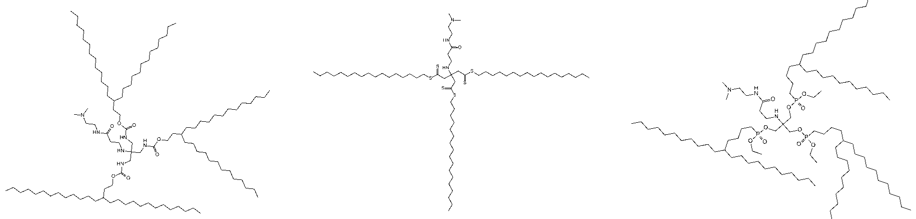

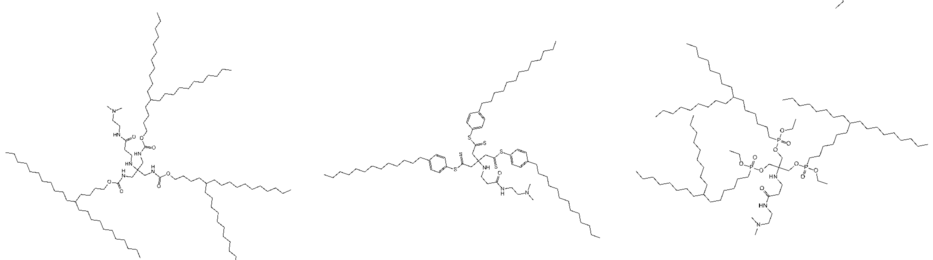

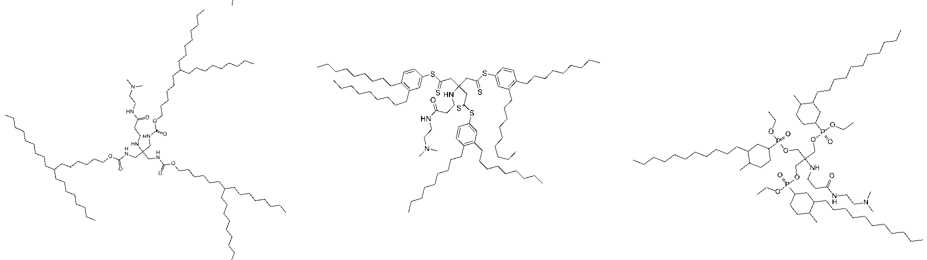

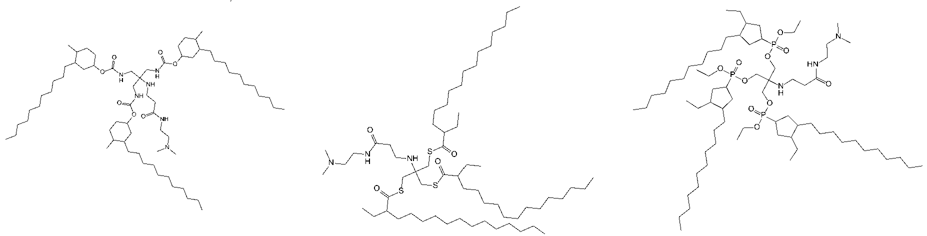

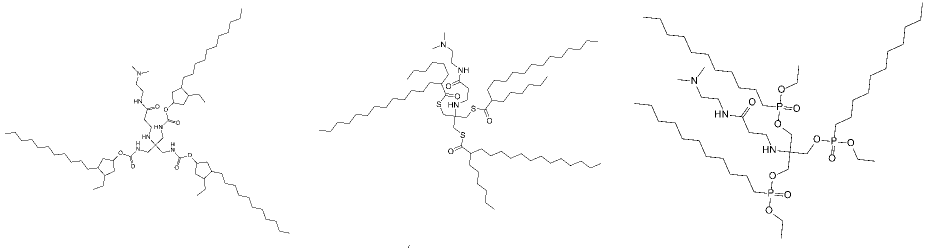

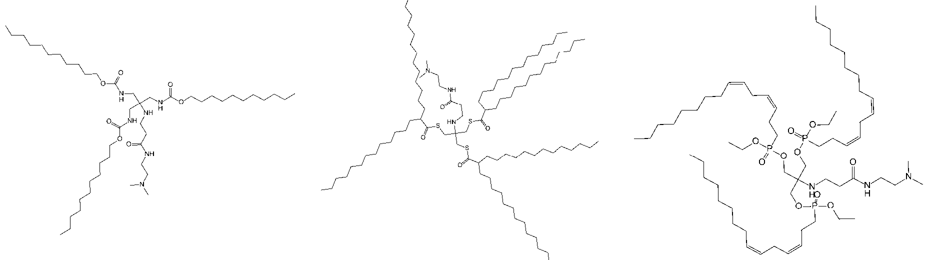

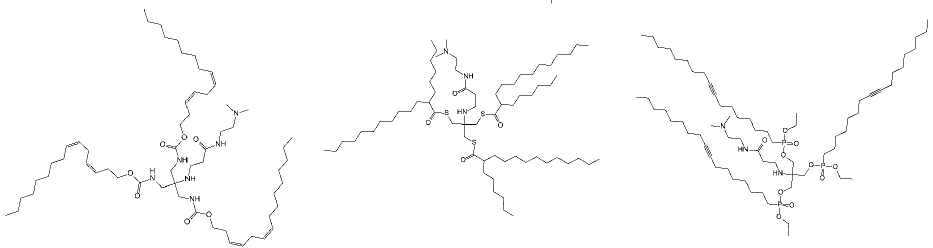

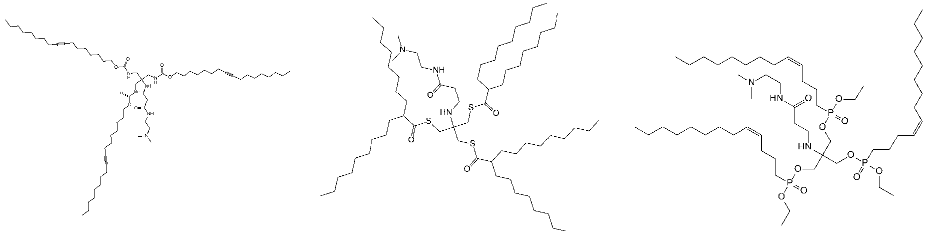

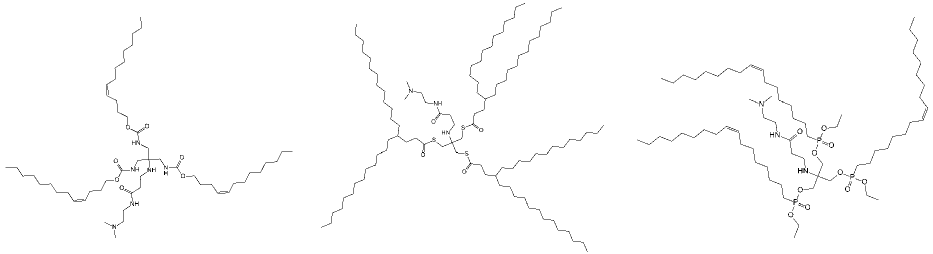

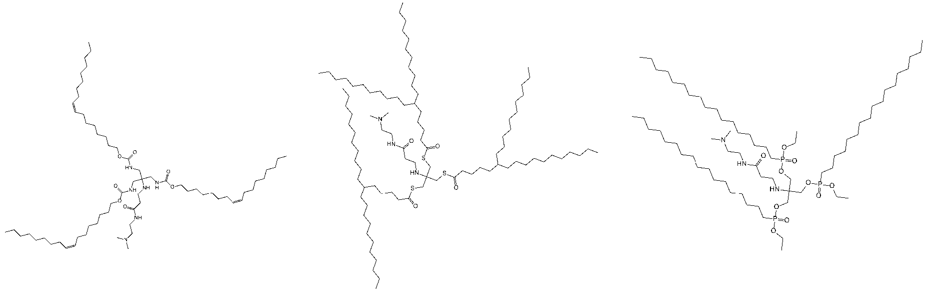

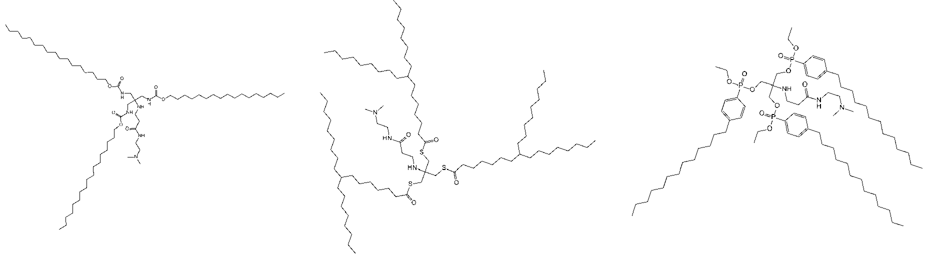

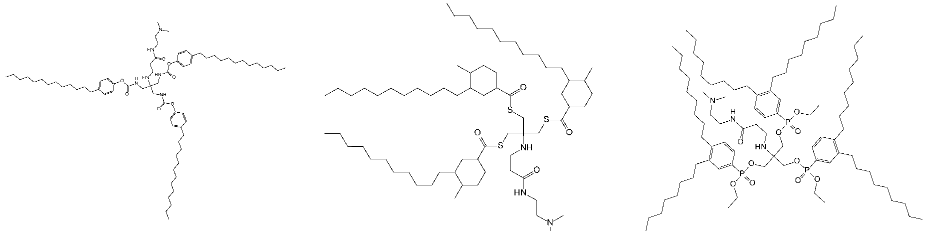

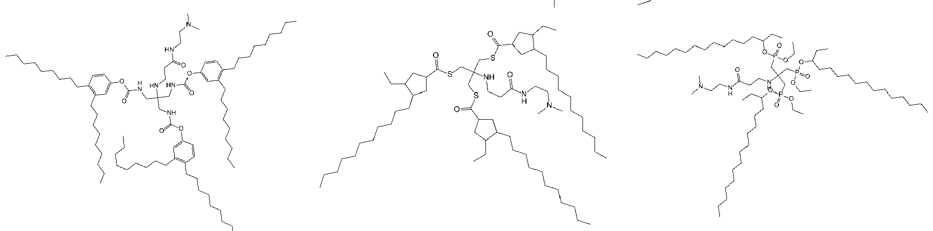

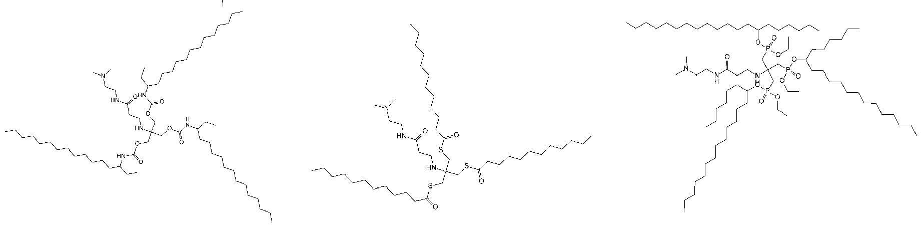

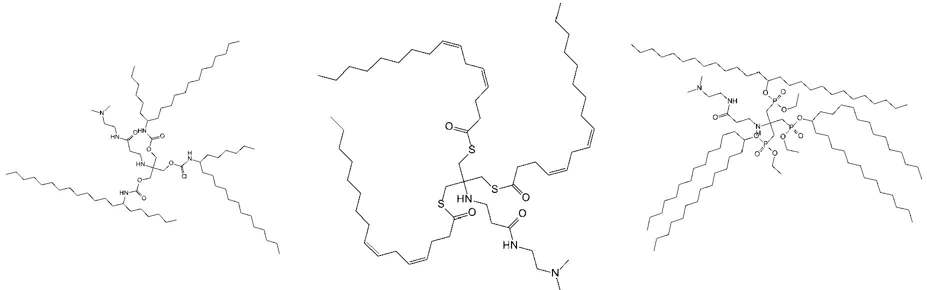

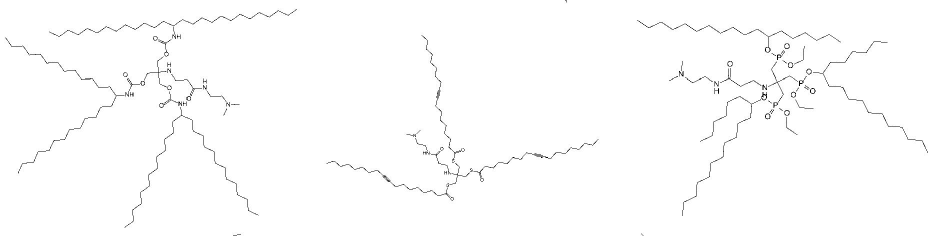

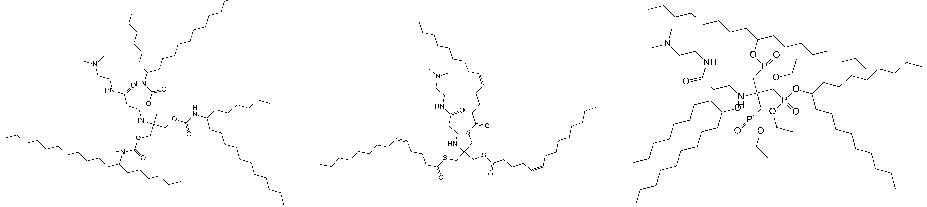

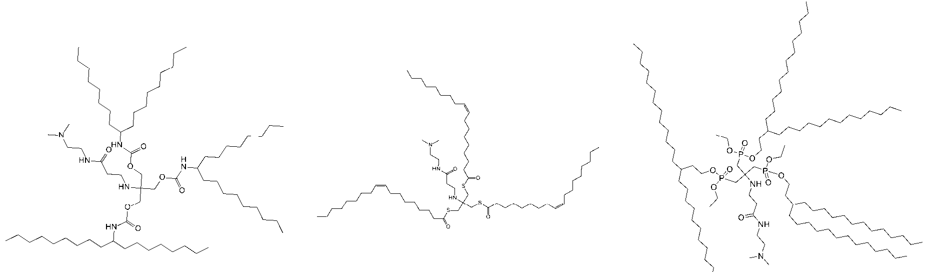

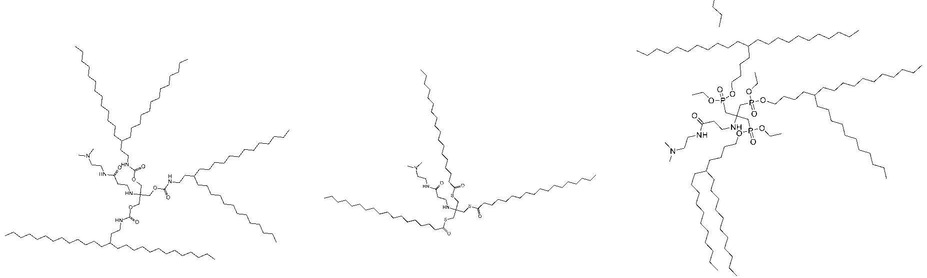

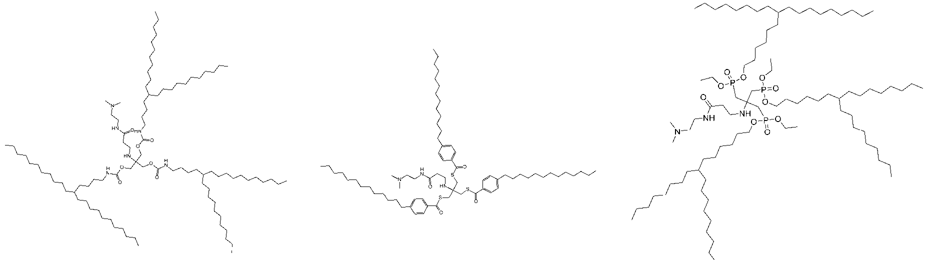

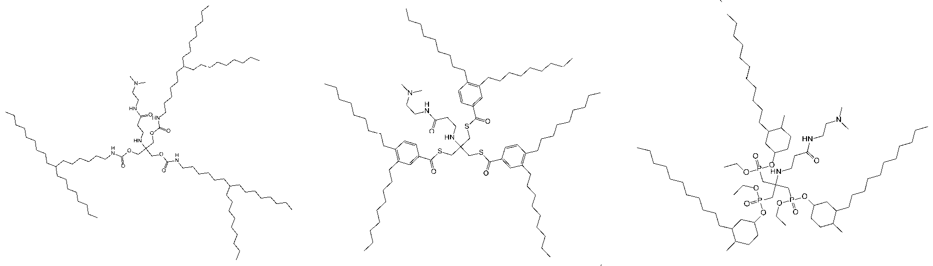

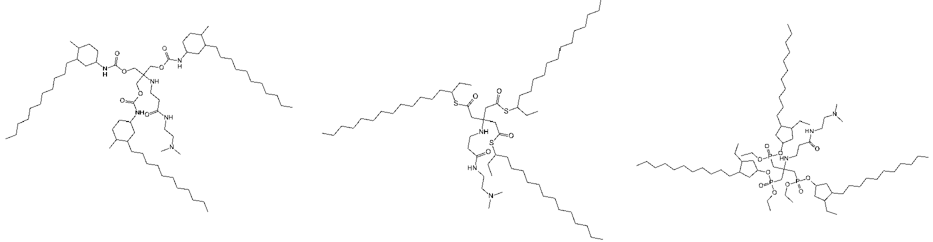

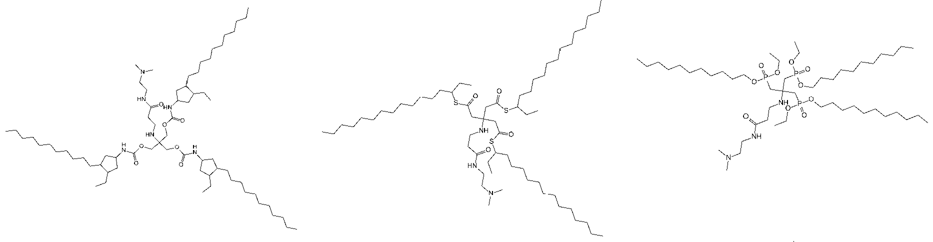

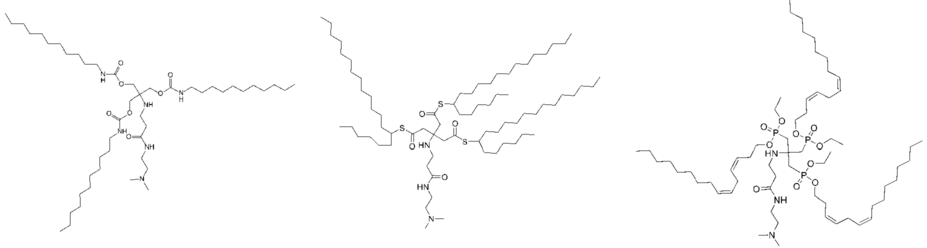

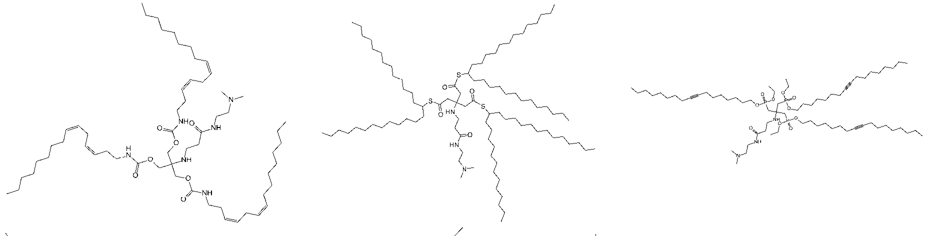

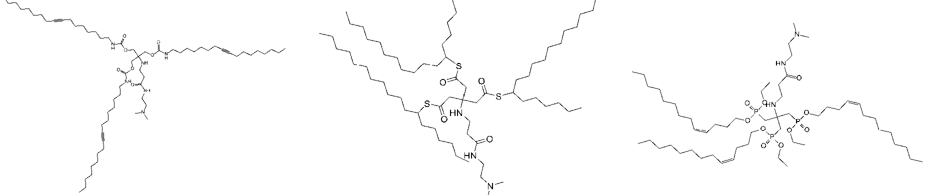

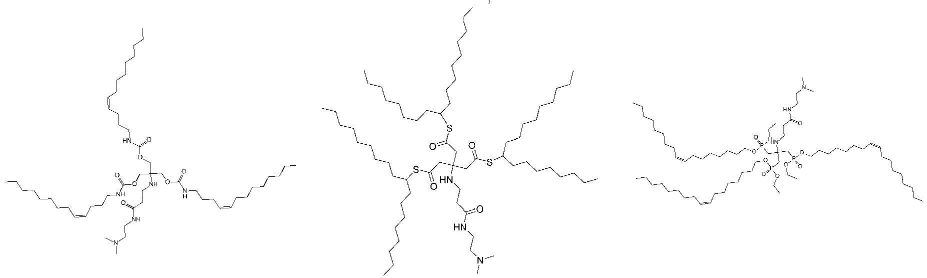

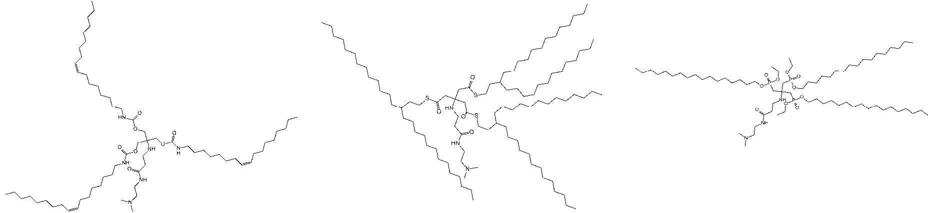

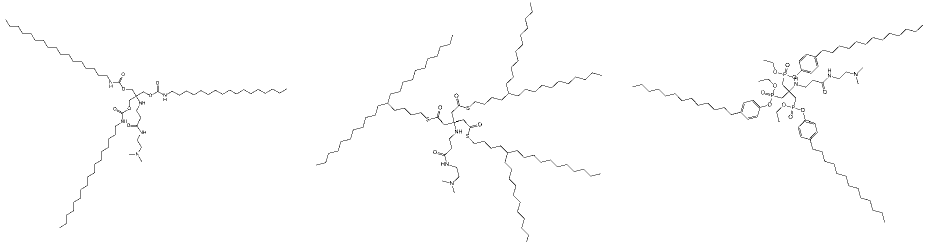

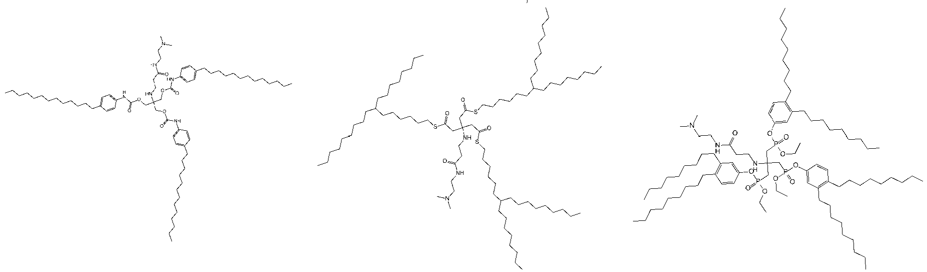

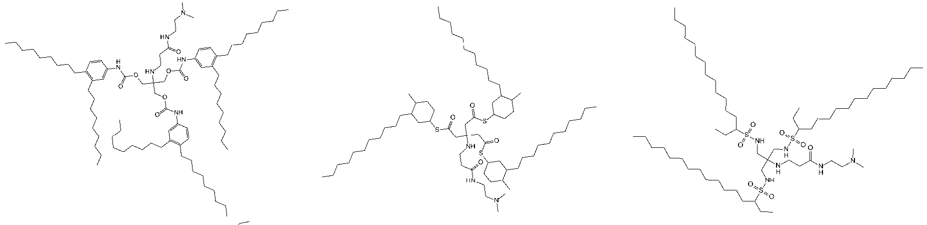

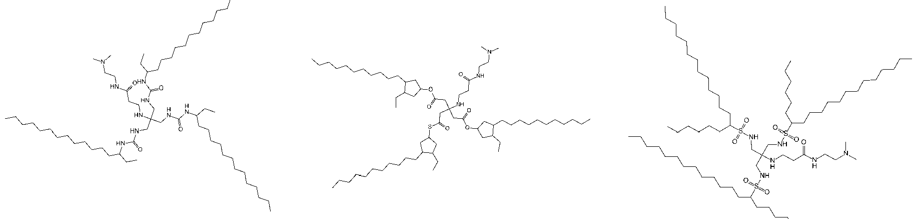

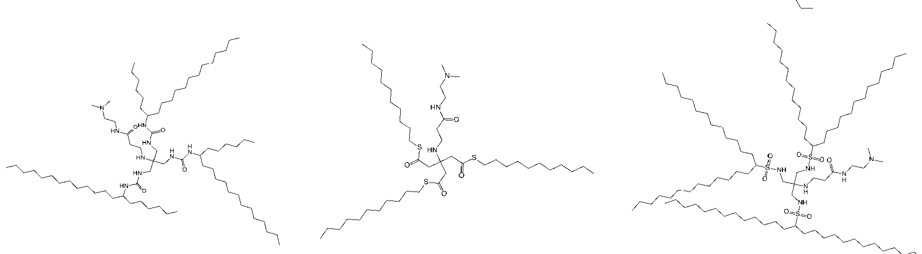

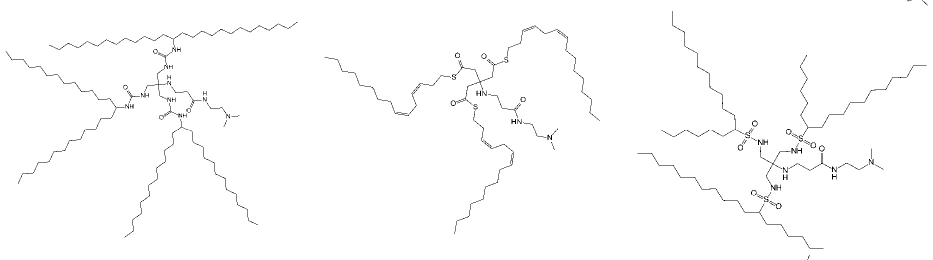

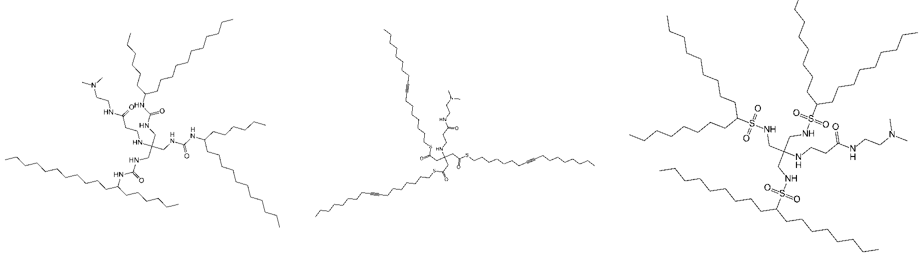

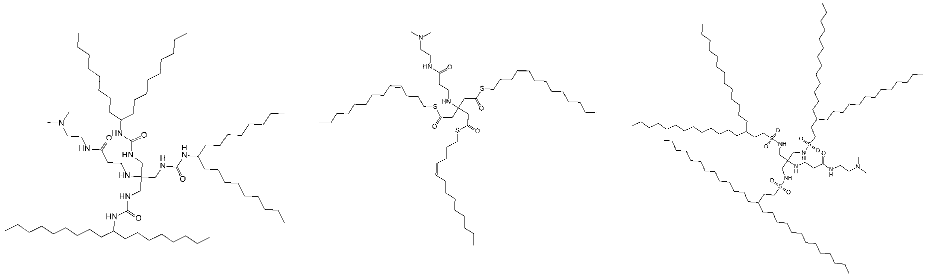

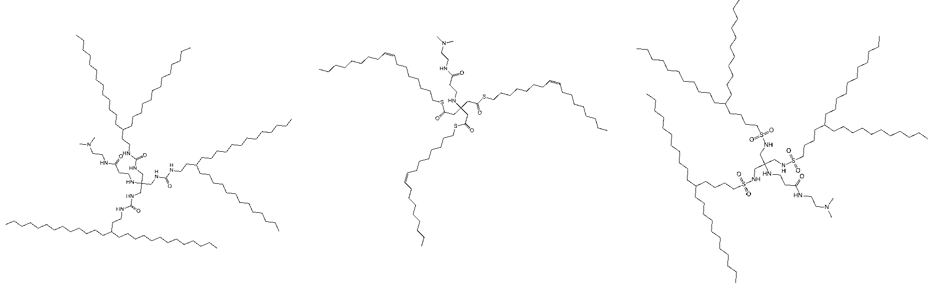

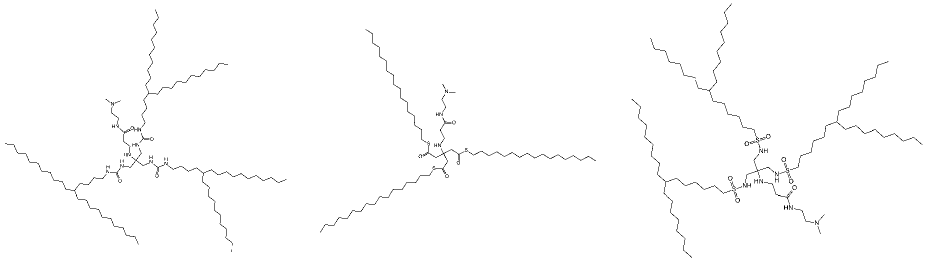

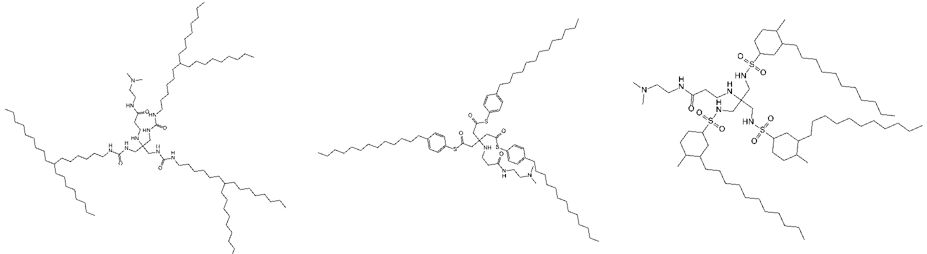

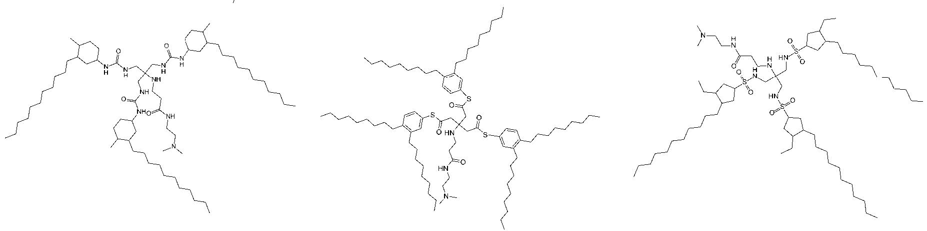

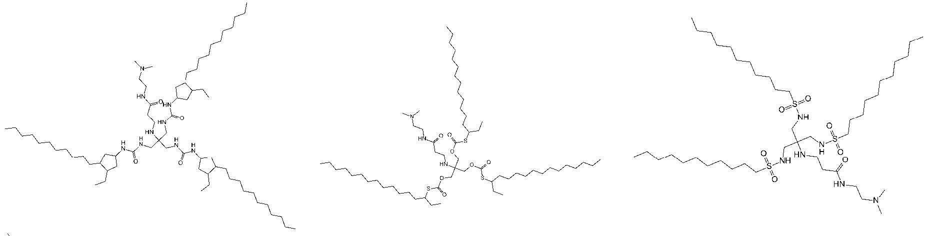

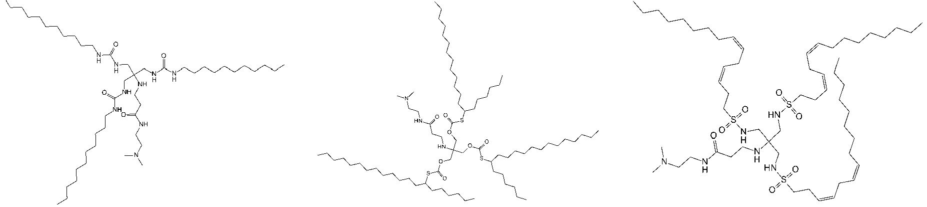

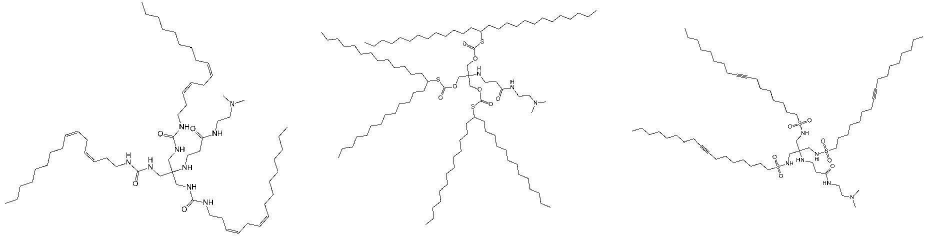

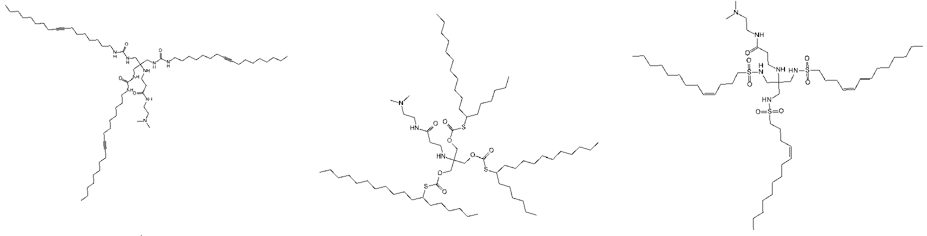

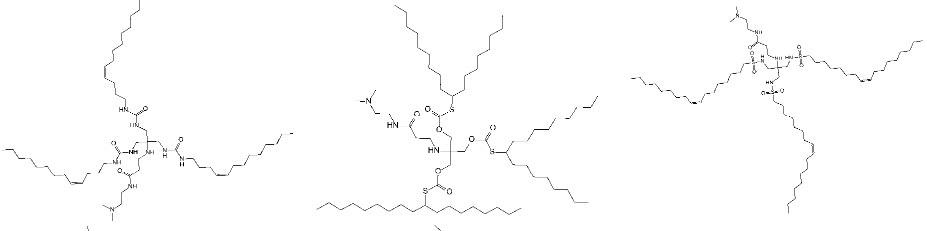

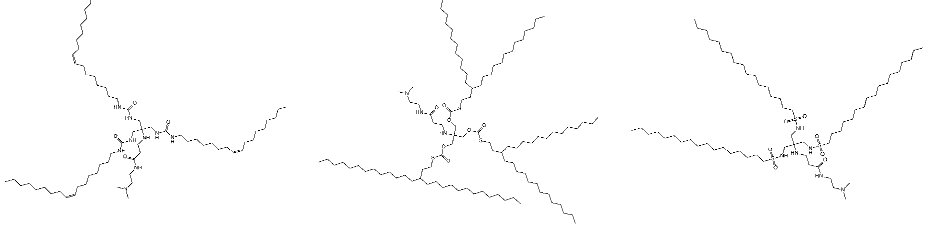

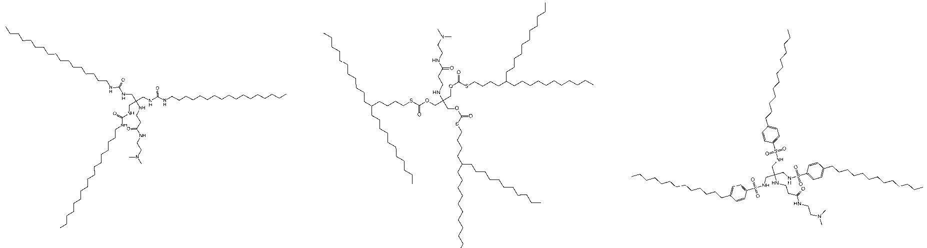

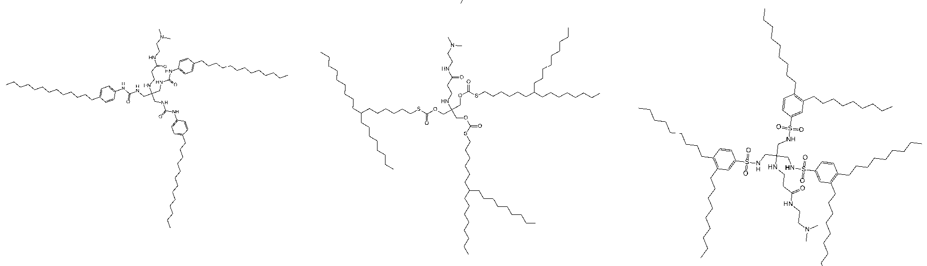

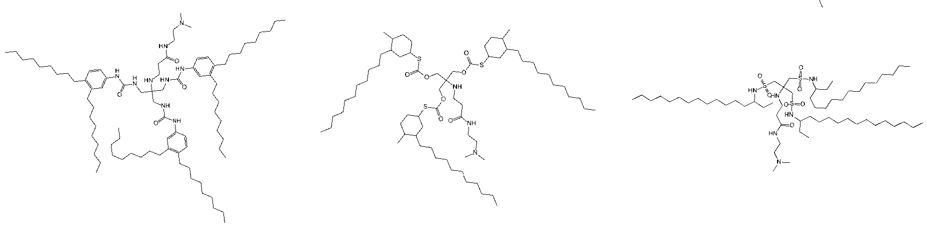

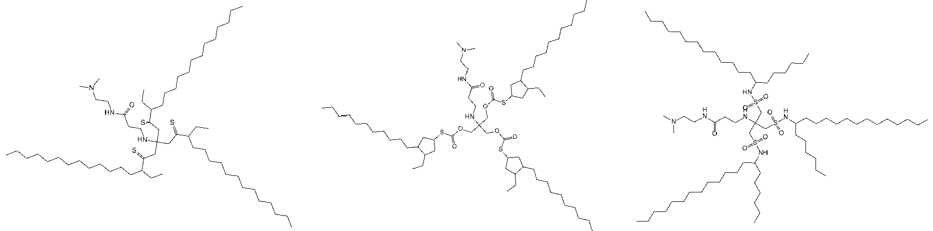

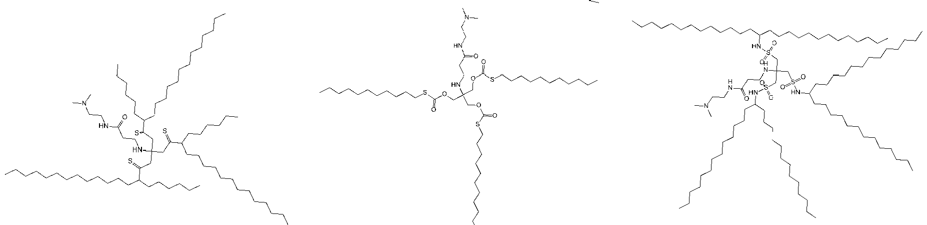

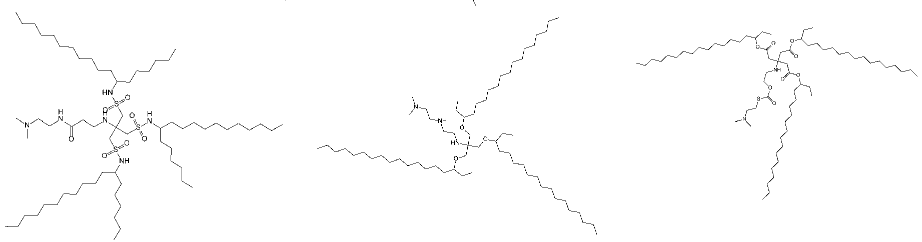

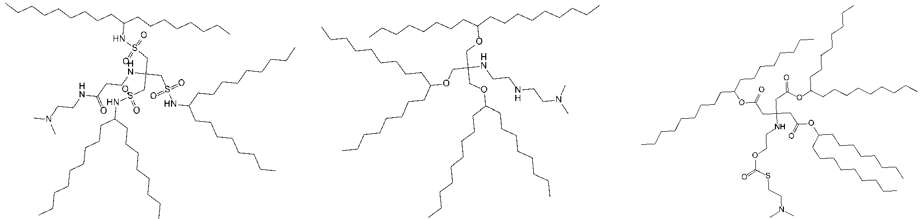

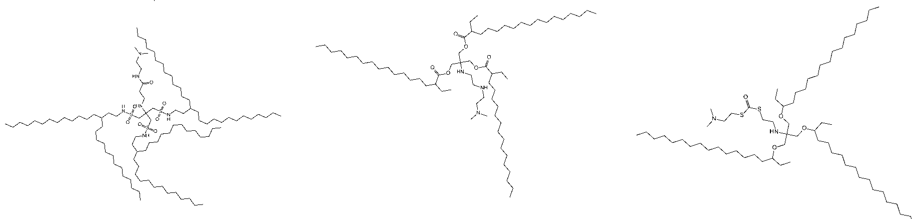

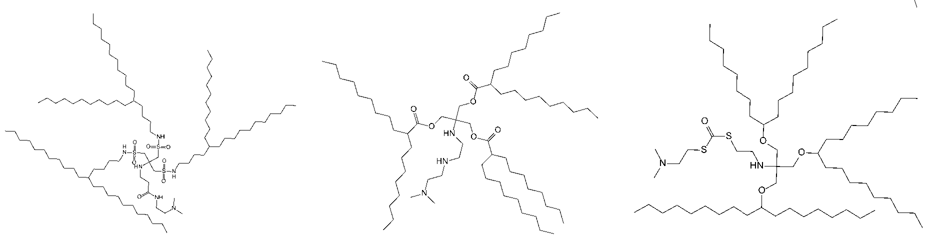

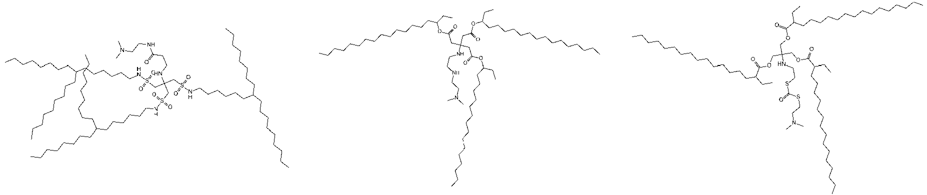

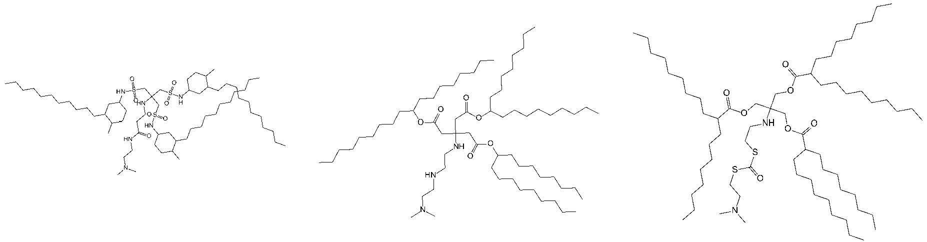

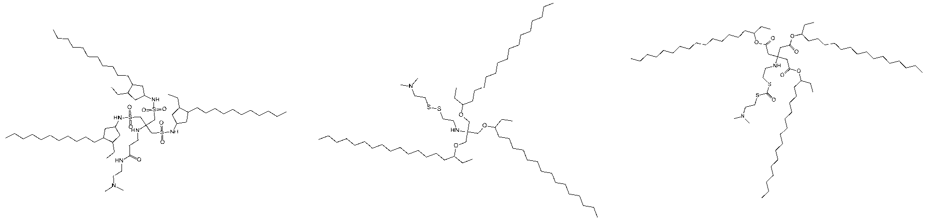

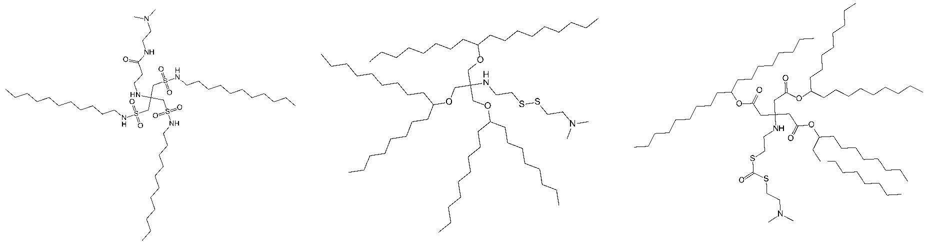

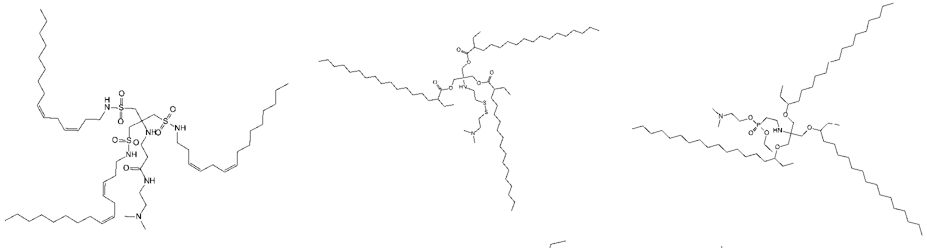

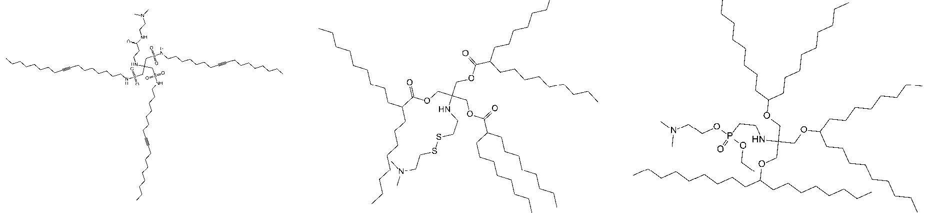

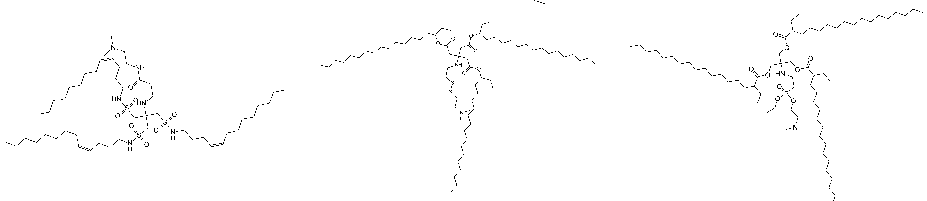

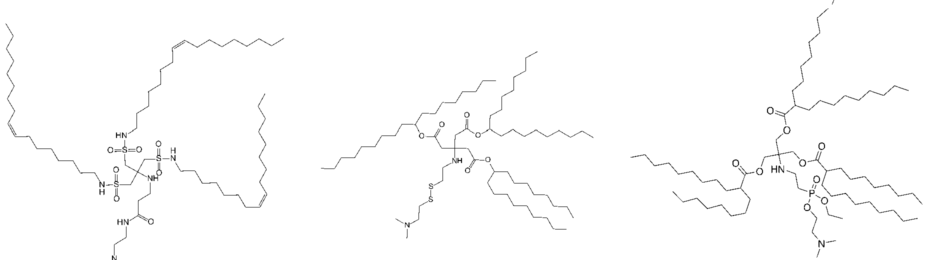

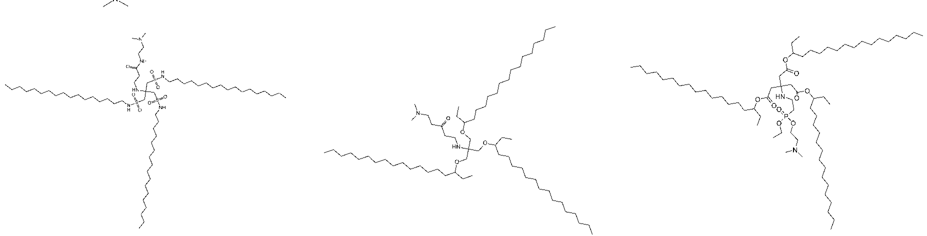

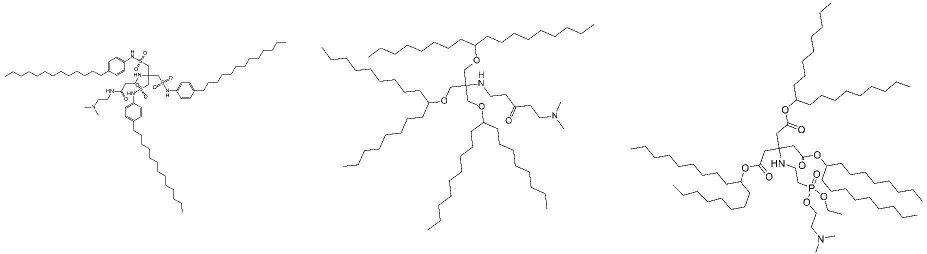

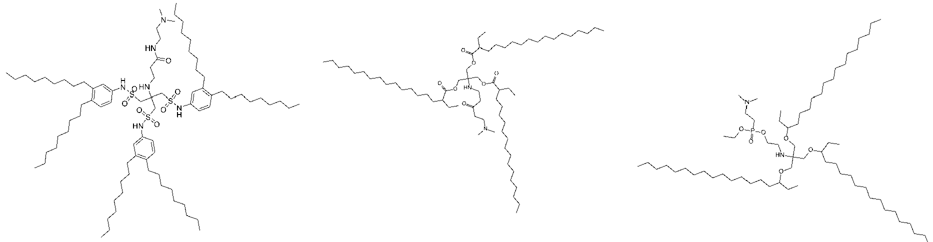

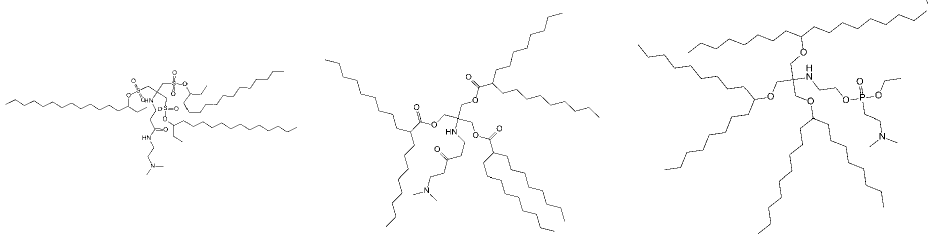

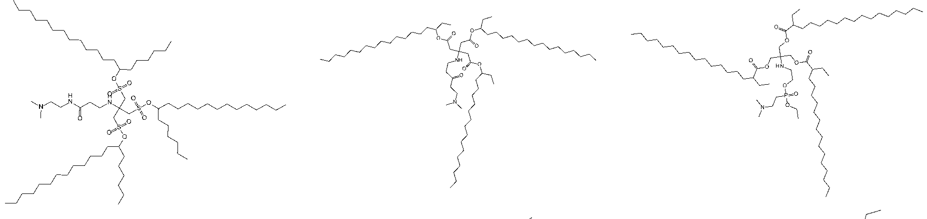

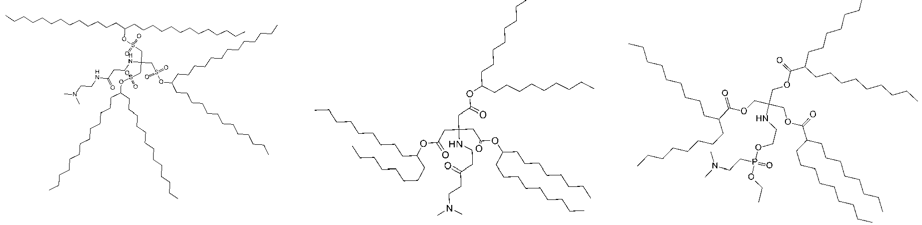

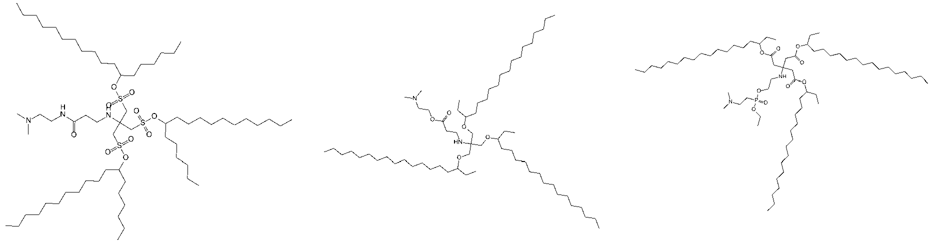

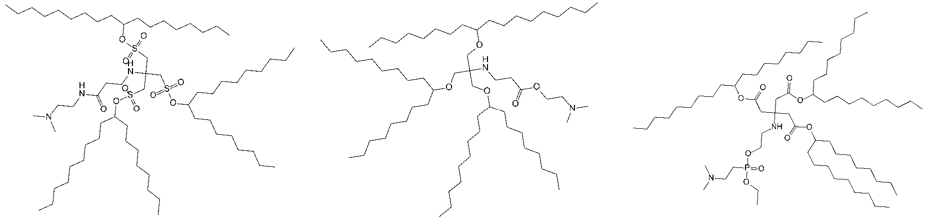

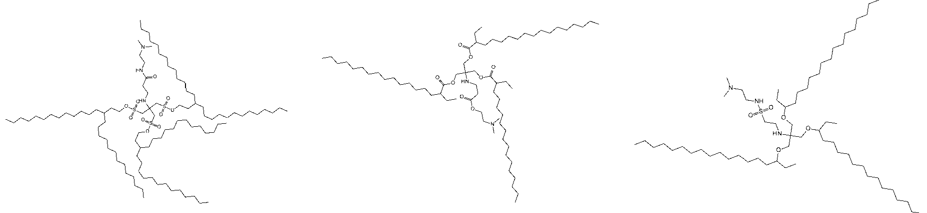

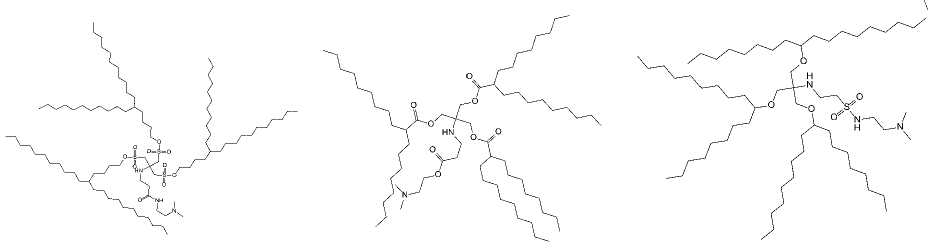

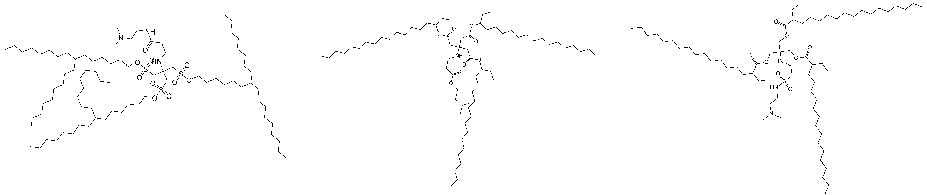

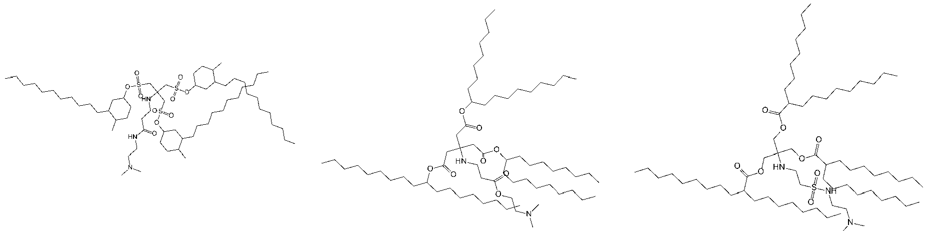

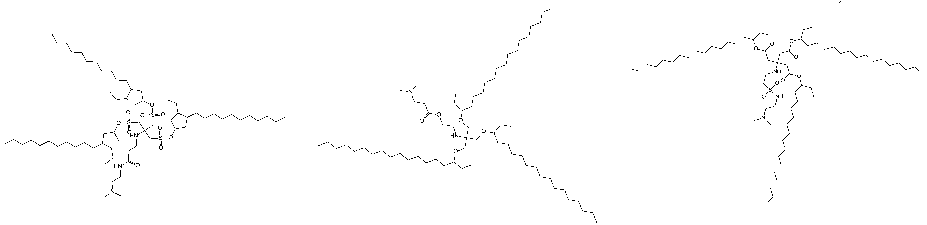

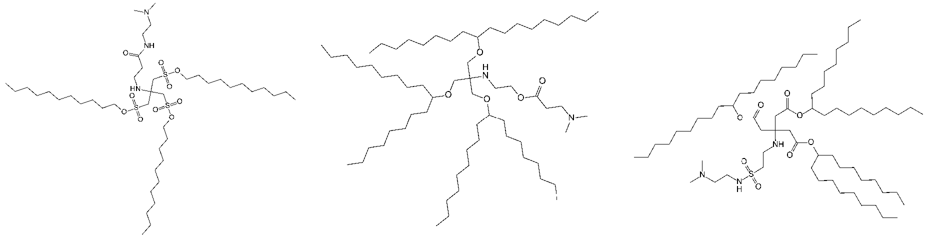

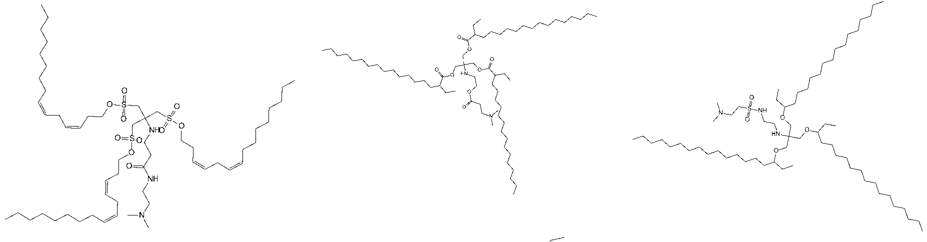

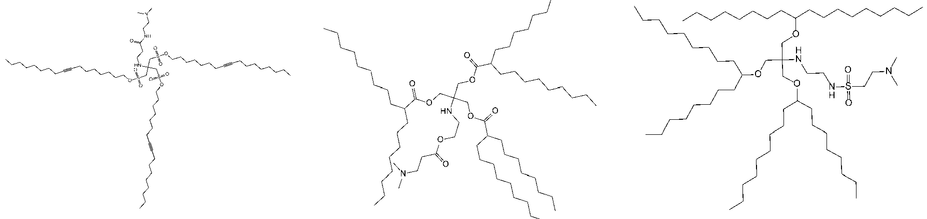

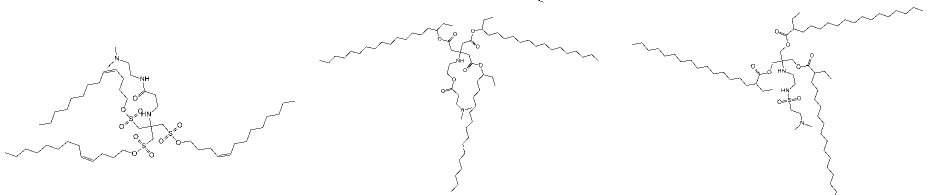

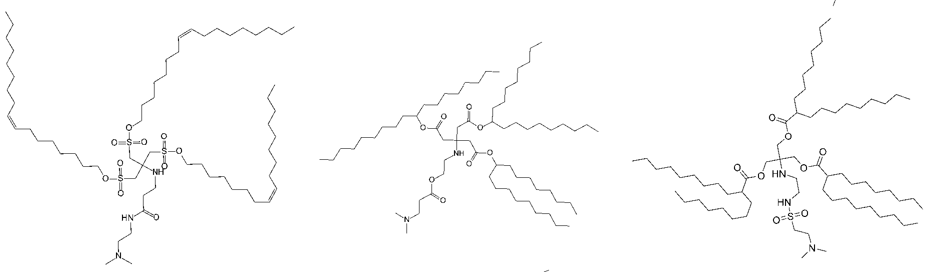

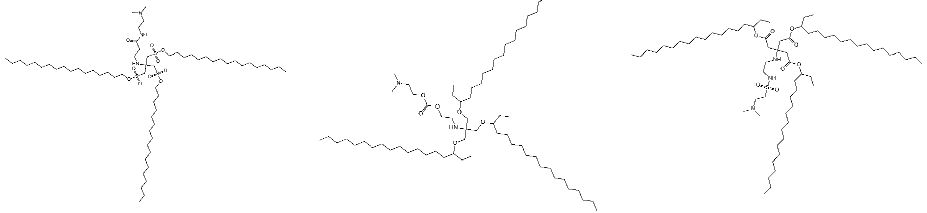

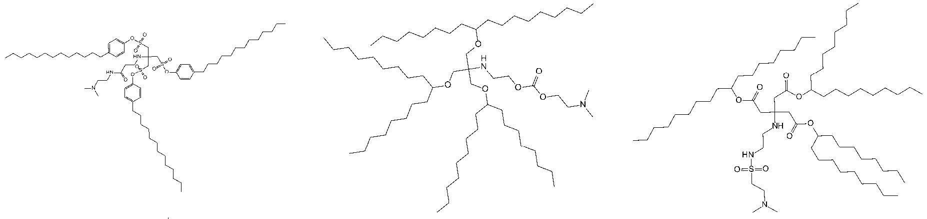

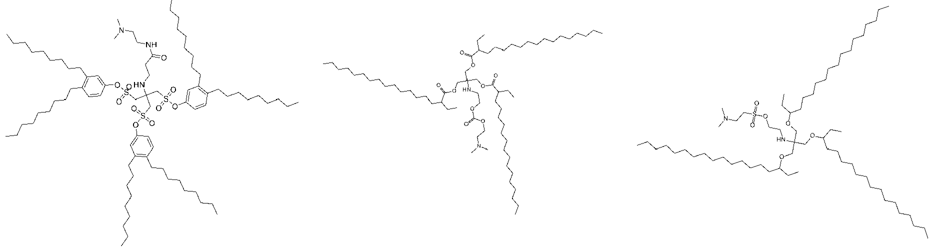

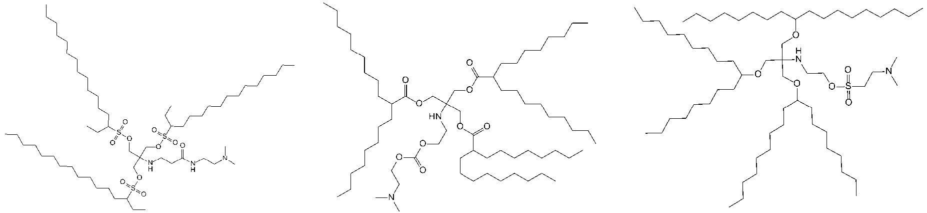

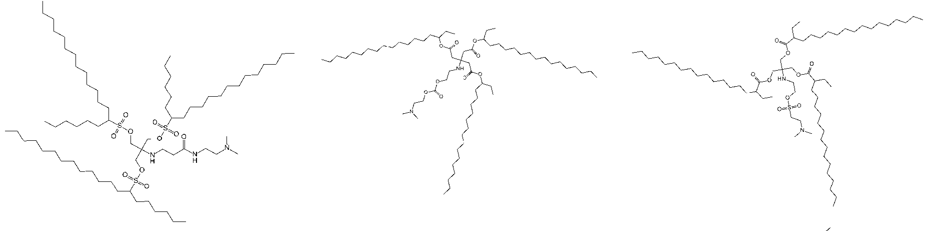

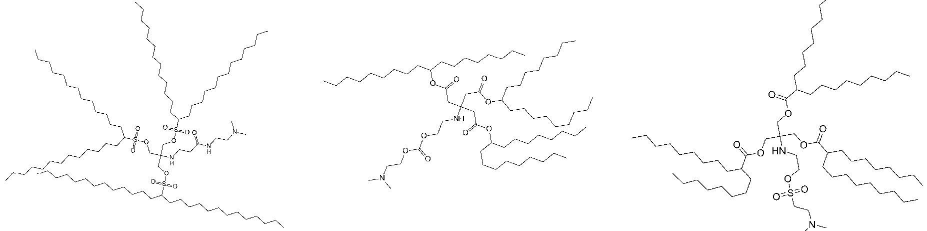

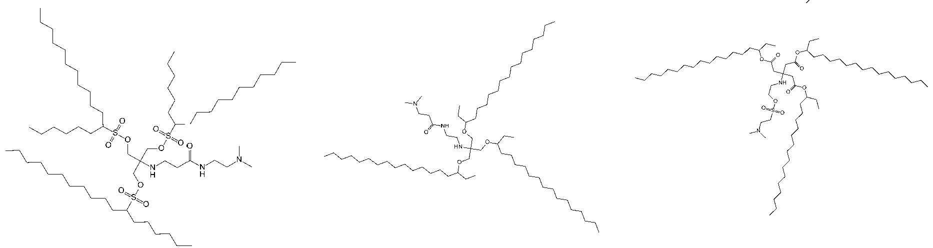

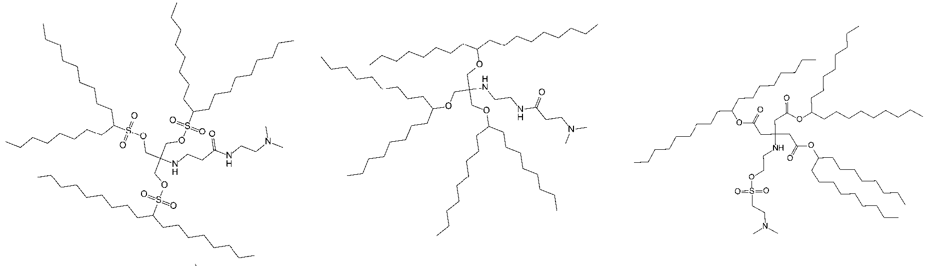

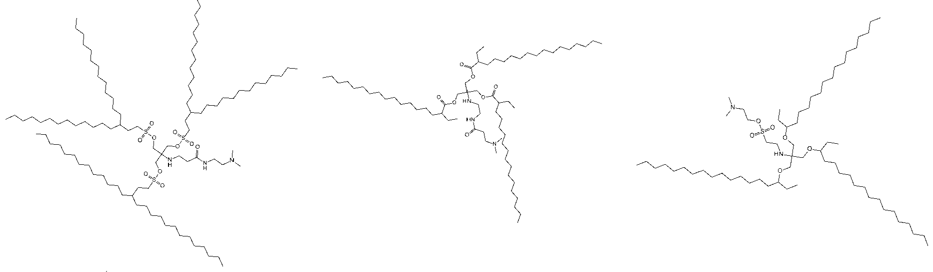

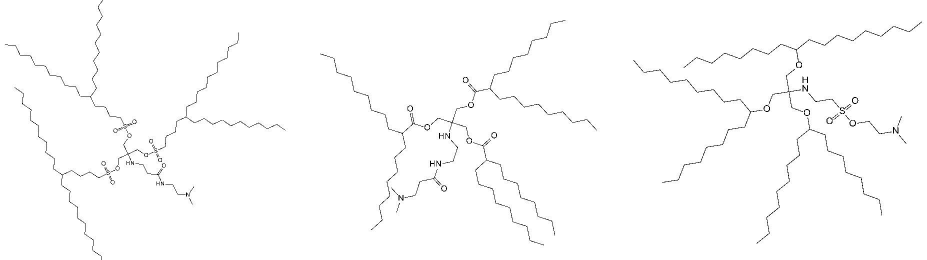

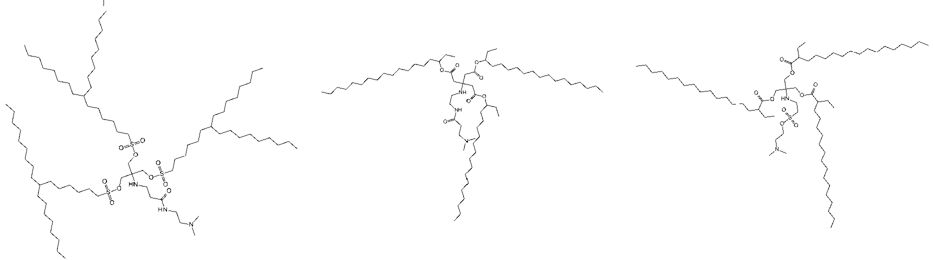

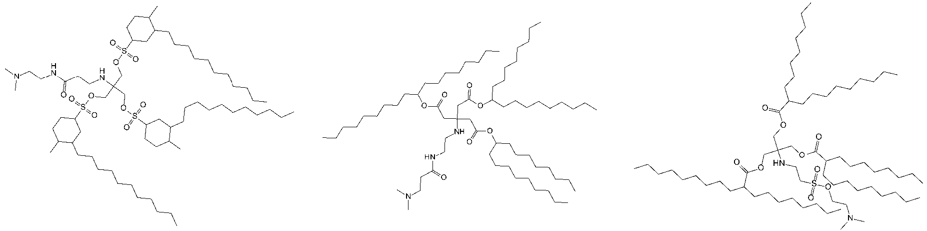

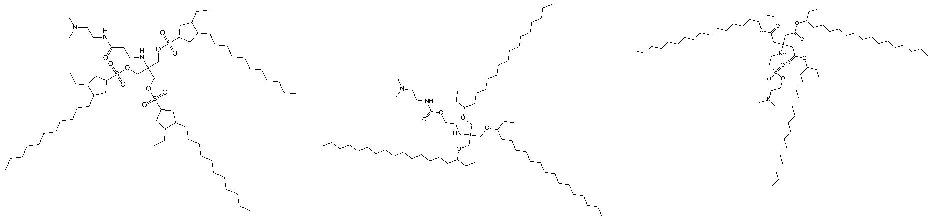

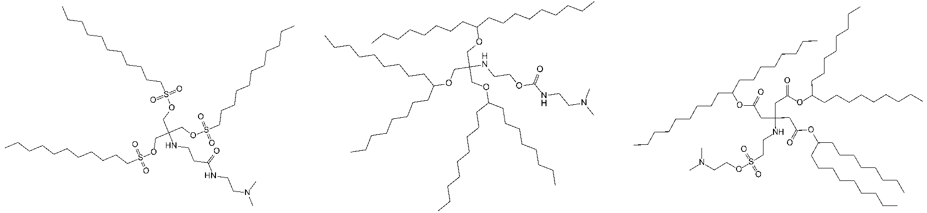

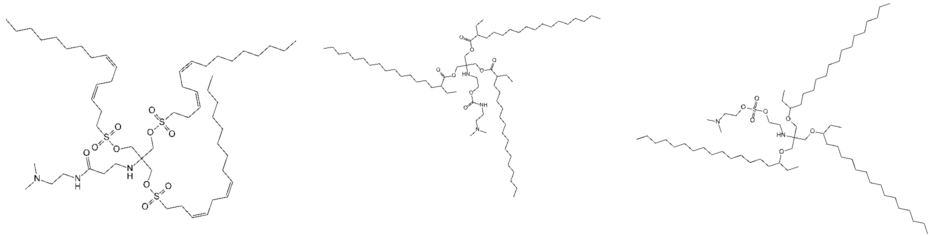

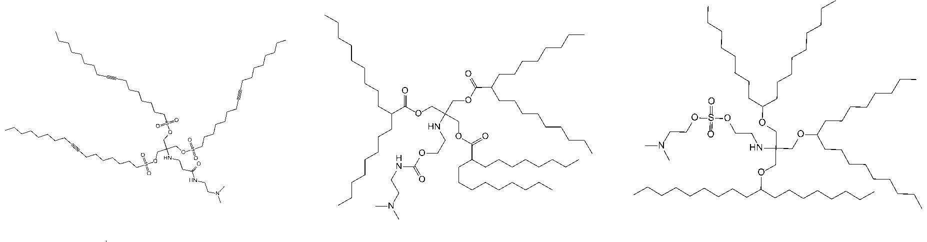

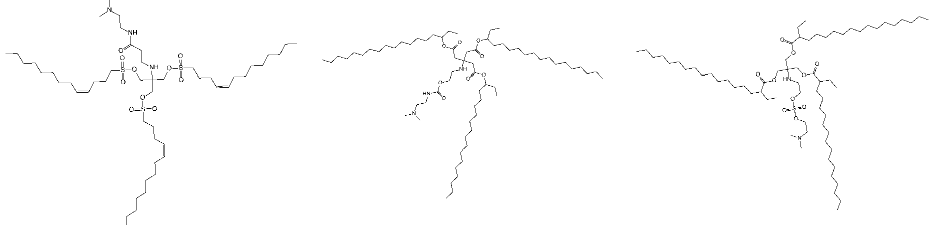

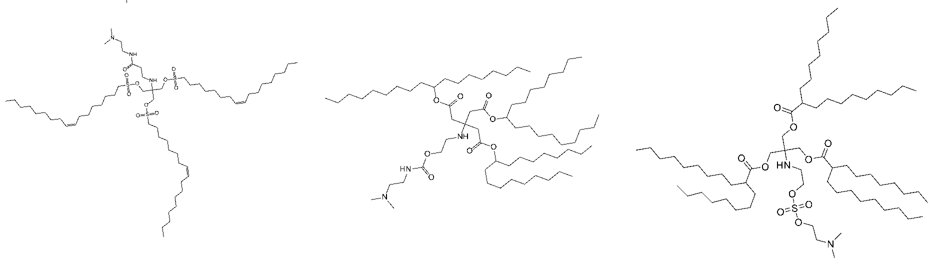

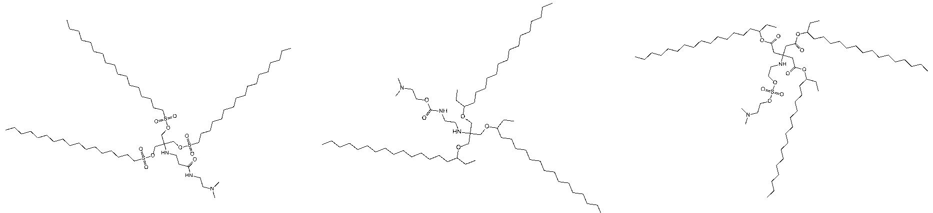

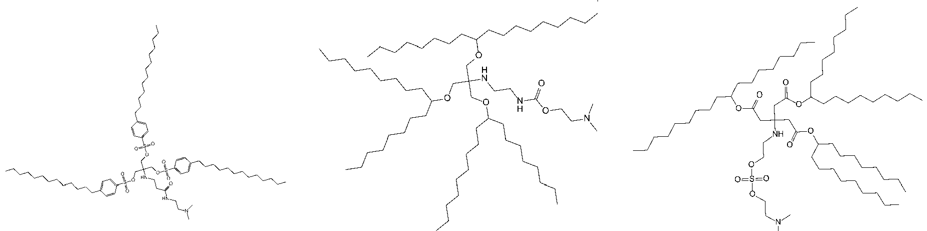

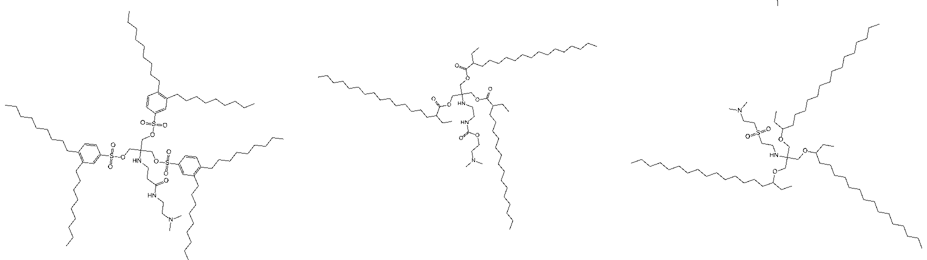

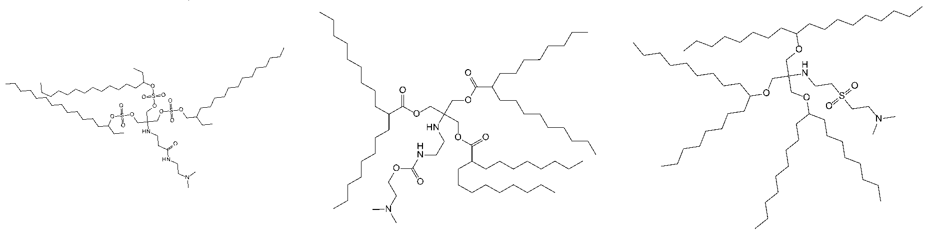

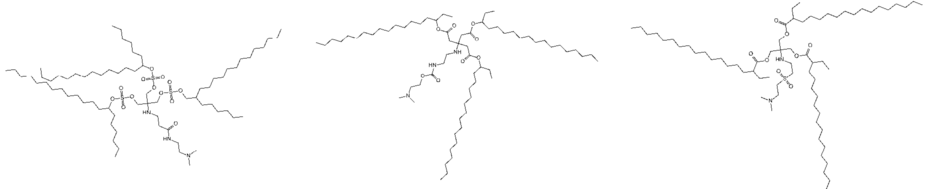

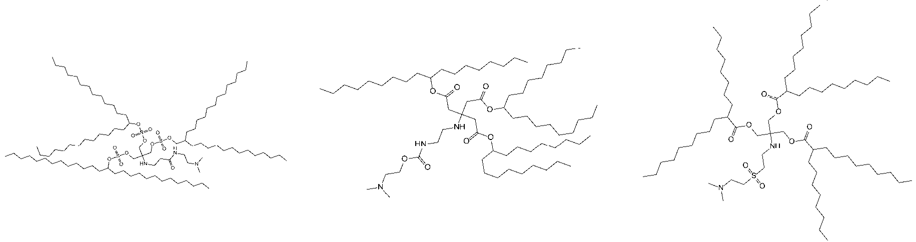

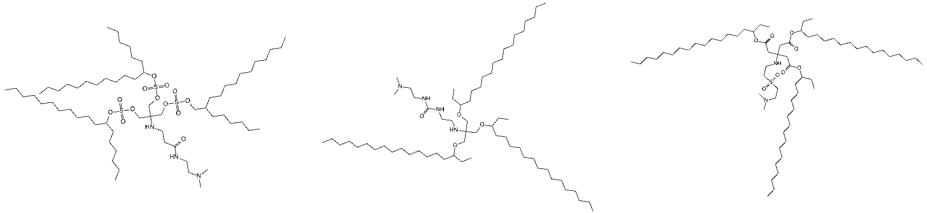

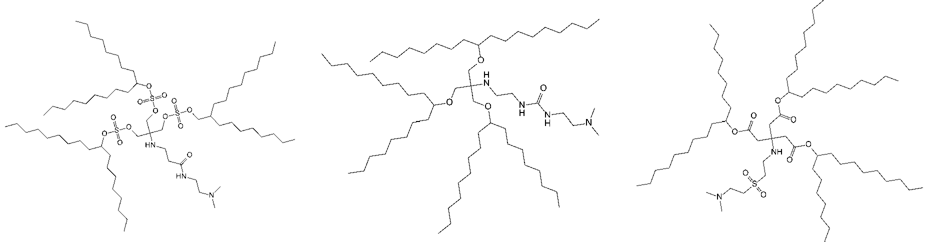

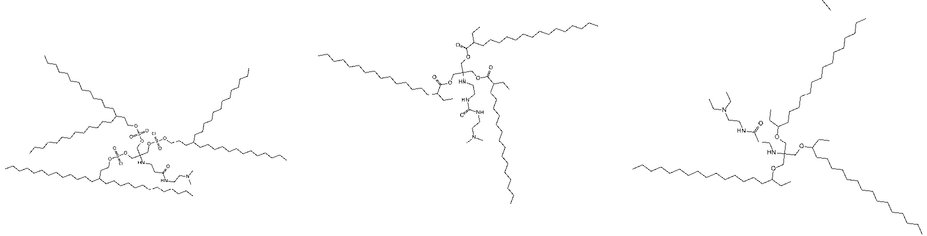

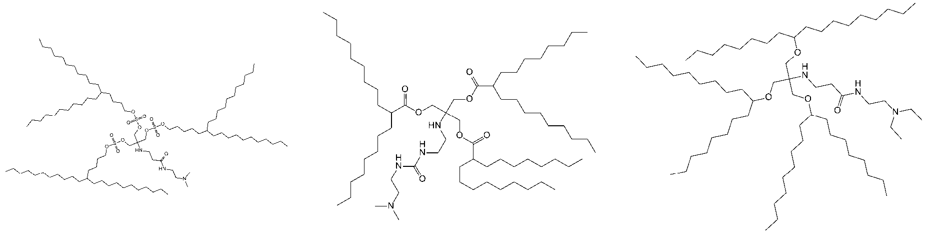

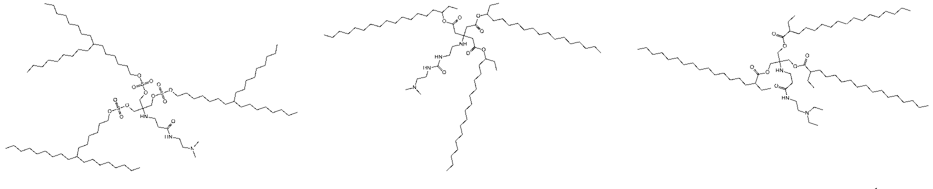

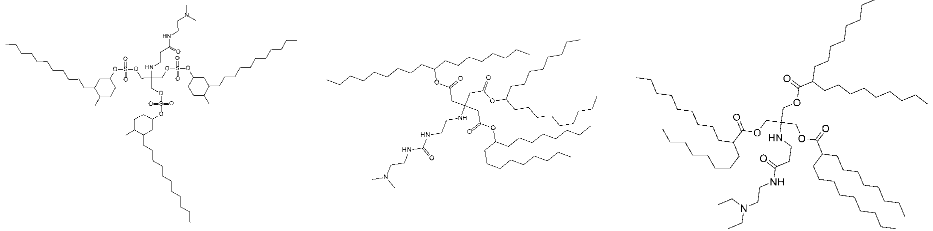

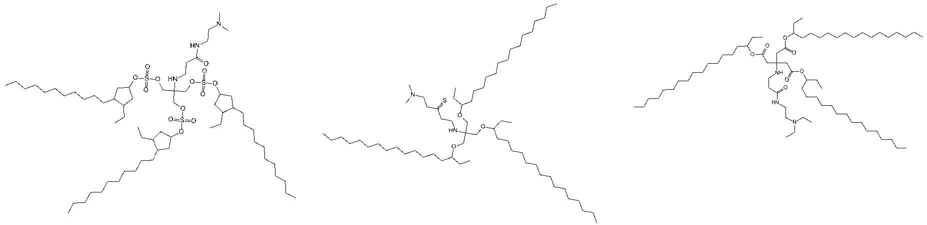

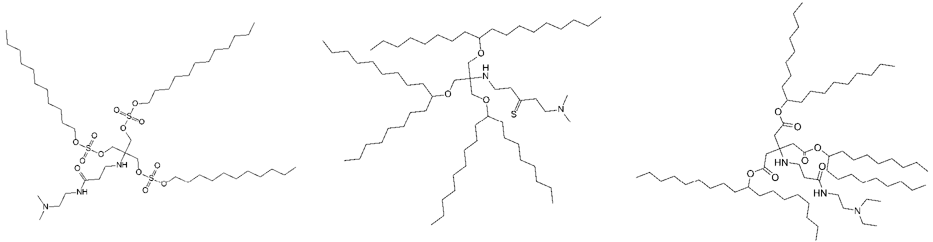

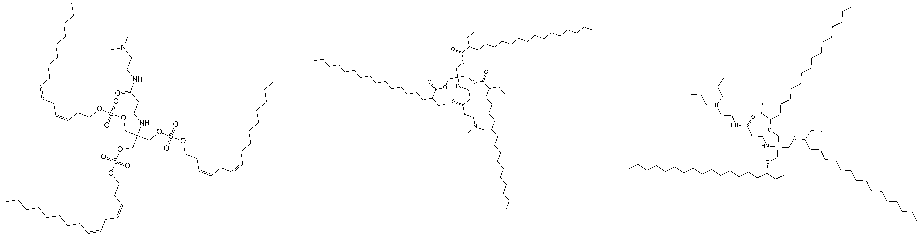

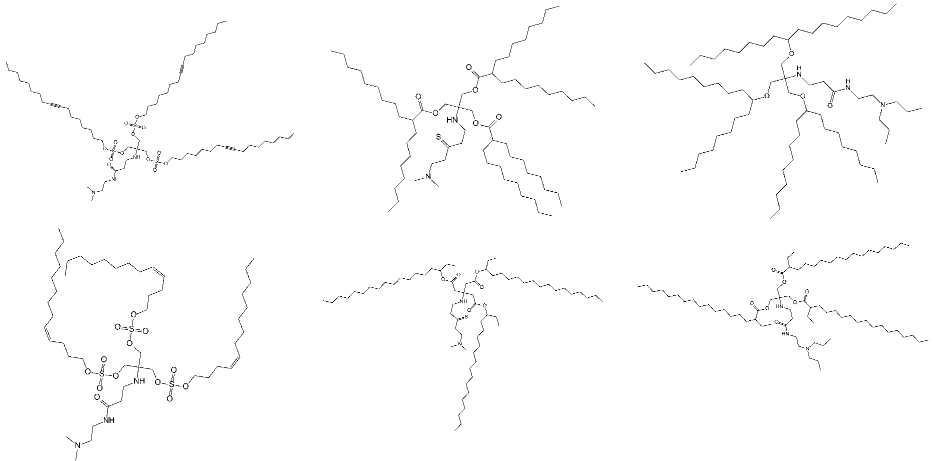

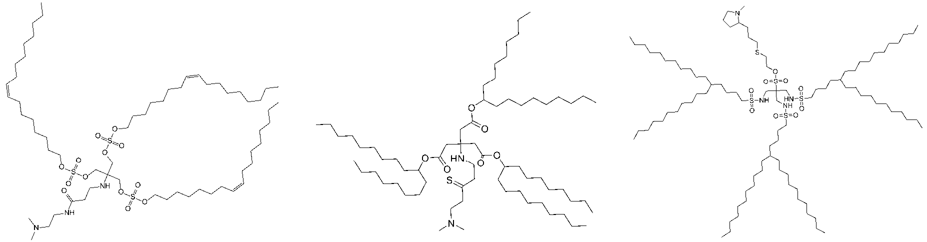

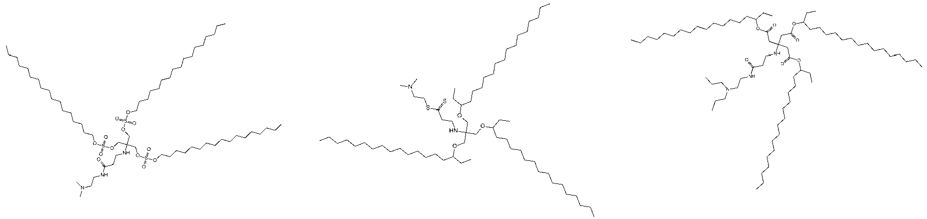

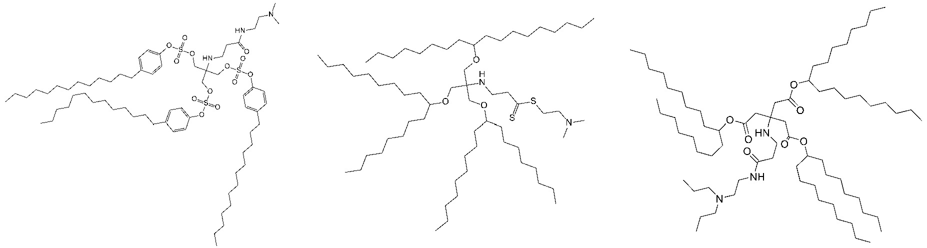

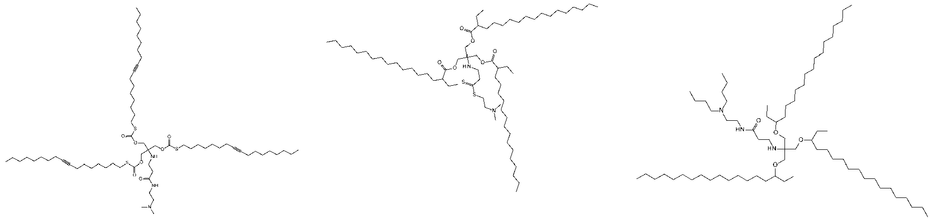

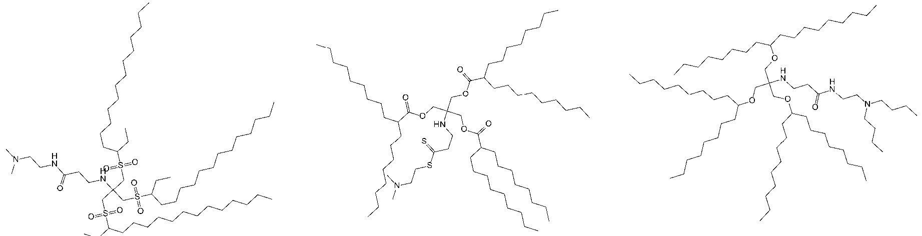

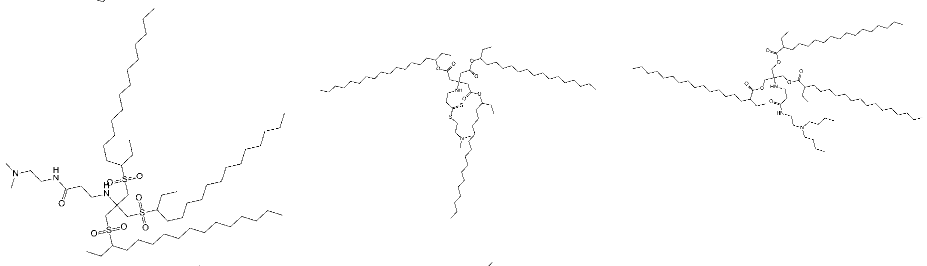

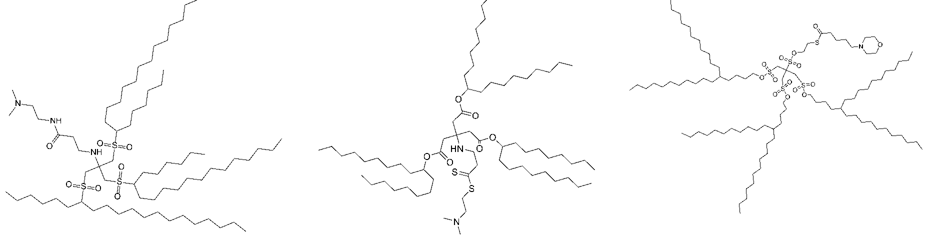

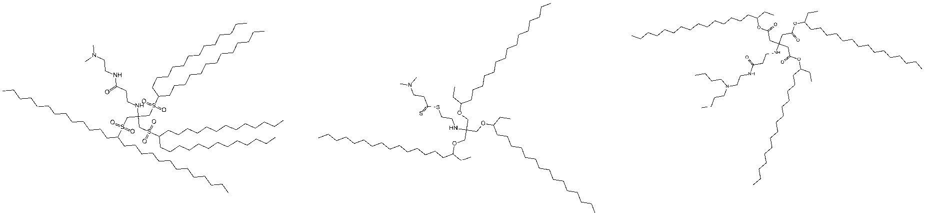

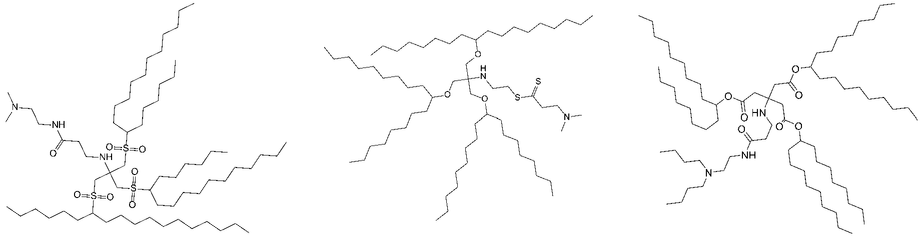

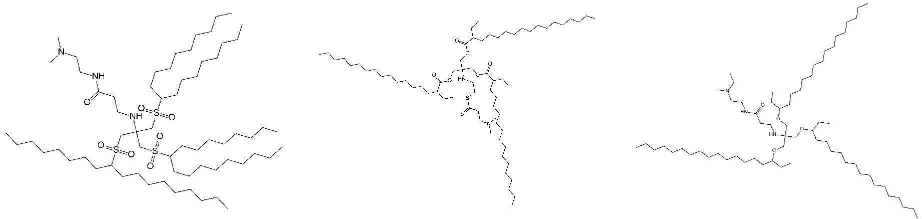

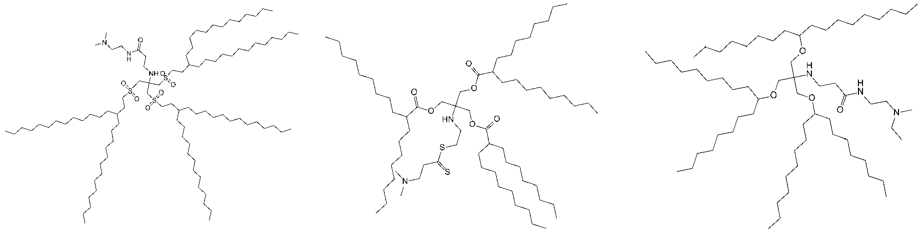

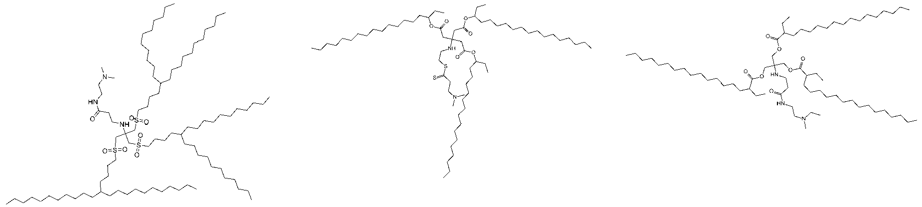

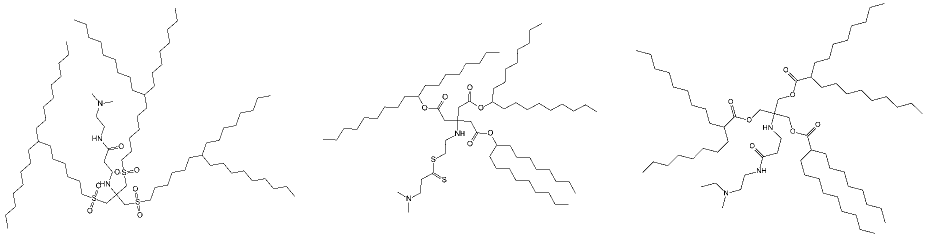

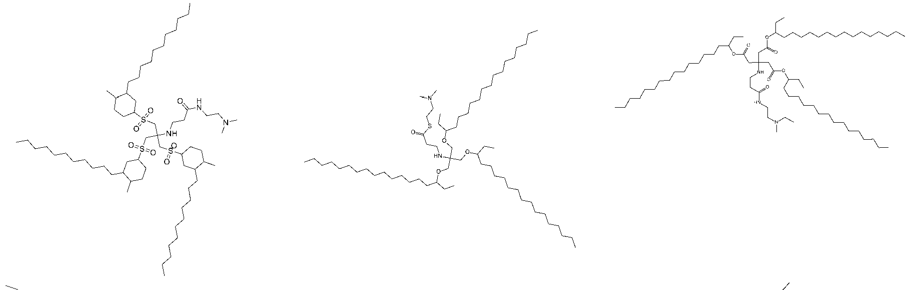

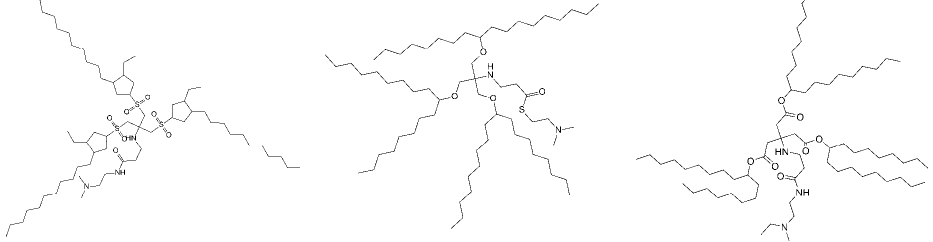

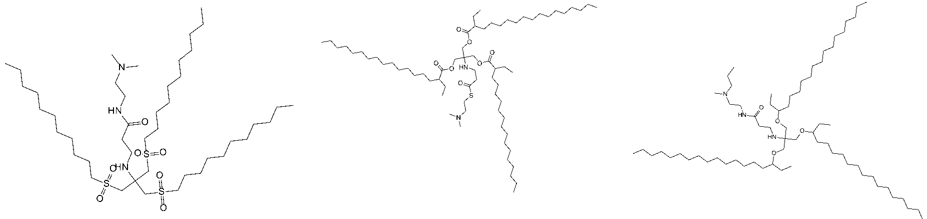

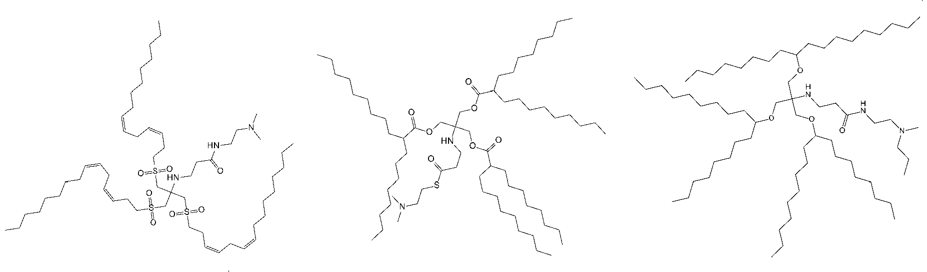

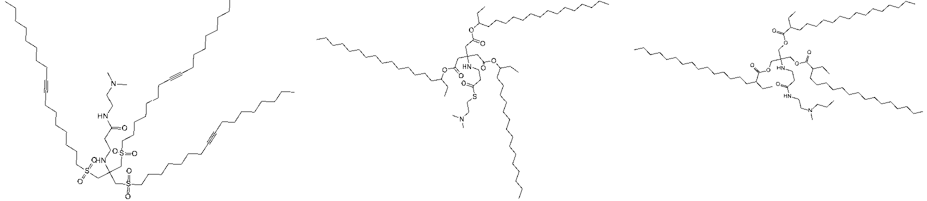

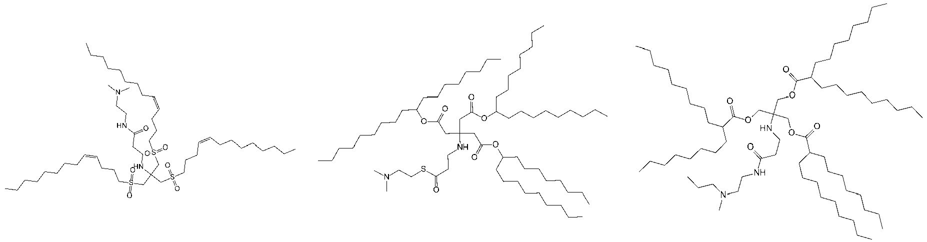

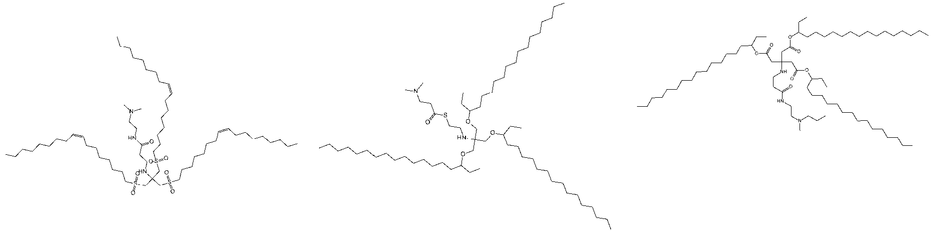

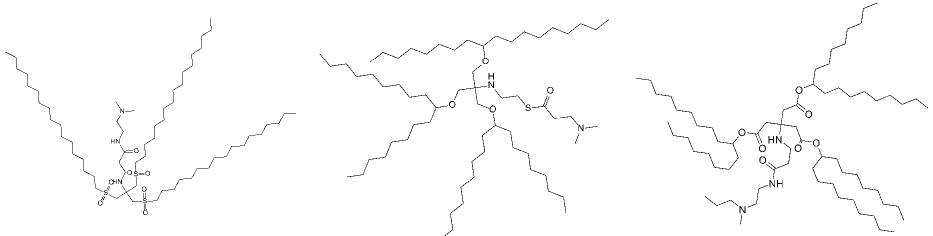

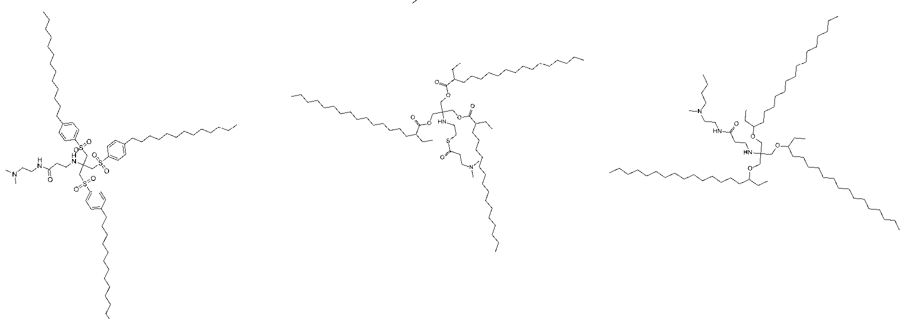

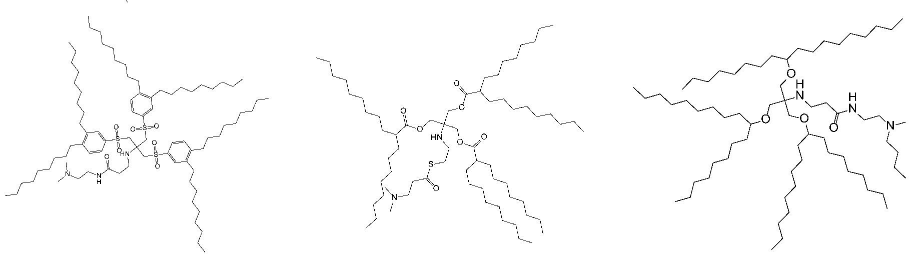

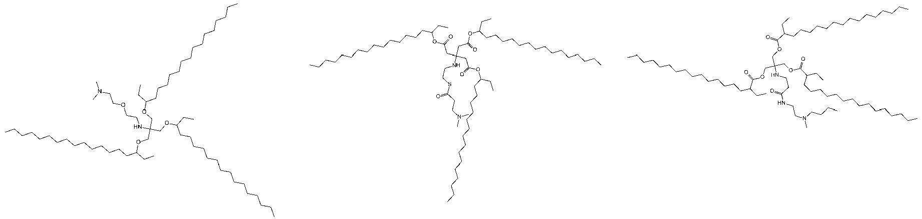

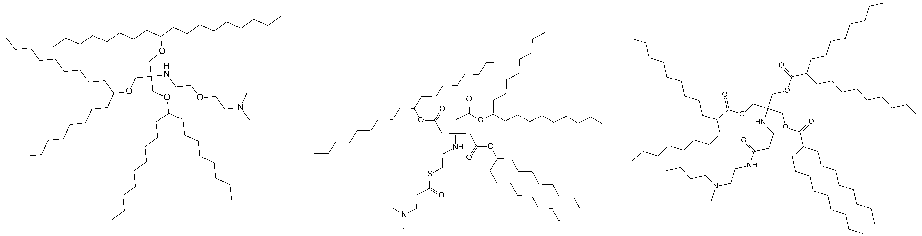

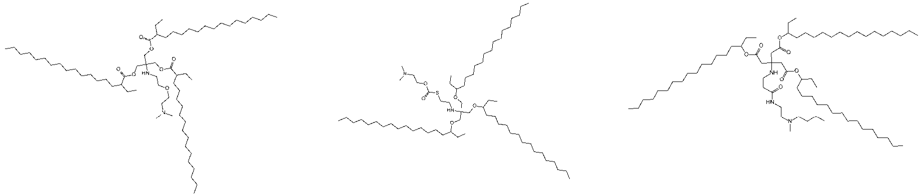

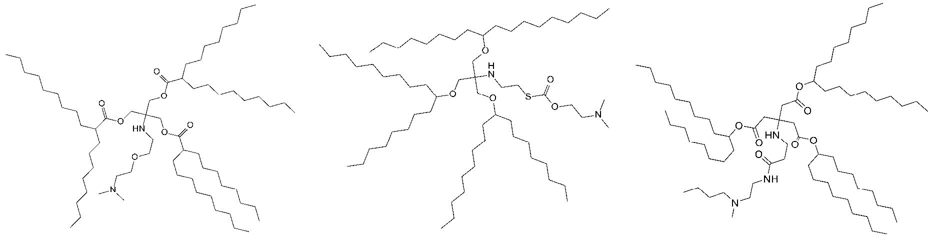

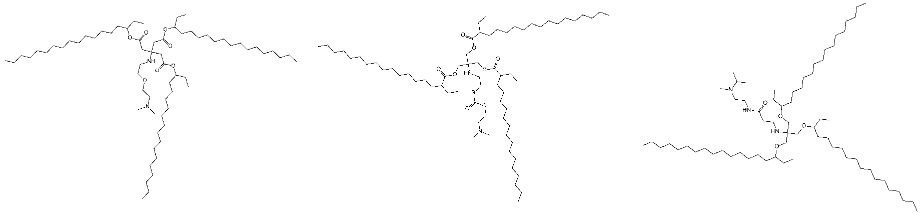

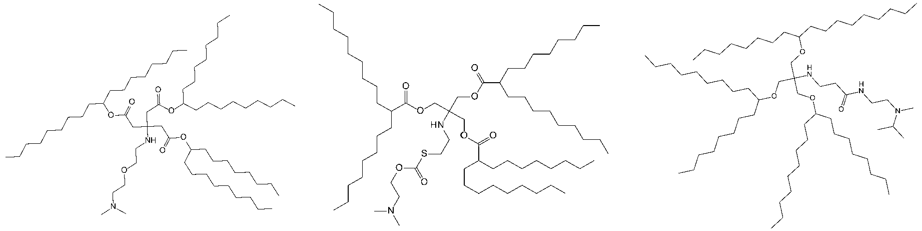

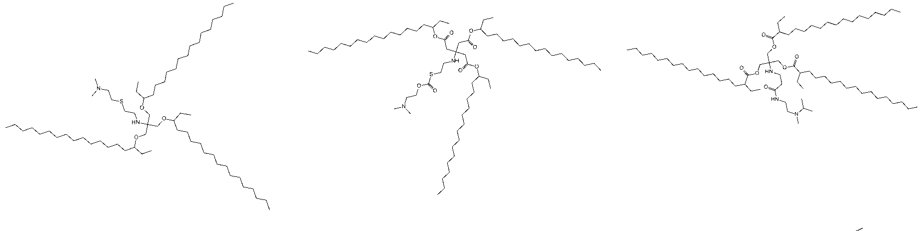

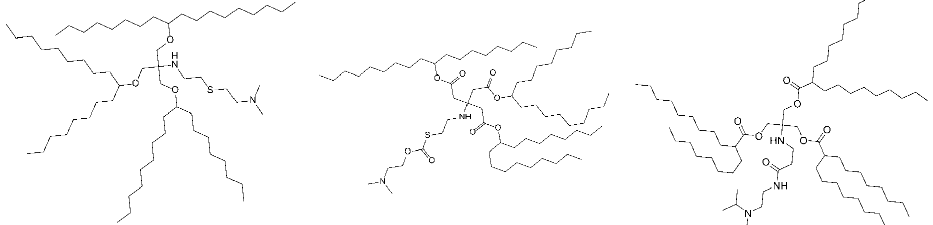

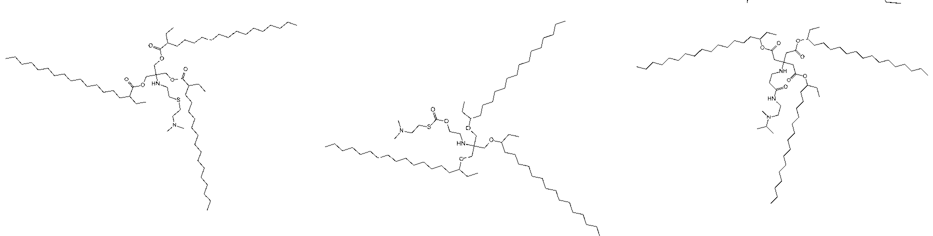

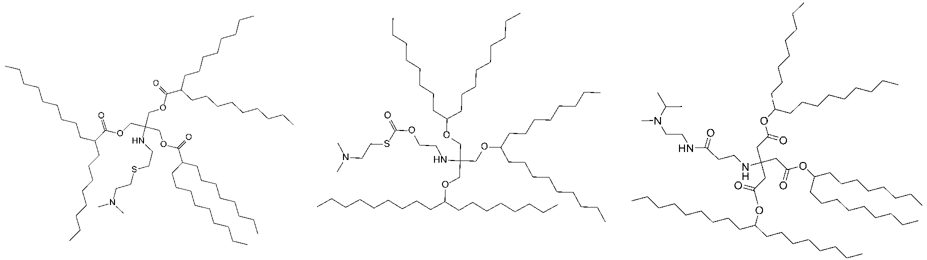

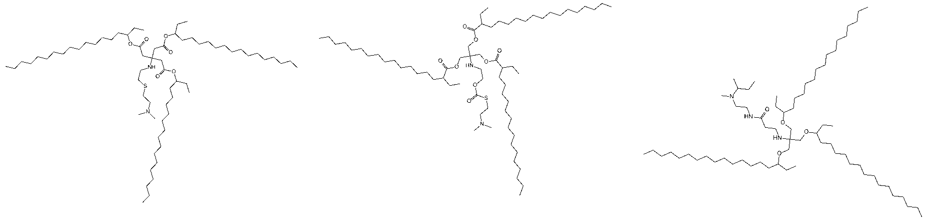

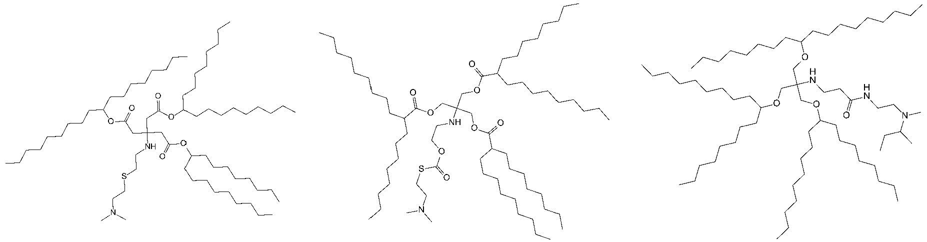

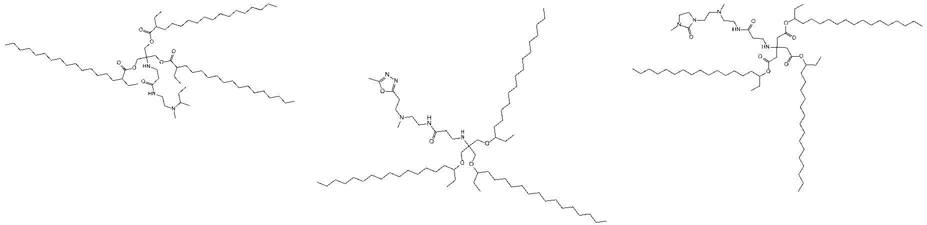

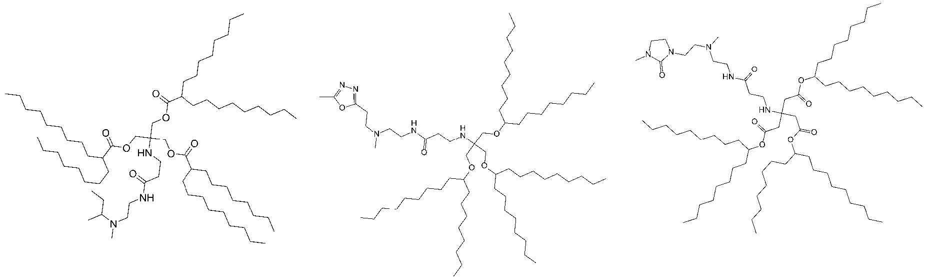

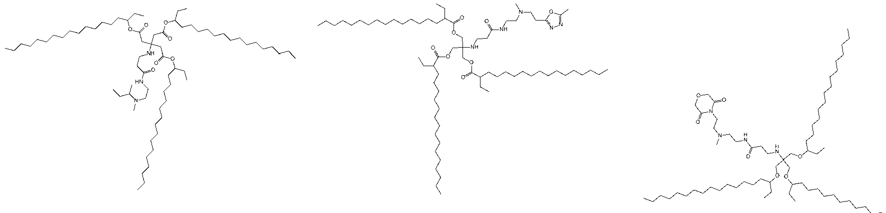

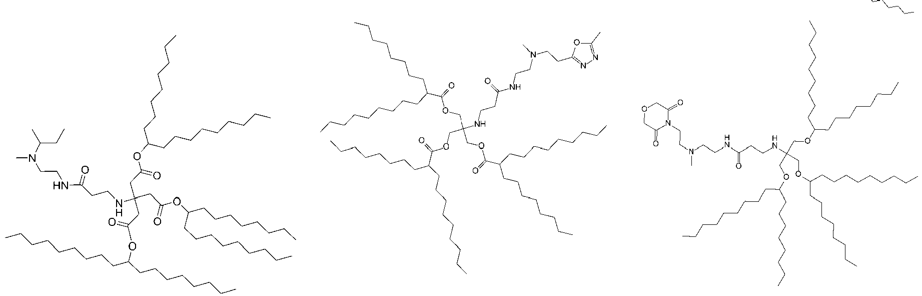

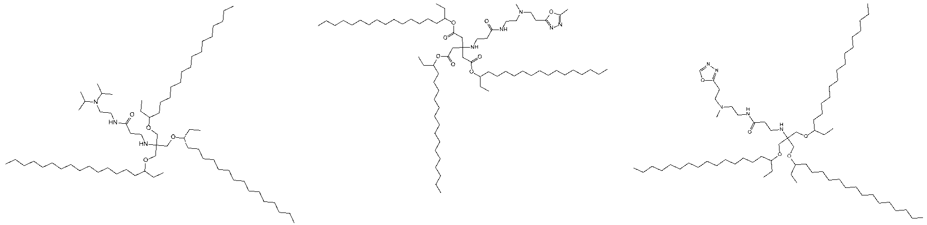

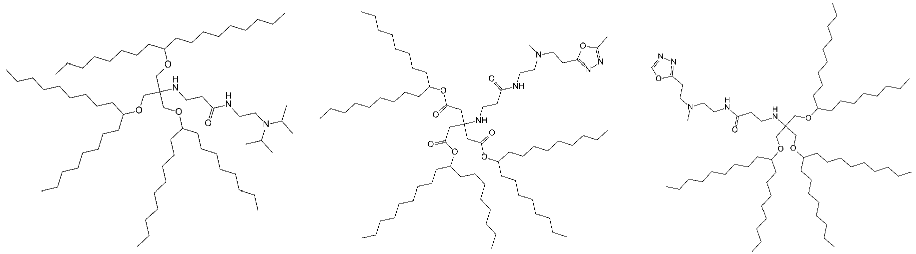

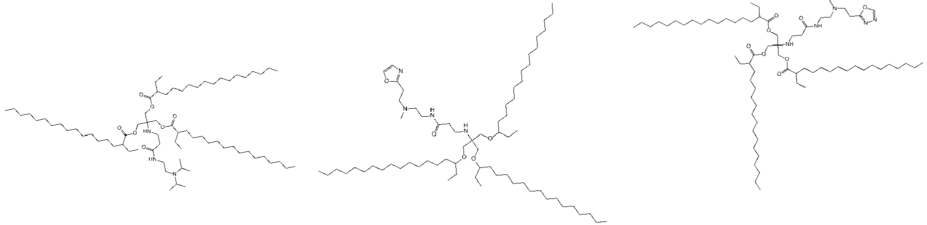

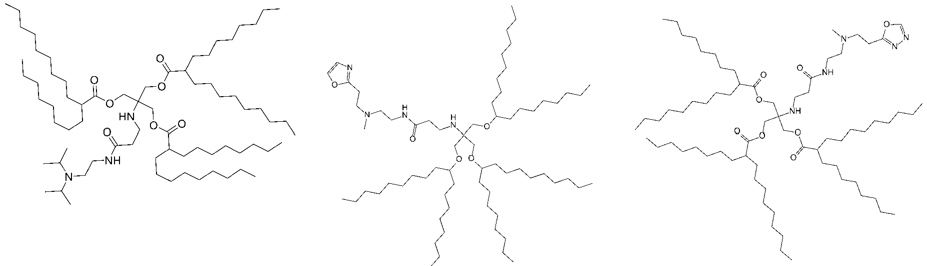

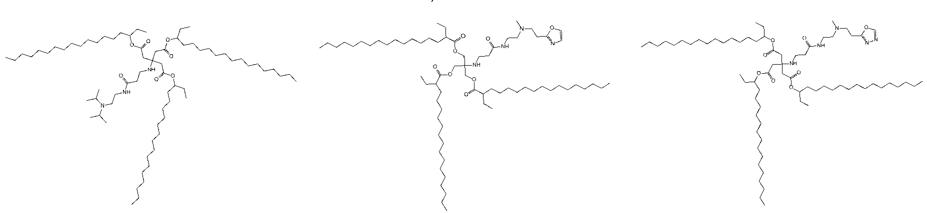

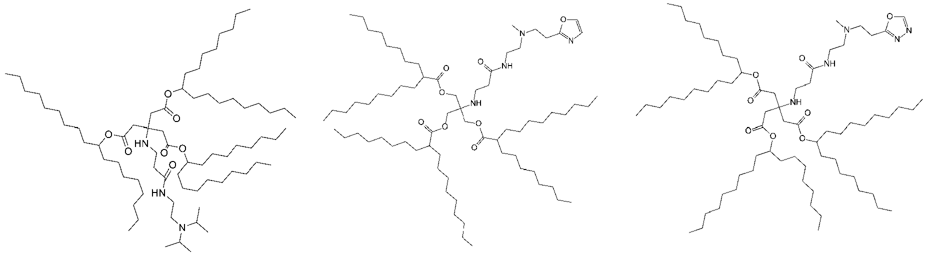

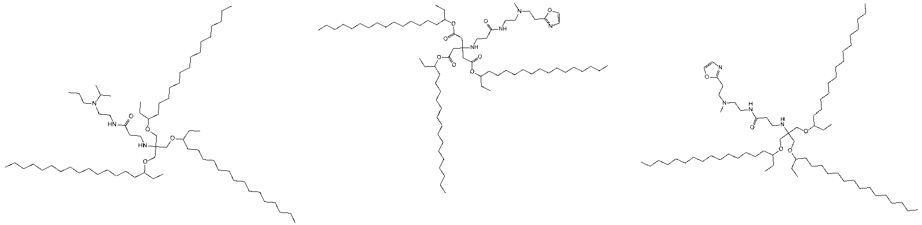

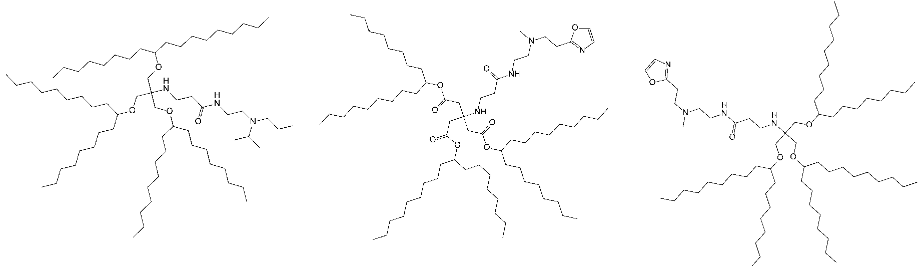

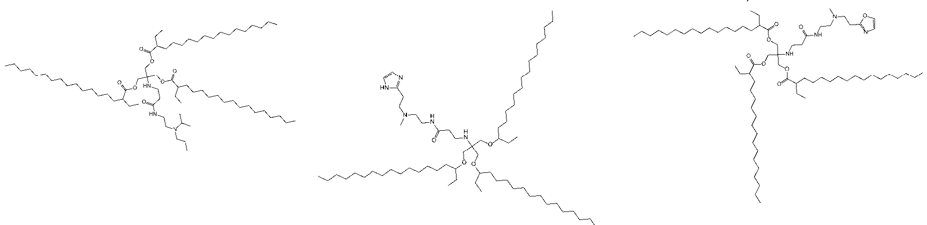

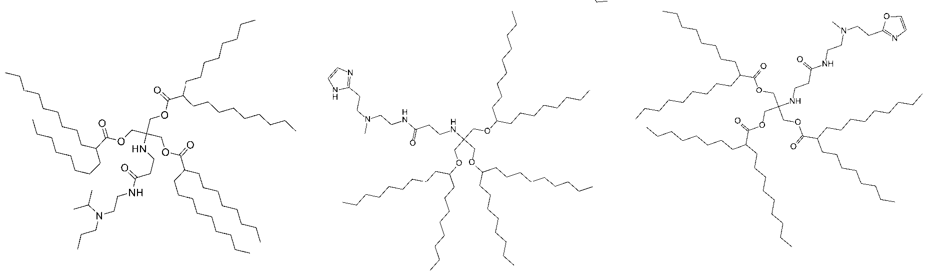

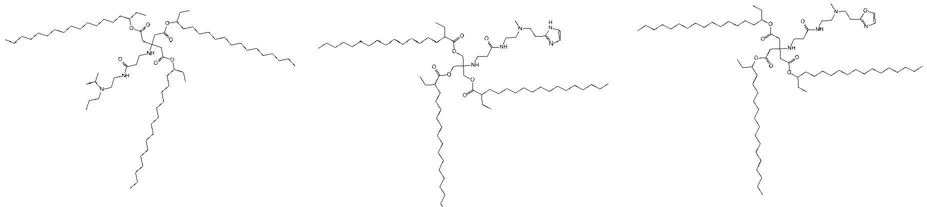

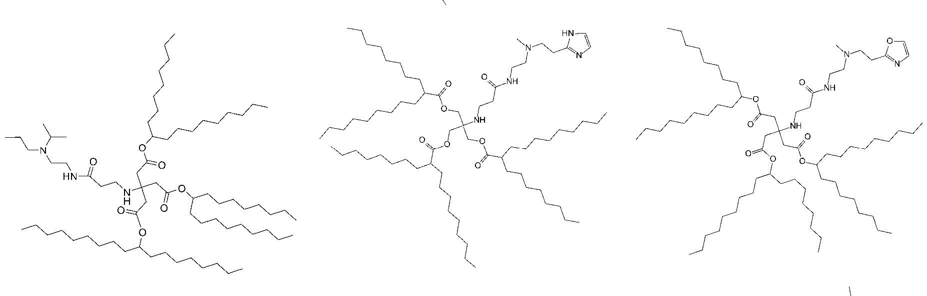

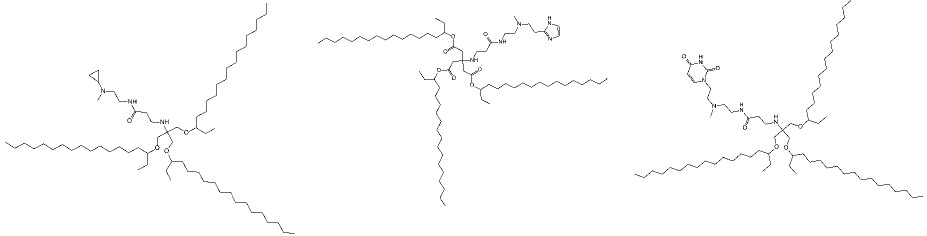

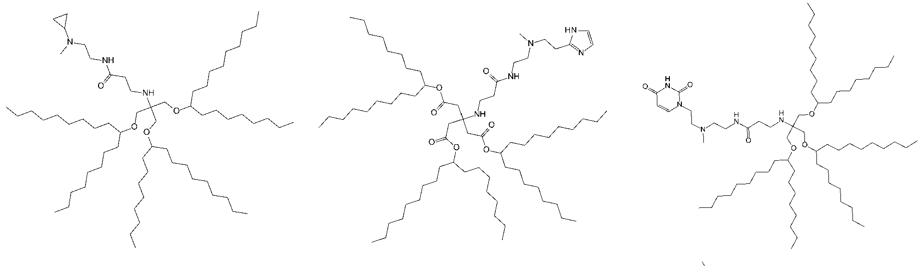

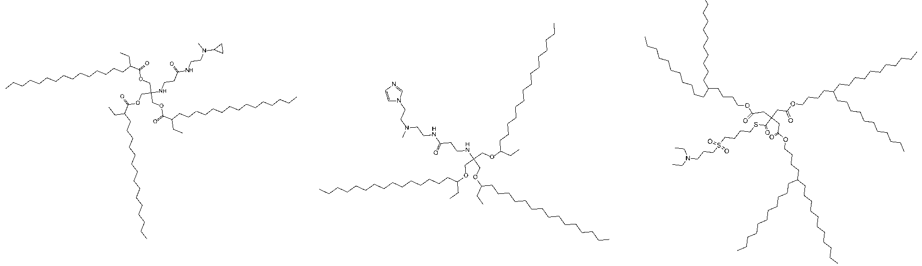

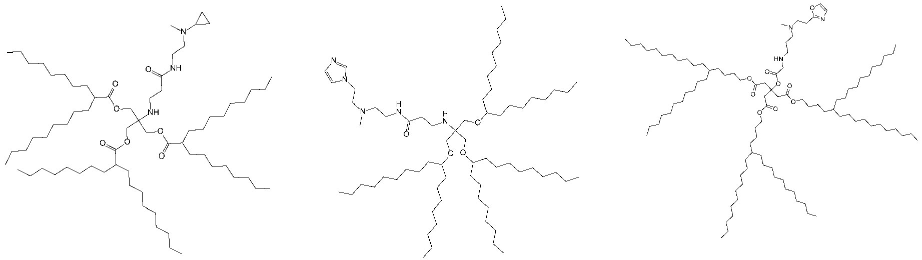

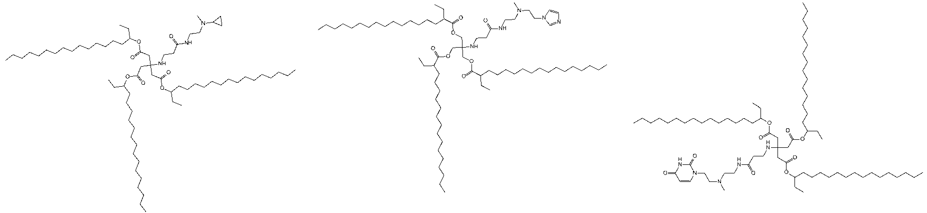

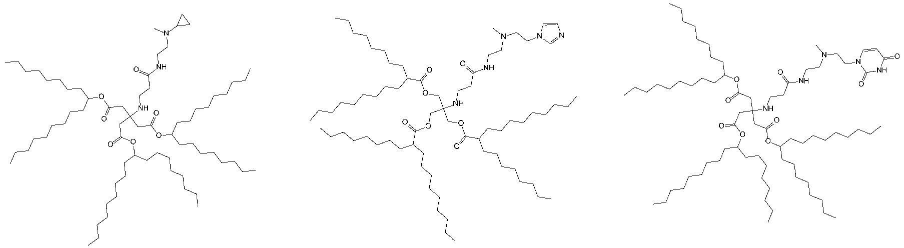

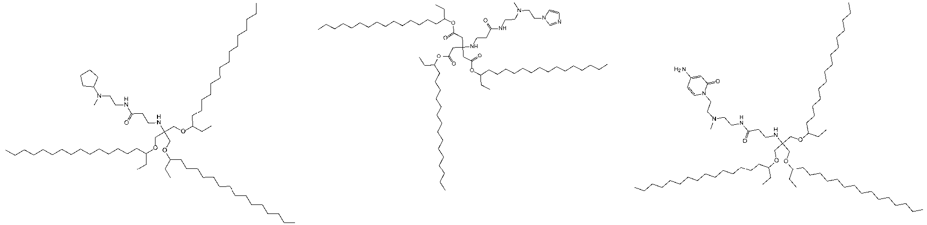

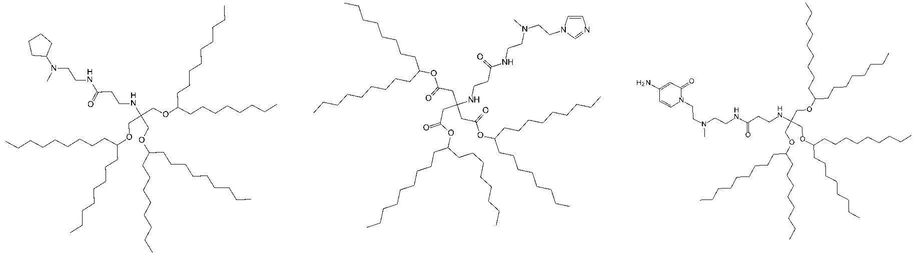

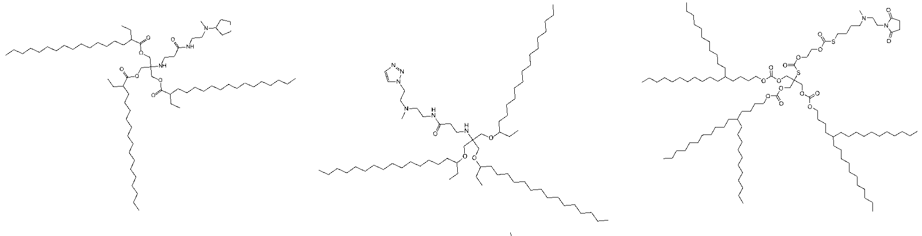

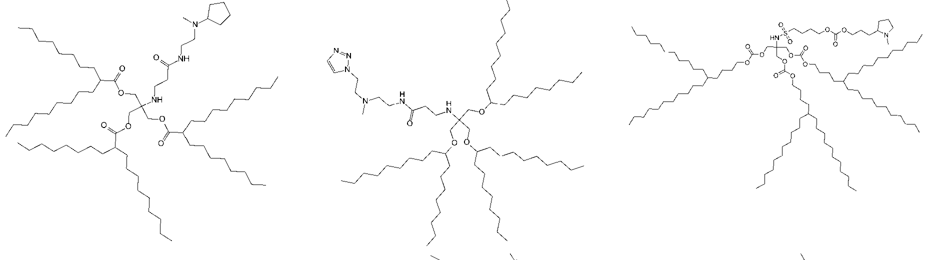

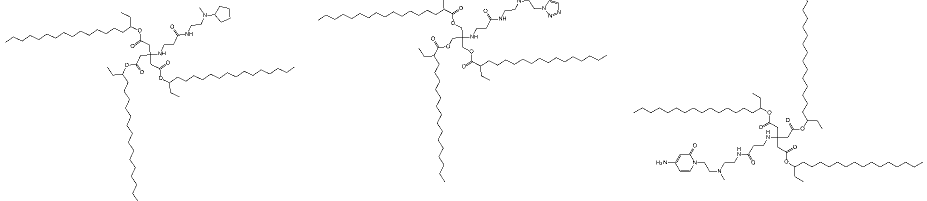

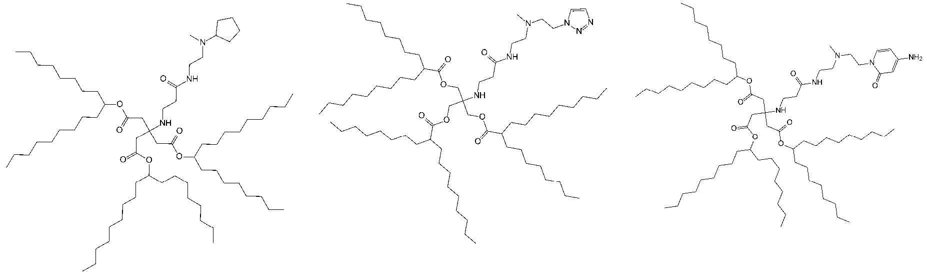

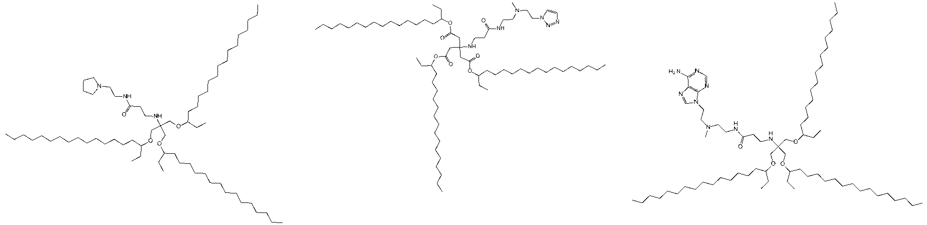

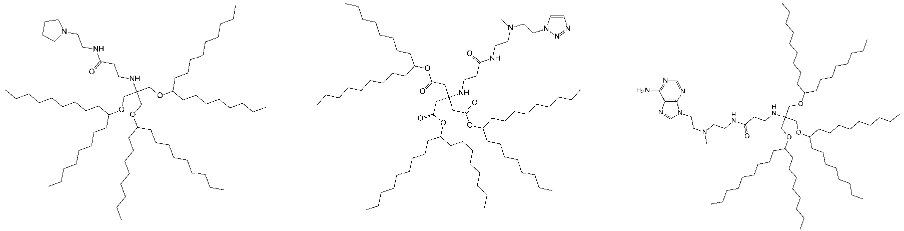

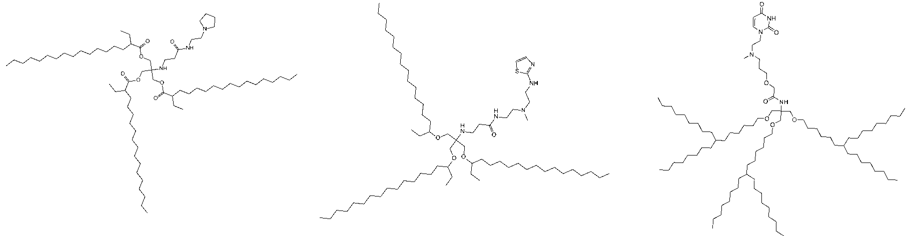

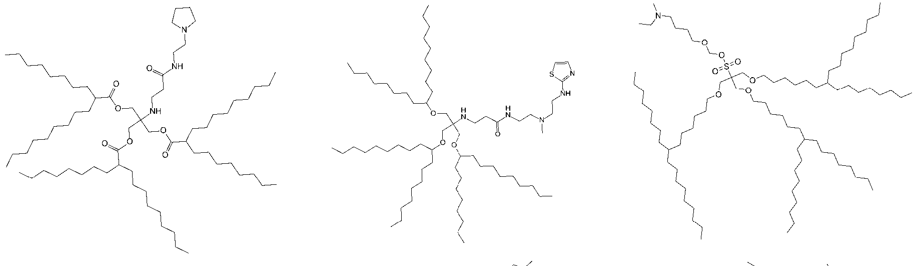

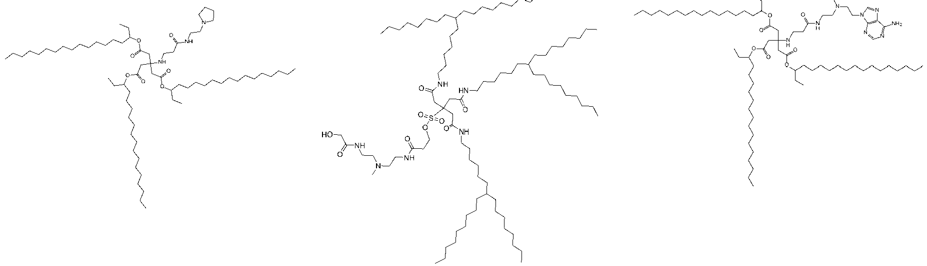

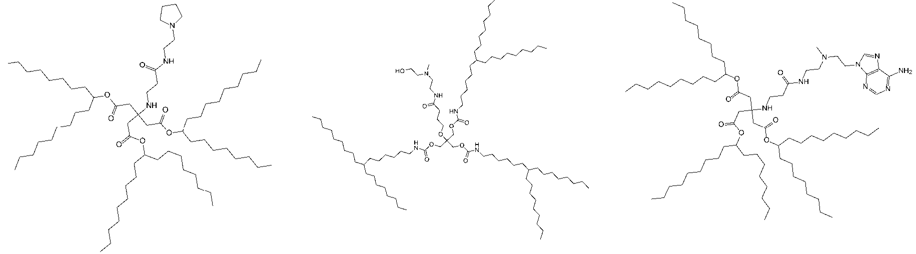

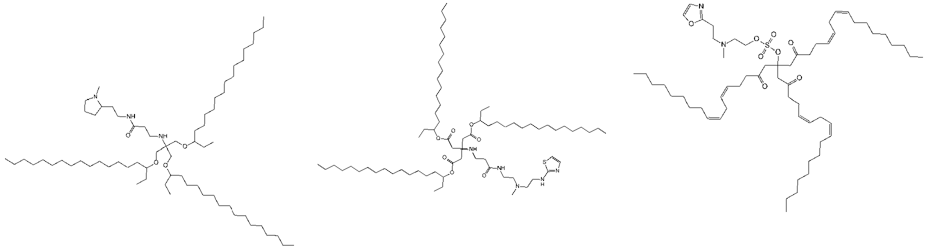

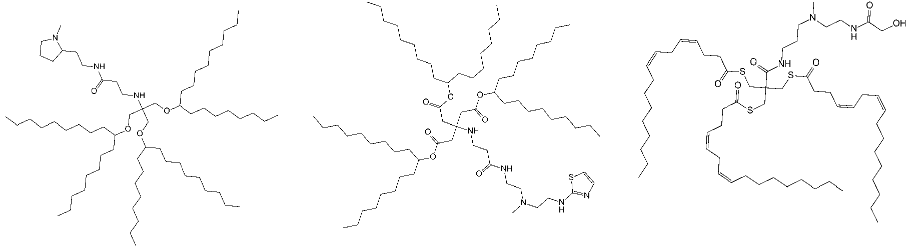

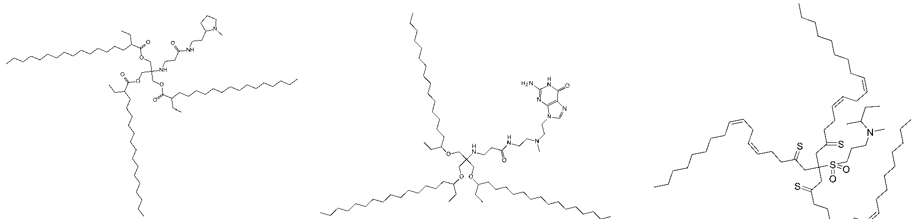

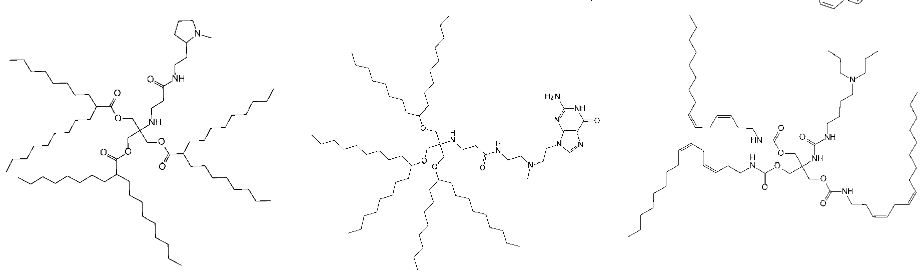

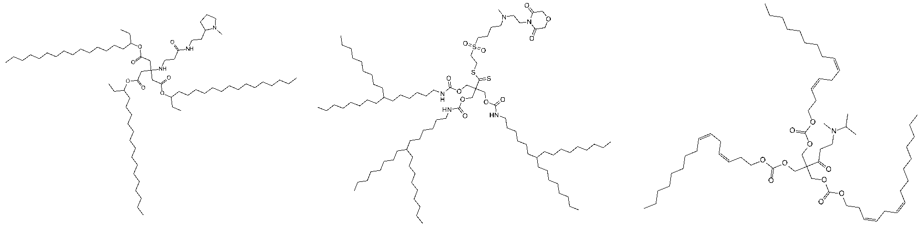

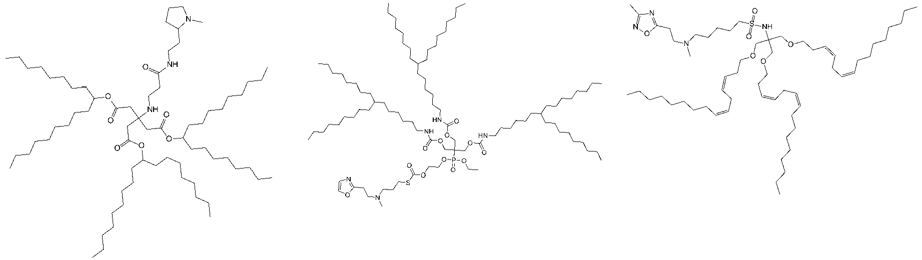

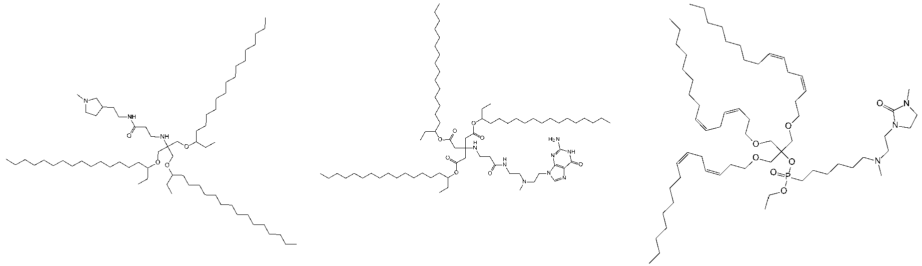

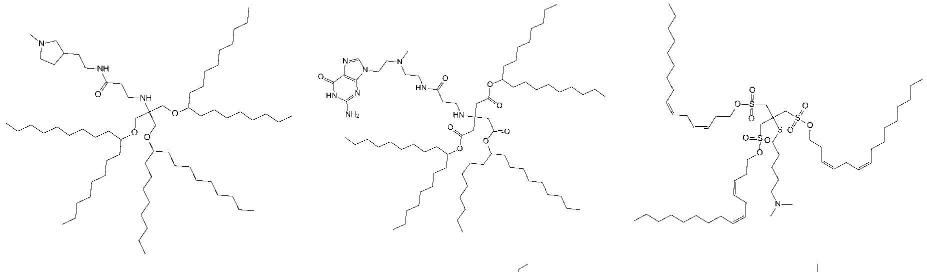

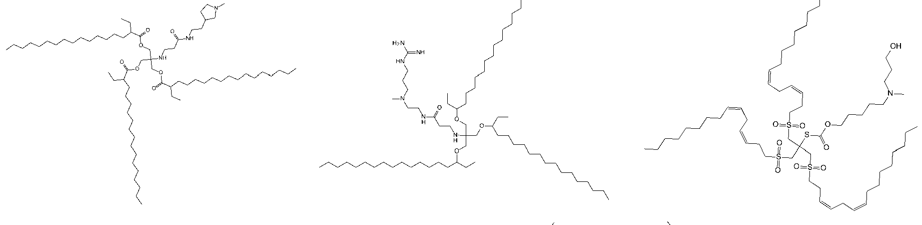

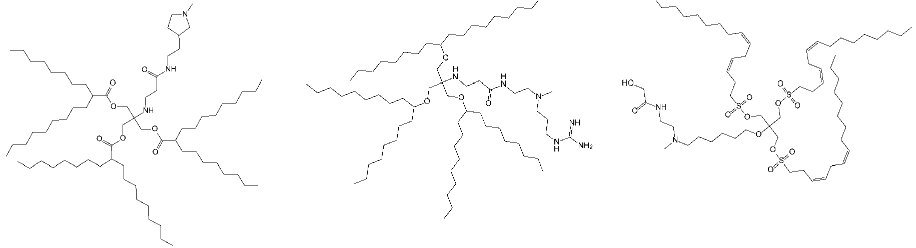

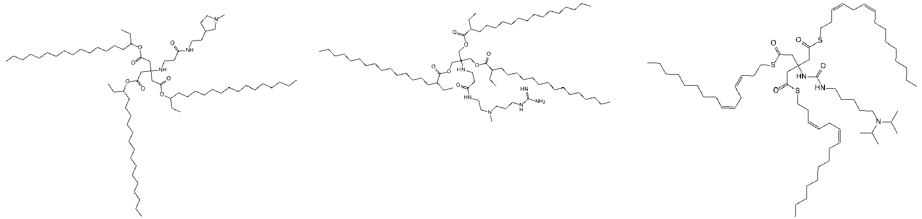

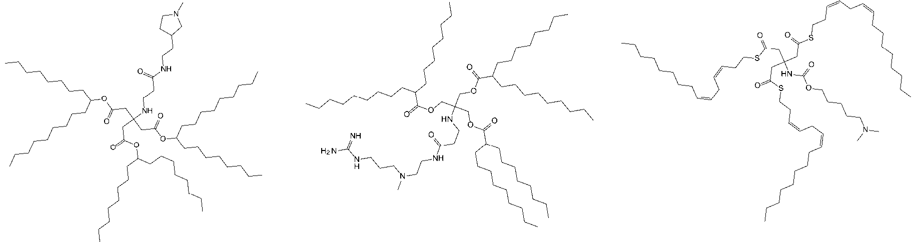

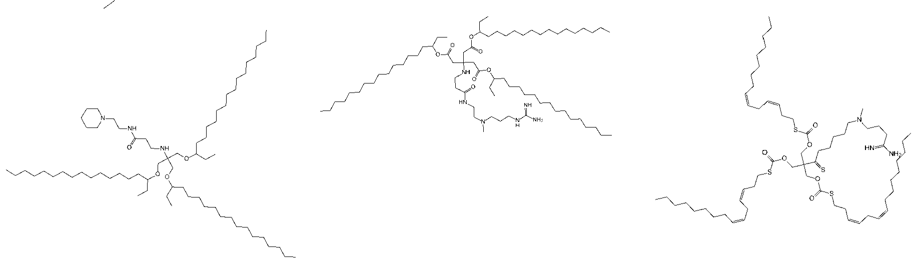

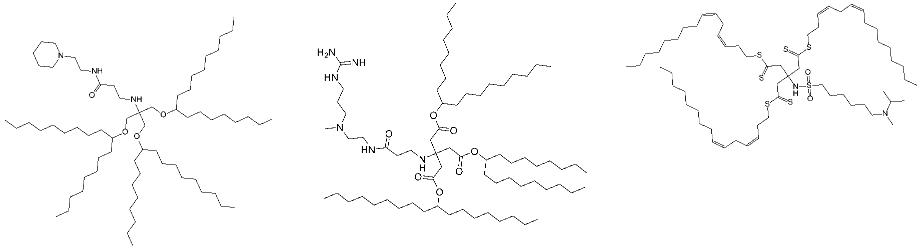

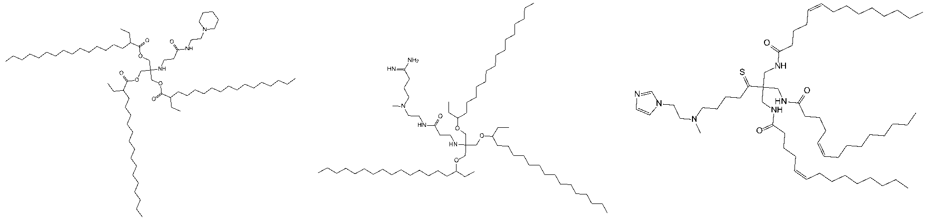

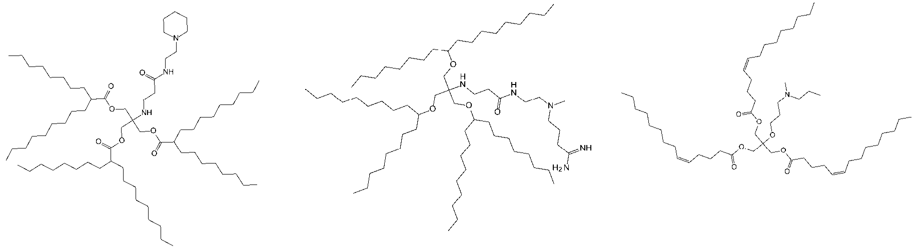

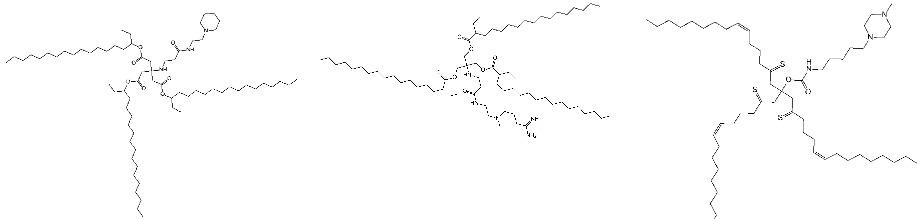

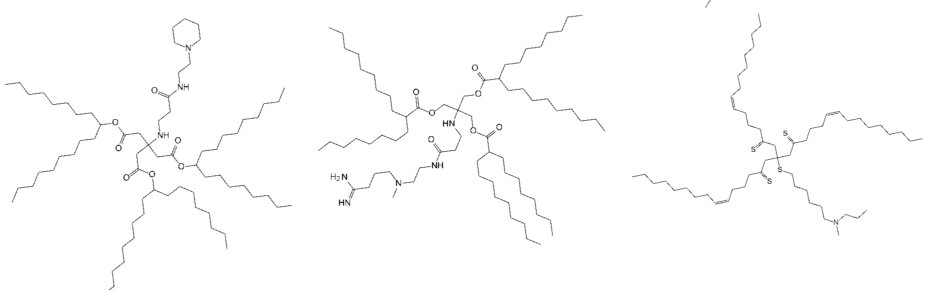

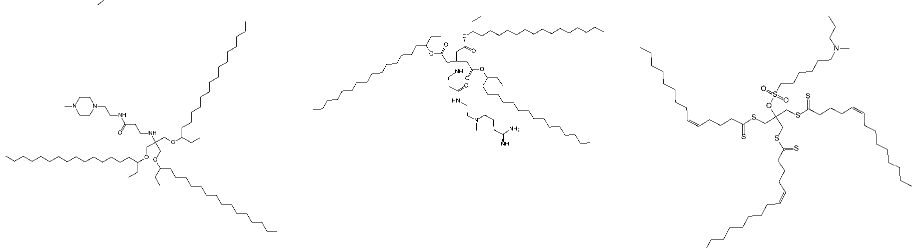

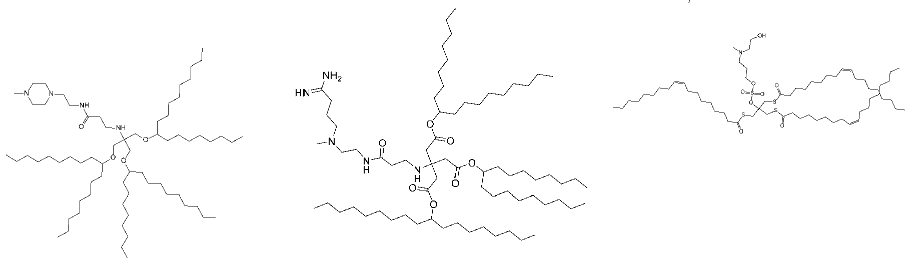

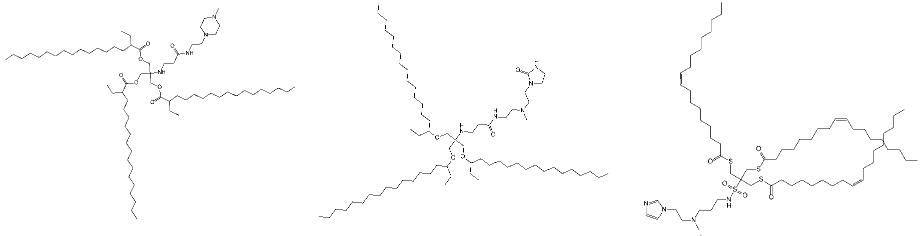

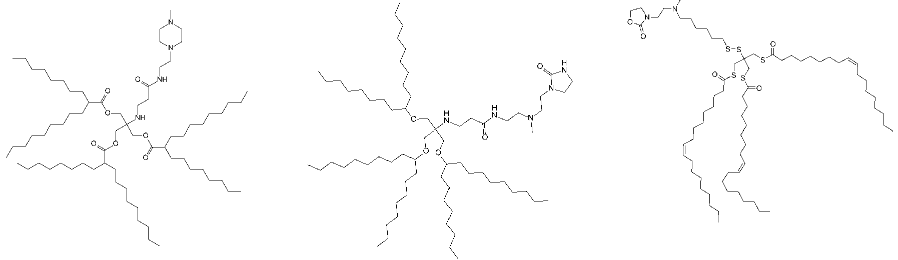

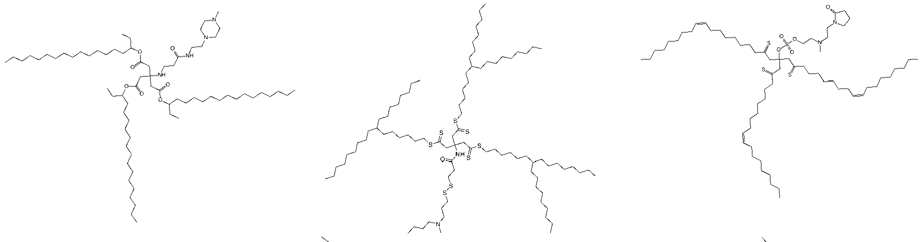

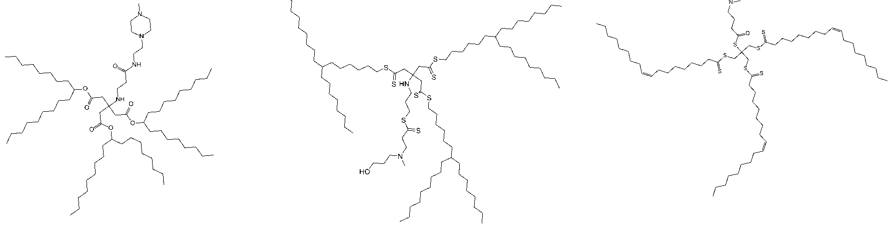

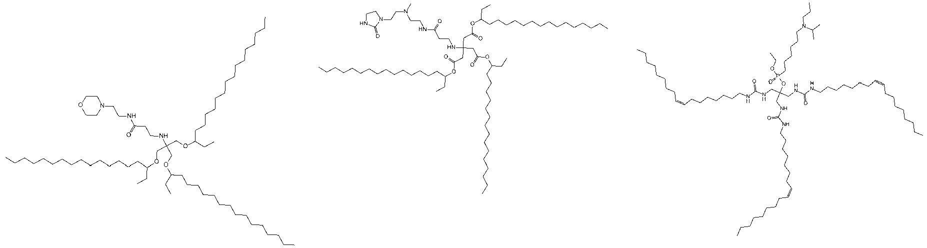

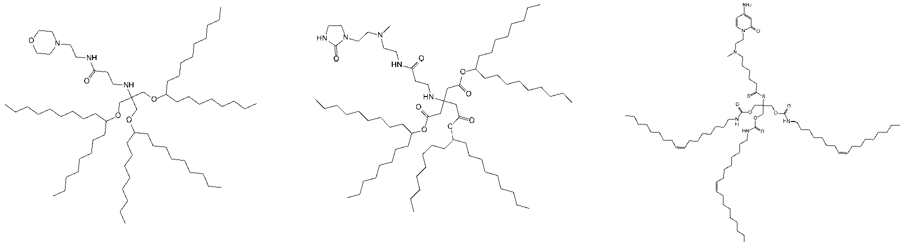

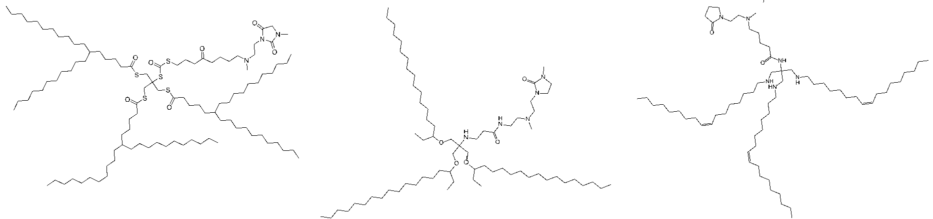

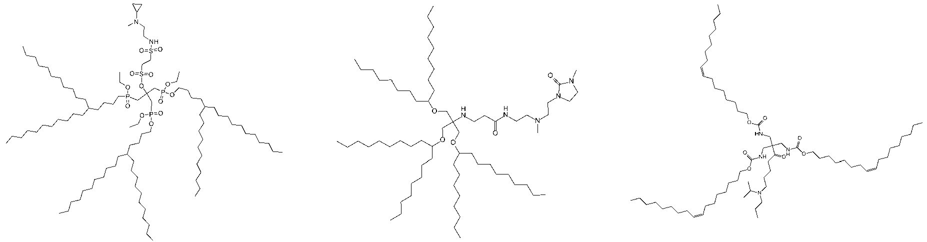

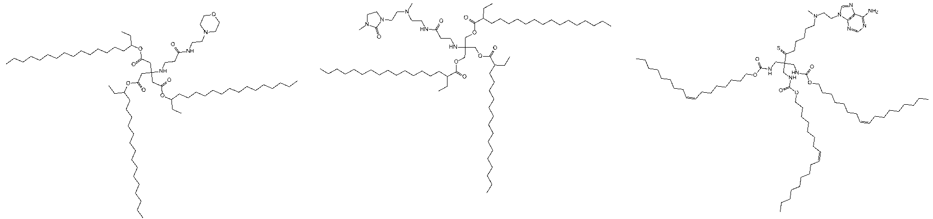

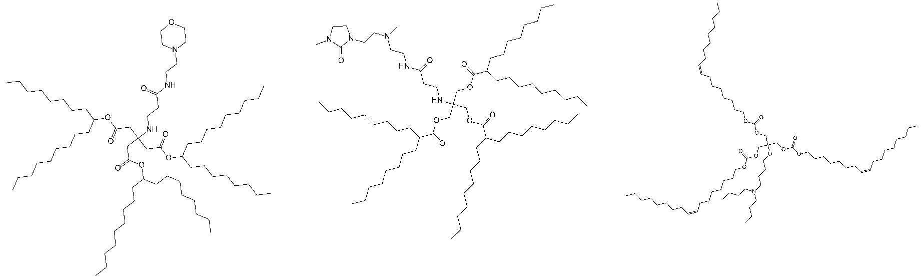

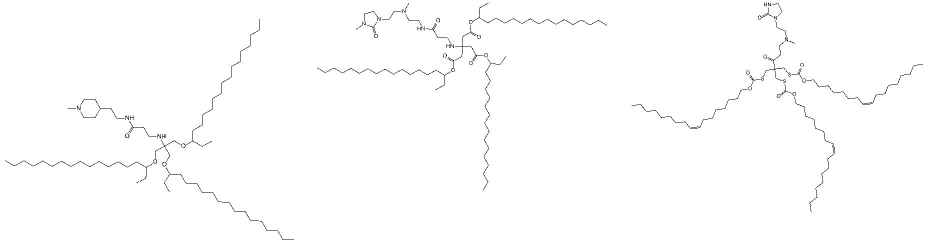

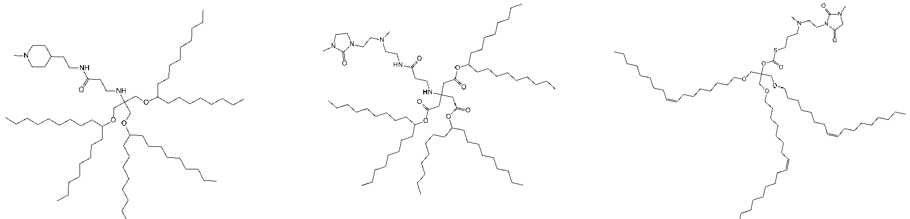

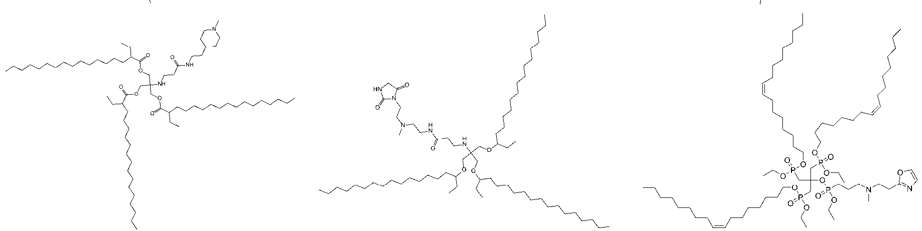

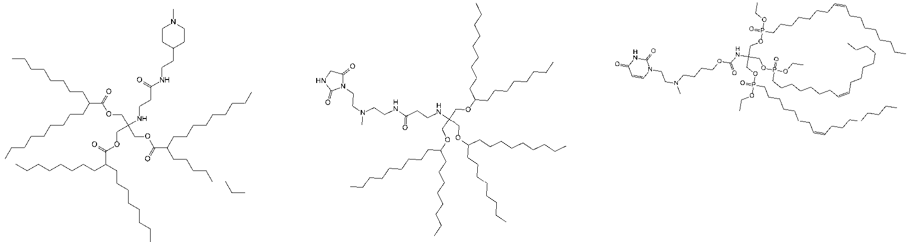

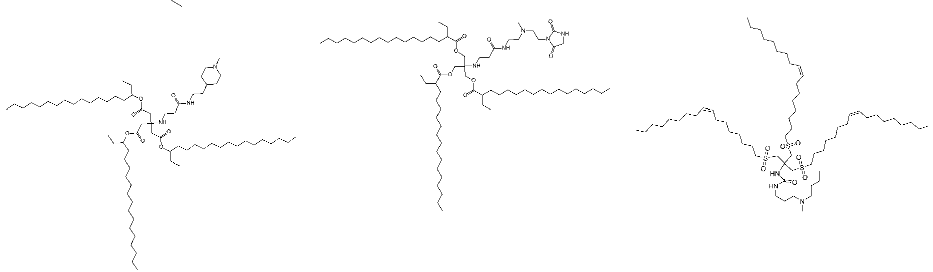

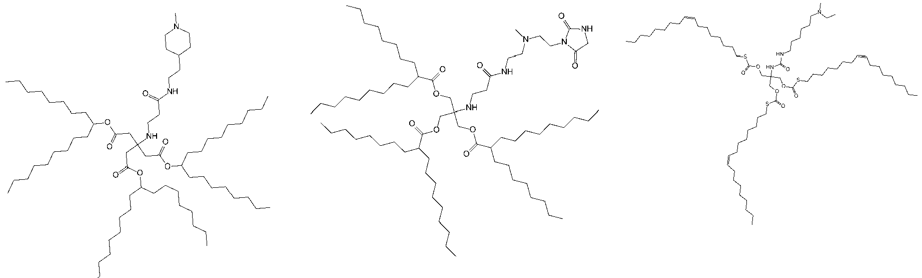

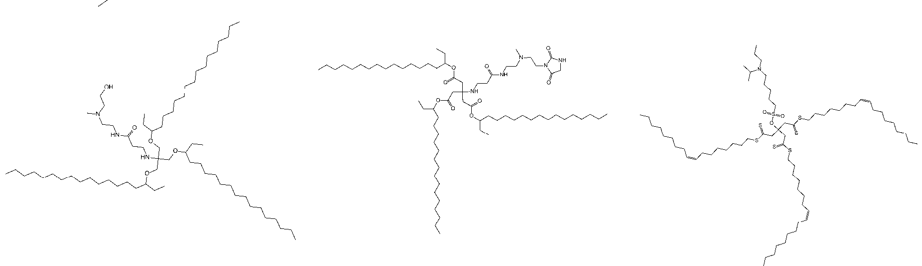

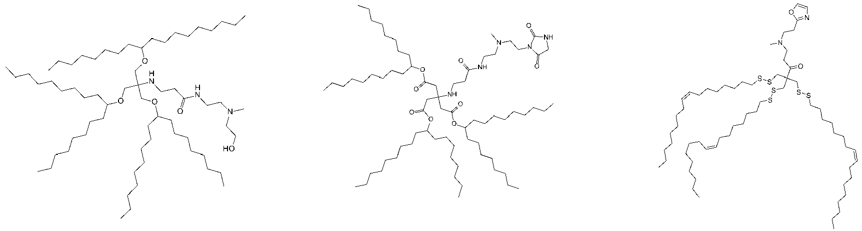

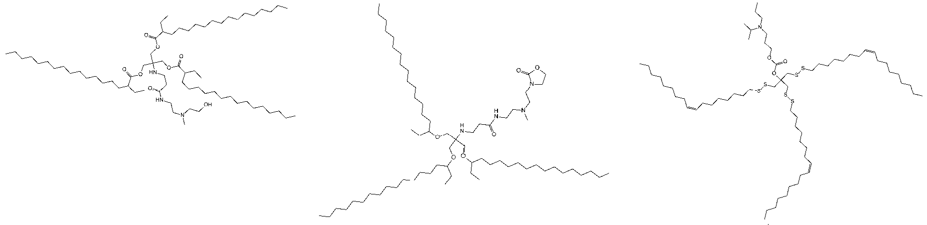

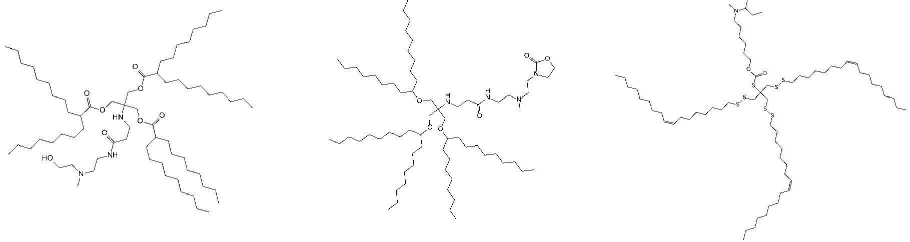

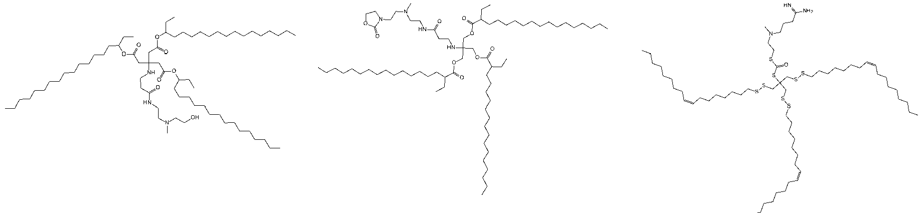

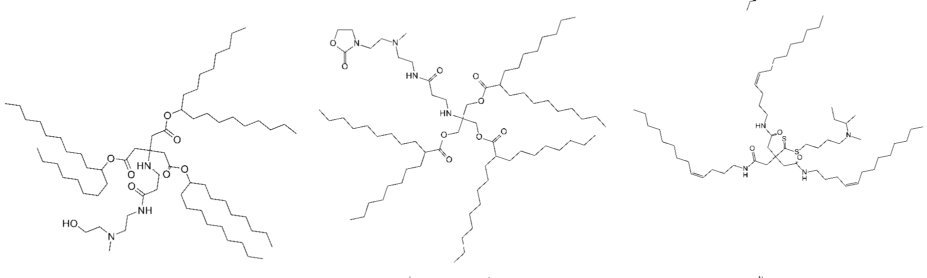

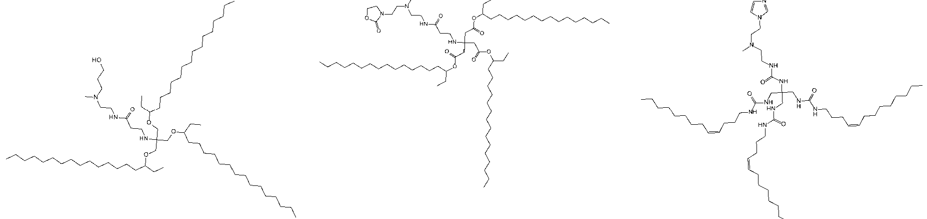

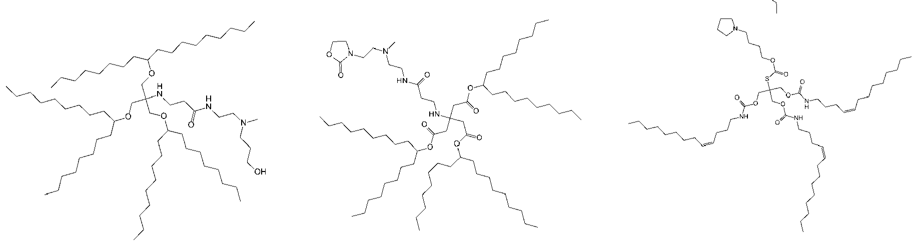

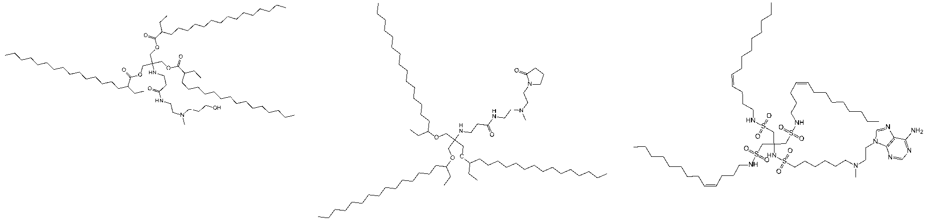

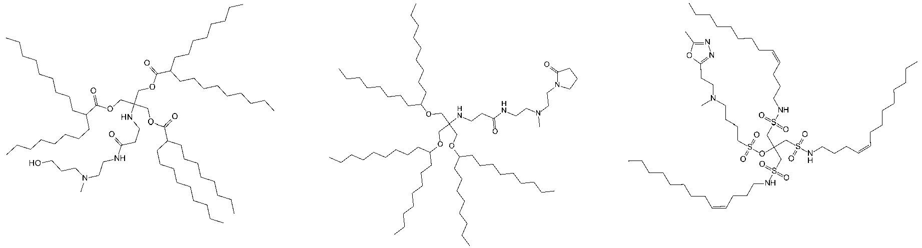

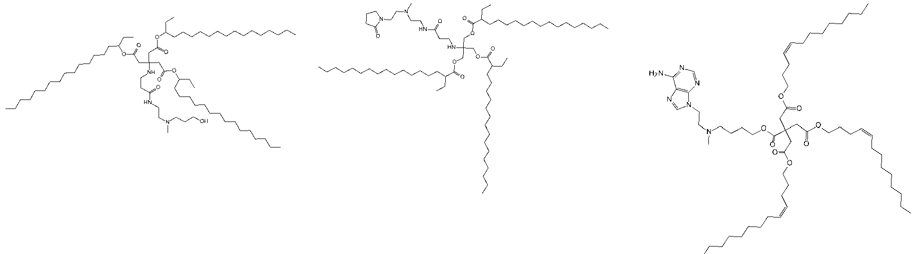

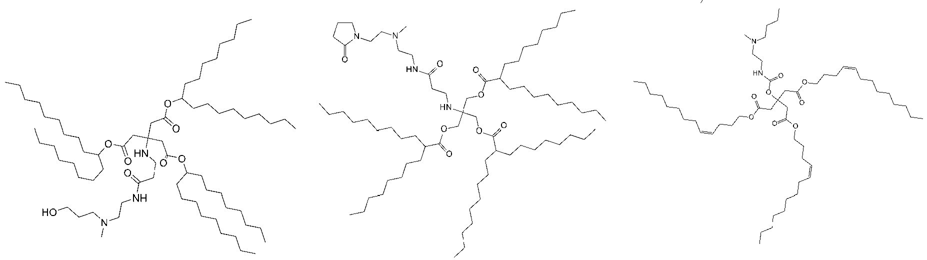

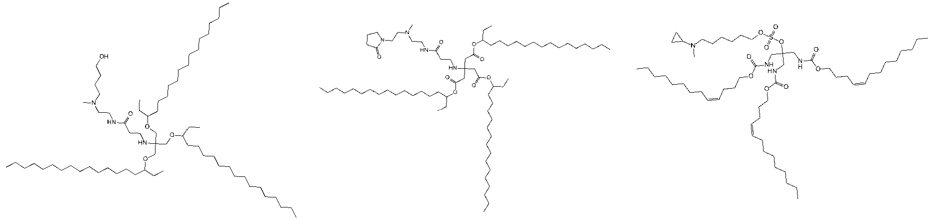

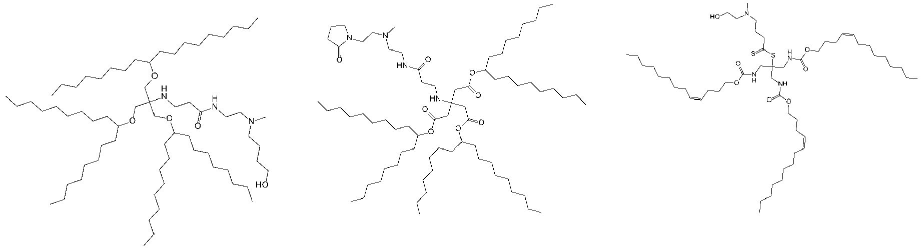

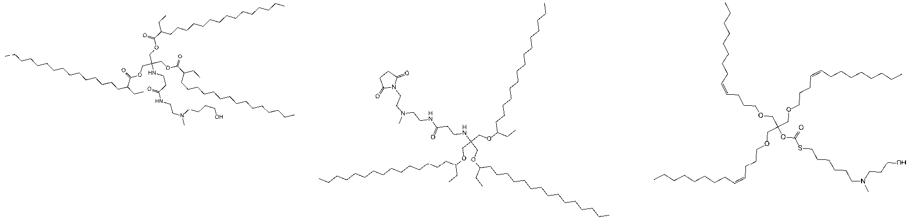

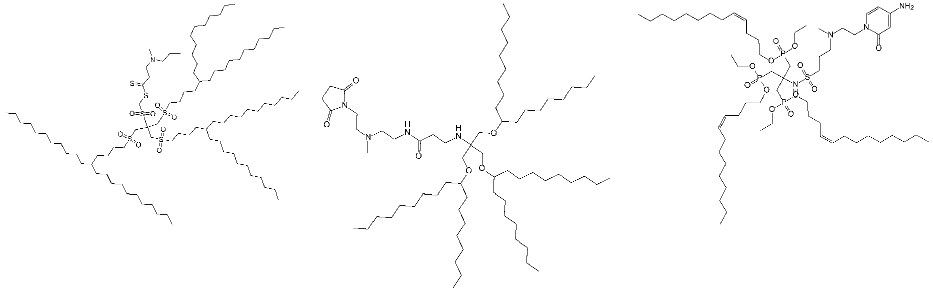

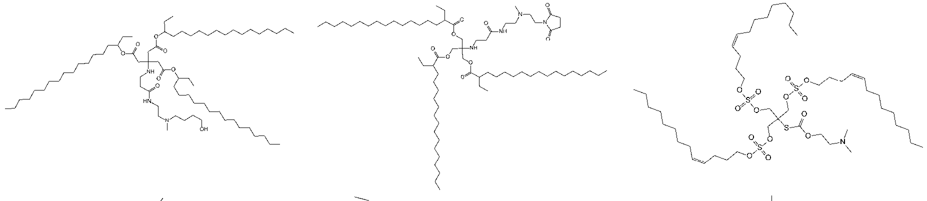

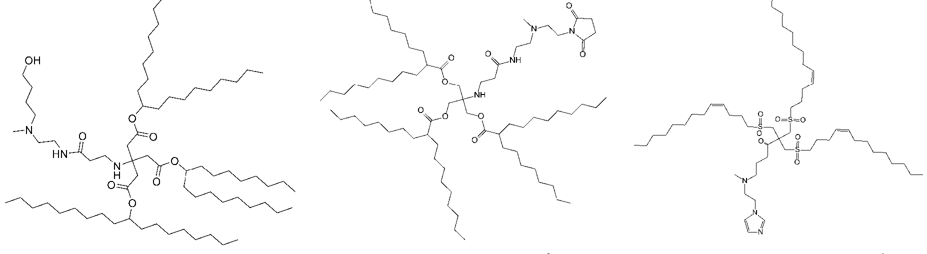

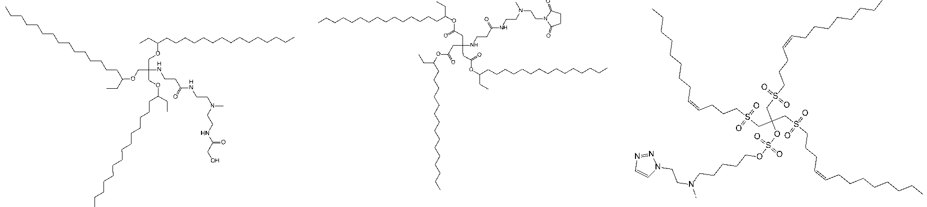

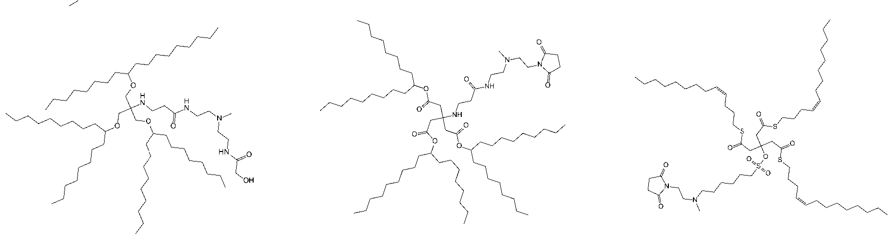

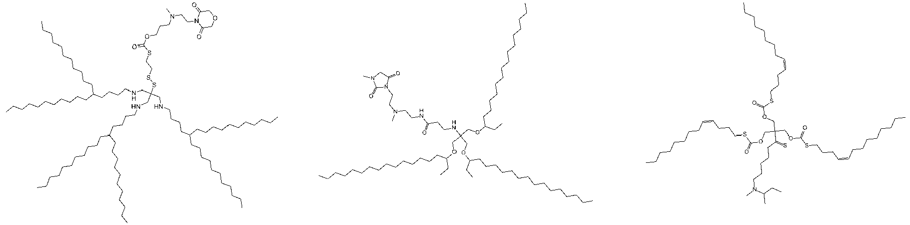

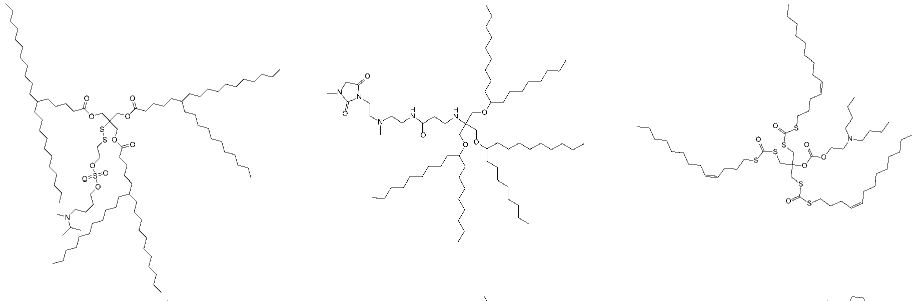

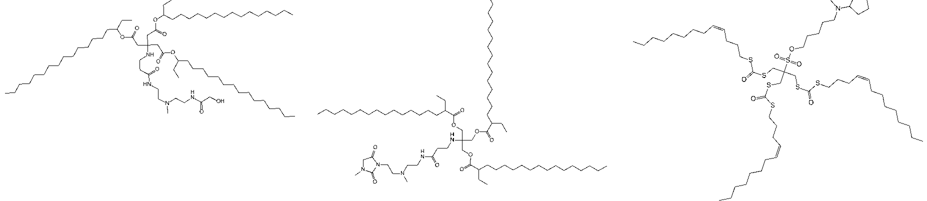

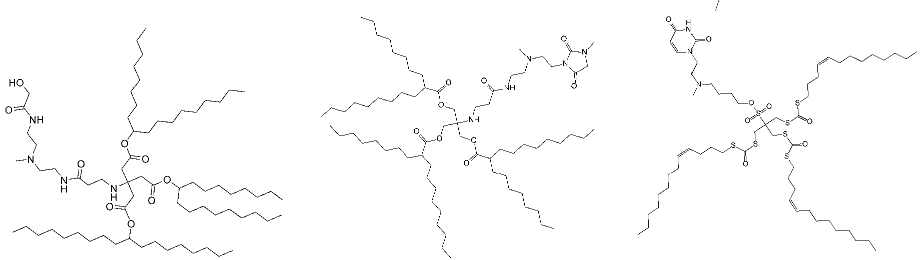

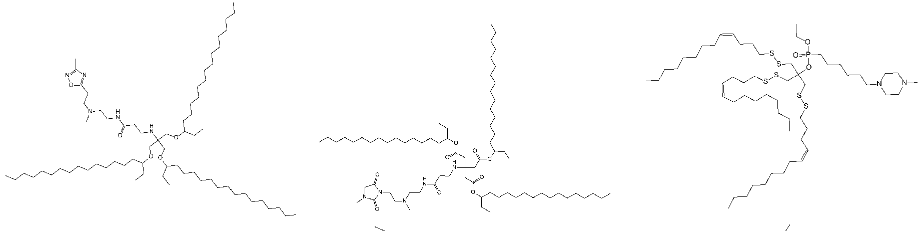

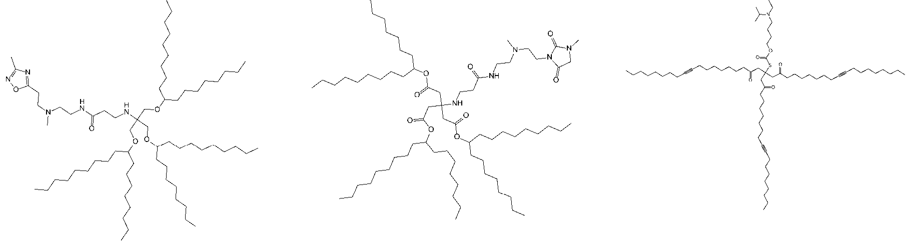

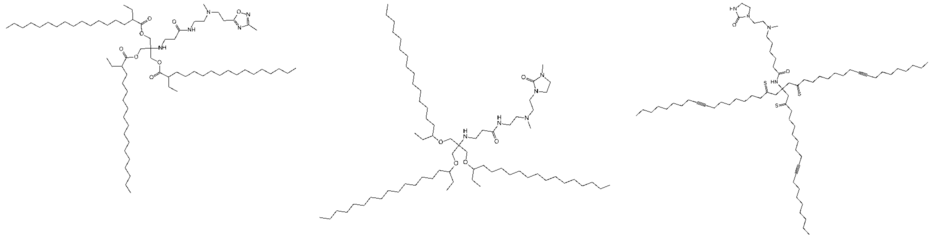

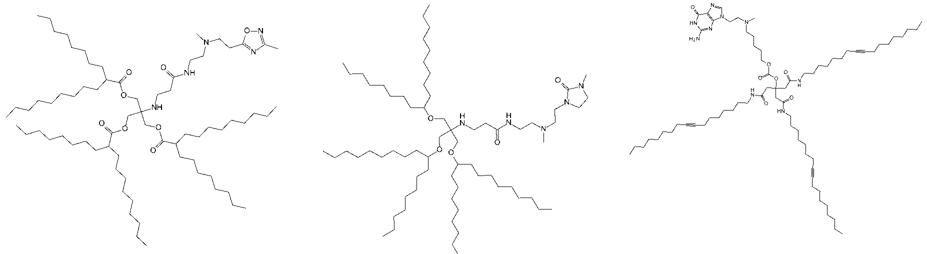

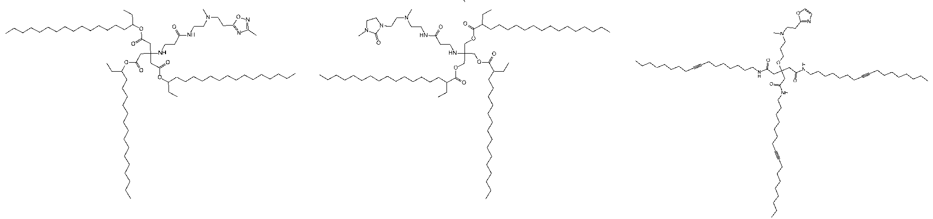

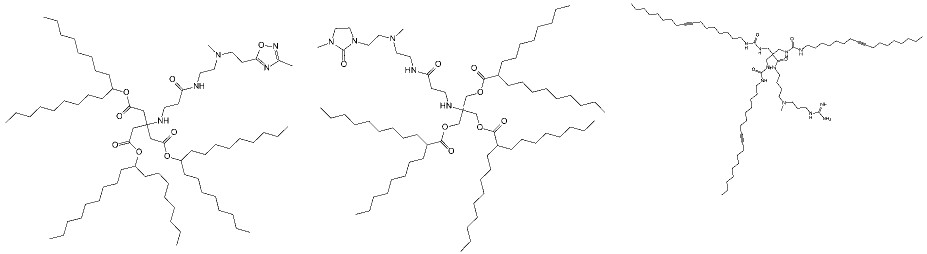

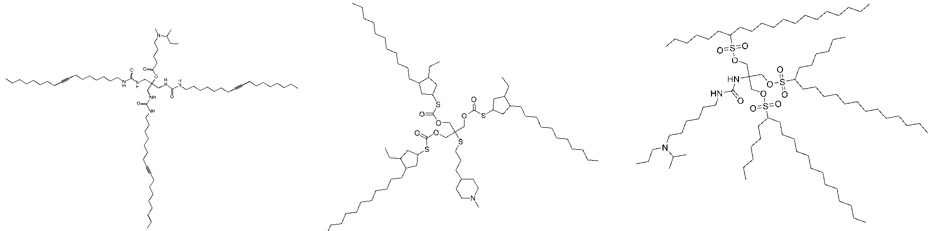

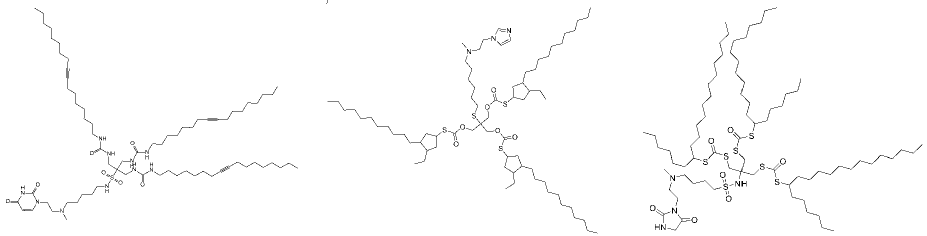

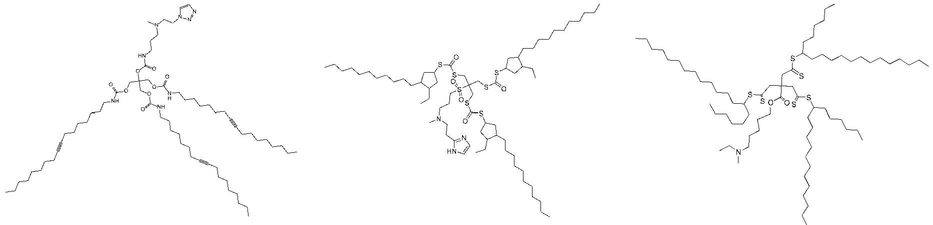

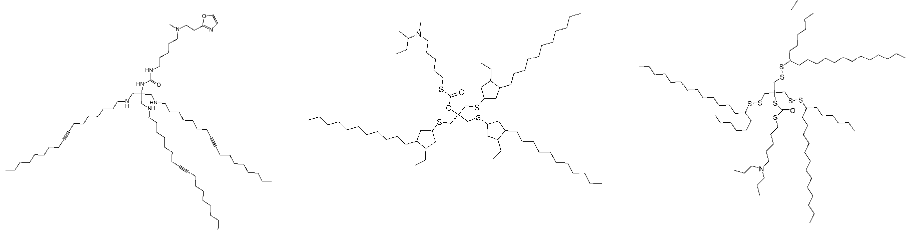

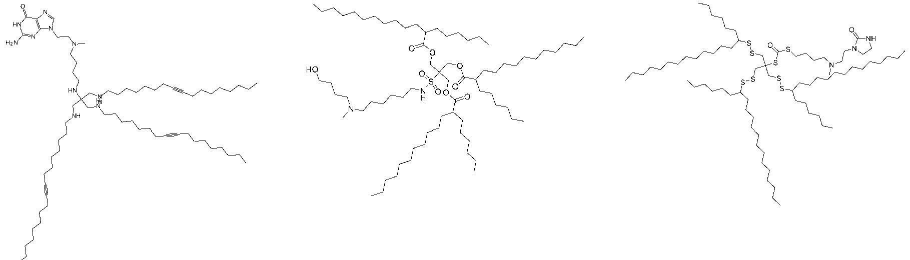

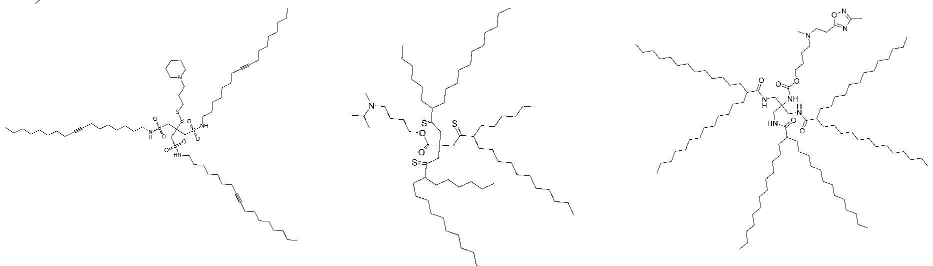

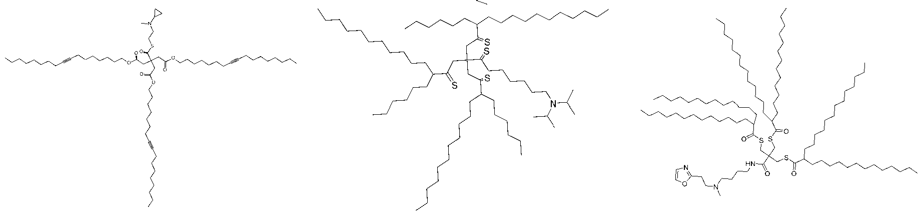

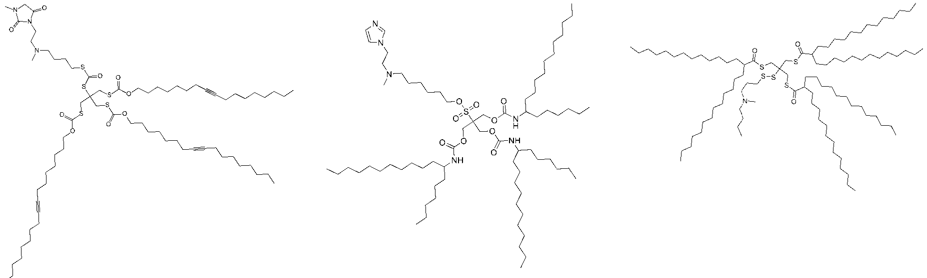

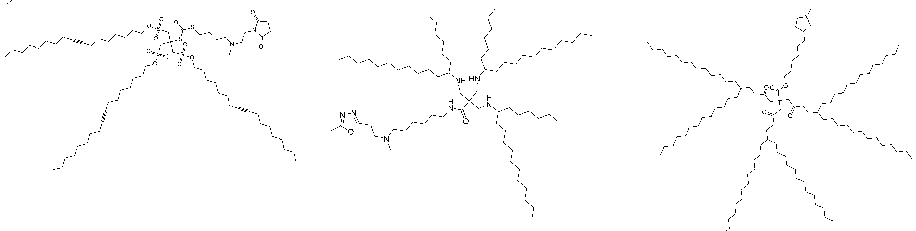

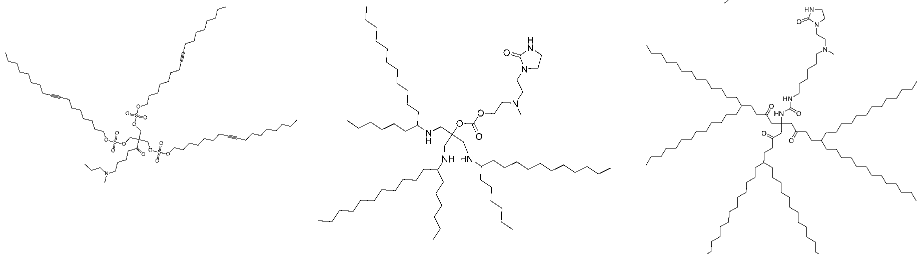

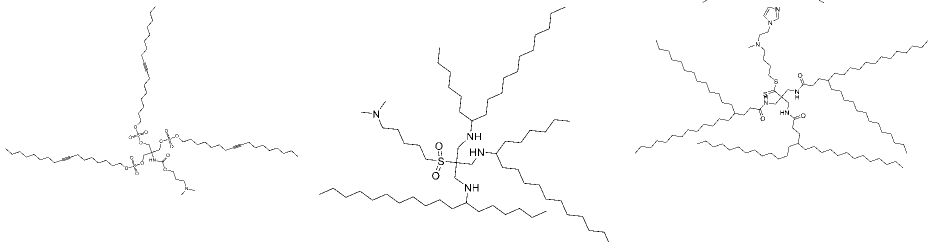

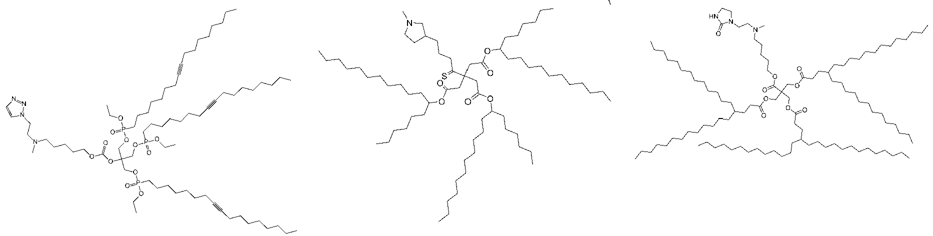

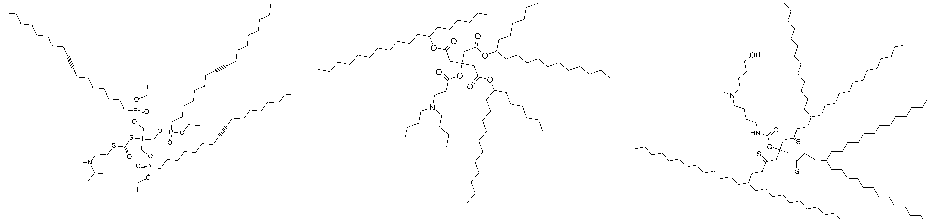

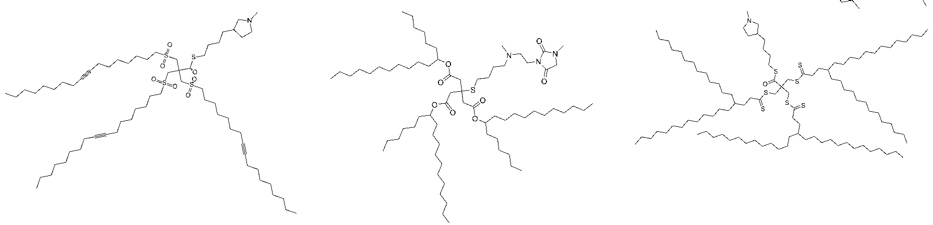

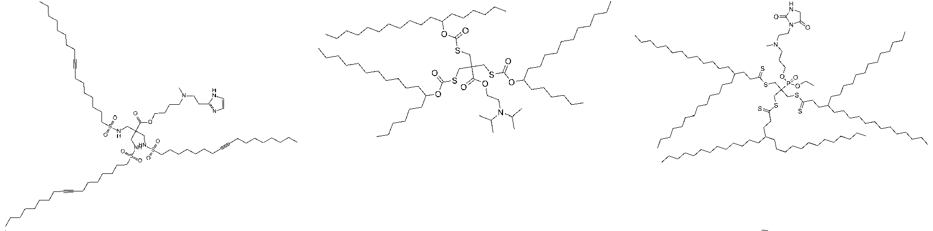

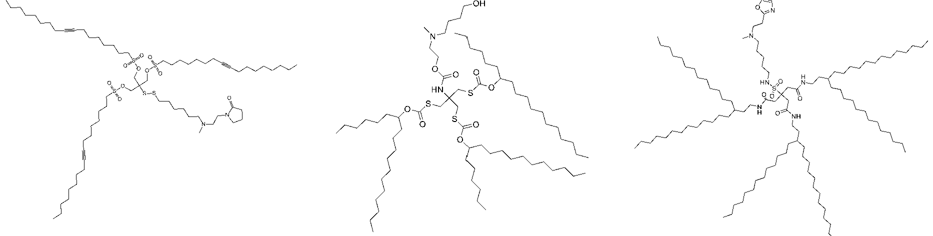

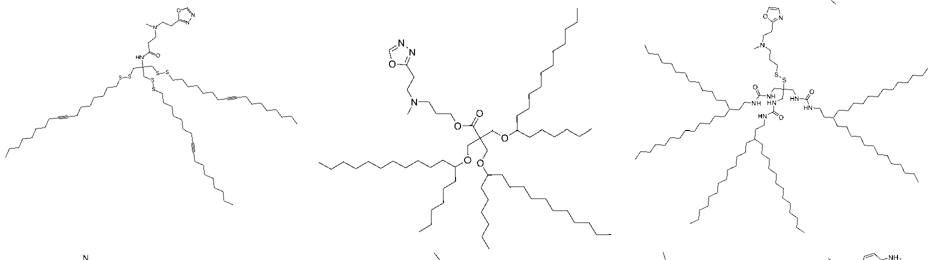

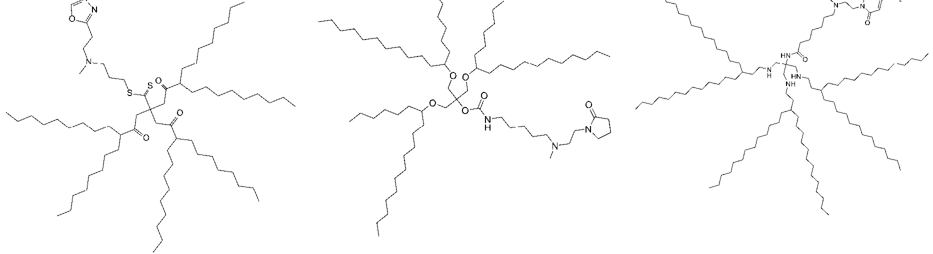

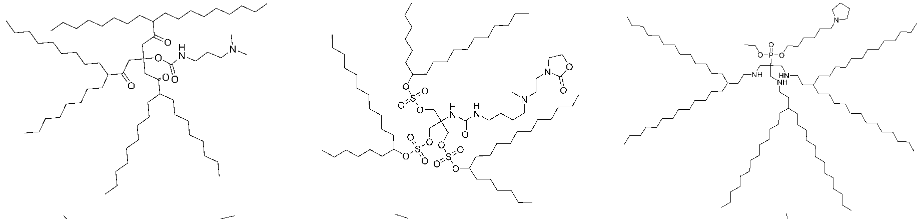

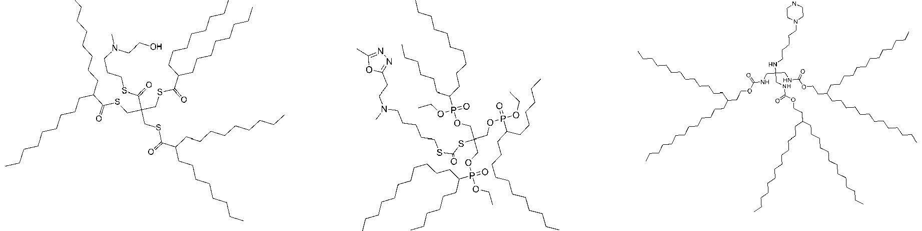

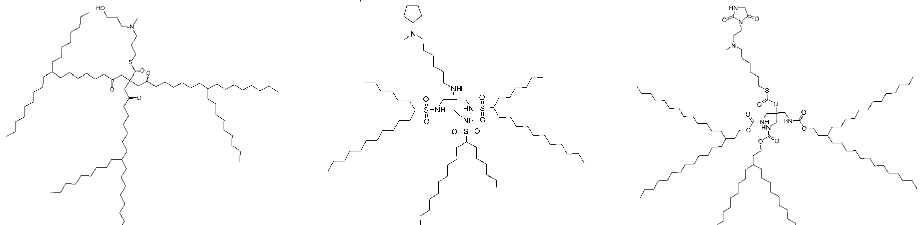

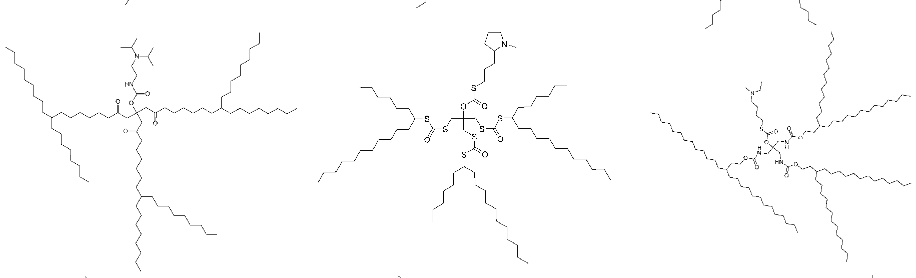

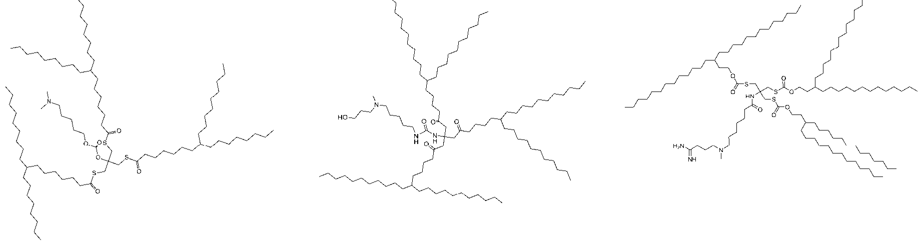

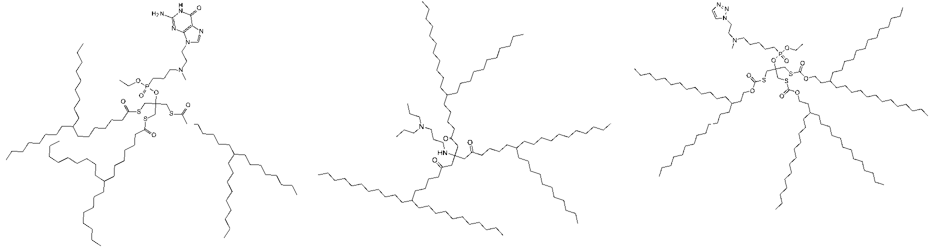

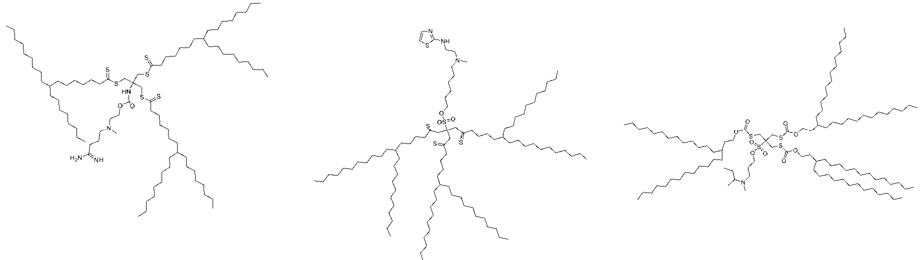

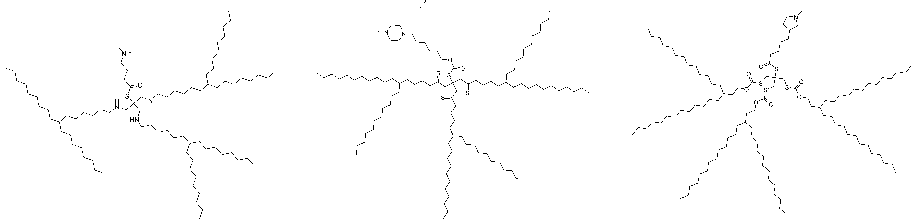

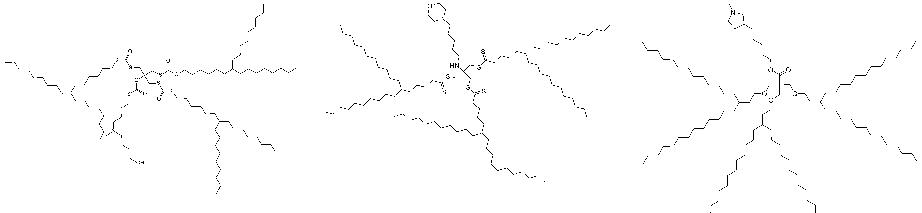

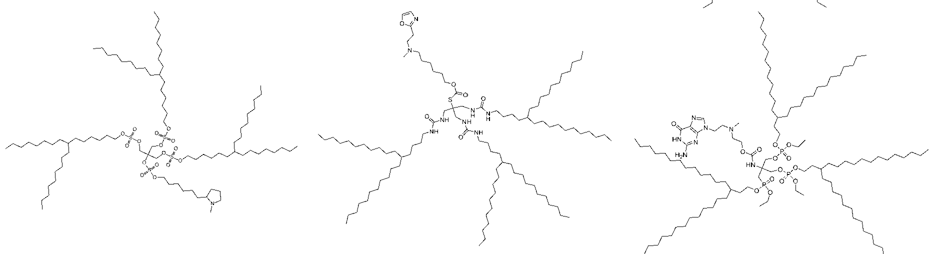

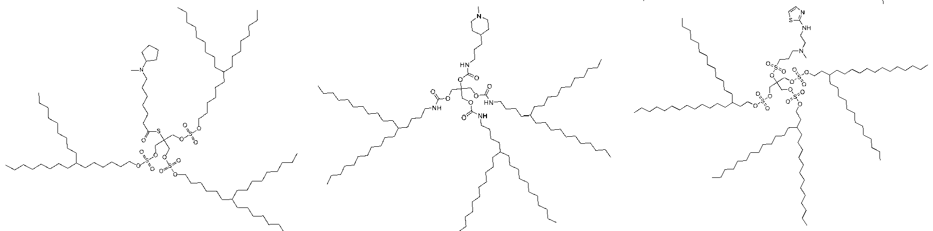

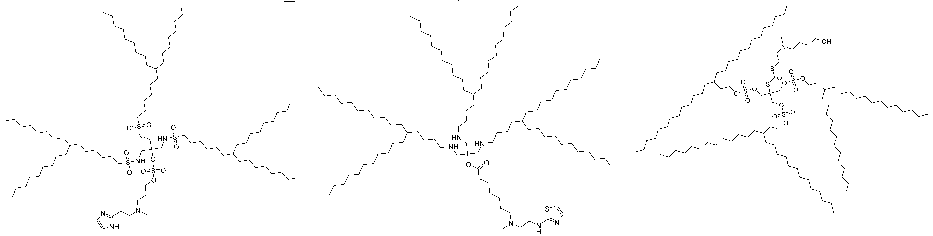

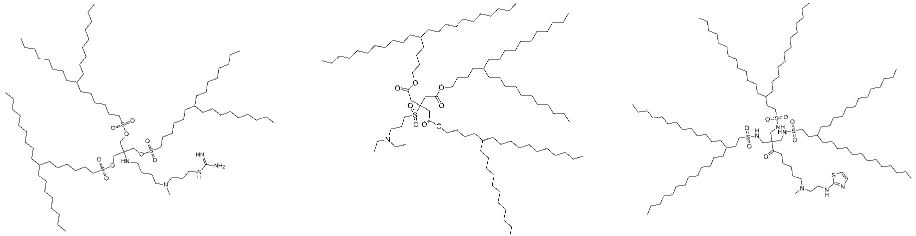

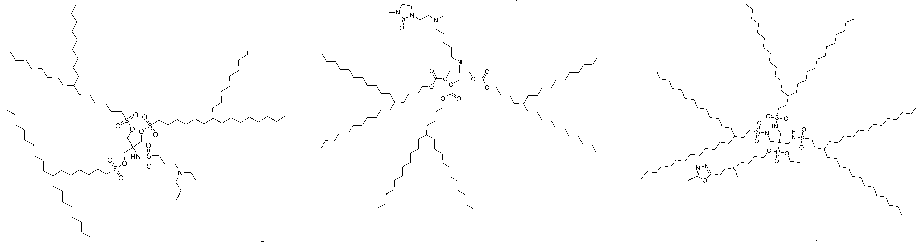

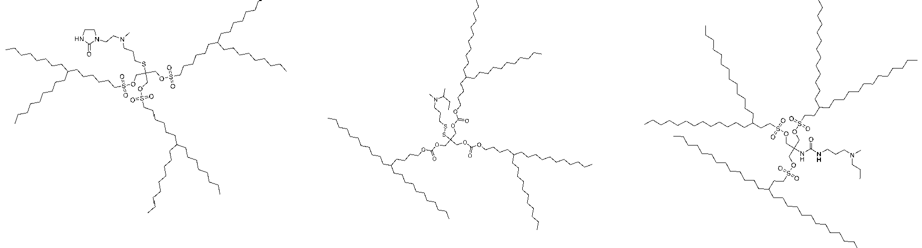

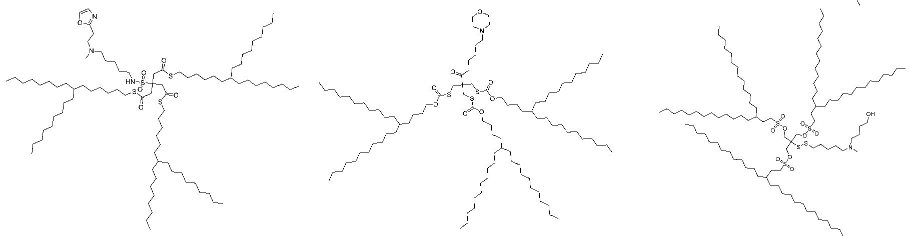

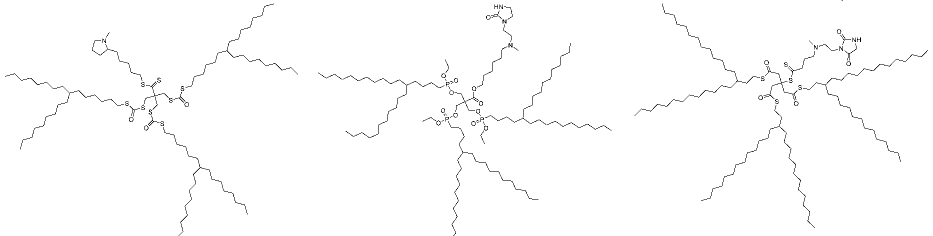

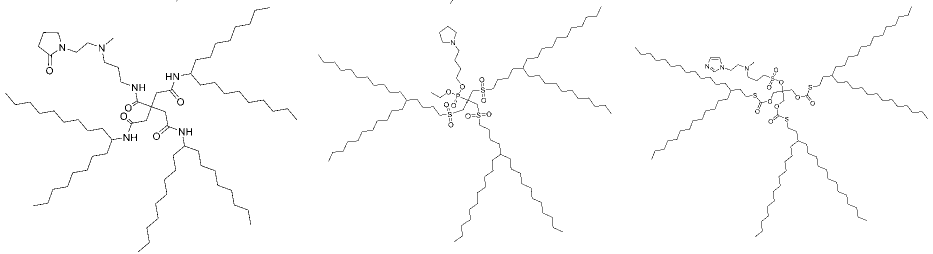

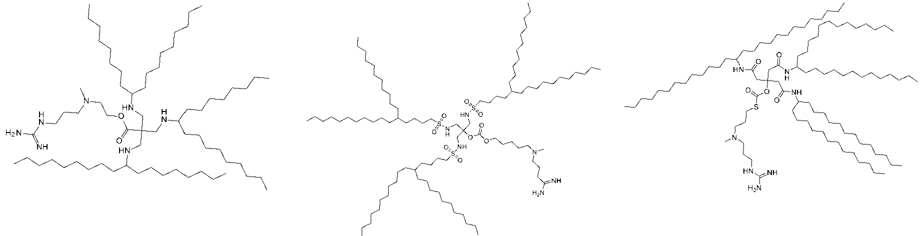

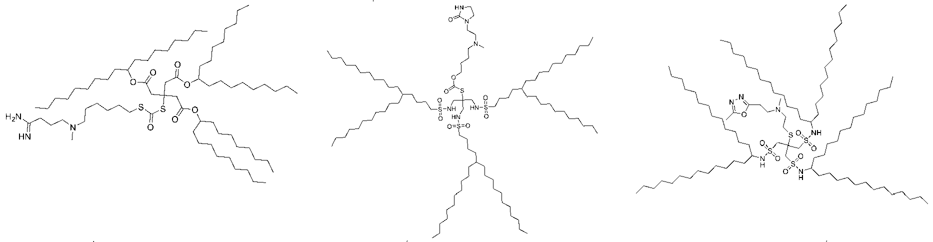

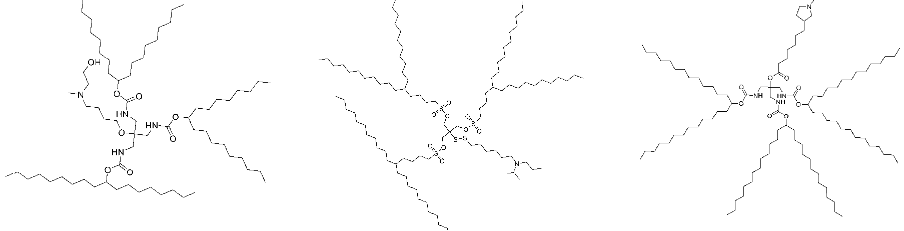

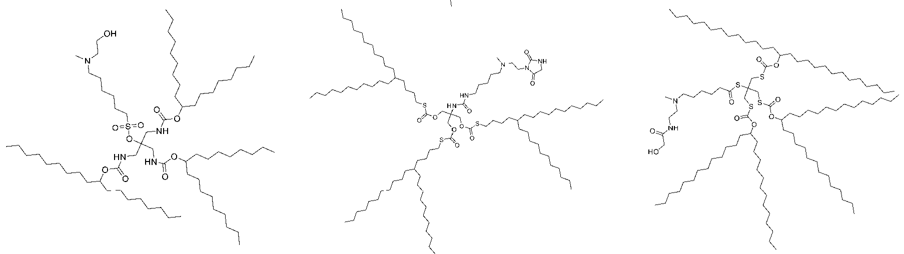

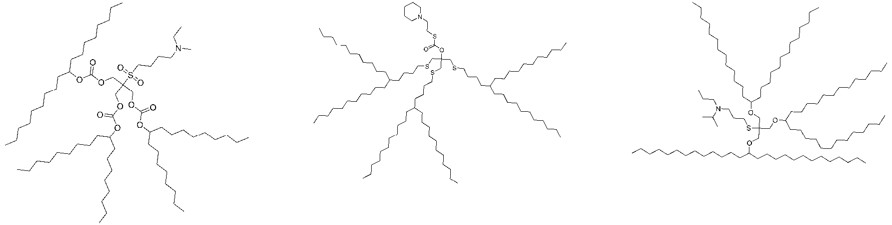

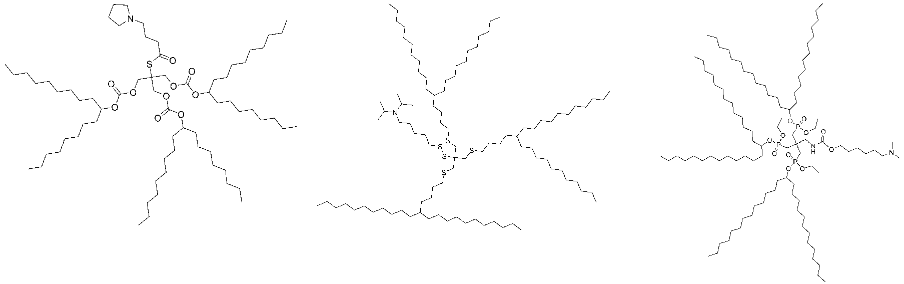

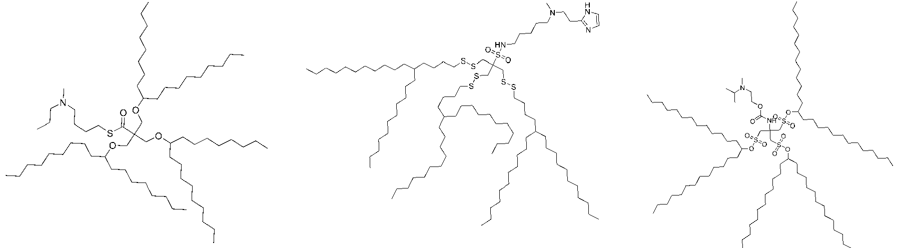

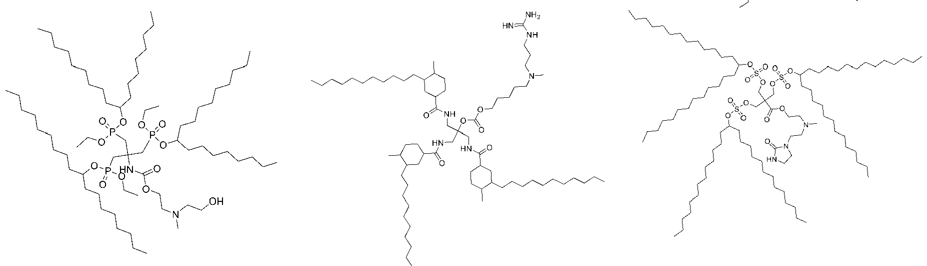

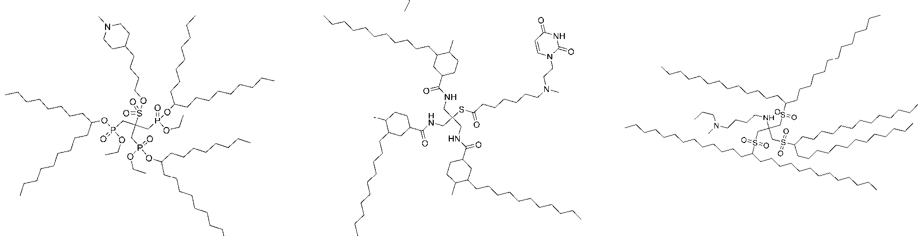

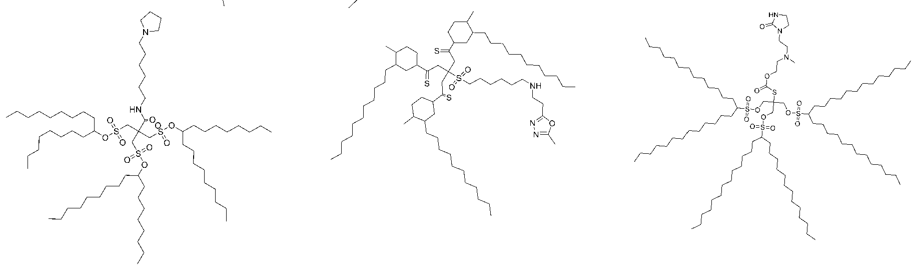

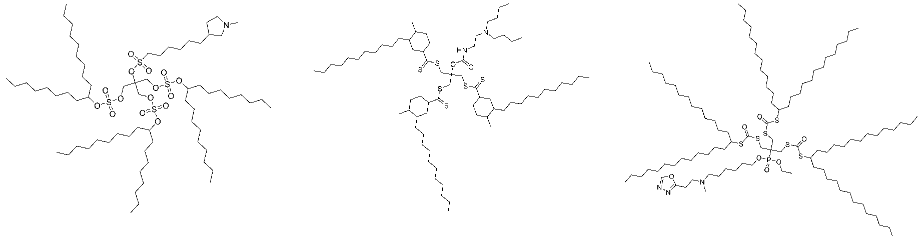

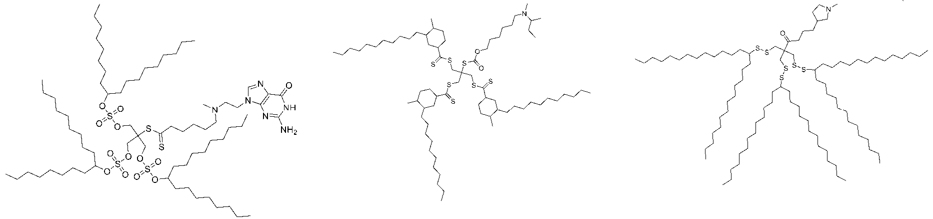

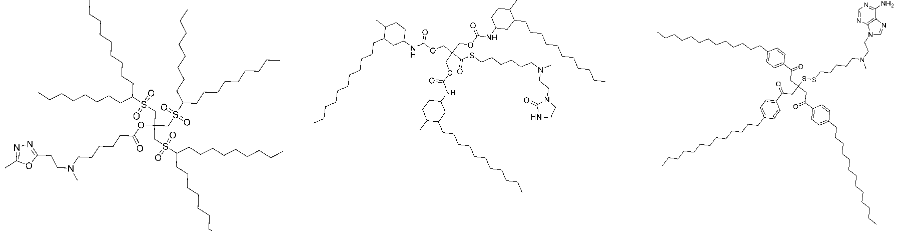

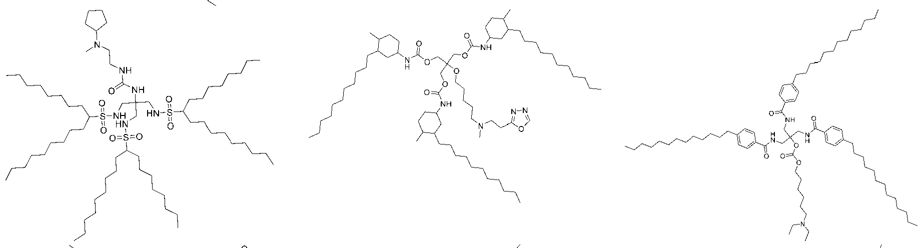

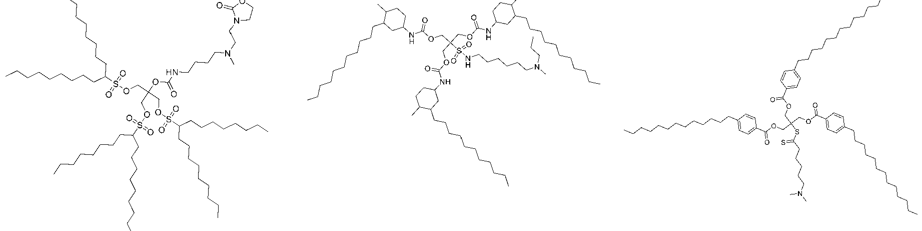

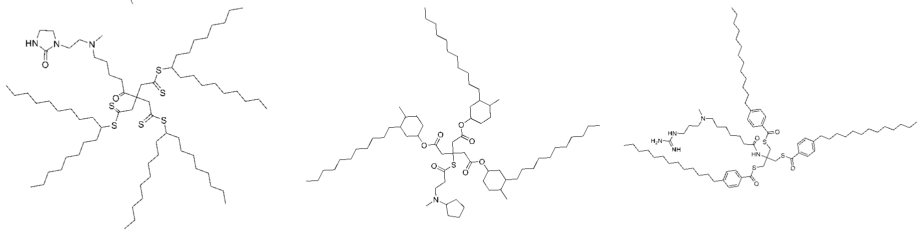

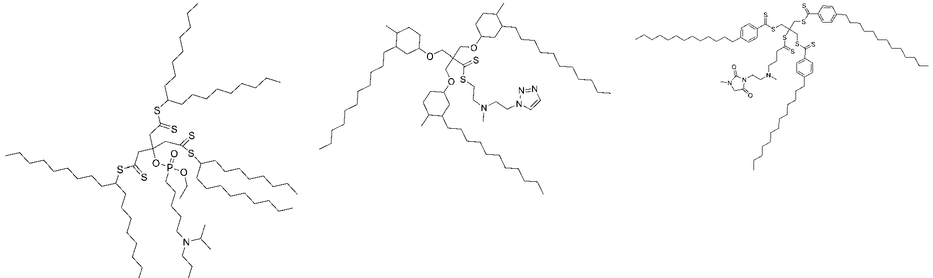

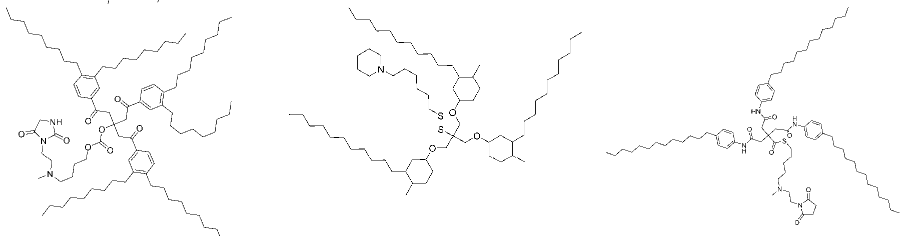

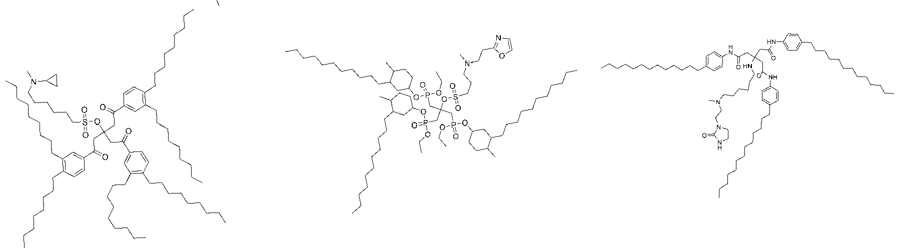

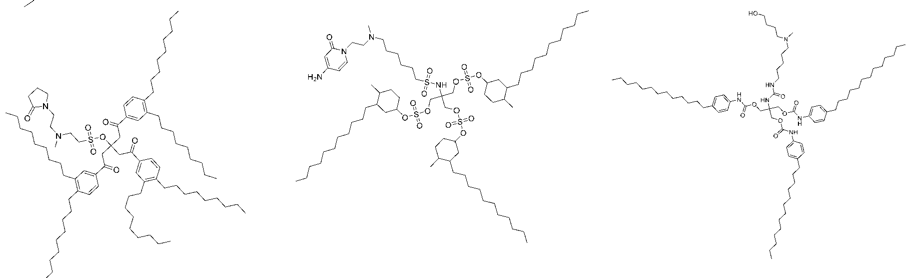

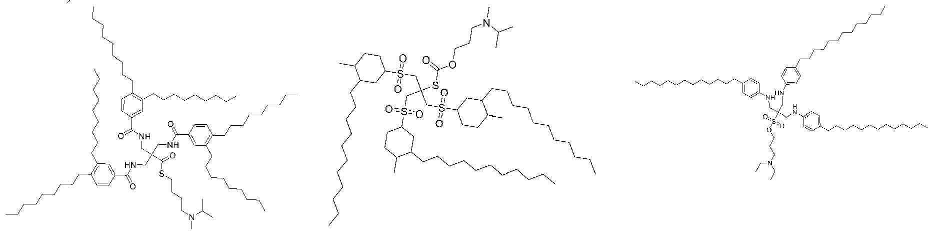

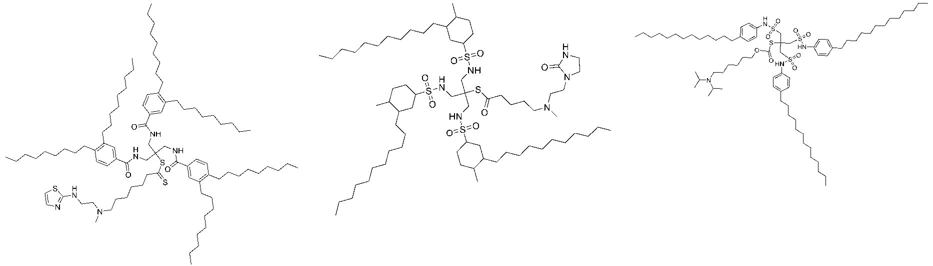

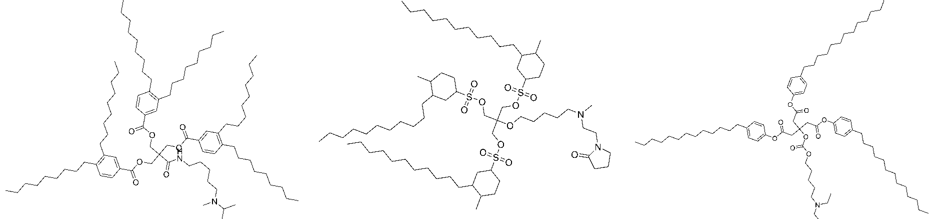

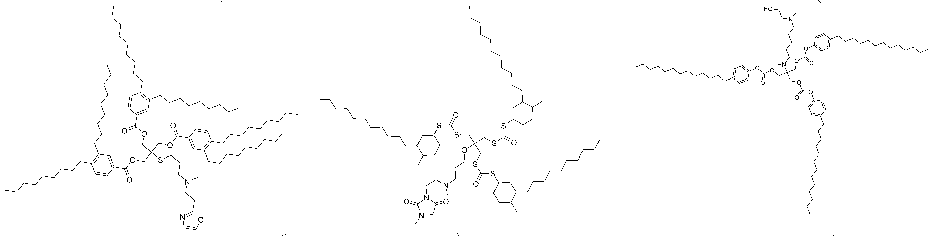

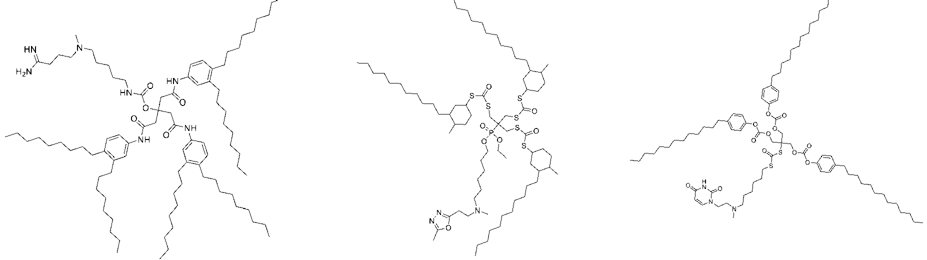

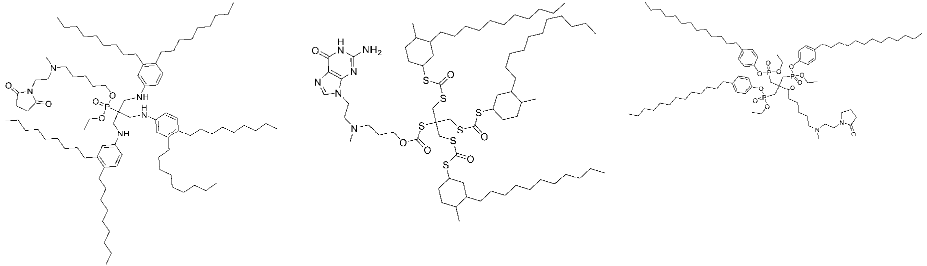

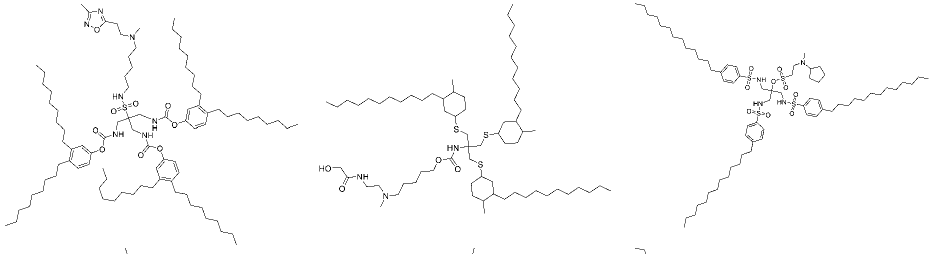

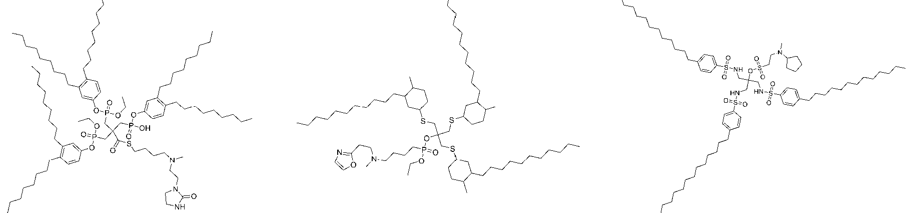

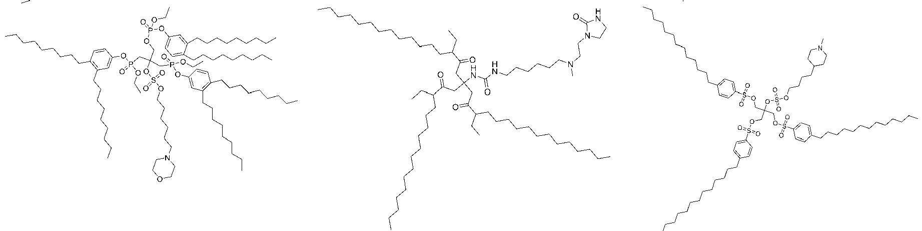

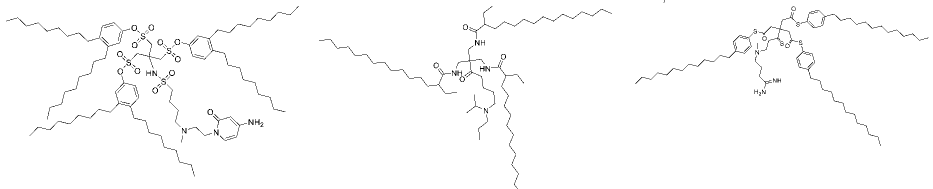

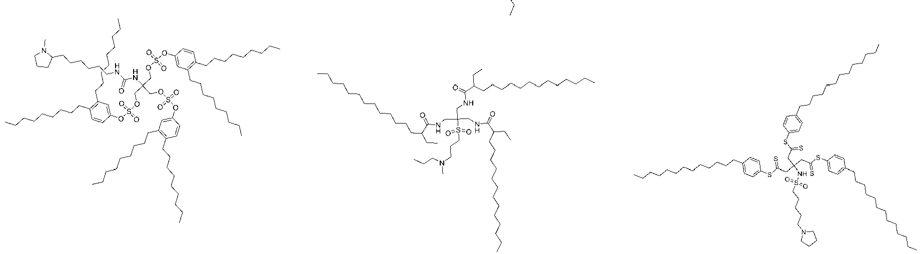

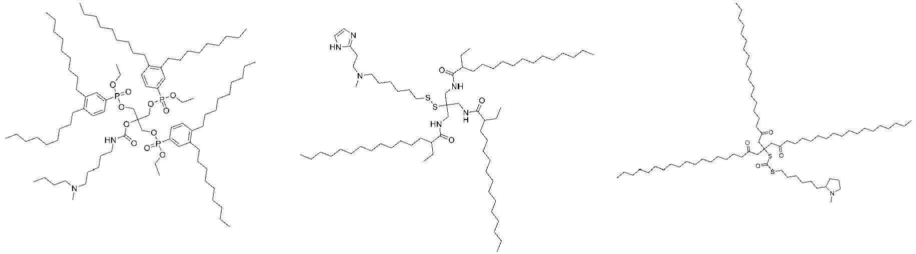

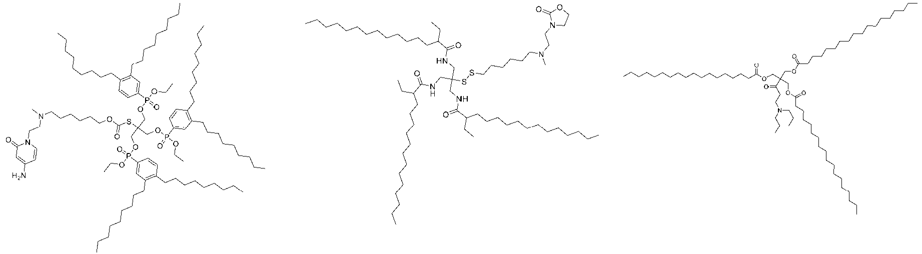

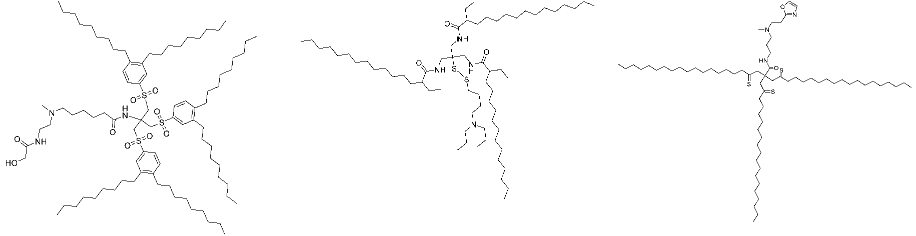

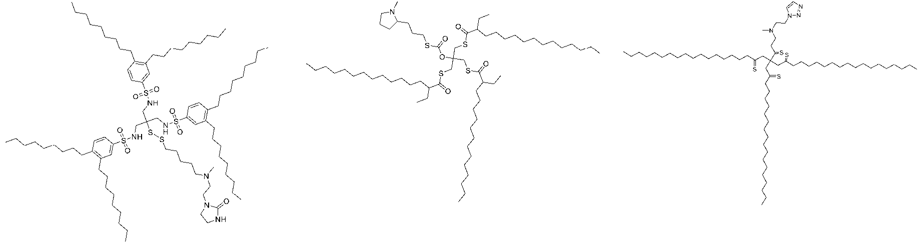

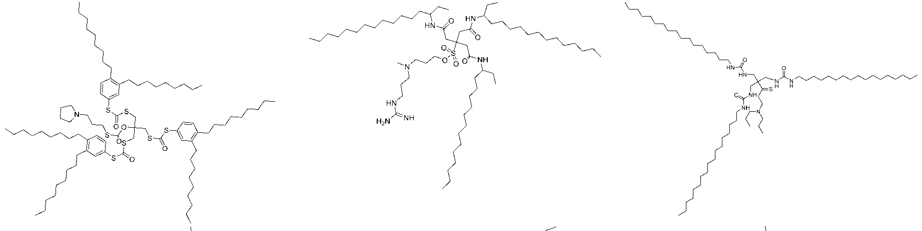

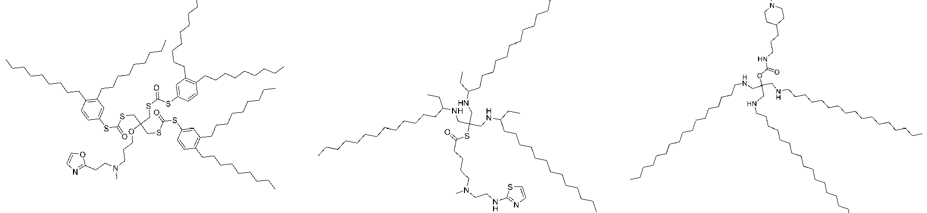

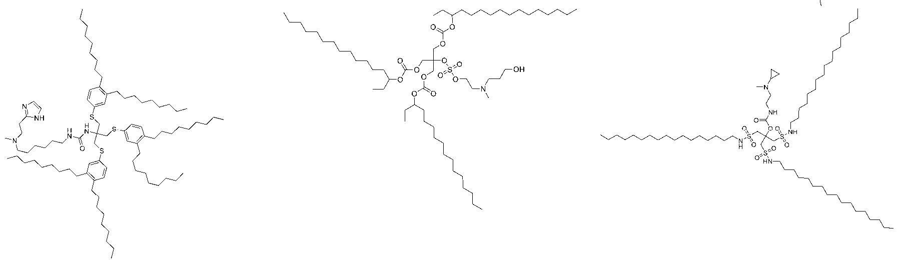

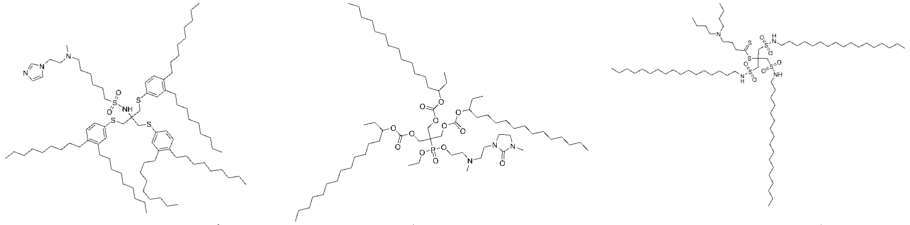

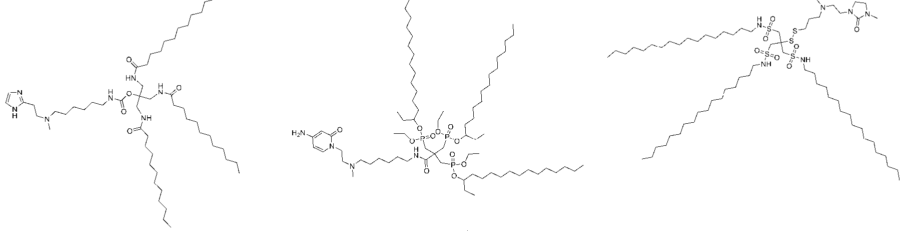

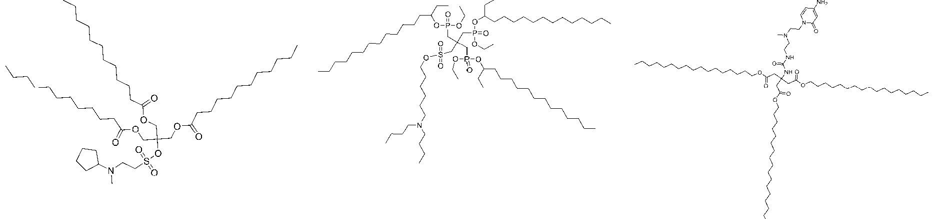

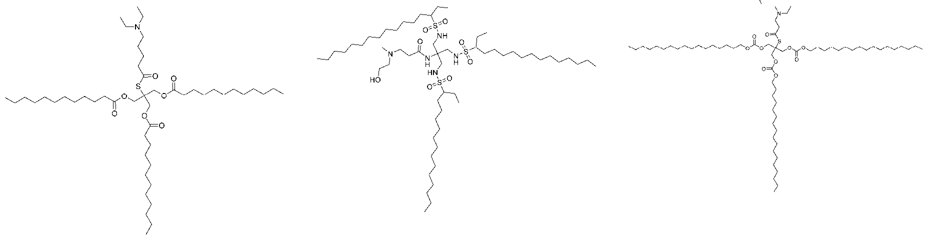

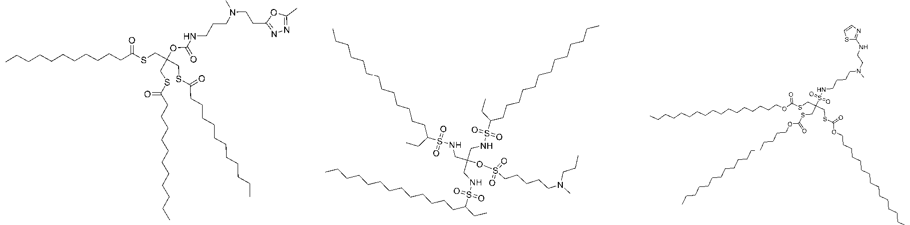

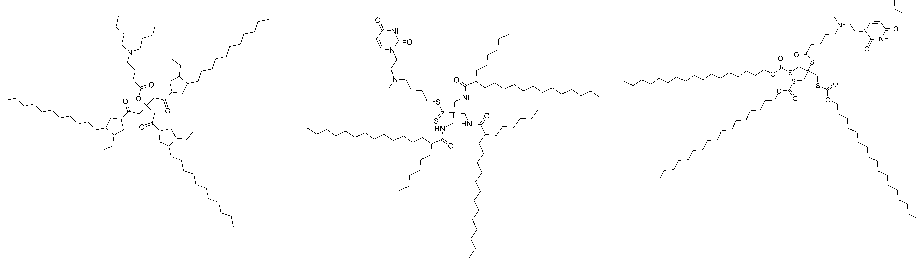

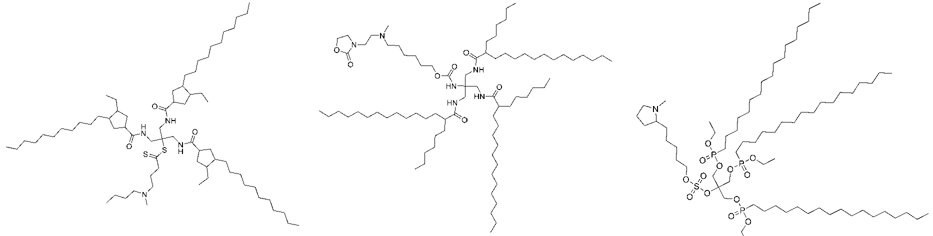

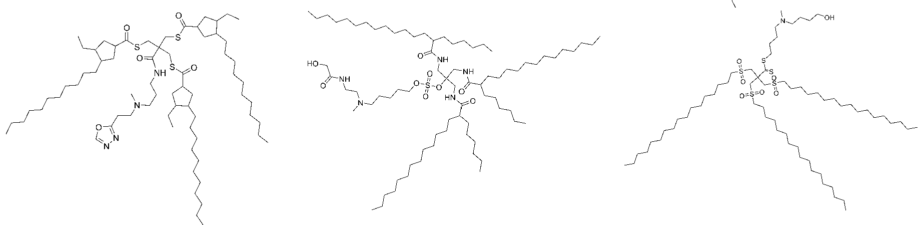

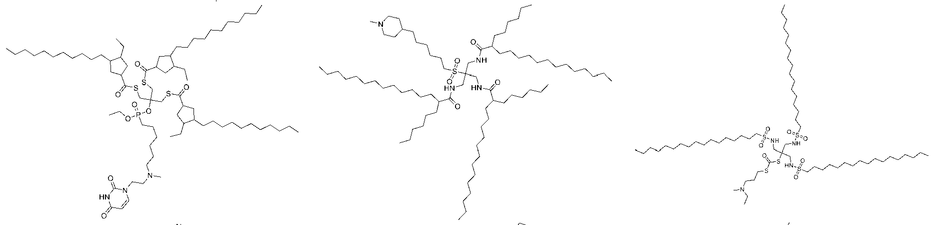

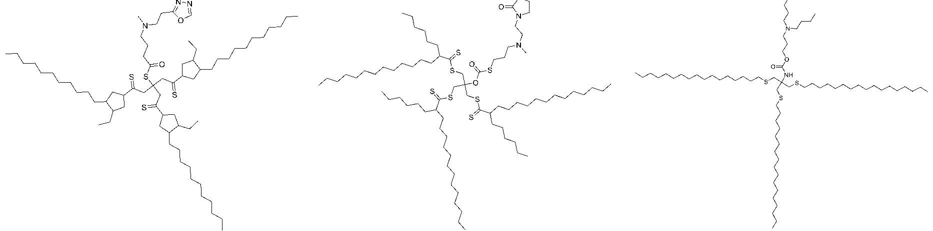

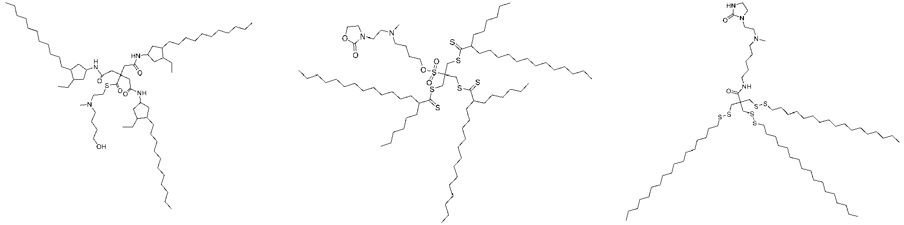

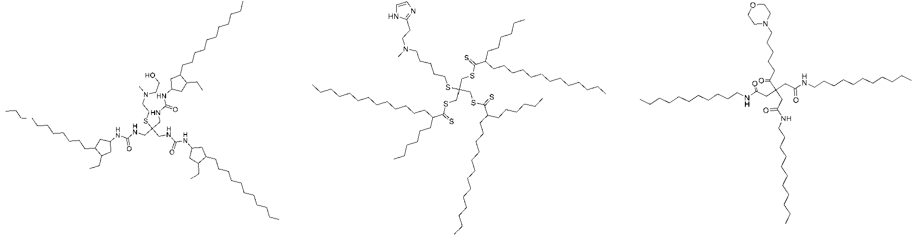

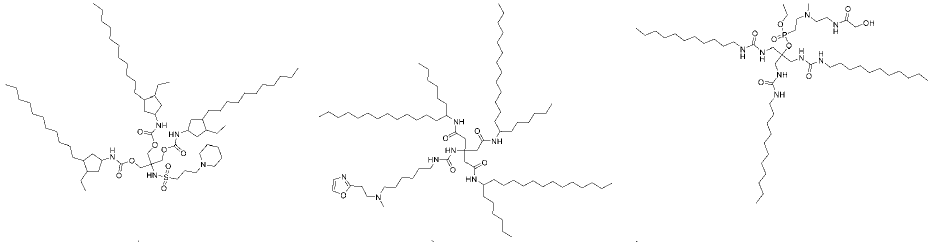

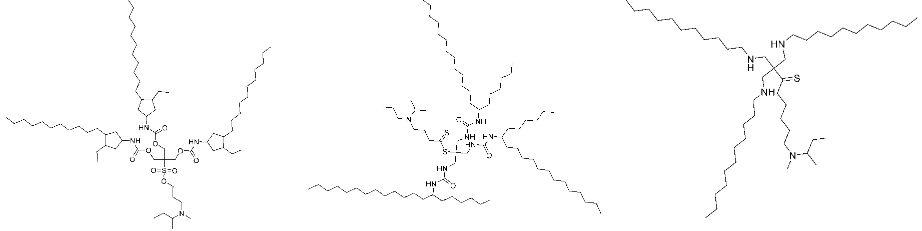

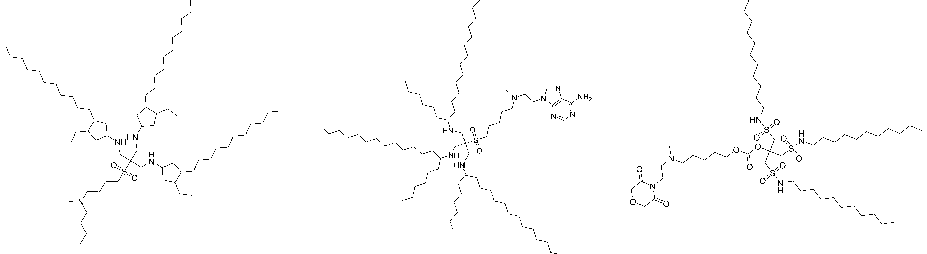

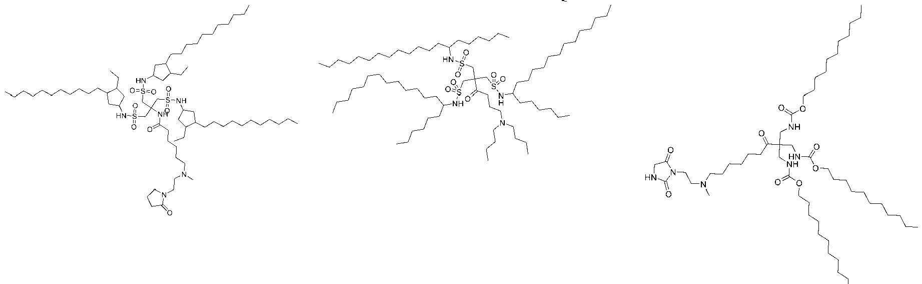

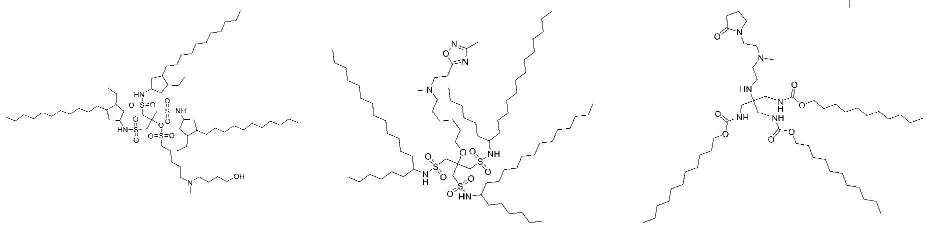

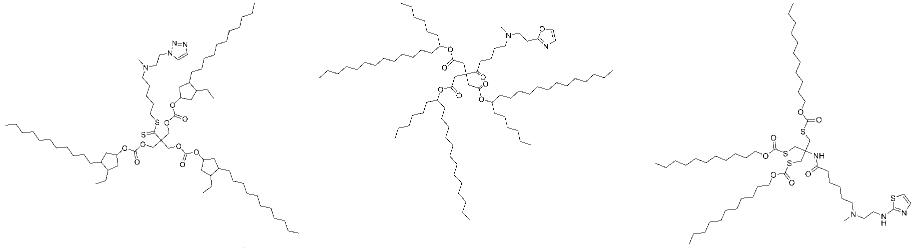

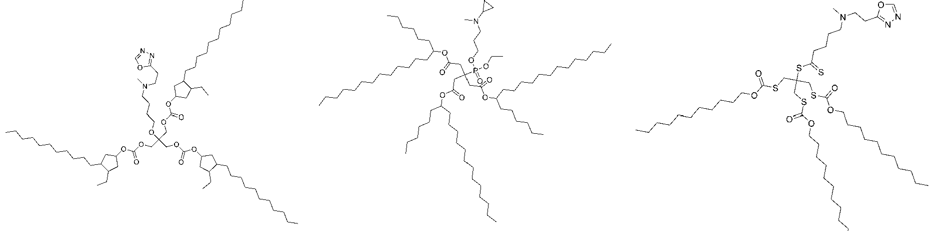

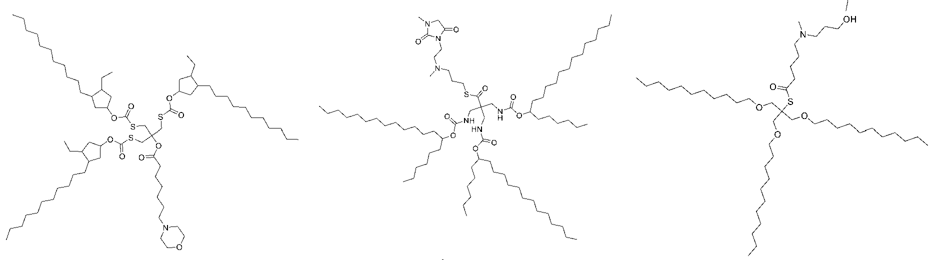

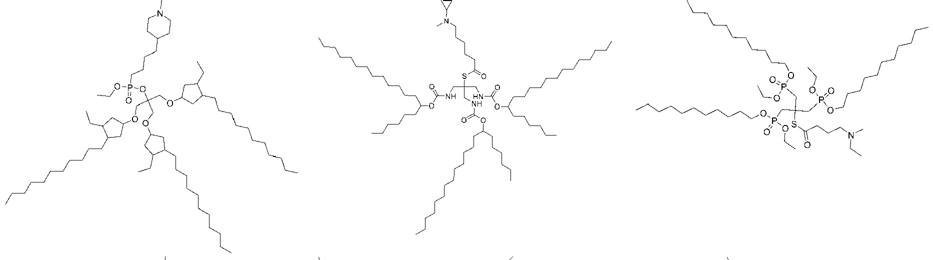

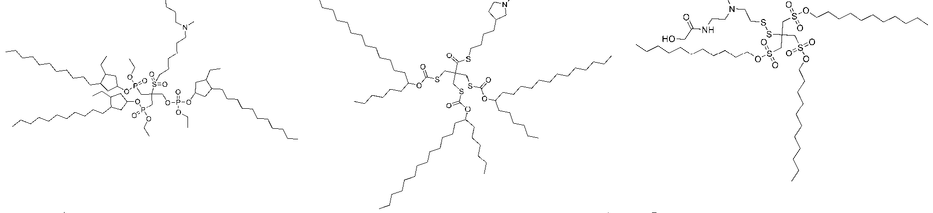

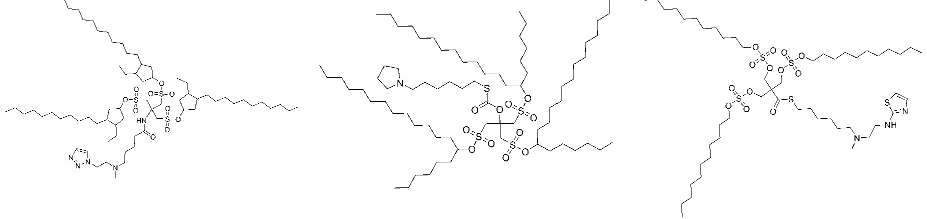

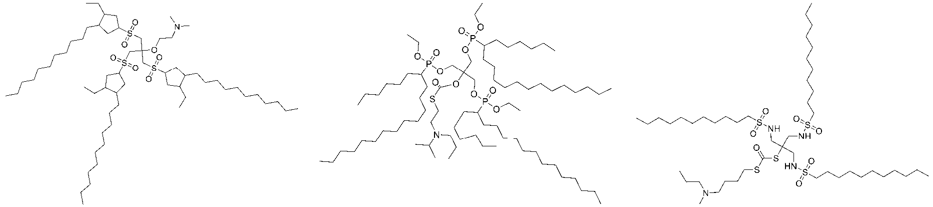

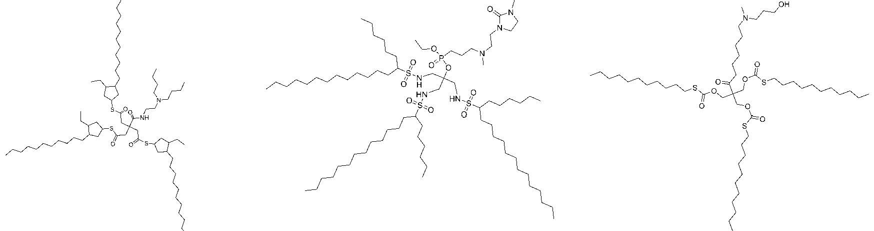

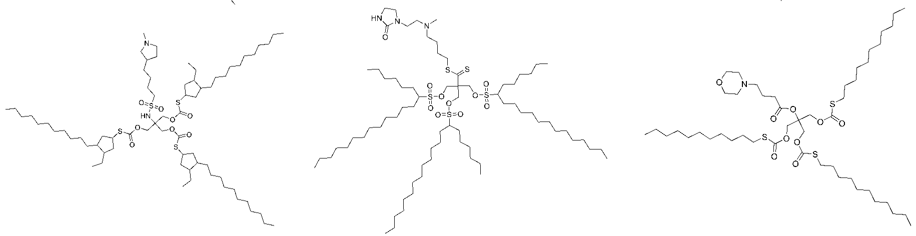

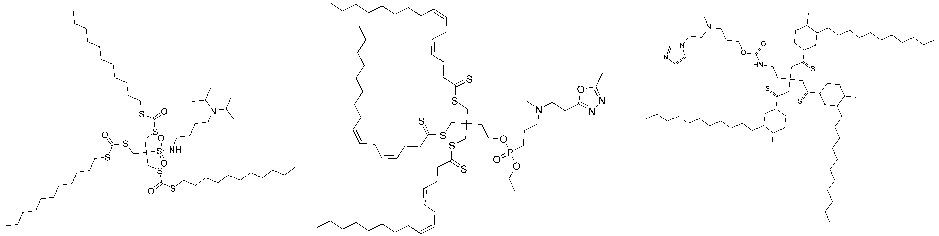

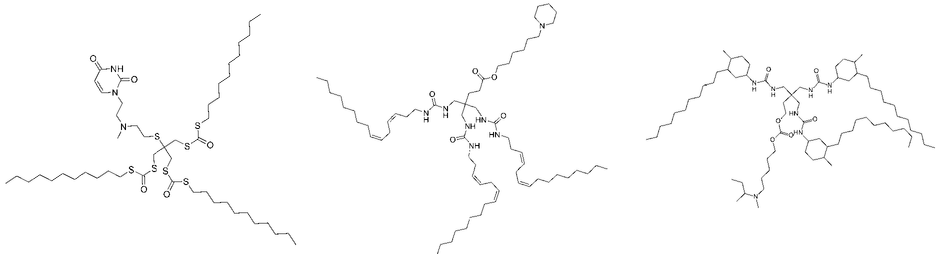

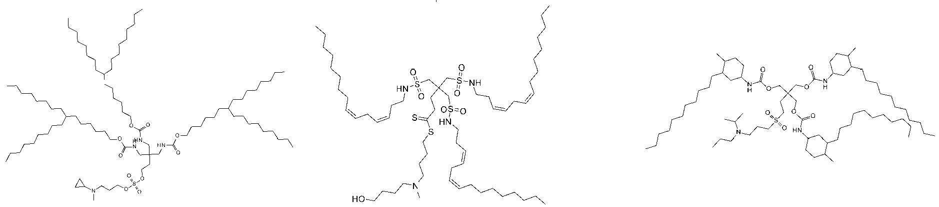

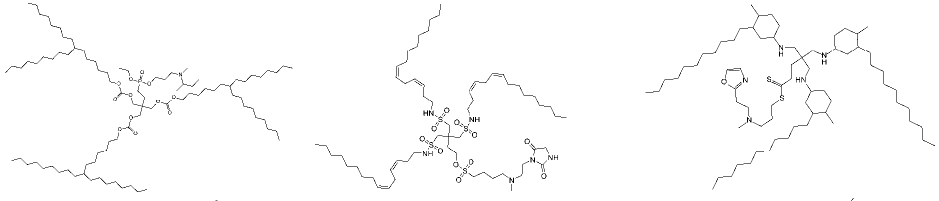

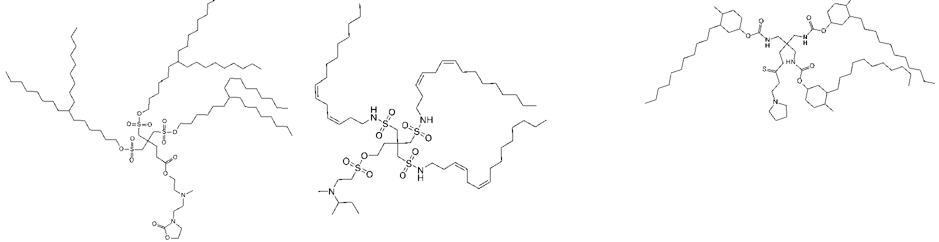

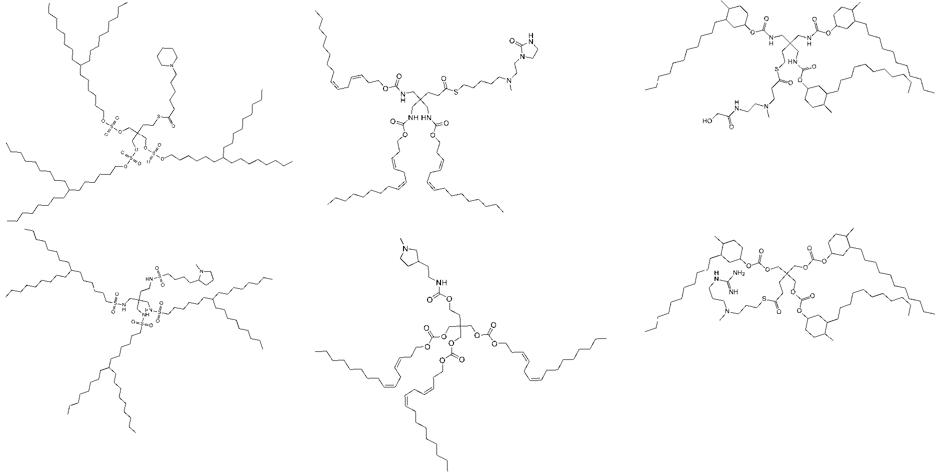

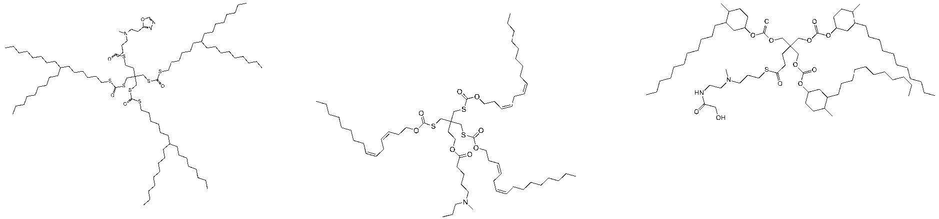

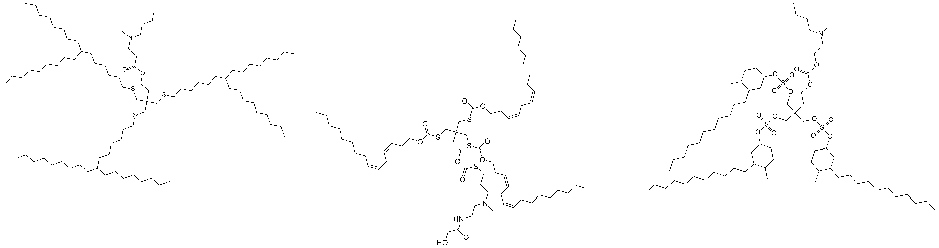

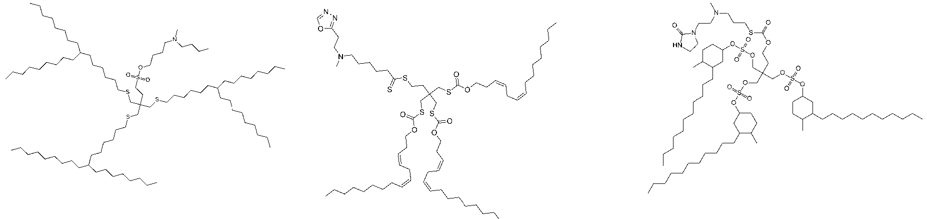

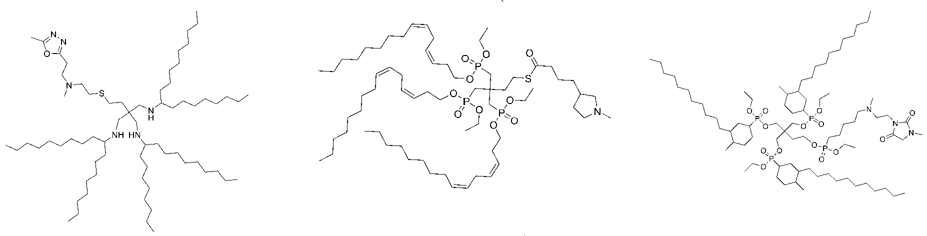

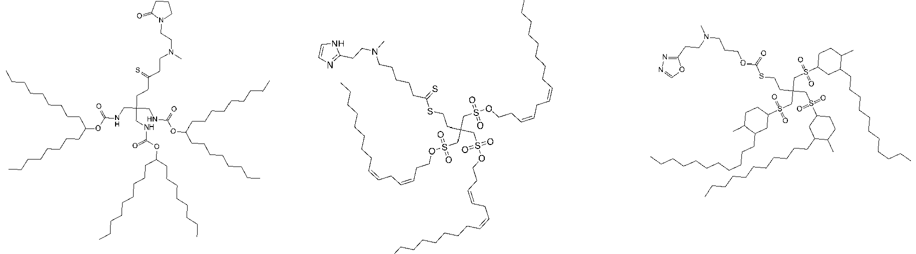

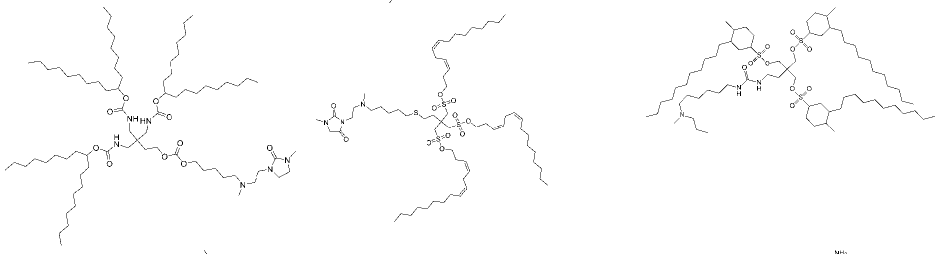

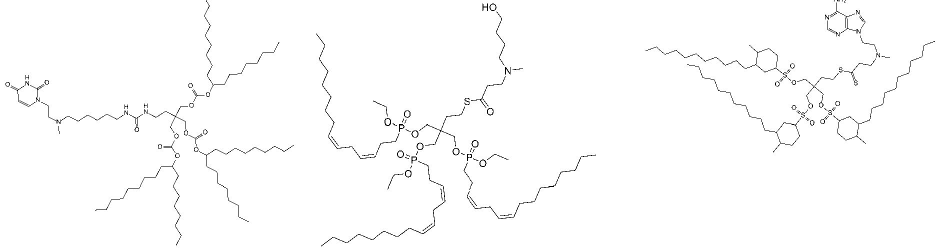

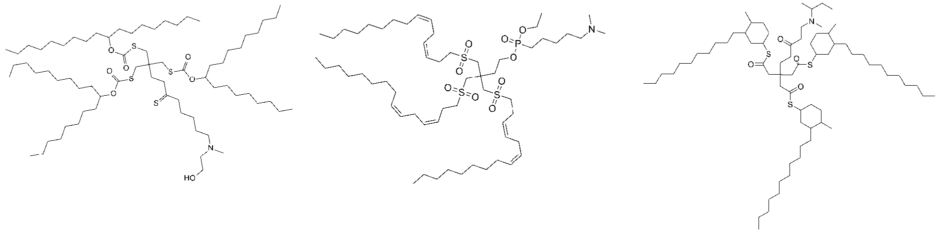

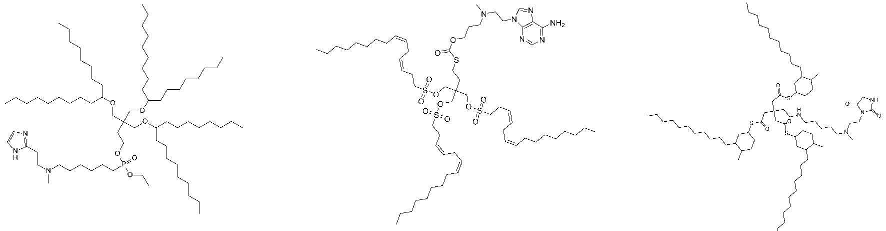

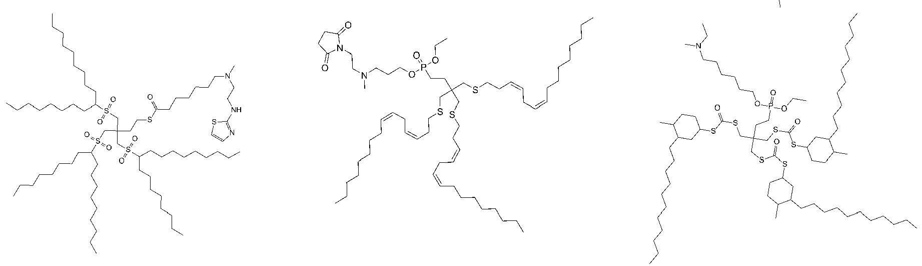

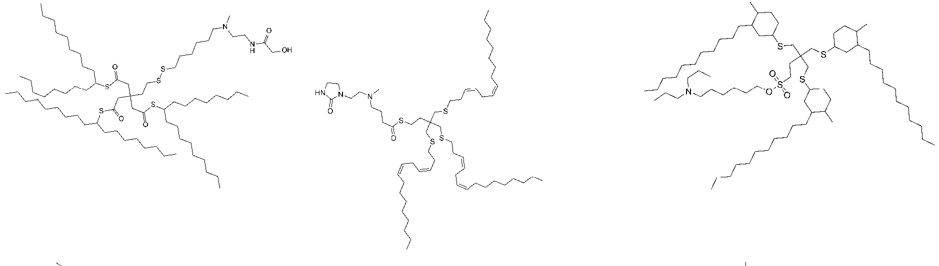

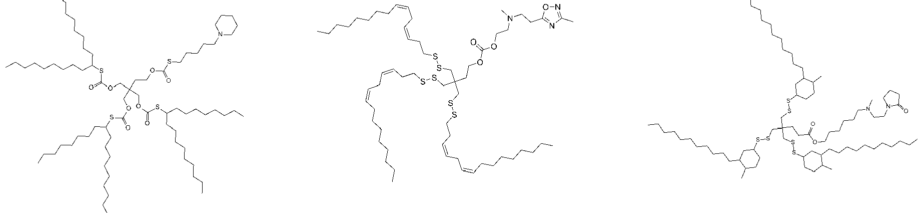

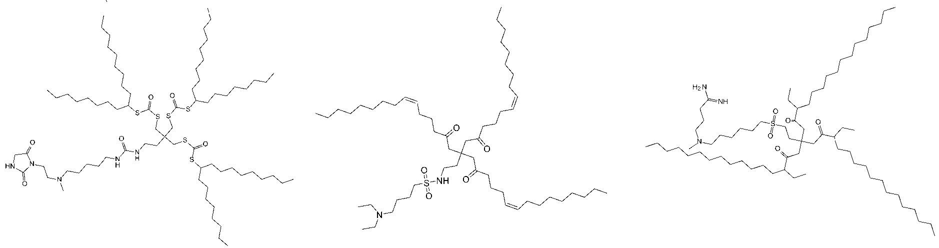

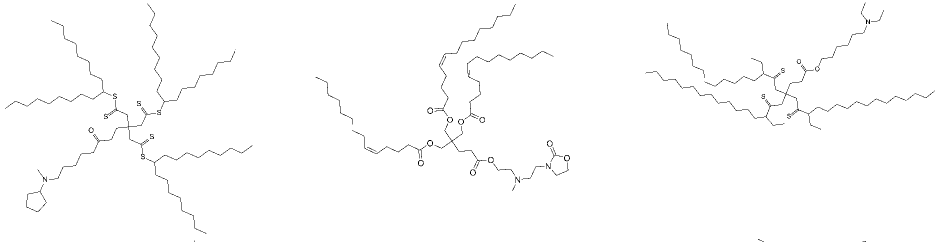

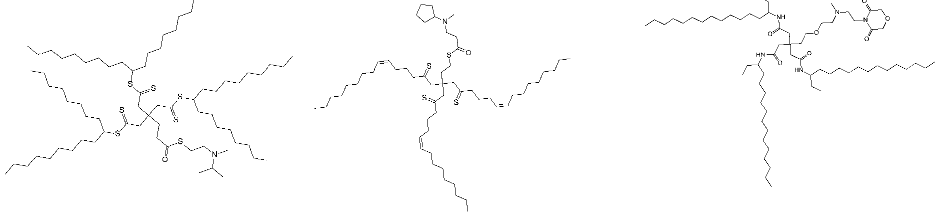

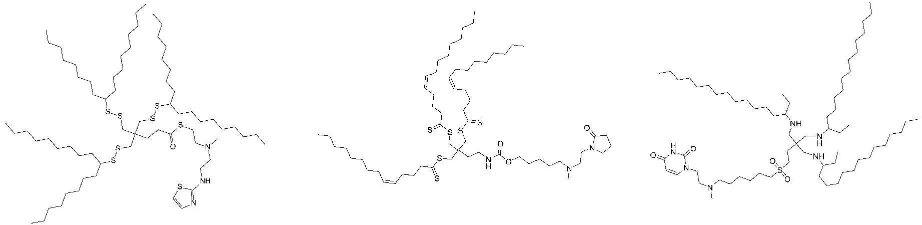

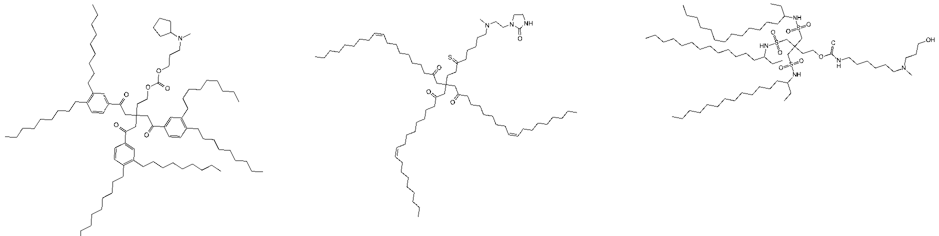

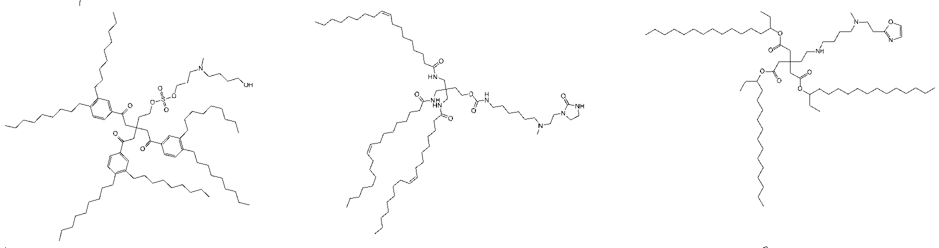

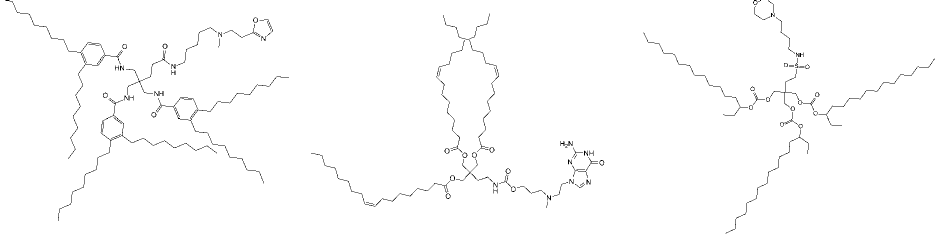

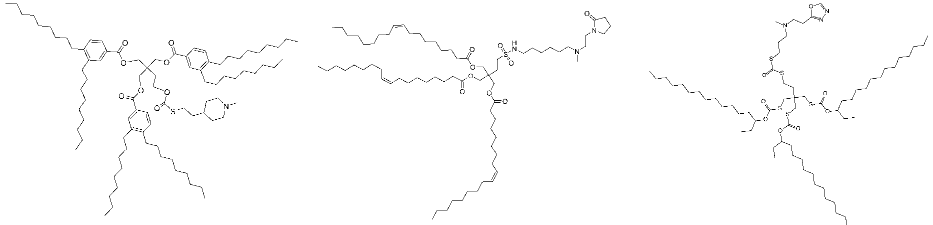

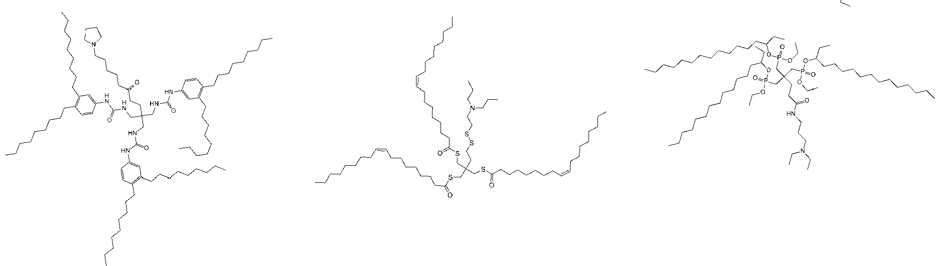

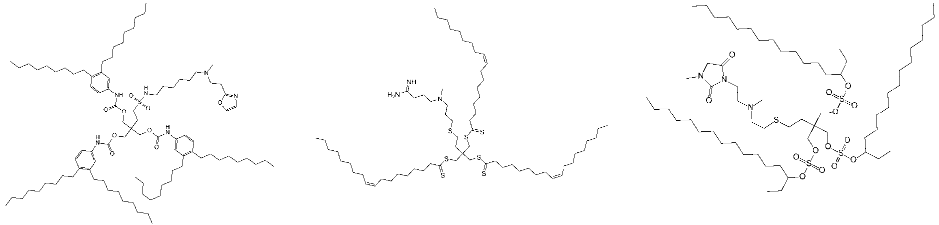

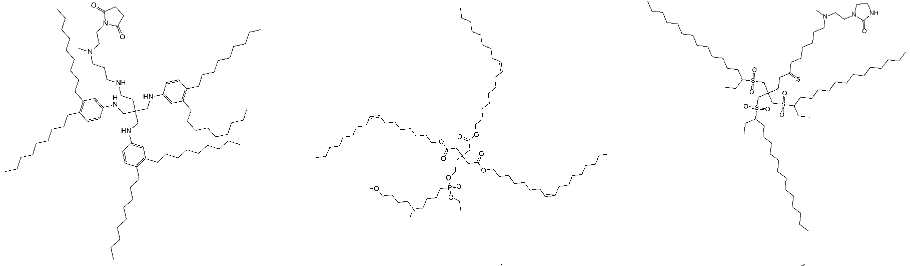

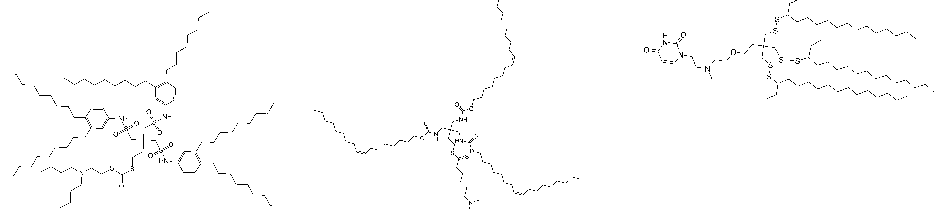

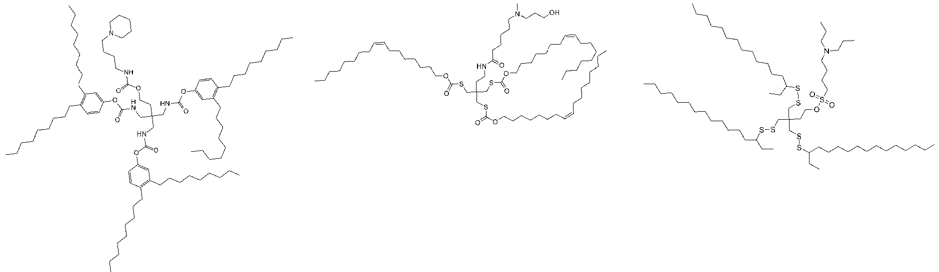

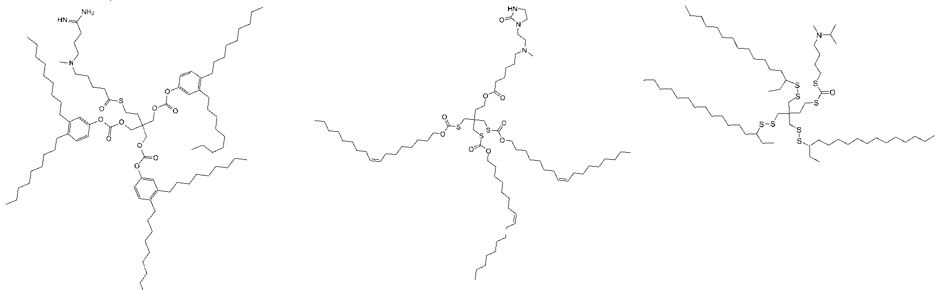

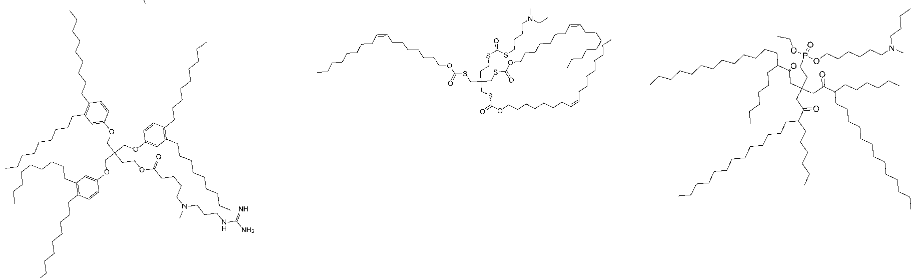

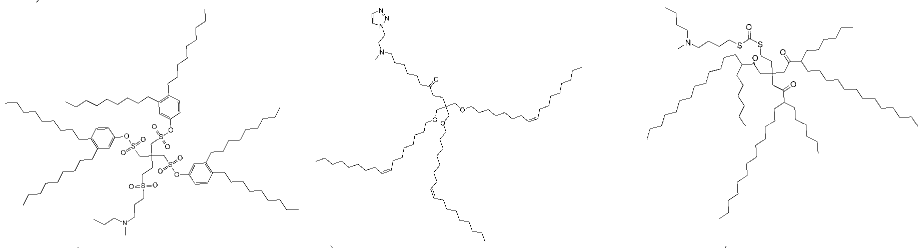

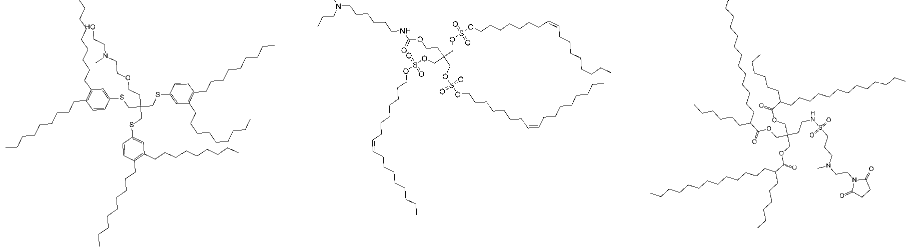

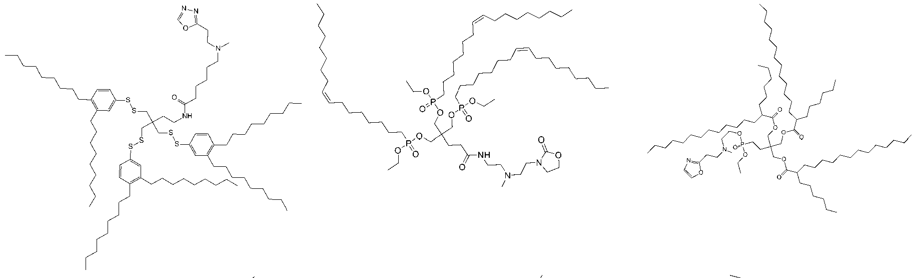

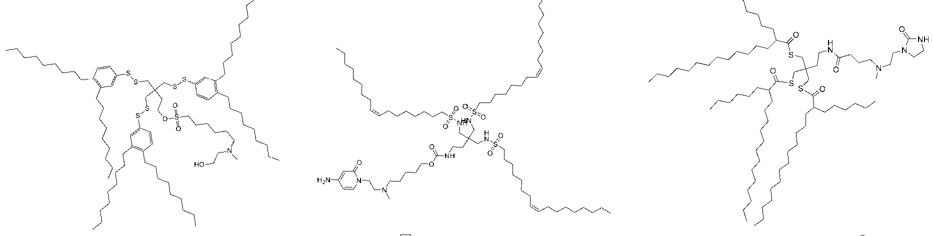

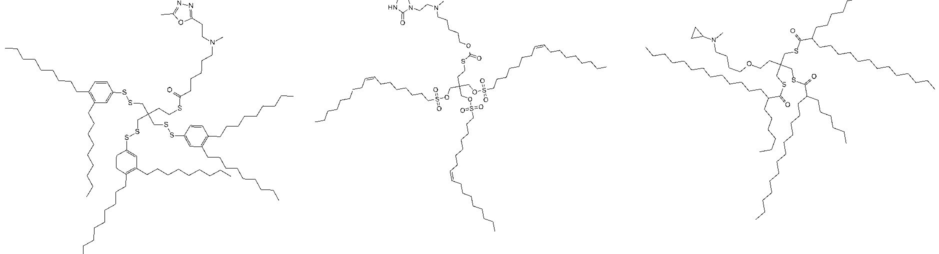

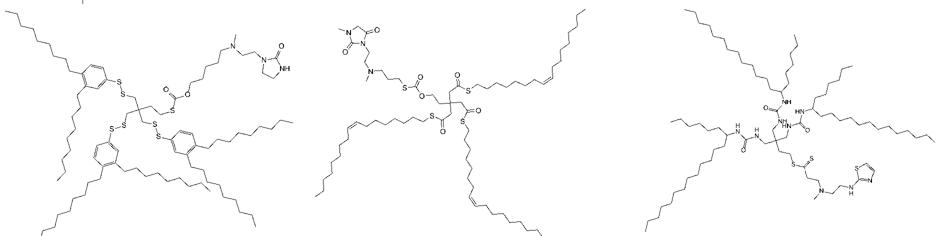

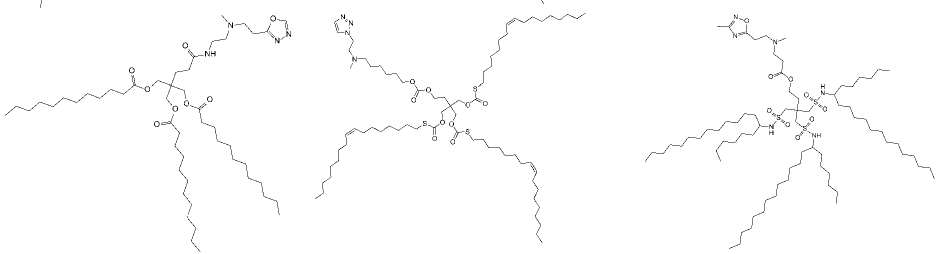

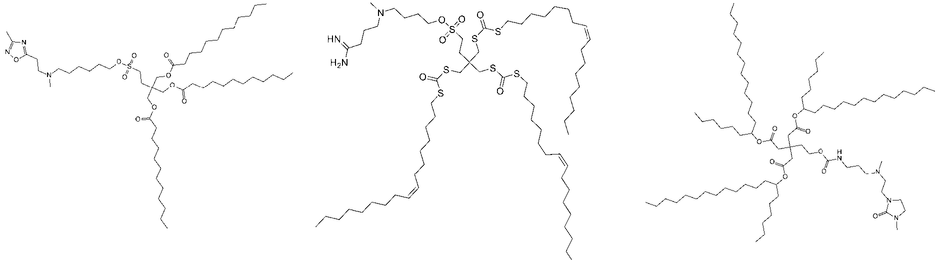

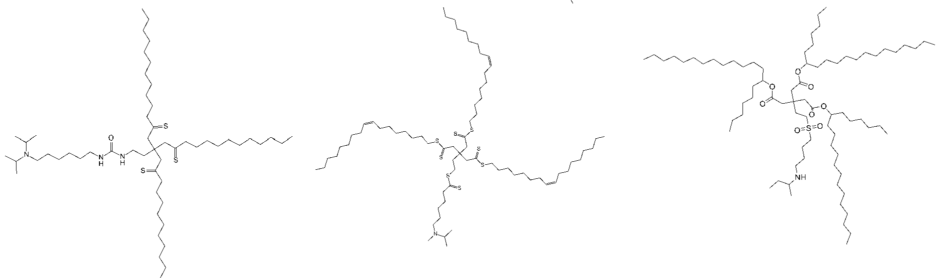

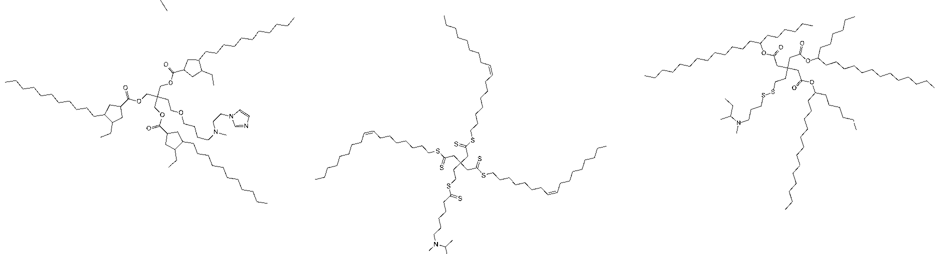

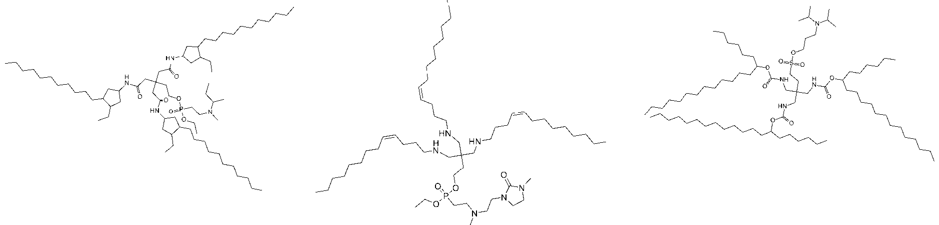

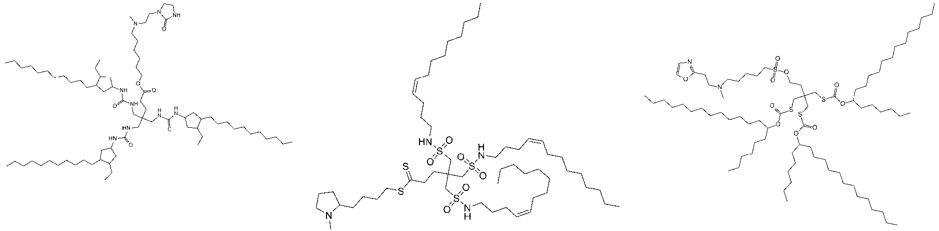

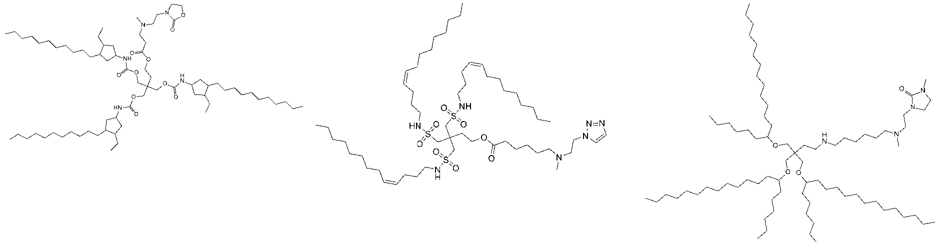

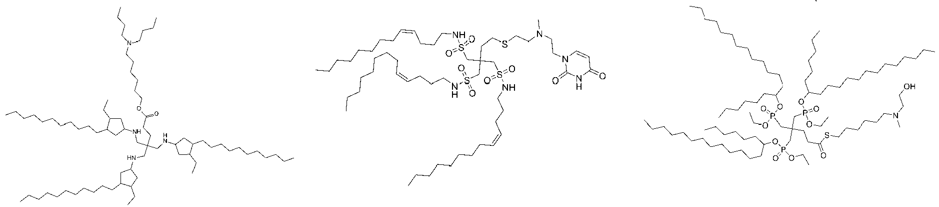

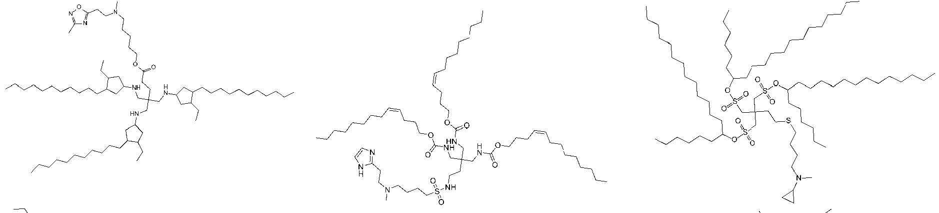

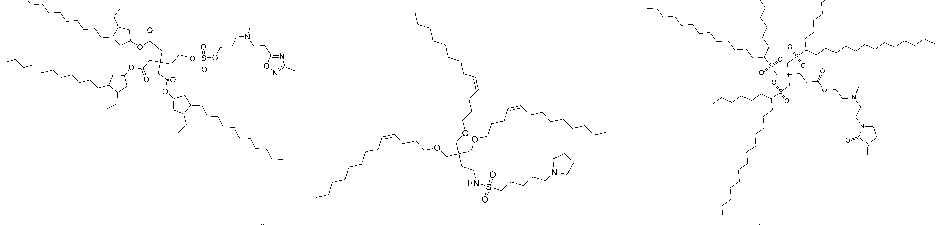

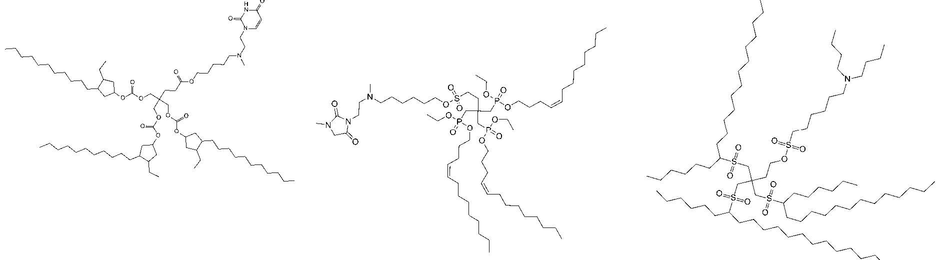

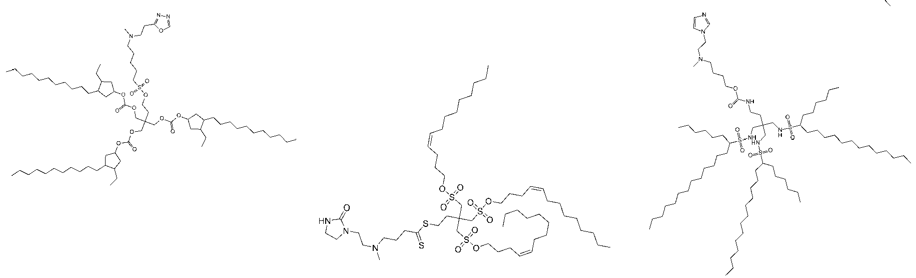

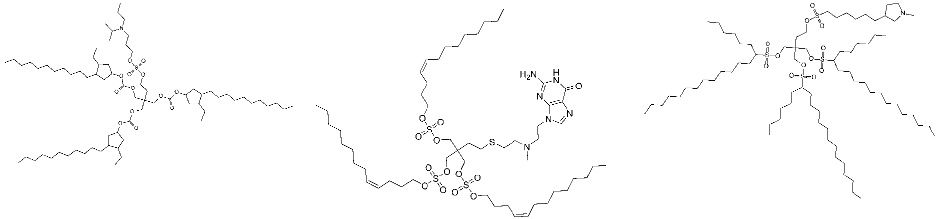

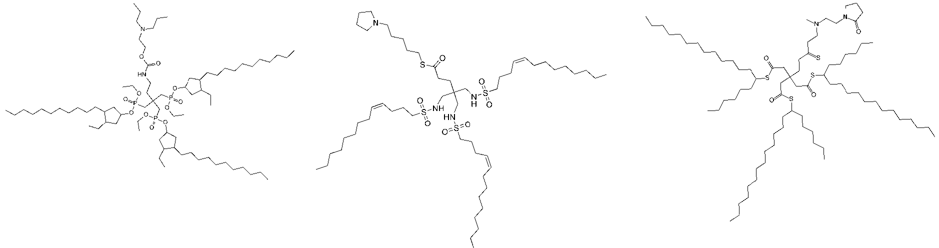

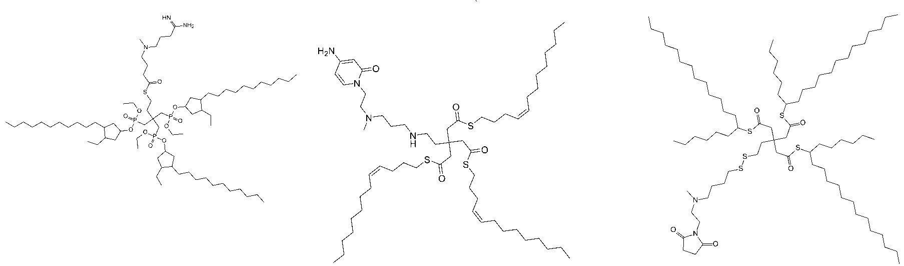

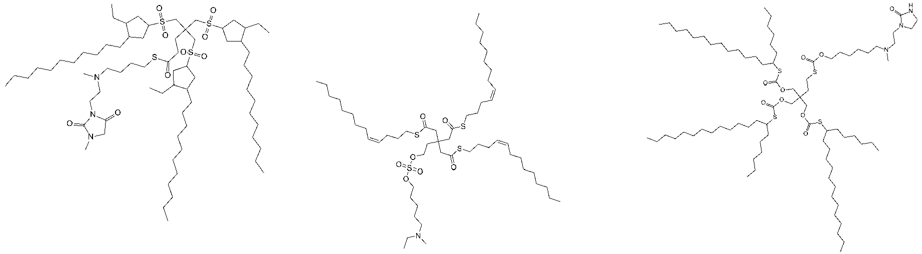

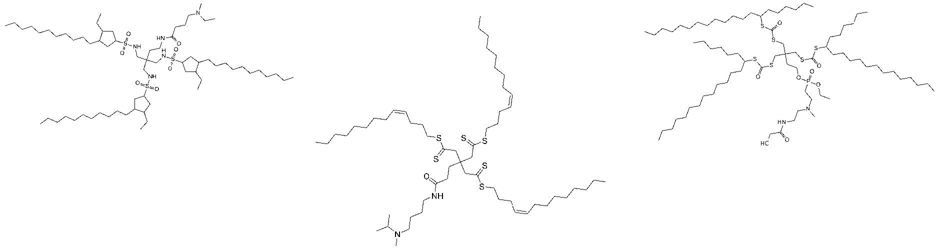

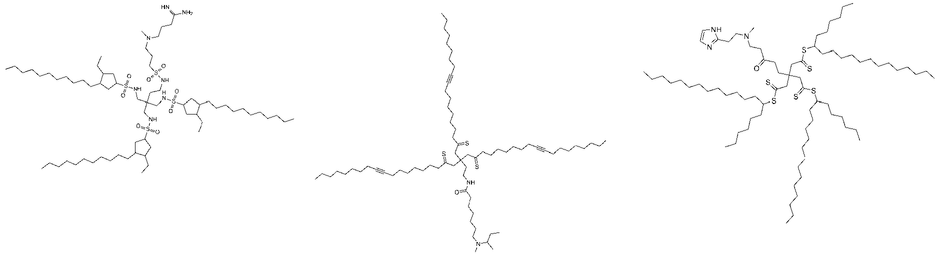

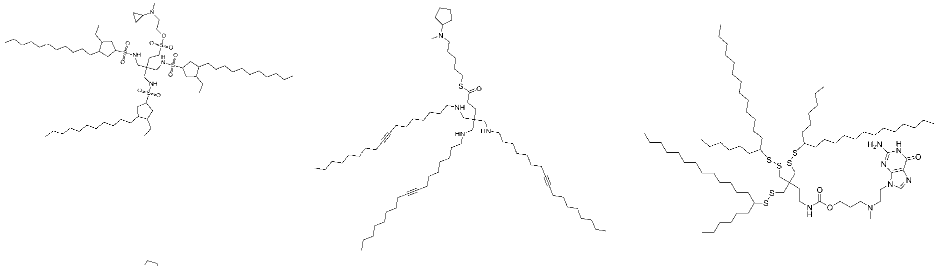

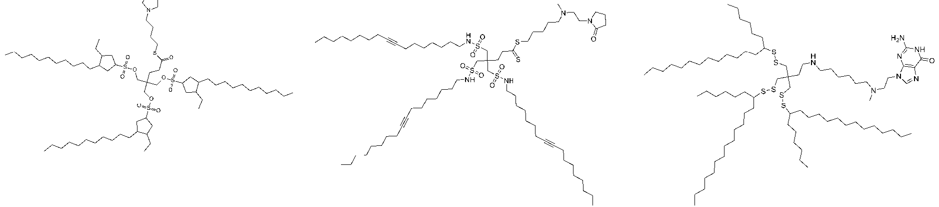

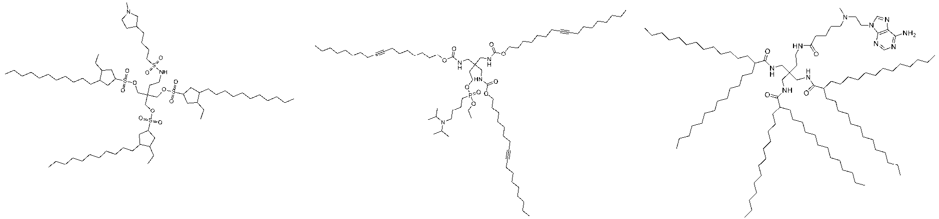

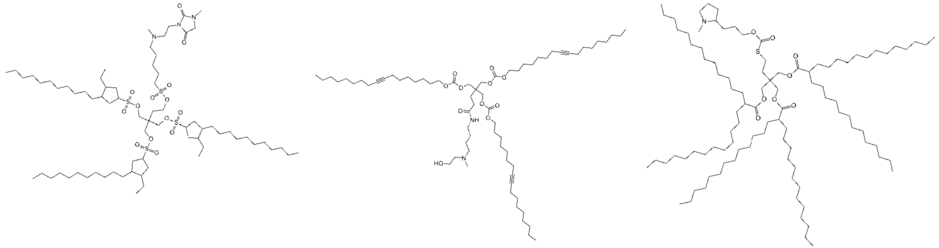

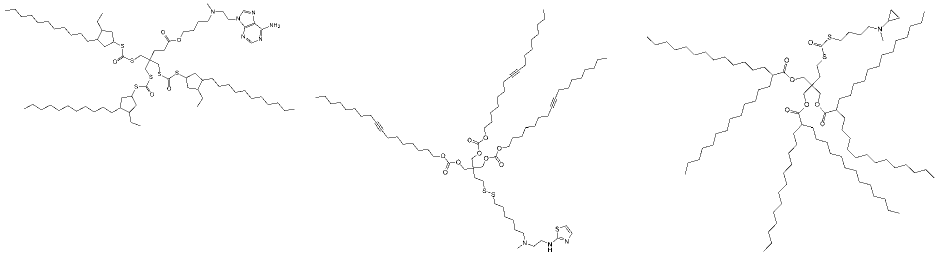

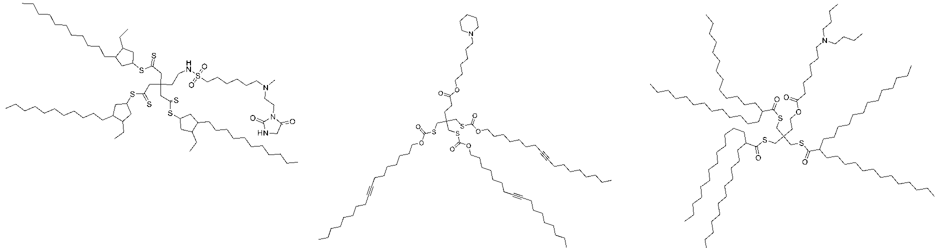

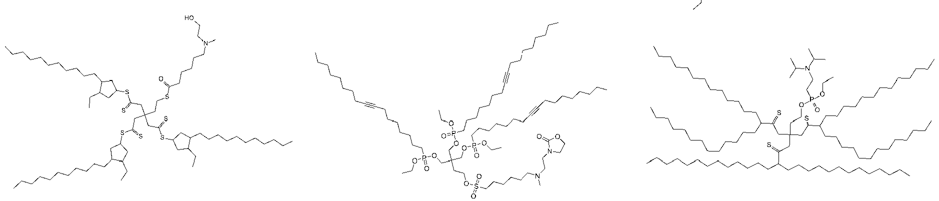

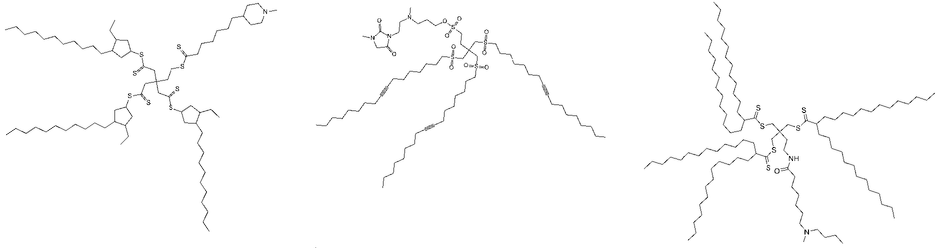

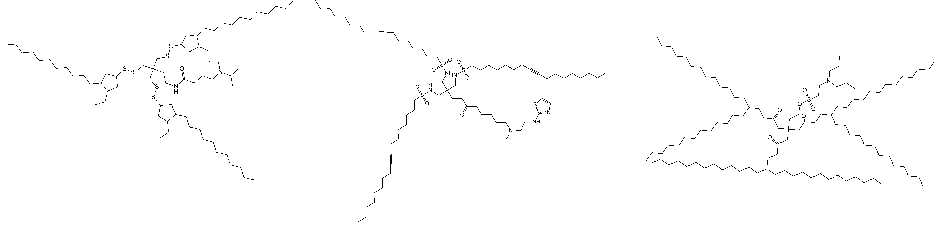

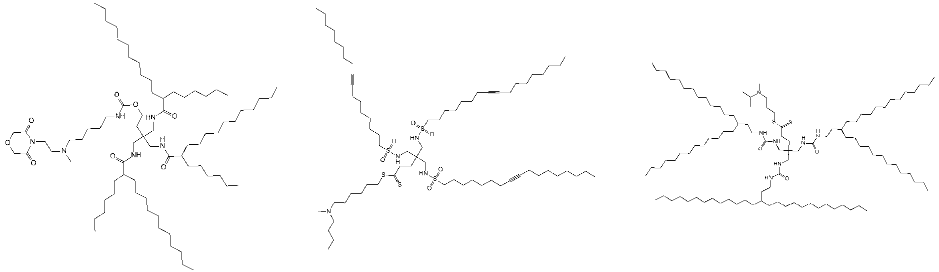

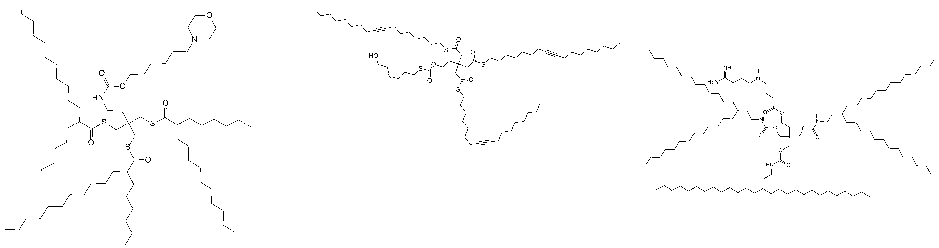

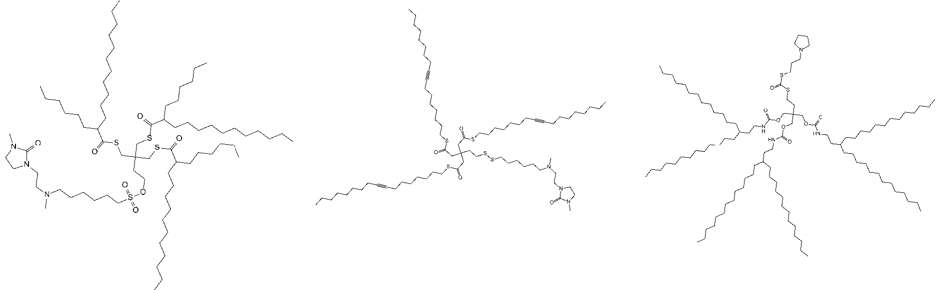

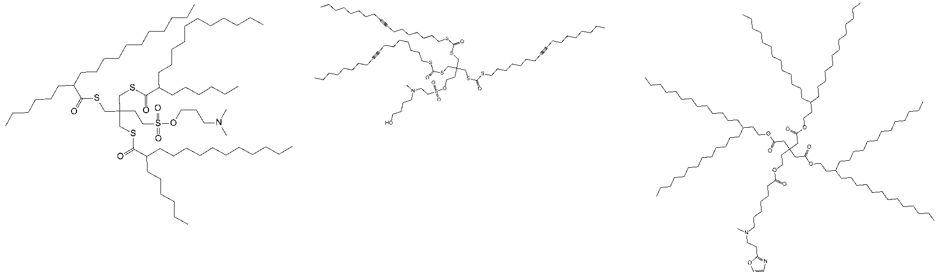

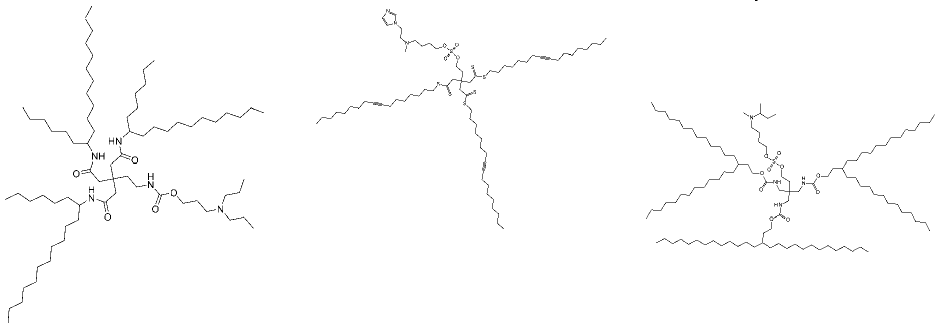

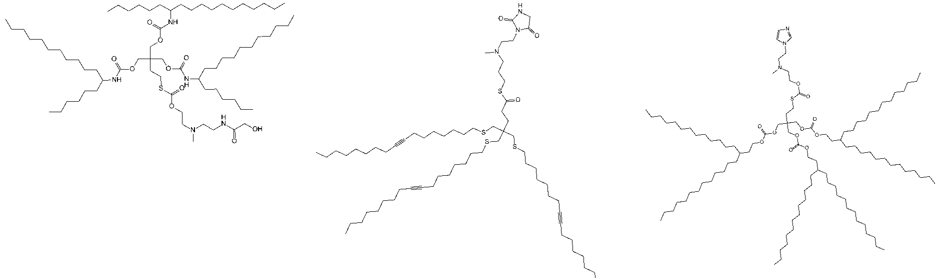

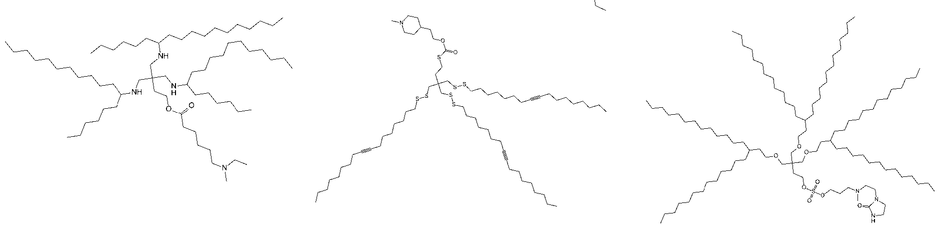

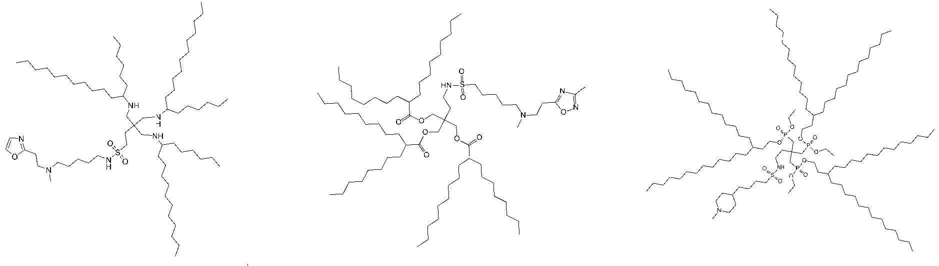

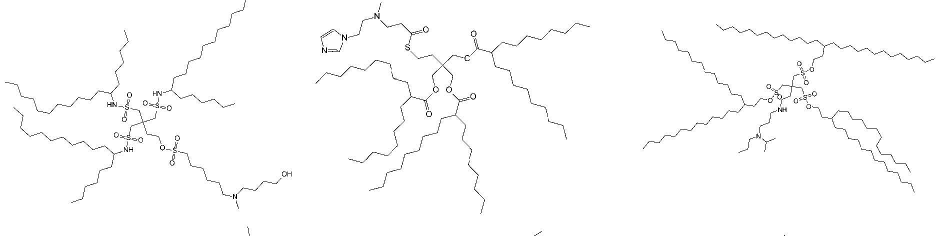

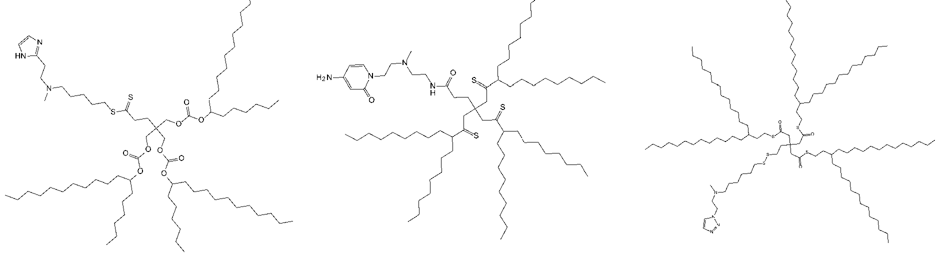

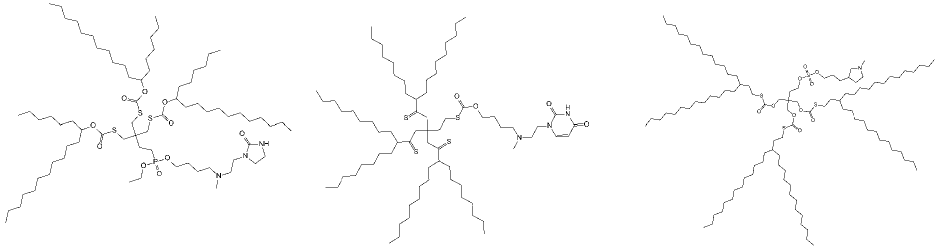

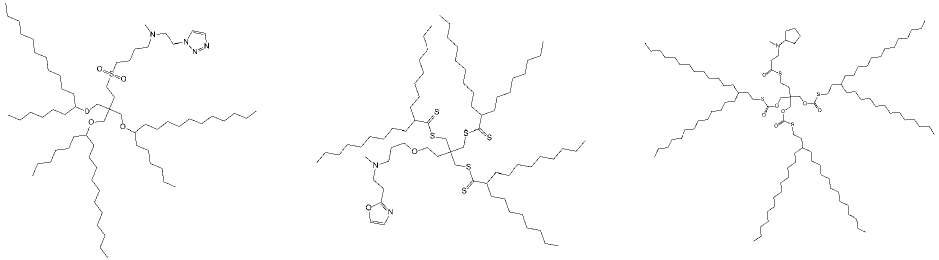

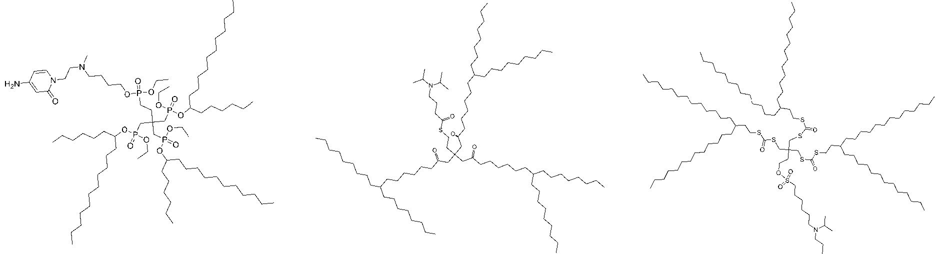

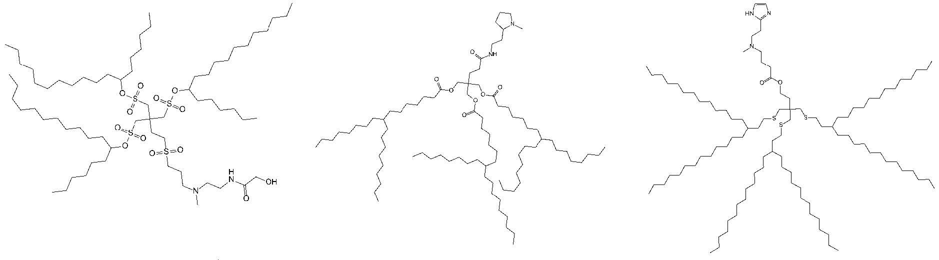

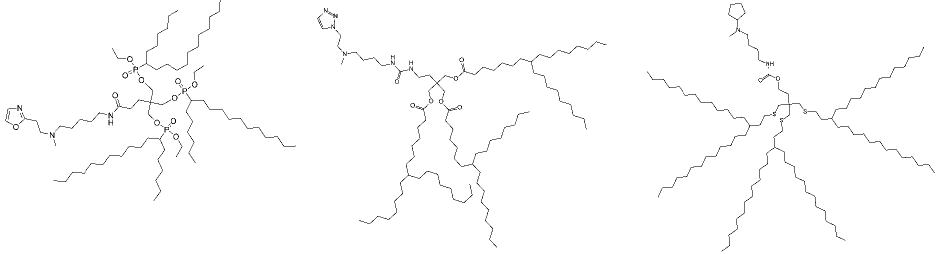

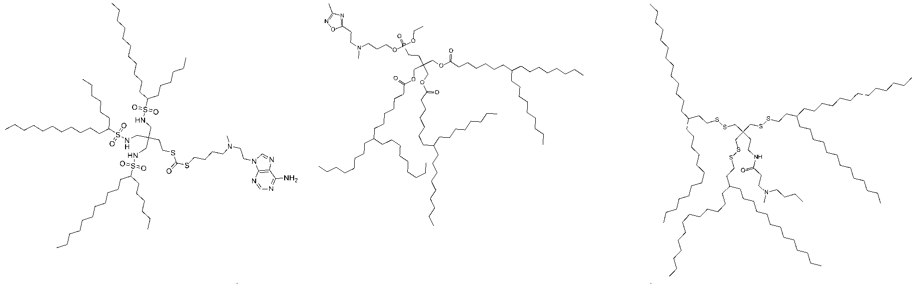

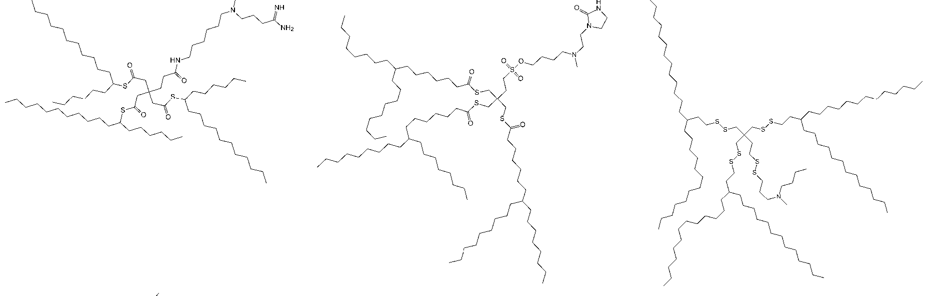

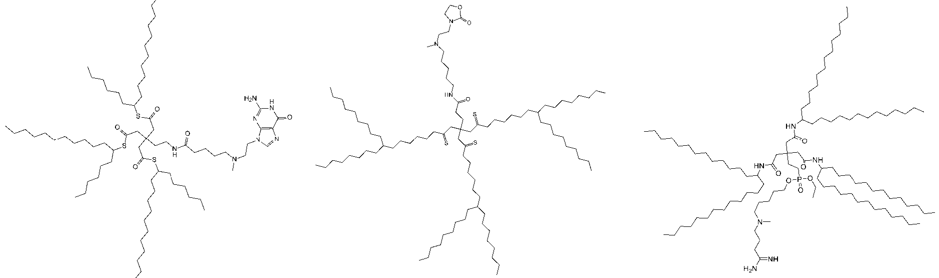

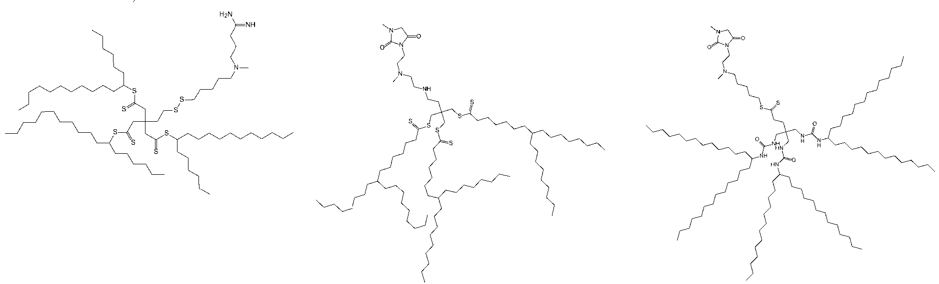

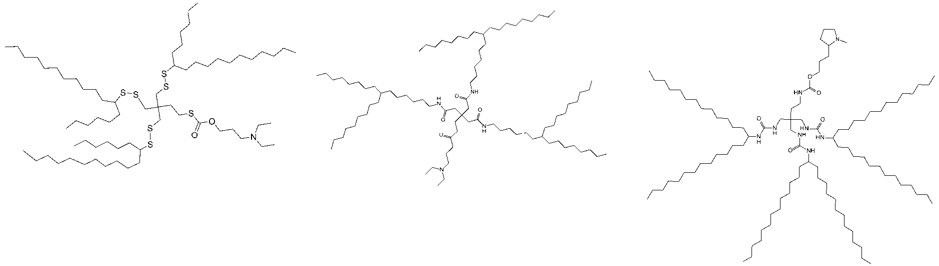

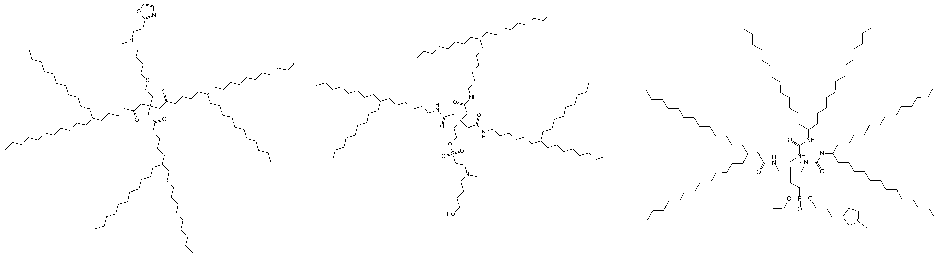

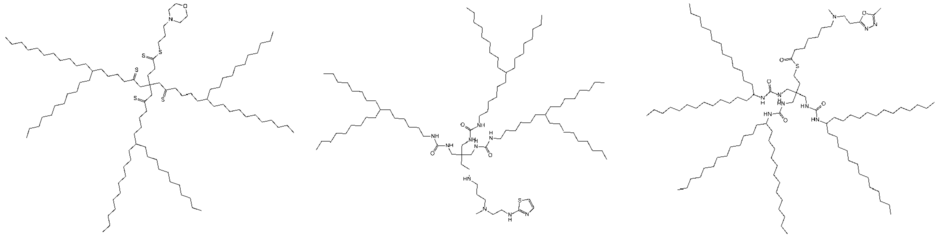

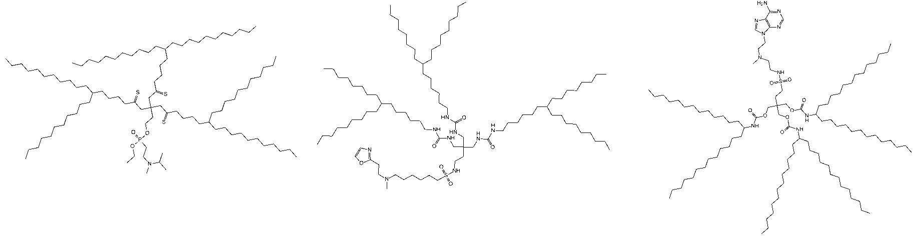

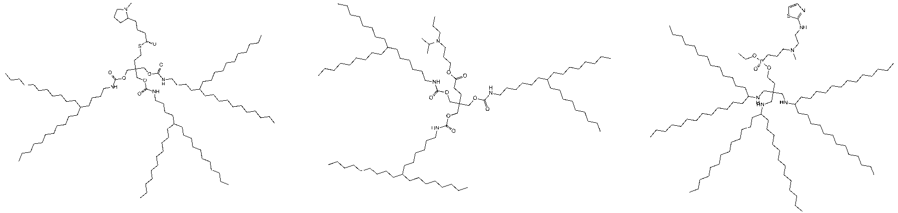

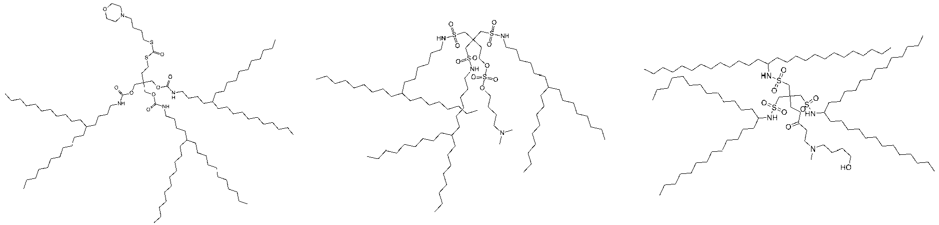

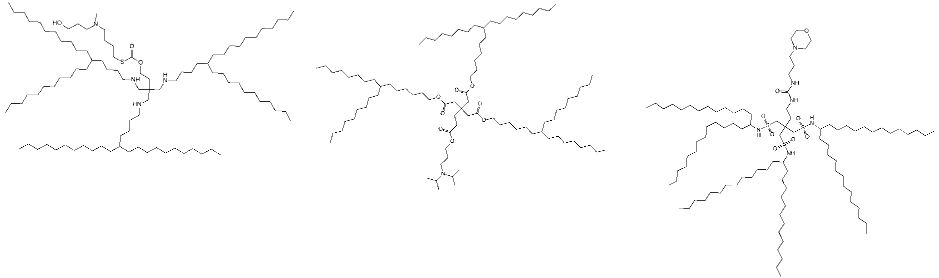

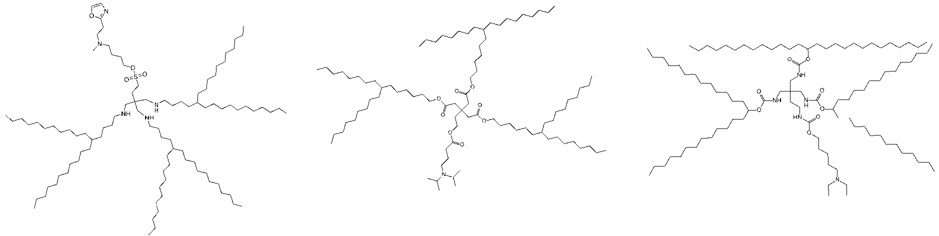

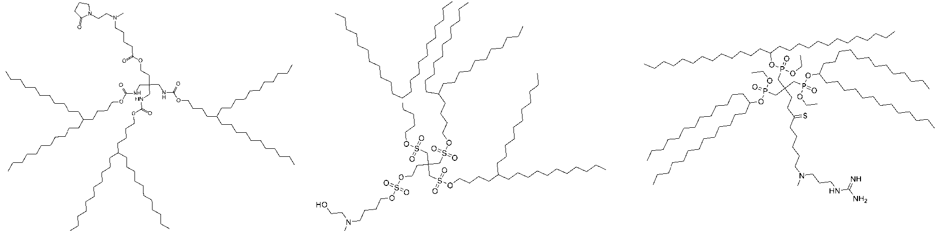

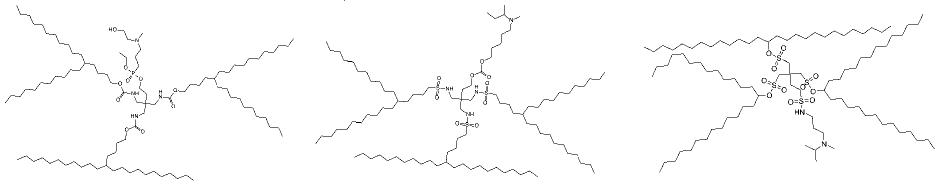

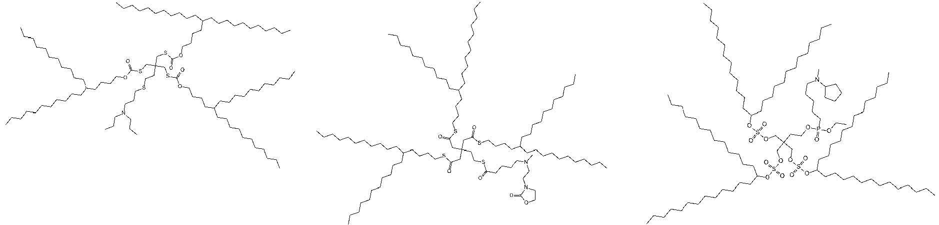

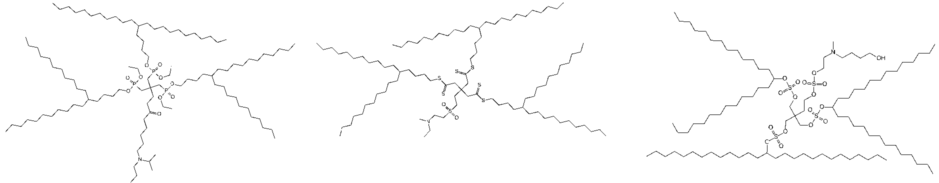

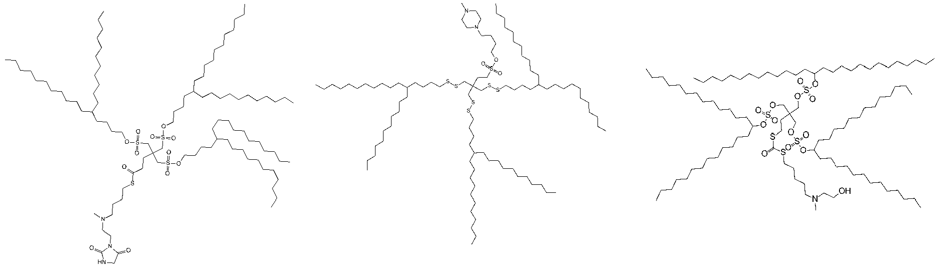

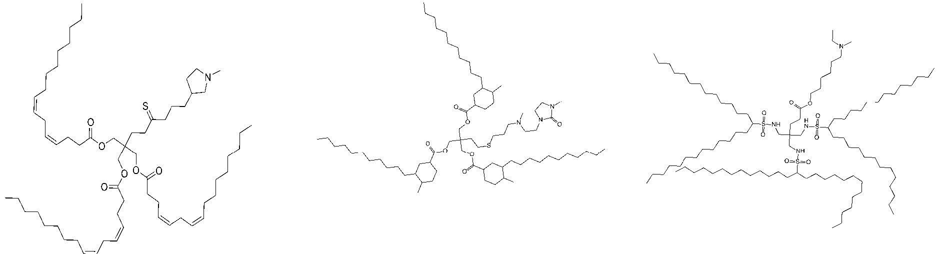

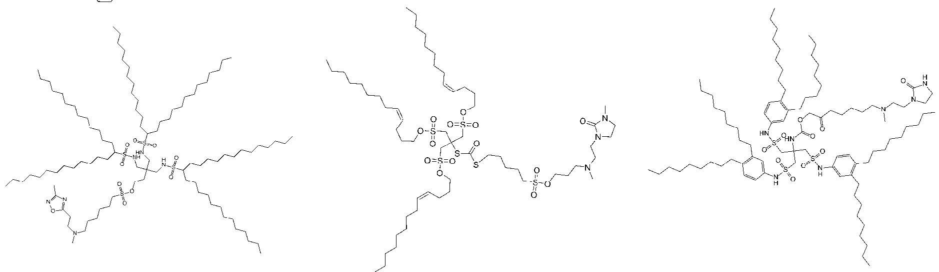

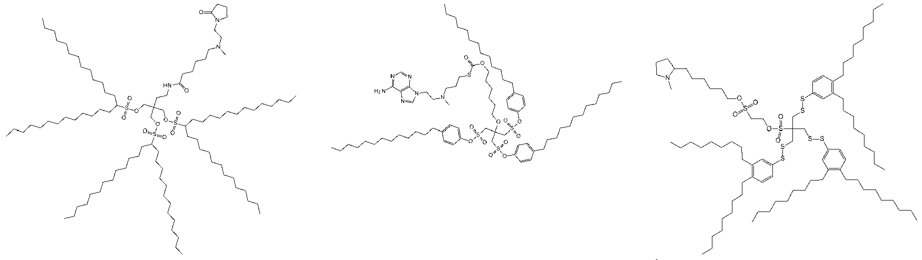

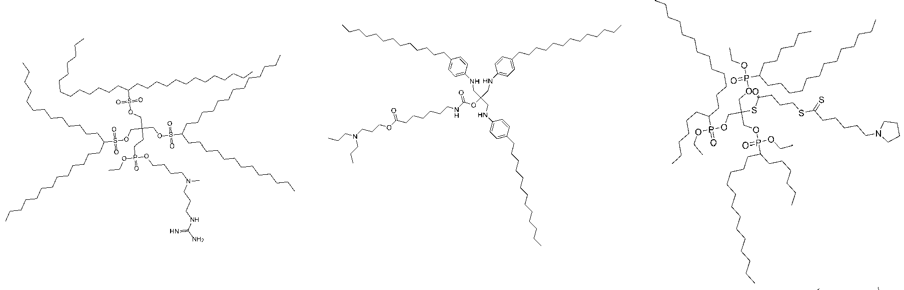

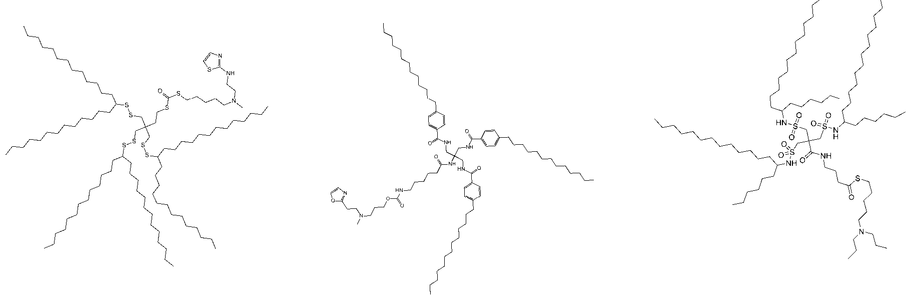

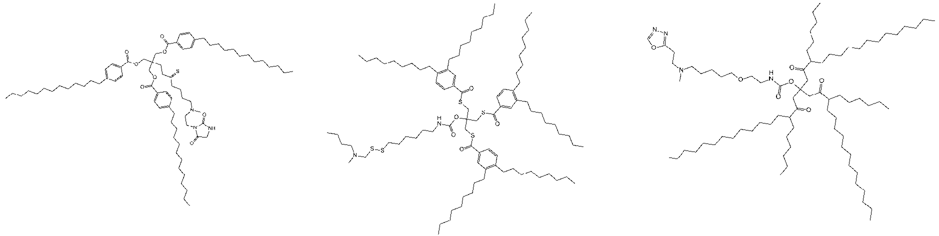

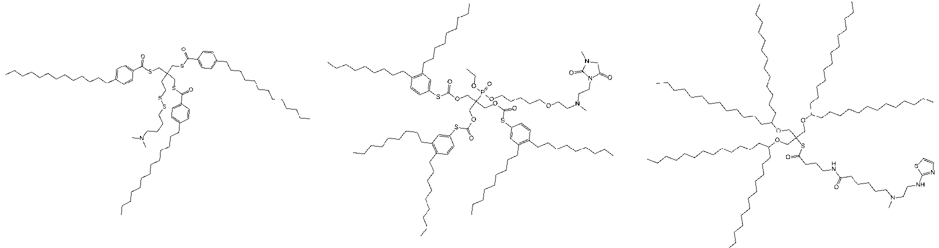

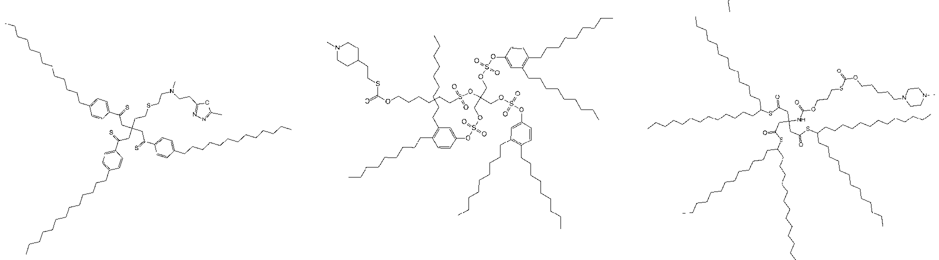

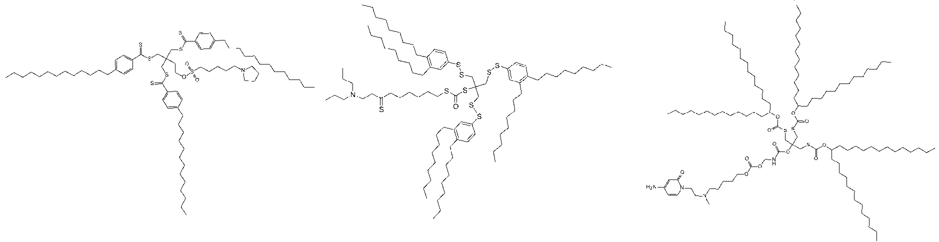

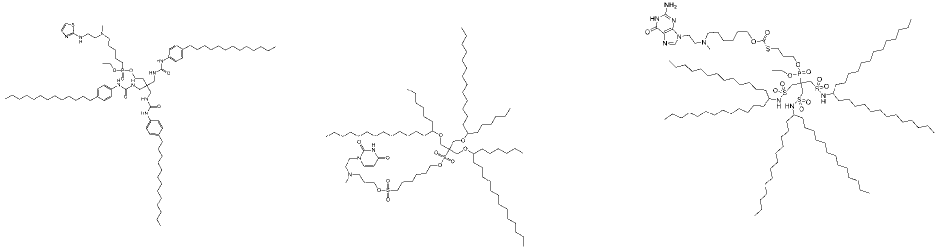

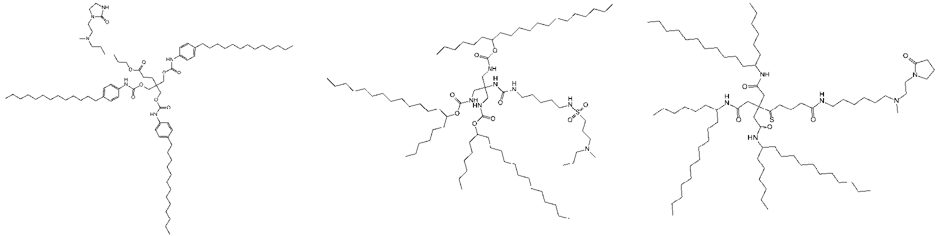

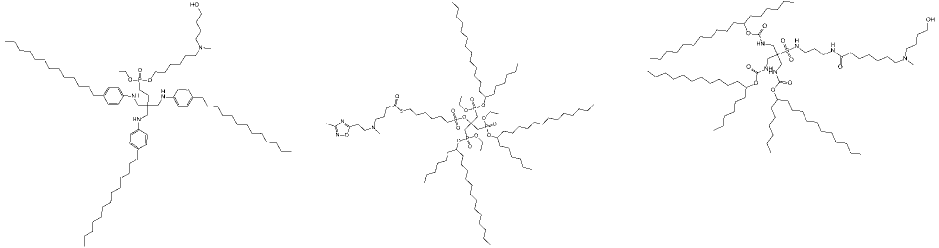

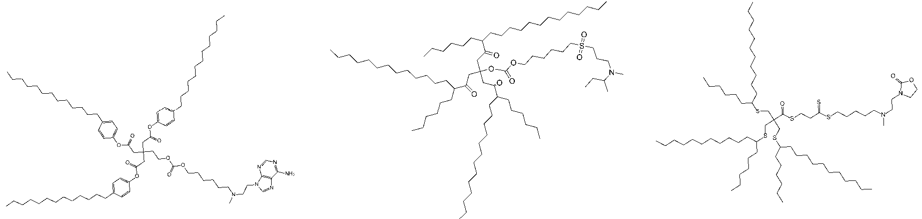

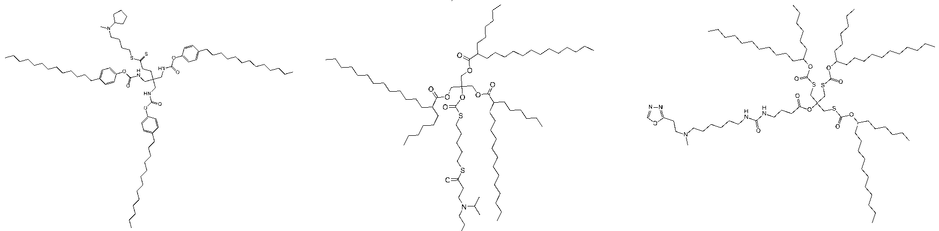

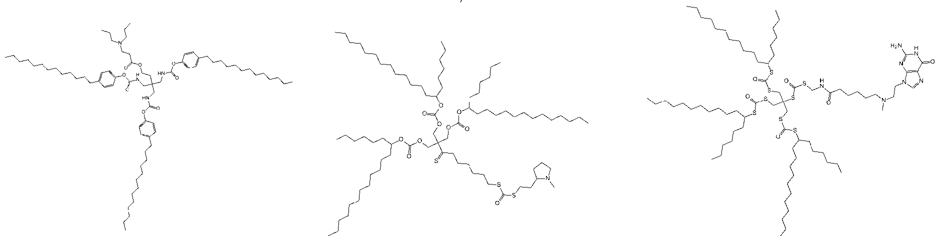

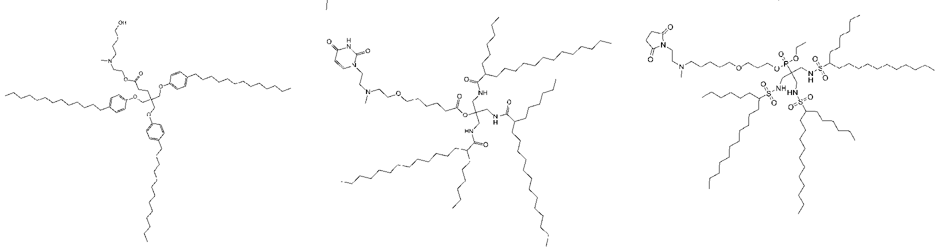

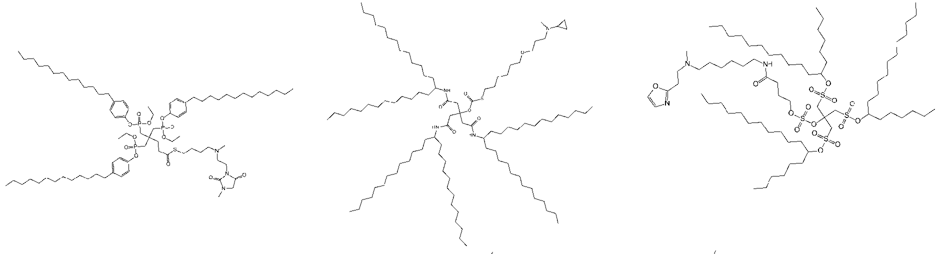

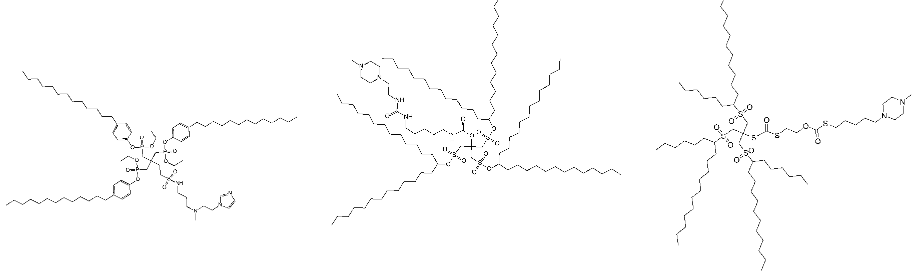

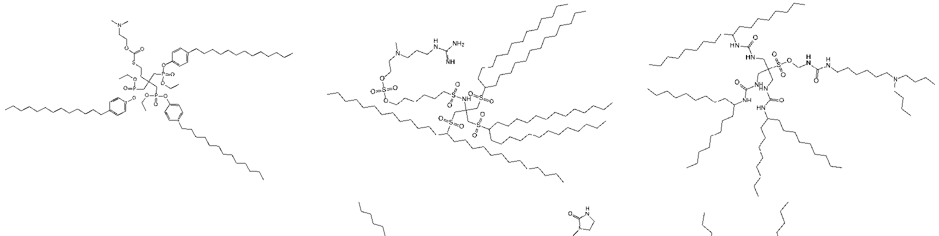

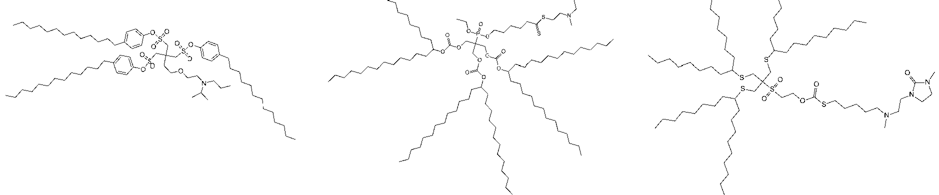

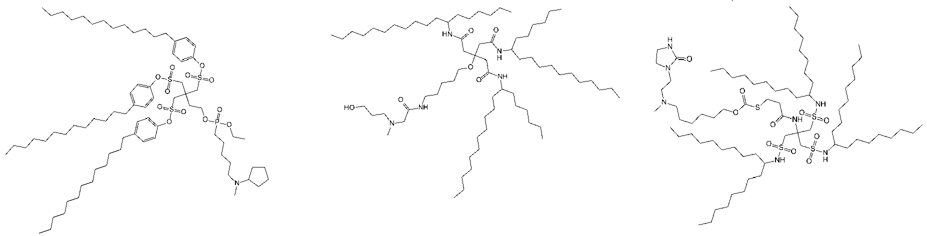

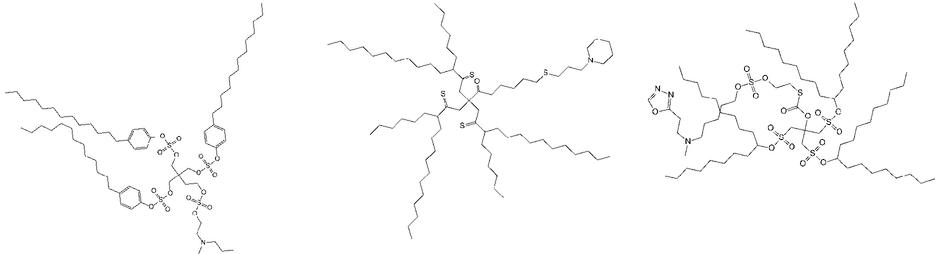

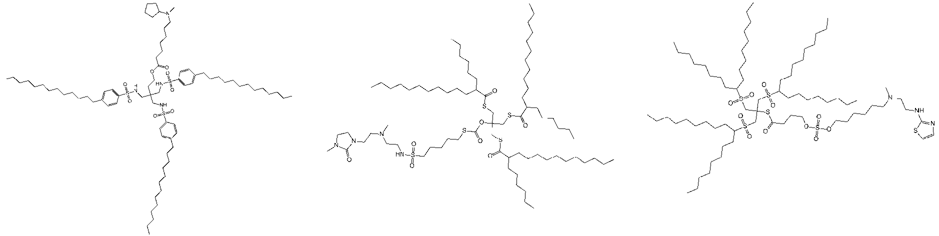

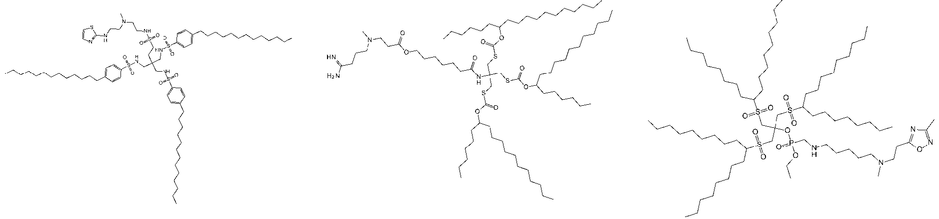

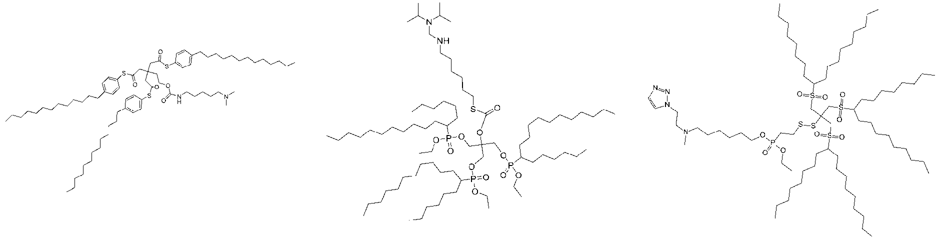

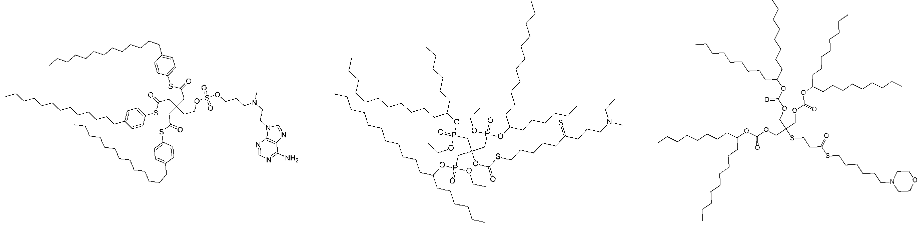

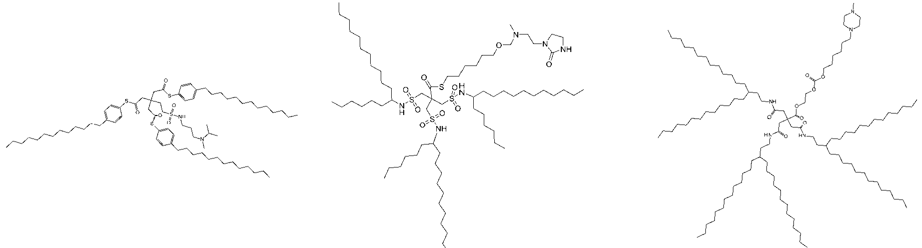

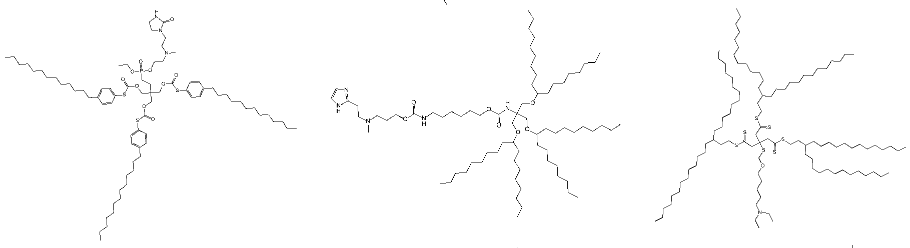

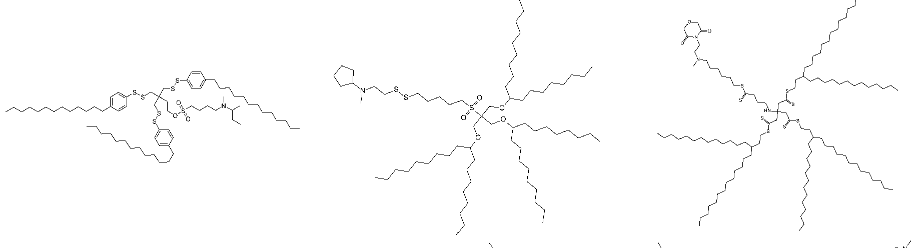

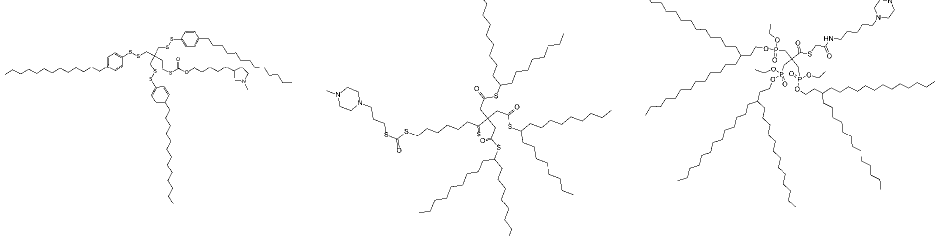

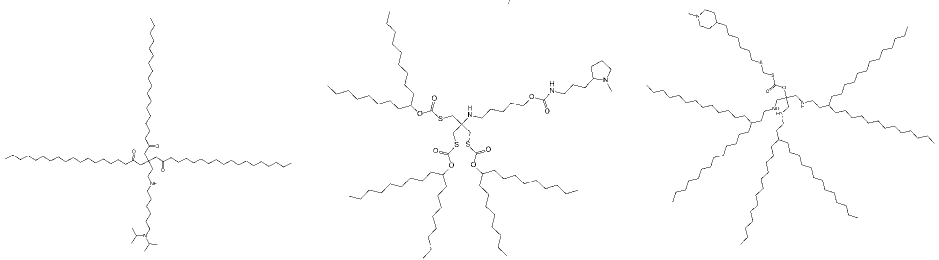

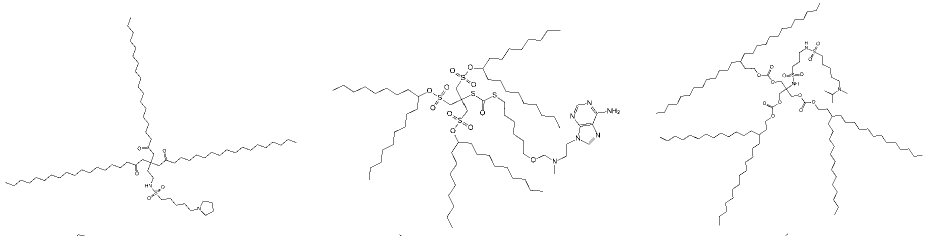

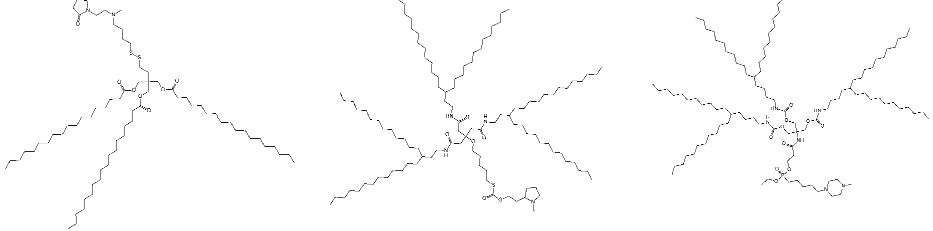

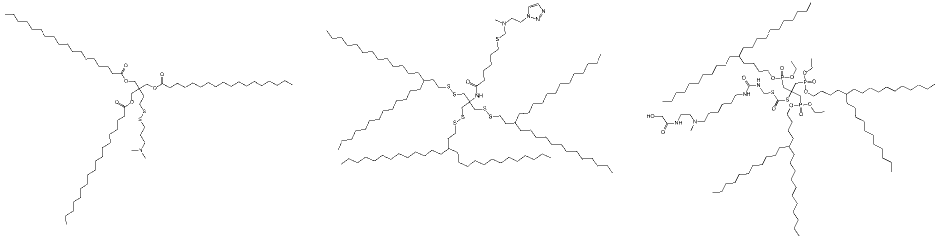

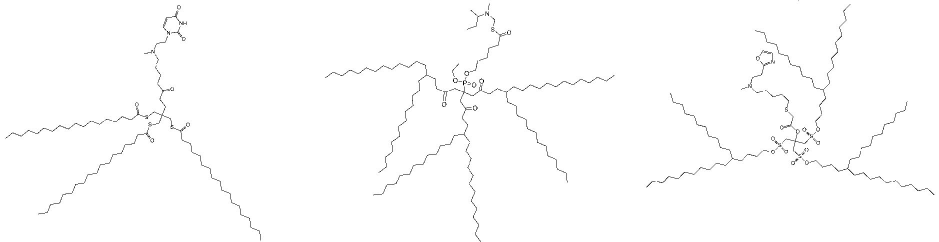

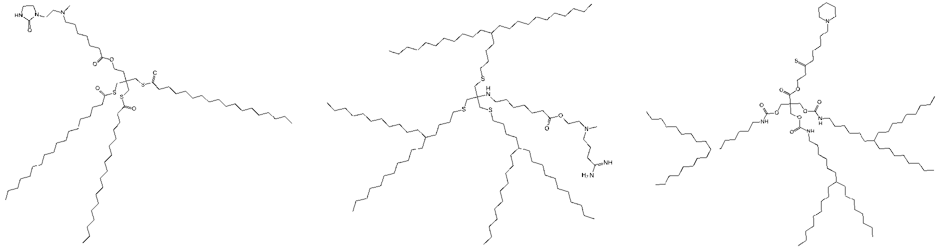

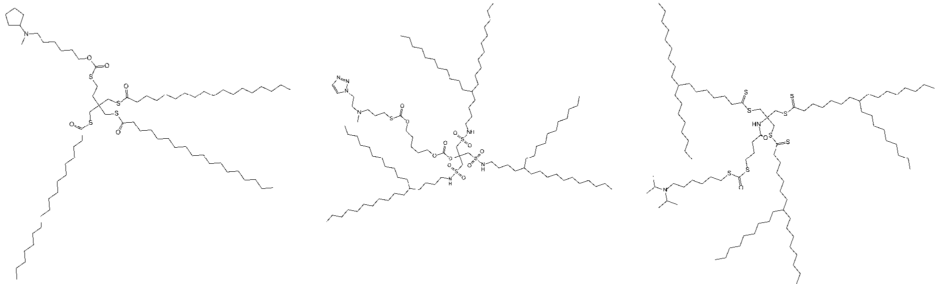

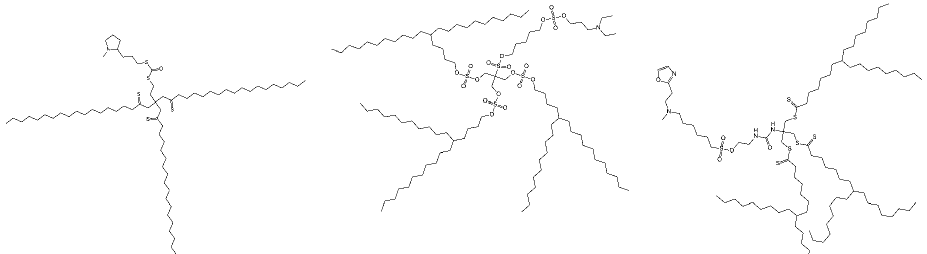

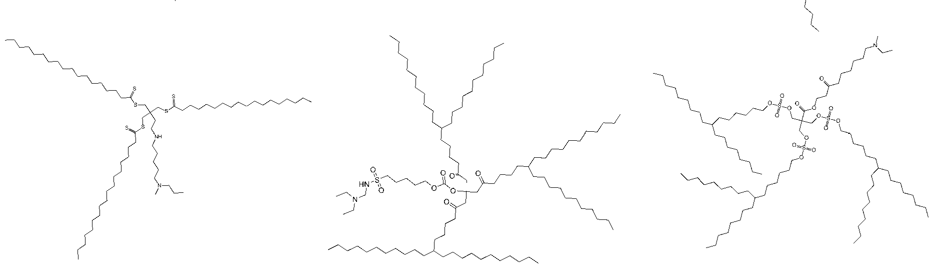

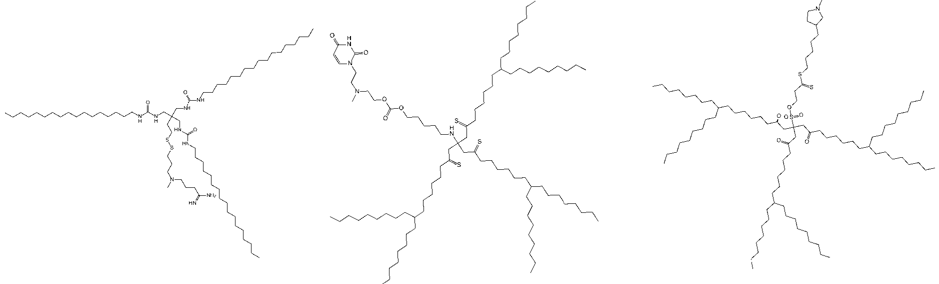

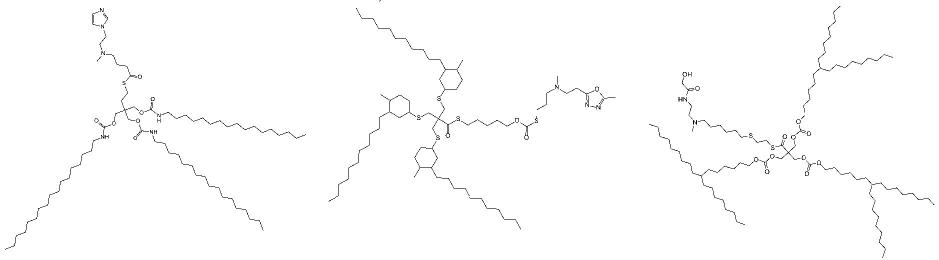

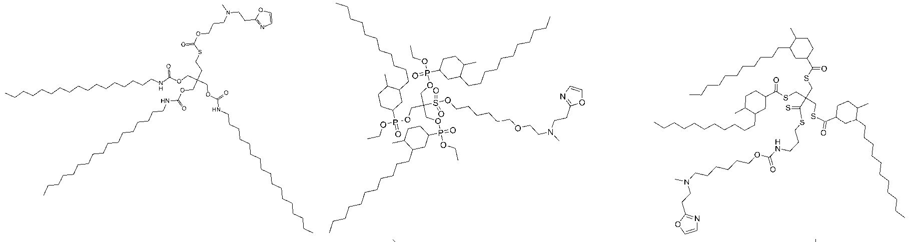

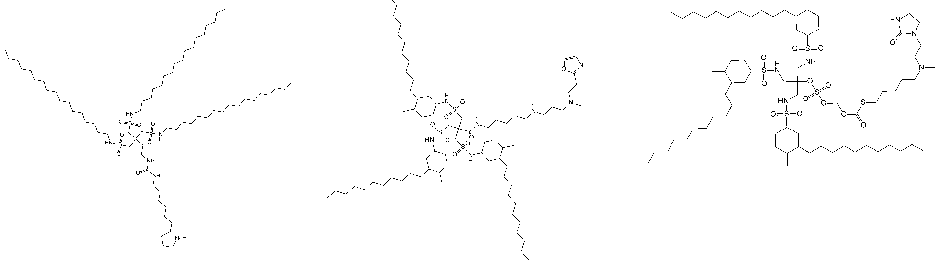

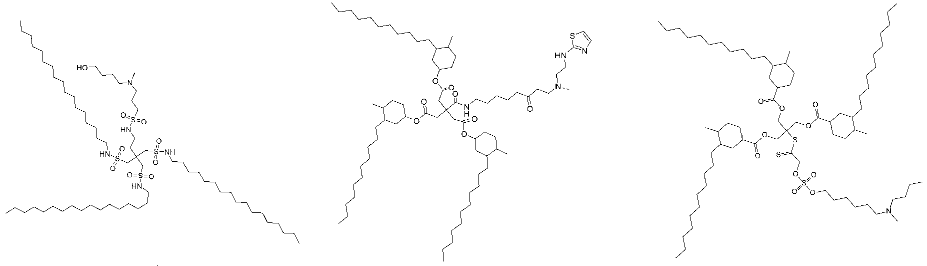

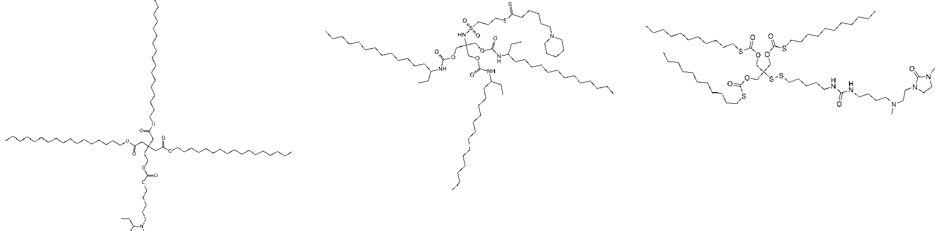

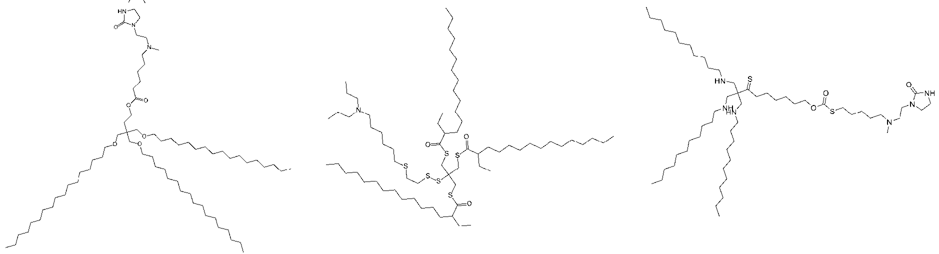

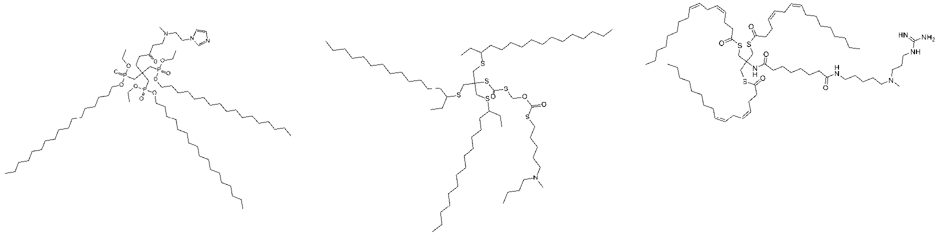

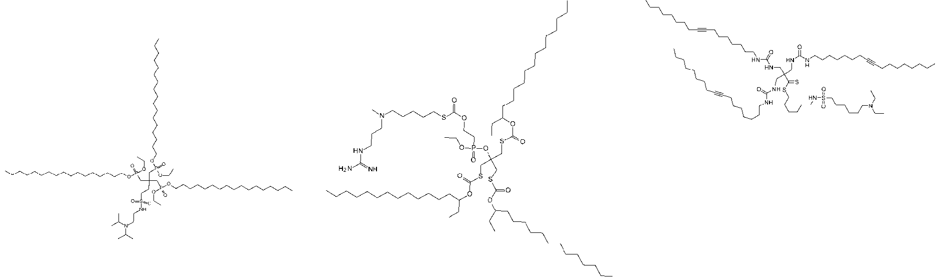

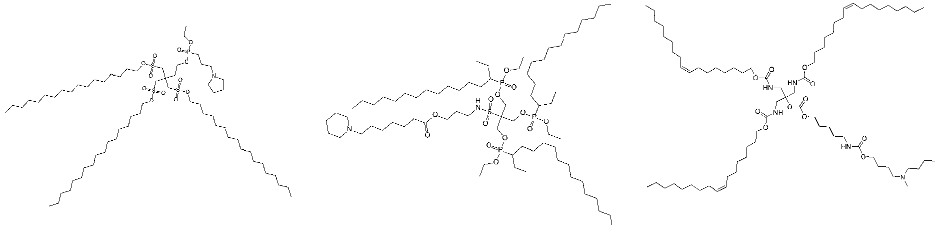

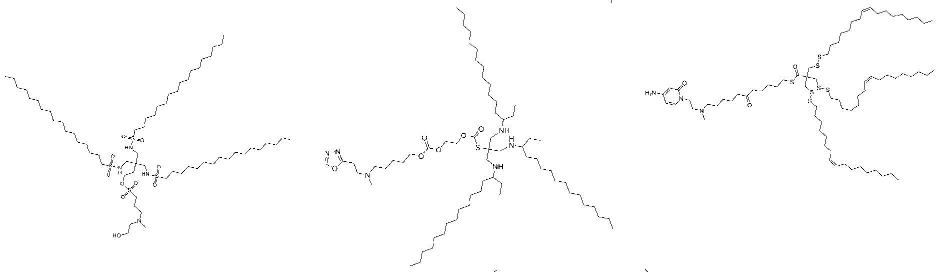

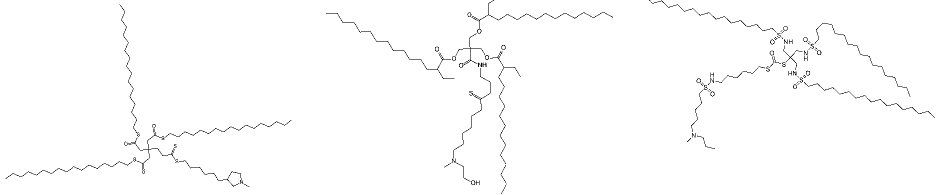

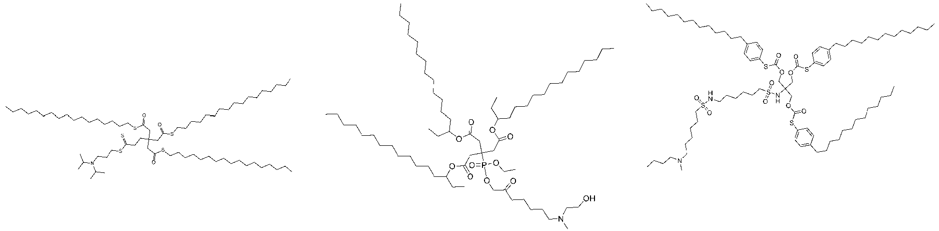

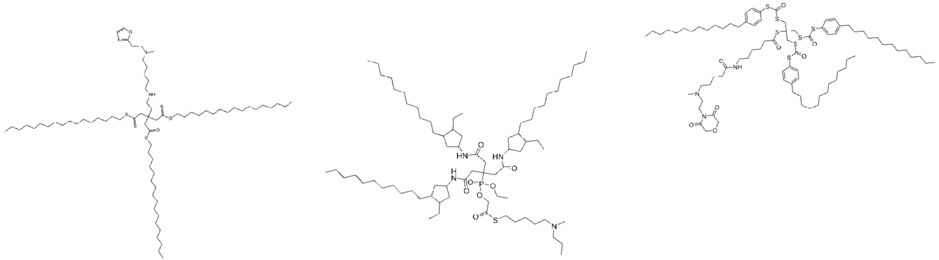

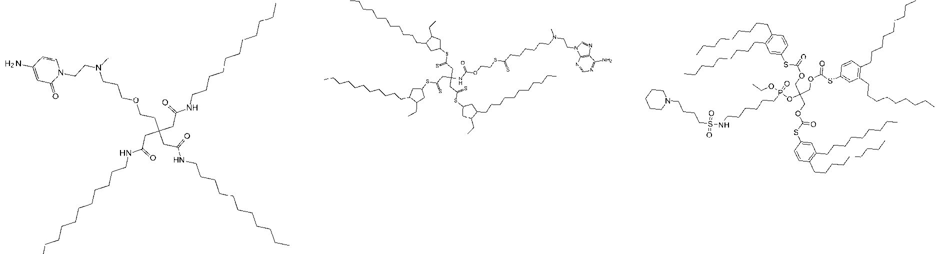

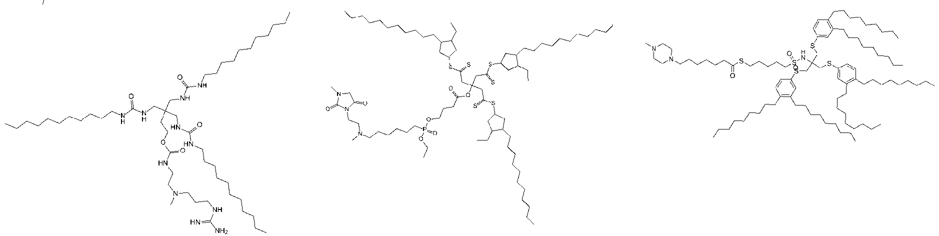

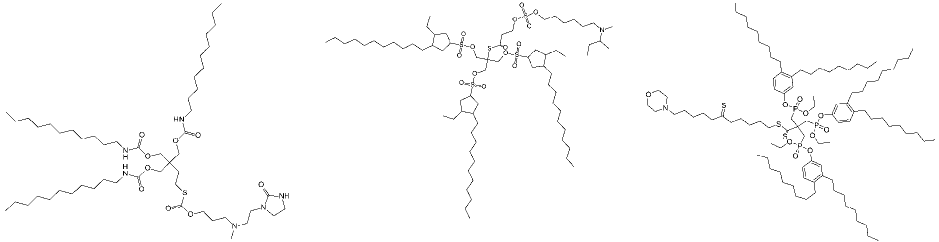

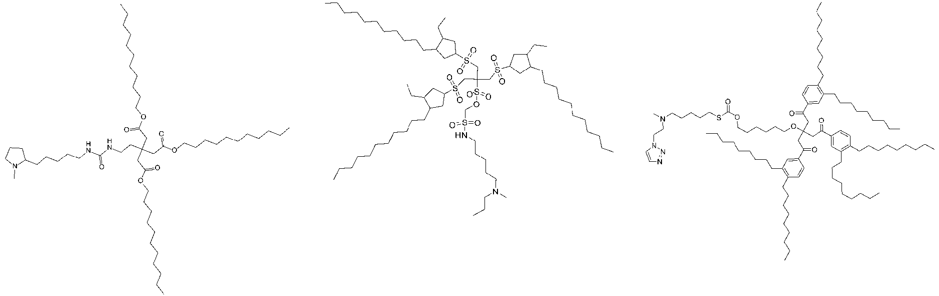

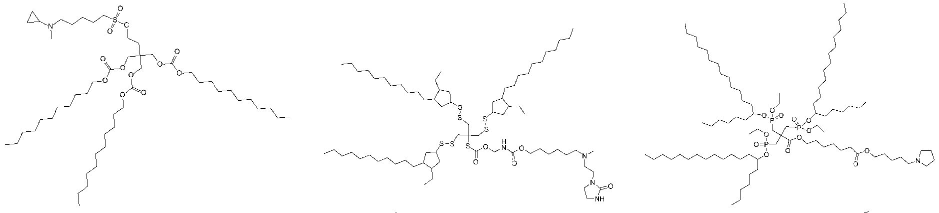

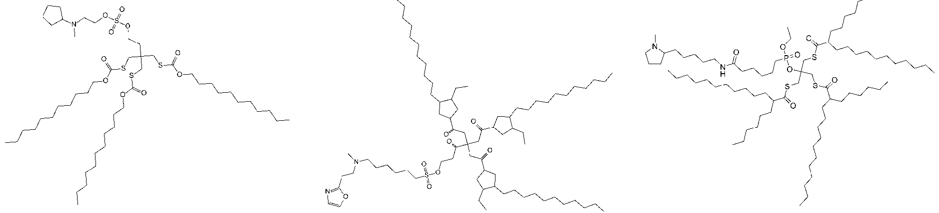

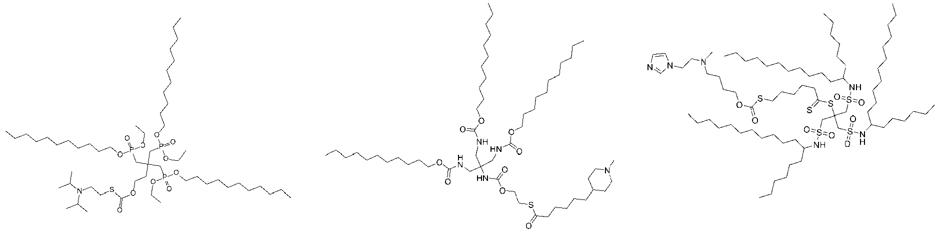

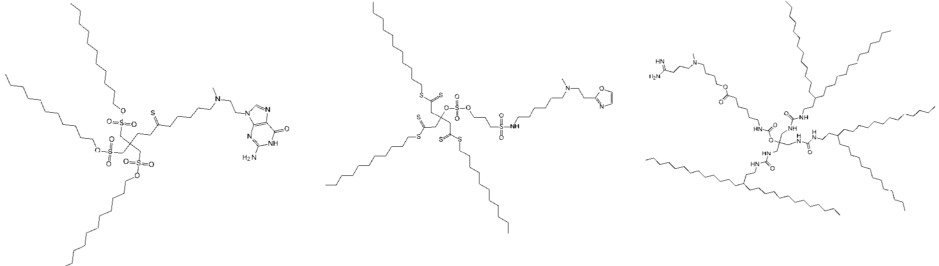

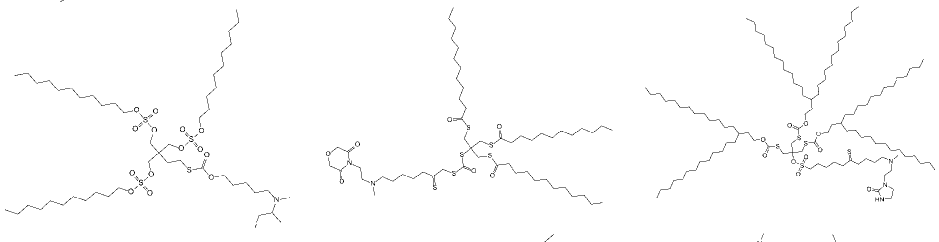

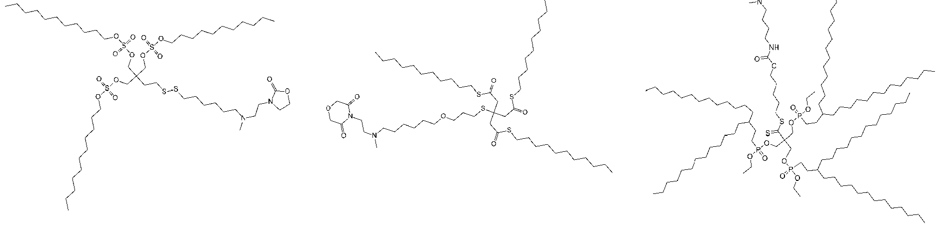

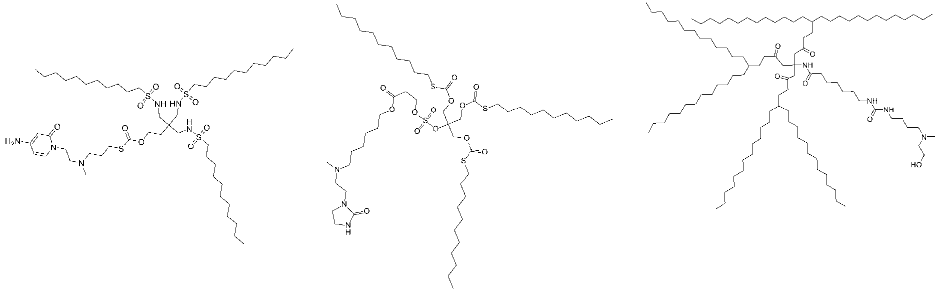

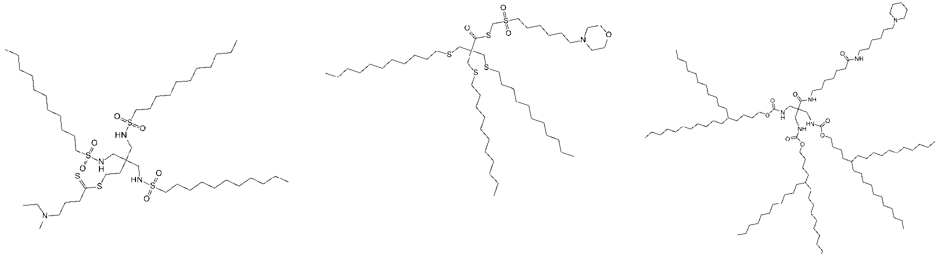

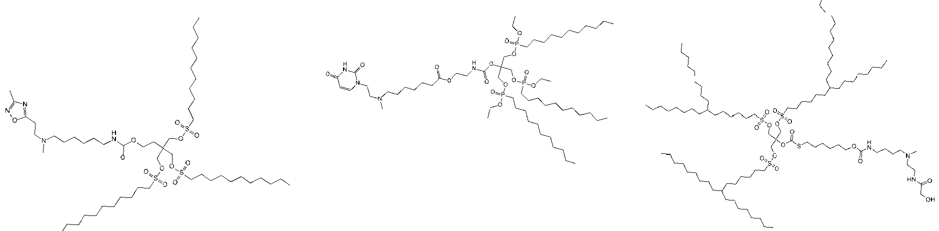

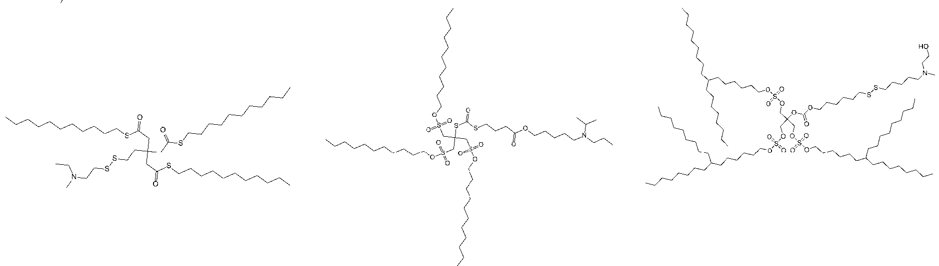

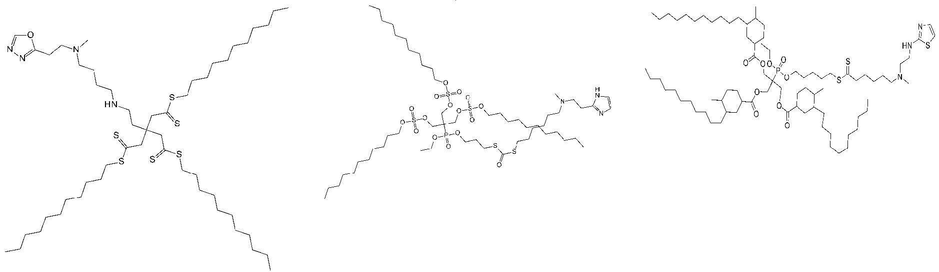

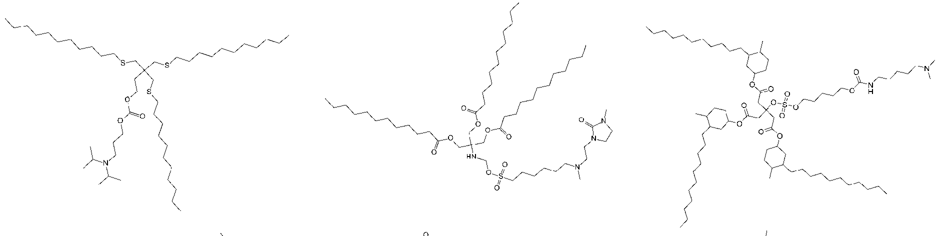

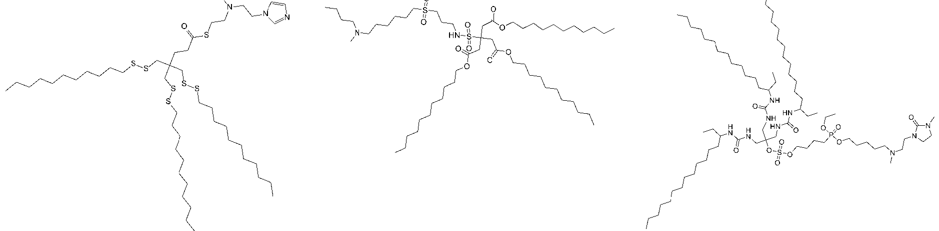

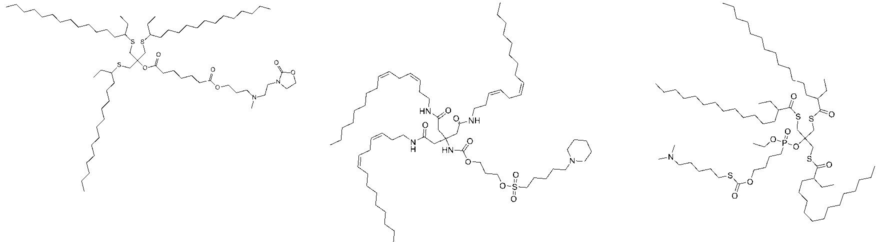

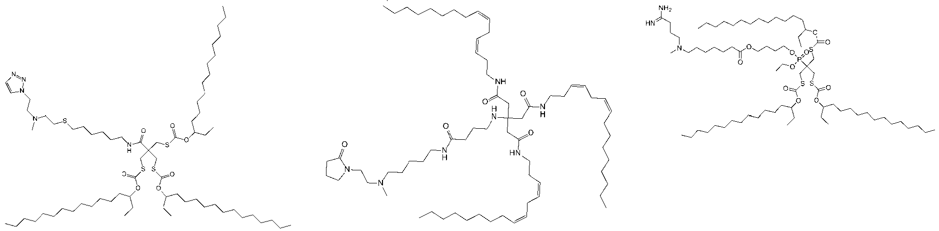

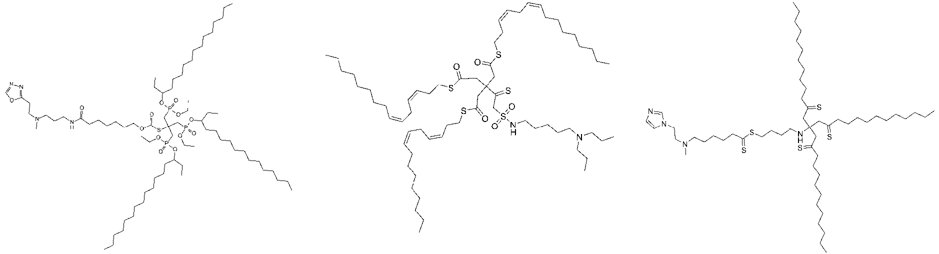

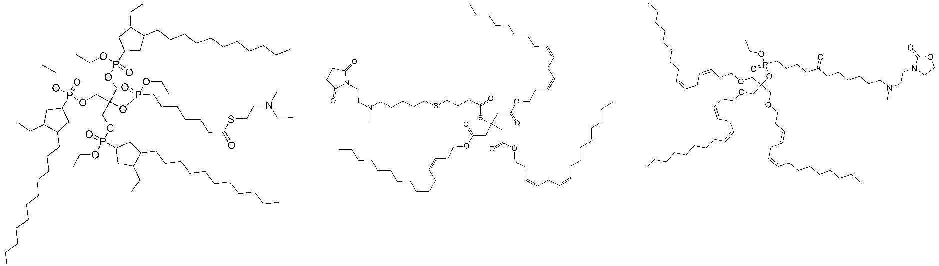

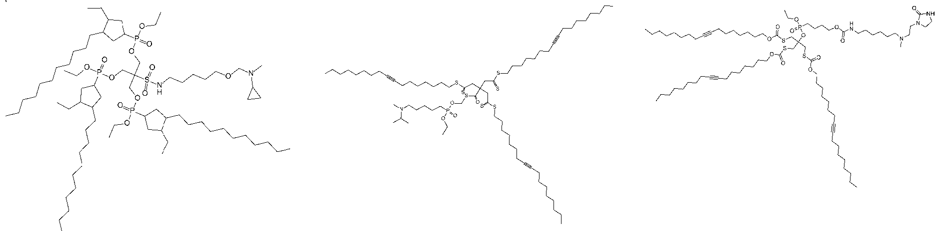

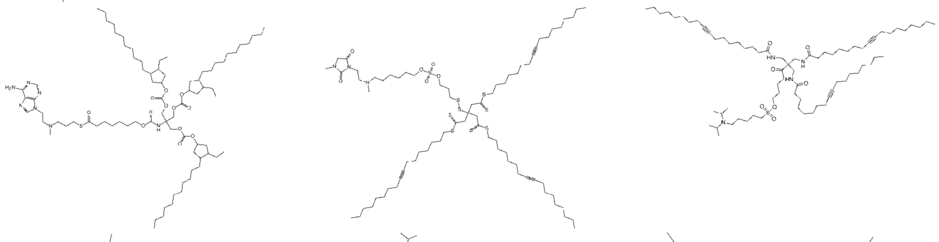

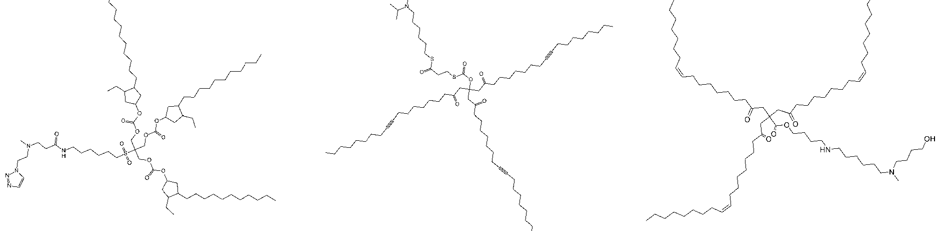

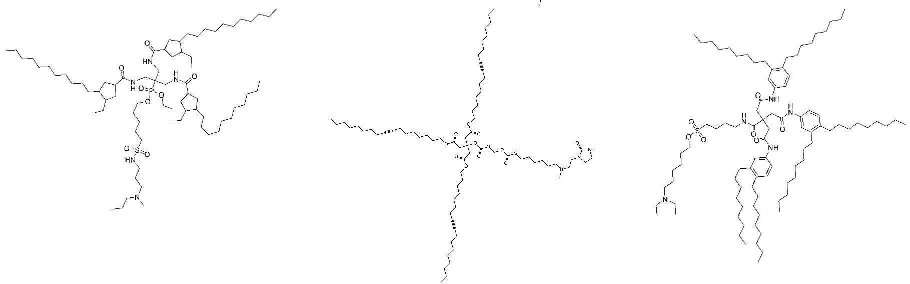

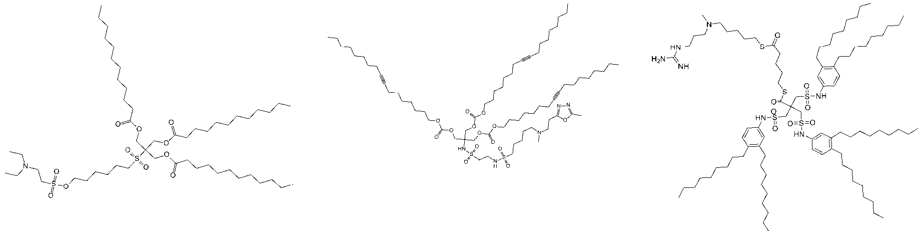

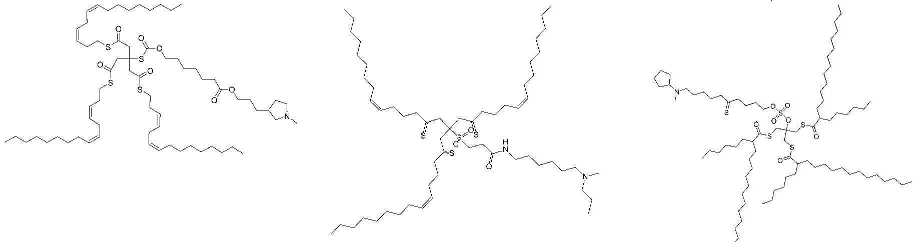

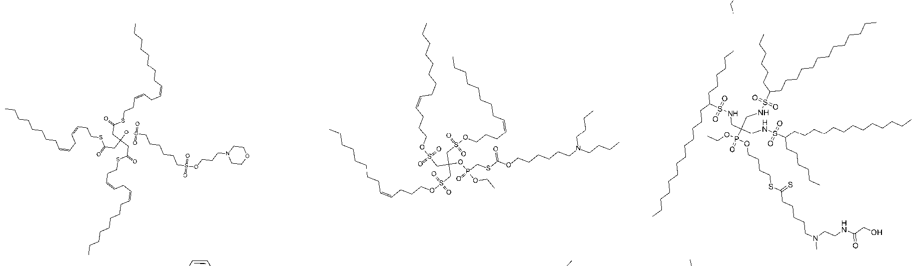

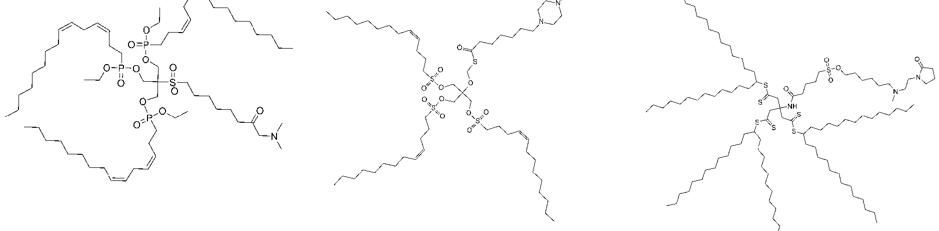

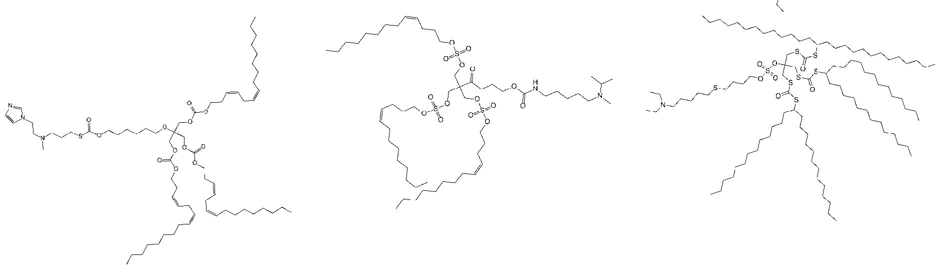

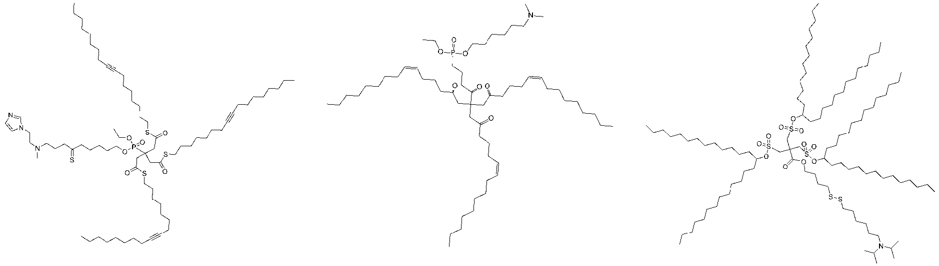

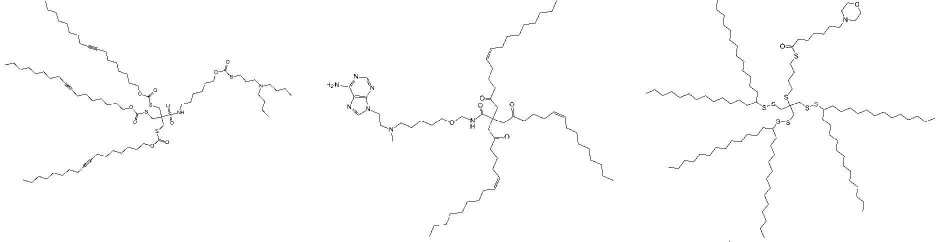

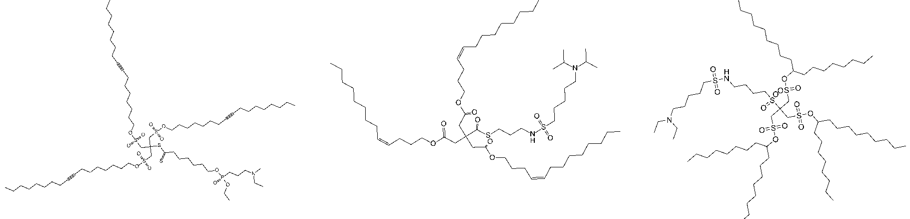

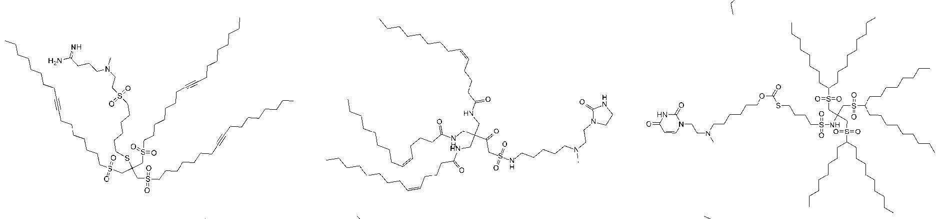

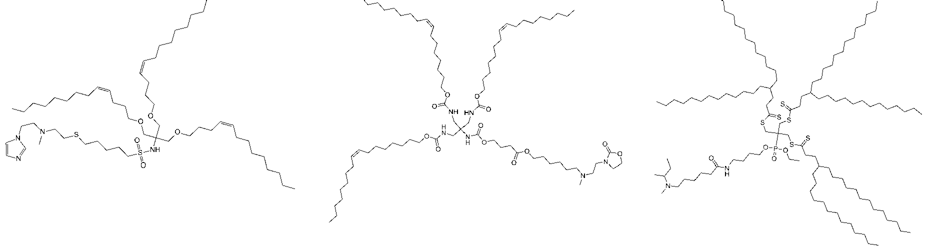

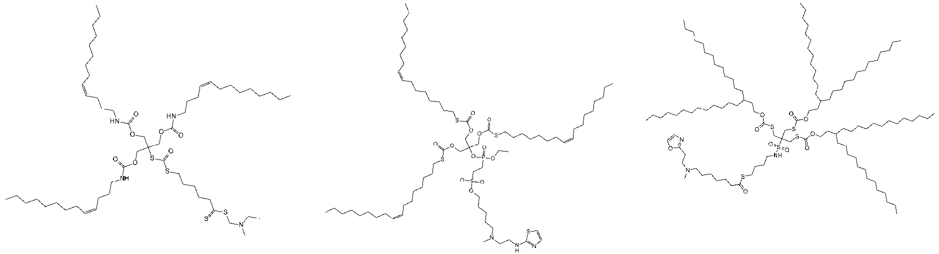

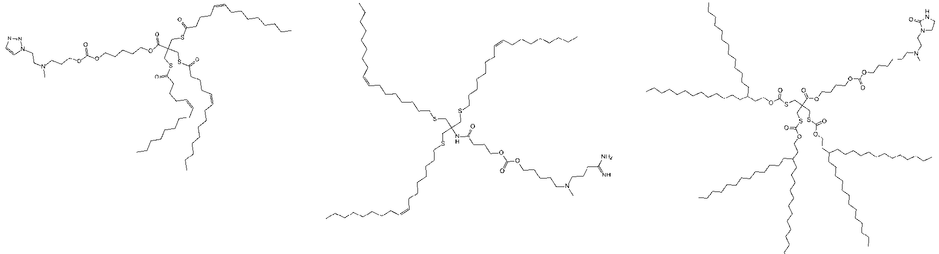

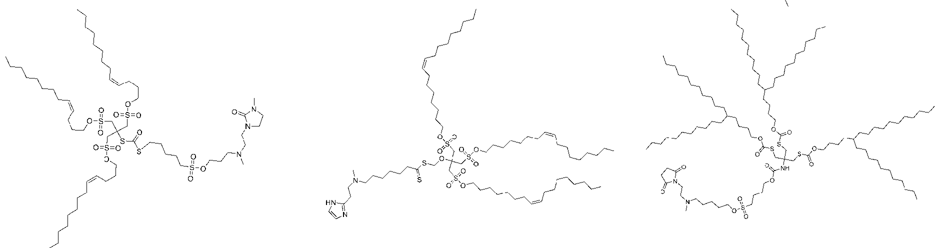

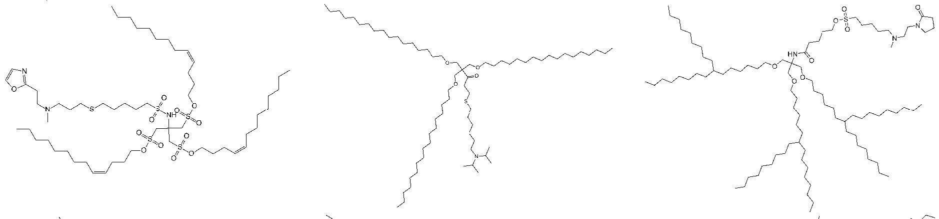

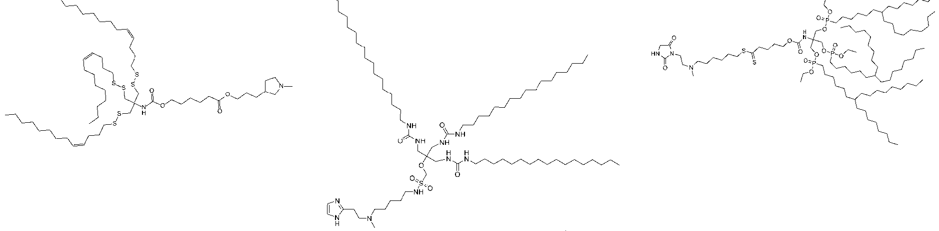

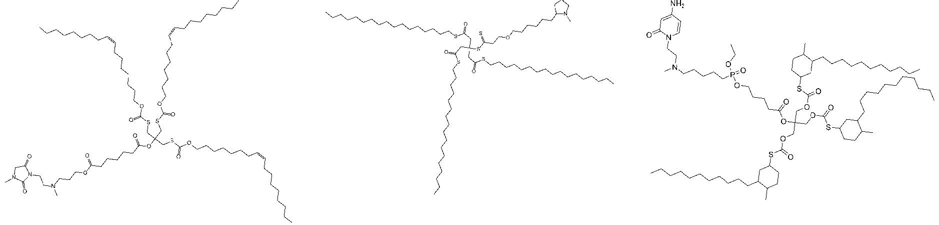

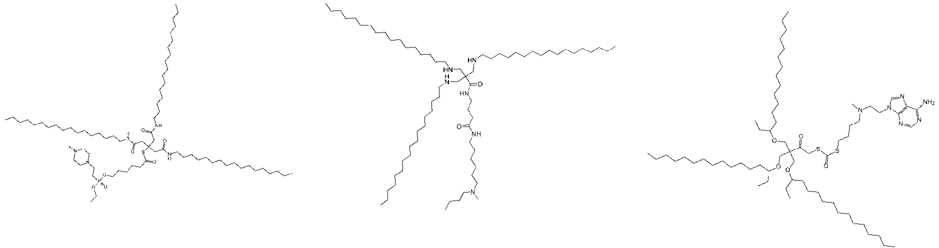

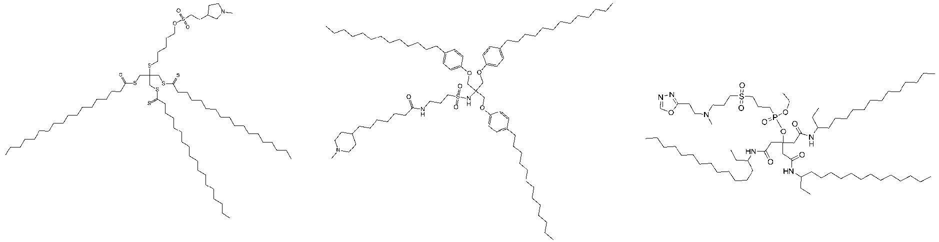

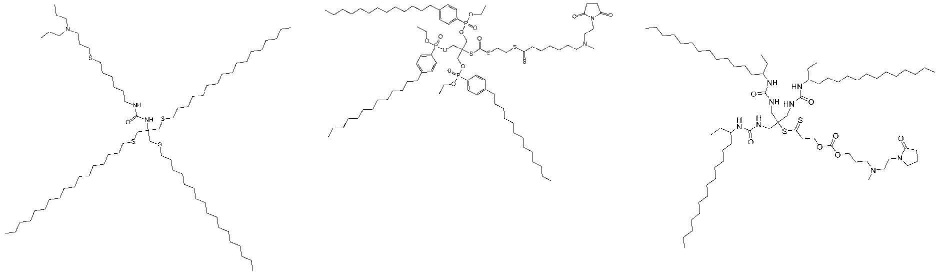

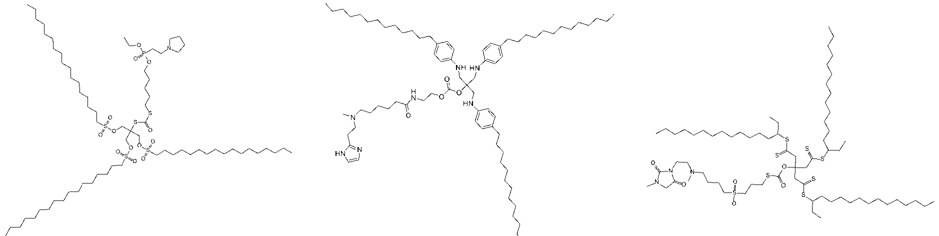

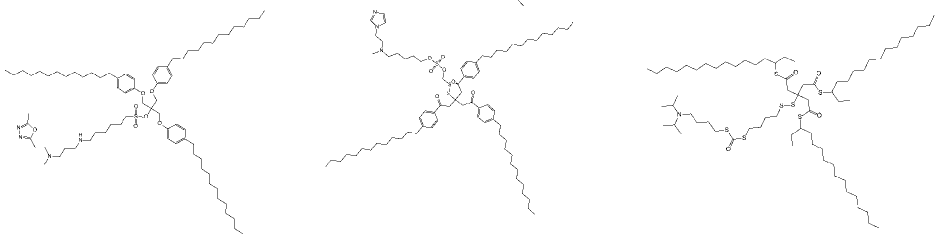

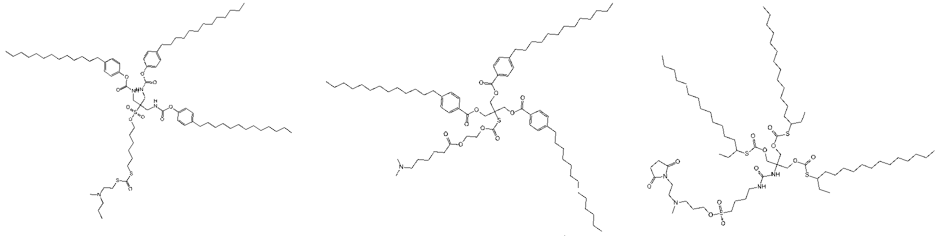

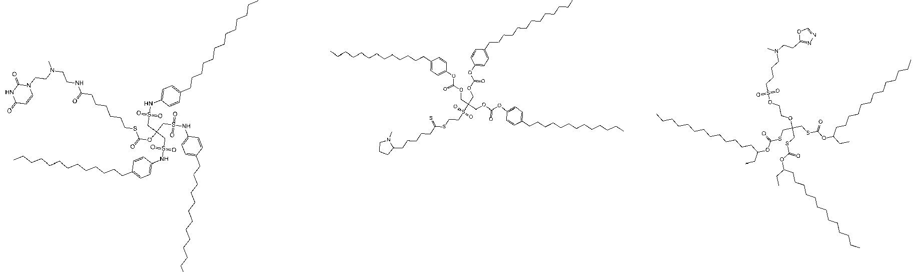

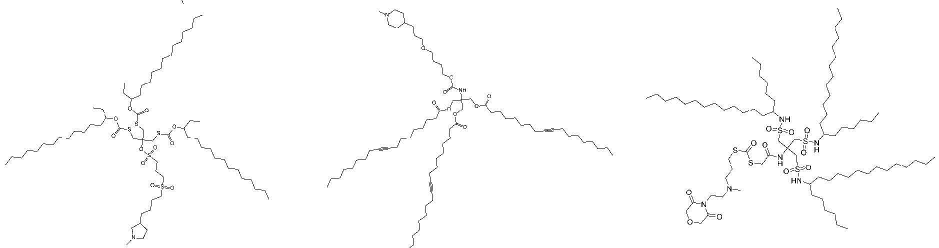

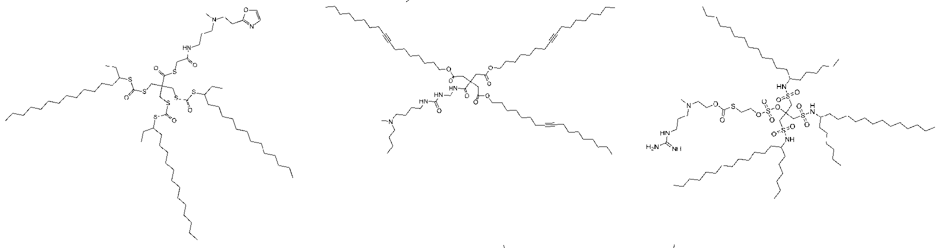

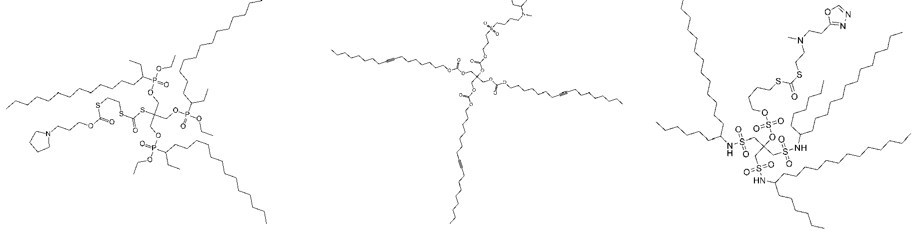

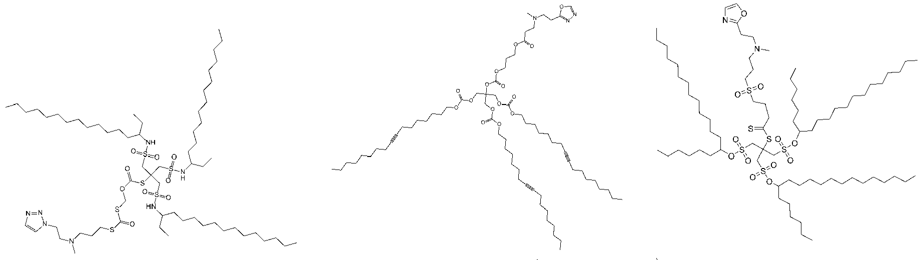

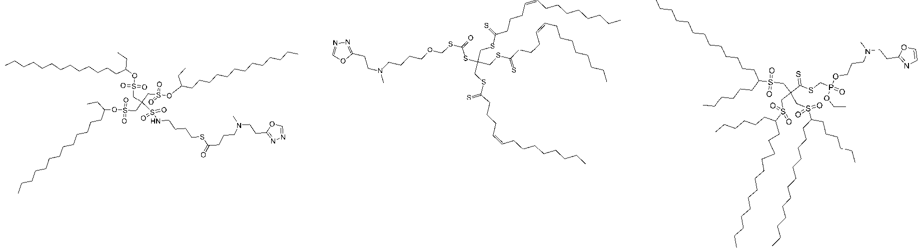

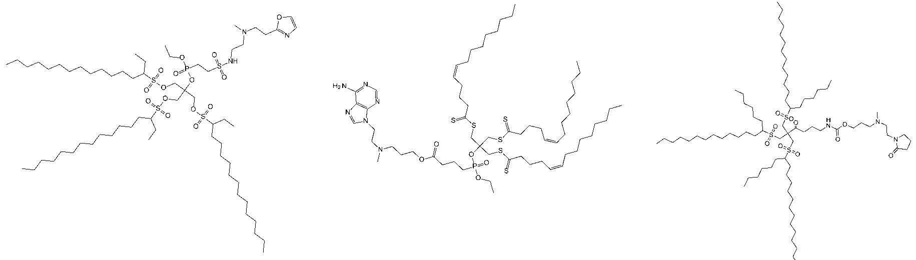

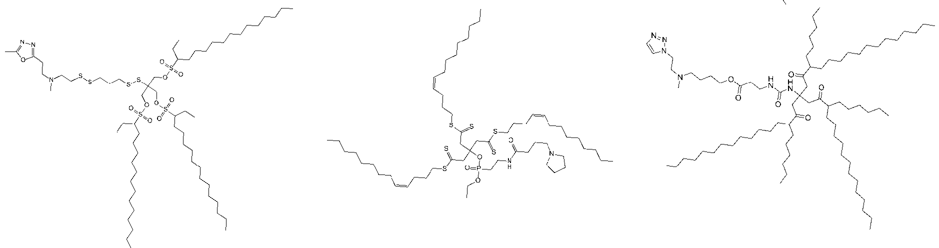

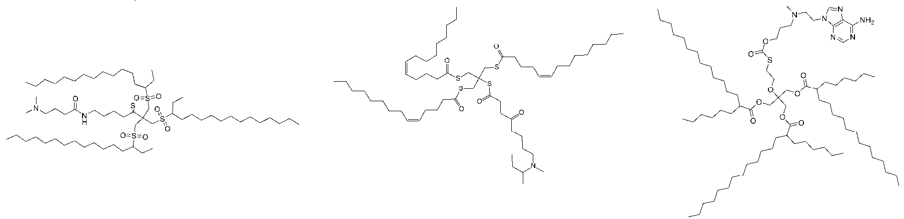

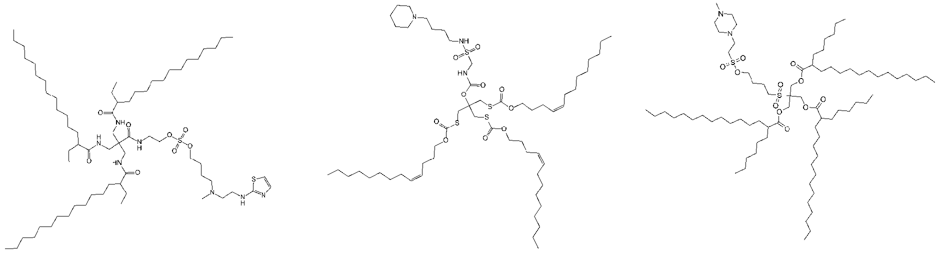

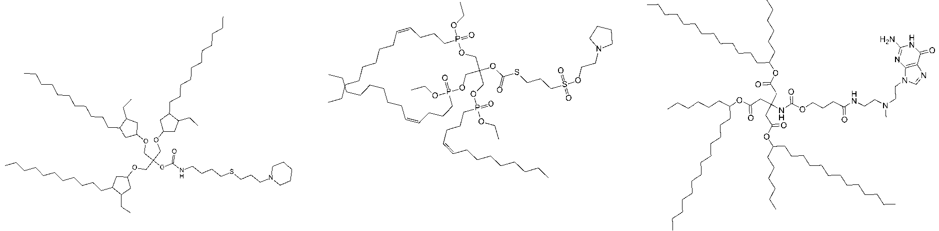

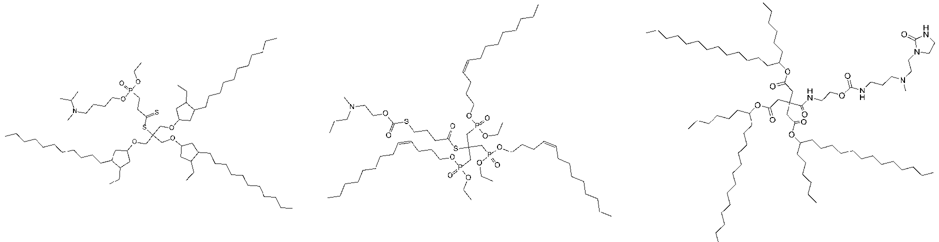

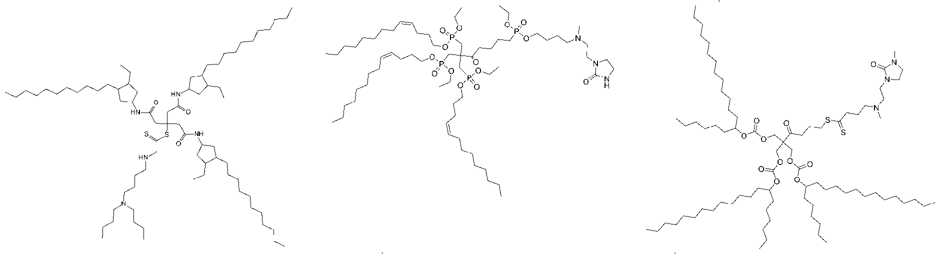

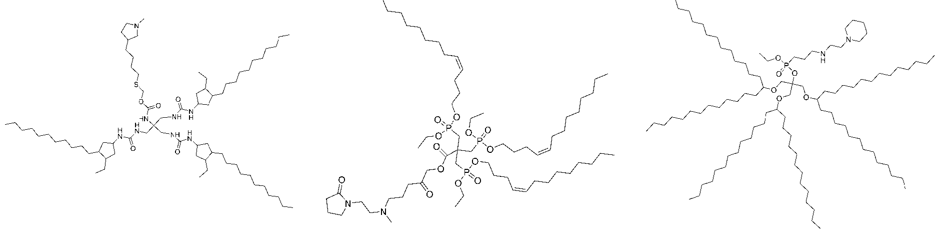

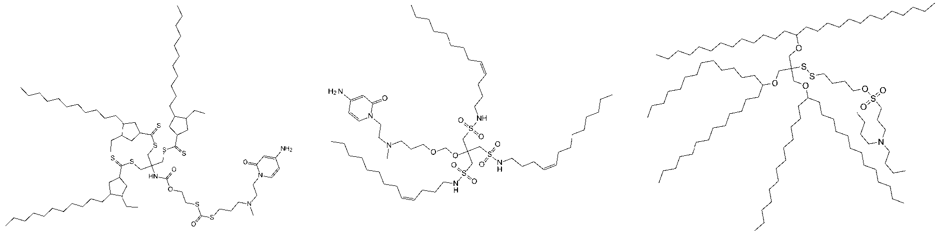

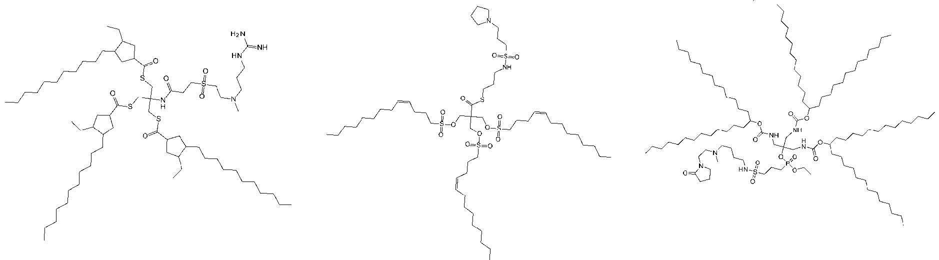

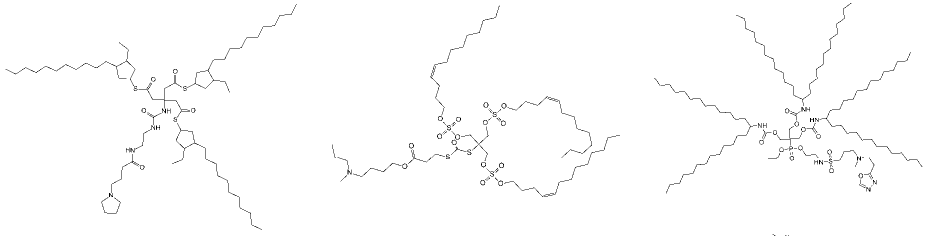

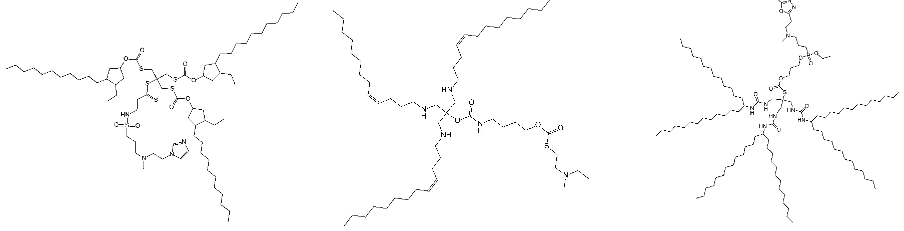

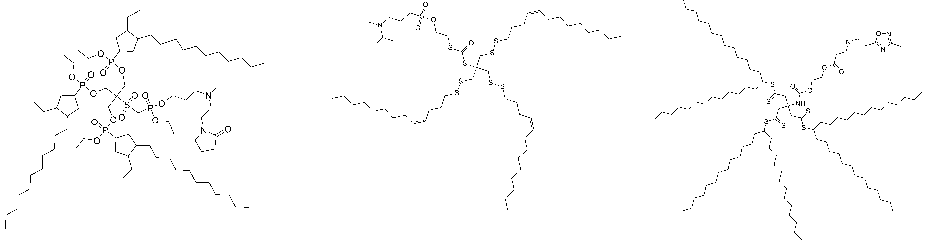

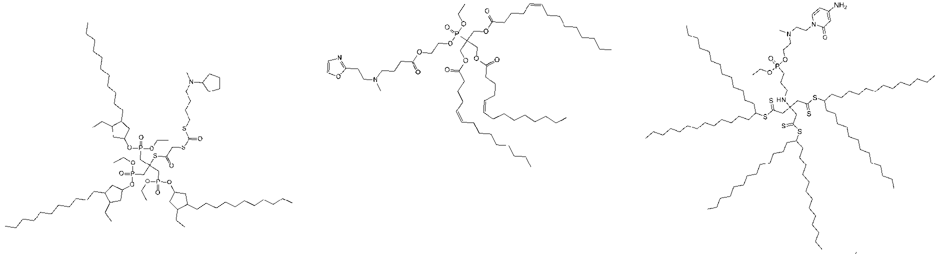

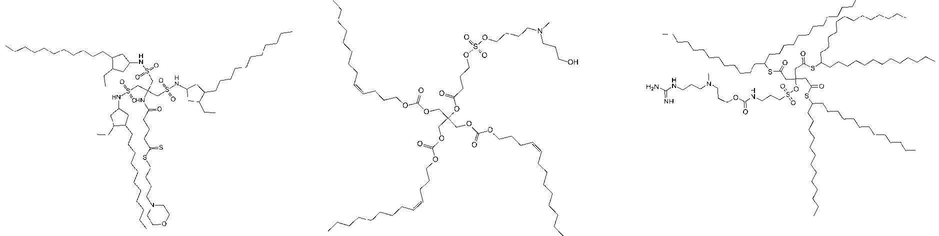

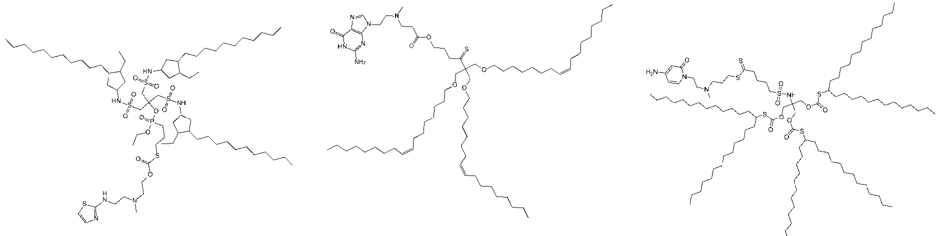

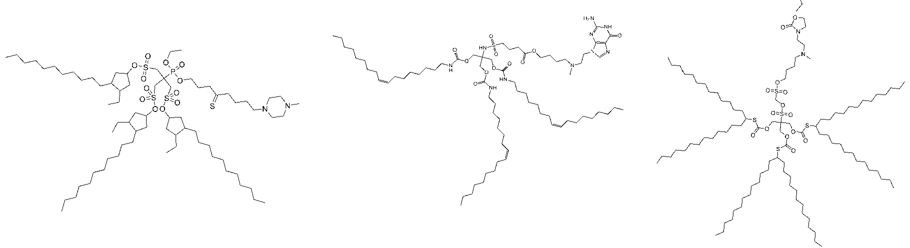

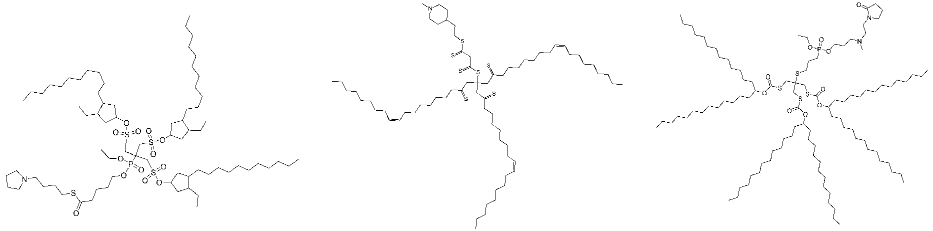

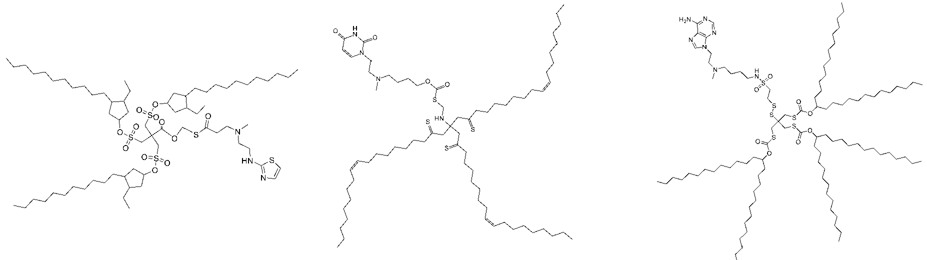

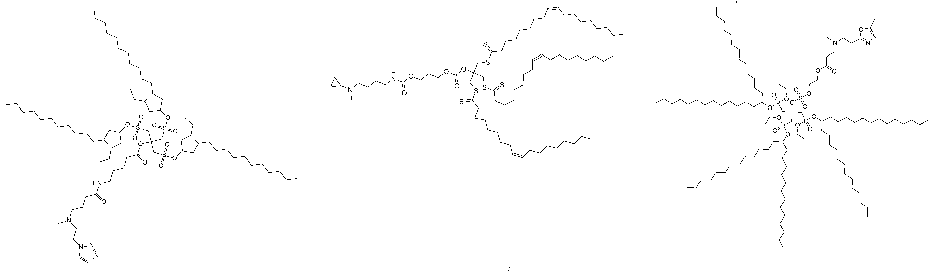

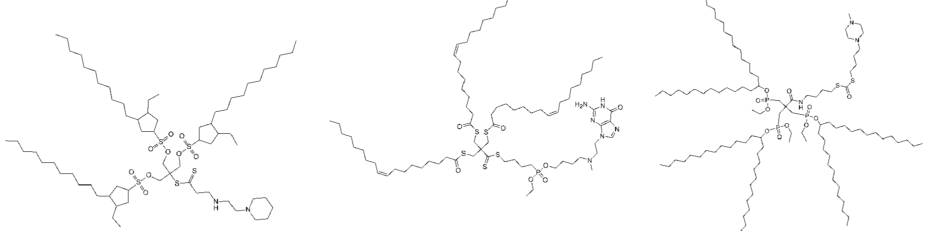

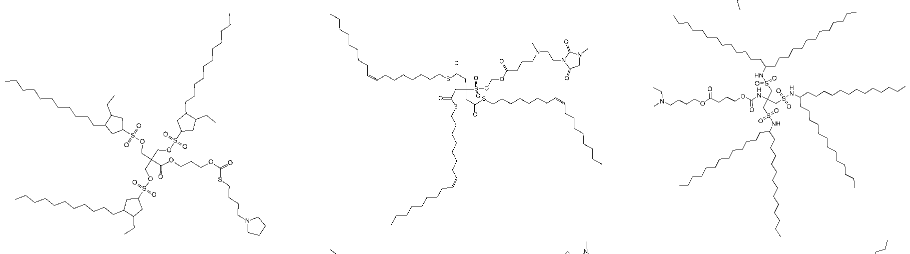

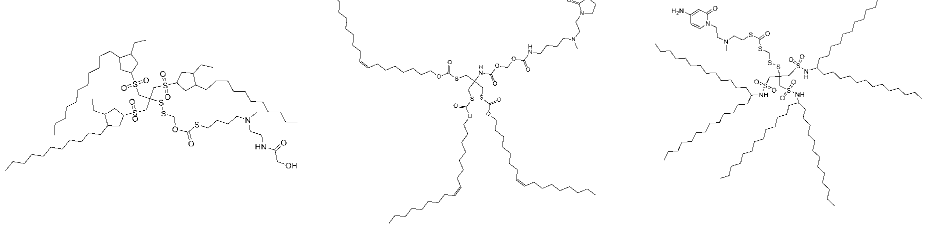

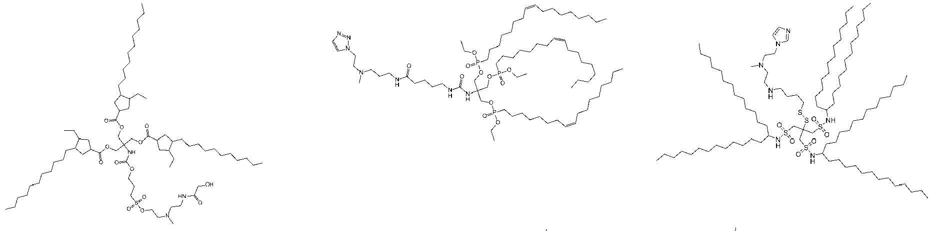

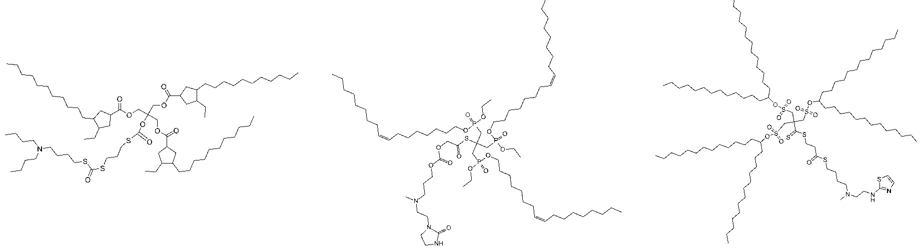

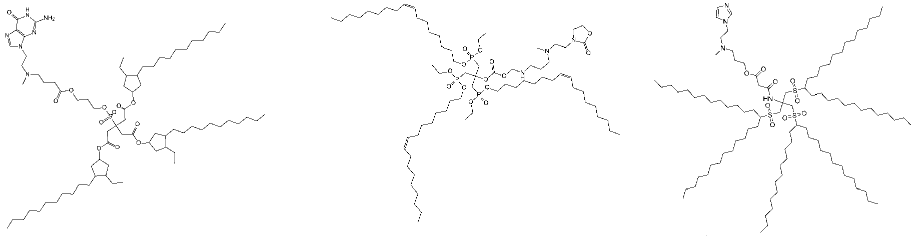

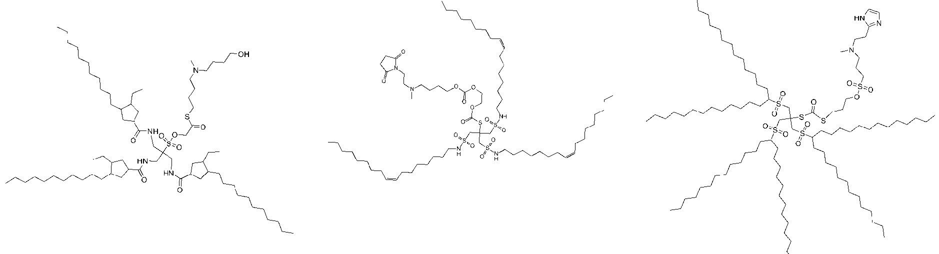

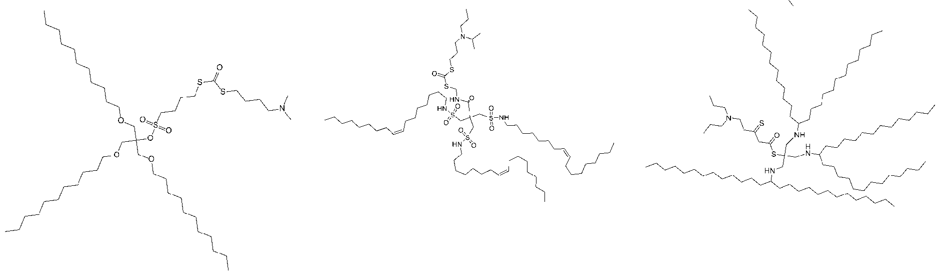

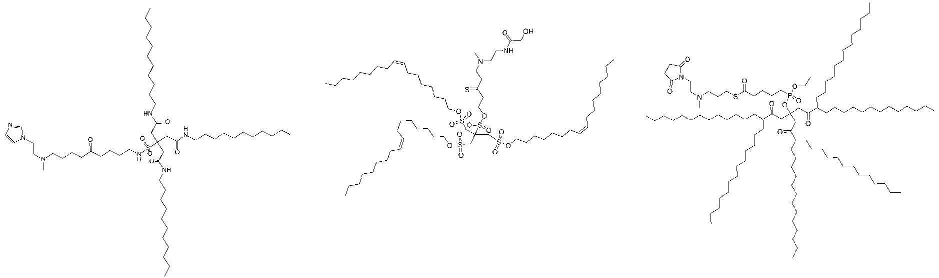

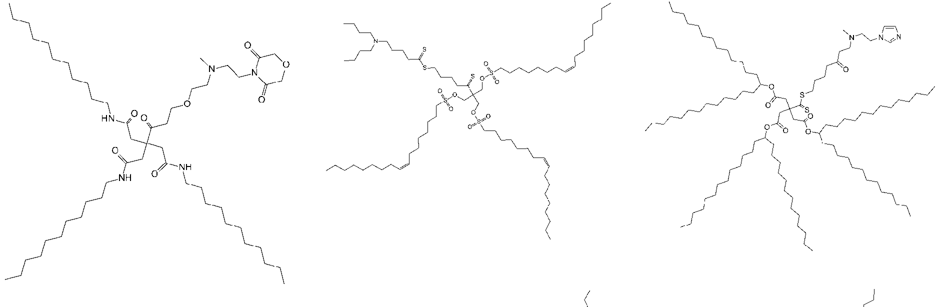

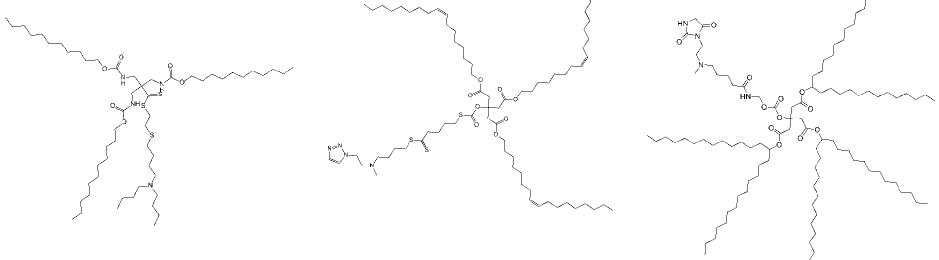

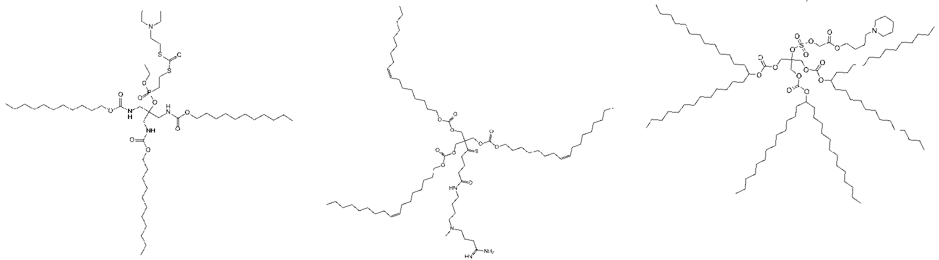

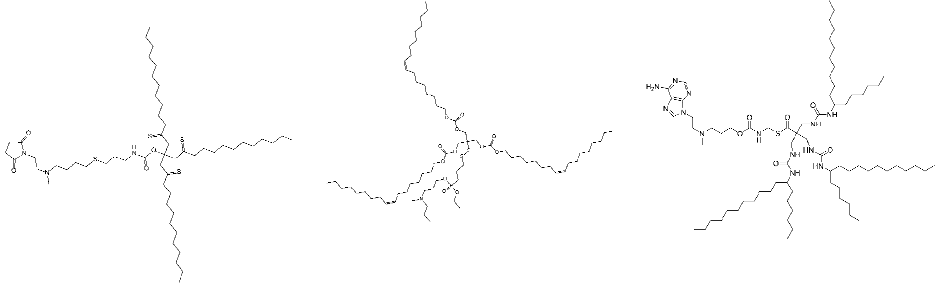

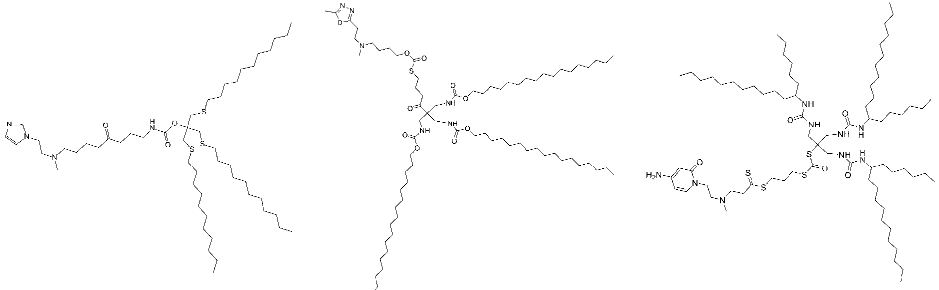

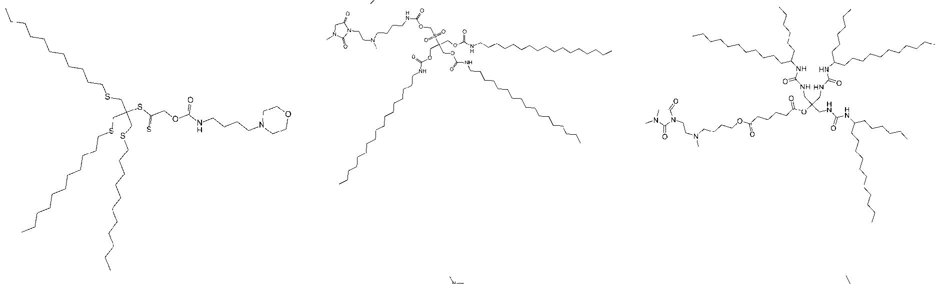

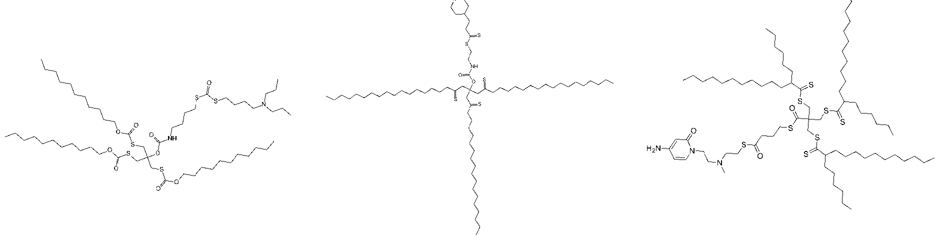

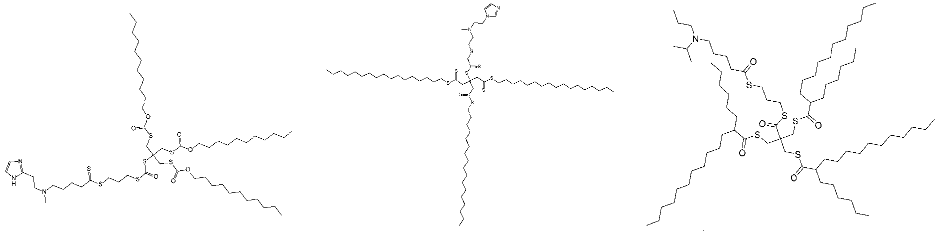

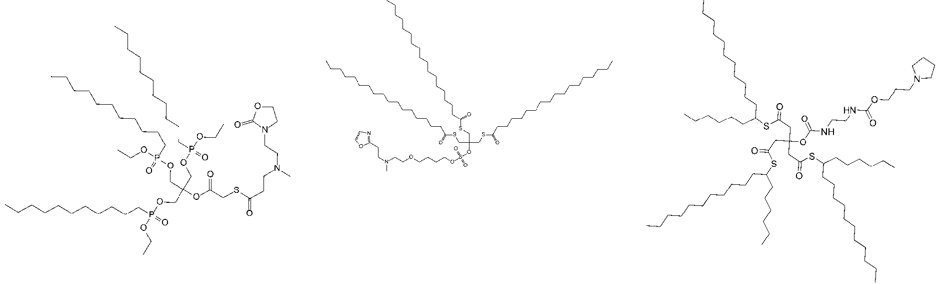

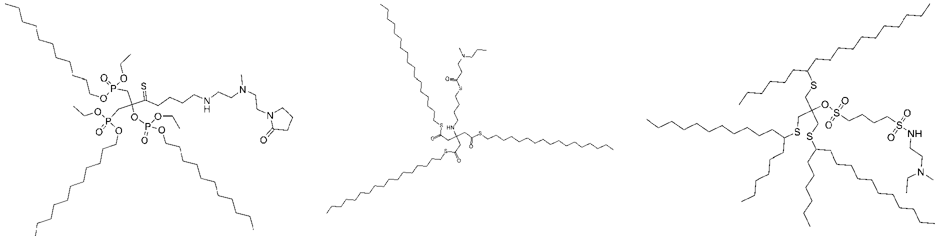

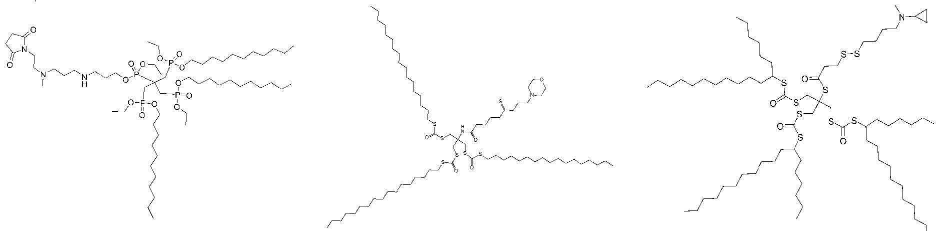

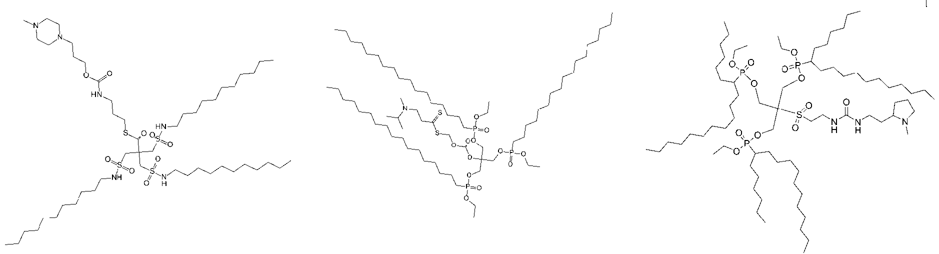

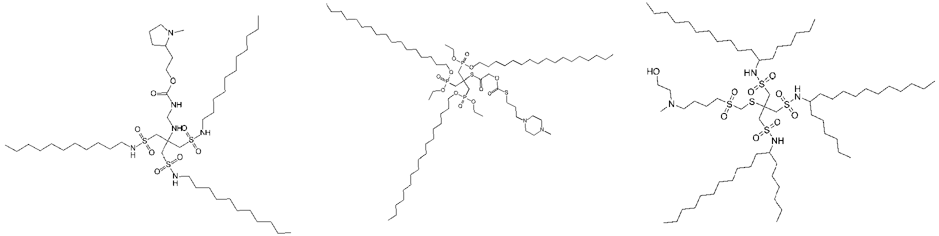

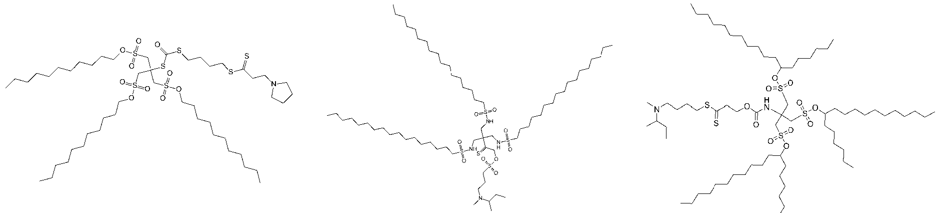

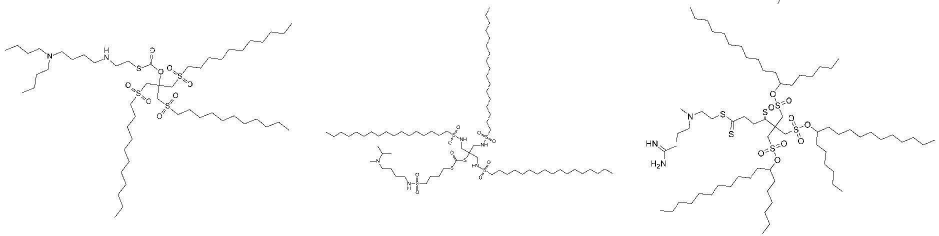

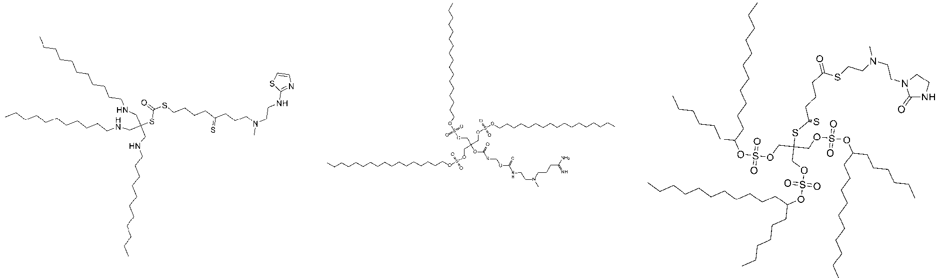

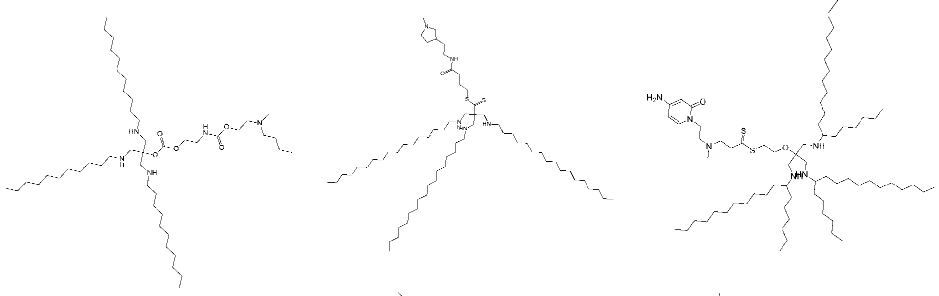

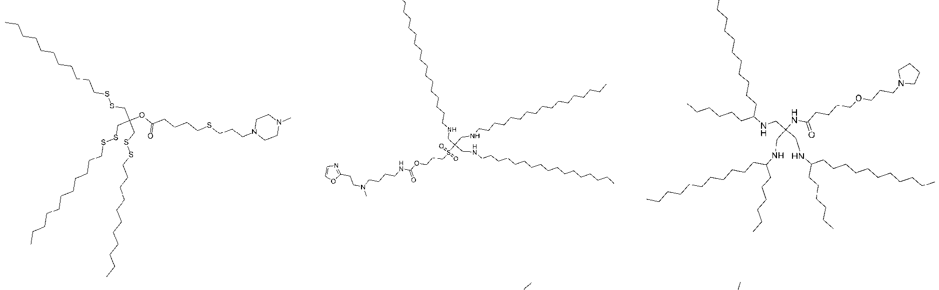

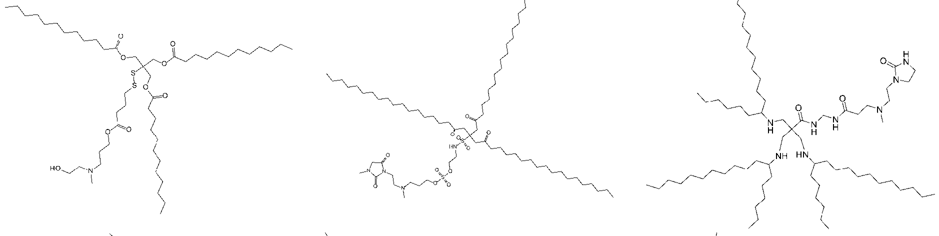

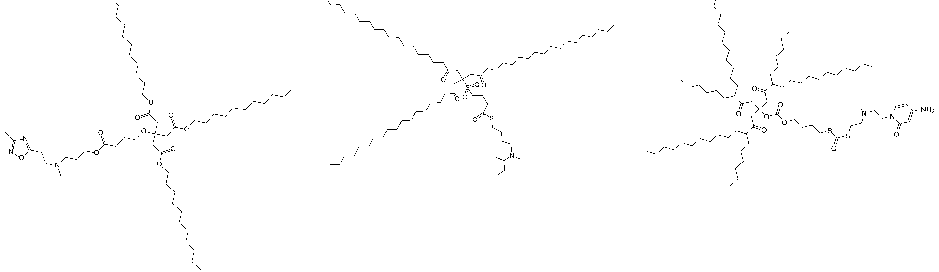

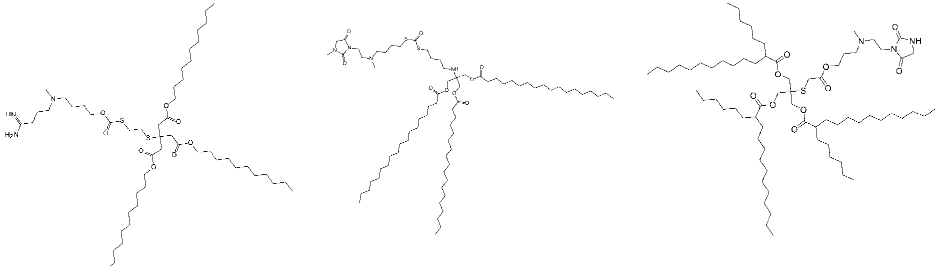

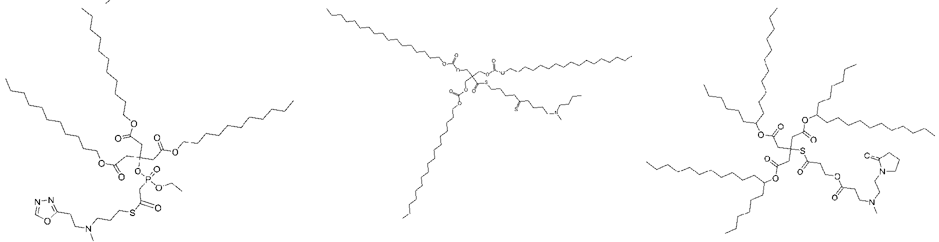

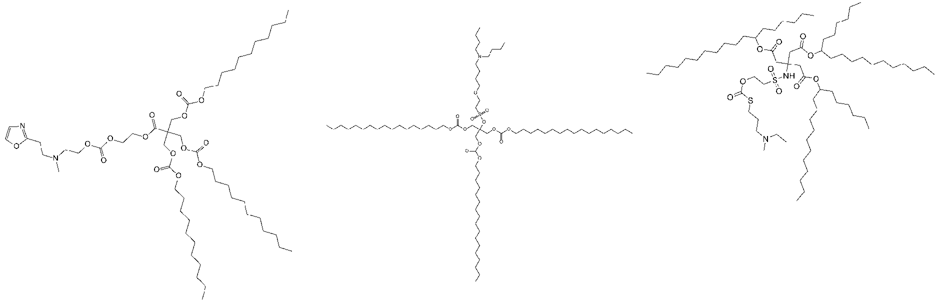

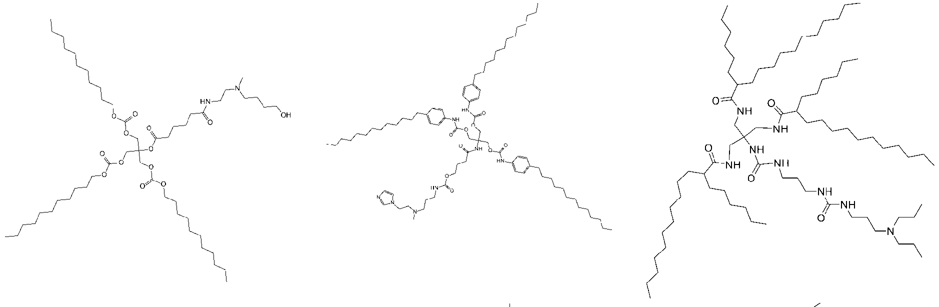

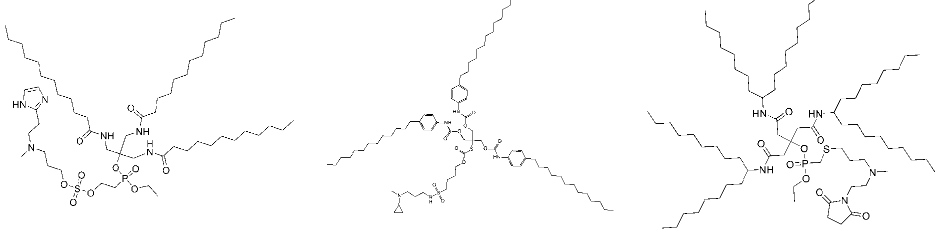

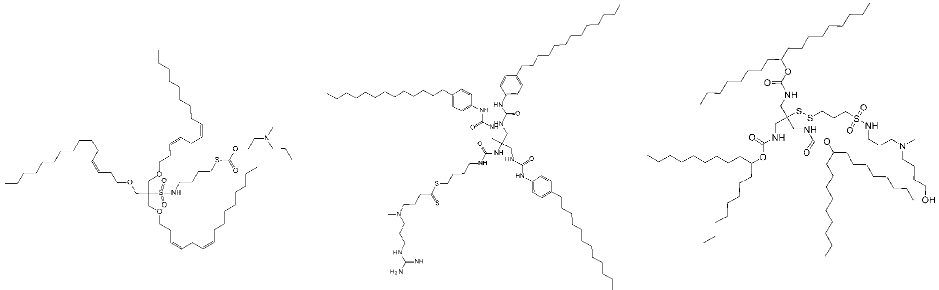

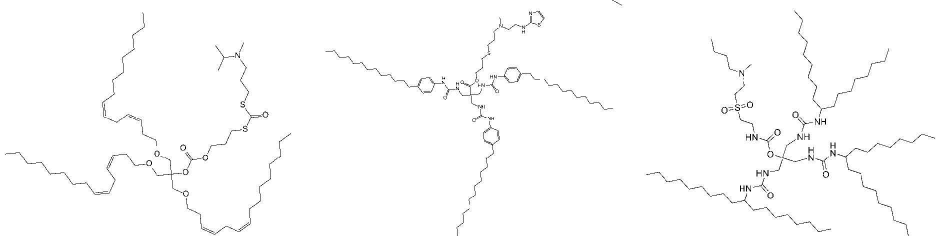

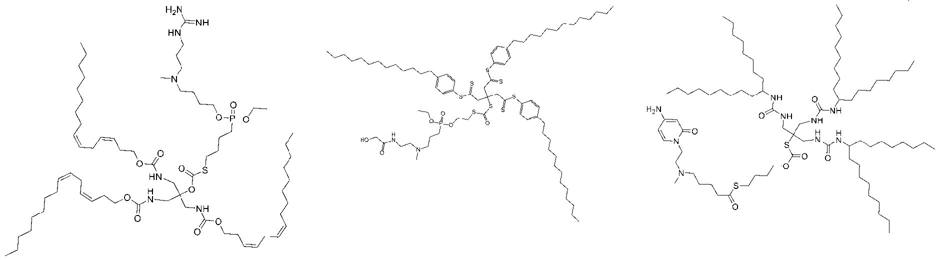

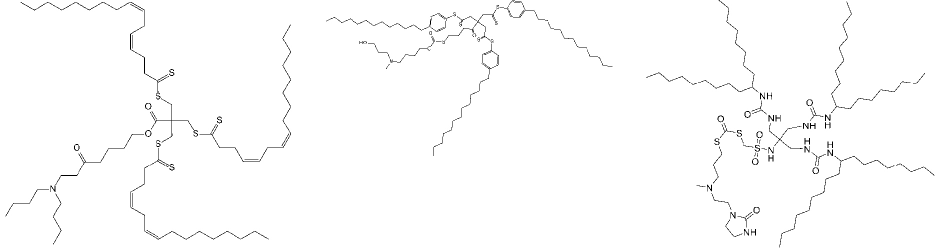

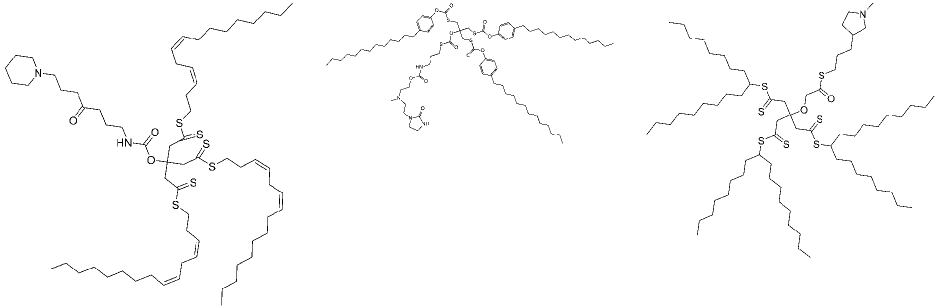

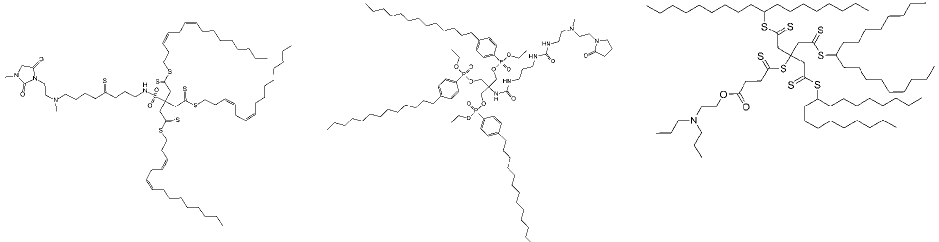

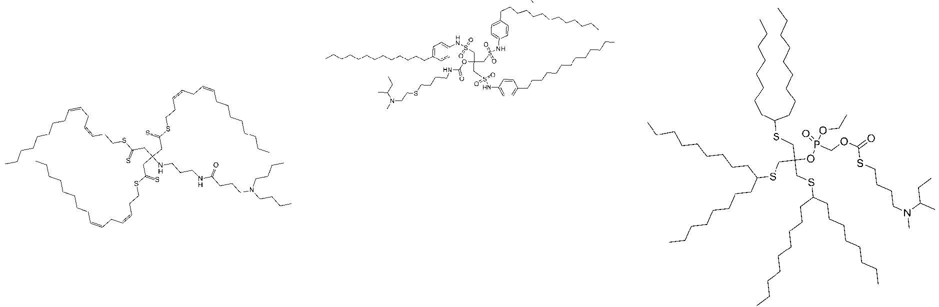

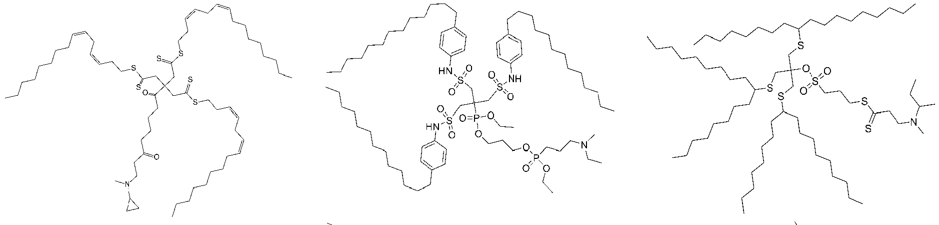

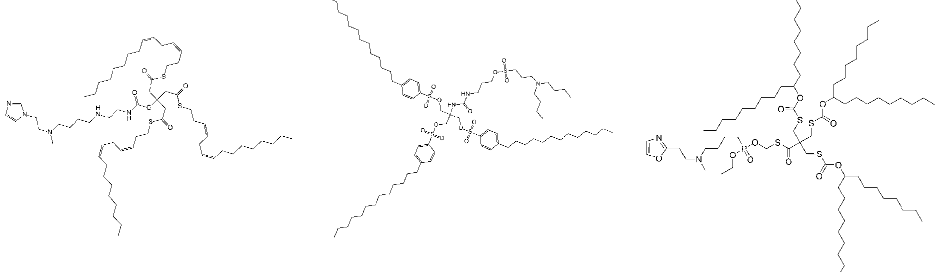

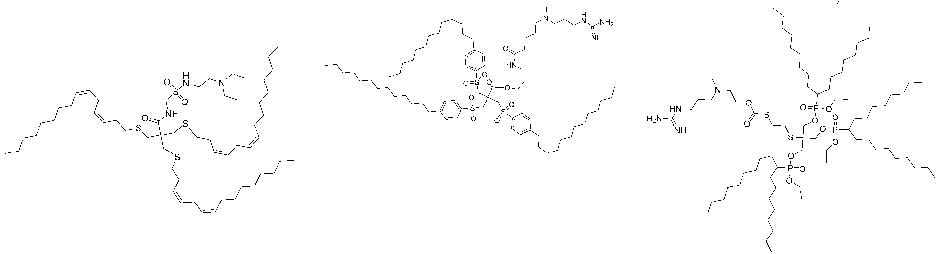

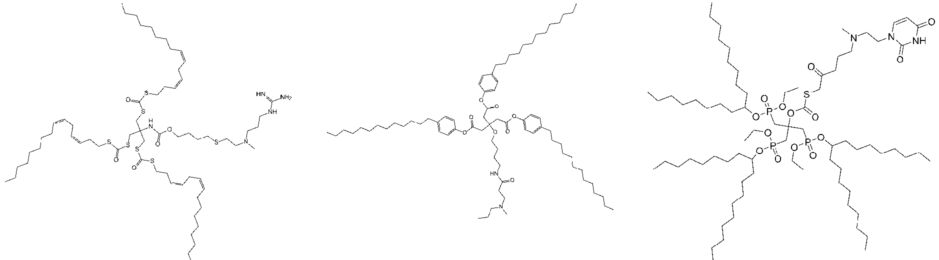

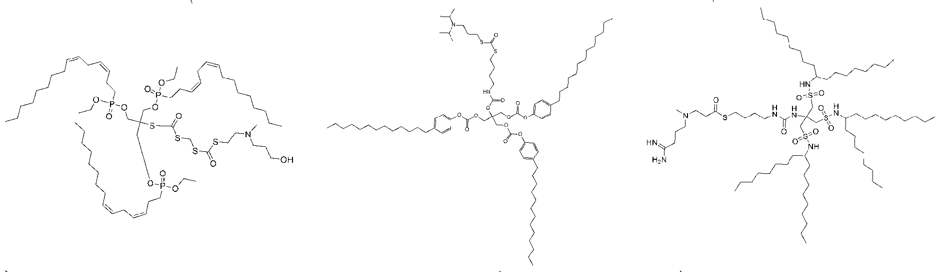

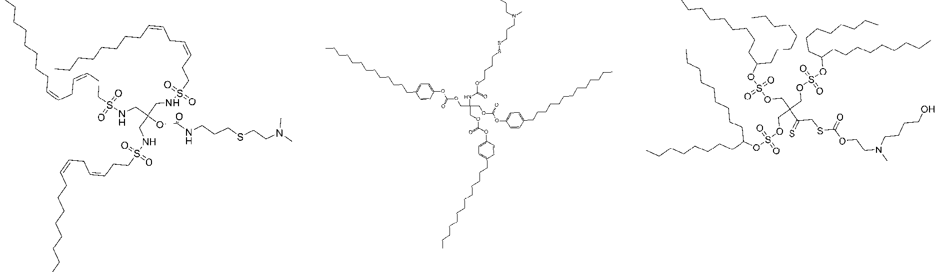

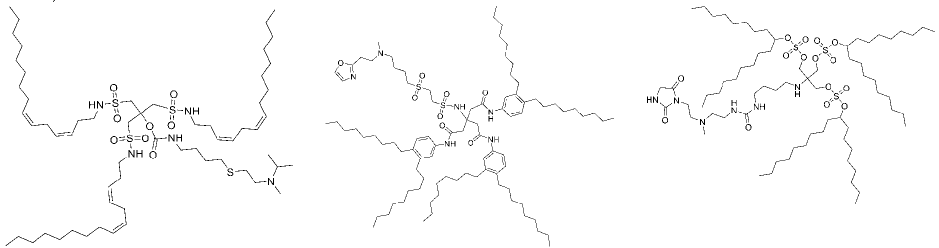

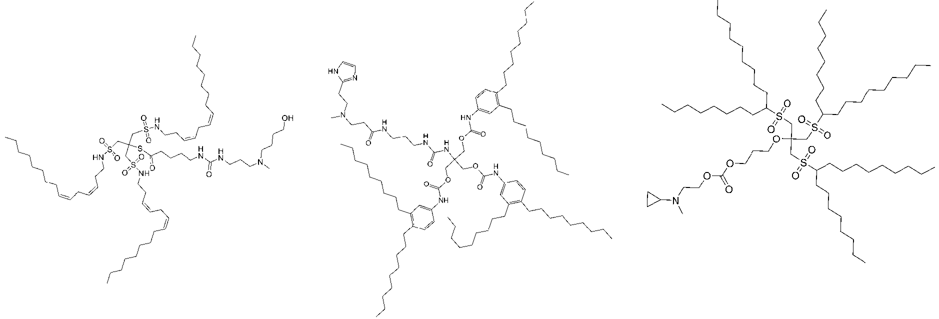

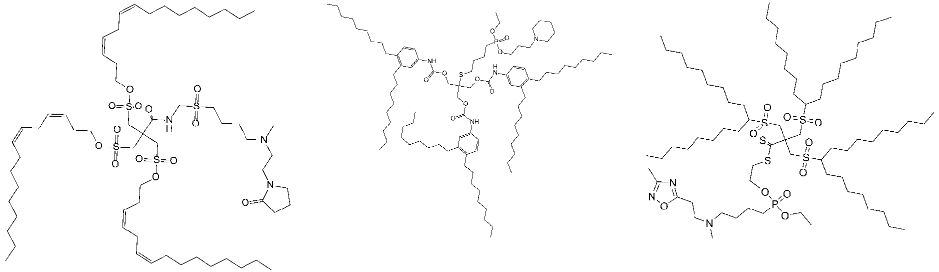

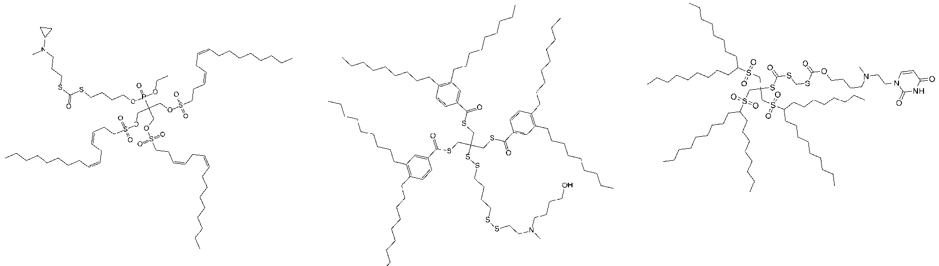

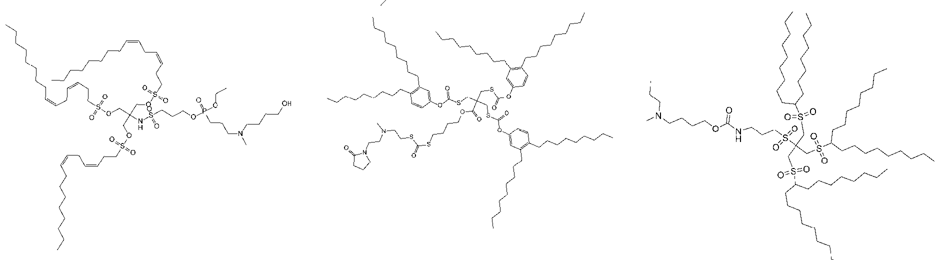

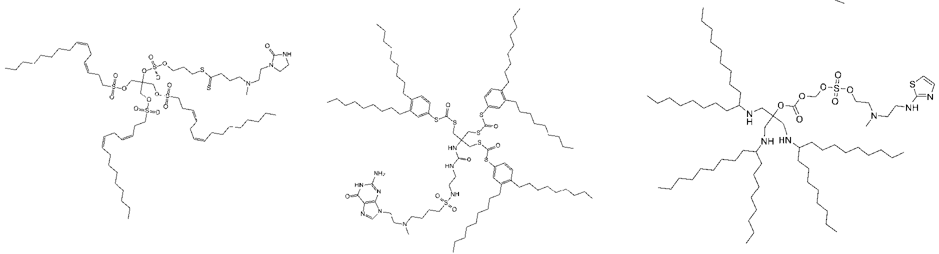

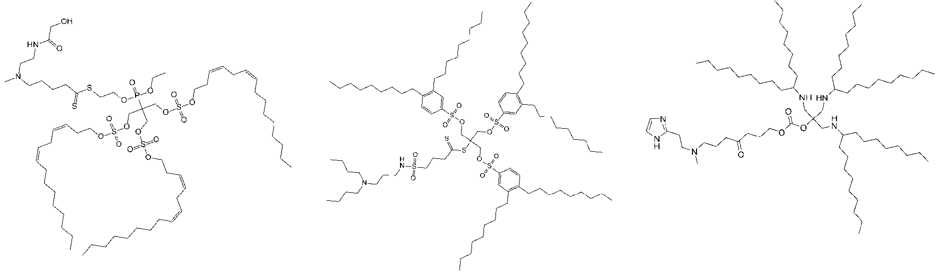

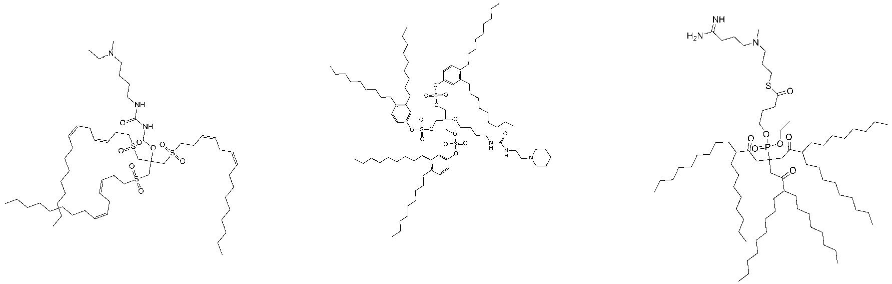

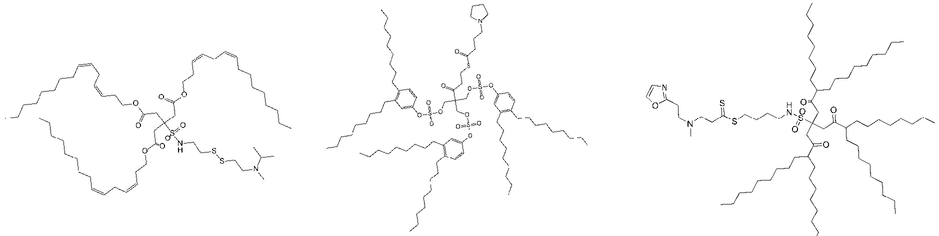

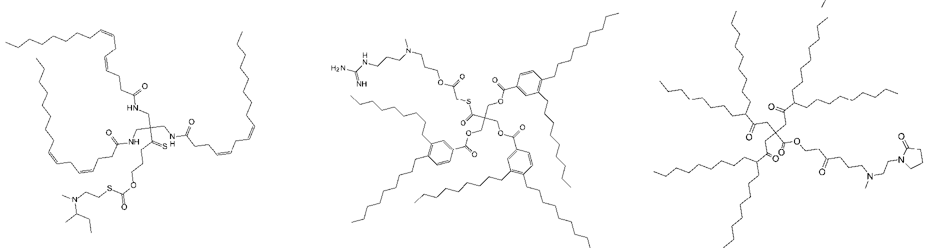

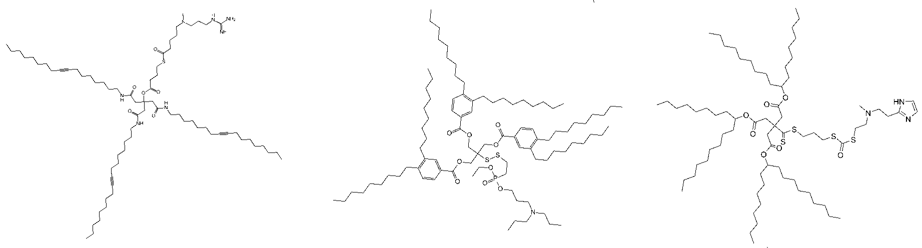

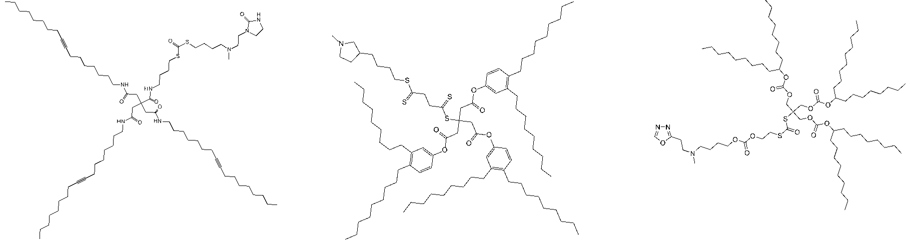

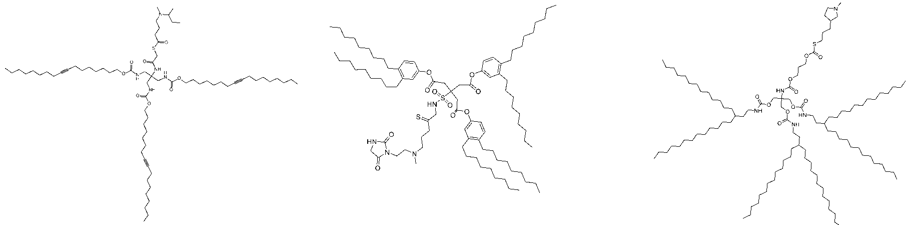

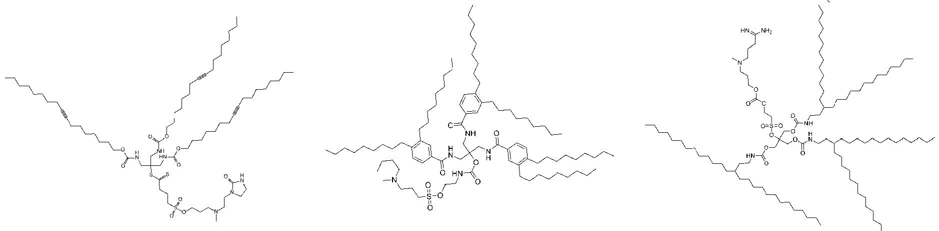

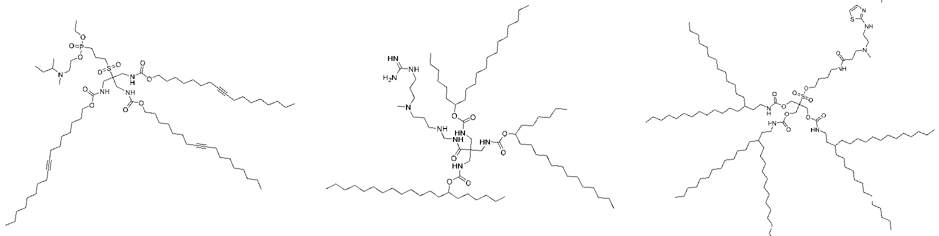

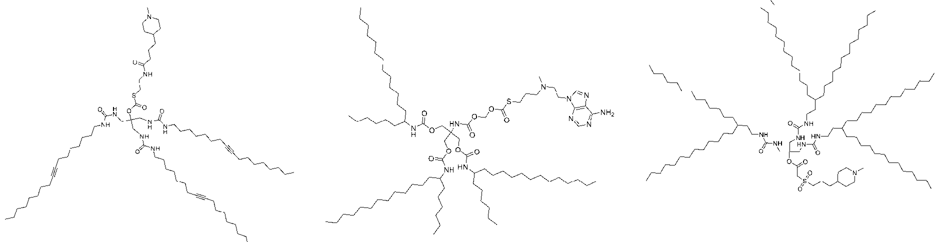

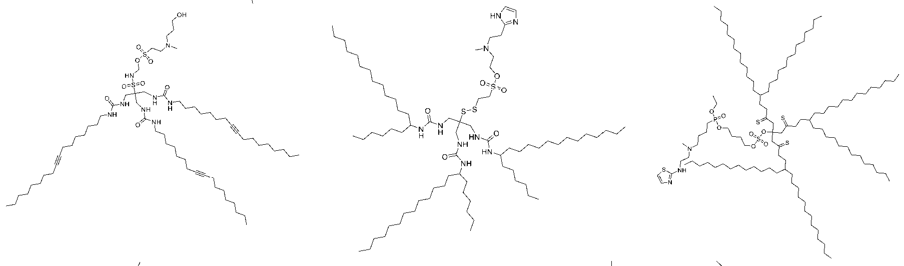

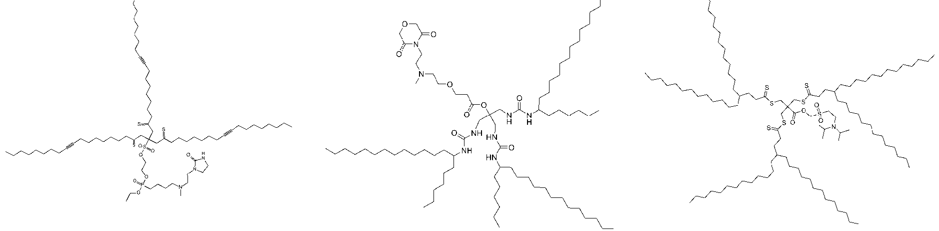

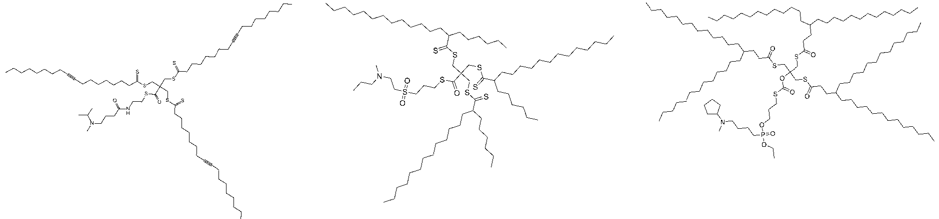

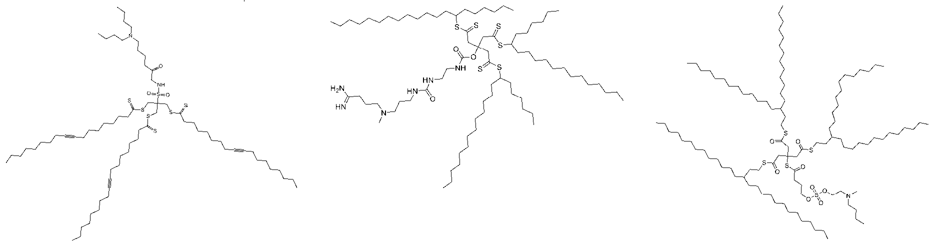

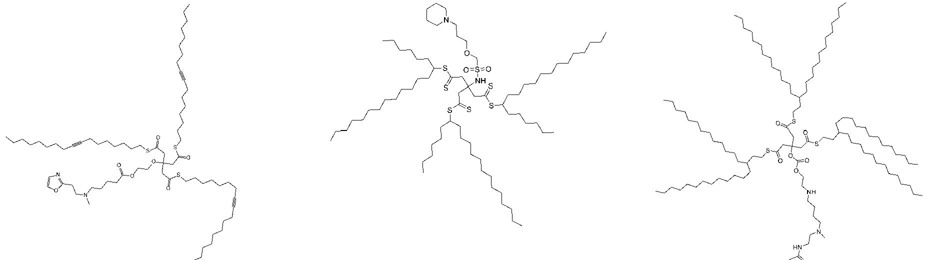

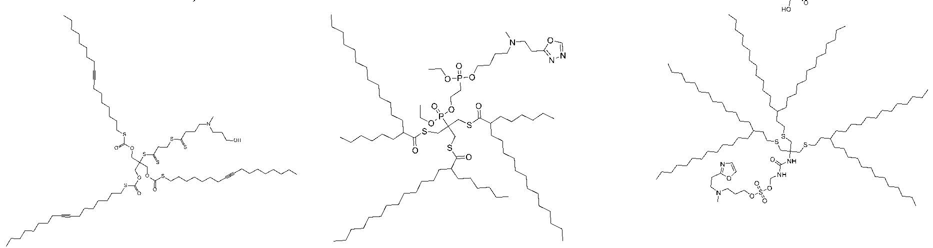

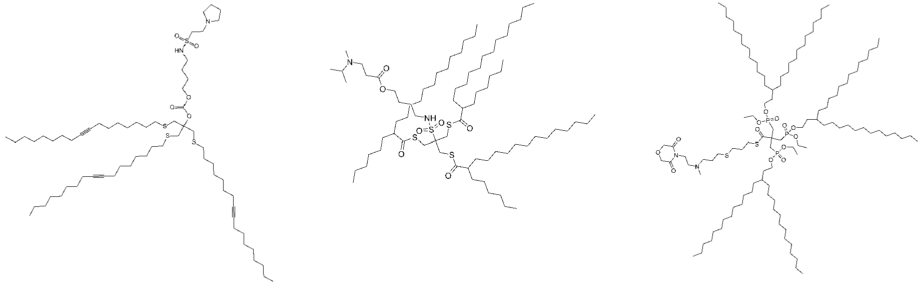

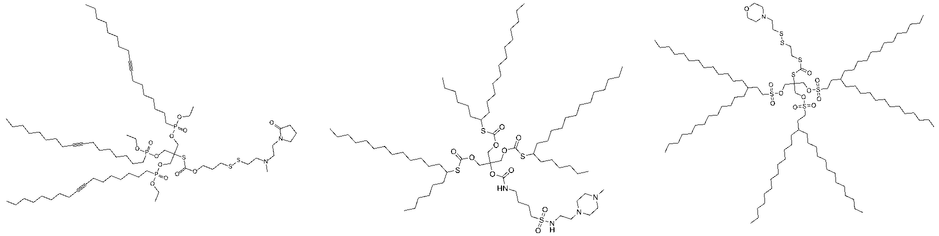

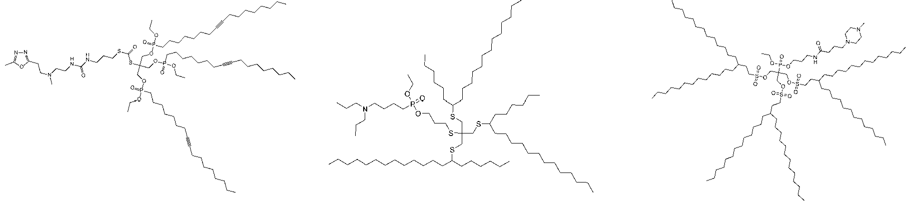

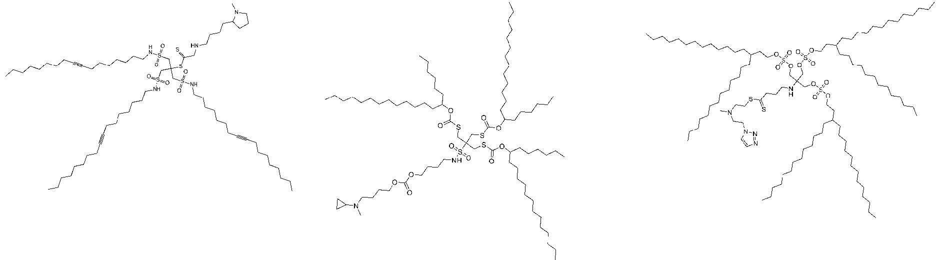

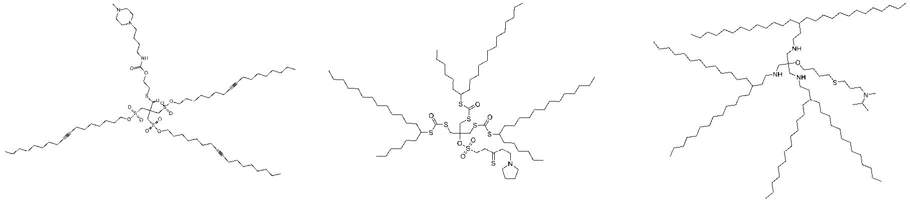

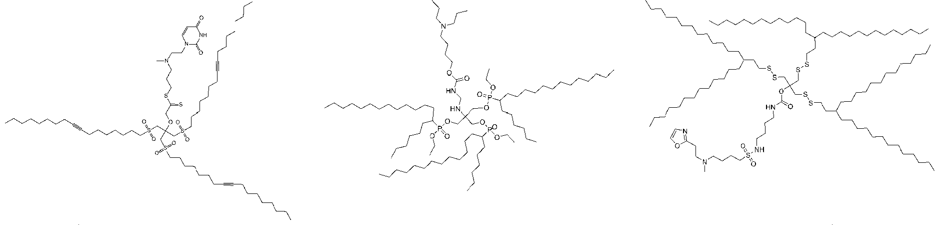

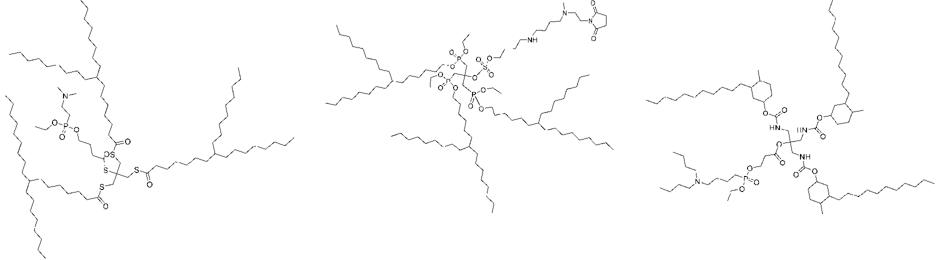

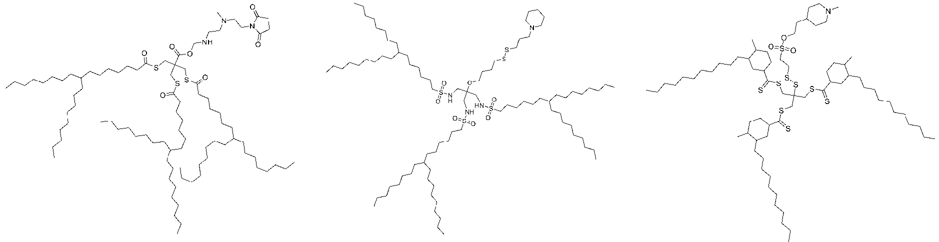

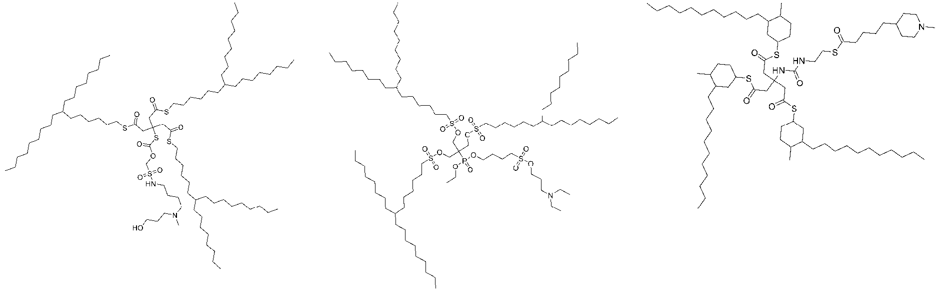

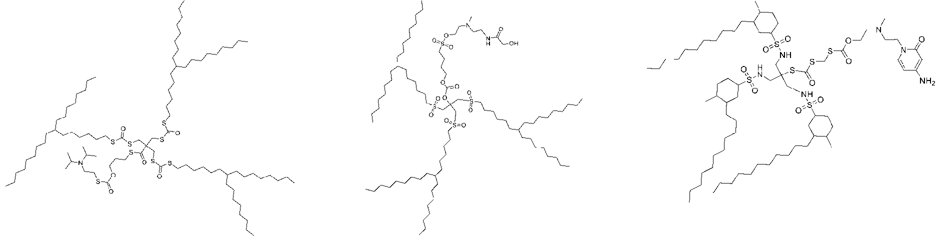

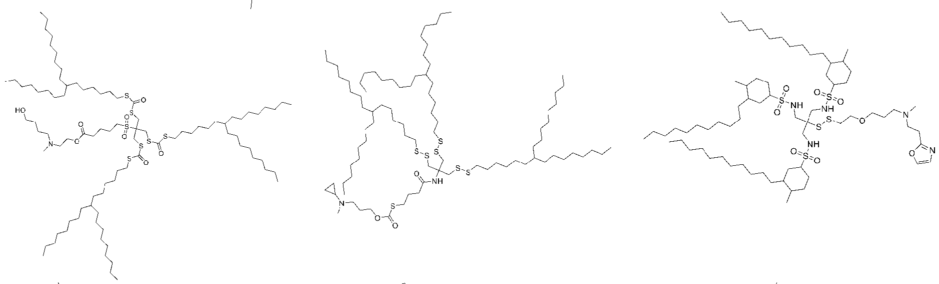

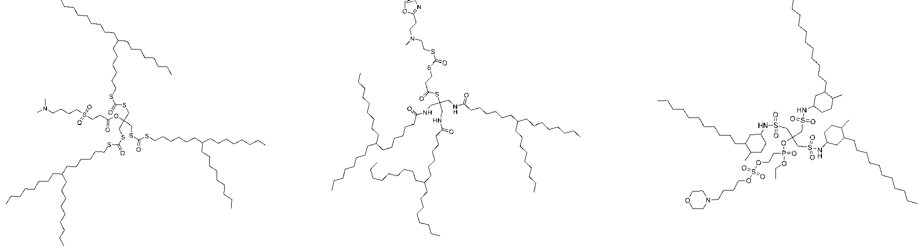

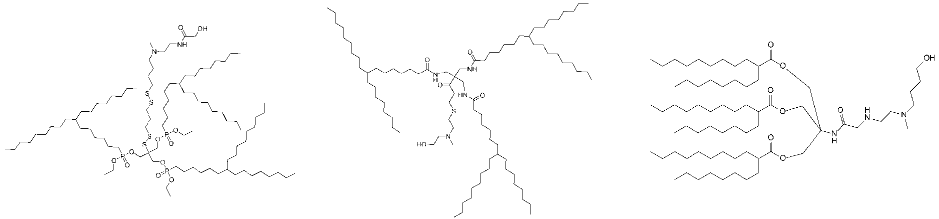

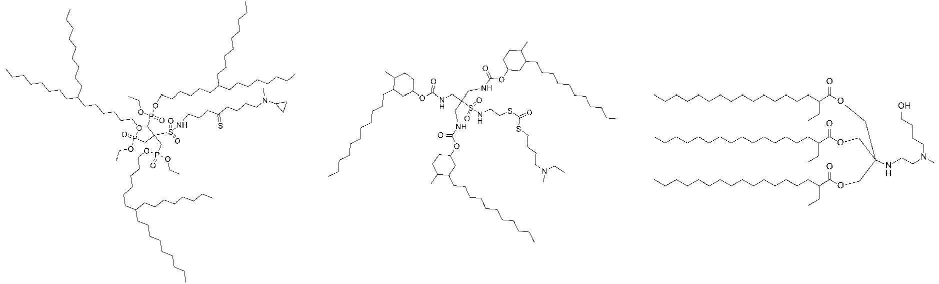

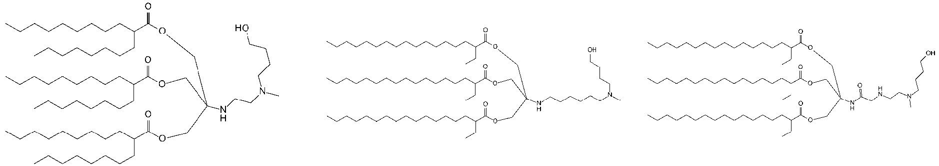

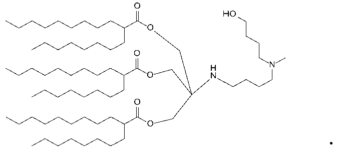

23. A method for preparing the ionizable cationic lipid compound according to any one of claims 1-22, comprising a step of reacting a compound of Formula (II) with a compound of Formula (III): 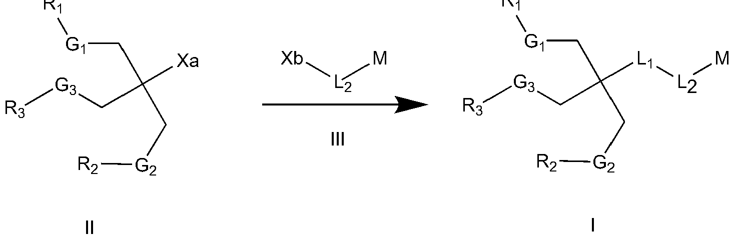 wherein Xₐ and X_{b} are each a group containing a leaving group or a nucleophilic group, and Xₐ and X_{b} form L₁ through a nucleophilic reaction or a condensation reaction.

24. A method for preparing the ionizable cationic lipid compound according to any one of claims 1-22, comprising a step of reacting a compound of Formula (IV) with a compound of Formula (V): 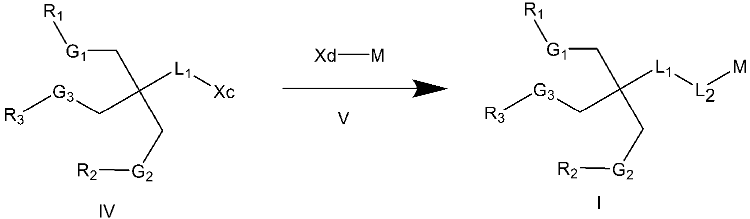 wherein X_{c} and X_{d} are each a group containing a leaving group or a nucleophilic group, and X_{c} and X_{d} form L₂ through a nucleophilic reaction or a condensation reaction.

25. A method for preparing the ionizable cationic lipid compound according to any one of claims 1-22, comprising a step of subjecting a compound of Formula (VI) to a reaction: 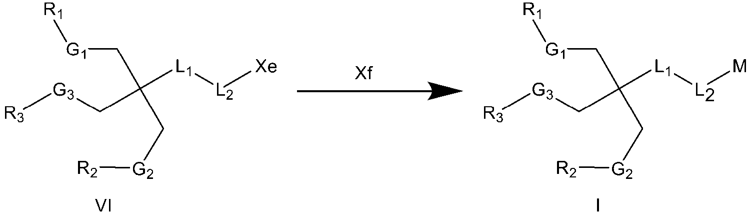 wherein Xₑ is a group containing a leaving group or a nucleophilic group, X_{f} is a compound containing a leaving group or a nucleophilic group, and Xₑ and X_{f} form M through a nucleophilic reaction or a condensation reaction.

26. A method for preparing the ionizable cationic lipid compound according to any one of claims 1-22, comprising a step of reacting a compound of Formula (VII) sequentially with a compound of Formula (VIII), a compound of Formula (IX), and a compound of Formula (X): 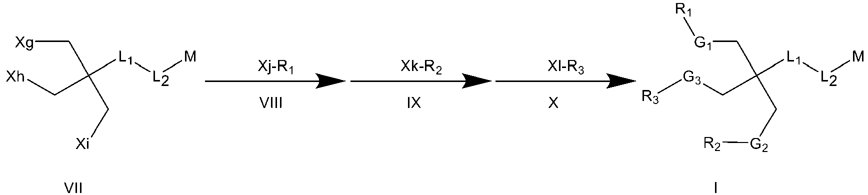 wherein X_{g}, Xₕ, Xᵢ, Xⱼ, Xₖ, and Xₗ are each a group containing a leaving group or a nucleophilic group, and X_{g} and Xⱼ form G₁ through a nucleophilic reaction or a condensation reaction, Xₙ and Xₖ form G₂ through a nucleophilic reaction or a condensation reaction, and Xᵢ and Xₗ form G₃ through a nucleophilic reaction or a condensation reaction.

27. A method for preparing the ionizable cationic lipid compound according to any one of claims 1-22, comprising a step of reacting a compound of Formula (XI) with a compound of Formula (XII): 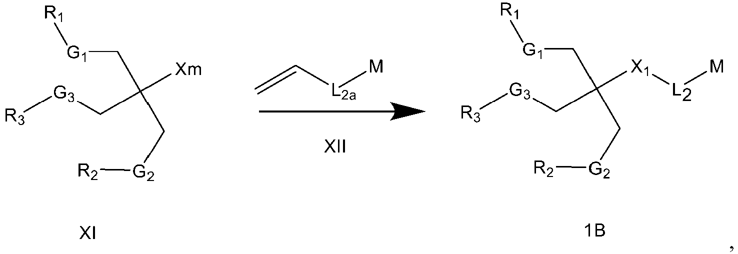 or
a step of reacting a compound of Formula (XIII) with a compound of Formula (XII): 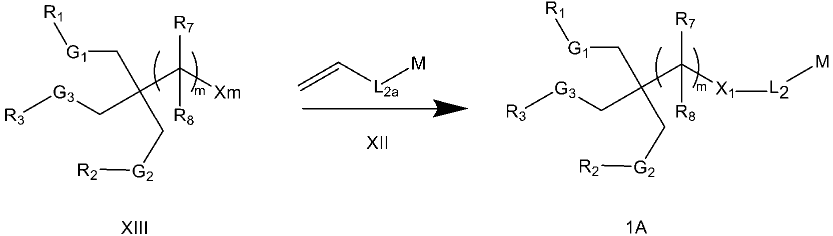
wherein Xₘ is a group containing a nucleophilic group, and Xₘ reacts with C=C-L₂ₐ of the compound of Formula (XII) through an addition reaction to form X₁-L₂.

28. The method according to any one of claims 23 to 27, wherein R₁, R₂ and R₃ are each independently the following group: 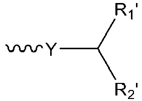
wherein Y, R₁' and R₂' are as defined in claim 2;
wherein the method further comprises a step of forming tail chains R₁, R₂ and R₃: 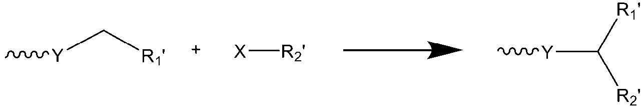
wherein X is a leaving group.

29. The method according to any one of claims 23-27, wherein the raw materials used in the reaction further contain protective groups, and the reaction steps include steps of protection and/or deprotection.

30. Use of the ionizable cationic lipid compound according to any one of claims 1-22 as a surfactant.

31. The use according to claim 30, wherein the ionizable cationic lipid compound is used as emulsifier, suspending agent, dispersant, solubilizer, lubricant, thickener, bacteriostatic agent, or preservative.

32. Use of the ionizable cationic lipid compound according to any one of claims 1-22 in preparing a cosmetic composition.

33. Use of the ionizable cationic lipid compound according to any one of claims 1-22 in preparing a lipid composition, wherein the ionizable cationic lipid compound is used as a drug delivery vehicle.

34. A lipid composition, wherein the lipid components of the lipid composition comprise the ionizable cationic lipid compound according to any one of claims 1-22.

35. The lipid composition according to claim 34, wherein the lipid components of the lipid composition further comprise additional lipids, including phospholipids, structural lipids, and PEG-conjugated lipids; the molar ratio of the ionizable cationic lipid compound to additional lipid components is 1:0.2-5; the active ingredient of the lipid nanoparticle composition comprises a therapeutic agent and/or prophylactic agent; the therapeutic agent and/or prophylactic agent is a vaccine or compound capable of eliciting an immune response; and the mass ratio of the lipid components to the active ingredient is 10:1 to 60:1.

36. The lipid composition according to claim 35, wherein the therapeutic agent and/or prophylactic agent is a nucleic acid; and the nucleic acid is DNA or RNA.

37. Use of the lipid composition according to any one of claims 34-36 in a medicament for treating a disease in a mammal, wherein the disease is **characterized by** dysfunctional or abnormal protein or polypeptide activity; and the disease is selected from infectious diseases, cancer, proliferative diseases, genetic diseases, autoimmune diseases, diabetes, neurodegenerative diseases, cardiovascular diseases, renovascular diseases, and metabolic diseases.

38. A pharmaceutical composition, comprising the lipid composition according to any one of claims 34-36 and a pharmaceutically acceptable carrier.

39. The pharmaceutical composition according to claim 38, wherein the pharmaceutical composition further comprises a pharmaceutically active compound selected from anti-inflammatory compounds, steroids, statins, estradiol, BTK inhibitors, S1P1 agonists, glucocorticoid receptor modulators, and antihistamines.
